(19) Europäisches Patentamt
European Patent Office
Office européen des brevets

(11) EP 4 289 428 A1

(12) EUROPEAN PATENT APPLICATION
published in accordance with Art. 153(4) EPC

(43) Date of publication:
13.12.2023 Bulletin 2023/50

(21) Application number: 22748921.8

(22) Date of filing: 24.01.2022

(51) International Patent Classification (IPC):
*A61K 31/444* (2006.01) *C07D 519/00* (2006.01)
*A61P 35/00* (2006.01)

(52) Cooperative Patent Classification (CPC):
A61K 31/437; A61K 31/439; A61K 31/444;
A61K 31/4545; A61K 31/497; A61P 35/00;
C07D 471/04; C07D 519/00

(86) International application number:
PCT/CN2022/073467

(87) International publication number:
WO 2022/166642 (11.08.2022 Gazette 2022/32)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 08.02.2021 CN 202110172167

(71) Applicant: Applied Pharmaceutical Science, Inc.
Beijing 100176 (CN)

(72) Inventors:
- ZHONG, Jun
  Beijing 100176 (CN)
- ZHU, Yongkuan
  Beijing 100176 (CN)
- LIU, Yongbo
  Beijing 100176 (CN)
- CHEN, Xiaohu
  Beijing 100176 (CN)
- LIU, Libin
  Beijing 100176 (CN)
- CHEN, Minchun
  Beijing 100176 (CN)
- WANG, Hao
  Beijing 100176 (CN)
- GAO, Qin
  Beijing 100176 (CN)

(74) Representative: Gille Hrabal
Partnerschaftsgesellschaft mbB
Patentanwälte
Brucknerstraße 20
40593 Düsseldorf (DE)

# (54) NITROGEN-CONTAINING POLYCYCLIC FUSED RING COMPOUND, PHARMACEUTICAL COMPOSITION THEREOF, PREPARATION METHOD THEREFOR, AND USE THEREOF

(57) A nitrogen-containing polycyclic fused ring compound represented by formula I, a pharmaceutical composition thereof, a preparation method therefor, and a use thereof. As a highly selective and very effective RET inhibitor, the compound has a strong inhibitory effect on RET gatekeeper residue mutant RET V804M mutation, RET solvent front residue mutant G810R, and other clinically relevant RET mutants and RET-wt. The compound can also significantly inhibit the growth of a TT cell line derived from thyroid cancer and Ba/F3 cells transformed by various RET mutants, and has a stronger inhibition effect than selective RET inhibitor LOXO-292. In addition, the compound can greatly block cellular RET autophosphorylation and downstream pathways, and can significantly induce TT cell death.

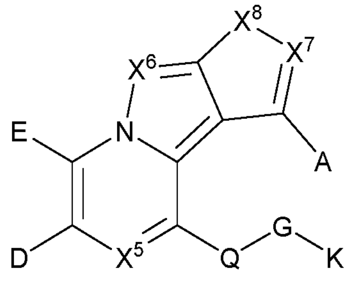

I

EP 4 289 428 A1

## Description

The present application claims priority to Chinese Patent Application No. 202110172167.6 filed with China National Intellectual Property Administration on Feb. 8, 2021, entitled "NITROGEN-CONTAINING POLYCYCLIC FUSED RING COMPOUND, PHARMACEUTICAL COMPOSITION THEREOF, PREPARATION METHOD THEREFOR AND USE THEREOF", which is incorporated herein by reference in its entirety.

## TECHNICAL FIELD

The present disclosure relates to the field of medicinal chemistry, and in particular to a nitrogen-containing polycyclic fused ring compound, a pharmaceutical composition thereof, a preparation method therefor and use thereof.

## BACKGROUND

Researches have demonstrated that mutations in RET genes are closely related to the occurrence of many diseases, including papillary thyroid carcinoma (PTC) (Cell, 1990, 60(4):557-563), medullary thyroid carcinoma (MTC) (hyroid, 2009, 19(6):565-612), multiple endocrineneoplasia type II (MEN2) (Endocr Rev, 2006, 27(5):535-560), Hirschsprung's disease (Proc Natl Acad Sci USA, 2000, 97(1):268-273), lung adenocarcinoma (Nat Med, 2012, 18(3):375-377), and the like. At present, only four RET fusion genes of KIF5B-RET, CCDC6-RET, TRIM33-RET, NCOA4-RET have been reported in non-small cell lung cancer, with KIF5B-RET predominated (Cancer, 2013, 119(8):1486-1494). KIF5B-RET, a fusion gene formed by the chromosome inversion (p11; q 11) of KIF5B (kinesin family member 5B) gene and RET gene, was first demonstrated in adenocarcinoma in non-smoking Koreans through whole-genome and transcriptome sequencing. KIF5B-RET accounts for a low proportion in lung cancer patients, but is common in non-smokers and adenocarcinoma patients, and is repelled with other mutations such as EGFR, KRAS, BRAF, ErbB2 and EML4-ALK (Genome Res, 2012, 22(3):436-445). The KIF5B-RET fusion protein contains a motor domain and a coiled-coil domain of KIF5B. Through the dimerization of the coiled-coil domain, the tyrosine kinase of RET in the fusion protein can be abnormally activated, thereby promoting lung tumorigenesis (Cancer, 2011, 117(12):2709-2718). In the research by Qian et al (Mol Cancer, 2014, 13: 176), KIF5B-RET fusion kinase was demonstrated to have significant oncogenic activity both *in vitro* and *in vivo*, and the STAT3 signal transduction pathway may be the main downstream mediator of tumorigenesis. It is evident that KIF5B-RET can regulate the continuous activation of STAT3. KIF5B-RET fusion kinase can bind to STAT3 to directly phosphorylate and activate STAT3-Tyr705. It also mediates the activation of STAT3-Tyr705 through the JAK/STAT3-dependent pathway, and triggers the phosphorylation of Ser727 through the RAS/RAF/MEK/ERK1 pathway.

The demonstration that the RET fusions are drivers in some cancers facilitates the use of multi-kinase inhibitors, which have RET inhibitory activity, in the treatment of tumor patients loaded with RET fusion proteins. At present, there are no approved agents that can specifically target this oncogene. The current treatments for RET-specific cancers are limited to multi-kinase inhibitors and chemotherapies, which, however, have poor clinical performance - undesirable ORR (objective response rate) and significant off-target toxicity. Furthermore, one of the biggest challenges in cancer treatment is that tumor cells may become resistant to treatment after a certain duration. Generally, such resistance greatly limits the treatment options for patients, and in most cases, the cancer remains progressive and uncontrolled. It is found that in many human cancers such as thyroid cancer, the RET kinase signal transduction plays an important role. The mutation in gatekeeper residue RET 804V of RET is an important cause of tumor resistance to currently approved non-selective RET inhibitors (such as cabozantinib and vandetanib). One of the important mutations in the extracellular or intracellular domain of RET in isolated familial medullary thyroid carcinoma, i.e., the mutation in gatekeeper residue V804M in the kinase ATP binding site, leads to a decrease in the affinity of existing drugs for the ATP binding site. It has been reported in the document (RET Solvent Front Mutations Mediate Acquired Resistance to Selective RET Inhibition in RET-Driven Malignancies, Journal of Thoracic Oncology, 2020, Vol. 15, No. 4, 541-549) that upon the use of the selective RET inhibitor LOXO-292 (selpercatinib), the aforementioned RET 804V mutation can be avoided, but there are still other mutations that lead to drug resistance. For example, mutations in residue G810 such as G810R, G810S, and G801C, which can result in a solvent-front of the kinase ATP binding site in non-small cell lung cancer, lead to a decrease in the binding of LOXO-292 to the ATP binding site, resulting in drug resistance and cancer progression. Accordingly, there is a need for developing RET mutation inhibitors with improved performance.

## SUMMARY

To solve the problems described above, the present disclosure provides a compound represented by formula I, or a stereoisomer, a racemate, a tautomer, an isotopically labeled compound, a nitrogen oxide or a pharmaceutically acceptable salt thereof:

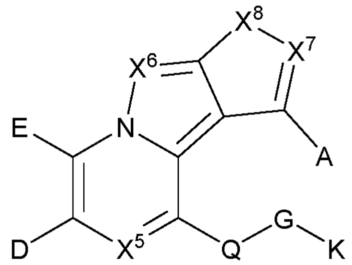

I

wherein Q is selected from

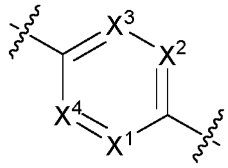 and 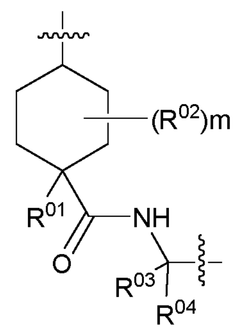 ;

wherein $X^1$, $X^2$, $X^3$, $X^4$, $X^5$, $X^6$, and $X^7$ are identical or different, and are independently selected from $CR^1$ and N; $X^8$ is selected from $CR^1R^{1'}$ and $NR^1$;

wherein each $R^1$ and $R^{1'}$ is identical or different, and is independently selected from H, halogen, CN, OH, and the following groups unsubstituted or optionally substituted with one, two, or more substituents independently selected from $R^a$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $NR^2R^3$, $-C(O)R^4$, $-OCR^5$, $-S(O)_2R^6$, and $OS(O)_2R^7$;

A is selected from H, halogen, CN, OH, $NH_2$, and the following groups unsubstituted or optionally substituted with one, two, or more substituents independently selected from $R^b$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $NR^2R^3$, $-C(O)R^4$, $-OCR^5$, $-S(O)_2R^6$, and $OS(O)_2R^7$;

D and E are identical or different, and are independently selected from H, halogen, CN, OH, $B(OH)_2$, $-C(O)R^4$, $-OCR^5$, $-S(O)_2R^6$, $OS(O)_2R^7$, $-O-R^{21}$, $C(O)OR^{22}$, $-P(O)R^{23}R^{24}$, and the following groups unsubstituted or optionally substituted with one, two, or more substituents independently selected from $R^c$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, and $NH_2$, provided that at least one of D and E is not H, e.g., at least one of D and E is selected from $-O-R^{21}$, or at least one of D and E is selected from the following groups unsubstituted or optionally substituted with one, two, or more substituents independently selected from $R^c$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, and $NH_2$;

$R^{21}$, $R^{22}$, $R^{23}$, and $R^{24}$ are identical or different, and are independently selected from H and the following groups unsubstituted or optionally substituted with one, two, or more substituents independently selected from $R^b$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, and 3- to 20-membered heterocyclyl;

G is selected from halogen and the following groups unsubstituted or optionally substituted with one, two, or more substituents independently selected from $R^e$: 5- to 20-membered heteroaryl having at least one heteroatom selected from N, 3- to 20-membered heterocyclyl having at least one heteroatom selected from N, and $C_{3-40}$ cycloalky-NH-;

K is absent or selected from H, halogen, CN, OH, and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$,

$-OCR^5$, $-S(O)_2R^6$, and $OS(O)_2R^7$;

each $R^2$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $-C(O)R^4$, and $-S(O)_2R^6$;

each $R^3$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $-C(O)R^4$, and $-S(O)_2R^6$;

or, $R^2$ and $R^3$, together with the N atom attached thereto, form the following groups unsubstituted or optionally substituted with one, two, or more substituents independently selected from $R^f$: 5- to 20-membered heteroaryl and 3- to 20-membered heterocyclyl;

each $R^4$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, and $-NR^2R^3$;

each $R^5$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkylcarbonyl, $C_{2-40}$ alkenylcarbonyl, $C_{2-40}$ alkynylcarbonyl, $C_{3-40}$ cycloalkylcarbonyl, $C_{3-40}$ cycloalkenylcarbonyl, $C_{3-40}$ cycloalkynylcarbonyl, $C_{6-20}$ arylcarbonyl, 5- to 20-membered heteroarylcarbonyl, and 3- to 20-membered heterocyclylcarbonyl;

each $R^6$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, and $-NR^2R^3$;

each $R^7$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, and 3- to 20-membered heterocyclyl;

m is 0, 1, 2, 3, 4, 5, 6, 7, or 8;

each $R^{01}$, $R^{02}$, $R^{03}$, and $R^{04}$ is identical or different, and is independently selected from H, halogen, CN, OH, SH, oxo (=O), $NO_2$, and the following groups unsubstituted or optionally substituted with one, two, or more substituents independently selected from $R^g$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$, $-OCR^5$, $-S(O)_2R^6$, and $OS(O)_2R^7$;

each $R^a$, $R^b$, $R^c$, $R^d$, $R^e$, and $R^f$ is identical or different, and is independently selected from halogen, CN, OH, SH, oxo (=O), $NO_2$, and the following groups unsubstituted or optionally substituted with one, two, or more substituents independently selected from $R^g$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$, $-OCR^5$, $-S(O)_2R^6$, and $OS(O)_2R^7$;

each $R^g$ is identical or different, and is independently selected from halogen, CN, OH, SH, oxo (=O), $NO_2$, and the following groups unsubstituted or optionally substituted with one, two, or more substituents independently selected from $R^h$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$, $-OCR^5$, $-S(O)_2R^6$, and $OS(O)_2R^7$; or, when a cyclic group

(including but not limited to, $C_{3-40}$ cycloalkyl, $C_{3-40}$ cycloalkenyl, $C_{3-40}$ cycloalkynyl, 3- to 20-membered heterocyclyl, and the like) is substituted with two or more substituents at different positions, two of the substituents can also form a bridged ring with the cyclic group, wherein the bridge atoms other than the bridgehead atoms in the bridged ring can comprise 1, 2, 3, 4, or 5 divalent groups selected from $CH_2$, O, and NH;

each $R^h$ is identical or different, and is independently selected from halogen, CN, OH, SH, oxo (=O), $NO_2$, and the following groups unsubstituted or optionally substituted with one, two, or more substituents independently selected from $R^i$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$, $-OCR^5$, $-S(O)_2R^6$, and $OS(O)_2R^7$;

each $R^i$ is identical or different, and is independently selected from halogen, CN, OH, SH, oxo (=O), $NO_2$, and the following groups unsubstituted or optionally substituted with one, two, or more substituents $R^j$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$, $-OCR^5$, $-S(O)_2R^6$, and $OS(O)_2R^7$;

or, when a cyclic group (including but not limited to, $C_{3-40}$ cycloalkyl, $C_{3-40}$ cycloalkenyl, $C_{3-40}$ cycloalkynyl, 3- to 20-membered heterocyclyl, and the like) is substituted with two or more substituents at different positions, two of the substituents can also form a bridged ring with the cyclic group, wherein the bridge atoms other than the bridgehead atoms in the bridged ring can comprise 1, 2, 3, 4, or 5 divalent groups selected from $CH_2$, O, and NH;

or, when one atom (e.g., carbon atom or nitrogen atom) is substituted with two or more substituents, two of the substituents can also, together with the shared atom connected thereto, form the following groups unsubstituted or optionally substituted with one, two, or more substituents independently selected from $R^f$: cyclic groups unsubstituted or optionally substituted with one, two, or more substituents independently selected from $R^f$ (including but not limited to, the following groups unsubstituted or optionally substituted with one, two, or more substituents independently selected from $R^f$: $C_{3-40}$ cycloalkyl, $C_{3-40}$ cycloalkenyl, $C_{3-40}$ cycloalkynyl, 5- to 20-membered heteroaryl, 3- to 20-membered heterocyclyl, and the like).

For example, when $NH_2$ is substituted with two substituents $R^c$, the two substituents $R^c$ may, together with the shared nitrogen atom connected thereto, form a 5- to 20-membered heteroaryl or 3- to 20-membered heterocyclyl unsubstituted or optionally substituted with one, two, or more substituents independently selected from $R^f$, such as 5-, 6-, or 7-membered heteroaryl unsubstituted or optionally substituted with one, two, or more substituents independently selected from $R^f$, or 3-, 4-, 5-, 6-, or 7-membered heterocyclyl unsubstituted or optionally substituted with one, two, or more substituents independently selected from $R^f$.

According to an embodiment of the present disclosure, $X^1$, $X^2$, $X^3$, $X^4$, $X^5$, $X^6$, and $X^7$ are identical or different, and are independently selected from $CR^1$ and N; for example, at least one, e.g., 1, 2, 3, 4, 5, 6, or 7, of $X^1$, $X^2$, $X^3$, $X^4$, $X^5$, $X^6$, and $X^7$ is N.

According to an embodiment of the present disclosure, $X^8$ is selected from $CR^1R^{1'}$ and $NR^1$. According to an embodiment of the present disclosure, each $R^1$ and $R^{1'}$ is identical or different, and is independently selected from H, halogen, CN, OH, $C_{1-6}$ alkyl, $C_{3-10}$ cycloalkyl, and $C_{1-6}$ alkoxy. According to an embodiment of the present disclosure, A is selected from H, halogen, CN, OH, $C_{1-6}$ alkyl, and $C_{1-6}$ alkyloxy.

According to an embodiment of the present disclosure, D and E are identical or different, and are independently selected from H, halogen, CN, $NH_2$ unsubstituted or optionally substituted with one, two, or more substituents independently selected from $R^c$, and $-O-R^{21}$, provided that at least one of D and E is not H, e.g., at least one of D and E is selected from $-O-R^{21}$ and $NH_2$ unsubstituted or optionally substituted with one, two, or more substituents independently selected from $R^c$. According to an embodiment of the present disclosure, $R^{21}$ is selected from $C_{1-6}$ alkyl unsubstituted or optionally substituted with one, two, or more substituents $R^d$.

According to an embodiment of the present disclosure, $R^2$ and $R^3$ may, together with the N atom attached thereto, form the following groups unsubstituted or optionally substituted with one, two, or more substituents independently selected from $R^f$: 5- to 20-membered heteroaryl and 3- to 20-membered heterocyclyl, e.g., 5-, 6-, or 7-membered heteroaryl or 3-, 4-, 5-, 6-, or 7-membered heterocyclyl.

According to an embodiment of the present disclosure, each $R^{01}$, $R^{02}$, $R^{03}$, and $R^{04}$ is identical or different, and is independently selected from H, $C_{1-6}$ alkyl, and $C_{1-6}$ alkoxy.

According to an embodiment of the present disclosure, each $R^a$, $R^b$, $R^c$, $R^d$, $R^e$ and $R^f$ is identical or different, and is independently selected from halogen, $NH_2$, CN, OH, and the following groups unsubstituted or optionally substituted with one, two, or more substituents $R^g$: $C_{1-6}$ alkyl, $C_{1-6}$ alkyloxy, $C_{3-10}$ cycloalkyl, $C_{3-10}$ cycloalkyloxy, $C_{2-6}$ alkynyloxy,

3- to 8-membered heterocyclyl, and 3- to 8-membered heteroaryl.

According to an embodiment of the present disclosure, each $R^g$ is identical or different, and is independently selected from OH, halogen, and $C_{3-10}$ cycloalkyl.

According to an embodiment of the present disclosure, G is selected from halogen, $C_{3-10}$ cycloalkyl, $C_{3-10}$ cycloalky-NH-, $C_{6-14}$ aryl, 5- to 14-membered heteroaryl, 3- to 12-membered heterocyclyl, for example, 6- to 12-membered heterocyclyl having a monocyclic, bicyclic, tricyclic, or bridged ring structure comprising 1, 2, 3, or 4 heteroatoms independently selected from N, O, and S, provided that at least one heteroatom is selected from N, e.g., 1, 2, or 3 heteroatoms are selected from N.

According to an embodiment of the present disclosure, K is selected from -$C_{1-6}$ alkyl-$C_{3-10}$ cycloalkyl, -$C_{1-6}$ alkyl-$C_{6-14}$ aryl, -$C_{1-6}$ alkyl-5- to 14-membered heteroaryl, -$C_{1-6}$ alkyl-3- to 10-membered heterocyclyl, -C(O)$NH_2$, -C(O)-$C_{3-10}$ cycloalkyl, -C(O)-$C_{6-14}$ aryl, -C(O)-5- to 14-membered heteroaryl, -C(O)-3- to 10-membered heterocyclyl, -C(O)-$C_{1-6}$ alkyl-$C_{3-10}$ cycloalkyl, -C(O)-$C_{1-6}$ alkyl-$C_{6-14}$ aryl, -C(O)-$C_{1-6}$ alkyl-5- to 14-membered heteroaryl, and -C(O)-$C_{1-6}$ alkyl-3-to 10-membered heterocyclyl, wherein a group on a ring or acyclic group of the $C_{3-10}$ cycloalkyl, $C_{6-14}$ aryl, 5- to 14-membered heteroaryl, 3- to 10-membered heterocyclyl, -C(O)-$C_{3-10}$ cycloalkyl, -C(O)-$C_{6-14}$ aryl, -C(O)-5- to 14-membered heteroaryl, -C(O)-3- to 10-membered heterocyclyl, -C(O)-$C_{1-6}$ alkyl-$C_{3-10}$ cycloalkyl, -C(O)-$C_{1-6}$ alkyl-$C_{6-14}$ aryl, -C(O)-$C_{1-6}$ alkyl-5- to 14-membered heteroaryl, or -C(O)-$C_{1-6}$ alkyl-3- to 10-membered heterocyclyl, or -C(O)$NH_2$ is further optionally substituted with one, two, or more groups selected from OH, halogen, CN, $C_{1-6}$ alkyl, and $C_{1-6}$ alkyloxy; wherein the heterocyclyl can be pyridinyl (e.g., pyridin-2-yl, pyridin-3-yl, pyridin-4-yl, pyridin-5-yl, and pyridin-6-yl), and the aryl can be phenyl;

or, K is absent or selected from H, OH, and the following groups unsubstituted or optionally substituted with one, two, or more substituents independently selected from $R^f$: phenyl-C(O)-, phenyl-C(O)-NH-, phenyl-C(O)-NH-$C_{1-6}$ alkyl-, phenyloxy-C(O)-NH-, phenylalkyl-NH-C(O)-, phenylalkyl-C(O)-NH-, pyridinyl-C(O)-, pyridinyl-C(O)-NH-, pyridinyl-C(O)-NH-$C_{1-6}$ alkyl-, pyridinyloxy-C(O)-NH-, pyridinylalkyl-NH-C(O)-, pyridinylalkyl-C(O)-NH-, pyrrolidinyl-C(O)-NH-, pyrrolidinyloxy-C(O)-NH-, $C_{1-6}$ alkyl-C(O)-, $C_{1-6}$ alkyl-C(O)-NH-, $C_{1-6}$ alkoxy-C(O)-NH-, $C_{3-8}$ cycloalkyl-C(O)-NH-, $C_{3-8}$ cycloalkoxy-C(O)-NH-, pyridinyl-NH-C(O)-, $C_{1-6}$ alkyl-NH-C(O)-, $C_{3-8}$ cycloalkyl-NH-C(O)-, pyridinyloxy-, pyridinylalkoxy-, pyridinyloxyalkyl-, phenyloxy-, phenylalkoxy-, phenyloxyalkyl-, $C_{1-6}$ alkyl-$S(O)_2$-, $C_{1-6}$ alkyl-$S(O)_2$-NH-, $C_{1-6}$ alkyl-NH-$S(O)_2$-, pyridinyl $C_{1-6}$ alkyl-, pyridinyl-$S(O)_2$-, pyridinyl-$C_{1-6}$ alkyl-$S(O)_2$-, pyridinyl-$S(O)_2$-NH-, pyridinyl-NH-$S(O)_2$-, pyridinyl-$C_{1-6}$ alkyl-NH-, phenyl $C_{1-6}$ alkyl-, phenyl-$S(O)_2$-, phenyl-$C_{1-6}$ alkyl-$S(O)_2$-, phenyl-$S(O)_2$-NH-, phenyl-NH-$S(O)_2$-, phenyl-$C_{1-6}$ alkyl-NH-, $C_{1-6}$ alkoxy-C(O)-,

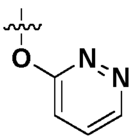, and 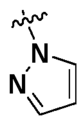.

According to an exemplary embodiment of the present disclosure, $X^1$, $X^2$, $X^3$, $X^4$, $X^5$, $X^6$, and $X^7$ are identical or different, and are independently selected from CH and N; for example, at least one, e.g., 1, 2, 3, 4, 5, 6, or 7, of $X^1$, $X^2$, $X^3$, $X^4$, $X^5$, $X^6$, and $X^7$ is N.

According to an exemplary embodiment of the present disclosure, $X^8$ is selected from $NR^1$.

According to an exemplary embodiment of the present disclosure, $R^{01}$ is methoxy.

According to an exemplary embodiment of the present disclosure, $R^{02}$ is H.

According to an exemplary embodiment of the present disclosure, $R^{03}$ is methyl, and $R^{04}$ is H.

According to an exemplary embodiment of the present disclosure, $R^1$ is H.

According to an exemplary embodiment of the present disclosure, A is selected from H, $NH_2$, methyl, ethyl, propyl, and isopropyl.

According to an exemplary embodiment of the present disclosure, E is selected from H and $NH_2$.

According to an exemplary embodiment of the present disclosure, D is selected from the following groups: halogen, BnO-, H, CN, $NH_2$, $OCH_3$, COOH, $B(OH)_2$,

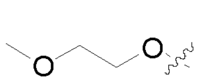 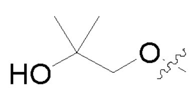 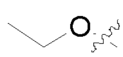

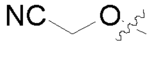 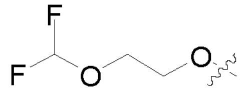 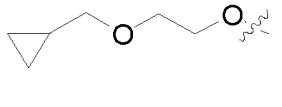

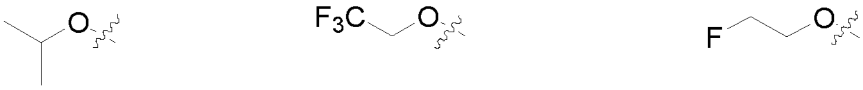

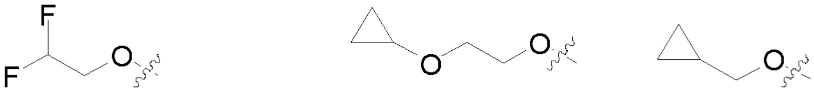

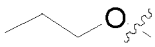 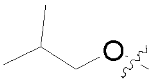 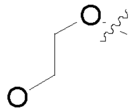

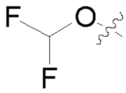 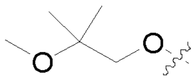

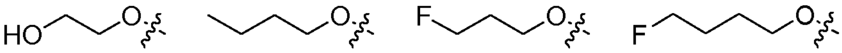,

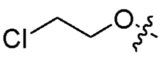 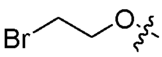 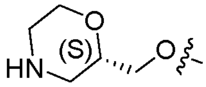, 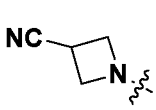, 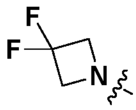, 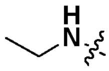, 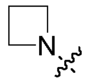,

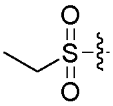, 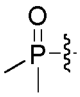, 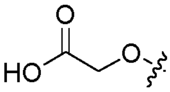, 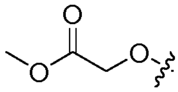, 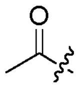, 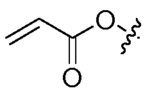, 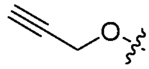,

and

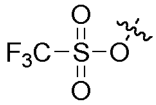.

According to an exemplary embodiment of the present disclosure, G is selected from F and the following groups unsubstituted or optionally substituted with one, two, or more substituents independently selected from $R^e$:

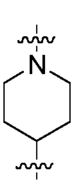, 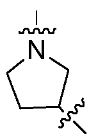, 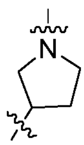, 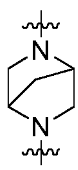, 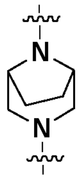, 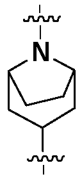, 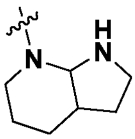,

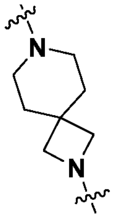, 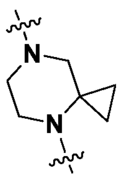, 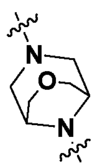, 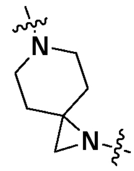, 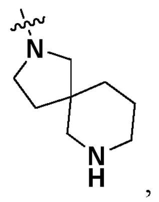, 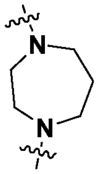, 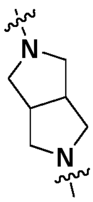, 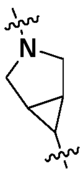, 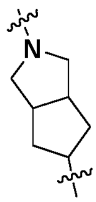,

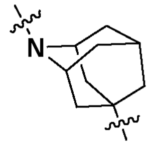, 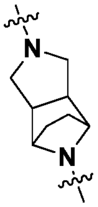, 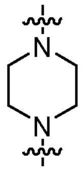, 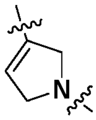, 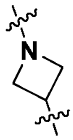, 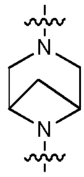, 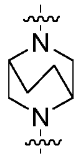, 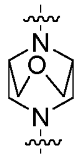, 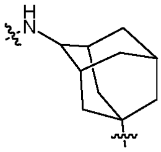, 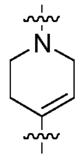,

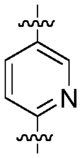, 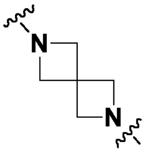, 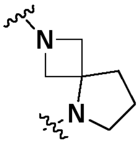, 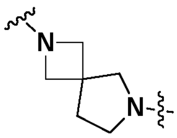, 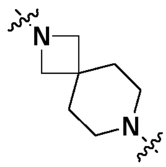, 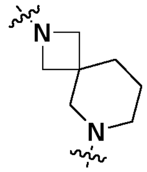, 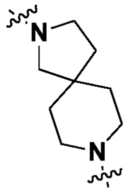,

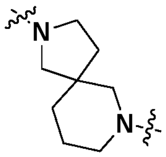, 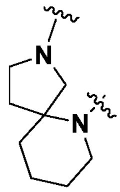, 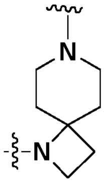, 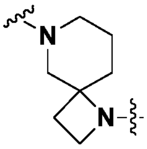, 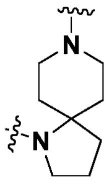, 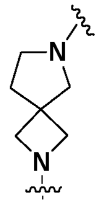, 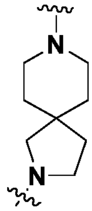, 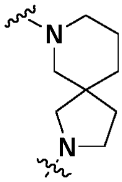,

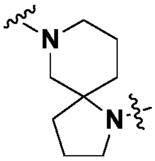, 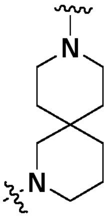, 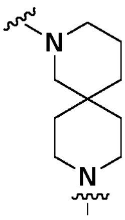, 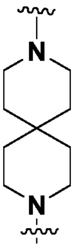, 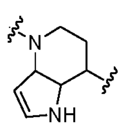, and 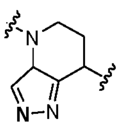.

According to an exemplary embodiment of the present disclosure, when group G is substituted with $R^e$, $R^e$ may substitute H on $-CH_2-$ or -CH= constituting group G, forming, for example, a group selected from:

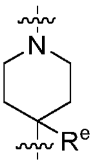, 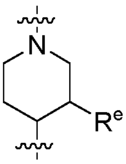, 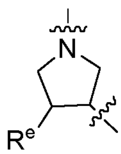, 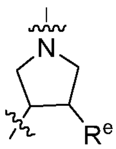, and 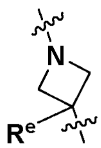.

According to an embodiment of the present disclosure, unless otherwise specified, chemical bonds marked with wavy lines represent connection sites to other groups. For example, when two wavy lines are present in group G, either of them may be connected to the ring in which $X^1$, $X^2$, $X^3$, and $X^4$ are located, while the other is connected to group K when group K is present.

According to an exemplary embodiment of the present disclosure, when the ring-forming atoms of group G include a carbon atom and a nitrogen atom, group G may be connected to the ring in which $X^1$, $X^2$, $X^3$, and $X^4$ are located through one of the carbon atom and the nitrogen atom (e.g., one of the ring-forming atoms marked with wavy lines in the exemplary groups described above) and to group K through the other one of the carbon atom and the nitrogen atom.

According to an exemplary embodiment of the present disclosure, K is absent or selected from H, OH, and the following groups unsubstituted or optionally substituted with one, two, or more substituents independently selected from $R^f$: phenyl-C(O)-, phenyl-C(O)-NH-, phenyl-C(O)-NH-$C_{1-6}$ alkyl-, phenyloxy-C(O)-NH-, phenylalkyl-NH-C(O)-, phenylalkyl-C(O)-NH-, pyridinyl-C(O)-, pyridinyl-C(O)-NH-, pyridinyl-C(O)-NH-$C_{1-6}$ alkyl-, pyridinyloxy-C(O)-NH-, pyridinylalkyl-NH-C(O)-, pyridinylalkyl-C(O)-NH-, pyrrolidinyl-C(O)-NH-, pyrrolidinyloxy-C(O)-NH-, $C_{1-6}$ alkyl-C(O)-, $C_{2-6}$ alkenyl-C(O)-,

$C_{1-6}$ alkyl-C(O)-NH-, $C_{1-6}$ alkoxy-C(O)-NH-, $C_{3-8}$ cycloalky-C(O)-NH-, $C_{3-8}$ cycloalkoxy-C(O)-NH-, pyridinyl-NH-C(O)-, $C_{1-6}$ alkyl-NH-C(O)-, $C_{3-8}$ cycloalky-NH-C(O)-, pyridinyloxy-, pyridinylalkoxy-, pyridinyloxyalkyl-, phenyloxy-, phenylalkoxy-, phenyloxyalkyl-, $C_{1-6}$ alkyl-$S(O)_2$-, $C_{1-6}$ alkyl-$S(O)_2$-NH-, $C_{1-6}$ alkyl-NH-$S(O)_2$-, pyridinyl $C_{1-6}$ alkyl-, pyridinyl-$S(O)_2$-, pyridinyl-$C_{1-6}$ alkyl-$S(O)_2$-, pyridinyl-$S(O)_2$-NH-, pyridinyl-NH-$S(O)_2$-, pyridinyl-$C_{1-6}$ alkyl-NH-, phenyl $C_{1-6}$ alkyl-, phenyl-$S(O)_2$-, phenyl-$C_{1-6}$ alkyl-$S(O)_2$-, phenyl-$S(O)_2$-NH-, phenyl-NH-$S(O)_2$-, phenyl-$C_{1-6}$ alkyl-NH-, $C_{1-6}$ alkoxy-C(O)-,

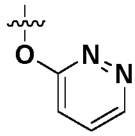, 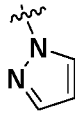, 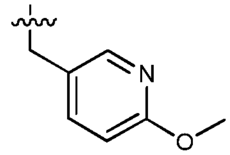, 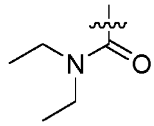,

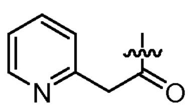, 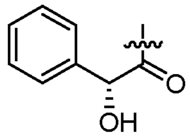, 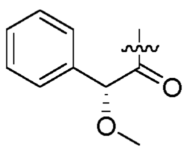, and 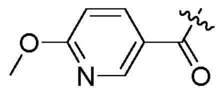.

According to an embodiment of the present disclosure, the compound represented by formula I has a structure represented by the following formula II or III:

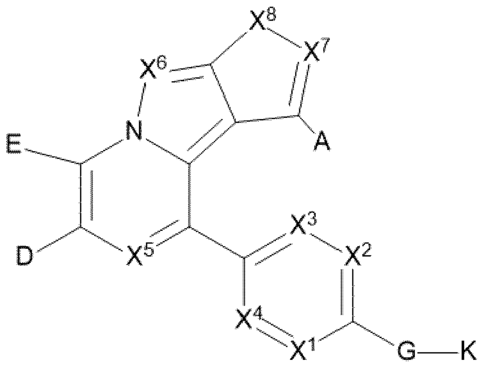

II

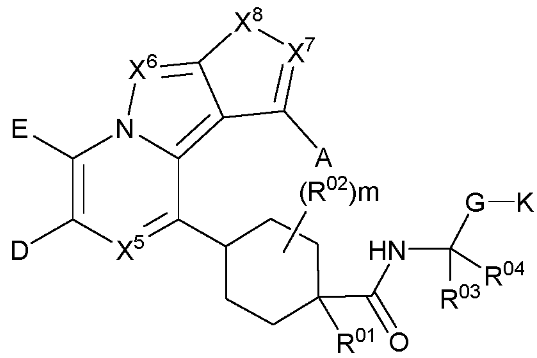

III

;

wherein $X^1$, $X^2$, $X^3$, $X^4$, $X^5$, $X^6$, $X^7$, $X^8$, $R^{01}$, $R^{02}$, $R^{03}$, $R^{04}$, A, D, E, G, K, and m are as defined above.

According to an embodiment of the present disclosure, the compound represented by formula I has a structure represented by the following formula IV or V:

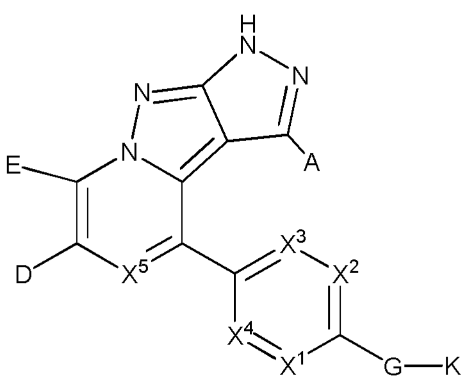

IV

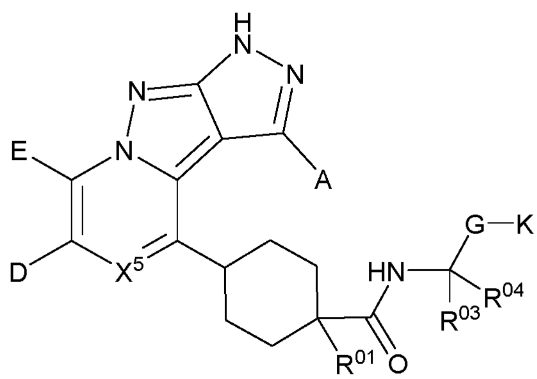

V

;

wherein $X^1$, $X^2$, $X^3$, $X^4$, $X^5$, $R^{01}$, $R^{03}$, $R^{04}$, A, D, E, G, and K are as defined above.

According to an embodiment of the present disclosure, the compound is selected from the following compounds:

| No. | Chemical structure | No. | Chemical structure |
|---|---|---|---|
| 1 | 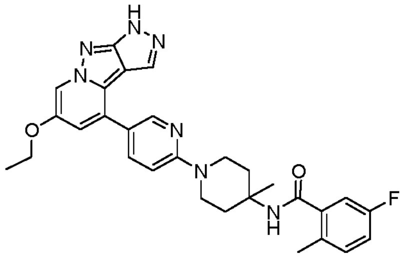 | 134 | 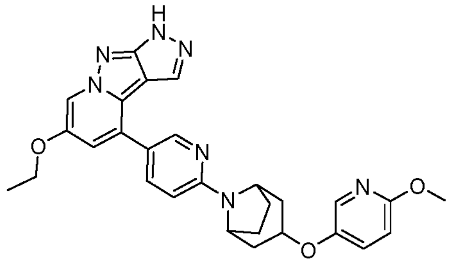 |
| 2 | 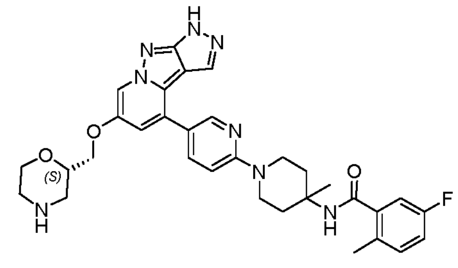 | 135 | 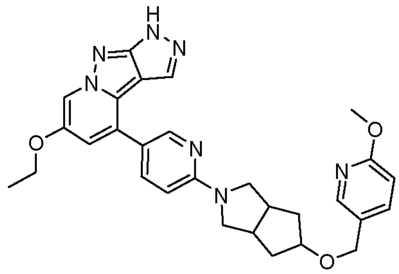 |
| 3 | 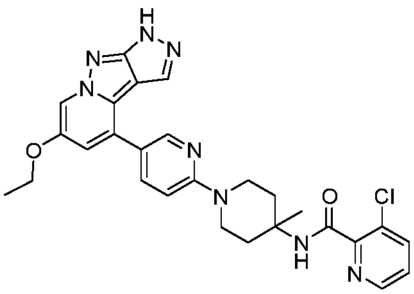 | 136 | 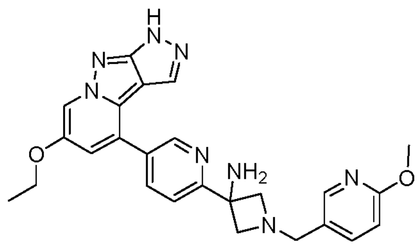 |
| 4 | 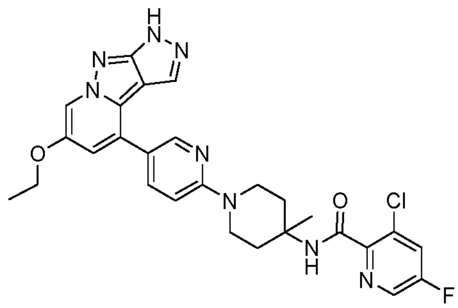 | 137 | 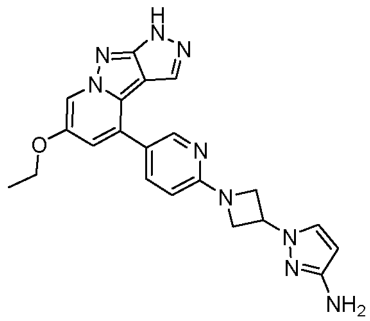 |
| 5 | 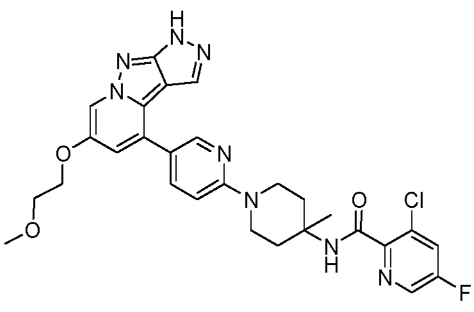 | 138 | 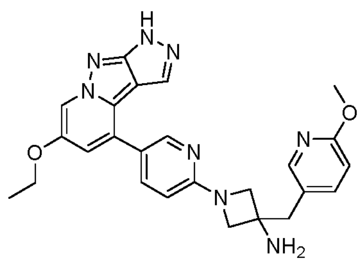 |

(continued)

| No. | Chemical structure | No. | Chemical structure |
|---|---|---|---|
| 6 | 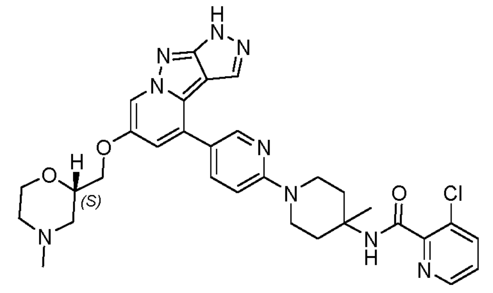 | 139 | 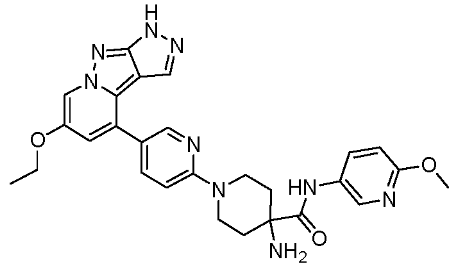 |
| 7 | 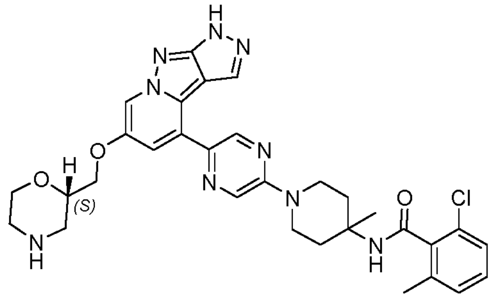 | 140 | 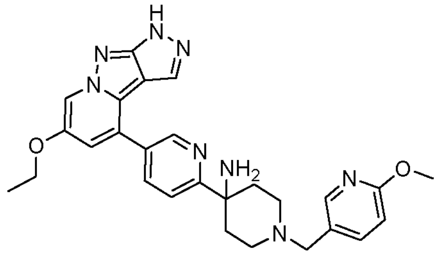 |
| 8 | 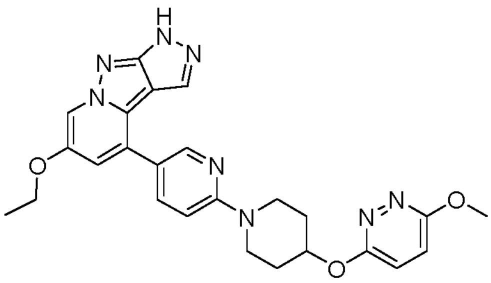 | 141 | 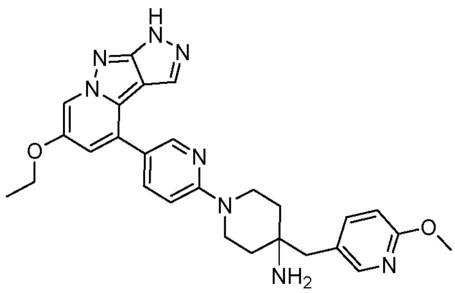 |
| 9 | 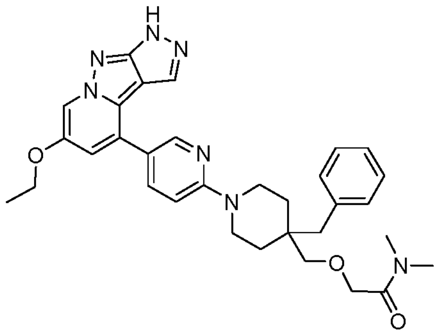 | 142 | 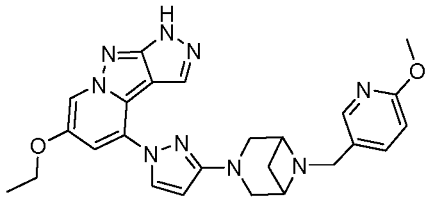 |
| 10 | 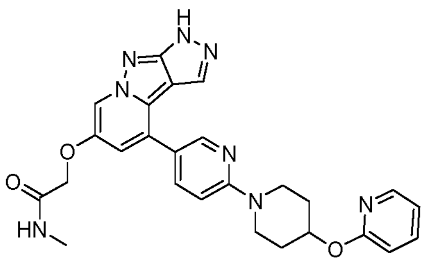 | 143 | 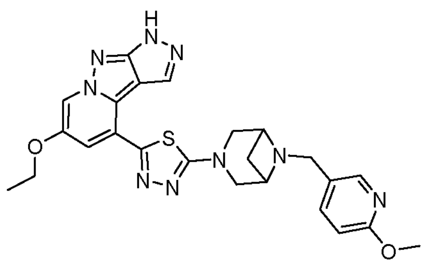 |

(continued)

| No. | Chemical structure | No. | Chemical structure |
|---|---|---|---|
| 11 | 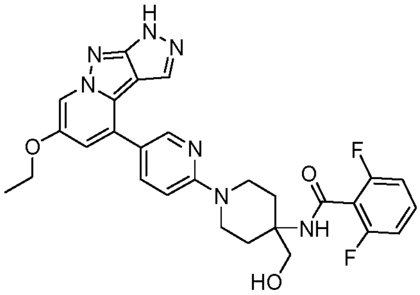 | 144 | 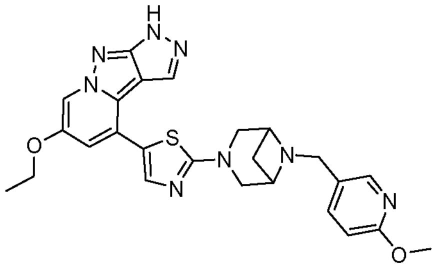 |
| 12 | 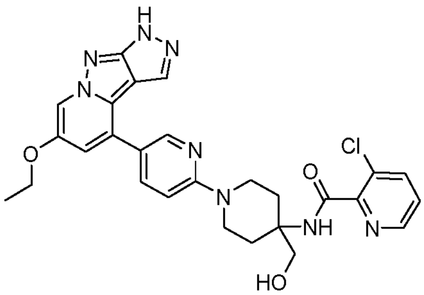 | 145 | 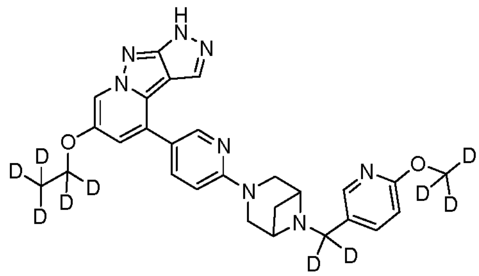 |
| 13 | 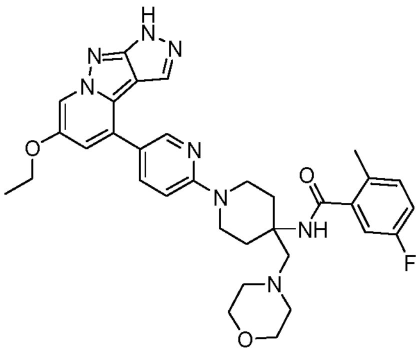 | 146 | 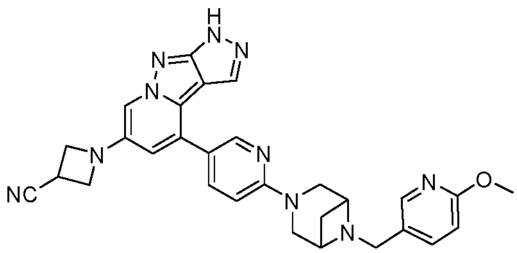 |
| 14 | 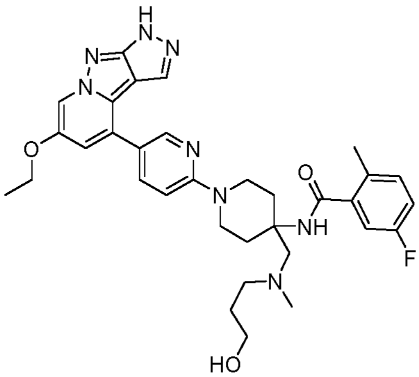 | 147 | 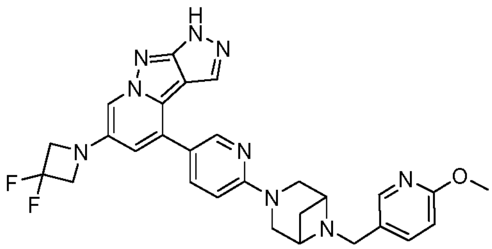 |

(continued)

| No. | Chemical structure | No. | Chemical structure |
|---|---|---|---|
| 15 | 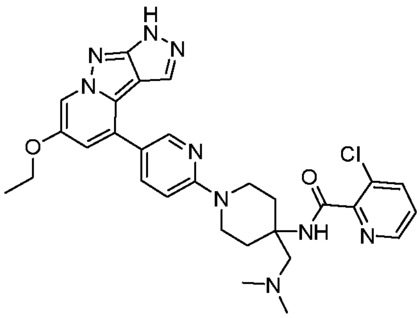 | 148 | 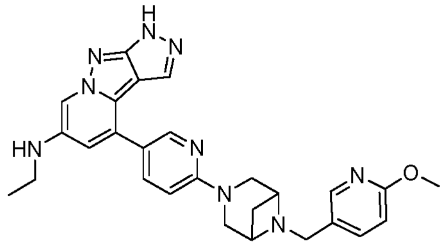 |
| 16 | 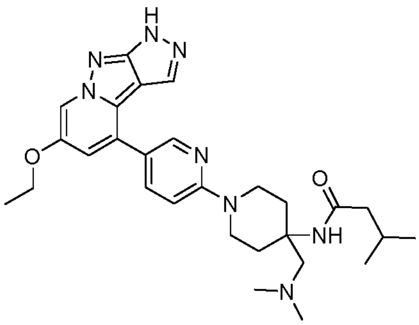 | 149 | 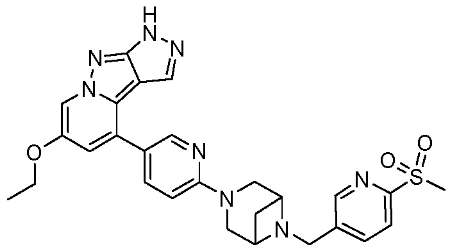 |
| 17 | 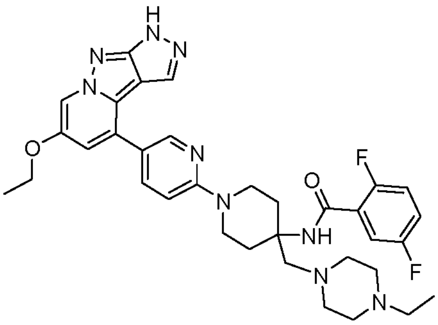 | 150 | 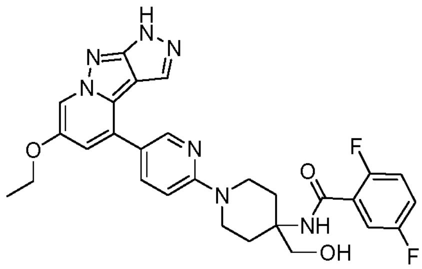 |
| 18 | 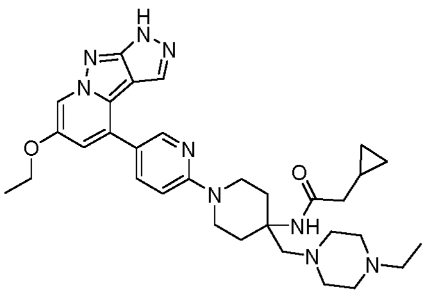 | 151 | 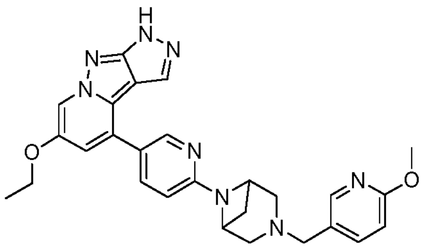 |
| 19 | 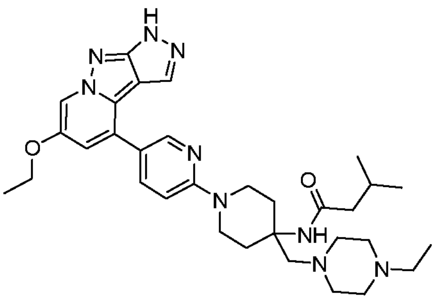 | 152 | 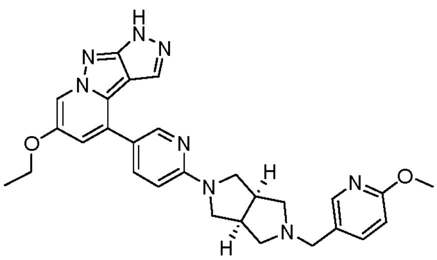 |

(continued)

| No. | Chemical structure | No. | Chemical structure |
|---|---|---|---|
| 20 | 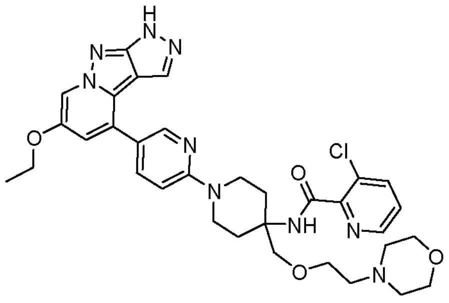 | 153 | 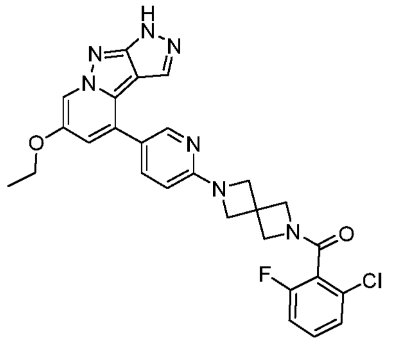 |
| 21 | 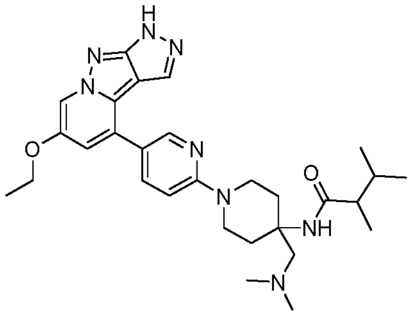 | 154 | 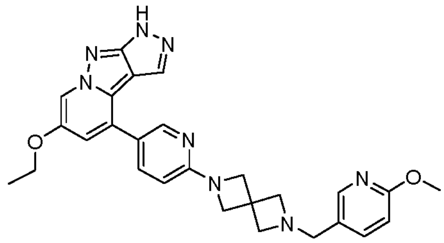 |
| 22 | 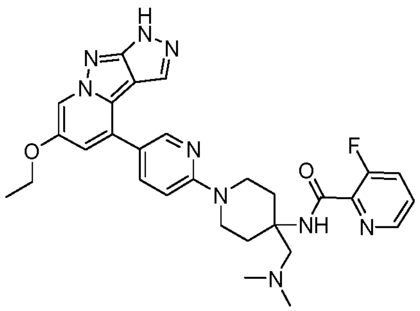 | 155 | 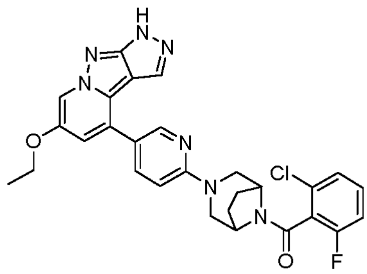 |
| 23 | 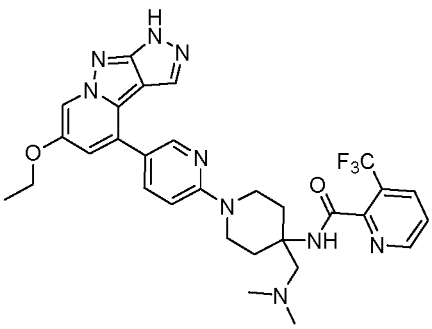 | 156 | 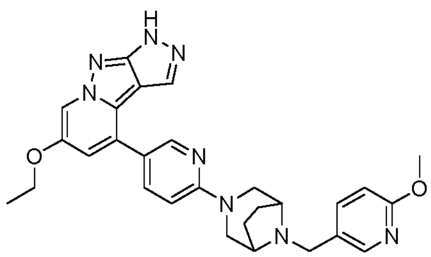 |
| 24 | 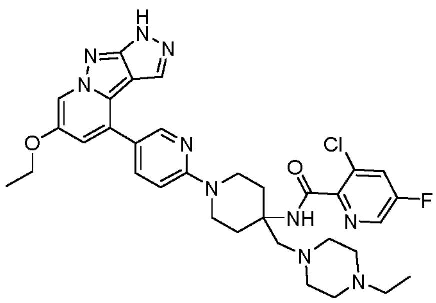 | 157 | 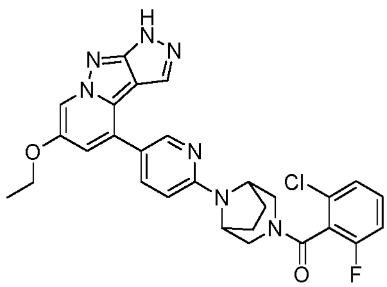 |

(continued)

| No. | Chemical structure | No. | Chemical structure |
|---|---|---|---|
| 25 | 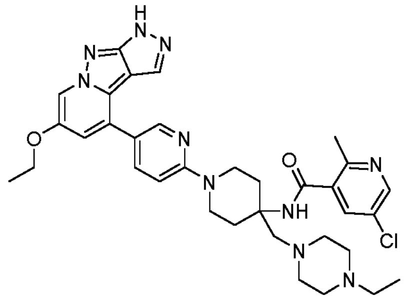 | 158 | 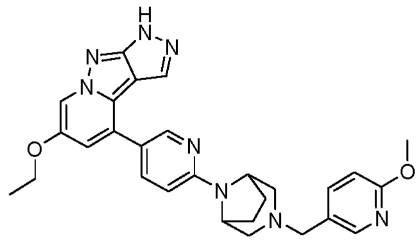 |
| 26 | 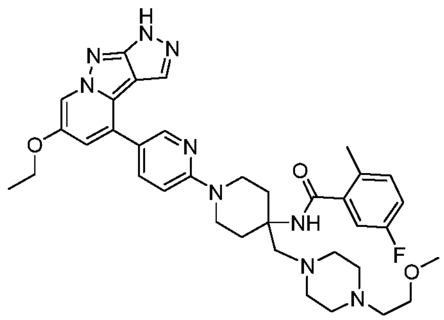 | 159 | 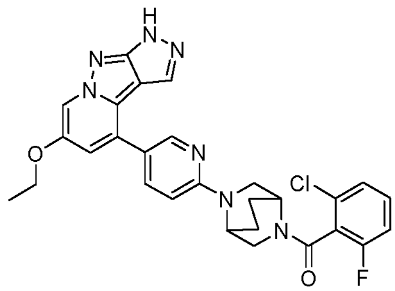 |
| 27 | 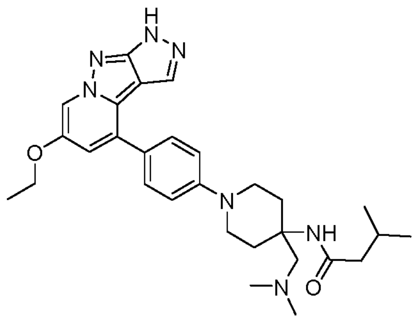 | 160 | 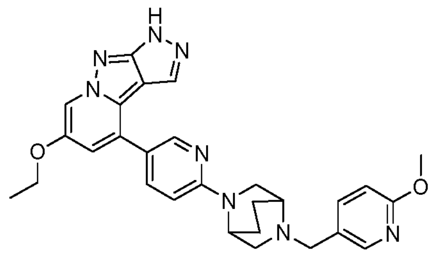 |
| 28 | 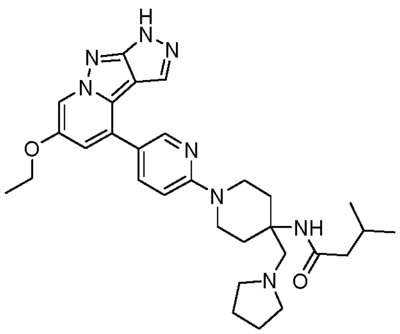 | 161 | 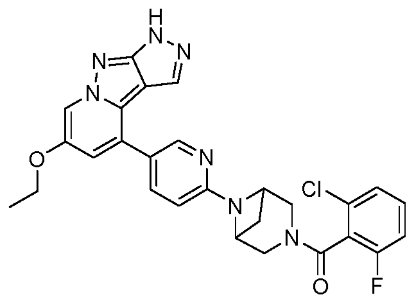 |
| 29 | 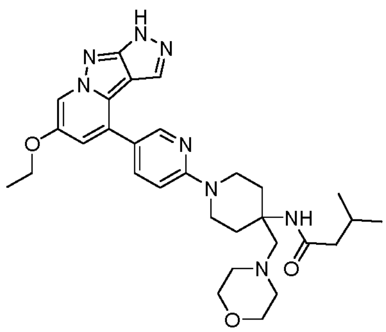 | 162 | 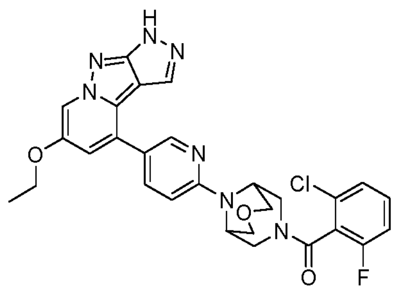 |

(continued)

| No. | Chemical structure | No. | Chemical structure |
|---|---|---|---|
| 30 | 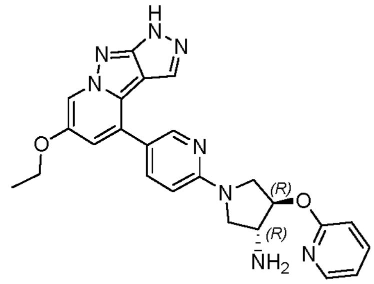 | 163 | 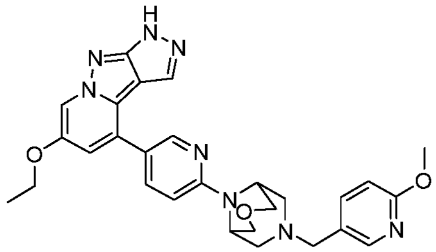 |
| 31 | 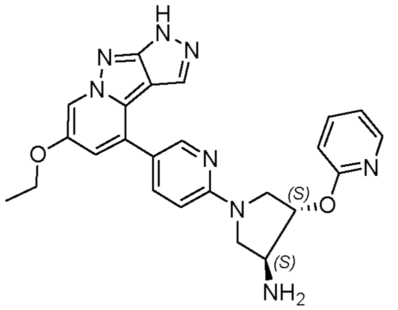 | 164 | 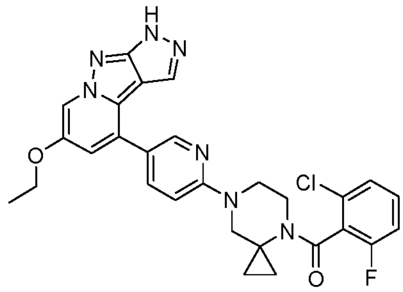 |
| 32 | 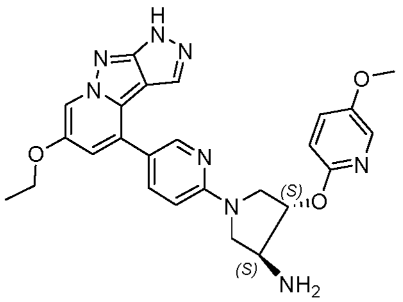 | 165 | 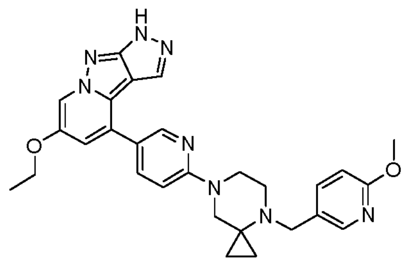 |
| 33 | 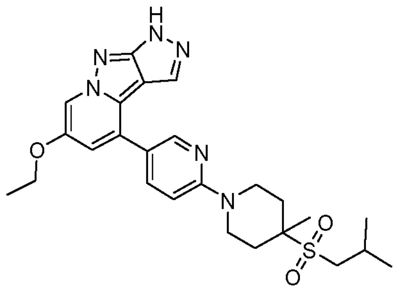 | 166 | 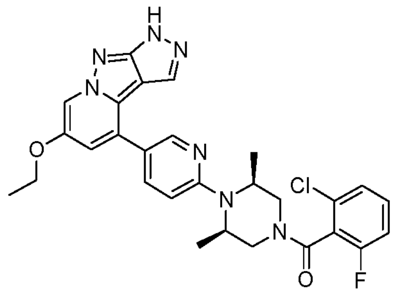 |
| 34 | 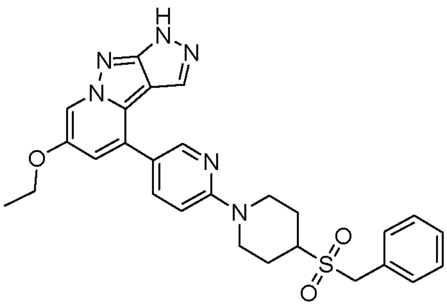 | 167 | 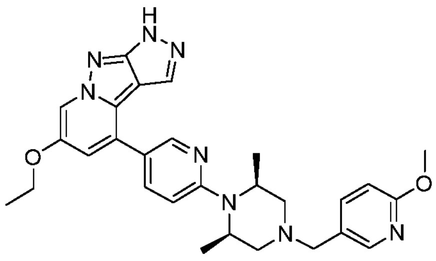 |

(continued)

| No. | Chemical structure | No. | Chemical structure |
|---|---|---|---|
| 35 | 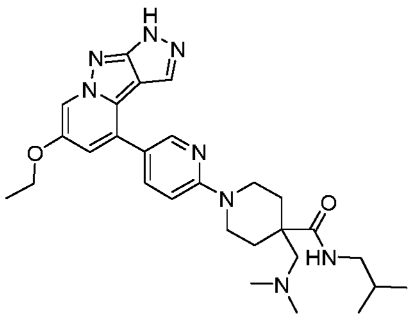 | 168 | 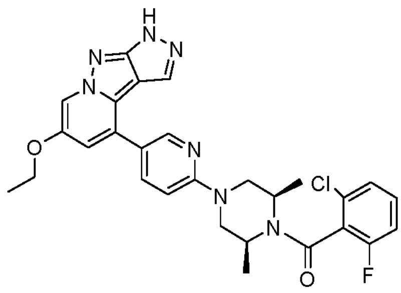 |
| 36 | 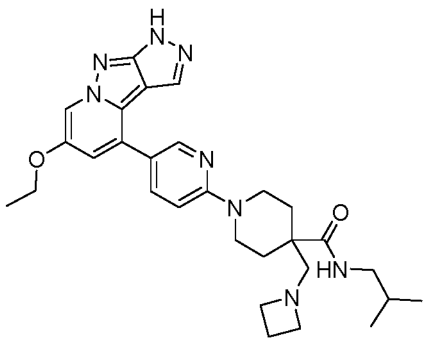 | 169 | 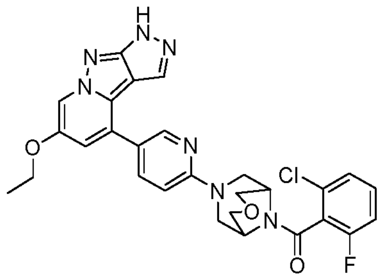 |
| 37 | 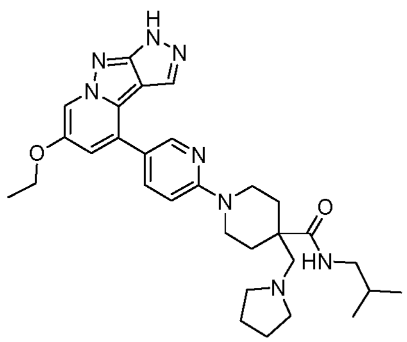 | 170 | 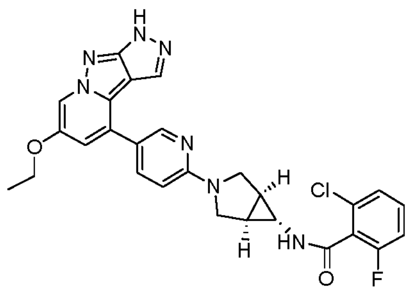 |
| 38 | 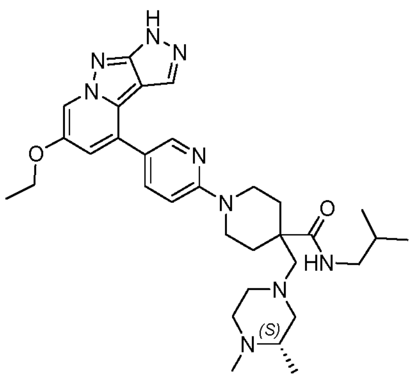 | 171 | 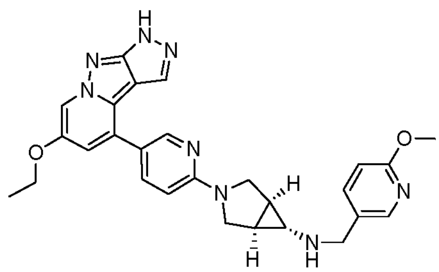 |

(continued)

| No. | Chemical structure | No. | Chemical structure |
|---|---|---|---|
| 39 | 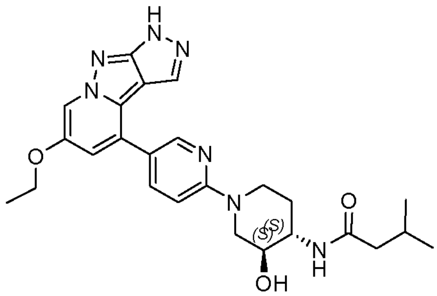 | 172 | 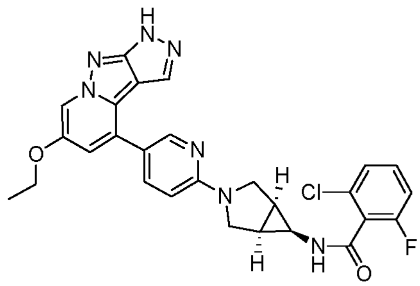 |
| 40 | 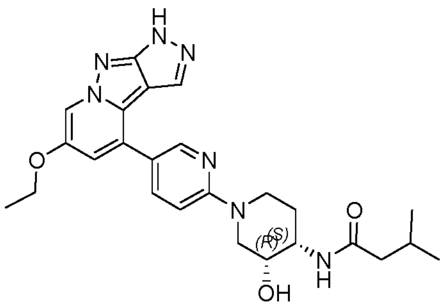 | 173 | 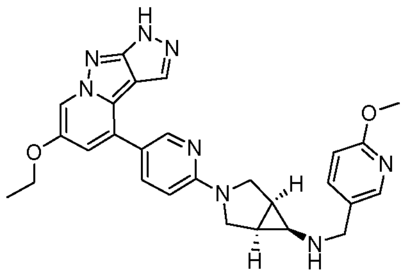 |
| 41 | 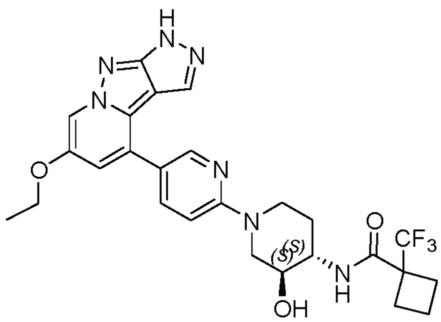 | 174 | 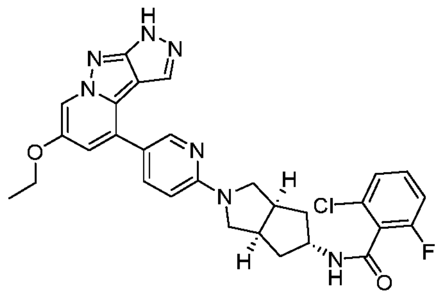 |
| 42 | 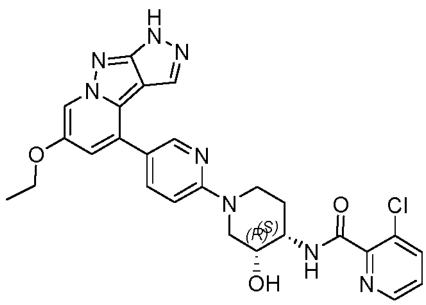 | 175 | 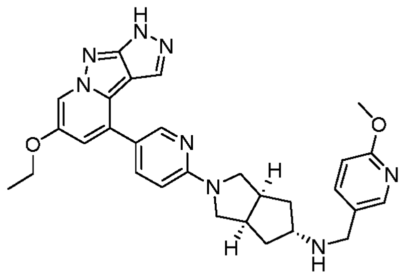 |
| 43 | 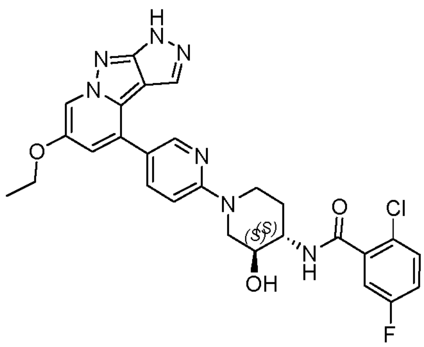 | 176 | 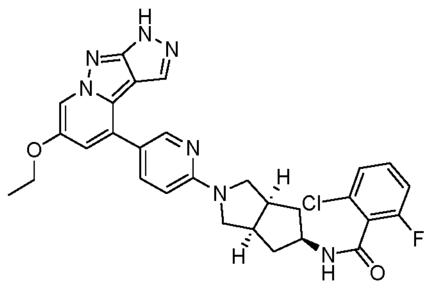 |

(continued)

| No. | Chemical structure | No. | Chemical structure |
|---|---|---|---|
| 44 | 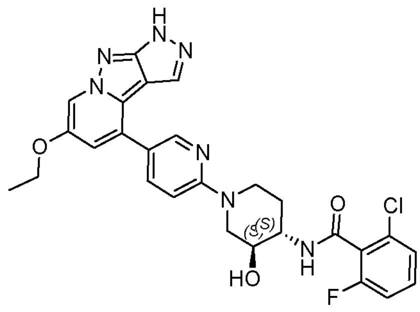 | 177 | 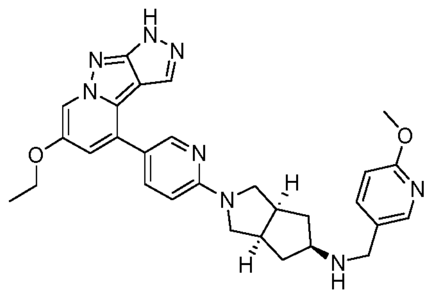 |
| 45 | 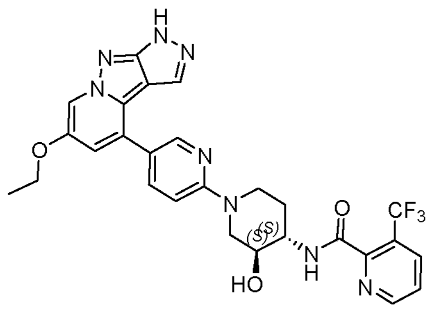 | 178 | 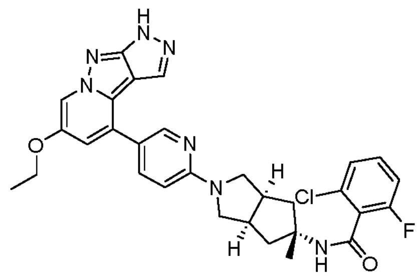 |
| 46 | 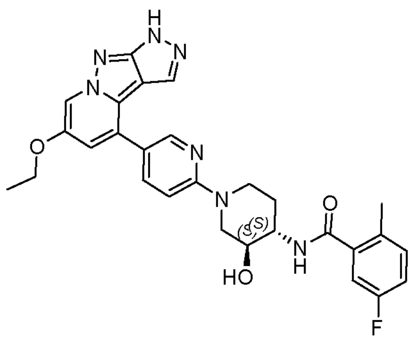 | 179 | 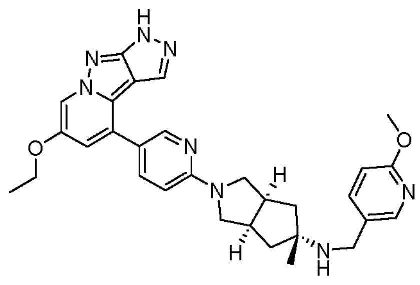 |
| 47 | 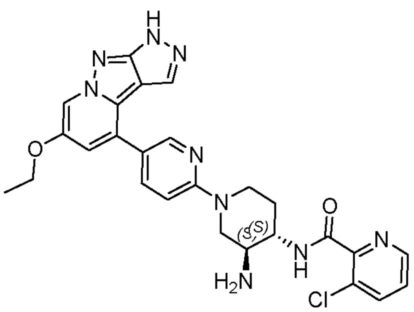 | 180 | 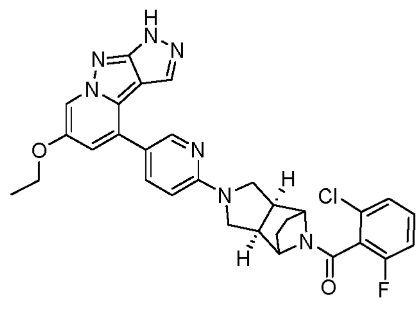 |
| 48 | 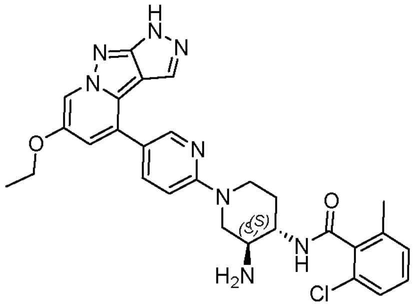 | 181 | 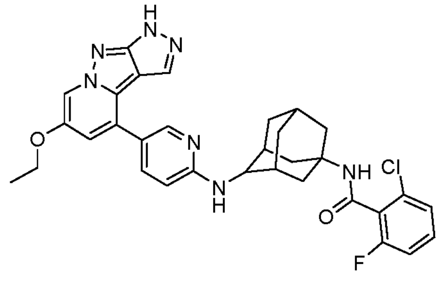 |

(continued)

| No. | Chemical structure | No. | Chemical structure |
|---|---|---|---|
| 49 | 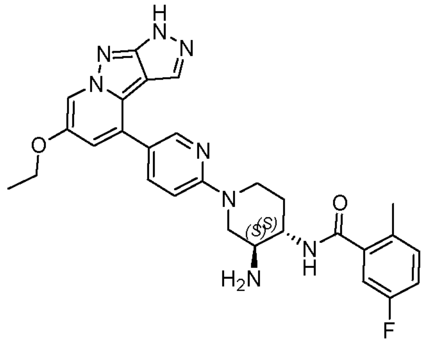 | 182 | 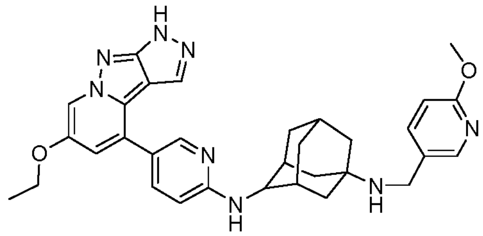 |
| 50 | 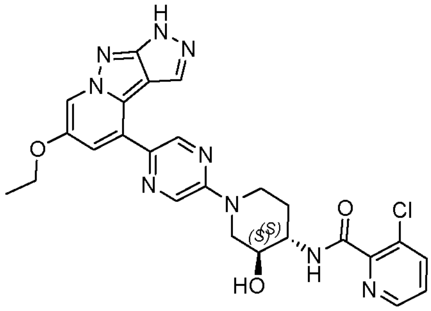 | 183 | 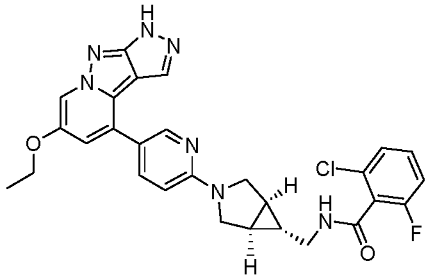 |
| 51 | 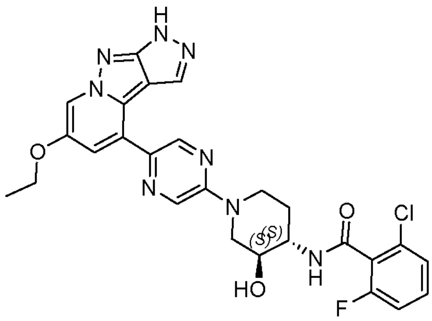 | 184 | 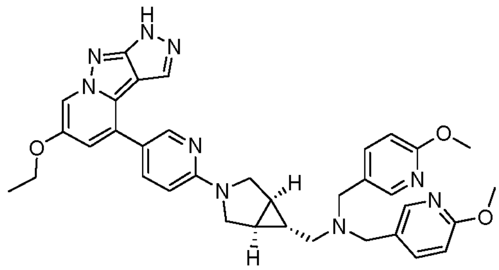 |
| 52 | 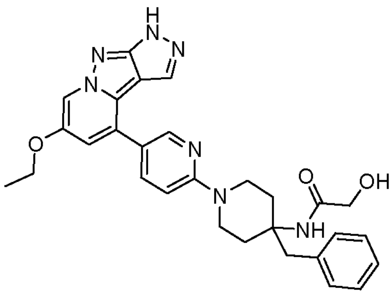 | 185 | 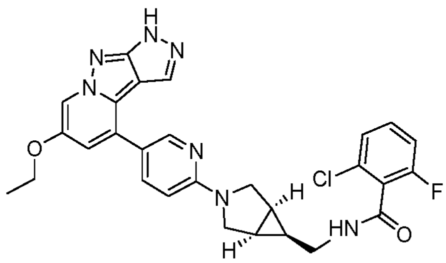 |
| 53 | 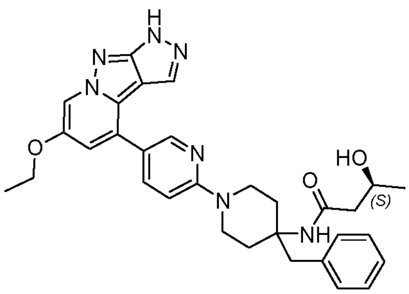 | 186 | 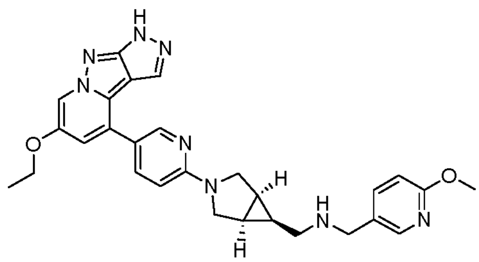 |

(continued)

| No. | Chemical structure | No. | Chemical structure |
|---|---|---|---|
| 54 | 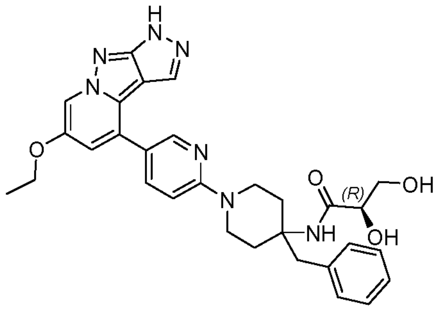 | 187 | 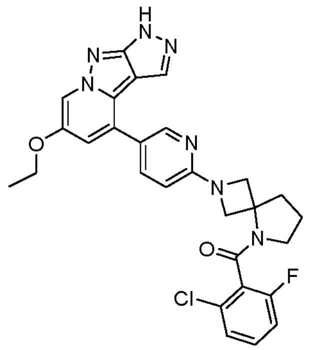 |
| 55 | 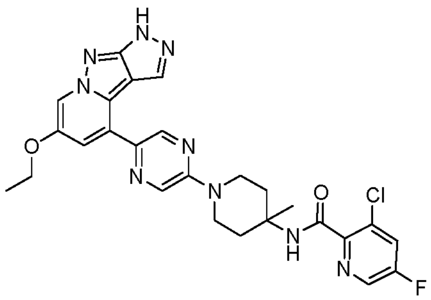 | 188 | 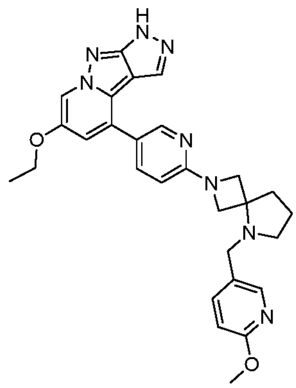 |
| 56 | 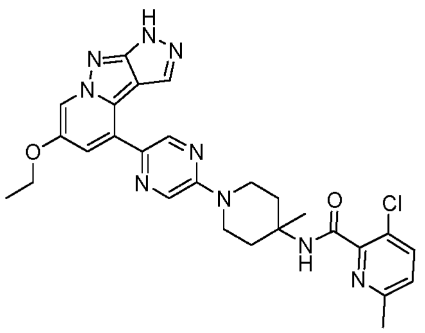 | 189 | 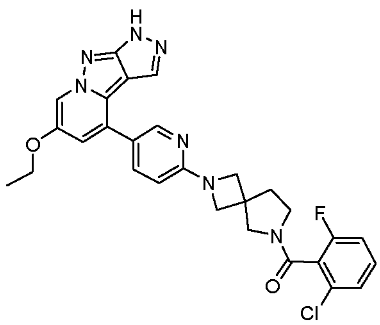 |
| 57 | 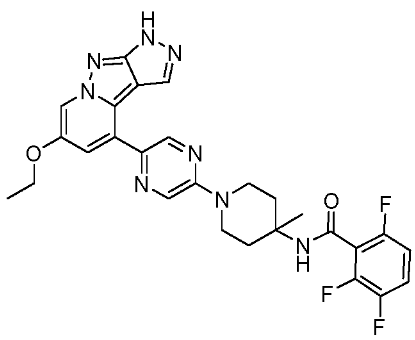 | 190 | 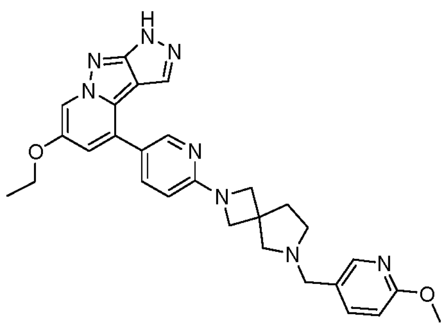 |

(continued)

| No. | Chemical structure | No. | Chemical structure |
|---|---|---|---|
| 58 | 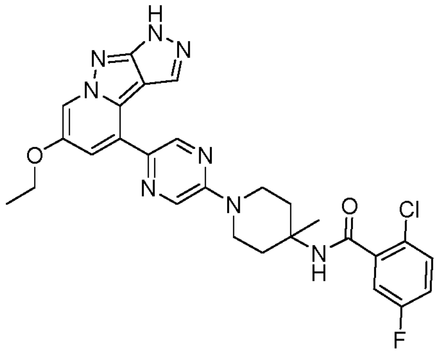 | 191 | 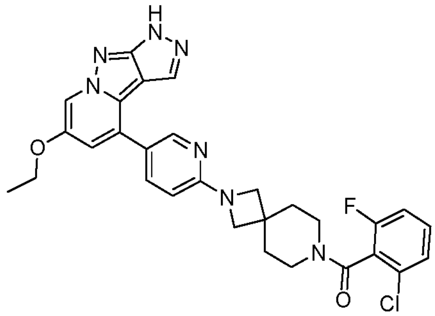 |
| 59 | 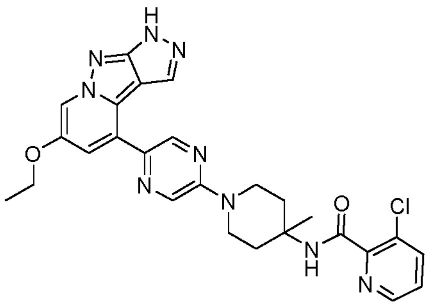 | 192 | 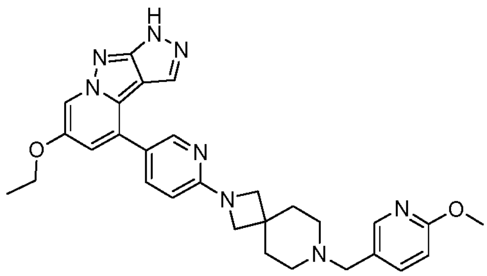 |
| 60 | 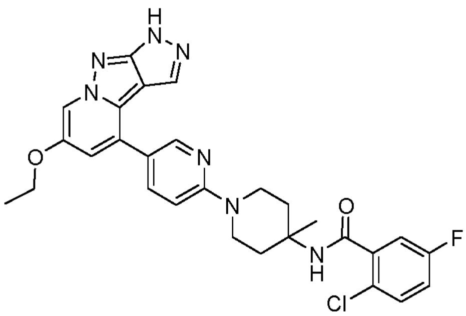 | 193 | 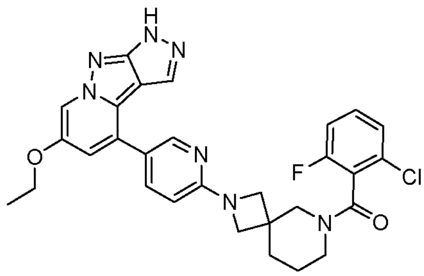 |
| 61 | 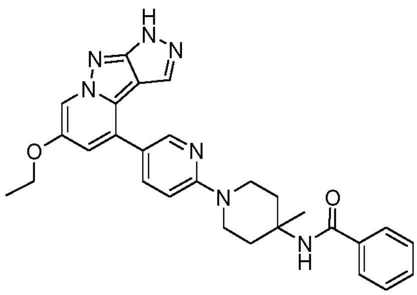 | 194 | 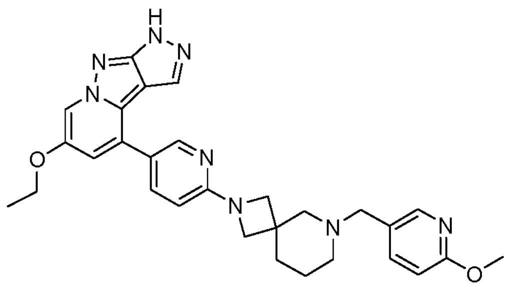 |

(continued)

| No. | Chemical structure | No. | Chemical structure |
|---|---|---|---|
| 62 | 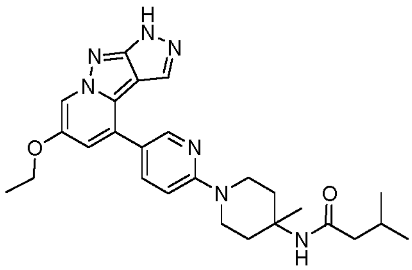 | 195 | 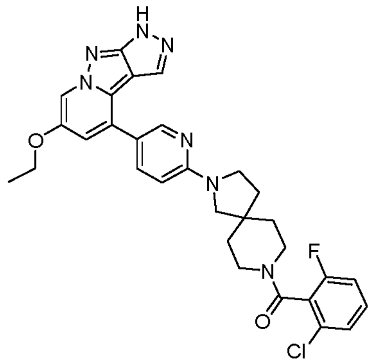 |
| 63 | 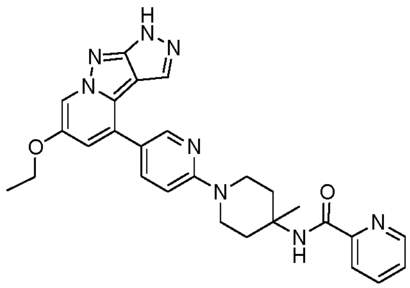 | 196 | 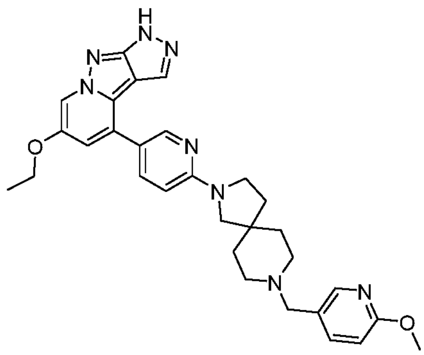 |
| 64 | 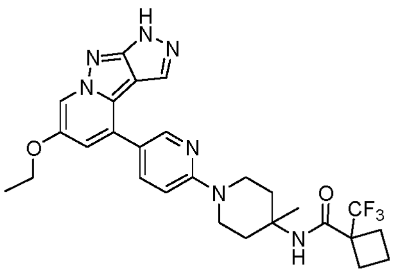 | 197 | 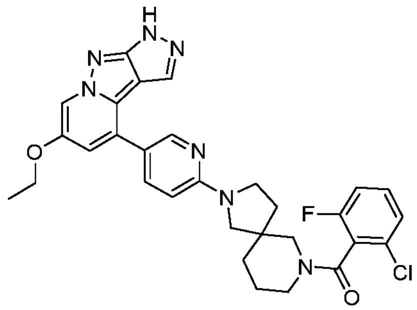 |
| 65 | 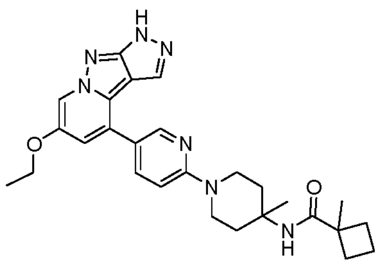 | 198 | 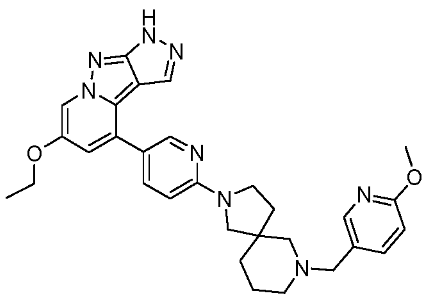 |

(continued)

| No. | Chemical structure | No. | Chemical structure |
|---|---|---|---|
| 66 | 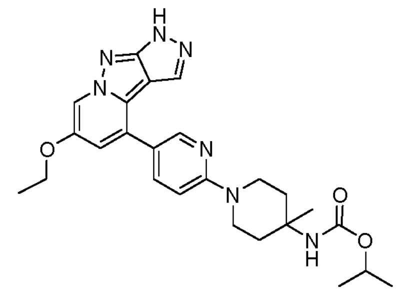 | 199 | 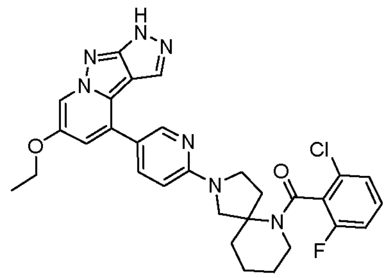 |
| 67 | 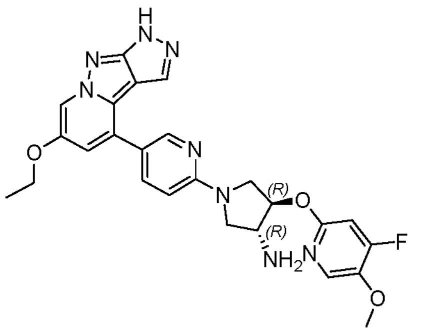 | 200 | 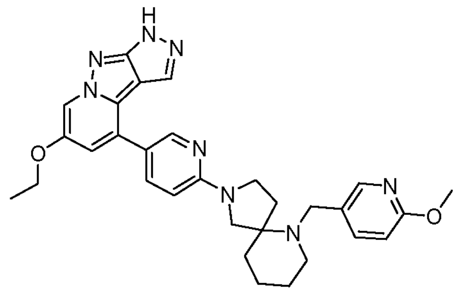 |
| 68 | 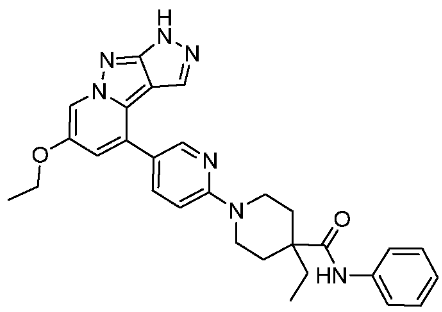 | 201 | 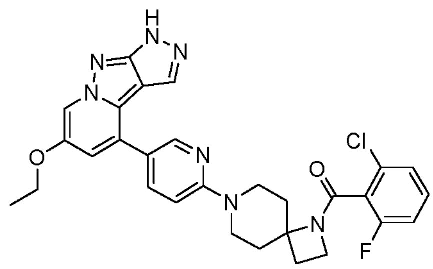 |
| 69 | 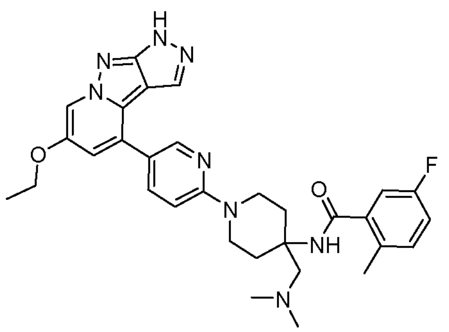 | 202 | 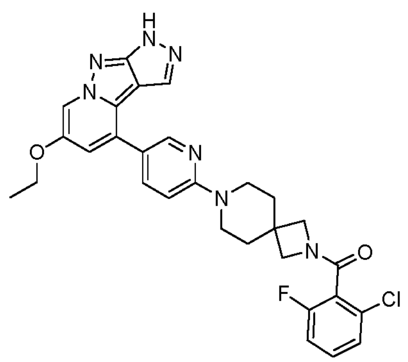 |

(continued)

| No. | Chemical structure | No. | Chemical structure |
|---|---|---|---|
| 70 | 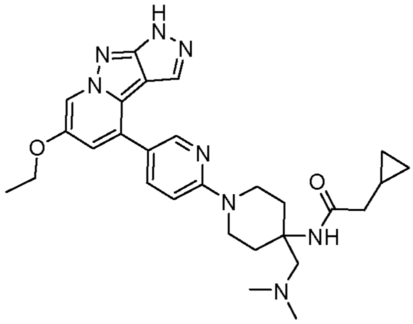 | 203 | 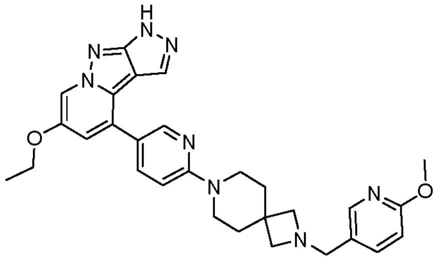 |
| 71 | 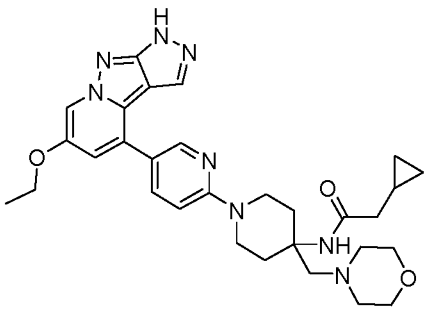 | 204 | 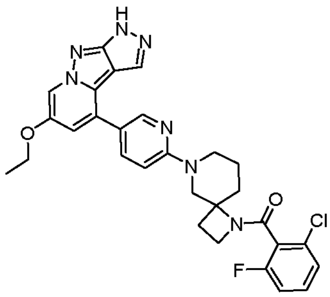 |
| 72 | 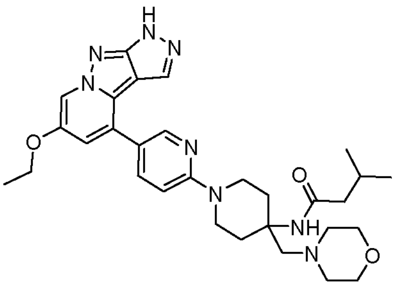 | 205 | 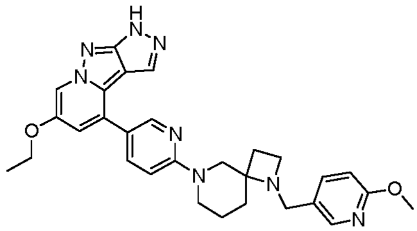 |
| 73 | 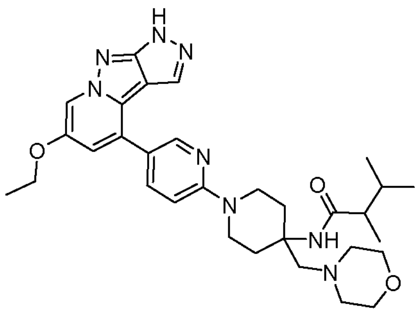 | 206 | 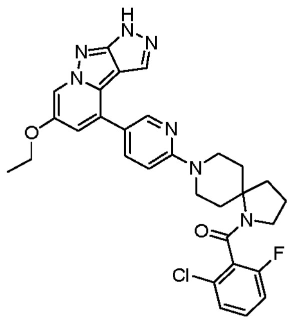 |
| 74 | 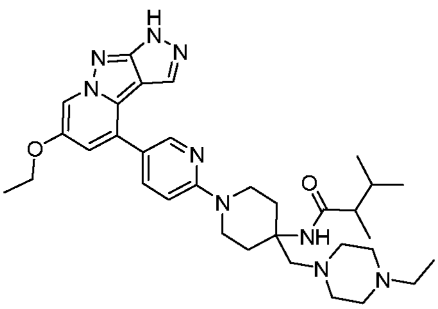 | 207 | 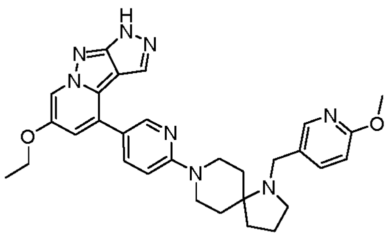 |

(continued)

| No. | Chemical structure | No. | Chemical structure |
|---|---|---|---|
| 75 | 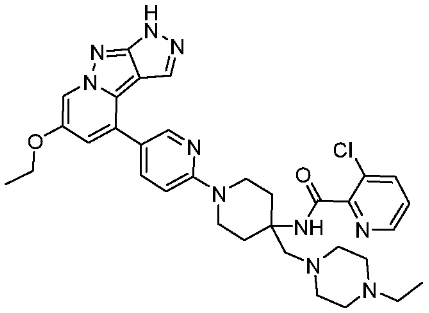 | 208 | 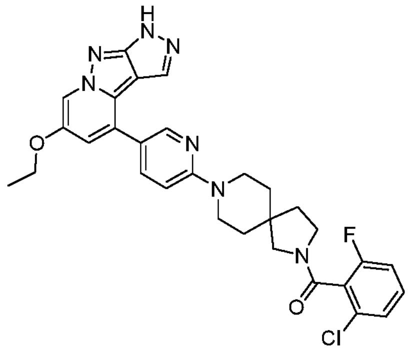 |
| 76 | 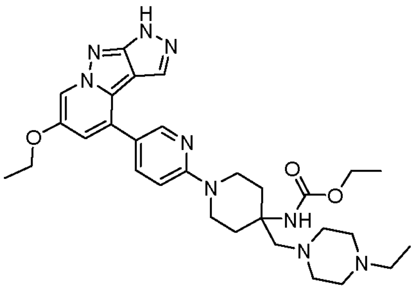 | 209 | 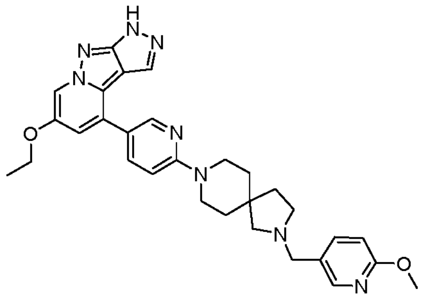 |
| 77 | 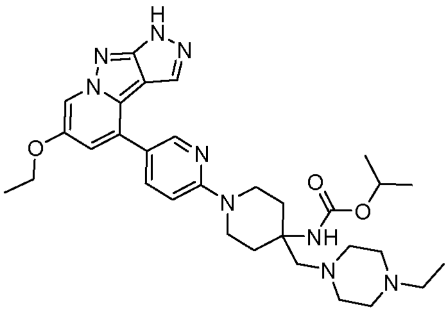 | 210 | 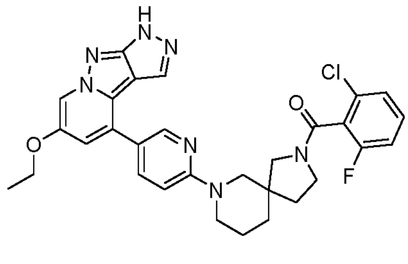 |
| 78 | 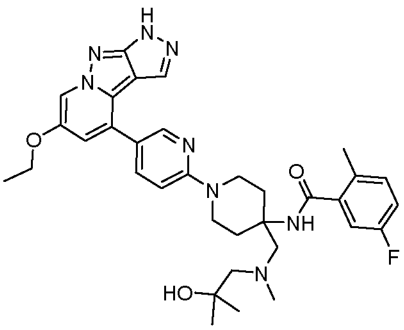 | 211 | 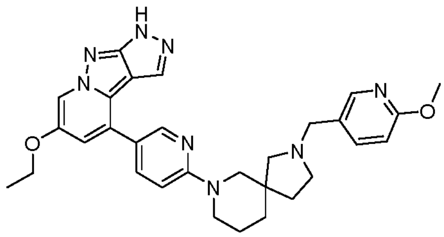 |
| 79 | 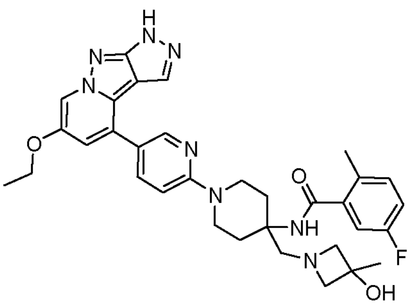 | 212 | 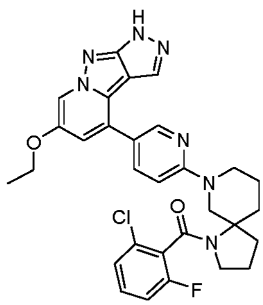 |

(continued)

| No. | Chemical structure | No. | Chemical structure |
|---|---|---|---|
| 80 | 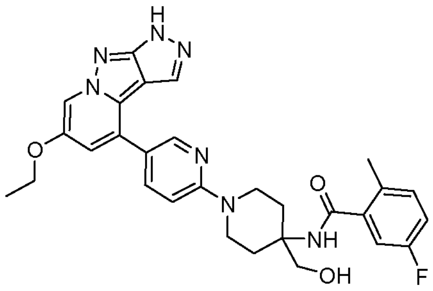 | 213 | 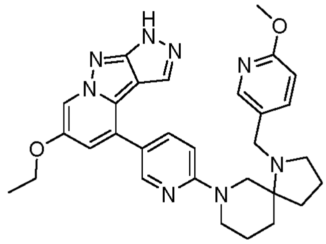 |
| 81 | 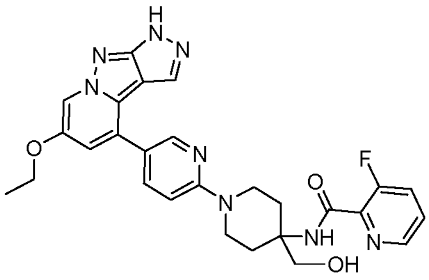 | 214 | 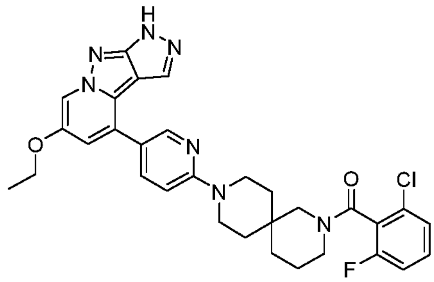 |
| 82 | 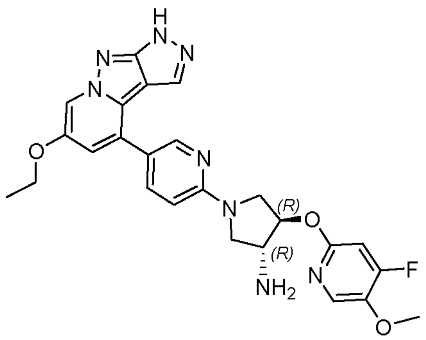 | 215 | 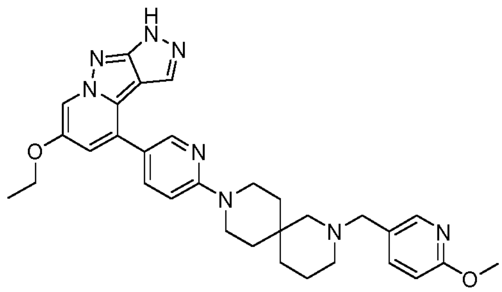 |
| 83 | 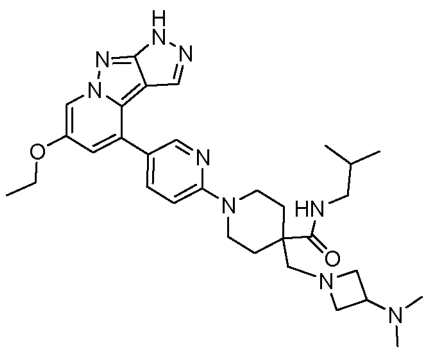 | 216 | 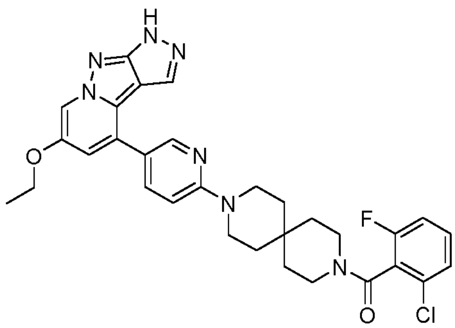 |

(continued)

| No. | Chemical structure | No. | Chemical structure |
|---|---|---|---|
| 84 | 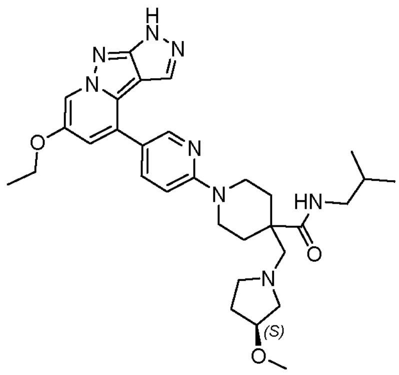 | 217 | 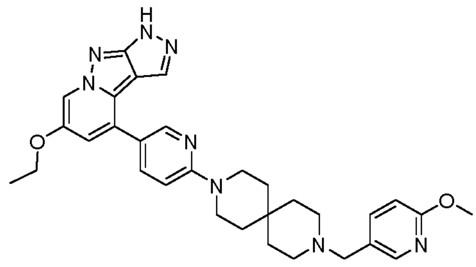 |
| 85 | 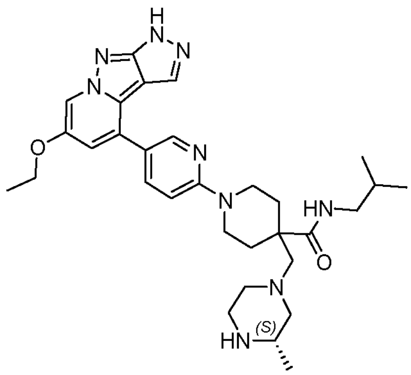 | 218 | 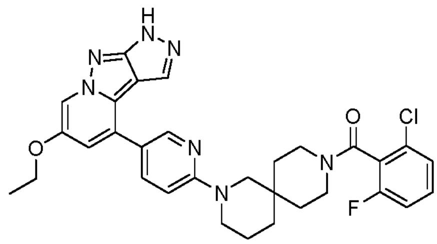 |
| 86 | 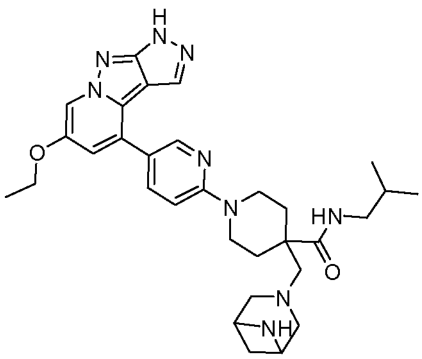 | 219 | 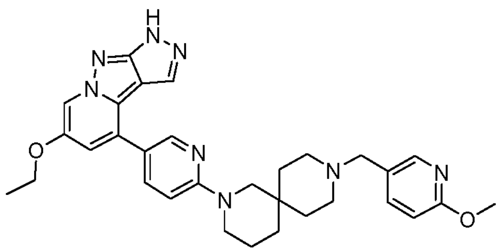 |
| 87 | 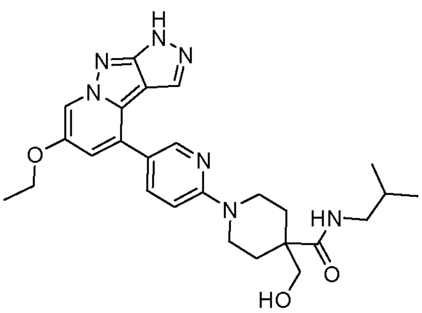 | 220 | 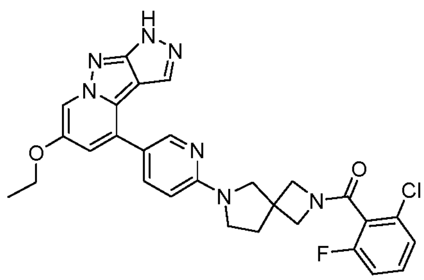 |

(continued)

| No. | Chemical structure | No. | Chemical structure |
|---|---|---|---|
| 88 | 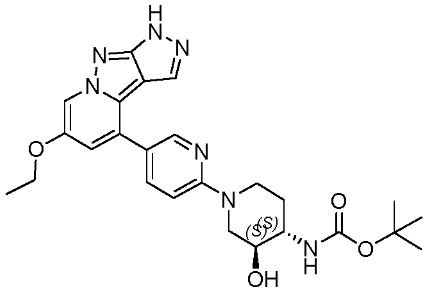 | 221 | 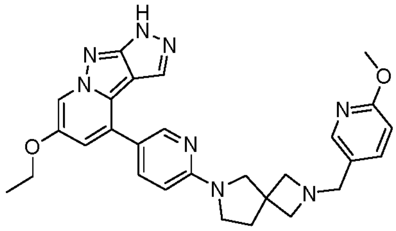 |
| 89 | 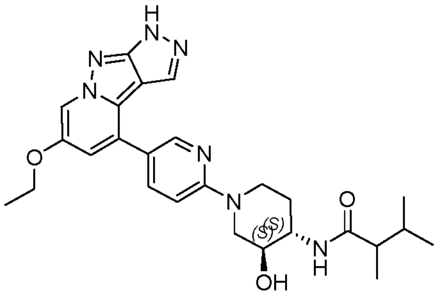 | 222 | 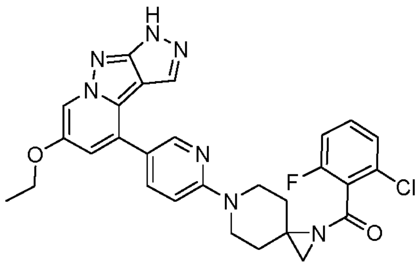 |
| 90 | 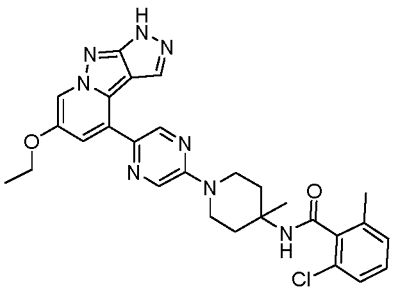 | 223 | 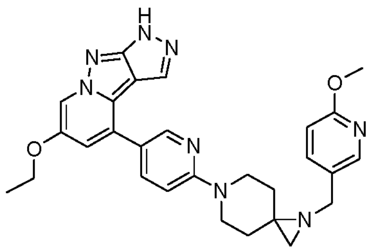 |
| 91 | 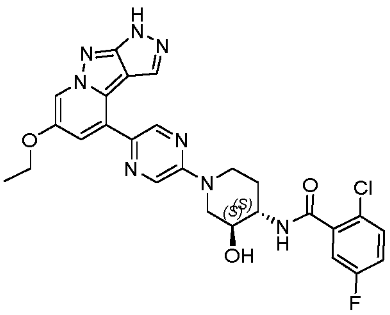 | 224 | 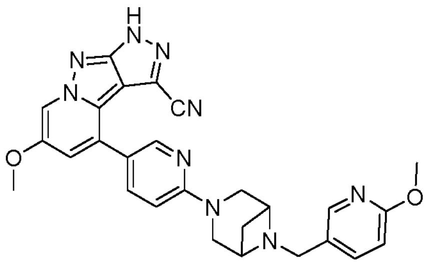 |
| 92 | 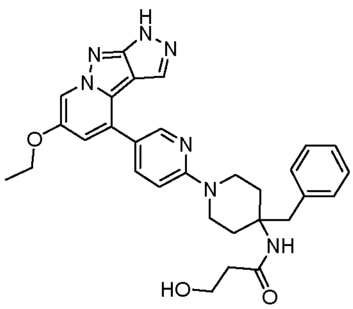 | 225 | 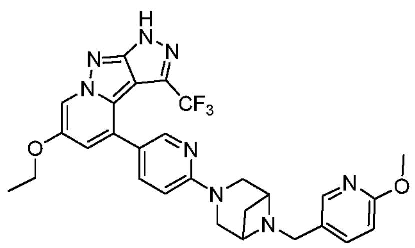 |

(continued)

| No. | Chemical structure | No. | Chemical structure |
|---|---|---|---|
| 93 | 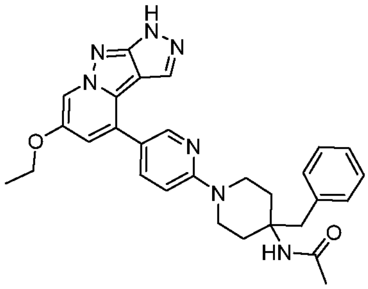 | 226 | 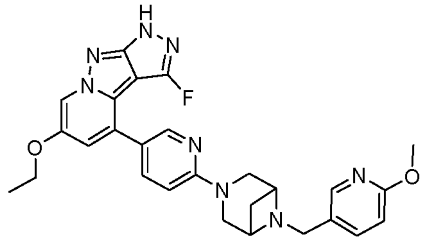 |
| 94 | 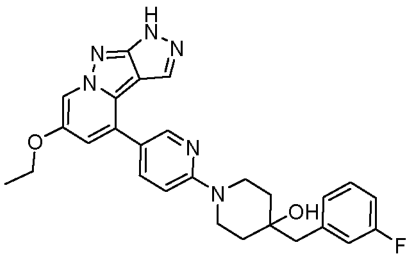 | 227 | 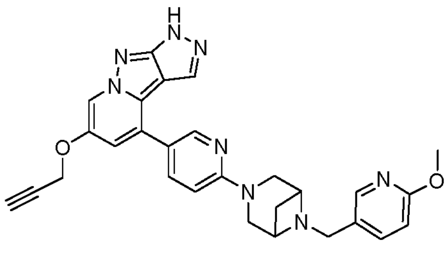 |
| 95 | 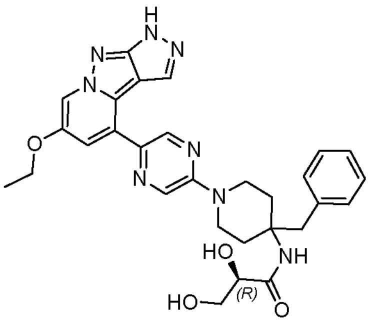 | 228 | 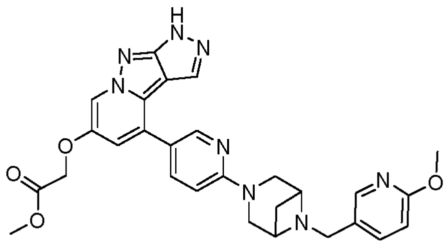 |
| 96 | 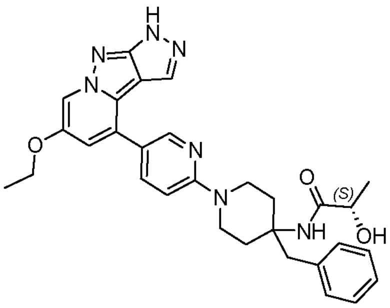 | 229 | 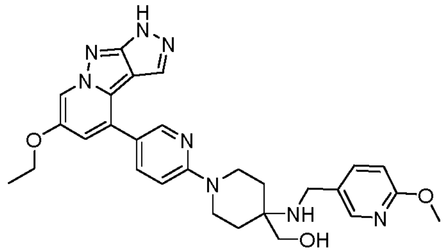 |
| 97 | 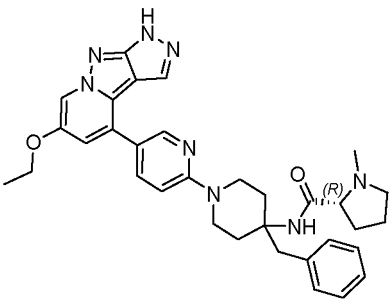 | 230 | 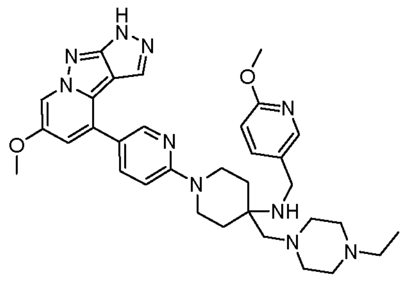 |

(continued)

| No. | Chemical structure | No. | Chemical structure |
|---|---|---|---|
| 98 | 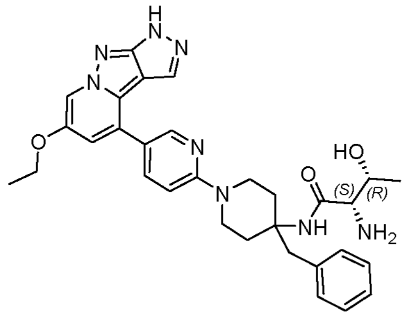 | 231 | 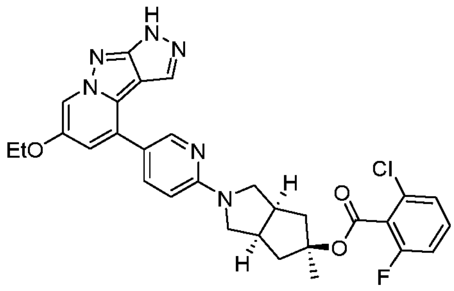 |
| 99 | 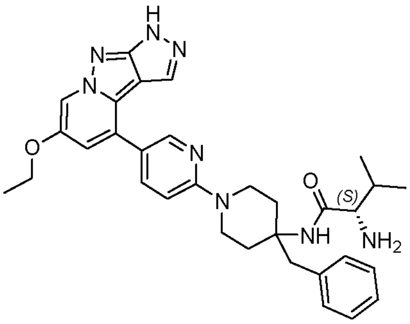 | 232 | 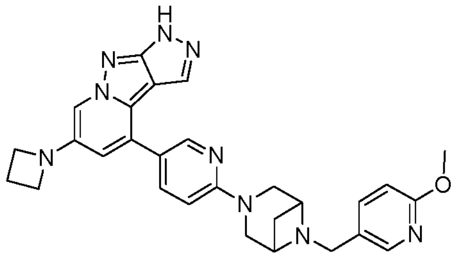 |
| 100 | 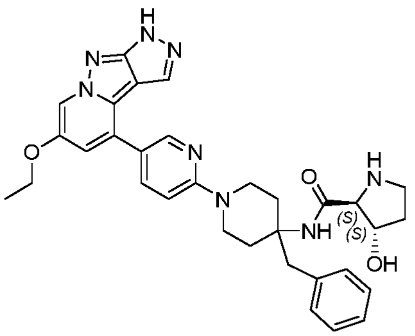 | 233 | 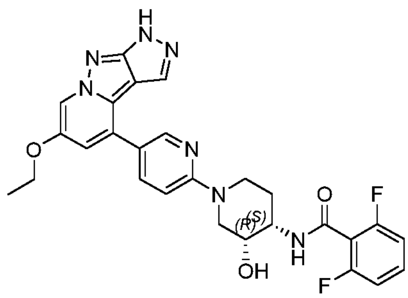 |
| 101 | 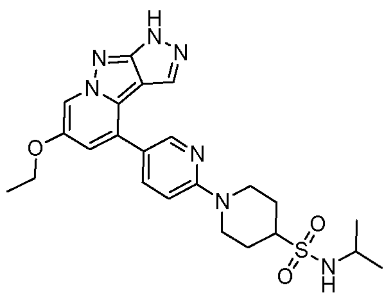 | 234 | 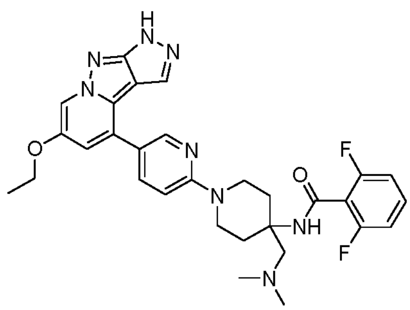 |
| 102 | 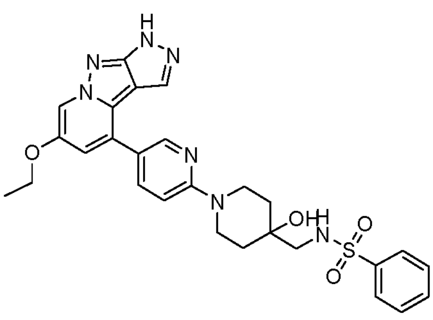 | 235 | 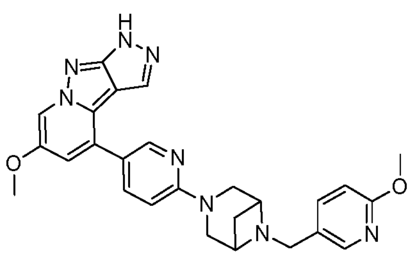 |

(continued)

| No. | Chemical structure | No. | Chemical structure |
|---|---|---|---|
| 103 | 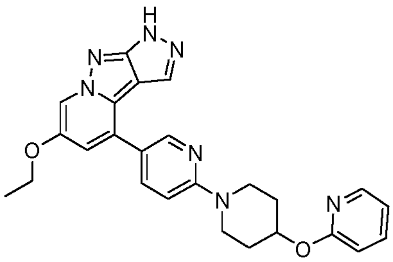 | 236 | 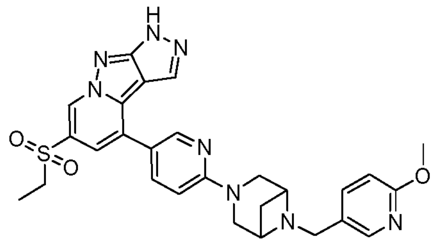 |
| 104 | 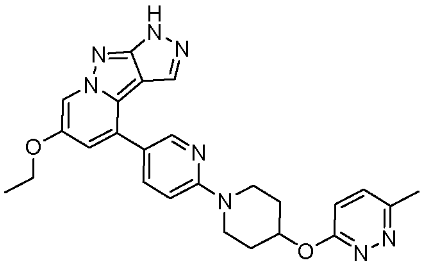 | 237 | 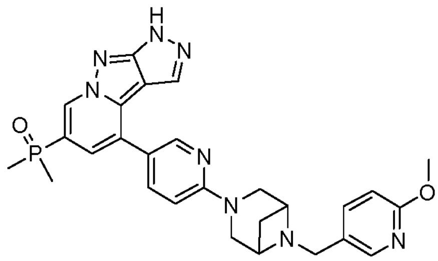 |
| 105 | 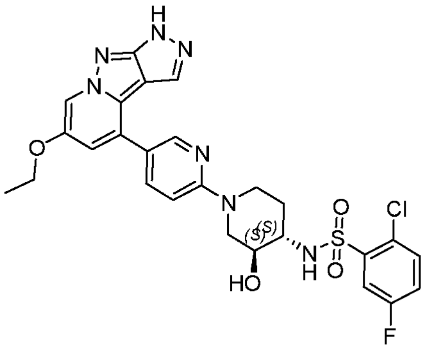 | 238 | 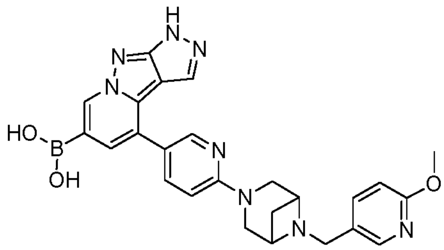 |
| 106 | 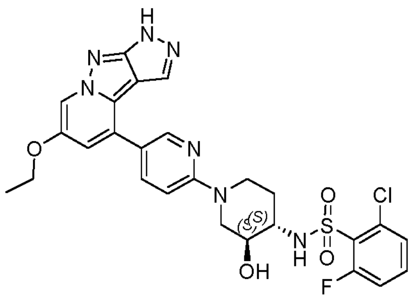 | 239 | 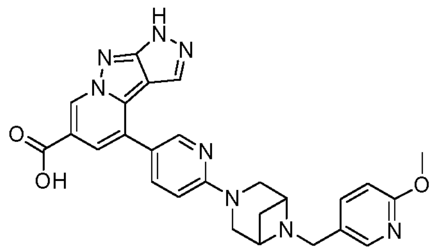 |
| 107 | 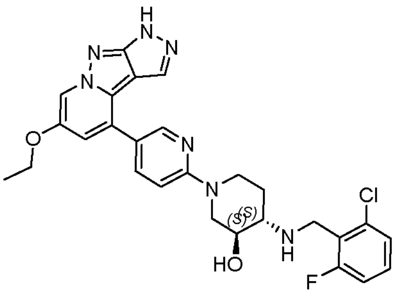 | 240 | 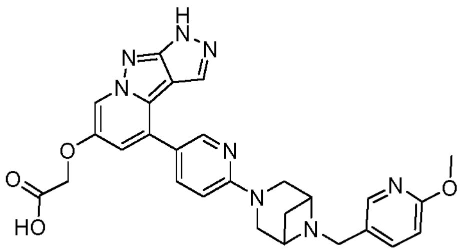 |

(continued)

| No. | Chemical structure | No. | Chemical structure |
|---|---|---|---|
| 108 | 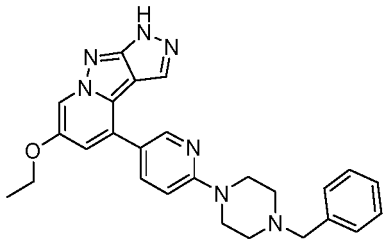 | 241 | 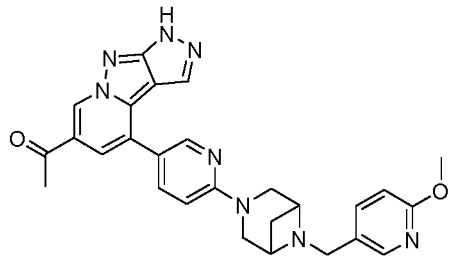 |
| 109 | 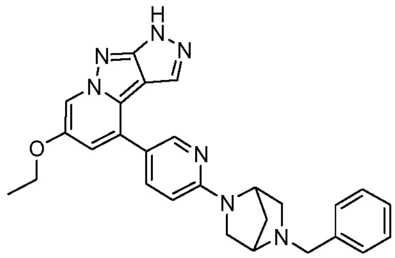 | 242 | 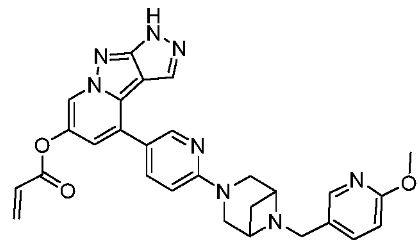 |
| 110 | 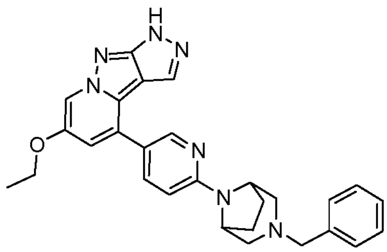 | 243 | 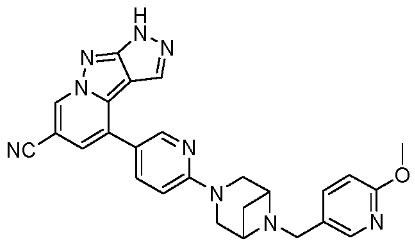 |
| 111 | 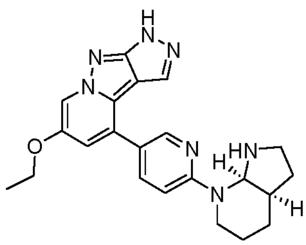 | 244 | 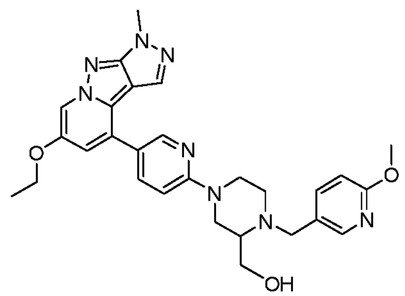 |
| 112 | 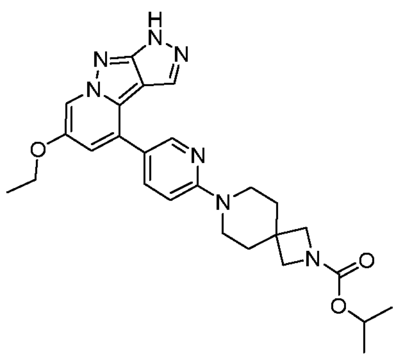 | 245 | 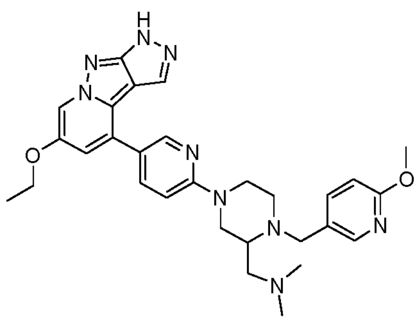 |

(continued)

| No. | Chemical structure | No. | Chemical structure |
|---|---|---|---|
| 113 | 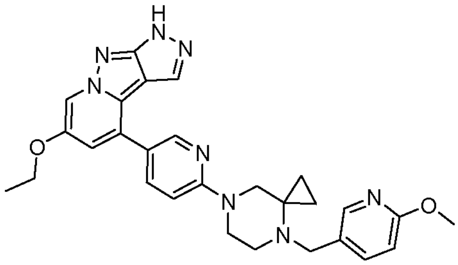 | 246 | 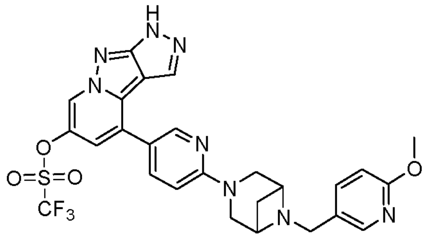 |
| 114 | 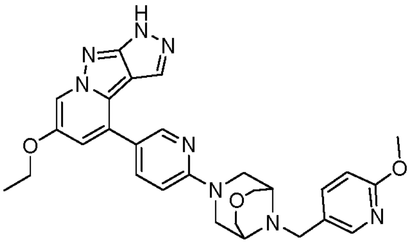 | 247 | 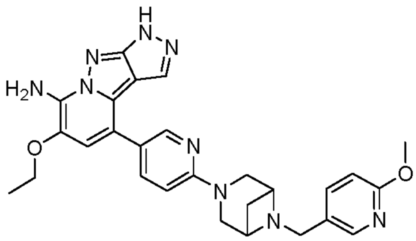 |
| 115 | 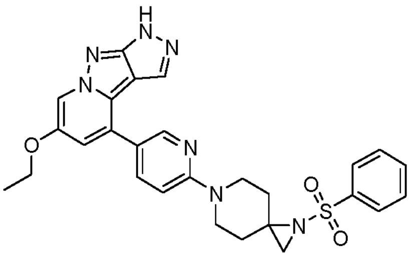 | 248 | 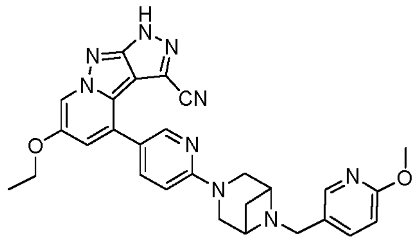 |
| 116 | 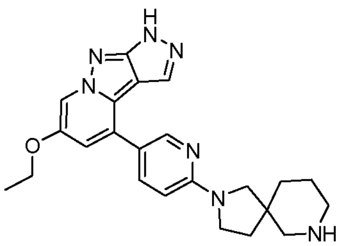 | 249 | 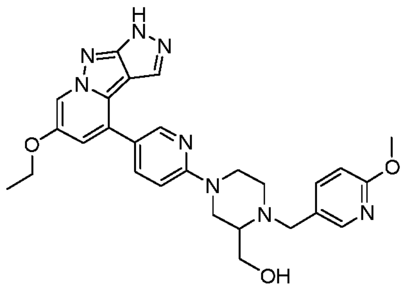 |
| 117 | 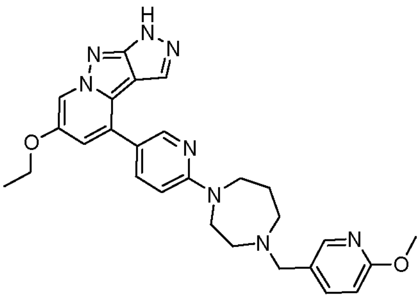 | 250 | 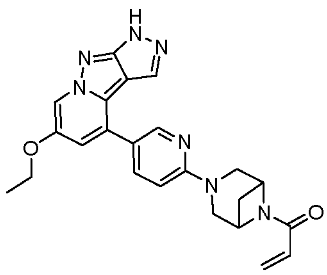 |

(continued)

| No. | Chemical structure | No. | Chemical structure |
|---|---|---|---|
| 118 | 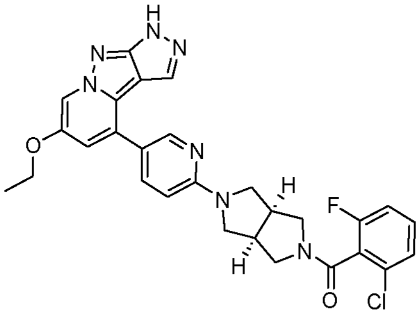 | 251 | 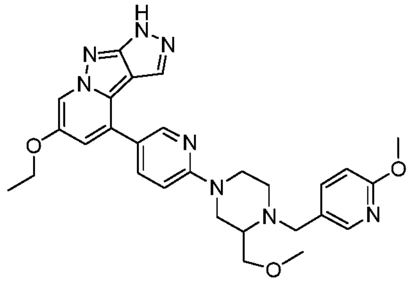 |
| 119 | 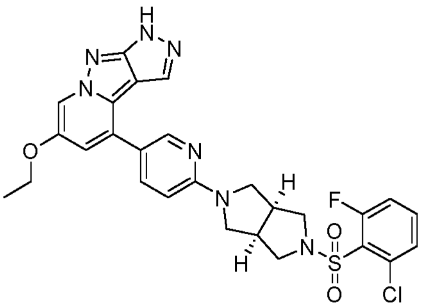 | 252 | 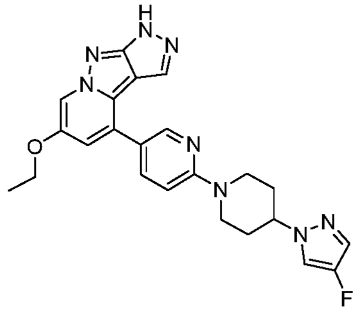 |
| 120 | 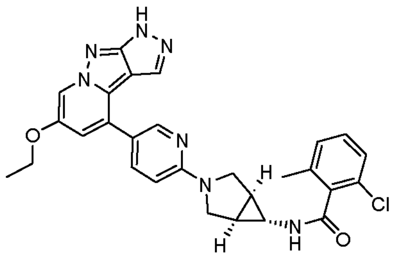 | 253 | 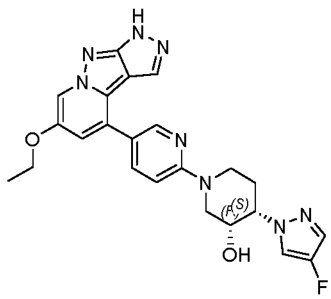 |
| 121 | 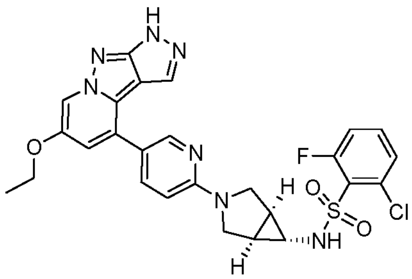 | 254 | 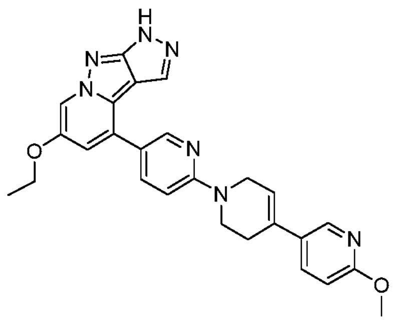 |
| 122 | 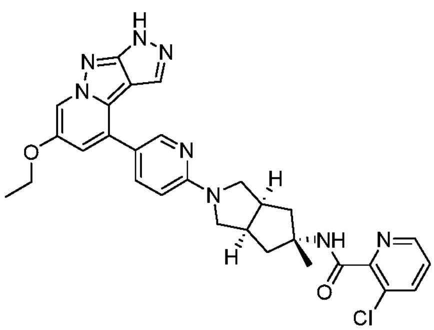 | 255 | 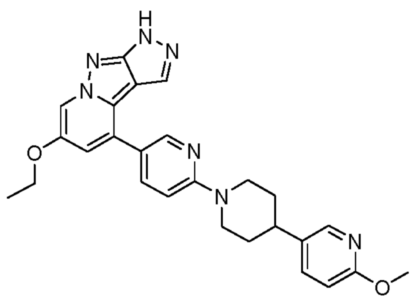 |

(continued)

| No. | Chemical structure | No. | Chemical structure |
|---|---|---|---|
| 123 | 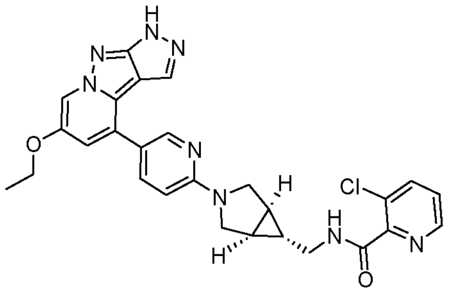 | 256 | 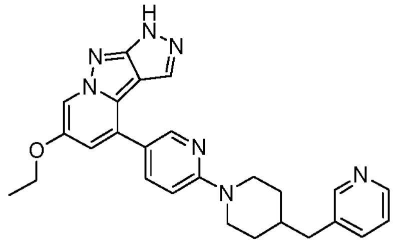 |
| 124 | 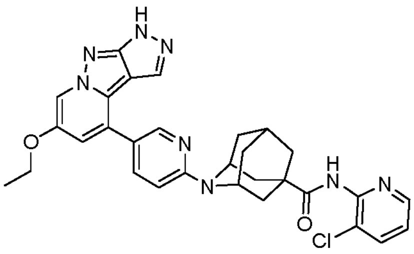 | 257 | 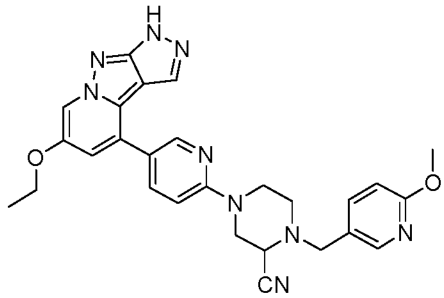 |
| 125 | 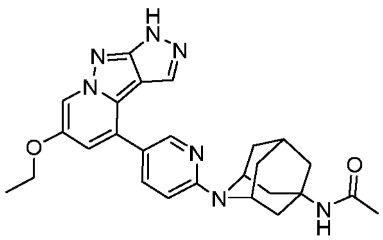 | 258 | 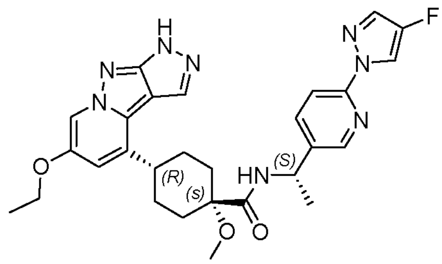 |
| 126 | 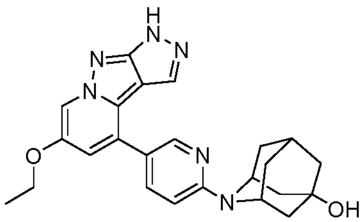 | 259 | 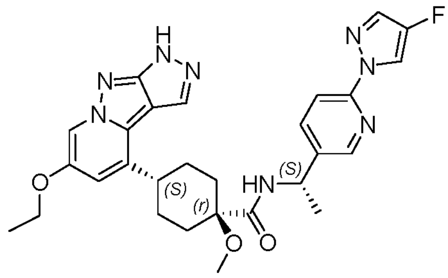 |
| 127 | 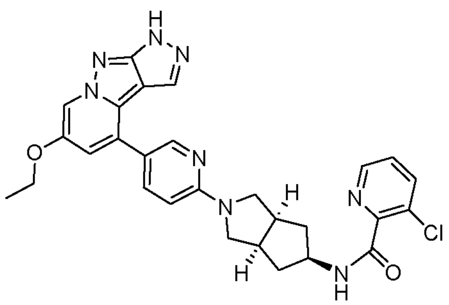 | 260 | 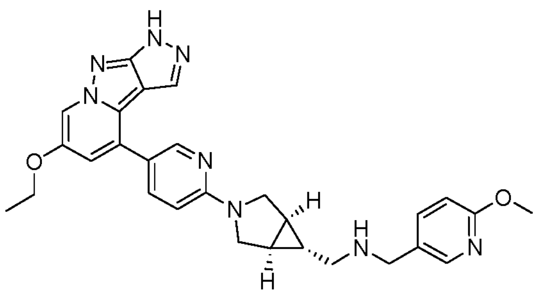 |

(continued)

| No. | Chemical structure | No. | Chemical structure |
|---|---|---|---|
| 128 | 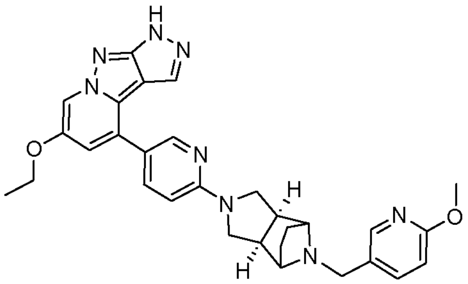 | 261 | 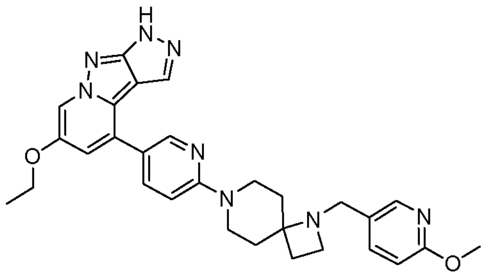 |
| 129 | 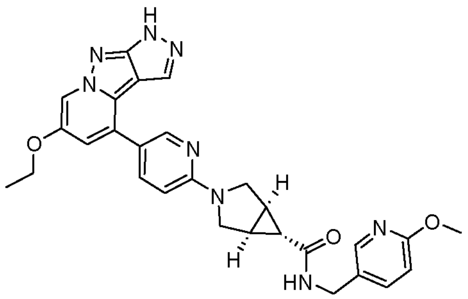 | 262 | 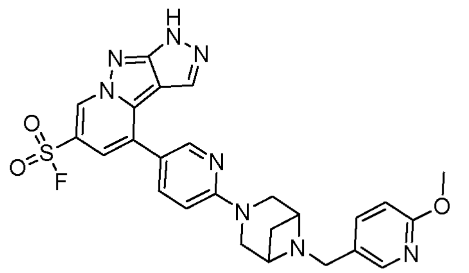 |
| 130 | 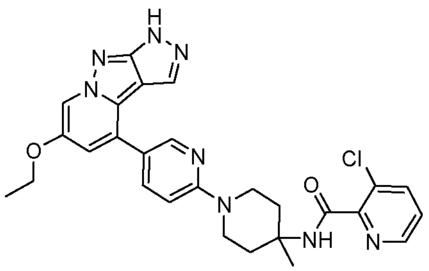 | 263 | 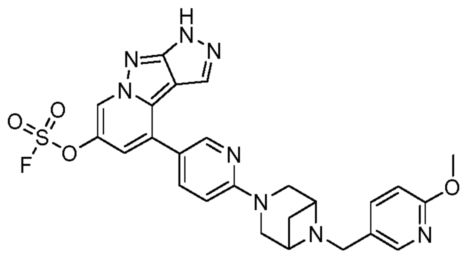 |
| 131 | 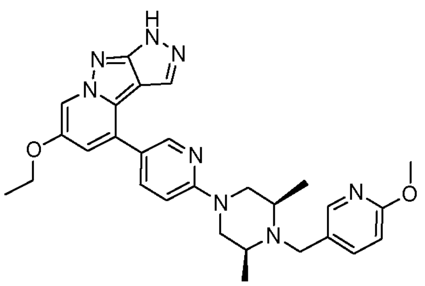 | 264 | 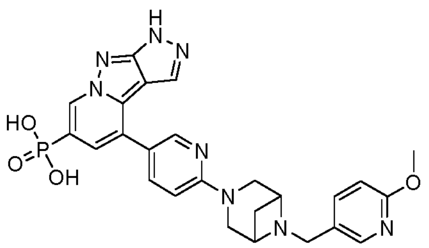 |
| 132 | 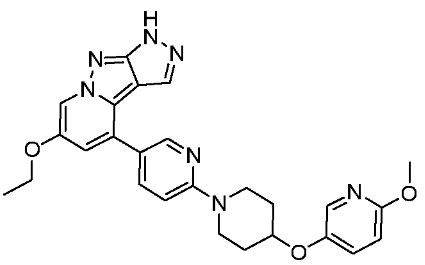 | 265 | 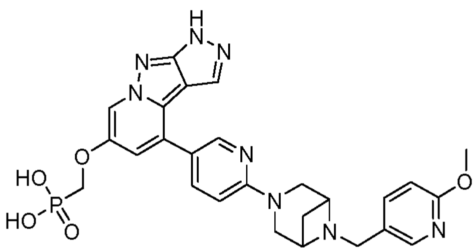 |

(continued)

| No. | Chemical structure | No. | Chemical structure |
| --- | --- | --- | --- |
| 133 | 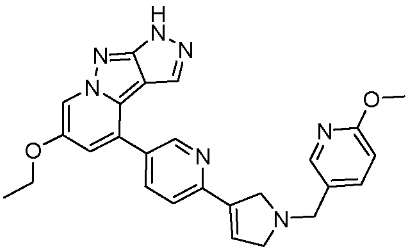 | 266 | 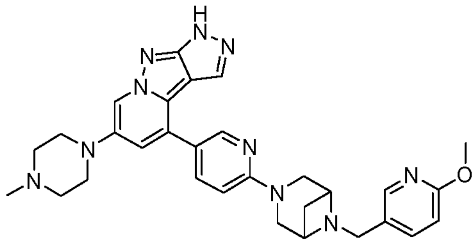 |

| No. | Chemical structure | No. | Chemical structure |
| --- | --- | --- | --- |
| 267 | 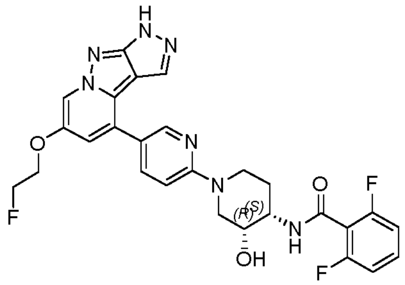 | 283 | 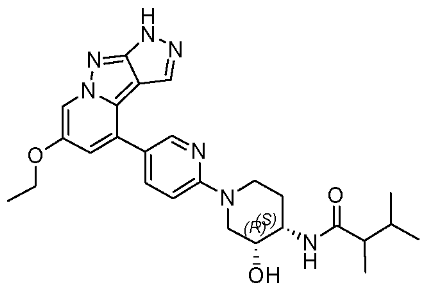 |
| 268 | 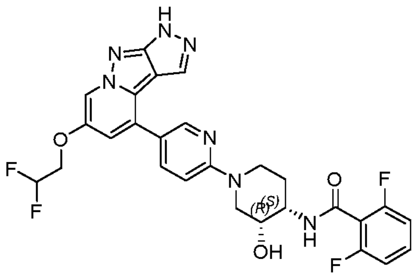 | 284 | 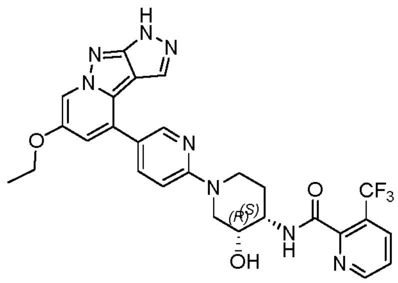 |
| 269 | 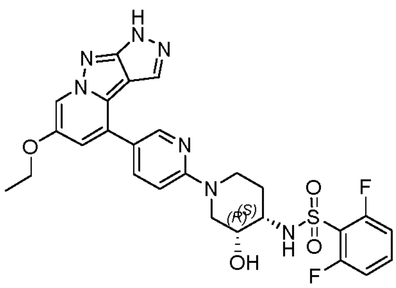 | 285 | 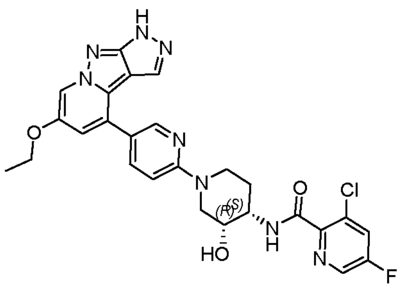 |
| 270 | 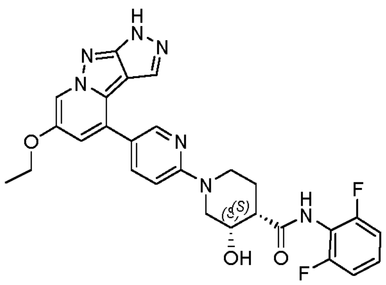 | 286 | 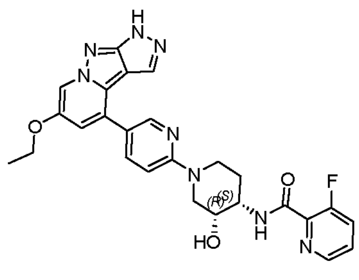 |

(continued)

| No. | Chemical structure | No. | Chemical structure |
|---|---|---|---|
| 271 | 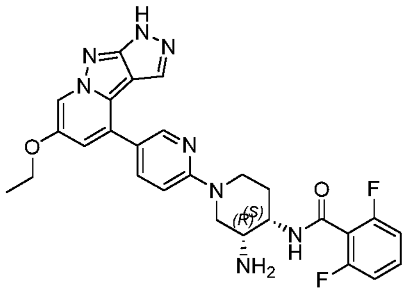 | 287 | 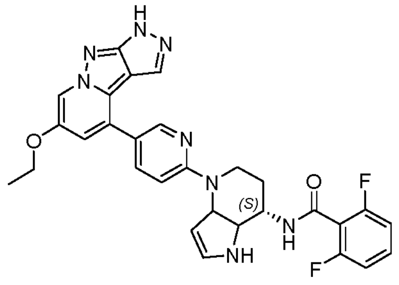 |
| 272 | 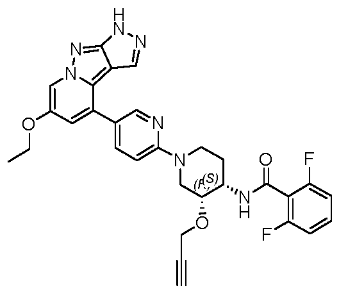 | 288 | 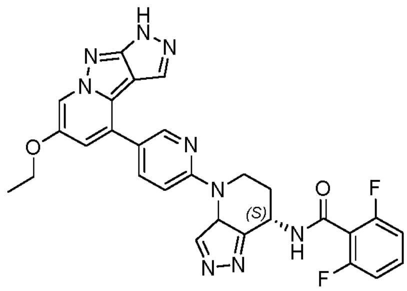 |
| 273 | 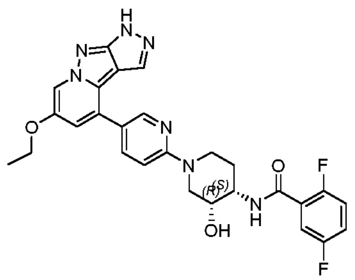 | 289 | 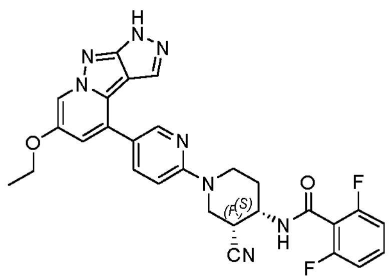 |
| 274 | 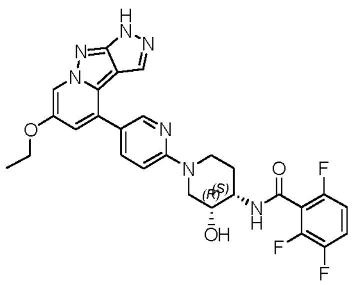 | 290 | 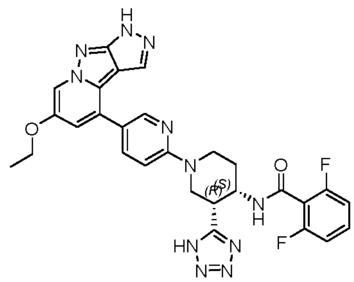 |
| 275 | 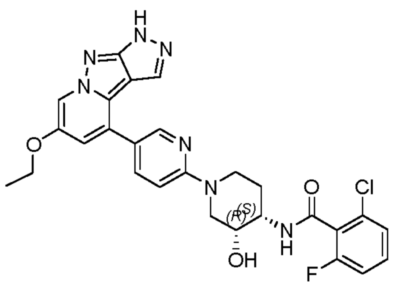 | 291 | 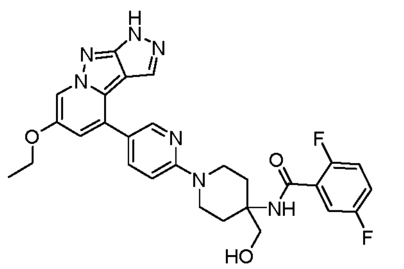 |

(continued)

| No. | Chemical structure | No. | Chemical structure |
|---|---|---|---|
| 276 | 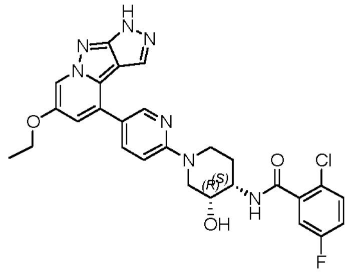 | 292 | 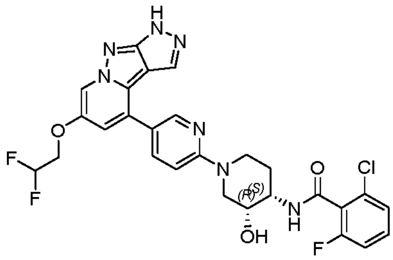 |
| 277 | 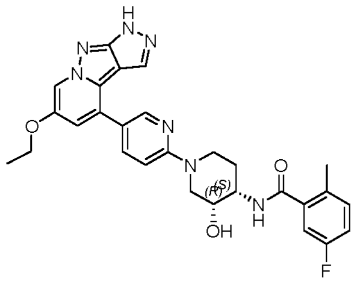 | 293 | 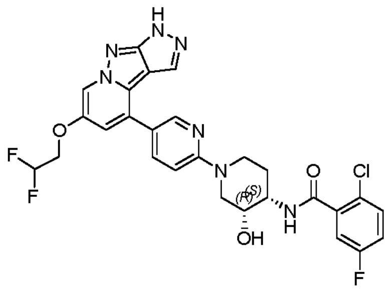 |
| 278 | 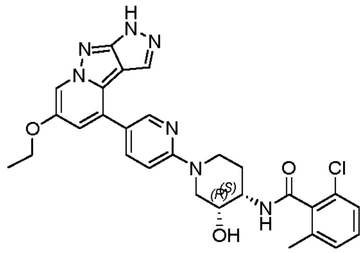 | 294 | 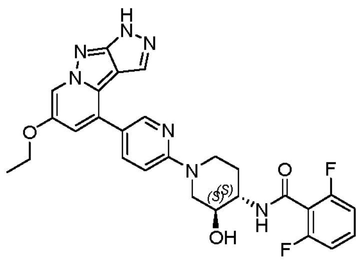 |
| 279 | 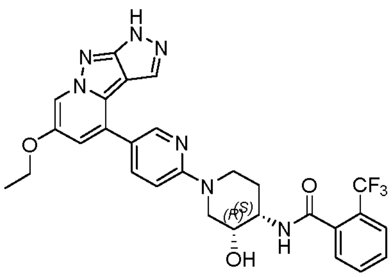 | 295 | 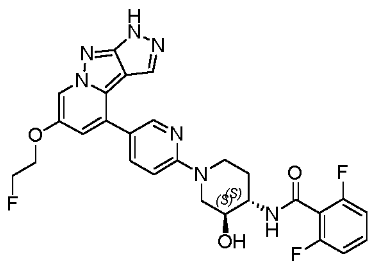 |
| 280 | 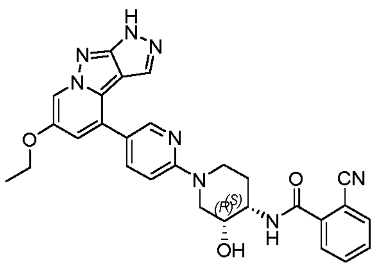 | 296 | 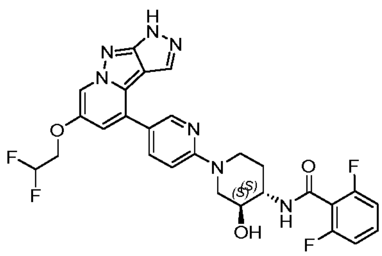 |

(continued)

| No. | Chemical structure | No. | Chemical structure |
|---|---|---|---|
| 281 | 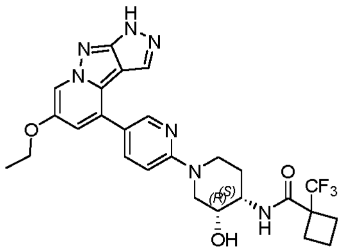 | 297 | 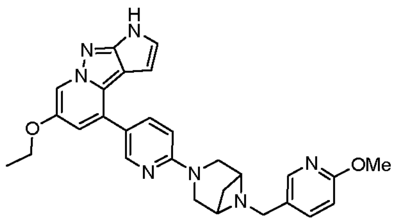 |
| 282 | 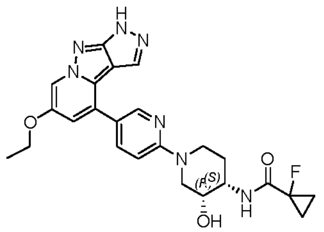 | 298 | 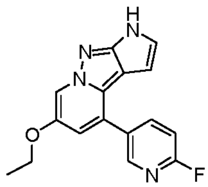 |

The present disclosure further provides a preparation method for the compound represented by formula I, comprising:

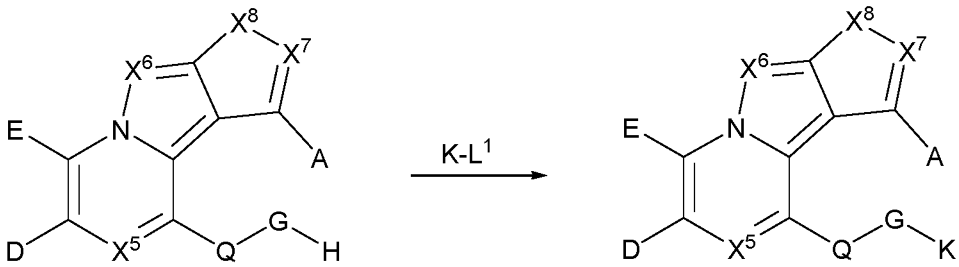


I-1 I

wherein A, D, E, Q, G, K, $X^5$, $X^6$, $X^7$, and $X^8$ are as defined above; $L^1$ is selected from a leaving group.

Alternatively, the preparation method comprises the following steps:

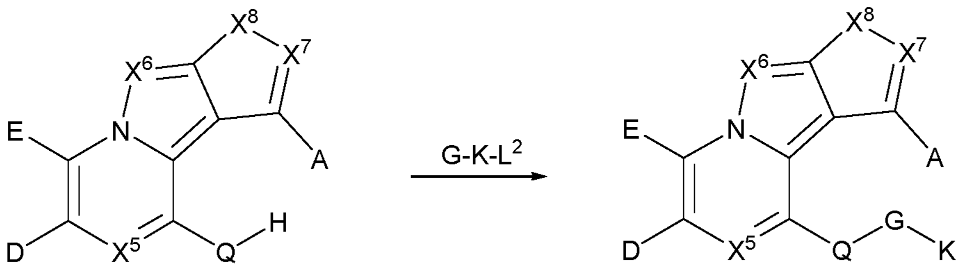


I-2 I

wherein A, D, E, Q, G, K, $X^5$, $X^6$, $X^7$, and $X^8$ are as defined above; $L^2$ is selected from a leaving group.

Alternatively, the preparation method comprises the following steps:

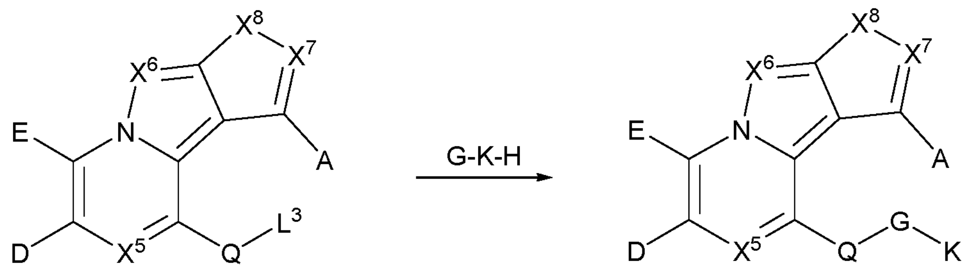


I-3

I

wherein A, D, E, Q, G, K, $X^5$, $X^6$, $X^7$, and $X^8$ are as defined above; $L^3$ is selected from a leaving group.

Alternatively, the preparation method comprises reacting a compound represented by formula I substituted with a protecting group under a condition where the protecting group is removed to give the compound represented by formula I. According to the substituents in the compound represented by formula I, the protecting group may be selected from at least one of a hydroxyl protecting group, an amino protecting group, and the like.

The present disclosure further provides a preparation method for the compound represented by formula II, comprising the following steps:

reacting a compound represented by formula II-1 with compound $R^{21}$-L to give the compound represented by formula II,

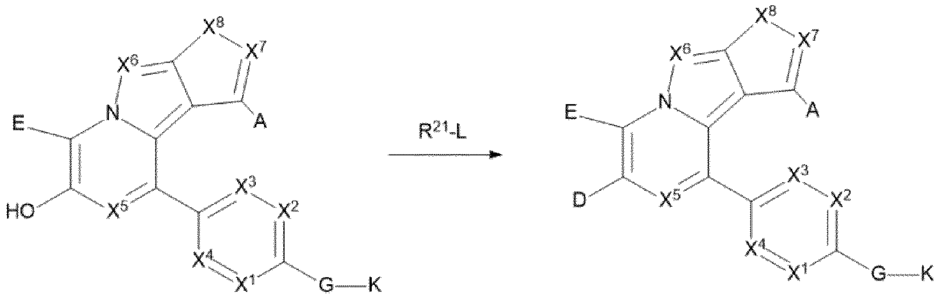


II-1

II

wherein A, E, G, K, $X^1$, $X^2$, $X^3$, $X^4$, $X^5$, $X^6$, $X^7$, $X^8$, and $R^{21}$ are as defined above;
D is selected from -O-$R^{21}$; L is selected from a leaving group.

According to an embodiment of the present disclosure, the leaving group is selected from OH, halogen, and OTf.

According to an embodiment of the present disclosure, the reaction is conducted in the presence of alkali, for example, potassium carbonate.

According to an embodiment of the present disclosure, the reaction is conducted at a temperature of 50-100 °C for a period of 1-24 h.

According to an embodiment of the present disclosure, the reaction may be conducted in the presence of an organic solvent (for example, DMF).

The present disclosure further provides a preparation method for the compound represented by formula II-1, comprising reacting a compound represented by formula II-2 to give the compound represented by formula II-1:

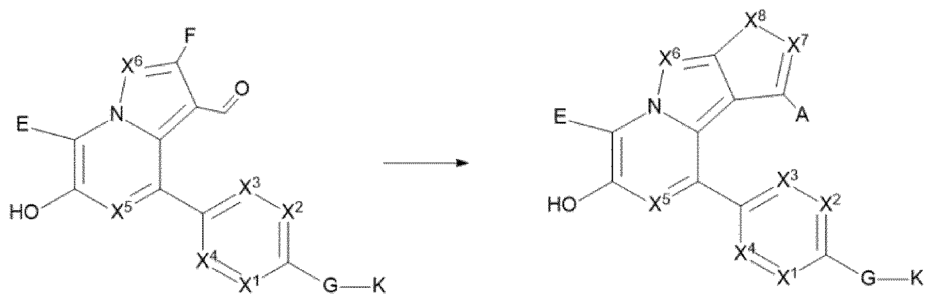

II-2 II-1

wherein A, D, E, G, K, $X^1$, $X^2$, $X^3$, $X^4$, $X^5$, $X^6$, $X^7$, and $X^8$ are as defined above.

According to an embodiment of the present disclosure, the reaction is conducted in the presence of hydrazine hydrate, and $X^7$ is N and $X^8$ is NH.

According to an embodiment of the present disclosure, the reaction is conducted in the presence of an organic solvent (for example, DMF).

According to an embodiment of the present disclosure, the reaction is conducted in a heating condition.

The present disclosure further provides a compound represented by formula I-1, formula I-2, formula I-3, formula II-1, or formula II-2 described above.

The present disclosure further provides use of the compound represented by formula I-1, formula I-2, formula I-3, or formula II-1 for manufacturing the compound represented formula I.

The present disclosure further provides a pharmaceutical composition, comprising at least one selected from the compound represented by formula I, and a stereoisomer, a racemate, a tautomer, an isotopically labeled compound, a nitrogen oxide and a pharmaceutically acceptable salt thereof, for example, a therapeutically effective amount of at least one selected from the compound represented by formula I, and the stereoisomer, the racemate, the tautomer, the isotopically labeled compound, the nitrogen oxide and the pharmaceutically acceptable salt thereof.

According to an embodiment of the present disclosure, the pharmaceutical composition further comprises one, two, or more pharmaceutically acceptable auxiliary material, such as carriers and/or excipients.

According to an embodiment of the present disclosure, the pharmaceutical composition further comprises one or more additional therapeutic agents.

The present disclosure further provides a method for inhibiting cell proliferation *in vitro* or *in vivo*, comprising contacting a cell with an effective amount of the compound represented by formula I, or the stereoisomer, the racemate, the tautomer, the isotopically labeled compound, the nitrogen oxide or the pharmaceutically acceptable salt thereof, or the pharmaceutical composition thereof of the present disclosure.

The present disclosure further provides a method for treating a disease mediated by RET gene and/or RET kinase, comprising administering to a patient a therapeutically effective amount of at least one of the compound represented by formula I, and the stereoisomer, the racemate, the tautomer, the isotopically labeled compound, the nitrogen oxide and the pharmaceutically acceptable salt thereof.

According to an embodiment of the present disclosure, when referring to RET, RET gene, or RET kinase, it means an RET gene or RET kinase selected from the group including but not limited to, RET-wt, V804M, V804L, V804E, G810R, G810S, G810C, G810V, and S904F.

The present disclosure further provides a method for treating a disease or condition associated with RET in a patient in need of treatment, comprising administering to the patient a therapeutically effective amount of the compound represented by formula I, or the stereoisomer, the racemate, the tautomer, the isotopically labeled compound, the nitrogen oxide or the pharmaceutically acceptable salt thereof, or the pharmaceutical composition thereof of the present disclosure.

The present disclosure further provides a method for treating a cancer and/or inhibiting a metastasis associated with the cancer in a patient in need of treatment, comprising administering to the patient a therapeutically effective amount of the compound represented by formula I, or the stereoisomer, the racemate, the tautomer, the isotopically labeled compound, the nitrogen oxide or the pharmaceutically acceptable salt thereof, or the pharmaceutical composition thereof of the present disclosure.

The present disclosure further provides a method for treating irritable bowel syndrome (IBS) and/or a pain associated with IBS in a patient in need of treatment, comprising administering to the patient a therapeutically effective amount of the compound represented by formula I, or the stereoisomer, the racemate, the tautomer, the isotopically labeled compound, the nitrogen oxide or the pharmaceutically acceptable salt thereof, or the pharmaceutical composition thereof of the present disclosure.

The present disclosure further provides a method for providing a supportive care for a cancer patient, including preventing or minimizing a gastrointestinal disease (for example, diarrhea) associated with a treatment (including a

chemotherapy), comprising administering to the patient a therapeutically effective amount of the compound represented by formula I, or the stereoisomer, the racemate, the tautomer, the isotopically labeled compound, the nitrogen oxide or the pharmaceutically acceptable salt thereof, or the pharmaceutical composition thereof of the present disclosure.

The present disclosure further provides use of at least one of the compound represented by formula I, or the stereoisomer, the racemate, the tautomer, the isotopically labeled compound, the nitrogen oxide or the pharmaceutically acceptable salt thereof for manufacturing a medicament for treating a RET kinase mediated disease, inhibiting RET kinase activity, treating a cancer and/or inhibiting a metastasis associated with the cancer, treating irritable bowel syndrome (IBS) or a pain associated with IBS, providing supportive care for a cancer patient, treating a disease or condition associated with RET, reversing or preventing acquired resistance to an anti-cancer drug, or delaying and/or preventing the development of resistance to an anti-cancer drug in an individual or an increased possibility of developing resistance to an anti-cancer drug.

According to an embodiment of the present disclosure, the disease or condition associated with RET is selected from a disease mediated by RET gene and/or RET kinase, wherein RET, RET gene, or RET kinase is an RET gene or RET kinase selected from the group including but not limited to, RET-wt, V804M, V804L, V804E, G810R, G810S, G810C, G810V, and S904F.

The present disclosure further provides use of at least one of the compound represented by formula I, and the stereoisomer, the racemate, the tautomer, the isotopically labeled compound, the nitrogen oxide and the pharmaceutically acceptable salt thereof for manufacturing a medicament for treating a disease mediated by RET kinase.

The present disclosure further provides use of at least one of the compound represented by formula I, and the stereoisomer, the racemate, the tautomer, the isotopically labeled compound, the nitrogen oxide and the pharmaceutically acceptable salt thereof for manufacturing a medicament for treating a cancer and/or inhibiting a metastasis associated with a specific cancer.

The present disclosure further provides use of at least one of the compound represented by formula I, and the stereoisomer, the racemate, the tautomer, the isotopically labeled compound, the nitrogen oxide and the pharmaceutically acceptable salt thereof for manufacturing a medicament for treating irritable bowel syndrome (IBS) or a pain associated with IBS.

The present disclosure further provides use of at least one of the compound represented by formula I, and the stereoisomer, the racemate, the tautomer, the isotopically labeled compound, the nitrogen oxide and the pharmaceutically acceptable salt thereof for manufacturing a medicament for providing a supportive care, including preventing or minimizing a gastrointestinal condition associated with a treatment (including a chemotherapy), for example, diarrhea, for a cancer patient. The present disclosure further provides use of at least one of the compound represented by formula I, and the stereoisomer, the racemate, the tautomer, the isotopically labeled compound, the nitrogen oxide and the pharmaceutically acceptable salt thereof for manufacturing a medicament for inhibiting RET kinase activity.

The present disclosure further provides use of at least one of the compound represented by formula I, and the stereoisomer, the racemate, the tautomer, the isotopically labeled compound, the nitrogen oxide and the pharmaceutically acceptable salt thereof for manufacturing a medicament for treating a disease or condition associated with RET.

The present disclosure further provides a method for treating a cancer in a patient in need, comprising: (a) determining whether the cancer is associated with the dysregulation of: RET gene, RET kinase, or the expression, activity, or level of either (for example, a cancer associated with RET); (b) if it is determined that the cancer is associated with the dysregulation of: RET gene, RET kinase, or the expression, activity, or level of either (for example, a cancer associated with RET), administering to the patient a therapeutically effective amount of at least one of the compound represented by formula I, and the stereoisomer, the racemate, the tautomer, the isotopically labeled compound, the nitrogen oxide and the pharmaceutically acceptable salt thereof, or the pharmaceutical composition thereof.

The present disclosure further provides a method for reversing or preventing acquired resistance to an anti-cancer drug, comprising administering to a patient developing or at risk of having the acquired resistance to the anti-cancer drug a therapeutically effective amount of at least one of the compound represented by formula I, and the stereoisomer, the racemate, the tautomer, the isotopically labeled compound, the nitrogen oxide and the pharmaceutically acceptable salt thereof. The present disclosure further provides a method for delaying and/or preventing the development of a resistance to an anti-cancer drug in an individual, comprising administering to the individual an effective amount of at least one of the compound represented by formula I, and the stereoisomer, the racemate, the tautomer, the isotopically labeled compound, the nitrogen oxide and the pharmaceutically acceptable salt thereof before, during, or after administering an effective amount of the anti-cancer drug.

The present disclosure further provides a method for treating an individual suffering from a cancer and having an increased possibility of developing a resistance to an anti-cancer drug, comprising co-administering to the individual: (a) an effective amount of at least one of the compound represented by formula I, and the stereoisomer, the racemate, the tautomer, the isotopically labeled compound, the nitrogen oxide and the pharmaceutically acceptable salt thereof; and (b) an effective amount of the anti-cancer drug.

The present disclosure further provides a method for treating an individual suffering from a cancer associated

with RET, wherein the cancer has one or more RET inhibitor resistance mutations increasing the resistance of the cancer to an RET inhibitor other than at least one of the compound represented by formula I, and the stereoisomer, the racemate, the tautomer, the isotopically labeled compound, the nitrogen oxide and the pharmaceutically acceptable salt thereof (for example, RET-wt, substitutions at amino acid positions 804, 810, and 904, e.g. V804M, V804L, V804E, G810R, G810S, G810C, G810V and S904F), and the method comprises administering at least one of the compound represented by formula I, and the stereoisomer, the racemate, the tautomer, the isotopically labeled compound, the nitrogen oxide and the pharmaceutically acceptable salt thereof before, during, or after administering an additional anti-cancer drug.

The present disclosure further provides a method for treating an individual suffering from a cancer associated with RET, comprising administering at least one of the compound represented by formula I, and the stereoisomer, the racemate, the tautomer, the isotopically labeled compound, the nitrogen oxide and the pharmaceutically acceptable salt thereof before, during, or after administering an additional anti-cancer drug.

The present disclosure provides a method for treating a cancer (for example, a cancer associated with RET) in a patient in need, comprising administering to the patient a therapeutically effective amount of at least one of the compound represented by formula I and the pharmaceutically acceptable salt thereof, or the pharmaceutical composition thereof.

In some embodiments of any of the methods or use described herein, the cancer (e.g., the cancer associated with RET) is a hematologic cancer. In some embodiments of any of the methods or use described herein, the cancer (e.g., the cancer associated with RET) is a solid tumor. In some embodiments of any of the methods or use described herein, the cancer (e.g., the cancer associated with RET) is lung cancer (e.g., small cell lung cancer or non-small cell lung cancer), papillary thyroid cancer, medullary thyroid cancer, differentiated thyroid cancer, recurrent thyroid cancer, refractory differentiated thyroid cancer, lung adenocarcinoma, bronchiolar lung cancer, multiple endocrine neoplasia type 2A or 2B (MEN2A or MEN2B), pheochromocytoma, parathyroid hyperplasia, breast cancer, colorectal cancer (e.g., metastatic colorectal cancer), papillary renal cell carcinoma, gangliocytomatosis of the gastrointestinal mucosa, inflammatory myofibroblastoma, or cervical cancer. In some embodiments of any of the methods or use described herein, the cancer (e.g., the cancer associated with RET) is selected from: acute lymphocytic leukemia (ALL), acute myeloid leukemia (AML), juvenile cancer, adrenocortical carcinoma, anal cancer, appendiceal cancer, astrocytoma, atypical teratoma/rhabdomyoid tumor, basal cell carcinoma, cholangiocarcinoma, bladder cancer, bone cancer, brainstem glioma, brain tumor, breast cancer, bronchial tumor, Burkitt's lymphoma, carcinoid tumor, unknown primary cancer, heart tumor, cervical cancer, childhood cancer, chordoma, chronic lymphocytic leukemia (CLL), chronic myelogenous leukemia (CML), chronic myeloproliferative tumor, colon cancer, colorectal cancer, craniopharyngioma, cutaneous T-cell lymphoma, cholangiocarcinoma, ductal carcinoma in situ, embryonal tumor, endometrial cancer, ependymoma, esophageal cancer, esthesioneuroblastoma, Ewing's sarcoma, extracranial germ cell tumor, extragonadal germ cell tumor, extrahepatic cholangiocarcinoma, eye cancer, fallopian tube cancer, osteofibrohistiocytoma, gallbladder cancer, gastric cancer, gastrointestinal carcinoid tumor, gastrointestinal stromal tumor (GIST), germ cell tumor, gestational trophoblastic disease, neuroglioma, hairy cell tumor, hairy cell leukemia, head and neck cancer, heart cancer, hepatocellular carcinoma, histiocytosis, Hodgkin's lymphoma, hypopharynx cancer, intraocular melanoma, islet cell tumor, pancreatic neuroendocrine tumor, Kaposi's sarcoma, kidney cancer, Langerhans cell histiocytosis, laryngeal cancer, leukemia, lip and oral cavity cancer, liver cancer, lung cancer, lymphoma, macroglobulinemia, malignant fibrous histiocytoma of bone, bone cancer, melanoma, Merkel cell carcinoma, mesothelioma, metastatic squamous neck cancer, midline carcinoma, oral cancer, multiple endocrine tumor syndrome, multiple myeloma, granulomatous mycosis fungoides, myelodysplastic syndrome, myelodysplastic/myeloproliferative tumor, myeloid leukemia, myelogenous leukemia, multiple myeloma, myeloproliferative tumors, nasal and sinus cancer, nasopharyngeal cancer, neuroblastoma, non-Hodgkin's lymphoma, non-small cell lung cancer, mouth cancer, oral cavity cancer, lip cancer, oropharyngeal cancer, osteosarcoma, ovarian cancer, pancreatic cancer, papillomatosis, paragangliomas, paranasal sinuses and nasal cavity cancer, parathyroid cancer, penile cancer, pharyngeal cancer, pheochromocytoma, pituitary cancer, plasmacytoma, pleuropneumocytoma, pregnancy and breast cancer, primary central nervous system lymphoma, primary peritoneal cancer, prostate cancer, rectal cancer, renal cell carcinomas, retinoblastoma, rhabdomyosarcoma, salivary gland carcinoma, sarcoma, Sézary syndrome, skin cancer, small cell lung cancer, small bowel cancer, soft tissue sarcoma, squamous cell carcinoma, squamous neck cancer, gastric cancer, T-cell lymphoma, testicular cancer, hypopharyngeal cancer, thymoma and thymic carcinoma, thyroid cancer, transitional cell carcinoma of the renal pelvis and ureter, cancer of unknown primary, urinary tract cancer, uterine cancer, uterine sarcoma, vaginal cancer, vulval cancer, and Wilms tumor.

In some embodiments, the hematological cancer (e.g., hematological cancer associated with RET) is selected from leukemia, lymphoma (non-Hodgkin's lymphoma), Hodgkin's disease (also known as Hodgkin's lymphoma), and myeloma, for example, acute lymphocytic leukemia (ALL), acute myelogenous leukemia (AML), acute promyelocytic leukemia (APL), chronic lymphocytic leukemia (CLL), chronic myelogenous leukemia (CML), chronic myelomonocytic leukemia (CMML), chronic neutrophilic leukemia (CNL), acute undifferentiated leukemia (ALL), anaplastic large cell lymphoma (ALCL), prolymphocytic leukemia (PML), juvenile myelomonocytic leukemia (JMML), adult T-cell ALL, AML with trilineage myelodysplasia (AML/TMDS), mixed lineage leukemia (MLL), myelodysplastic syndrome (MDS), myeloproliferative disease (MPD), and multiple myeloma (MM). Other examples of the hematological cancer include myeloproliferative disease (MPD), such as polycythemia vera (PV), primary essential thrombocytopenia (ET), and idiopathic primary myelofibrosis (IMF/IPF/PMF). In an embodiment, the hematologic cancer (e.g., a hematologic cancer associated with RET) is AML or CMML.

In some embodiments, the cancer (e.g., a cancer associated with RET) is a solid tumor. Examples of solid tumors (e.g., a solid tumor associated with RET) include, for example, thyroid cancer (e.g., papillary thyroid cancer, medullary thyroid cancer), lung cancer (e.g., lung adenocarcinoma, small cell lung cancer), pancreatic cancer, pancreatic ductal carcinoma, breast cancer, colon cancer, colorectal cancer, prostate cancer, renal cell carcinoma, head and neck tumor, neuroblastoma, and melanoma. See, for example, Nature Reviews Cancer, 2014, 14, 173-186.

In some embodiments, the cancer is selected from lung cancer papillary thyroid cancer, medullary thyroid cancer, differentiated thyroid cancer, recurrent thyroid cancer, refractory differentiated thyroid cancer, multiple endocrine neoplasia type 2A or 2B (MEN2A or MEN2B), pheochromocytoma, parathyroid hyperplasia, breast cancer, colorectal cancer, papillary renal cell carcinoma, gangliocytomatosis of the gastrointestinal mucosa, and cervical cancer.

In some embodiments, the patient is a human.

The compounds of formula I and the pharmaceutically acceptable salt thereof may also be used for treating a cancer associated with RET.

The present disclosure further provides a method for treating an irritable bowel syndrome (IBS) in a patient in need, comprising: (a) determining whether the IBS is associated with the dysregulation of: RET gene, RET kinase, or the expression, activity, or level of either; (b) if it is determined that the IBS is associated with the dysregulation of: RET gene, RET kinase, or the expression, activity, or level of either, administering to the patient a therapeutically effective amount of at least one of the compound of general formula I and the pharmaceutically acceptable salt thereof, or the pharmaceutical composition thereof.

The present disclosure further provides a pharmaceutical combination for treating irritable bowel syndrome (IBS) in a patient in need, comprising: administering (a) at least one of the compound represented by formula I, and the stereoisomer, the racemate, the tautomer, the isotopically labeled compound, the nitrogen oxide and the pharmaceutically acceptable salt thereof, (b) an additional therapeutic agent, and (c) optionally at least one pharmaceutically acceptable carrier, for concurrent, separate, or sequential use in treating IBS, wherein the amount of the at least one of the compound represented by formula I, and the stereoisomer, the racemate, the tautomer, the isotopically labeled compound, the nitrogen oxide and the pharmaceutically acceptable salt thereof, and the amount of the additional therapeutic agent are jointly effective in treating IBS. The present disclosure further provides a pharmaceutical composition comprising the combination. The present disclosure further provides use of the combination in preparing a medicament for treating IBS. The present disclosure further provides a commercial package or product comprising the combination as combined formulations for concurrent, separate, or sequential use; and relates to a method for treating IBS in a patient in need.

When used as a medicament, the compound of the present disclosure may be administered in the form of a pharmaceutical composition. Those compositions may be prepared in a manner well known in the pharmaceutical arts and may be administered by a variety of routes depending on whether local or systemic treatment is desired and the area to be treated. The pharmaceutical composition can be administered topically (e.g., by transdermal, dermal, ocular and mucosal routes, including intranasal, vaginal and rectal administration routes), pulmonarily (e.g., by inhalation or insufflation of powders or aerosols, including by nebulizer; by intratracheal and intranasal routes), orally or parenterally. The parenteral administration includes intravenous, intra-arterial, subcutaneous, intraperitoneal or intramuscular injection or infusion; or intracranial (e.g., intrathecal or intracerebroventricular) administration. The pharmaceutical composition may be administered parenterally in a single bolus form, or may be administered by, for example, continuous infusion pump. The pharmaceutical composition and formulation administered topically may include a transdermal patch, an ointment, a lotion, a cream, a gel, a drop, a suppository, a spray, a liquid and a powder. Conventional pharmaceutical carriers, water, powders or oily bases, thickeners, and the like may be necessary or desirable. Coated condoms, gloves, and the like may also be useful.

In preparing the composition of the present disclosure, the active ingredient is typically mixed with an excipient, diluted with an excipient or enclosed within such a carrier as capsules, sachets, paper, or other container forms. When used as a diluent, the excipient may be a solid, semi-solid or liquid substance that serves as a vehicle, a carrier, or a medium for the active ingredient. Thus, the composition may be in the following forms: tablets, pills, powders, lozenges, sachets, cachets, elixirs, suspensions, emulsions, solution formulations, syrups, aerosols (solid or dissolved in a liquid vehicle); ointments, soft and hard gelatin capsules, suppositories, sterile solutions for injection, and sterile packaged powders containing, for example, up to 10% by weight of active compound.

Certain examples of suitable excipients include lactose, glucose, sucrose, sorbitol, mannitol, starch, acacia, calcium phosphate, alginate, tragacanth, gelatin, calcium silicate, microcrystalline cellulose, polyvinylpyrrolidone, cellulose, water, syrup, and methylcellulose. The formulation may further contain a lubricant such as talc, magnesium stearate, and mineral oil; a wetting agent; an emulsifier and a suspending agent; a preservative such as methyl benzoate and hydroxypropyl benzoate; a sweetening agent and a flavoring agent. The composition of the present disclosure may be

formulated by the methods known in the art so as to provide immediate, sustained, or delayed release of the active ingredient after administration to the patient.

The composition may be formulated in a unit dosage form. Each dose contains about 5-1000 mg, more typically about 100-500 mg, of the active ingredient. The term "unit dosage form" refers to physically discrete single dosage units suitable for use in human patients and other mammals, each unit containing a predetermined amount of active substance mixed with a suitable pharmaceutical excipient that can produce the desired therapeutic effect by calculation.

The active compound may be effective in a wide range of doses and is generally administered in a pharmaceutically effective amount. However, it will be appreciated that the amount of the compound actually administered is usually determined by a physician, in the light of the relevant circumstances, including the disorder to be treated, the selected route of administration, the compound actually administered; the age, weight, and response of an individual patient; the severity of patient's symptoms and the like.

In preparing solid compositions such as tablets, the main active ingredient is mixed with pharmaceutical excipients to form a solid preformulation composition of a homogeneous mixture comprising the compound of the present disclosure. When those preformulation compositions are referred to be homogeneous, it is meant that the active ingredient is generally distributed evenly throughout the composition so that the composition may be readily divided into equally effective unit dosage forms such as tablets, pills and capsules. The solid preformulation is then divided into unit dosage forms of the above type containing, for example, about 0.1 mg to 1000 mg of the active ingredient of the present disclosure.

The tablets or pills of the present disclosure may be coated or compounded to obtain a dosage form affording the advantage of long-acting effect. For example, the tablets or pills contain an inner dosage component and an outer dosage component, the latter being in the form of an envelope of the former. The two components may be separated by an enteric-coated layer which serves to prevent the disintegration in the stomach to allow the inner component to pass through the duodenum entirely or to be delayed in release. A variety of substances may be used for such enteric-coated layers or coatings, including various polymeric acids and mixtures of polymeric acids and such substances as shellac, cetyl alcohol and cellulose acetate.

Liquid forms for oral administration or injection administration in which the compound and composition of the present disclosure may be incorporated include aqueous solutions, suitable flavoring syrups, aqueous or oil suspensions; and emulsions flavored with edible oils such as cottonseed oil, sesame oil, coconut oil or peanut oil; as well as elixirs and similar pharmaceutical vehicles.

Compositions for inhalation or insufflation include solution formulations and suspensions in pharmaceutically acceptable aqueous or organic solvents, or mixtures thereof, and powders. The liquid or solid compositions may contain suitable pharmaceutically acceptable excipients as described above. In certain embodiments, the composition is administered by the oral or intranasal or respiratory route for local or systemic effect. The composition may be nebulized by using inert gases. The nebulized solution may be inhaled directly from the nebulizing device or the nebulizing device may be attached to a face mask, tent or an intermittent positive pressure ventilator. The solution, suspension, or powder compositions may be administered orally, or nasally by means of a device which delivers the formulation in a suitable manner.

The amount of the compound or composition administered to a patient is not fixed and is dependent on the drug administered, the purpose of the administration such as prevention or treatment; the condition of the patient, the route of administration, etc. In therapeutic applications, the composition may be administered to a patient suffering from a disease in an amount sufficient to cure or at least partially arrest the symptoms of the disease and its complications. The effective dosage should be determined based on the state of the disease being treated and the judgment of the attending clinician that depends on factors such as the severity of the disease, the age, weight and general condition of the patient, and the like.

The composition administered to the patient may be in the form of the pharmaceutical composition as described above. These compositions can be sterilized by using conventional sterilization techniques or by filtration. The aqueous solutions may be packaged for use as is, or lyophilized, or the lyophilized formulation is mixed with a sterile aqueous carrier prior to administration. The compound formulation usually has a pH of 3-11, more preferably 5-9, and most preferably 7-8. It will be appreciated that the use of certain excipients, carriers, or stabilizers as described above may result in the formation of a pharmaceutical salt.

The therapeutic dosage of the compound of the present disclosure can be determined, for example, according to: the specific use of the treatment, the route of administration of the compound, the health and condition of the patient, and the judgment of the prescriber. The proportion or concentration of the compound of the present disclosure in the pharmaceutical composition may vary depending on a variety of factors including the dosage, chemical properties (e.g., hydrophobicity), and the route of administration. For example, the compound of the present disclosure may be provided for parenteral administration by a physiological buffered aqueous solution containing about 0.1-10% w/v of the compound. Certain typical dosage ranges from about 1 μg/kg body weight/day to about 1 g/kg body weight/day. In certain embodiments, the dosage ranges from about 0.01 mg/kg body weight/day to about 100 mg/kg body weight/day. The dosage is likely dependent on such variables as the type and degree of progression of the disease or disorder, the general health

condition of a particular patient, the relative biological potency of the compound selected, the excipient formulation, and its route of administration. Effective dosage can be extrapolated from dose-response curves derived from *in vitro* or animal model test systems.

**Definitions and Description**

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the present specification.

Unless otherwise stated, a numerical range set forth in the description and claims shall be construed as at least including each specific integer within the range. For example, the numerical range "1-40" shall be construed as including each integer value in the numerical range "1-10", i.e., 1, 2, 3, 4, 5, 6, 7, 8, 9, and 10, and each integer value in the numerical range "11-40", i.e., 11, 12, 13, 14, 15,..., 35, 36, 37, 38, 39, and 40. It should be understood that when one, two, or more are used to describe a substituent herein, "more" shall refer to an integer $\geq$ 3, such as 3, 4, 5, 6, 7, 8, 9, or 10. Moreover, when certain numerical ranges are defined as "a number", it shall be construed as including both endpoints of the range, each integer within the range, and each decimal within the range. For example, "a number of 0-10" shall be construed as including not only each of integers 0, 1, 2, 3, 4, 5, 6, 7, 8, 9, and 10, but also at least the sums of each integer and 0.1, 0.2, 0.3, 0.4, 0.5, 0.6, 0.7, 0.8, or 0.9.

The term "halogen" refers to fluorine, chlorine, bromine, and iodine.

The term "$C_{1-40}$ alkyl" preferably refers to a linear or branched saturated monovalent hydrocarbyl group having 1-40 carbon atoms. For example, "$C_{1-6}$ alkyl" refers to a linear or branched alkyl group having 1, 2, 3, 4, 5, or 6 carbon atoms. The alkyl is, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof.

The term "$C_{2-40}$ alkenyl" preferably refers to a linear or branched monovalent hydrocarbyl comprising one or more double bonds and having 2-40 carbon atoms, and is preferably "$C_{2-6}$ alkenyl". "$C_{2-6}$ alkenyl" preferably refers to a linear or branched monovalent hydrocarbyl group comprising one or more double bonds and having 2, 3, 4, 5, or 6 carbon atoms, in particular 2 or 3 carbon atoms ("$C_{2-3}$alkenyl"); it should be understood that in the case where the alkenyl comprises more than one double bond, the double bonds can be separated from one another or conjugated. The alkenyl is, for example, vinyl, allyl, (E)-2-methylvinyl, (Z)-2-methylvinyl, (E)-but-2-enyl, (Z)-but-2-enyl, (*E*)-but-1-enyl, (*Z*)-but-1-enyl, pent-4-enyl, (E)-pent-3-enyl, (Z)-pent-3-enyl, (E)-pent-2-enyl, (Z)-pent-2-enyl, (*E*)-pent-1-enyl, (*Z*)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, or 1-isopropylvinyl.

The term "$C_2$-$_{40}$ alkynyl" refers to a linear or branched monovalent hydrocarbyl group comprising one or more triple bonds and having 2-40 carbon atoms, and is preferably "$C_2$-$C_6$ alkynyl". The term "$C_2$-$C_6$ alkynyl" refers to a linear or branched monovalent hydrocarbyl group comprising one or more triple bonds and having 2, 3, 4, 5, or 6 carbon atoms, in particular 2 or 3 carbon atoms ("$C_2$-$C_3$ alkynyl"). The $C_2$-$C_6$ alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or 3,3-dimethylbut-1-ynyl. In particular, the alkynyl is ethynyl, prop-1-ynyl, or prop-2-ynyl.

The term "$C_{3-40}$ cycloalkyl" refers to a saturated monovalent monocyclic or bicyclic hydrocarbon ring or bridged cycloalkane having 3-40 carbon atoms, preferably "$C_{3-10}$ cycloalkyl". The term "$C_{3-10}$ cycloalkyl" refers to a saturated monovalent monocyclic or bicyclic hydrocarbon ring or bridged cycloalkane having 3, 4, 5, 6, 7, 8, 9, or 10 carbon atoms. The $C_{3-10}$ cycloalkyl may be a monocyclic hydrocarbyl such as cyclopropyl, cyclobutyl, cyclopentyl, cyclohexyl, cycloheptyl, cyclooctyl, cyclononyl, or cyclodecyl, or may be a bicyclic hydrocarbyl such as a decahydronaphthalene ring.

The term "3- to 20-membered heterocyclyl" refers to a saturated monovalent monocyclic or bicyclic hydrocarbon ring or bridged cycloalkane, which is a non-aromatic cyclic group with the total number of ring atoms of 3-20 (such as 3, 4, 5, 6, 7, 8, 9, and 10) comprising 1-5 heteroatoms independently selected from N, O, and S, preferably a "3- to 10-membered heterocyclyl". The term "3- to 10-membered heterocyclyl" refers to a saturated monovalent monocyclic or bicyclic hydrocarbon ring or bridged cycloalkane, which comprises 1-5, preferably 1-3 heteroatoms independently selected from N, O, and S, for example, 1, 2, or 3 heteroatoms independently selected from N, O, and S. The heterocyclyl may be connected to the rest of the molecule through any of the carbon atoms or the nitrogen atom (if present). In particular, the heterocyclyl may include, but is not limited to: 4-membered rings such as azetidinyl and oxetanyl (e.g., azetidin-1-yl); 5-membered rings such as tetrahydrofuranyl, dioxolyl, pyrrolidinyl, imidazolidinyl, pyrazolidinyl and pyrrolinyl; 6-membered rings such as tetrahydropyranyl, piperidyl, morpholinyl, dithianyl, thiomorpholinyl, piperazinyl and trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[*c*]pyrrol-2(1*H*)-yl ring, or a 5,6-membered bicyclic ring such as a hexahydropyrrolo[1,2-*a*]pyrazin-2(1*H*)-yl ring. The ring containing nitrogen atoms may be partially unsaturated, i.e., it may comprise one or more double bonds, for example, but not limited to, 2,5-dihydro-1*H*-pyrrolyl, 4H-[1,3,4]thiadiazinyl, 4,5-dihydrooxazolyl or 4H-[1,4]thiazinyl, or it may be benzo-fused, for example, but not limited to, dihydroisoquinolyl. According to the present disclosure, the heterocyclyl is non-aromatic. When the 3- to 20-membered heterocyclyl is connected to another group to form the compound of the present disclosure, the group may be connected to the carbon atom on the 3- to 20-membered heterocyclyl, or may be connected to the heteroatom on the 3- to 20-membered heterocyclyl. For example, when the 3- to 20-membered heterocyclyl is selected from piperazinyl, the group may be connected to the nitrogen atom on the piperazinyl. Alternatively, when the 3- to 20-membered heterocyclyl is selected from piperidyl, the group may be connected to the nitrogen atom on the piperidyl ring or the carbon atom in the para position.

The term "$C_{6-20}$ aryl" preferably refers to an aromatic or partially aromatic monovalent monocyclic, bicyclic, or tricyclic hydrocarbon ring having 6-20 carbon atoms, preferably "$C_{6-14}$ aryl". The term "$C_{6-14}$ aryl" preferably refers to an aromatic or partially aromatic monovalent monocyclic, bicyclic, or tricyclic hydrocarbon ring having 6, 7, 8, 9, 10, 11, 12, 13, or 14 carbon atoms ("$C_{6-14}$ aryl"), in particular a ring having 6 carbon atoms ("$C_6$ aryl"), such as phenyl or biphenyl, a ring having 9 carbon atoms ("$C_9$ aryl"), such as indanyl or indenyl, a ring having 10 carbon atoms ("$C_{10}$ aryl"), such as tetrahydronaphthyl, dihydronaphthyl or naphthyl, a ring having 13 carbon atoms ("$C_{13}$ aryl"), such as fluorenyl, or a ring having 14 carbon atoms ("$C_{14}$ aryl"), such as anthryl. When the $C_{6-20}$ aryl is substituted, it may be monosubstituted or polysubstituted. In addition, the substitution site is not limited, and may be, for example, ortho-substitution, para-substitution, or meta-substitution.

The term "5- to 20-membered heteroaryl" refers to a monovalent monocyclic, bicyclic, or tricyclic aromatic ring system which has 5-20 ring atoms and comprises 1-5 heteroatoms independently selected from N, O, and S, such as "5- to 14-membered heteroaryl". The term "5- to 14-membered heteroaryl" refers to a monovalent monocyclic, bicyclic, or tricyclic aromatic ring system that has 5, 6, 7, 8, 9, 10, 11, 12, 13, or 14 ring atoms, in particular 5, 6, 9, or 10 carbon atoms, comprises 1-5, preferably 1-3 heteroatoms independently selected from N, O, and S, and may be benzo-fused in each case. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4*H*-pyrazolyl and the like and benzo derivatives thereof, such as benzofuranyl, benzothienyl, benzoxazolyl, benzoisoxazolyl, benzimidazolyl, benzotriazolyl, indazolyl, indolyl, and isoindolyl; or pyridinyl, pyridazinyl, pyrimidinyl, pyrazinyl, triazinyl and the like and benzo derivatives thereof, such as quinolyl, quinazolinyl, and isoquinolyl; or azocinyl, indolizinyl, purinyl and the like and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like. When the 5- to 20-membered heteroaryl is connected to another group to form the compound of the present disclosure, the group may be connected to the carbon atom on the 5- to 20-membered heteroaryl ring, or may be connected to the heteroatom on the 5- to 20-membered heteroaryl ring. When the 5- to 20-membered heteroaryl is substituted, it may be monosubstituted or polysubstituted. In addition, the substitution site is not limited. For example, hydrogen connected to the carbon atom on the heteroaryl ring may be substituted, or hydrogen connected to the heteroatom on the heteroaryl ring may be substituted.

Unless otherwise stated, the heterocyclyl, heteroaryl, or heteroarylene includes all possible isomeric forms thereof, e.g., positional isomers thereof. Thus, for some illustrative non-limiting examples, forms that involving substitutions at or bonding to other groups at one, two, or more of positions 1, 2, 3, 4, 5, 6, 7, 8, 9, 10, 11, 12, and the like (if present) are included, including pyridin-2-yl, pyridinylene-2-yl, pyridin-3-yl, pyridinylene-3-yl, pyridin-4-yl and pyridinylene-4-yl; thienyl or thienylene, including thien-2-yl, thien-2-ylene, thien-3-yl, and thien-3-ylene; pyrazol-1-yl, pyrazol-3-yl, pyrazol-4-yl and pyrazol-5-yl.

The term "oxo" refers to that the carbon atom, nitrogen atom, or sulfur atom in the substituent is substituted with an oxy group formed after oxidation (=O).

Unless otherwise stated, the definitions of terms used herein are also applicable to groups comprising the terms. For example, the definition of $C_{1-6}$ alkyl is also applicable to $C_{1-6}$ alkyloxy, $-N(C_{1-6}$ alkyl$)_2$, $-NHC_{1-6}$ alkyl, $-S(O)_2-C_{1-6}$ alkyl and the like.

It will be appreciated by those skilled in the art that the compound represented by formula I may be present in the form of various pharmaceutically acceptable salts. If such compounds have basic centers, they can form acid addition

salts; if such compounds have acidic centers, they can form base addition salts; if these compounds comprise both acidic centers (e.g., carboxyl) and basic centers (e.g., amino), they can also form internal salts.

The compound disclosed herein may be present in the form of a solvate (e.g., hydrate), and the compound disclosed herein contains a polar solvent as a structural element of the crystal lattice of the compound, particularly, for example, water, methanol, or ethanol. The amount of the polar solvent, especially water, may be present in a stoichiometric or non-stoichiometric ratio. According to the molecular structure, the compound disclosed herein may be chiral and may therefore be present in various enantiomeric forms. These compounds may therefore be present in a racemic or optically active form. The compounds disclosed herein or intermediates thereof may be separated into enantiomers by chemical or physical methods well known to those skilled in the art, or used in such form for synthesis. In the case of racemic amines, diastereoisomers are prepared from mixtures by reaction with optically active resolving agents. Examples of suitable resolving agents are optically active acids such as *R*- or S-tartaric acid, diacetyltartaric acid, dibenzoyltartaric acid, mandelic acid, malic acid, lactic acid, suitable *N*-protected amino acids (e.g., *N*-benzoylproline or *N*-benzenesulfonylproline), or various optically active camphorsulfonic acids. Enantiomeric resolution by chromatography can be advantageously conducted with the aid of optically active resolving agents, such as dinitrobenzoylphenylglycine, cellulose triacetate or other carbohydrate derivatives or chirally derivatized methacrylate polymers immobilized on silica gel. Suitable eluents for this purpose are mixtures of solvents containing water or alcohol, for example, hexane/isopropanol/acetonitrile.

The term "tautomer" refers to functional isomers resulting from the rapid movement of an atom in a molecule between two positions. The compounds disclosed herein may exhibit the tautomerism. Tautomeric compounds may be present in two or more interconvertible forms. Prototropic tautomers result from the migration of a covalently bonded hydrogen atom between two atoms.

Tautomers are generally present in an equilibrium form. Efforts to separate a single tautomer usually lead to a mixture, the physicochemical properties of which are consistent with the mixture of the compound. The position of the equilibrium depends on the chemical properties of the molecule. For example, in many aliphatic aldehydes and ketones such as acetaldehyde, the keto form predominates; whereas in phenol, the enol form predominates. In the present disclosure, all tautomeric forms of the compound are included.

The corresponding stable isomers can be separated according to known methods, such as extraction, filtration, or column chromatography.

The term "patient" refers to any animal including mammals, preferably mice, rats, other rodents, rabbits, dogs, cats, pigs, cattle, sheep, horses, or primates, and most preferably humans.

The phrase "therapeutically effective amount" used herein refers to the amount of the active compound or drug that causes a biological or medical response that researchers, veterinarians, physicians, or other clinicians are looking for in tissues, systems, animals, individuals, or humans, including one or more of the following effects: (1) disease prevention: for example, the prevention of a disease, disorder, or condition in an individual who is susceptible to the disease, disorder, or condition but has not yet experienced or exhibited the pathology or symptoms of the disease; (2) disease inhibition: for example, the inhibition of a disease, disorder, or condition in an individual who is experiencing or exhibiting the pathology or symptoms of the disease, disorder, or condition.(i.e., the prevention of the further development of the pathology and/or symptoms); and (3) disease alleviation: for example, the alleviation of a disease, disorder, or condition in an individual who is experiencing or exhibiting the pathology or symptoms of the disease, disorder, or condition (i.e., the reverse of the pathology and/or symptoms).

## Beneficial Effects

The compounds disclosed herein can be used as an RET inhibitor with high selectivity or inhibitory effect. For example, the compound has excellent inhibitory effect on the RET gatekeeper residue mutant RET V804M, RET solvent-front residue mutant G810R, other clinically relevant RET mutants, and RET-wt. In addition, the preferred compounds disclosed herein can effectively inhibit the growth of TT cell lines derived from thyroid cancer and Ba/F3 cells derived from various RET mutants, block the RET autophosphorylation and downstream pathways in cells, and significantly induce the death of TT cells. In addition, the preferred compounds disclosed herein also have excellent pharmacokinetics, and can be administered to patients in smaller doses as active ingredients, thereby reducing the cost of treatment for patients.

## DETAILED DESCRIPTION

The technical solutions of the present disclosure will be further illustrated in detail with reference to the following specific examples. It should be understood that the following examples are merely exemplary illustrations and explanations of the present disclosure, and should not be construed as limiting the protection scope of the present disclosure. All techniques implemented based on the content of the present disclosure described above are included within the

protection scope of the present disclosure.

Unless otherwise stated, the starting materials and reagents used in the following examples are all commercially available products or can be prepared using known methods.

**Example 1: Preparation of compound 1**

Step A: *N*-(1-(5-(6-ethoxy-1*H*-pyrazolo[3',4':3,4]pyrazolo[1,5-*a*]pyridin-4-yl)pyridin-2-yl)-4-methylpiperidi n-4-yl)-5-fluoro-2-methylbenzamide

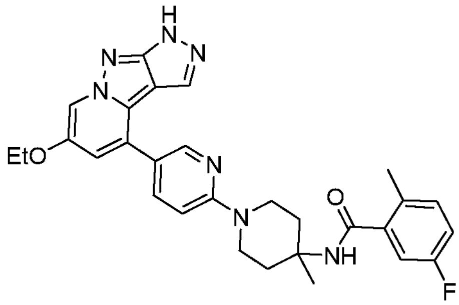

1-(5-(6-ethoxy-1*H*-pyrazolo[3',4':3,4]pyrazolo[1,5-*a*]pyridin-4-yl)pyridin-2-yl)-4-methylpiperidine-4-amino hydrochloride (100 mg, 0.2 mmol), 5-fluoro-2-methylbenzoic acid (37 mg, 0.24 mmol), DIEA (129 mg, 1.0 mmol), HATU (76 mg, 0.2 mmol) and 2 mL of DMF were added to a reaction flask. The mixture was reacted at 25 °C for 12 h. The reaction was quenched by adding water and stirring. The reaction liquid was extracted with ethyl acetate. The organic phase was dried over anhydrous sodium sulfate, concentrated under reduced pressure, and separated by column chromatography to give a product (8.5 mg). m/z = 528.2$[M+1]^+$. $^1H$ NMR (400 MHz, DMSO-$d_6$) $\delta$ 12.65 (brs, 1H), 8.58 (d, *J* = 2.0Hz, 1H), 8.51 (d, *J* = 2.0Hz, 1H), 8.01-8.06 (m, 2H), 7.60 (s, 1H), 7.26-7.29 (m, 2H), 7.10-7.17 (m, 3H), 4.15 (q, *J* = 6.8Hz, 2H), 4.05-4.08 (m, 2H), 3.34-3.68 (m, 2H), 2.32-2.37 (m, 5H), 1.60-1.65 (m, 2H), 1.46 (s, 3H), 1.38-1.45 (m, 3H).

**Example 2: Preparation of compound 3**

Step A: 1-amino-3-bromo-5-methoxypyridin-1-ium 2,4,6-trimethylbenzenesulfonate

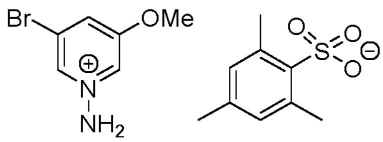

3-bromo-5-methoxypyridine (6.0 g, 32.0 mmol) was added to a solution of 2-[(aminooxy)sulfonyl]-1,3,5-trimethylbenzene (6.8 g, 31.7 mmol) in dichloromethane (50 mL) at 0 °C. The mixture was stirred at 0 °C for 3 h to precipitate a large quantity of white solids. After the reaction was completed, diethyl ether (50 mL) was added at 0 °C, and the mixture was stirred for 10 min. The reaction liquid was filtered under reduced pressure, rinsed with diethyl ether, and dried in vacuum to give a product (15 g), which was directly used in the next step without further purification, m/z = 204 $[M+1]^+$.

Step B: 4-bromo-2-fluoro-6-methoxypyrazolo[1,5-*a*]pyridine

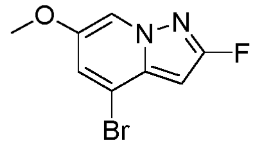

Potassium carbonate (1.4 g, 10.0 mmol) was added to a solution of 2,4,6-trimethylbenzenesulfonic acid 1-amino-3-bromo-5-methoxypyridin-1-ium (1.0 g, 2.3 mmol) in DMF (30 mL) at room temperature. The reaction system was cooled to 0 °C, and 2,2-difluorovinyl p-toluenesulfonate (0.5 g, 2.3 mmol) was added in batches. The mixture was warmed to room temperature and stirred for 1 h, and then stirred at 90 °C for 1 h. After the reaction was completed and the reaction liquid was cooled to room temperature, the reaction was quenched by adding water. The reaction liquid was extracted with ethyl acetate. The organic phases were combined, washed with water, concentrated under reduced pressure, and separated by column chromatography to give a product (80 mg), m/z = 245 $[M+1]^+$, $^1$HNMR (400MHz, $CDCl_3$) δ 8.07 (s, 1H), 6.61 (s, 1H), 6.18 (d, 1H), 3.85 (s, 3H).

Step C: 4-bromo-2-fluoro-6-methoxypyrazolo[1,5-*a*]pyridine-3-carbaldehyde

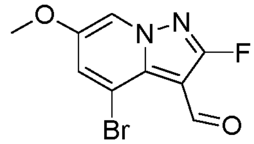

Phosphorus oxychloride (1.0 g, 6.5 mmol) was added dropwise to a solution of 4-bromo-2-fluoro-6-methoxypyrazolo[1,5-*a*]pyridine (294 mg, 1.2 mmol) in DMF (10 mL) at 0 °C.

After the dropwise addition, the mixture was naturally warmed to room temperature and reacted overnight. The reaction liquid was poured into ice water (100 mL), and the resulting mixture was adjusted to pH 7 with a 2 N NaOH solution and extracted with ethyl acetate. The organic phases were combined, concentrated under reduced pressure, and separated by column chromatography to give a product (240 mg). M/z = 273 $[M+1]^+$. $^1$HNMR (400MHz, DMSO-$d_6$) δ 10.51 (s, 1H), 8.72 (d, 1H), 8.04 (d, 1H), 3.87 (s, 3H).

Step D: 4-bromo-6-methoxy-1*H*-pyrazolo[3',4':3,4]pyrazolo[1,5-*a*]pyridine

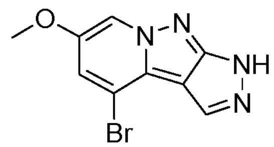

Hydrazine hydrate (2.0 mL) was added to a solution of 4-bromo-2-fluoro-6-methoxypyrazolo[1,5-*a*]pyridine-3-carbaldehyde (273 mg, 1 mmol) in DMF (10.0 mL). The mixture was heated to 100 °C and reacted for 12 h. Water was added, and the resulting mixture was extracted with ethyl acetate. The organic phases were combined, washed with water, dried over anhydrous sodium sulfate, filtered, concentrated under reduced pressure, and separated by column chromatography to give a product, m/z = 267 $[M+1]^+$, $^1$HNMR (400MHz, DMSO-$d_6$) δ 12.82 (s, 1H), 8.67 (d, 1H), 7.92 (s, 1H), 7.62 (d, 1H), 3.89 (s, 3H).

Step E: 4-bromo-6-hydroxy-1*H*-pyrazolo[3',4':3,4]pyrazolo[1,5-*a*]pyridine

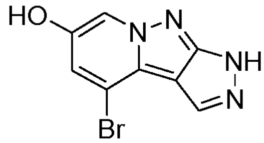

4-bromo-6-methoxy-1*H*-pyrazolo[3',4':3,4]pyrazolo[1,5-*a*]pyridine (267 mg, 1 mmol), DCE (20 mL), and aluminum chloride (446 mg, 3.36 mmol) were added to a 100 mL single-necked flask. Under nitrogen atmosphere, the mixture was heated and stirred at 80 °C, with the color gradually turning dark brown, and reacted for 3 h. After the reaction was completed as detected by TLC, the reaction was quenched by adding water and stirring. The reaction liquid was extracted with ethyl acetate. The organic phase was dried over anhydrous sodium sulfate, concentrated under reduced pressure, and separated by column chromatography to give a product, m/z = 253 $[M+1]^+$, $^1$HNMR (400 MHz, DMSO-$d_6$) $\delta$ 12.69 (brs, 1H), 10.23 (s, 1H), 8.33 (d, 1H), 7.88 (s, 1H), 7.42 (d, 1H).

Step F: 4-bromo-6-ethoxy-1*H*-pyrazolo[3',4':3,4]pyrazolo[1,5-*a*]pyridine

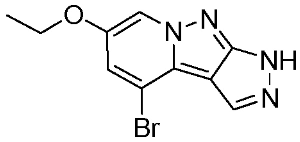

2-iodoethane (47 mg, 0.3 mmol) and potassium carbonate (83 mg, 0.6 mmol) were added to a solution of 4-bromo-6-hydroxy-1*H*-pyrazolo[3',4':3,4]pyrazolo[1,5-*a*]pyridine (76 mg, 0.3 mmol) in DMF (10.0 mL). The mixture was heated to 60 °C and reacted for 12 h. Water was added, and the resulting mixture was extracted with ethyl acetate. The organic phases were combined, washed with water, dried over anhydrous sodium sulfate, filtered, concentrated under reduced pressure, and separated by column chromatography to give a product, m/z = 281 $[M+1]^+$, $^1$HNMR (400MHz, DMSO-$d_6$) $\delta$ 12.79 (s, 1H), 8.64 (d, 1H), 7.91 (s, 1H), 7.59 (d, 1H), 4.17 (t, 2H), 1.38 (t, 3H).

G: 6-ethoxy-4-(6-fluoropyridin-3-yl)-1*H*-pyrazolo[3',4':3,4]pyrazolo[1,5-*a*]pyridine

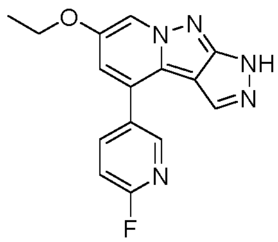

6-fluoropyridine-3-boronic acid (0.6 g, 4.3 mmol), sodium carbonate (1.5 g, 14 mmol), 1,4-dioxane (20 mL), water (3 mL), and tetrakis(triphenylphosphine)palladium(0) (0.12 g, 0.1 mmol) were added to 4-bromo-6-ethoxy-1*H*-pyrazolo[3',4':3,4]pyrazolo[1,5-*a*]pyridine (1.0 g, 3.6 mmol). The mixture was purged with nitrogen and reacted at 90 °C for 12 h. The reaction was quenched by adding water and stirring. The reaction liquid was extracted with ethyl acetate. The organic phase was dried over anhydrous sodium sulfate, concentrated under reduced pressure, and separated by column chromatography to give a product, m/z = 298 $[M+1]^+$, $^1$H NMR (400 MHz, DMSO-d6): 12.73 (s, 1H), 8.61-8.76 (m, 2H), 8.28 (s, 1H), 8.48 (s, 1H), 7.63 (s, 1H), 4.16 (q, 2H), 1.39 (t, *J* = 6.4Hz, 3H).

Step H: tert-butyl (1-(5-(6-ethoxy-1*H*-pyrazolo[3',4':3,4]pyrazolo[1,5-*a*]pyridin-4-yl)pyridin-2-yl)-4-methylpiperidin-4-yl)aminocarbonate

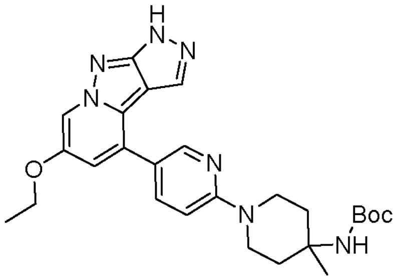

6-ethoxy-4-(6-fluoropyridin-3-yl)-1*H*-pyrazolo[3',4':3,4]pyrazolo[1,5-*a*]pyridine (500 mg, 1.7 mmol), tert-butyl (4-methylpiperidin-4-yl)carbonate (721 mg, 3.4 mmol), DIEA (650 mg, 5.1 mmol), and 10 mL of DMSO were added to a reaction flask. The mixture was reacted at 90 °C for 4 h. The reaction was quenched by adding water and stirring. The reaction liquid was extracted with ethyl acetate. The organic phase was dried over anhydrous sodium sulfate, concentrated under reduced pressure, and separated by column chromatography to give a product, m/z = 492 [M+1]$^+$.

Step I: 1-(5-(6-ethoxy-1*H*-pyrazolo[3',4':3,4]pyrazolo[1,5-*a*]pyridin-4-yl)pyridin-2-yl)-4-methylpiperidin-4 -amine hydrochloride

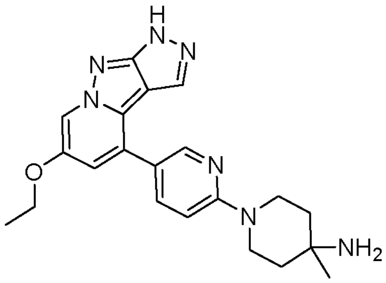

Tert-butyl

(1-(5-(6-ethoxy-1*H*-pyrazolo[3',4':3,4]pyrazolo[1,5-*a*]pyridin-4-yl)pyridin-2-yl)-4-methylpiperidin-4-yl)aminocarbonate (390 mg, 0.8 mmol) and a 5 N methanolic hydrochloric acid solution (8 mL, 40 mmol) were added to a reaction flask. The mixture was reacted at 25 °C for 2 h. The reaction liquid was concentrated under reduced pressure to give a product, m/z = 392 [M+1]$^+$.

Step J: 3-chloro-*N*-(1-(5-(6-ethoxy-1*H*-pyrazolo[3',4':3,4]pyrazolo[1,5-*a*]pyridin-4-yl)pyridin-2-yl)-4-meth ylpiperidin-4-yl)-2-picolinamide

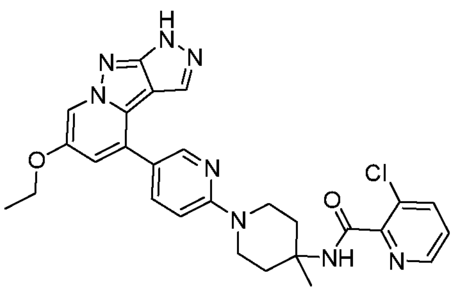

1-(5-(6-ethoxy-1*H*-pyrazolo[3',4':3,4]pyrazolo[1,5-*a*]pyridin-4-yl)pyridin-2-yl)-4-methylpiperidine-4-amine hydrochloride (100 mg, 0.2 mmol), 3-chloropyridine-2-carboxylic acid (37 mg, 0.24 mmol), DIEA (85mg, 0.66mmol), HATU (84 mg, 0.22 mmol), and 2 mL of DMF were added to a reaction flask. The mixture was reacted at 25 °C for 2 h. The reaction was quenched by adding water and stirring. The reaction liquid was extracted with ethyl acetate. The organic

phase was dried over anhydrous sodium sulfate, concentrated under reduced pressure, and separated by column chromatography to give a product, m/z = 531 $[M+1]^+$, $^1H$ NMR (400 MHz, DMSO-d6): 8.53-8.57 (m, 3H), 8.32 (s, 1H), 8.00-8.10 (m, 2H), 7.61 (s, 1H), 7.50 (dd, *J1* = 8.0Hz, *J2* = 2.4Hz, 1H), 7.28 (s,1H), 7.16 (d, *J* = 8.4Hz, 1H), 4.15-4.23 (m, 2H), 4.07-4.10 (m, 2H), 3.35-3.42 (m, 2H), 2.33-2.37 (m, 2H), 1.60-1.65 (m, 2H), 1.45-1.48 (m, 6H).

**Example 3: Preparation of compound 4**

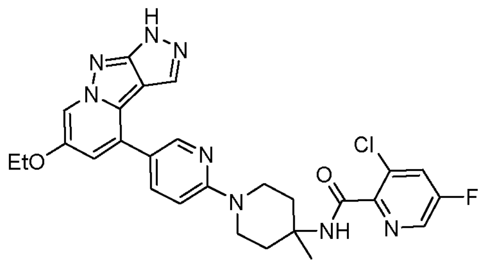

The compound was prepared by the same method as that in step J of Example 1, m/z = 549 $[M+1]^+$, $^1H$ NMR (400 MHz, DMSO-d6): 12.76 (s, 1H), 8.57-8.62 (m, 2H), 8.51 (s, 1H), 8.30 (s, 1H), 8.19 (dd,1H), 8.19 (dd, 1H), 7.59 (s, 1H), 7.11 (d, 1H), 4.15-4.22 (m, 2H), 4.06-4.10 (m, 2H), 3.33-3.36 (m, 2H), 2.30-2.33 (m, 2H), 1.58-1.65 (m, 2H), 1.43-1.47 (m, 6H).

**Example 4: Preparation of compound 8**

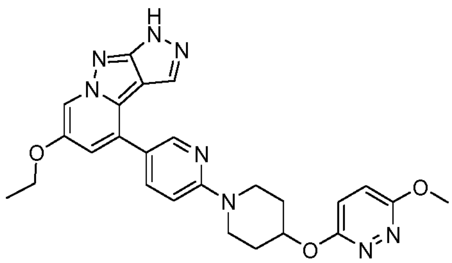

2 mL of DMSO and 0.5 mL of DIEA, 3-methoxy-6-(piperidin-4-oxy)pyridazine (400 mg, 1.3 mmol), and 6-ethoxy-4-(6-fluoropyridin-3-yl)-1*H*-pyrazolo[3',4':3,4]pyrazolo[1,5-*a*]pyridine (70 mg, 0.2 mmol) were sequentially added to a reaction flask. The mixture was reacted at 100 °C for 48 h. The reaction was quenched by adding water and stirring. The reaction liquid was extracted with ethyl acetate. The organic phase was dried over anhydrous sodium sulfate, concentrated under reduced pressure, and separated by column chromatography to give a product, m/z = 487 $[M+1]^+$.

**Example 5: Preparation of compound 11**

Step A: ethyl 4-((*tert*-butoxycarbonyl)amino)-1-(5-(6-ethoxy-1*H*-pyrazolo[3',4':3,4]pyrazolo[1,5-*a*]pyridin-4-yl)p yridin-2-yl)piperidine-4-carboxylate

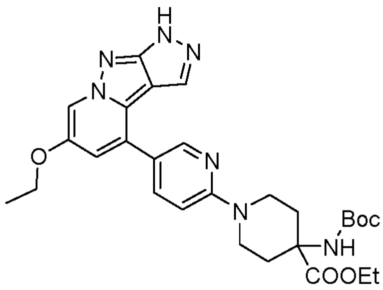

6-ethoxy-4-(6-fluoropyridin-3-yl)-1*H*-pyrazolo[3',4':3,4]pyrazolo[1,5-*a*]pyridine (2.5 g, 8.4 mmol), ethyl 4-(*tert*-butylcarbonyl)aminopiperidine-4-carboxylate (2.8 g, 9.2 mmol), DIEA (3.2 g, 25.2 mmol), and DMSO (50 mL) were added to a reaction flask. The mixture was reacted at 90 °C for 12 h. The reaction was quenched by adding water and stirring. The reaction liquid was extracted with ethyl acetate. The organic phase was dried over anhydrous sodium sulfate, concentrated under reduced pressure, and separated by column chromatography to give a product, m/z = 550 $[M+1]^+$.

Step B: ethyl 4-amino-1-(5-(6-ethoxy-1*H*-pyrazolo[3',4':3,4]pyrazolo[1,5-*a*]pyridin-4-yl)pyridin-2-yl)piperidine-4-carboxylate

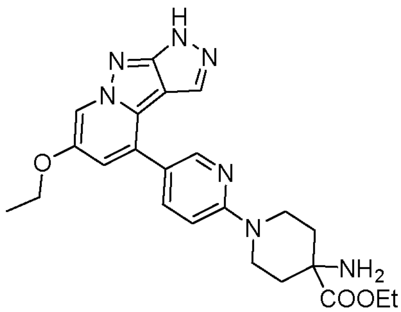

Ethyl

4-((*tert*-butylcarbonyl)amino)-1-(5-(6-ethoxy-1*H*-pyrazolo[3',4':3,4]pyrazolo[1,5-*a*]pyridin-4-yl)pyr idin-2-yl)piperidine-4-carboxylate (1 g, 1.8 mmol) was added to 10 mL of MeOH/HCl (4 M). The mixture was reacted at 25 °C for 4 h. The reaction liquid was concentrated by rotary evaporation to remove the solvent to give a product, m/z = 450 $[M+1]^+$.

Step C: ethyl 4-(2,6-difluorobenzamido)-1-(5-(6-ethoxy-1*H*-pyrazolo[3',4':3,4]pyrazolo[1,5-*a*]pyridin-4-yl)pyridi n-2-yl)piperidine-4-carboxylate

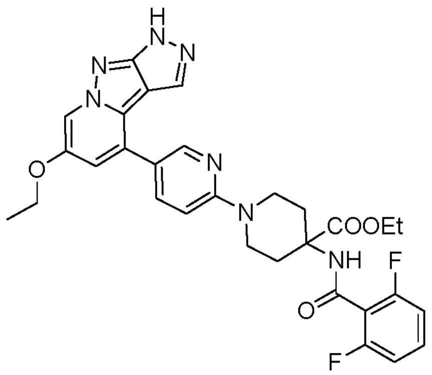

Ethyl

4-(amino)-1-(5-(6-ethoxy-1*H*-pyrazolo[3',4':3,4]pyrazolo[1,5-*a*]pyridin-4-yl)pyridin-2-yl)piperidine -4-carboxylate (120 mg, 0.26 mmol), 2,6-difluorobenzoic acid (38 mg, 0.24 mmol), DIEA (100 mg, 0.78 mmol), HATU (108 mg, 0.28 mmol), and DMF (2 mL) were added to a reaction flask. The mixture was reacted at 25 °C for 12 h. The reaction was quenched by adding water and stirring. The reaction liquid was extracted with ethyl acetate. The organic phase was dried over anhydrous sodium sulfate, concentrated under reduced pressure, and separated by column chromatography to give a product, m/z = 590 $[M+1]^+$.

Step D: *N*-(1-(5-(6-ethoxy-1*H*-pyrazolo[3',4':3,4]pyrazolo[1,5-*a*]pyridin-4-yl)pyridin-2-yl)-4-(hydroxymeth yl)piperidin-4-yl)-2,6-difluorobenzamide

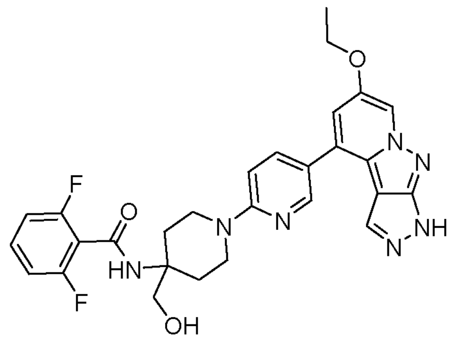

Ethyl

4-(2,6-difluorobenzamide)-1-(5-(6-ethoxy-1*H*-pyrazolo[3',4':3,4]pyrazolo[1,5-*a*]pyridin-4-yl)pyridi n-2-yl)piperidine-4-carboxylate (30 mg, 0.05mmol) and 2 mL of THF were added to a reaction flask, and $LiAlH_4$ (1.9 mg, 0.05 mmol) was added at 0 °C. The mixture was reacted for 1 h. The reaction was quenched by adding water and stirring. The reaction liquid was extracted with ethyl acetate. The organic phase was dried over anhydrous sodium sulfate, concentrated under reduced pressure, and separated by column chromatography to give a product, m/z = 548 $[M+1]^+$, $^1H$ NMR (400 MHz, DMSO-d6) $\delta$ 12.65 (s, 1H), 8.59 (d, 1H), 8.52 (d, 1H), 8.36 (s, 1H), 8.04 (dd, 1H), 7.60 (s, 1H), 7.50 (tt, 1H), 7.27 (d, 1H), 7.22-7.08 (m, 3H), 4.87 (t, 1H), 4.28 (d, 2H), 4.18 (q, 2H), 3.63 (d, 2H), 3.19 (t, 2H), 2.24 (d, 2H), 1.68 (td, 4.3 Hz, 2H), 1.41 (t, 3H).

**Example 6: Preparation of compound 12**

Step A: ethyl 4-(3-chloromethylpicolinamido)-1-(5-(6-ethoxy-1*H*-pyrazolo[3',4':3,4]pyrazolo[1,5-*a*]pyridin-4-yl) pyridin-2-yl)piperidine-4-carboxylate

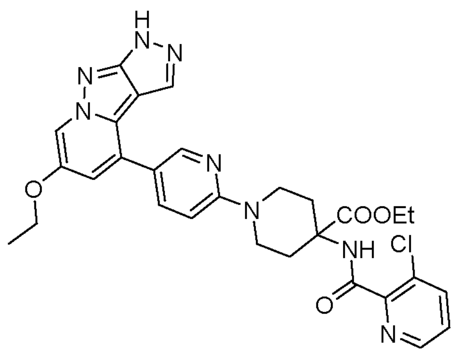

Ethyl

4-((*tert*-butylcarbonyl)amino)-1-(5-(6-ethoxy-1*H*-pyrazolo[3',4':3,4]pyrazolo[1,5-*a*]pyridin-4-yl)pyr idin-2-yl)piperidine-4-carboxylate hydrochloride (120 mg, 0.26 mmol), 3-chloropicolinic acid (37 mg, 0.24 mmol), DIEA (100 mg, 0.78 mmol), HATU (108 mg, 0.28 mmol), and DMF (2 mL) were added to a reaction flask. The mixture was reacted at 25 °C for 12 h. After the reaction was completed as monitored by LCMS, the reaction was quenched by adding water and stirring. The reaction liquid was extracted with ethyl acetate. The organic phase was dried over anhydrous sodium sulfate, concentrated under reduced pressure, and separated by column chromatography to give a product (80 mg), m/z = 589.2 $[M+1]^+$.

Step B: 3-chloro-*N*-(1-(5-(6-ethoxy-1*H*-pyrazolo[3',4':3,4]pyrazolo[1,5-*a*]pyridin-4-yl)pyridin-2-yl)-4-(hydr oxymethyl)piperidin-4-yl)picolinamide

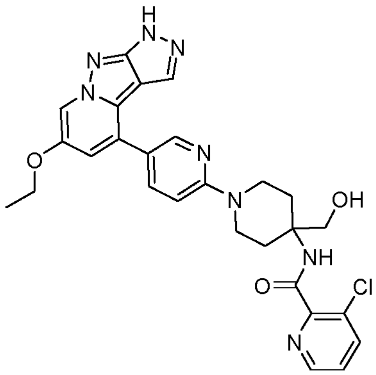

Ethyl

4-(3-chloromethylpyridinamide)-1-(5-(6-ethoxy-1*H*-pyrazolo[3',4':3,4]pyrazolo[1,5-*a*]pyridin-4-yl) pyridin-2-yl)piperidine-4-carboxylate (30 mg, 0.05 mmol) and 2 mL of THF were added to a reaction flask, and $LiAlH_4$ (1.9 mg, 0.05 mmol) was added at 0 °C. The mixture was reacted for 1 h. The reaction was quenched by adding water and stirring. The reaction liquid was extracted with ethyl acetate. The organic phase was dried over anhydrous sodium sulfate, concentrated under reduced pressure, and separated by column chromatography to give a product (18 mg), m/z = 547.2 $[M+1]^+$, $^1$H NMR (400 MHz, DMSO-$d_6$) $\delta$ 12.65 (s, 1H), 8.62-8.49 (m, 3H), 8.23 (s, 1H), 8.03 (m, 2H), 7.60 (s, 1H), 7.52 (m, 1H), 7.27 (d, 1H), 7.11 (d, 1H), 4.90 (t, 1H), 4.27-4.07 (m, 5H), 3.66 (d, 2H), 3.18 (d, 1H), 2.29 (d, 2H), 1.72 (m, 2H), 1.40 (t, 3H). M/z = 547.2 $[M+1]^+$.

**Example 7: Preparation of compound 15**

Step A: *tert*-butylcarbonyl (1-5-(6-ethoxy-1*H*-pyrazolo[3',4':3,4]pyrazolo[1,5-a]pyridin-4-yl)pyridin-2-yl)-4-(hydroxymethyl)p iperidin-4-yl)carbamate

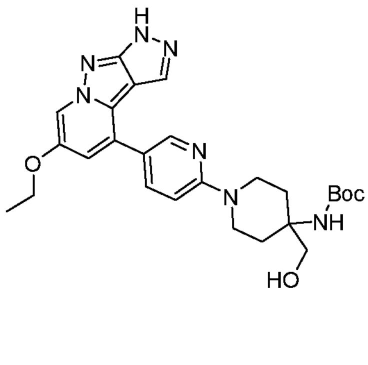

Ethyl

4-((*tert*-butylcarbonyl)amino)-1-(5-(6-ethoxy-1*H*-pyrazolo[3',4':3,4]pyrazolo[1,5-*a*]pyridin-4-yl)pyr idin-2-yl)piperidine-4-carboxylate (2.7 g, 5 mmol) and THF (100 mL) were added to a reaction flask, and $LiBH_4$ (0.55 g, 25 mmol) was added at 0 °C. The mixture was reacted at room temperature for 12 h. After the reaction was completed as monitored by LCMS, the reaction liquid was poured into 200 mL of water, and the resulting mixture was extracted with ethyl acetate. The organic phase was dried and concentrated by rotary evaporation to remove the solvent to give a product (2.1 g), m/z = 508 $[M+1]^+$.

Step B: *tert*-butylcarbonyl (1-(5-(6-ethoxy-1*H*-pyrazolo[3',4':3,4]pyrazolo[1,5-*a*]pyridin-4-yl)pyridin-2-yl)-4-formylpiperidin-4-yl)carbamate

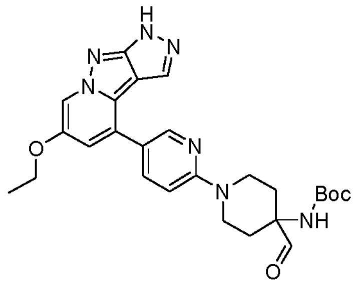

Ethyl

4-((*tert*-butylcarbonyl)amino)-1-(5-(6-ethoxy-1*H*-pyrazolo[3',4':3,4]pyrazolo[1,5-*a*]pyridin-4-yl)pyr idin-2-yl)piperidine-4-carboxylate (1 g, 2.0 mmol) was added to 40 mL of DCM, and Dess-Martin (0.93 g, 2.2 mmol) was added at 0 °C. The mixture was reacted. After the reaction was completed as monitored by LCMS, the reaction was quenched by adding water and stirring. The reaction liquid was extracted with ethyl acetate. The organic phase was dried over anhydrous sodium sulfate, concentrated under reduced pressure, and separated by column chromatography to give a product (0.8 g), m/z = 506 $[M+1]^+$.

Step C: *tert*-butylcarbonyl (4-((2-methylamino)methyl)-1-(5-(6-ethoxy-1*H*-pyrazolo[3',4':3,4]pyrazolo[1,5-*a*]pyridin-4-yl)pyri din-2-yl)piperidin-4-yl)carbamate

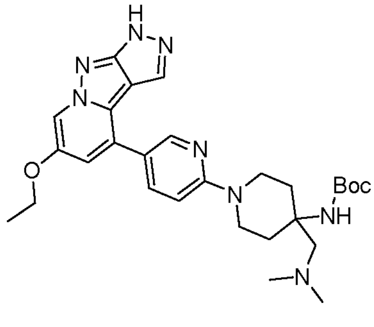

*Tert*-butyl carbonyl

(1-(5-(6-ethoxy-1*H*-pyrazolo[3',4':3,4]pyrazolo[1,5-*a*]pyridin-4-yl)pyridin-2-yl)-4-formylpiperidin-4-yl)carbamate (505 mg, 1 mmol), a solution of dimethylamine in tetrahydrofuran (2 M/L, 3 mL, 3 mmol), $NaBH(OAc)_3$ (424 mg, 2 mmol), and DCE (10 mL) were added to a reaction flask. The mixture was reacted at 25 °C for 12 h. After the reaction was completed as monitored by LCMS, the reaction was quenched by adding water and stirring. The reaction liquid was extracted with ethyl acetate. The organic phase was dried over anhydrous sodium sulfate, concentrated under reduced pressure, and separated by column chromatography to give a product (400 mg), m/z = 534.2 $[M+1]^+$.

Step D: 4-((2-dimethylamino)methyl)-1-(5-(6-ethoxy-1*H*-pyrazolo[3',4':3,4]pyrazolo[1,5-*a*]pyridin-4-yl)pyr idin-2-yl)piperidin-4-amine hydrochloride

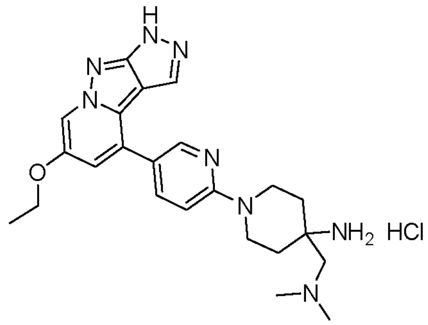

*Tert*-butyl carbonyl

(4-((2-methylamino)methyl)-1-(5-(6-ethoxy-1*H*-pyrazolo[3',4':3,4]pyrazolo[1,5-*a*]pyridin-4-yl)pyri din-2-yl)piperidin-4-yl)carbamate (100 mg, 0.19 mmol) and 2 mL of methanolic hydrochloric acid were added to a reaction flask. The mixture was reacted at 25 °C for 5 h. The reaction liquid was directly concentrated by rotary evaporation to remove the solvent to give a product (80 mg), m/z = 435.2 $[M+1]^+$.

Step E: 3-chloro-*N*-(4-((2-dimethylamino)methyl)-1-(5-(6-ethoxy-1*H*-pyrazolo[3',4':3,4]pyrazolo[1,5-*a*]pyri din-4-yl)pyridin-2-yl)piperidin-4-yl)picolinamide

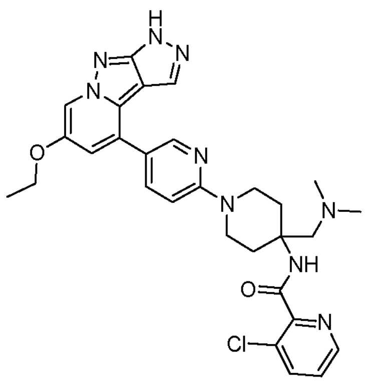

4-((2-dimethylamino)methyl)-1-(5-(6-ethoxy-1*H*-pyrazolo[3',4':3,4]pyrazolo[1,5-*a*]pyridin-4-yl)pyr idin-2-yl)piperidin-4-amine (80 mg, 0.19 mmol), 3-chloropicolinic acid (29.8 mg, 0.19 mmol), HATU (72.2 mg, 0.19 mmol), DIEA (73.5 mg, 0.57mmol), and DMF (10 mL) were added to a reaction flask. The mixture was reacted at 25 °C for 15 h. The reaction was quenched by adding water and stirring. The reaction liquid was extracted with ethyl acetate. The organic phase was dried over anhydrous sodium sulfate, concentrated under reduced pressure, and separated by column chromatography to give a product (60 mg), m/z = 574.2 $[M+1]^+$, $^1H$ NMR (400 MHz, DMSO-$d_6$) δ 12.69 (s, 1H), 8.62-8.49 (m, 3H), 8.28 (s, 1H), 8.08-7.98 (m, 2H), 7.60 (s, 1H), 7.52 (dd, *J* = 8.2, 4.7 Hz, 1H), 7.27 (d, *J* = 2.1 Hz, 1H), 7.10 (d, *J* = 9.0 Hz, 1H), 4.26-4.13 (m, 4H), 3.25 (d, *J* = 12.4 Hz, 2H), 2.70 (s, 2H), 2.38 (d, *J* = 13.2 Hz, 2H), 2.31 (s, 6H), 2.30 (d, *J* = 4.2 Hz, 1H), 1.63 (td, *J* = 13.0, 12.4, 4.1 Hz, 2H), 1.41 (t, *J* = 6.9 Hz, 3H), 1.24 (s, 1H), 0.85 (d, *J* = 7.3 Hz, 1H), 0.5 (s, 1H).

**Example 8: Preparation of compound 17**

Step A: *tert*-butylcarbonyl (1-(5-(6-ethoxy-1*H*-pyrazolo[3',4':3,4]pyrazolo[1,5-*a*]pyridin-4-yl)pyridin-2-yl)-4-((4-ethylpiperazi n-1-yl)methyl)piperidin-4-yl)carbamate

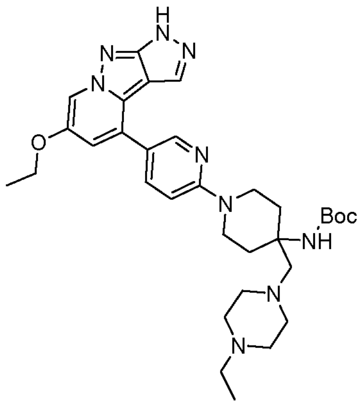

*Tert*-butyl carbonyl

(1-(5-(6-ethoxy-1*H*-pyrazolo[3',4':3,4]pyrazolo[1,5-*a*]pyridin-4-yl)pyridin-2-yl)-4-formylpiperidin-4-yl)carbamate (505 mg, 1 mmol), ethylpiperazine (342 mg, 3 mmol), $NaBH(OAc)_3$ (424 mg, 2 mmol), and DCE (10 mL) were added to a reaction flask. The mixture was reacted at 25 °C for 12 h. After the reaction was completed as monitored by LCMS, the reaction was quenched by adding water and stirring. The reaction liquid was extracted with dichloromethane. The organic phase was dried over anhydrous sodium sulfate, concentrated under reduced pressure, and separated by column chromatography to give a product (800 mg), m/z = 604.4 $[M+1]^+$.

Step B: 1-(5-(6-ethoxy-1*H*-pyrazolo[3',4':3,4]pyrazolo[1,5-*a*]pyridin-4-yl)pyridin-2-yl)-4-((4-ethylpiperazin -1-yl)methyl)piperidin-4-amine hydrochloride

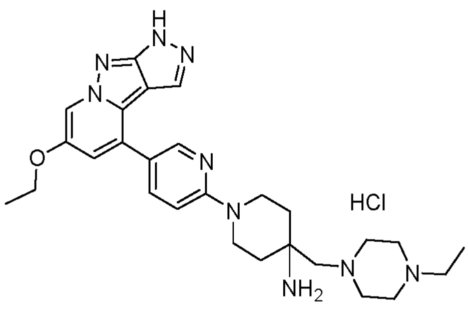

*Tert*-butyl carbonyl

(1-(5-(6-ethoxy-1*H*-pyrazolo[3',4':3,4]pyrazolo[1,5-*a*]pyridin-4-yl)pyridin-2-yl)-4-((4-ethylpiperazi n-1-yl)methyl)piperidin-4-yl)carbamate (120 mg, 0.2 mmol) and 2 mL of methanolic hydrochloric acid were added to a reaction flask. The mixture was reacted at 25 °C for 5 h. The reaction liquid was directly concentrated by rotary evaporation to remove the solvent to give a product (100 mg), m/z = 504.2 $[M+1]^+$.

Step C: *N*-(1-(5-(6-ethoxy-1*H*-pyrazolo[3',4':3,4]pyrazolo[1,5-*a*]pyridin-4-yl)pyridin-2-yl)-4-((4-ethylpipera zin-1-yl)methyl)piperidin-4-yl)-2,5-difluorobenzamide

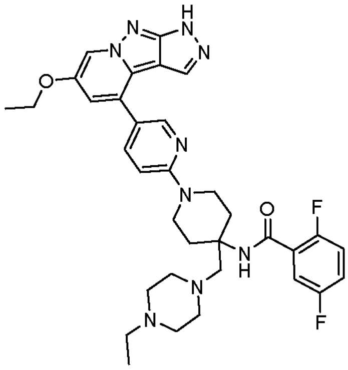

4-((2-dimethylamino)methyl)-1-(5-(6-ethoxy-1*H*-pyrazolo[3',4':3,4]pyrazolo[1,5-*a*]pyridin-4-yl)pyr idin-2-yl)piperidin-4-amine (100 mg, 0.2 mmol), 2,5-difluorobenzoic acid (31.6 mg, 0.2 mmol), HATU (76.0 mg, 0.2 mmol), DIEA (77.4 mg, 0.6 mmol), and DMF (10 mL) were added to a reaction flask. The mixture was reacted at 25 °C for 15 h. The reaction liquid was poured into water, and the resulting mixture was extracted with ethyl acetate. The organic phase was dried, concentrated by rotary evaporation to remove the solvent, and purified by silica gel column chromatography to give a product (67 mg), m/z = 644.2 $[M+1]^+$. $^1H$ NMR (400 MHz, DMSO-$d_6$): δ 12.65 (s, 1H), 8.59 (d, *J* = 2.8 Hz, 1H), 8.52 (d, *J* = 2.0 Hz, 1H), 8.13 (s, 1H), 8.04 (d, *J* = 8.8 Hz, 1H), 7.60 (s, 1H), 7.41-7.29 (m, 3H), 7.26 (d, *J* = 2.1 Hz, 1H), 7.10 (d, *J* = 9.0 Hz, 1H), 4.19 (m, 4H), 3.32 (s, 1H), 3.27-3.15 (m, 2H), 2.73 (s, 2H), 2.61-2.55 (m, 7H), 2.34 (d, *J* = 13.3 Hz, 4H), 1.62 (m, 2H), 1.41 (t, *J* = 6.9 Hz, 3H), 0.99 (m, 3H). M/z = 644.2 $[M+1]^+$.

**Example 9: Preparation of compound 29**

Step A: *tert*-butylcarbonyl (1-(5-(6-ethoxy-1*H*-pyrazolo[3',4':3,4]pyrazolo[1,5-*a*]pyridin-4-yl)pyridin-2-yl)-4-(morpholinometh yl)piperidin-4-yl)carbamate

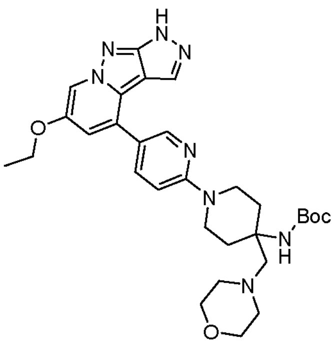

Tert-butylcarbonyl

(1-(5-(6-ethoxy-1*H*-pyrazolo[3',4':3,4]pyrazolo[1,5-*a*]pyridin-4-yl)pyridin-2-yl)-4-formylpiperidin-4-yl)carbamate (505 mg, 1 mmol), morpholine (261 mg, 3 mmol), $NaBH(OAc)_3$ (424 mg, 2 mmol), and DCE (10 mL) were added to a reaction flask. The mixture was reacted at 25 °C for 12 h. After the reaction was completed as monitored by LCMS, the reaction liquid was poured into 20 mL of water, and the resulting mixture was extracted with DCM. The organic solvent was dried, concentrated by rotary evaporation to remove the solvent, and purified by column chromatography to give a product (300 mg), m/z = 576.2 $[M+1]^+$.

Step B: 1-(5-(6-ethoxy-1*H*-pyrazolo[3',4':3,4]pyrazolo[1,5-*a*]pyridin-4-yl)pyridin-2-yl)-4-((4-ethylpiperazin -1-yl)methyl)piperidin-4-amine hydrochloride

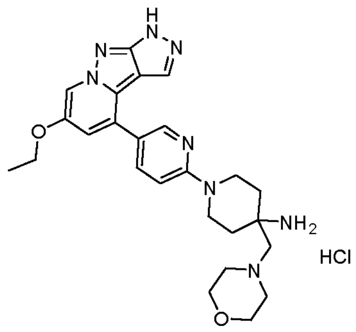

*tert*-butyl carbonyl

(1-(5-(6-ethoxy-1*H*-pyrazolo[3',4':3,4]pyrazolo[1,5-*a*]pyridin-4-yl)pyridin-2-yl)-4-(methylmorpholi ne)piperidin-4-yl)carbamate (120 mg, 0.2 mmol) and 2 mL of methanolic hydrochloric acid were added to a reaction flask. The mixture was reacted at 25 °C for 5 h. The reaction liquid was directly concentrated by rotary evaporation to remove the solvent to give a product (90mg), m/z = 477.2 $[M+1]^+$.

Step C: *N*-(1-(5-(6-ethoxy-1*H*-pyrazolo[3',4':3,4]pyrazolo[1,5-*a*]pyridin-4-yl)pyridin-2-yl)-4-((4-ethylpipera zin-1-yl)methyl)piperidin-4-yl)-3-methylbutanamide

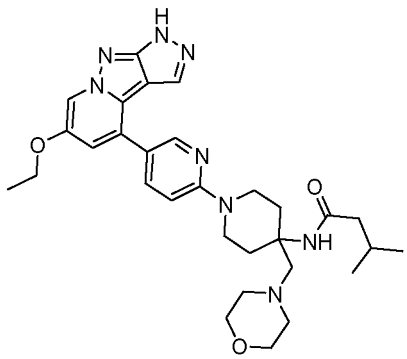

1-(5-(6-ethoxy-1*H*-pyrazolo[3',4':3,4]pyrazolo[1,5-*a*]pyridin-4-yl)pyridin-2-yl)-4-(methylmorpholin e)piperidin-4-amine (90 mg, 0.19 mmol), isovaleric acid (20.4 mg, 0.2 mmol), HATU (76.0 mg, 0.2 mmol), DIEA (77.4mg, 0.6 mmol), and DMF (10 mL) were added to a reaction flask. The mixture was reacted at 25 °C for 15 h. The reaction was quenched by adding water and stirring. The reaction liquid was extracted with dichloromethane. The organic phase was dried over anhydrous sodium sulfate, concentrated under reduced pressure, and separated by column chromatography to give a product (71 mg), m/z = 561.2 $[M+1]^+$, $^1$H NMR (400 MHz, DMSO-$d_6$): δ 12.65 (s, 1H),δ 8.58 (d, *J* = 2.6 Hz, 1H), 8.52 (d, *J* = 2.1 Hz, 1H), 8.36 (s, 1H), 8.03 (dd, *J* = 8.9, 2.6 Hz, 1H), 7.59 (s, 1H), 7.26 (d, *J* = 2.1 Hz, 1H), 7.07 (d, *J* = 9.0 Hz, 1H), 4.18 (m, 3H), 3.55 (m, 4H), 3.10 (m, 2H), 2.60 (s, 2H), 2.46 (m, 5H), 2.26 (d, *J* = 13.2 Hz, 2H), 2.02 (d, *J* = 5.4 Hz, 2H), 1.54 (dt, *J* = 12.8, 6.7 Hz, 2H), 1.40 (m, 3H), 1.24 (s, 1H), 0.90 (d, *J* = 6.2 Hz, 6H).

**Example 10: Preparation of compound 31**

Step A: *tert*-butyl ((3*S*,4*R*)-1-(5-bromopyridin-2-yl)-4-hydroxypyrrolidin-3-yl)aminocarbonate

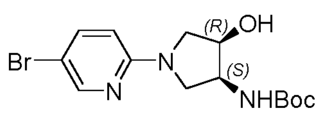

5-bromo-2-fluoropyridine (240 mg, 1.36 mmol), DMSO (2.5 mL), potassium carbonate (340 mg, 2.47 mmol), and *tert*-butyl ((3*S*,4*R*)-4-hydroxypyrrolidin-3-yl)aminocarbonate (250 mg, 1.24 mmol) were sequentially added. After the addition, the mixture was heated to 90 °C and reacted for 16 h. After the reaction was completed, the reaction liquid was poured into water, and the resulting mixture was extracted with dichloromethane. The mixture was dried over anhydrous sodium sulfate and concentrated by rotary evaporation to give a product (390 mg), m/z = 358/360 $[M+1]^+$.

Step B: *tert*-butyl ((3*S*,4*S*)-1-(5-bromopyridin-2-yl)-4-(pyridin-2-yloxy)pyrrolidin-3-yl)aminocarbonate

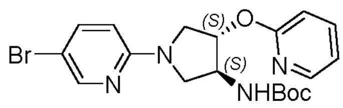

THF (20 mL), TMAD (260 mg, 1.51 mmol), and BuPs (305 mg, 1.51 mmol) were sequentially added to a reaction flask. The mixture was stirred at room temperature for 30 min, and then a solution of tert-butyl ((3*R*,4*S*)-1-(5-bromopyridin-2-yl)-4-hydroxypyrrolidin-3-yl)aminocarbonate (270 mg, 0.75 mmol) in THF (10 mL) was added dropwise. After the dropwise addition, the mixture was stirred for 1 min, and then 2-hydroxypyridine (143 mg, 1.51 mmol) was added. The mixture was heated to 50 °C and reacted for 3 h. The reaction liquid was poured into water, and the resulting mixture was extracted with dichloromethane and purified by column chromatography to give a product (140 mg), m/z = 435/437 $[M+1]^+$.

Step C: *tert*-butyl ((3*S*,4*S*)-1-(5-(6-ethoxy-1-(tetrahydro-2*H*-pyran-2-yl)-1*H*-pyrazolo[3',4':3,4]pyrazolo[1,5-*a*]pyridin -4-yl)pyridin-2-yl)-4-(pyridin-2-yloxy)pyrrolidin-3-yl)aminocarbonate

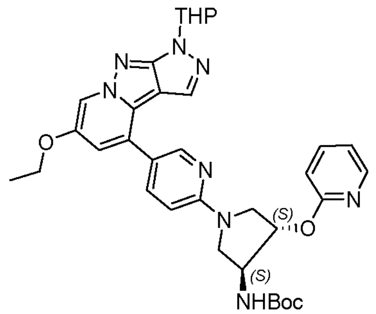

1,4-dioxane (1 mL), *tert*-butyl ((3*S*,4*S*)-1-(5-bromopyridin-2-yl)-4-(pyridin-2-yloxy)pyrrolidin-3-yl)aminocarbonate (60 mg, 0.14 mmol), 6-ethoxy-1-(tetrahydro-2*H*-pyran-2-yl)-4-(4,4,5,5-tetramethyl-1,3,2-dioxaborane-2-yl)-1*H*-pyrazolo [3',4':3,4]pyrazolo[1,5-*a*]pyridine (47.5 mg, 0.12 mmol), potassium carbonate (32.8 mg, 0.23 mmol), tetrakis(triphenylphosphine)palladium(0) (13.3 mg, 0.006 mmol), and water (0.5 mL) were sequentially added to a reaction flask. The mixture was heated to 90 °C and reacted for 16 h. The reaction liquid was poured into water, and the resulting mixture was extracted with dichloromethane and purified by column chromatography to give a product (40 mg), m/z = 641 $[M+1]^+$.

Step D: (3*S*,4*S*)-1-(5-(6-ethoxy-1*H*-pyrazolo[3',4':3,4]pyrazolo[1,5-*a*]pyridin-4-yl)pyridin-2-yl)-4-(pyridin-2 -yloxy)pyrrolidin-3-amine

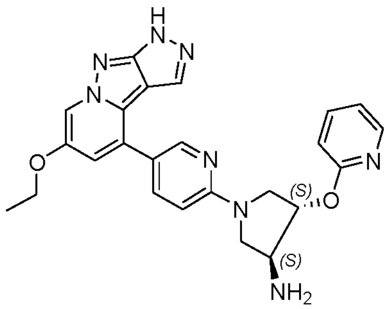

*Tert*-butyl

((3*S*,4*S*)-1-(5-(6-ethoxy-1-(tetrahydro-2*H*-pyran-2-yl)-1*H*-pyrazolo[3',4':3,4]pyrazolo[1,5-*a*]pyridin -4-yl)pyridin-2-yl)-4-(pyridin-2-yloxy)pyrrolidin-3-yl)aminocarbonate (40 mg, 0.06 mmol), methanol (2 mL), and a solution of HCl in dioxane (4 M, 2 mL) were added to a 50 mL reaction flask. The mixture was stirred at room temperature for 2 h. The reaction liquid was poured into water, and the resulting mixture was extracted with dichloromethane and purified by column chromatography to give a product (6.9 mg), m/z = 457 $[M+1]^+$. $^1H$ NMR (400 MHz, DMSO-$d_6$) δ 12.63 (s, 1H), 8.55-8.54 (m, 2H), 8.23-8.21 (m, 1H), 7.80-8.02 (m, 1H), 7.71-7.75 (m, 1H), 7.59 (s, 1H), 7.21-7.22 (m, 1H), 7.00-7.04 (m, 1H), 6.81-6.85 (m, 1H), 6.69-6.71 (m, 1H), 5.28-5.29 (m, 1H), 4.14-4.19 (m, 2H), 4.01-4.05 (m, 1H), 3.74-3.78 (m, 1H), 3.61-3.67 (m, 3H), 1.38-1.41 (m, 3H).

**Example 11: Preparation of compound 33**

Step A: 6-benzyl-1-oxa-6-azaspiro[2.5]octane

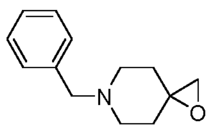

*N*-benzyl-4-piperidone (5.7 g, 30 mmol) and dimethylsulfoxide (75 mL) were added to a reaction flask. The mixture was stirred, and NaH (1.44 g, 33 mmol) was added. The mixture was reacted for 1 h, and trimethyl sulfoxide iodide (7.3 g, 33 mmol) was added. The mixture was reacted at room temperature for 1 h. The reaction was quenched by adding water. The reaction liquid was extracted with ethyl acetate, dried over anhydrous sodium sulfate, filtered, and concentrated under reduced pressure to dryness to give a product (5.6 g), m/z = 204 $[M+1]^+$.

Step B: 6-benzyl-1-thia-6-azaspiro[2.5]octane

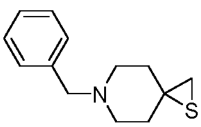

6-benzyl-1-oxa-6-azaspiro[2.5]octane (5.6 g, 27.6 mmol) and methanol (80 mL) were sequentially added to a reaction flask. The mixture was stirred, and thiourea (2.3 g, 30.4 mmol) was added. The mixture was reacted at 40 °C for 20 h. After the reaction was completed, water was added, and the resulting mixture was extracted ethyl acetate, dried over anhydrous sodium sulfate, filtered, concentrated under reduced pressure to dryness, and purified by silica gel column chromatography to give a product (4.63 g), m/z = 220 $[M+1]^+$.

Step C: 1-benzyl-4-methylpiperidine-4-thiol

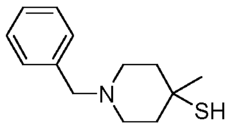

6-benzyl-1-thia-6-azaspiro[2.5]octane (4.3 g, 19.6 mmol) and tetrahydrofuran (50 mL) were sequentially added to a reaction flask. The mixture was cooled to 5 °C, and lithium aluminum hydride (1.1 g, 29.4 mmol) was added. The mixture was reacted for 1 h with the temperature maintained. After the reaction was completed, 5 mL of water was slowly added, and a saturated sodium hydroxide solution was added to adjust the pH to 14. The reaction mixture was extracted with dichloromethane, dried over anhydrous sodium sulfate, concentrated under reduced pressure to dryness, and purified by silica gel column chromatography to give a product (3.8 g), m/z = 222 $[M+1]^+$.

Step D: 1-benzyl-4-(isobutylthio)-4-methylpiperidine

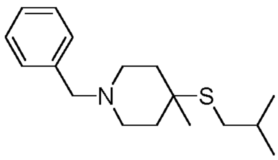

1-benzyl-4-methylpiperidine-4-thiol (1.3 g, 6 mmol) and 30 mL of *N*,*N*-dimethylformamide were sequentially added to a reaction flask. The mixture was cooled to 5 °C, and 1-bromo-2-methylpropane (0.9 g, 6.6 mmol) was added, followed by potassium carbonate (2.5 g, 18 mmol). The mixture was reacted at 40 °C for 16 h. After the reaction was completed, the reaction was quenched by adding water and stirring. The reaction liquid was extracted with dichloromethane. The organic phase was dried over anhydrous sodium sulfate, concentrated under reduced pressure, and separated by column chromatography to give a product (1.0 g), m/z = 278 $[M+1]^+$.

Step E: 1-benzyl-4-(isobutylsulfonyl)-4-methylpiperidine

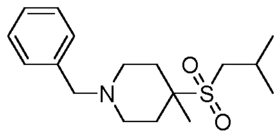

1-benzyl-4-(isobutylsulfanyl)-4-methylpiperidine (500 mg, 1.8 mmol) and dichloromethane (6 mL) were sequentially added to a reaction flask. The mixture was cooled to 5 °C, and *m*-chloroperoxybenzoic acid (930 mg, 5.4 mmol) was added. The mixture was reacted at room temperature for 16 h. After the reaction was completed, the reaction was quenched by adding water and stirring. The reaction liquid was extracted with dichloromethane. The organic phase was dried over anhydrous sodium sulfate, concentrated under reduced pressure, and separated by column chromatography to give a product (250 mg), m/z = 310 $[M+1]^+$.

Step F: 4-(isobutylsulfonyl)-4-methylpiperidine

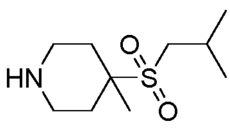

1-benzyl-4-(isobutylsulfonyl)-4-methylpiperidine (250 mg, 0.8 mmol) and ethanol (10 mL) were sequentially

added to a reaction flask, and then 10% Pd/C (200 mg) was added. The mixture was purged with hydrogen and reacted at room temperature for 16 h. After the reaction was completed, the reaction liquid was filtered and concentrated under reduced pressure to dryness to give a product (180 mg), m/z = 220 [M+1]$^+$.

Step G: 6-ethoxy-4-(6-(4-(isobutylsulfonyl)-4-methylpiperidin-1-yl)pyridin-3-yl)-1*H*-pyrazolo[3',4':3,4]pyr azolo[1,5-*a*]pyridine

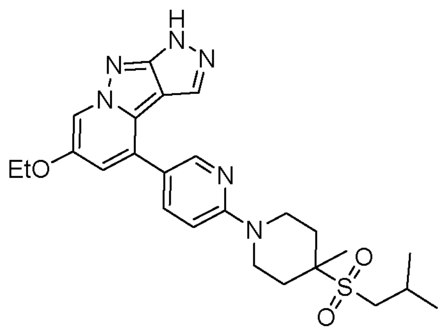

2 mL of DMSO and 0.5 mL of DIEA, 4-(isobutylsulfonyl)-4-methylpiperidine (180 mg, 0.82 mmol), and 6-ethoxy-4-(6-fluoropyridin-3-yl)-1*H*-pyrazolo[3',4':3,4]pyrazolo[1,5-*a*]pyridine (100 mg, 0.34 mmol) were sequentially added to a reaction flask. The mixture was reacted at 100 °C for 60 h. After the reaction was completed, the reaction was quenched by adding water and stirring. The reaction liquid was extracted with dichloromethane. The organic phase was dried over anhydrous sodium sulfate, concentrated under reduced pressure, and separated by column chromatography to give a product (66 mg), m/z = 497 [M+1]$^+$. $^1$H-NMR (400 MHz, DMSO-$d_6$) $\delta$ 12.66 (s, 1H), 8.60 (d, *J* = 2.5 Hz, 1H), 8.52 (d, *J* = 2.1 Hz, 1H), 8.07 (dd, *J* = 8.9, 2.6 Hz, 1H), 7.59 (s, 1H), 7.28 (d, *J* = 2.1 Hz, 1H), 7.13 (d, *J* = 8.9 Hz, 1H), 4.43 (d, *J* = 13.7 Hz, 2H), 4.18 (q, *J* = 6.9 Hz, 2H), 3.16 (m, 2H), 2.96 (d, *J* = 6.6 Hz, 2H), 2.25 (m, 1H), 2.01 (m, 2H), 1.72 (d, *J* = 13.0 Hz, 2H), 1.53 (s, 3H), 1.40 (t, *J* = 6.9 Hz, 3H), 1.08 (d, *J* = 6.7 Hz, 6H).

**Example 12: Preparation of compound 34**

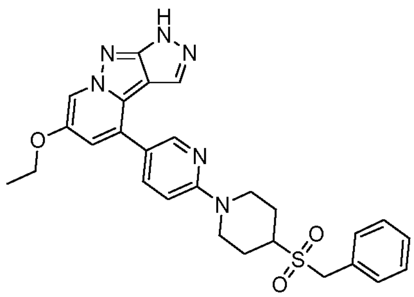

2 mL of DMSO and 0.5 mL of DIEA, 4-benzylsulfonic piperidine (140 mg, 0.51 mmol), and 6-ethoxy-4-(6-fluoropyridin-3-yl)-1*H*-pyrazolo[3',4':3,4]pyrazolo[1,5-*a*]pyridine (50 mg, 0.17 mmol) were sequentially added to a reaction flask. The mixture was reacted at 100 °C for 20 h. The reaction was quenched by adding water and stirring. The reaction liquid was extracted with ethyl acetate. The organic phase was dried over anhydrous sodium sulfate, concentrated under reduced pressure, and separated by column chromatography to give a product, m/z = 517.

**Example 13: Preparation of compound 35** 4-((dimethylamino)methyl)-1-(5-(6-ethoxy-1*H*-pyrazolo[3',4':3,4]pyrazolo[1,5-*a*]pyridin-4-yl)pyrid in-2-yl)-*N*-isobutylpiperidine-4-carboxamide (compound APS03035)

Step A: 1-(5-bromopyridin-2-yl)-4-formyl-*N*-isobutylpiperidine-4-carboxamide

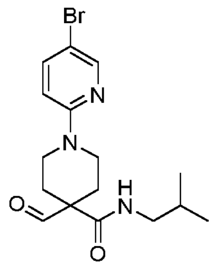

1-(5-bromopyridin-2-yl)-4-(hydroxymethyl)-*N*-isobutylpiperidine-4-carboxamide (170 mg, 0.46 mmol) and dichloromethane (10 mL) were added to a 50 mL reaction flask, and then Dess-Marting (292 mg, 0.69 mmol) was added in portions at room temperature. The mixture was stirred at room temperature for 0.5 h. The reaction was quenched by adding an aqueous sodium sulfite solution. The organic phase was concentrated by rotary evaporation and separated by column chromatography to give a product (210 mg), m/z = 368/370 $[M+1]^+$.

Step B: 1-(5-bromopyridin-2-yl)-4-((dimethylamino)methyl)-*N*-isobutylpiperidine-4-carboxamide

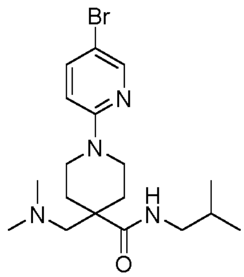

1-(5-bromopyridin-2-yl)-4-formyl-*N*-isobutylpiperidine-4-carboxamide (70 mg, 0.19 mmol), 1,2-dichloromethane (2 mL), dimethylamine (25.7 mg, 0.57 mmol), and sodium borohydride acetate (80.8 mg, 0.38 mmol) were added to a 50 mL reaction flask. The mixture was stirred at room temperature for 16 h. The reaction was quenched by adding water. The reaction liquid was concentrated and separated by column chromatography to give a product (50 mg), m/z = 397/399 $[M+1]^+$.

Step C: 1-(5-bromopyridin-2-yl)-4-((dimethylamino)methyl)-*N*-isobutylpiperidine-4-carboxamide

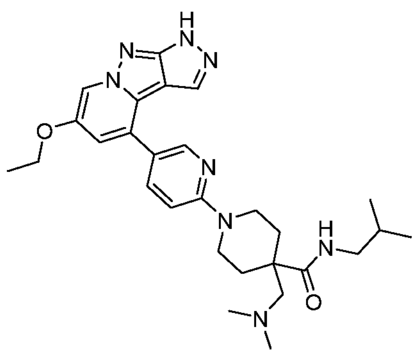

1-(5-bromopyridin-2-yl)-4-((dimethylamino)methyl)-*N*-isobutylpiperidine-4-carboxamide (50 mg, 0.13 mmol), 6-ethoxy-4-(4,4,5,5-tetramethyl-1,3,2-dioxaborane-2-yl)-1*H*-pyrazolo[3',4':3,4]pyrazolo[1,5-*a*]pyrid ine (56.2 mg, 0.15 mmol), tetrakis(triphenylphosphine)palladium(0) (23 mg, 0.02 mmol), potassium carbonate (34.9 mg, 0.25 mmol), water (0.3 mL), and 1,4-dioxane (0.6 mL) were sequentially added to a reaction flask. The mixture was heated to 90 °C for 16 h. The reaction liquid was poured into water, and the resulting mixture was extracted with dichloromethane and purified

by column chromatography to give a product (5 mg), m/z = 519 $[M+1]^+$. $^1H$ NMR (400MHz, DMSO-$d_6$) δ 12.64 (s, 1H), 8.50-8.56 (m, 2H), 8.00-8.03 (m, 1H), 7.83 (s, 1H), 7.58 (s, 1H), 7.25-7.25 (m, 1H), 7.03-7.05 (m, 1H), 4.15-4.20 (m, 2H), 4.04-4.07 (m, 2H), 3.16-3.21 (m, 2H), 2.96 (m, 2H), 2.42 (s, 1H), 2.09-2.17 (m, 8H), 1.75-1.79 (m, 1H), 1.46-1.52 (m, 2H), 1.30-1.40 (m, 3H), 1.21 (s, 1H), 0.80-0.90 (m, 6H).

**Example 14: Preparation of compound 36**

4-(azetidin-1-ylmethyl)-1-(5-(6-ethoxy-1*H*-pyrazolo[3',4':3,4]pyrazolo[1,5-*a*]pyridin-4-yl)pyridin-2 -yl)-*N*-isobutylpiperidine-4-carboxamide (compound APS03036)

Step A: 1-(5-bromopyridin-2-yl)-4-(azetidin-1-ylmethyl)-*N*-isobutylpiperidine-4-carboxamide

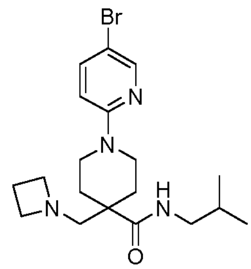

1-(5-bromopyridin-2-yl)-4-formyl-*N*-isobutylpiperidine-4-carboxamide (70 mg, 0.19 mmol), 1,2-dichloromethane (2 mL), azetidine (32.6 mg, 0.57 mmol), and sodium borohydride acetate (80.8 mg, 0.38 mmol) were added to a 50 mL reaction flask. The mixture was stirred at room temperature for 16 h. The reaction was quenched by adding water. The organic phase was concentrated by rotary evaporation and separated by column chromatography to give a product (60 mg), m/z = 409/411 $[M+1]^+$.

Step B: 4-(azetidin-1-ylmethyl)-1-(5-(6-ethoxy-1*H*-pyrazolo[3',4':3,4]pyrazolo[1,5-*a*]pyridin-4-yl)pyridin-2 -yl)-N-isobutylpiperidine-4-carboxamide

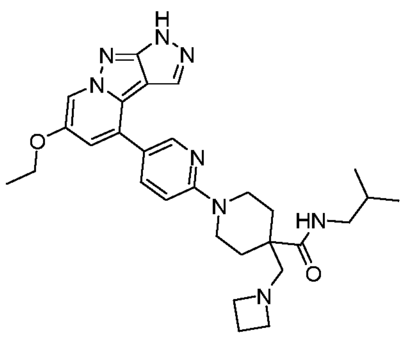

1-(5-bromopyridin-2-yl)-4-(azetidin-1-ylmethyl)-*N*-isobutylpiperidine-4-carboxamide (60 mg, 0.15 mmol), 6-ethoxy-4-(4,4,5,5-tetramethyl-1,3,2-dioxaborane-2-yl)-1*H*-pyrazolo[3',4':3,4]pyrazolo[1,5-*a*]pyrid ine (65.3 mg, 0.18 mmol), tetrakis(triphenylphosphine)palladium(0) (23 mg, 0.02 mmol), potassium carbonate (40.6 mg, 0.29 mmol), water (0.35 mL), and 1,4-dioxane (0.7 mL) were added to a reaction flask. The mixture was purged with nitrogen three times, heated to 90 °C, and reacted for 16 h. The reaction liquid was poured into water, and the resulting mixture was extracted with dichloromethane and purified by column chromatography to give a product (6 mg), m/z = 531 $[M+1]^+$. $^1H$ NMR (400MHz, DMSO-$d_6$) δ 12.65 (s, 1H), 8.50-8.56 (m, 2H), 7.94-8.03 (m, 2H), 7.71-7.81 (m, 1H), 7.58 (s, 1H), 7.24-7.25 (m, 1H), 7.02-7.04 (m, 1H), 4.14-4.20 (m, 2H), 4.04-4.07 (m, 2H), 3.15-3.20 (m, 6H), 2.93-2.96 (m, 2H), 2.04-2.17 (m, 2H), 1.88-1.96 (m, 2H), 1.74-1.80 (m, 1H), 1.34-1.44 (m, 5H), 0.85-0.88 (m, 7H).

**Example 15: Preparation of compound 37**

4-(pyrrolidin-1-ylmethyl)-1-(5-(6-ethoxy-1*H*-pyrazolo[3',4':3,4]pyrazolo[1,5-*a*]pyridin-4-yl)pyridin -2-yl)-*N*-isobutylpiperidine-4-carboxamide (compound APS03037)

Step A: 1-(5-bromopyridin-2-yl)-4-(pyrrolidin-1-ylmethyl)-*N*-isobutylpiperidine-4-carboxamide

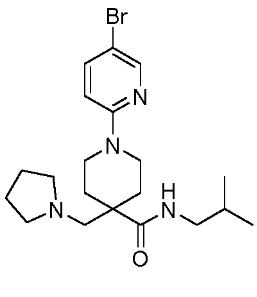

1-(5-bromopyridin-2-yl)-4-formyl-*N*-isobutylpiperidine-4-carboxamide (70 mg, 0.19 mmol), 1,2-dichloromethane (2 mL), pyrrolidine (40.6 mg, 0.57 mmol), and sodium borohydride acetate (80.8 mg, 0.38 mmol) were added to a 50 mL reaction flask. The mixture was stirred at room temperature for 16 h. The reaction was quenched by adding water. The organic phase was concentrated by rotary evaporation and separated by column chromatography to give a product (65 mg), m/z = 423/425 $[M+1]^+$.

Step B: 4-(pyrrolidin-1-ylmethyl)-1-(5-(6-ethoxy-1*H*-pyrazolo[3',4':3,4]pyrazolo[1,5-*a*]pyridin-4-yl)pyridin -2-yl)-*N*-isobutylpiperidine-4-carboxamide

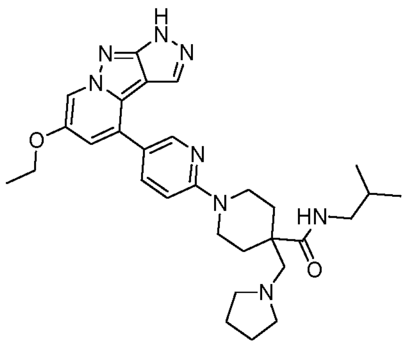

1-(5-bromopyridin-2-yl)-4-(pyrrolidin-1-ylmethyl)-*N*-isobutylpiperidine-4-carboxamide (65 mg, 0.15 mmol), 6-ethoxy-4-(4,4,5,5-tetramethyl-1,3,2-dioxaborane-2-yl)-1*H*-pyrazolo[3',4':3,4]pyrazolo[1,5-*a*]pyrid ine (68.3 mg, 0.18 mmol), tetrakis(triphenylphosphine)palladium(0) (23 mg, 0.02 mmol), potassium carbonate (42.5 mg, 0.31 mmol), water (0.3 mL), and 1,4-dioxane (0.7 mL) were sequentially added to a reaction flask. The mixture was purged with nitrogen three times, heated to 90 °C, and reacted for 16 h. The reaction liquid was poured into water, and the resulting mixture was extracted with dichloromethane and purified by reverse phase column chromatography to give a product (5 mg), m/z = 545 $[M+1]^+$. $^1H$ NMR (400MHz, DMSO-$d_6$) $\delta$ 12.67 (s, 1H), 8.50-8.56 (m, 2H), 7.71-8.03 (m, 4H), 7.56-7.65 (m, 2H), 7.40-7.41 (m, 1H), 7.24-7.25 (m, 1H), 7.02-7.05 (m, 1H), 4.14-4.20 (m, 2H), 4.04-4.07 (m, 2H), 3.15-3.24 (m, 2H), 2.93-2.96 (m, 2H), 2.51-2.62 (m, 2H), 2.11-2.17 (m, 2H), 1.73-1.79 (m, 1H), 1.63 (s, 4H), 1.38-1.51 (m, 5H), 0.84-0.87 (m, 6H).

**Example 16: Preparation of compound 39**

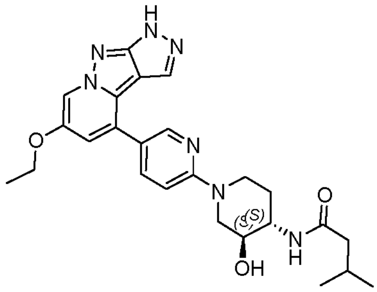

(3*S*,4*S*)-4-amino-1-(5-(6-ethoxy-1*H*-pyrazolo[3',4':3,4]pyrazolo[1,5-*a*]pyridin-4-yl)pyridin-2-yl)pip eridin-3-ol (256.5 mg, 0.51 mmol), 3-methylbutyric acid (78 mg, 0.77 mmol), DIEA (263 mg, 2.04 mmol) and DCM (10 mL), and HATU (292 mg, 0.77 mmol) were added to a 50 mL reaction flask. The mixture was reacted at room temperature for 0.5 h. The reaction was quenched by adding water and stirring. The reaction liquid was extracted with ethyl acetate, dried over anhydrous sodium sulfate, concentrated under reduced pressure, and separated by column chromatography to give a product (6.4 mg), m/z = 478 $[M+1]^+$, $^1H$ NMR (400 MHz, DMSO-$d_6$) $\delta$ 12.65 (s, 1H), 8.57 (s, 1H), 8.51 (s, 1H), 8.03 (d, 1H), 7.7 (d, 1H), 7.58 (s, 1H), 7.25 (s, 1H), 7.06 (d, 1H), 5.00 (br, 1H), 4.42-4.44 (m, 1H), 4.14-4.25 (m, 3H), 3.60-3.72 (m, 1H), 3.01-3.07 (m, 1H), 2.81-2.87 (m, 1H), 1.89-1.98 (m, 4H), 1.20-1.45 (m, 5H), 0.80-0.89 (m, 6H).

**Example 17: Preparation of compound 40**

Step A: *tert*-butyl ((3*R*,4*S*)-1-(5-(6-ethoxy-1*H*-pyrazolo[3',4':3,4]pyrazolo[1,5-*a*]pyridin-4-yl)pyridin-2-yl)-3-hydroxy piperidin-4-yl)aminocarbonate

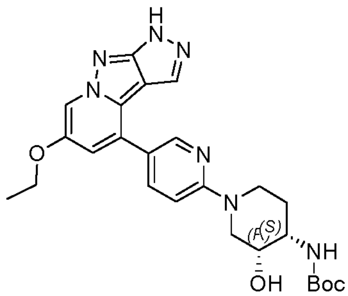

6-ethoxy-4-(6-fluoropyridin-3-yl)-1*H*-pyrazolo[3',4':3,4]pyrazolo[1,5-*a*]pyridine (1.0 g, 3.3 mmol), *tert*-butyl ((3*R*,4*S*)-3-hydroxypiperidin-4-yl)carbonate (720 mg, 3.3 mmol), DIEA (2.2 g, 10 mmol) and 20 mL of DMSO were added to a reaction flask. The mixture was reacted at 90 °C for 4 h. The reaction was quenched by adding water and stirring. The reaction liquid was extracted with ethyl acetate, dried over anhydrous sodium sulfate, concentrated under reduced pressure, and separated by column chromatography to give a product (1.4 g), m/z = 494 $[M+1]^+$.

Step B: ((3*R*,4*S*)-1-(5-(6-ethoxy-1*H*-pyrazolo[3',4':3,4]pyrazolo[1,5-*a*]pyridin-4-yl)pyridin-2-yl)-3-hydroxy piperidin-4-yl)amine hydrochloride

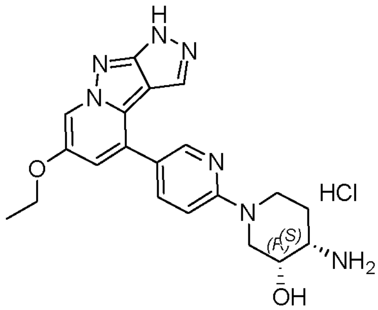

*Tert*-butyl

((3*R*,4*S*)-1-(5-(6-ethoxy-1*H*-pyrazolo[3',4':3,4]pyrazolo[1,5-*a*]pyridin-4-yl)pyridin-2-yl)-3-hydroxy piperidin-4-yl)aminocarbonate (1.4 g, 2.8 mmol) and a solution of hydrochloric acid in dioxane (30 mL, 120 mmol, 4 N) were added to a reaction flask. The mixture was reacted at 25 °C for 0.5 h. The reaction liquid was directly concentrated under reduced pressure to dryness to give a product (1.6 g), m/z = 394 $[M+1]^+$.

Step C: *N*-((3*R*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-3-methylbutanamide

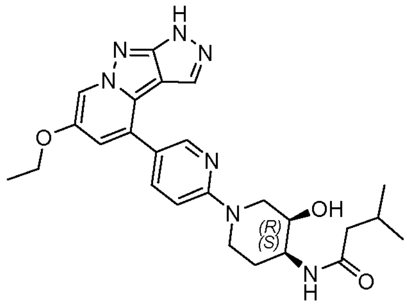

((3*R*,4*S*)-1-(5-(6-ethoxy-1*H*-pyrazolo[3',4':3,4]pyrazolo[1,5-*a*]pyridin-4-yl)pyridin-2-yl)-3-hydroxy piperidin-4-yl)amine hydrochloride (200 mg, 0.4 mmol), 3-methylbutyric acid (48 mg, 0.47 mmol), DIEA (166 mg, 1.29 mmol), HATU (179 mg, 0.47 mmol), and 2.5 mL of DMF were added to a reaction flask. The mixture was reacted at 25 °C for 1 h. The reaction was quenched by adding water and stirring. The reaction liquid was extracted with ethyl acetate, dried over anhydrous sodium sulfate, concentrated under reduced pressure, and separated by column chromatography to give a product (42 mg), m/z = 478 $[M+1]^+$, $^1H$ NMR (400 MHz, DMSO-*d6*): $\delta$ 12.64 (s, 1H), 8.37 (s, 1H), 8.50 (s, 1H), 8.00 (dd, 1H), 7.56-7.59 (m, 2H), 7.23 (s, 1H), 7.03 (d, 1H), 4.91 (s, 1H), 4.10-4.27 (m, 4H), 3.92-3.99 (m, 1H), 3.88-3.91 (m, 1H), 3.18-3.21 (m, 1H), 3.10-3.16 (m, 1H), 1.97-2.03 (m, 3H), 1.86-1.94 (m, 1H), 1.56-1.60 (m, 1H), 1.38-1.43 (m, 3H), 0.88-0.94 (m, 6H).

**Example 18: Preparation of compound 41**

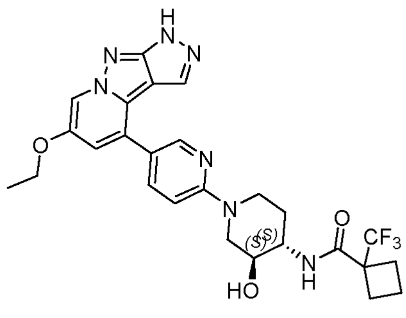

(3*S*,4*S*)-4-amino-1-(5-(6-ethoxy-1*H*-pyrazolo[3',4':3,4]pyrazolo[1,5-*a*]pyridin-4-yl)pyridin-2-yl)pip eridin-3-ol (256.5 mg, 0.51 mmol), 1-trifluoromethylcyclobutanoic acid (128 mg, 0.77 mmol), DIEA (263 mg, 2.04 mmol) and DCM (10 mL), and HATU (292 mg, 0.77 mmol) were added to a 50 mL reaction flask. The mixture was reacted at room temperature for 0.5 h. The reaction was quenched by adding water and stirring. The reaction liquid was extracted with ethyl acetate, dried over anhydrous sodium sulfate, concentrated under reduced pressure, and separated by column chromatography to give a product (9.6 mg), m/z = 544 $[M+1]^+$, $^1H$ NMR (400 MHz, DMSO-*d6*) $\delta$ 12.65 (s, 1H), 8.59 (s, 1H), 8.51 (s, 1H), 8.04 (d, 1H), 7.86 (d, 1H), 7.58 (s, 1H), 7.25 (s, 1H), 7.08 (d, 1H), 5.02 (br, 1H), 4.51-4.54 (m, 1H), 4.31-4.34 (m, 1H), 4.18 (q, 2H), 3.80-3.82 (m, 1H), 3.50-3.53 (m, 1H), 2.95-3.01 (m, 1H), 2.73-2.79 (m, 1H), 2.54-2.56 (m, 1H), 2.30-2.33 (m, 2H), 1.78-1.90 (m, 3H), 1.48-1.38 (m, 4H).

**Example 19: Preparation of compound 42**

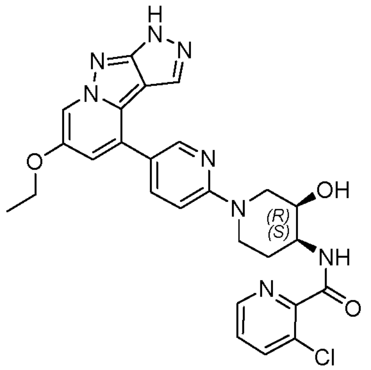

((3*R*,4*S*)-1-(5-(6-ethoxy-1*H*-pyrazolo[3',4':3,4]pyrazolo[1,5-*a*]pyridin-4-yl)pyridin-2-yl)-3-hydroxy piperidin-4-yl)amine hydrochloride (200 mg, 0.4 mmol), 3-chloro-2-pyridinecarboxylic acid (74 mg, 0.47 mmol), DIEA (166 mg, 1.29 mmol), HATU (179 mg, 0.47 mmol), and 2.5 mL of DMF were added to a reaction flask. The mixture was reacted at 25 °C for 1 h. The reaction was quenched by adding water and stirring. The reaction liquid was extracted with ethyl acetate, dried over anhydrous sodium sulfate, concentrated under reduced pressure, and separated by column chromatography to give a product (26 mg), m/z = 533 $[M+1]^+$, $^1H$ NMR (400 MHz, DMSO-*d6*) $\delta$ 12.64 (s, 1H), 8.54-8.57 (m, 3H), 8.51 (s, 1H), 8.00-8.04 (m, 2H), 7.54-7.59 (m, 2H), 8.19 (dd, 1H), 7.25 (s, 1H), 7.08 (d, 1H), 5.11 (s, 1H), 4.27-4.38 (m, 2H), 4.13-4.17 (m, 3H), 3.91-3.94 (m, 1H), 3.15-3.20 (m, 1H), 1.90-1.98 (m, 1H), 1.73-1.78 (m, 1H), 1.42-1.45 (m, 3H).

**Example 20: Preparation of compound 43**

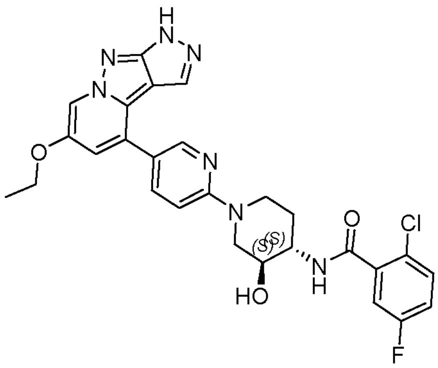

(3*S*,4*S*)-4-amino-1-(5-(6-ethoxy-1*H*-pyrazolo[3',4':3,4]pyrazolo[1,5-*a*]pyridin-4-yl)pyridin-2-yl)pip eridin-3-ol (256.5 mg, 0.51 mmol), DIEA (263 mg, 2.04 mmol) and DCM (10 mL), and 2-chloro-5-fluorobenzoyl chloride (98 mg, 0.51 mmol) were added to a 50mL reaction flask. The mixture was reacted at 0 °C for 0.5 h. The reaction was quenched by adding water and stirring. The reaction liquid was extracted with ethyl acetate. The organic phase was dried over anhydrous sodium sulfate, concentrated under reduced pressure, and separated by column chromatography to give a

product (24 mg), m/z = 550 [M+1]+. $^1$H-NMR (400 MHz, DMSO-$d_6$) δ 12.64 (brs, 1H), 8.57 (s, 1H), 8.52 (s, 1H), 8.46 (d, *J* = 8.4 Hz, 1H), 8.07 (m, 1H), 7.53-7.59 (m, 2H), 7.39-7.41 (m, 1H), 7.30-7.35 (m, 2H), 7.11-7.21 (m, 1H), 7.45-7.47 (m, 1H), 4.20-4.29 (m, 1H), 4.14-4.18 (m, 2H), 3.90-3.93 (m, 1H), 3.52-3.55 (m, 2H), 3.14-3.20 (m, 1H), 2.93-2.96 (m, 1H), 1.99-2.02 (m, 1H), 1.49-1.51 (m, 1H), 1.39 (t, *J* = 7.2 Hz, 3H).

**Example 21: Preparation of compound 44**

Step A: *tert*-butyl ((3*S*,4*S*)-1-(5-(6-ethoxy-1*H*-pyrazolo[3',4':3,4]pyrazolo[1,5-*a*]pyridin-4-yl)pyridin-2-yl)-3-hydroxy piperidin-4-yl)carbamate

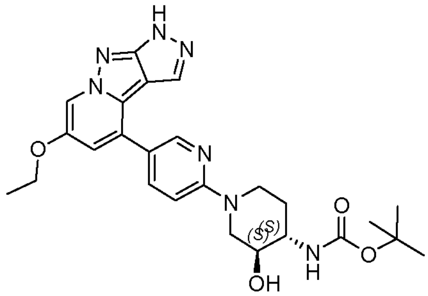

6-ethoxy-4-(6-fluoropyridin-3-yl)-1*H*-pyrazolo[3',4':3,4]pyrazolo[1,5-*a*]pyridine (2 g, 6.73 mmol), tert-butyl ((3*S*,4*S*)-3-hydroxypiperidin-4-yl)carbamate (1.46 g, 6.73 mmol), 10 mL of DMSO, and DIEA (2.61 g, 20.2 mmol) were added to a 50 mL reaction flask at room temperature. The mixture was stirred at 90 °C for 12 h. The reaction was quenched by adding water. The reaction liquid was extracted with ethyl acetate, dried over anhydrous sodium sulfate, concentrated under reduced pressure, and separated by column chromatography to give a product (2.7 g), m/z = 494 [M+1]+.

Step B: (3*S*,4*S*)-4-amino-1-(5-(6-ethoxy-1*H*-pyrazolo[3',4':3,4]pyrazolo[1,5-*a*]pyridin-4-yl)pyridin-2-yl)pip eridin-3-ol

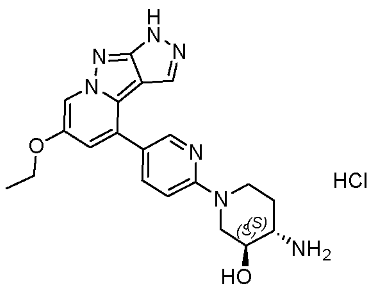

*Tert*-butyl

((3*S*,4*S*)-1-(5-(6-ethoxy-1*H*-pyrazolo[3',4':3,4]pyrazolo[1,5-*a*]pyridin-4-yl)pyridin-2-yl)-3-hydroxy piperidin-4-yl)carbamate (2.7 g, 5.45 mmol) and a solution of hydrochloric acid in 1,4-dioxane (4 M) were sequentially added to a reaction flask. The mixture was stirred for 3 h. The reaction was quenched by adding water and stirring. The reaction liquid was extracted with ethyl acetate. The organic phase was dried over anhydrous sodium sulfate, concentrated under reduced pressure, and separated by column chromatography to give a product (2.7 g), m/z = 394 [M+1]+.

Step C: 2-chloro-*N*-((3*S*,4*S*)-1-(5-(6-ethoxy-1*H*-pyrazolo[3',4':3,4]pyrazolo[1,5-*a*]pyridin-4-yl)pyridin-2-yl) -3-hydroxypiperidin-4-yl)-6-fluorobenzamide

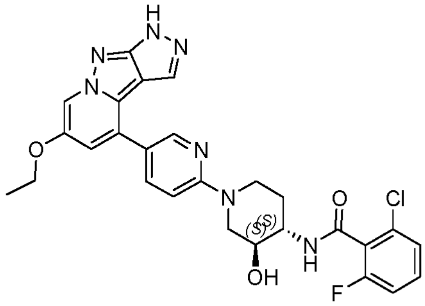

(3*S*,4*S*)-4-amino-1-(5-(6-ethoxy-1*H*-pyrazolo[3',4':3,4]pyrazolo[1,5-*a*]pyridin-4-yl)pyridin-2-yl)pip eridin-3-ol (256.5 mg, 0.51 mmol), DIEA (263 mg, 2.04 mmol) and DCM (10 mL), and 2-chloro-6-fluorobenzoyl chloride (98 mg, 0.51 mmol) were added to a 50 mL reaction flask. The mixture was reacted at 0 °C for 0.5 h. The reaction was quenched by adding water and stirring. The reaction liquid was extracted with ethyl acetate. The organic phase was dried over anhydrous sodium sulfate, concentrated under reduced pressure, and separated by column chromatography to give a product (26 mg), m/z = 550 $[M+1]^+$, $^1H$ NMR (400 MHz, DMSO-*d6*) δ 12.65 (s, 1H), 8.69 (d, 1H), 8.58 (s, 1H), 8.57 (s, 1H), 8.02-8.05 (m, 1H), 7.58 (s, 1H), 7.07-7.49 (m, 5H), 5.07 (br, 1H), 4.40-4.44 (m, 1H), 4.14-4.22 (m, 3H), 3.92-3.95 (m, 1H), 3.50-3.51 (m, 1H), 3.16-3.22 (m, 1H), 2.96-3.01 (m, 1H), 2.01-2.11 (m, 1H), 1.37-1.48 (m, 4H).

**Example 22: Preparation of compound 45**

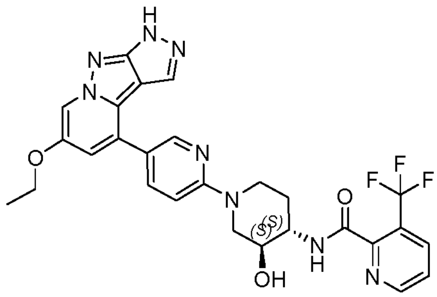

(3*S*,4*S*)-4-amino-1-(5-(6-ethoxy-1*H*-pyrazolo[3',4':3,4]pyrazolo[1,5-*a*]pyridin-4-yl)pyridin-2-yl)pip eridin-3-ol (256.5 mg, 0.51 mmol), DIEA (263 mg, 2.04 mmol) and DCM (10 mL), and 3-(trifluoromethyl)pyridine-2-carbonyl chloride (107 mg, 0.51 mmol) were added to a 50 mL reaction flask. The mixture was reacted at 0 °C for 0.5 h. The reaction was quenched by adding water and stirring. The reaction liquid was extracted with ethyl acetate, dried over anhydrous sodium sulfate, concentrated under reduced pressure, and separated by column chromatography to give a product (45 mg), m/z = 567 $[M+1]^+$, $^1H$-NMR (400 MHz, DMSO-$d_6$) δ 12.65 (brs, 1H), 8.85-8.86 (d, *J* = 4.0 Hz, 1H), 8.62-8.64 (d, *J* = 8.0 Hz, 1H), 8.58 (s, 1H), 8.57 (s, 1H), 8.29-8.31 (m, 1H), 8.04-8.07 (m, 1H), 7.72-7.75 (m, 1H), 7.59 (s, 1H), 7.26 (s, 1H), 7.09-7.11 (m, 1H), 5.10 (s, 1H), 4.46-4.50 (m, 1H), 4.26-4.29 (m, 1H), 4.14-4.20 (m, 2H), 3.92-3.96 (m, 1H), 3.54-3.59 (m, 1H), 3.10-3.16 (m, 1H), 2.90-2.95 (m, 1H), 2.01-2.05 (m, 1H), 1.49-1.52 (m, 1H), 1.39 (t, *J* = 6.8 Hz, 3H).

**Example 23: Preparation of compound 46**

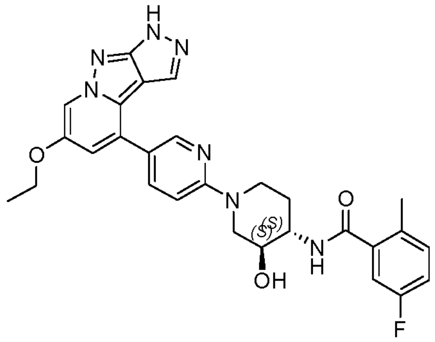

(3*S*,4*S*)-4-amino-1-(5-(6-ethoxy-1*H*-pyrazolo[3',4':3,4]pyrazolo[1,5-*a*]pyridin-4-yl)pyridin-2-yl)pip eridin-3-ol (256.5 mg, 0.51 mmol), DIEA (263 mg, 2.04 mmol) and DCM (10 mL), and 2-methyl-5-fluorobenzoyl chloride (78 mg, 0.51 mmol) were added to a 50 mL reaction flask. The mixture was reacted at 0 °C for 0.5 h. The reaction was quenched by adding water and stirring. The reaction liquid was extracted with ethyl acetate, dried over anhydrous sodium sulfate, concentrated under reduced pressure, and separated by column chromatography to give a product (33 mg), m/z = 530 $[M+1]^+$, $^1$H NMR (400 MHz, DMSO-*d6*) δ 12.65 (s, 1H), 8.58 (s, 1H), 8.57 (s, 1H), 8.21 (d, 1H), 8.04-8.06 (m, 1H), 7.59 (s, 1H), 7.09-7.28 (m, 5H), 5.15 (br, 1H), 4.50-4.54 (m, 1H), 4.31-4.35 (m, 3H), 4.17 (q, 2H), 3.91-3.93 (m, 1H), 3.50-3.54 (m, 1H), 3.04-3.10 (m, 1H), 2.82-2.88 (m, 1H), 2.32 (s, 3H), 1.96-1.98 (m, 1H), 1.23-1.52 (m, 4H).

**Example 24: Preparation of compound 48**

Step A: *tert*-butyl ((3*S*,4*R*)-1-(5-(6-ethoxy-1*H*-pyrazolo[3',4':3,4]pyrazolo[1,5-*a*]pyridin-4-yl)pyridin-2-yl)-4-hydroxy piperidin-3-yl)aminocarbonate

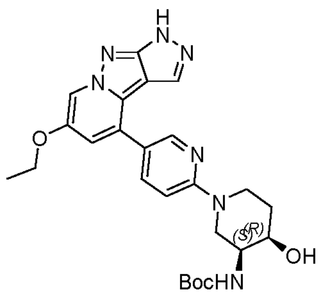

6-ethoxy-4-(6-fluoropyridin-3-yl)-1*H*-pyrazolo[3',4':3,4]pyrazolo[1,5-*a*]pyridine (1.25 g, 4.22 mmol), DMSO (12 mL), DIEA (1.63 g, 12.66 mmol), and *tert*-butyl ((3*S*,4*R*)-4-hydroxypiperidin-3-yl)aminocarbonate (1.0 g, 4.64 mmol) were added to a reaction flask. The mixture was heated to 90-95 °C and stirred for 24 h. The reaction was quenched by adding water and stirring. The reaction was extracted with ethyl acetate. The organic phase was dried over anhydrous sodium sulfate, concentrated under reduced pressure, and separated by column chromatography to give a product (1.2 g), m/z = 494 $[M+1]^+$.

Step B: *tert*-butyl ((3*S*,4*S*)-4-(1,3-phthalimido-2-yl)-1-(5-(6-ethoxy-1*H*-pyrazolo[3',4':3,4]pyrazolo[1,5-*a*]pyridin-4-yl )pyridin-2-yl)piperidin-3-yl)aminocarbonate

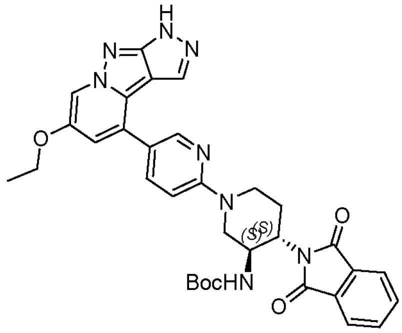

*Tert*-butyl

((3*S*,4*R*)-1-(5-(6-ethoxy-1*H*-pyrazolo[3',4':3,4]pyrazolo[1,5-*a*]pyridin-4-yl)pyridin-2-yl)-4-hydroxy piperidin-3-yl)aminocarbonate (1.1 g, 2.22 mmol), THF (10 mL), phthalimide (0.65 g, 4.45 mmol), and triphenylphosphine (1.2 g, 4.45 mmol) were added to a reaction flask. The mixture was stirred at 0-10 °C for 30 min, and then a solution of DEAD (0.77 g, 4.45 mmol) in THF (1 mL) was added dropwise. The mixture was warmed to room temperature and reacted for 20 h. The reaction was quenched by adding water and stirring. The reaction liquid was extracted with ethyl acetate, dried over anhydrous sodium sulfate, concentrated under reduced pressure, and separated by column chromatography to give a product (300 mg), m/z = 623 $[M+1]^+$.

Step C: *tert*-butyl ((*S*,4*S*)-4-amino-1-(5-(6-ethoxy-1*H*-pyrazolo[3',4':3,4]pyrazolo[1,5-*a*]pyridin-4-yl)pyridin-2-yl)pipe ridin-3-yl)aminocarbonate

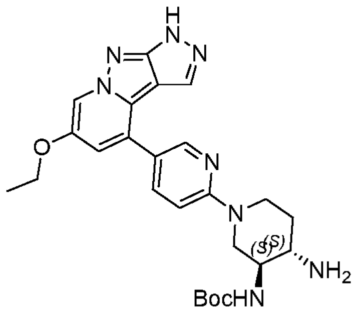

*Tert*-butyl

((3*S*,4*S*)-4-(1,3-phthalimido-2-yl)-1-(5-(6-ethoxy-1*H*-pyrazolo[3',4':3,4]pyrazolo[1,5-*a*]pyridin-4-yl )pyridin-2-yl)piperidin-3-yl)aminocarbonate (300 mg, 0.48 mmol), ethanol (2 mL), and hydrazine hydrate (1 mL) were added to a reaction flask. The mixture was heated to reflux for 1 h. The reaction was quenched by adding water and stirring. The reaction liquid was extracted with ethyl acetate. The organic phase was dried over anhydrous sodium sulfate, concentrated under reduced pressure, and separated by column chromatography to give a product (100 mg), m/z = 493 $[M+1]^+$.

Step D: *N*-((3*S*,4*S*)-3-amino-1-(5-(6-ethoxy-1*H*-pyrazolo[3',4':3,4]pyrazolo[1,5-*a*]pyridin-4-yl)pyridin-2-yl) piperidin-4-yl)-2-chloro-6-methylbenzamide

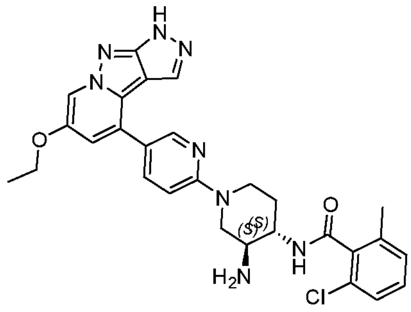

2-methyl-6-chlorobenzoic acid (51.7 mg, 0.3 mmol) and dichloromethane (2 mL) were added to a reaction flask A. The mixture was cooled to 0-10 °C, and then thionyl chloride (36.2 mg, 0.3 mmol) and 1 drop of DMF were added. The mixture was naturally warmed to room temperature and stirred for 2 h for later use.

*Tert*-butyl

((3*S*,4*S*)-4-amino-1-(5-(6-ethoxy-1*H*-pyrazolo[3',4':3,4]pyrazolo[1,5-*a*]pyridin-4-yl)pyridin-2-yl)pi peridin-3-yl)aminocarbonate (100 mg, 0.2 mmol), 2 mL of dichloromethane, and triethylamine (41 mg, 0.4 mmol) were added to a reaction flask B. The mixture was cooled to 0-10 °C. The reaction liquid in the reaction flask A was slowly added dropwise to the reaction flask B. After the dropwise addition, the resulting mixture was stirred at room temperature for 2 h. After the reaction was completed, 2 mL of water was added for liquid separation. The organic phase was collected and concentrated to dryness, and then 2 mL of methanol was added until complete dissolution was achieved. The solution was cooled to 0-10 °C, and 20% of solution of HCl in methanol was added. The mixture was naturally warmed to room temperature and stirred for 20 h. After the reaction was completed, the reaction liquid was concentrated to dryness, and 5 mL of water was added until complete dissolution was achieved. The solution was extracted twice with ethyl acetate. The aqueous phase was collected, adjusted to pH 10, and extracted twice with dichloromethane. The organic phase was collected and purified by column chromatography to give a product (18 mg), $^1$HNMR (400 MHz, DMSO-*d6*) δ 12.65 (s, 1H), 8.46-8.59 (m, 3H), 8.05-8.08 (m, 1H), 7.55-7.64 (m, 2H), 7.22-7.33 (m, 4H), 7.10-7.12 (d, 1H), 4.50-4.53 (m, 1H), 4.32 (m, 1H), 4.15-4.20 (m, 2H), 3.94 (m, 1H), 3.30 (m, 1H), 3.12 (m, 1H), 2.80-2.91 (m, 1H), 2.31-2.33 (m, 3H), 2.06-2.08 (m, 1H), 1.50-1.58 (m, 1H), 1.34-1.42 (m, 3H), 1.24 (s, 1H), m/z = 545.2 [M+1]$^+$.

**Example 25: Preparation of compound 50**

Step A: *tert*-butyl (3*S*,4*S*)-1-(5-(6-ethoxy-1-(tetrahydro-2*H*-pyran-2-yl)-1*H*-pyrazolo[3',4':3,4]pyrazolo[1,5-*a*]pyridin-4-yl)pyrazin-2-yl)-3-hydroxypiperidin-4-yl)aminocarbonate

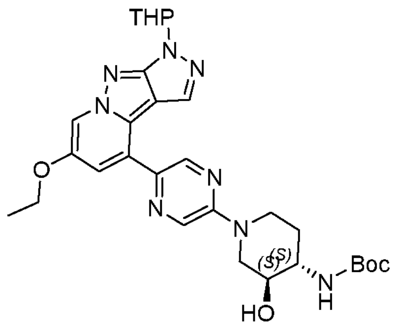

4-(5-chloropyrazin-2-yl)-6-ethoxy-1-(tetrahydro-2*H*-pyran-2-yl)-1*H*-pyrazolo[3',4':3,4]pyrazolo[1,5 -a]pyridine (500 mg, 1.25 mmol), *tert*-butyl ((3*S*,4*S*)-3-hydroxypiperidin-4-yl)aminocarbonate (542 mg, 2.5 mmol), DIEA (484 mg, 3.75 mmol), and 5 mL of DMSO were added to a reaction flask. The mixture was reacted at 80 °C overnight. After the reaction was completed, the reaction was quenched by adding water and stirring. The reaction liquid was extracted with dichloromethane, dried over anhydrous sodium sulfate, concentrated under reduced pressure, and separated by column chromatography to give a product (670 mg), m/z = 579.3 [M+1]$^+$.

Step B: (3*S*,4*S*)-4-amino-1-(5-(6-ethoxy-1*H*-pyrazolo[3',4':3,4]pyrazolo[1,5-*a*]pyridin-4-yl)pyrazin-2-yl)-3-hydroxypiperidine hydrochloride

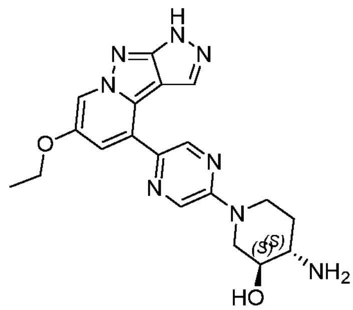

*Tert*-butyl

(3*S*,4*S*)-1-(5-(6-ethoxy-1-(tetrahydro-2*H*-pyran-2-yl)-1*H*-pyrazolo[3',4':3,4]pyrazolo[1,5-*a*]pyridin-4-yl)pyrazin-2-yl)-3-hydroxypiperidin-4-yl)aminocarbonate (670 mg, 1.1 mmol) and a 5 N methanolic hydrochloric acid solution (10 mL, 50 mmol) were added to a reaction flask. The mixture was reacted at 25 °C for 2 h. After the reaction was completed, the reaction liquid was directly concentrated under reduced pressure to dryness to give a product (710 mg), m/z = 395.2 $[M+1]^+$.

Step C: 3-chloro-*N*-((3*S*,4*S*)-(1-(5-(6-ethoxy-1*H*-pyrazolo[3',4':3,4]pyrazolo[1,5-*a*]pyridin-4-yl)pyrazin-2-yl )-3-hydroxypiperidin-4-yl)-2-picolinamide

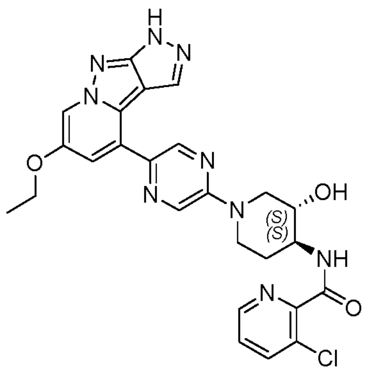

(3*S*,4*S*)-4-amino-1-(5-(6-ethoxy-1*H*-pyrazolo[3',4':3,4]pyrazolo[1,5-*a*]pyridin-4-yl)pyrazin-2-yl)-3-hydroxypiperidine hydrochloride (80 mg, 0.16 mmol), 3-chloropyridine-2-formic acid (38 mg, 0.24 mmol), DIEA (103 mg, 0.8 mmol), HATU (61 mg, 0.16 mmol), and 2 mL of DMF were added to a reaction flask. The mixture was reacted at 25 °C for 2 h. After the reaction was completed, the reaction was quenched by adding water and stirring, extracted with dichloromethane, dried over anhydrous sodium sulfate, concentrated under reduced pressure, and separated by column chromatography to give a product (37.6 mg), m/z = 534.1 $[M+1]^+$, $^1H$ NMR (400 MHz, DMSO-$d_6$) δ 12.45 (brs, 1H), 8.90 (s, 1H), 8.74 (d, *J* = 7.6Hz, 1H), 8.71 (s, 1H), 8.55 (s, 1H), 8.01 (s, 1H), 7.72 (s, 1H), 7.47 (dd, $J_1$ = 8.0Hz, $J_2$ = 6.0Hz, 1H), 7.36 (d, *J* = 8.0Hz, 1H), 7.28 (t, *J* = 8.8Hz, 1H), 5.01-5.42 (m, 1H), 4.36-4.41 (m, 1H), 4.17-4.24 (m, 2H), 3.95-4.02 (m, 1H), 3.62-3.71 (m, 2H), 3.12-3.21 (m, 2H), 2.10-2.18 (m, 1H), 1.48-1.60 (m, 1H), 1.39-1.43 (m, 3H).

**Example 26: Preparation of compound 51**

Step A: 2-chloro-*N*-((3*S*,4*S*)-(1-(5-(6-ethoxy-1*H*-pyrazolo[3',4':3,4]pyrazolo[1,5-*a*]pyridin-4-yl)pyrazin-2-yl )-3-hydroxypiperidin-4-yl)-6-fluorobenzamide

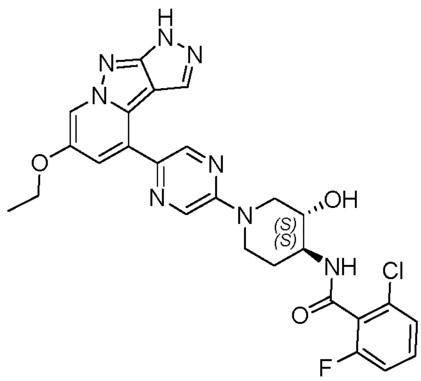

(3*S*,4*S*)-4-amino-1-(5-(6-ethoxy-1*H*-pyrazolo[3',4':3,4]pyrazolo[1,5-*a*]pyridin-4-yl)pyrazin-2-yl)-3-hydroxypiperidine hydrochloride (80 mg, 0.16 mmol), 2-chloro-6-fluorobenzoyl chloride (46 mg, 0.24 mmol), DIEA (103 mg, 0.8 mmol), and 2 mL of DMF were added to a reaction flask. The mixture was reacted at 25 °C for 1 h. After the reaction was completed, the reaction was quenched by adding water and stirring. The reaction liquid was extracted with dichloromethane, dried over anhydrous sodium sulfate, concentrated under reduced pressure, and separated by column chromatography to give a product (4.6 mg), m/z = 551.1 $[M+1]^+$. $^1H$ NMR (400 MHz, DMSO-$d_6$) δ 12.54 (brs, 1H), 8.91 (s, 1H), 8.72 (s, 1H), 8.59 (d, *J* = 8.4Hz, 1H), 8.55 (s, 1H), 8.04 (s, 1H), 8.02 (s, 1H), 7.73 (s, 1H), 7.52 (dd, $J_1$ = 8.4Hz, $J_2$ = 4.8Hz, 1H), 5.01-5.28 (m, 1H), 4.44-4.52 (m, 1H), 4.25-4.33 (m, 1H), 4.17 (q, *J* = 6.8Hz, 1H), 3.96-4.02 (m, 1H), 3.58-3.67 (m, 1H), 3.30-3.35 (m, 1H), 3.03-3.11 (m, 1H), 2.10-2.18 (m, 2H), 1.56-1.68 (m, 1H), 1.39 (t, *J* = 6.8Hz, 3H).

**Example 27: Preparation of compound 55**

Step A: 4-bromo-6-ethoxy-1-(tetrahydro-2*H*-pyran-2-yl)-1*H*-pyrazolo[3',4':3,4]pyrazolo[1,5-*a*]pyridine

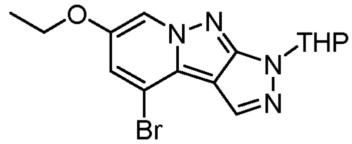

5 mL of dihydropyran was added to a solution of 4-bromo-6-hydroxy-1*H*-pyrazolo[3',4':3,4]pyrazolo[1,5-*a*]pyridine (2.0 g, 7.1 mmol) in tetrahydrofuran (20 mL), and then *p*-toluenesulfonic acid (245 mg, 1.4 mmol) was added. The mixture was heated to 50 °C and reacted for 12 h. Water was added, and the resulting mixture was extracted with ethyl acetate. The organic phases were combined, washed with water, dried over anhydrous sodium sulfate, filtered, concentrated under reduced pressure, and separated by column chromatography to give a product, m/z = 365 $[M+1]^+$.

Step B: 6-ethoxy-1-(tetrahydro-2*H*-pyran-2-yl)-4-(4,4,5,5-tetramethyl-1,3,2-dioxaborolan-2-yl)-1*H*-pyrazol o[3',4':3,4]pyrazolo[1,5-*a*]pyridine

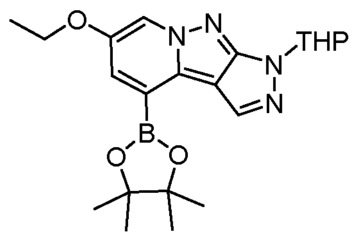

4-bromo-6-ethoxy-1-(tetrahydro-2*H*-pyran-2-yl)-1*H*-pyrazolo[3',4':3,4]pyrazolo[1,5-*a*]pyridine (5.4 g, 14.8 mmol), bis(pinacolato)diboron (11.3 g, 44.5 mmol), potassium acetate (5.8 g, 59.3 mmol), and 100 mL of 1,4-dioxane

were added to a reaction flask, and then Pd(dppf)$Cl_2$ (1.0 g, 1.5 mmol) was added. The mixture was purged with nitrogen and reacted at 80 °C for 12 h. The reaction liquid was filtered, and the filtrate was concentrated to give a product (5.0 g), m/z = 413 $[M+1]^+$.

Step C: 4-(5-chloropyrazin-2-yl)-6-ethoxy-1-(tetrahydro-2*H*-pyran-2-yl)-1*H*-pyrazolo[3',4':3,4]pyrazolo[1,5 -*a*]pyridine

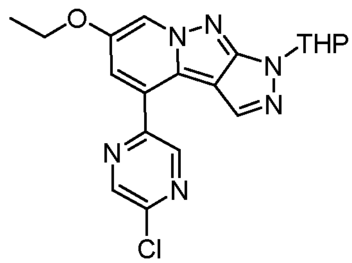

6-ethoxy-1-(tetrahydro-2*H*-pyran-2-yl)-4-(4,4,5,5-tetramethyl-1,3,2-dioxaborolan-2-yl)-1*H*-pyrazol o[3',4':3,4]pyrazolo[1,5-*a*]pyridine (4.5 g, 10.9 mmol), 2,5-dichloropyrazine (4.8 g, 32.6 mmol), potassium carbonate (4.5 g, 32.7 mmol), 1,4-dioxane (50 mL) and water (5 mL) were added to a reaction flask, and then Pd(dppf)$Cl_2$ (1.0 g, 1.5 mmol) was added. The mixture was purged with nitrogen and reacted at 80 °C for 4 h. Water was added, and the resulting mixture was extracted with ethyl acetate. The organic phases were combined, washed with water, dried over anhydrous sodium sulfate, filtered, concentrated under reduced pressure, and separated by column chromatography to give a product (2.5 g), m/z = 399 $[M+1]^+$.

Step D: *tert*-butyl (1-(5-(6-ethoxy-1-(tetrahydro-2*H*-pyran-2-yl)-1*H*-pyrazolo[3',4':3,4]pyrazolo[1,5-*a*]pyridin-4-yl)py razin-2-yl)-4-methylpiperidin-4-yl)aminocarbonate

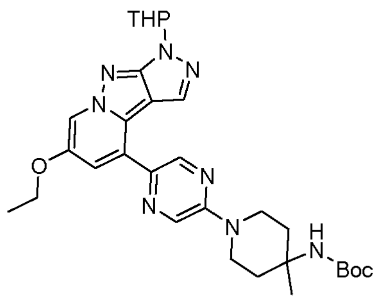

4-(5-chloropyrazin-2-yl)-6-ethoxy-1-(tetrahydro-2*H*-pyran-2-yl)-1*H*-pyrazolo[3',4':3,4]pyrazolo[1,5 -*a*]pyridine (1.0 g, 2.5 mmol), *tert*-butyl (4-methylpiperidin-4-yl)carbonate (1.0 g, 5.0 mmol), DIEA (972 mg, 7.5 mmol), and 12 mL of DMSO were added to a reaction flask. The mixture was reacted at 80 °C for 12 h. Water was added, and the resulting mixture was extracted with ethyl acetate, dried over anhydrous sodium sulfate, filtered, concentrated under reduced pressure, and separated by column chromatography to give a product (1.3 g), m/z = 577 $[M+1]^+$.

Step E: (1-(5-(6-ethoxy-1*H*-pyrazolo[3',4':3,4]pyrazolo[1,5-*a*]pyridin-4-yl)pyrazin-2-yl)-4-methylpiperidin-4-amine hydrochloride

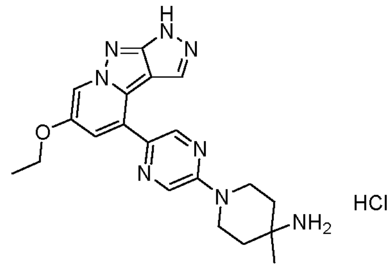

*Tert*-butyl

(1-(5-(6-ethoxy-1-(tetrahydro-2*H*-pyran-2-yl)-1*H*-pyrazolo[3',4':3,4]pyrazolo[1,5-*a*]pyridin-4-yl)py razin-2-yl)-4-methylpiperidin-4-yl)aminocarbonate (1.25 g, 2.2 mmol) and a 5 N methanolic hydrochloric acid solution (10 mL, 50 mmol) were added to a reaction flask. The mixture was reacted at 25 °C for 2 h. The reaction liquid was concentrated under reduced pressure to dryness to give a product (1.1 g), m/z = 393 $[M+1]^+$.

Step F: 3-chloro-*N*-(1-(5-(6-ethoxy-1*H*-pyrazolo[3',4':3,4]pyrazolo[1,5-*a*]pyridin-4-yl)pyrazin-2-yl)-4-meth ylpiperidin-4-yl)-5-fluoro-2-picolinamide

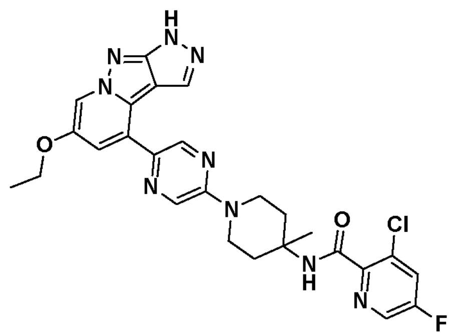

(1-(5-(6-ethoxy-1*H*-pyrazolo[3',4':3,4]pyrazolo[1,5-*a*]pyridin-4-yl)pyrazin-2-yl)-4-methylpiperidin-4-amine hydrochloride (150 mg, 0.3 mmol), 3-chloro-5-fluoropyridine-2-carboxylic acid (63 mg, 0.36 mmol), DIEA (193 mg, 1.5 mmol), HATU (114 mg, 0.3 mmol), and 2 mL of DMF were added to a reaction flask. The mixture was reacted at 25 °C for 2 h. Water was added, and the resulting mixture was extracted with ethyl acetate. The organic phases were combined, washed with water, dried over anhydrous sodium sulfate, filtered, concentrated under reduced pressure, and separated by column chromatography to give a product (11 mg), m/z = 550 $[M+1]^+$.

**Example 28: Preparation of compound 56**

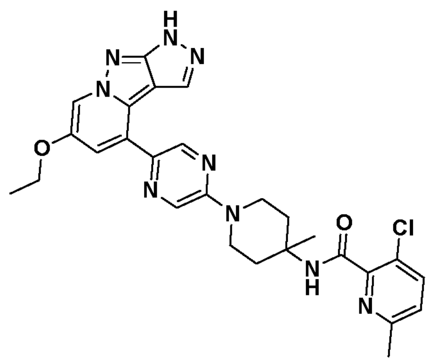

(1-(5-(6-ethoxy-1*H*-pyrazolo[3',4':3,4]pyrazolo[1,5-*a*]pyridin-4-yl)pyrazin-2-yl)-4-methylpiperidin-4-amine hydrochloride (150 mg, 0.3 mmol), 3-chloro-5-fluoropyridine-2-carboxylic acid (63 mg, 0.36 mmol), DIEA (193 mg, 1.5 mmol), HATU (114 mg, 0.3 mmol), and 2 mL of DMF were added to a reaction flask. The mixture was reacted at 25 °C for 2 h. Water was added, and the resulting mixture was extracted with ethyl acetate. The organic phases were combined, washed with water, dried over anhydrous sodium sulfate, filtered, concentrated under reduced pressure, and separated by column chromatography to give a product (13 mg), m/z = 546 $[M+1]^+$.

**Example 29: Preparation of compound 57**

Step A: *N*-(1-(5-(6-ethoxy-1*H*-pyrazolo[3',4':3,4]pyrazolo[1,5-*a*]pyridin-4-yl)pyrazin-2-yl)-4-methylpiperidi n-4-yl)-2,3,6-trifluorobenzamide

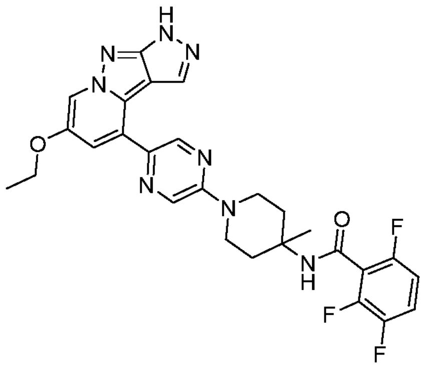

(1-(5-(6-ethoxy-1*H*-pyrazolo[3',4':3,4]pyrazolo[1,5-*a*]pyridin-4-yl)pyrazin-2-yl)-4-methylpiperidine -4-amine hydrochloride (100 mg, 0.2 mmol), 2,3,6-trifluorobenzoic acid (39 mg, 0.22 mmol), DIEA (129 mg, 1.0 mmol), HATU (76 mg, 0.2 mmol), and 2 mL of DMF were added to a reaction flask. The mixture was reacted at 25 °C for 2 h. After the reaction was completed, the reaction was quenched by adding water and stirring. The reaction liquid was extracted with dichloromethane, dried over anhydrous sodium sulfate, concentrated under reduced pressure, and separated by column chromatography to give a product (24.6 mg), m/z = 551.1 $[M+1]^+$. $^1H$ NMR (400 MHz, DMSO-$d_6$) $\delta$ 12.47 (brs, 1H), 8.91 (s, 1H), 8.73 (s, 1H), 8.60 (s, 1H), 8.55 (d, *J* = 2.0Hz, 1H), 8.02 (s, 1H), 7.72 (s, 1H), 7.55-7.63 (m, 1H), 7.21-7.26 (m, 1H), 4.16-4.24 (m, 4H), 3.30-3.35 (m, 2H), 1.62-1.66 (m, 2H), 1.39-1.44 (m, 6H).

**Example 30: Preparation of compound 58**

Step A: 2-chloro-*N*-(1-(5-(6-ethoxy-1*H*-pyrazolo[3',4':3,4]pyrazolo[1,5-*a*]pyridin-4-yl)pyrazin-2-yl)-4-meth ylpiperidin-4-yl)-5-fluorobenzamide

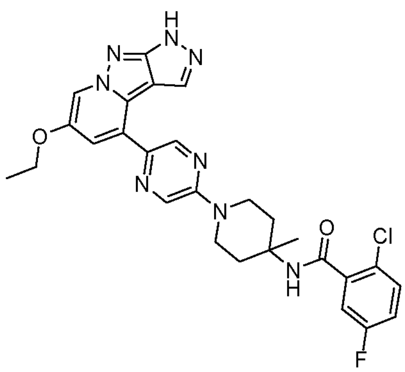

(1-(5-(6-ethoxy-1*H*-pyrazolo[3',4':3,4]pyrazolo[1,5-*a*]pyridin-4-yl)pyrazin-2-yl)-4-methylpiperidine -4-amine hydrochloride (150 mg, 0.3 mmol), 2-chloro-5-fluorobenzoyl chloride (63 mg, 0.36 mmol), DIEA (193 mg, 1.5 mmol), HATU

(76 mg, 0.2 mmol), and 2 mL of DMF were added to a reaction flask. The mixture was reacted at 25 °C for 1 h. After the reaction was completed, the reaction was quenched by adding water and stirring. The reaction liquid was extracted with dichloromethane, dried over anhydrous sodium sulfate, concentrated under reduced pressure, and separated by column chromatography to give a product (43.6 mg), m/z = 549.2 $[M+1]^+$. $^1H$ NMR (400 MHz, $CD_3OD$-$d_4$) δ 8.75 (s, 1H), 8.54 (s, 1H), 8.33 (s, 1H), 8.00 (s, 1H), 7.62 (s, 1H), 7.46-7.49 (m, 1H), 7.17-7.26 (m, 2H), 4.15-4.25 (m, 4H), 3.48-3.55 (m, 2H), 2.44-2.48 (m, 2H), 1.70-1.77 (m, 2H), 1.55 (s, 3H), 1.49 (t, *J* = 6.8Hz, 3H).

**Example 31: Preparation of compound 59**

Step A: 3-chloro-*N*-(1-(5-(6-ethoxy-1*H*-pyrazolo[3',4':3,4]pyrazolo[1,5-*a*]pyridin-4-yl)pyrazin-2-yl)-4-meth ylpiperidin-4-yl)-2-picolinamide

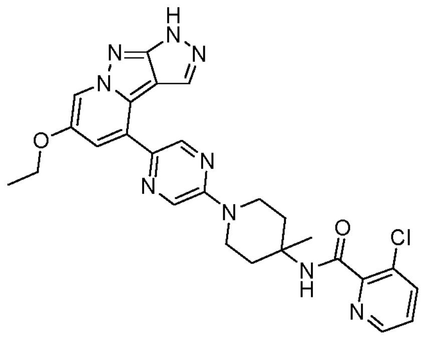

(1-(5-(6-ethoxy-1*H*-pyrazolo[3',4':3,4]pyrazolo[1,5-*a*]pyridin-4-yl)pyrazin-2-yl)-4-methylpiperidine -4-amine hydrochloride (100 mg, 0.2 mmol), 3-chloro-2-pyridinebenzoic acid (39 mg, 0.22 mmol), DIEA (129 mg, 1.0 mmol), HATU (76 mg, 0.2 mmol), and 2 mL of DMF were added to a reaction flask. The mixture was reacted at 25 °C for 2 h. After the reaction was completed, the reaction was quenched by adding water and stirring. The reaction liquid was extracted with dichloromethane, dried over anhydrous sodium sulfate, concentrated under reduced pressure, and separated by column chromatography to give a product (43.5 mg), m/z = 532.1 $[M+1]^+$. $^1H$ NMR (400 MHz, DMSO-$d_6$) δ 12.47 (brs, 1H), 8.91 (s, 1H), 8.72 (s, 1H), 8.52-8.55 (m, 2H), 8.35 (s, 1H), 8.01-8.03 (m, 2H), 7.72 (s, 1H), 7.50 (dd, $J_1$ = 8.0Hz, $J_2$ = 0.8Hz, 1H), 4.16-4.22 (m, 4H), 3.34-3.39 (m, 2H), 2.35-2.39 (m, 2H), 1.60-1.66 (m, 2H), 1.56 (s, 3H), 1.39 (t, *J* = 6.8Hz, 3H).

**Example 32: Preparation of compound 87**

1-(5-(6-ethoxy-1*H*-pyrazolo[3',4':3,4]pyrazolo[1,5-*a*]pyridin-4-yl)pyridin-2-yl)-4-(hydroxymethyl)-N-isobutylpiperidine-4-carboxamide (compound APS03087)

Step A: methyl 1-(5-bromopyridin-2-yl)piperidine-4-carboxylate

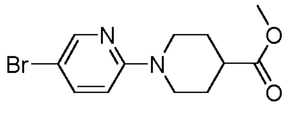

A solution of HCl in 1,4-dioxane (4 M, 4 mL) was added to 1-(*tert*-butoxycarbonyl)-4-(methoxycarbonyl)piperidine-4-carboxylic acid (1.9 g, 6.6 mmol). The mixture was stirred at room temperature for 1 h. The reaction liquid was concentrated by rotary evaporation, and DMSO (10 mL), potassium carbonate (3.3 g, 23.3 mmol), and 5-bromo-2-fluoropyridine (1.4 g, 7.9 mmol) were added. The mixture was heated to 90 °C and reacted for 16 h. The reaction was quenched by adding water and stirring. The reaction liquid was extracted with dichloromethane, dried over anhydrous sodium sulfate, concentrated under reduced pressure, and separated by column chromatography to give a product (1 g), m/z = 298/300 $[M+1]^+$.

Step B: methyl 4-((benzyloxy)methyl)-1-(5-bromopyridin-2-yl)piperidine-4-carboxylate

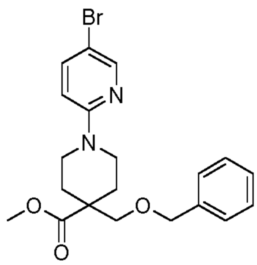

Methyl 1-(5-bromopyridin-2-yl)piperidine-4-carboxylate (1.0 g, 3.34 mmol) and tetrahydrofuran (15 mL) were added to a 100 mL reaction flask. The mixture was cooled to -70 °C, and *n*-butyllithium (2 M, 1.67 mL, 3.34 mmol) was added dropwise with the temperature controlled at -70 ± 5 °C. After the dropwise addition, the mixture was stirred for 10 min, and chloromethoxy benzyl ether (1.0 g, 6.68 mmol) was added dropwise with the temperature controlled at -70 ± 5 °C. After the dropwise addition, the mixture was naturally warmed to room temperature and stirred for 2 h. The reaction was quenched by adding an aqueous ammonium chloride solution. The reaction liquid was extracted with dichloromethane and separated by column chromatography to give a product (1.2 g). M/z = 418/420 $[M+1]^+$.

Step C: methyl 4-hydroxymethyl-1-(5-bromopyridin-2-yl)piperidine-4-carboxylate

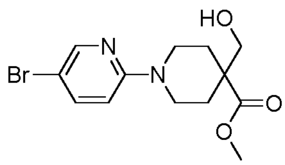

Methyl 4-((benzyloxy)methyl)-1-(5-bromopyridin-2-yl)piperidine-4-carboxylate (1.2 g, 2.86 mmol), dichloromethane (24 mL), and aluminum chloride (1.5 g, 11.45 mmol) were added to a 50 mL reaction flask. The mixture was stirred at room temperature for 3 h, adjusted to about pH 7 with sodium hydroxide, extracted with dichloromethane, and separated by column chromatography to give a product (0.71 g), m/z = 328/330 $[M+1]^+$.

Step D: 4-hydroxymethyl-1-(5-bromopyridin-2-yl)piperidine-4-carboxylic acid

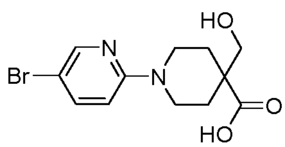

A solution of methyl 4-hydroxymethyl-1-(5-bromopyridin-2-yl)piperidine-4-carboxylate (0.71 g, 2.1 mmol), tetrahydrofuran (10 mL), methanol (10 mL), and lithium hydroxide (181 mg, 4.31 mmol) in water (10 mL) was added to a 50 mL reaction flask. The mixture was stirred at 60 °C for 2 h. The reaction liquid was concentrated, adjusted to pH 5-6 with hydrochloric acid, extracted with dichloromethane, and concentrated to give a product (315 mg), m/z = 314/316 $[M+1]^+$.

Step E: 1-(5-bromopyridin-2-yl)-4-(hydroxymethyl)-N-isobutylpiperidine-4-carboxamide

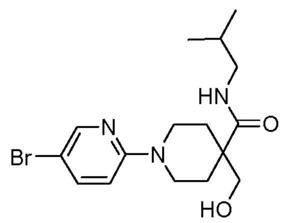

4-hydroxymethyl-1-(5-bromopyridin-2-yl)piperidine-4-carboxylic acid (315 mg, 1 mmol), acetonitrile (3 mL), triethylamine (303 mg, 3 mmol), and isobutylamine (146 mg, 2.0 mmol) were added to a 50 mL reaction flask, and HATU (570 mg, 1.5 mmol) was added. The mixture was stirred at room temperature for 0.5 h. The reaction liquid was poured into water, extracted with dichloromethane, and purified by column chromatography to give a product (270 mg), m/z = 370/372 $[M+1]^+$.

Step F: 1-(5-(6-ethoxy-1*H*-pyrazolo[3',4':3,4]pyrazolo[1,5-*a*]pyridin-4-yl)pyridin-2-yl)-4-(hydroxymethyl)-*N*-isobutylpiperidine-4-carboxamide

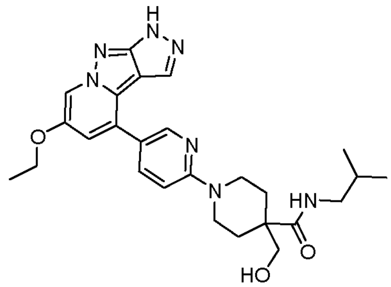

1-(5-bromopyridin-2-yl)-4-(hydroxymethyl)-*N*-isobutylpiperidine-4-carboxamide (36.7 mg, 0.13 mmol), 6-ethoxy-4-(4,4,5,5-tetramethyl-1,3,2-dioxaborane-2-yl)-1*H*-pyrazolo[3',4':3,4]pyrazolo[1,5-*a*]pyrid ine (50 mg, 0.13 mmol), tetrakis(triphenylphosphine)palladium(0) (23 mg, 0.02 mmol), potassium carbonate (37.3 mg, 0.27 mmol), water (0.25 mL), and 1,4-dioxane (0.5 mL) were added to a reaction flask. The mixture was purged with nitrogen three times, heated to 90 °C, and reacted for 16 h. The reaction liquid was poured into water, and the resulting mixture was extracted with dichloromethane and purified by column chromatography to give a product (6 mg), m/z = 492 $[M+1]^+$, $^1H$ NMR (400MHz, DMSO-$d_6$) δ 12.57 (s, 1H), 8.43-8.49 (m, 2H), 7.93-7.96 (m, 1H), 7.51-7.60 (m, 2H), 7.18-7.18 (d, 1H), 6.96-7.02 (m, 1H), 4.83-4.85 (m, 1H), 3.99-4.19 (m, 4H), 3.36-3.37 (m, 2H), 3.11 (m, 2H), 2.86-2.89 (m, 2H), 1.98-2.01 (m, 2H), 1.17-1.71 (m, 1H), 1.43 (m, 2H), 1.36-1.34 (m, 4H), 0.70-0.80 (m, 6H).

**Example 33: Preparation of compound 88**

*Tert*-butyl

((3*S*,4*S*)-1-(5-(6-ethoxy-1*H*-pyrazolo[3',4':3,4]pyrazolo[1,5-*a*]pyridin-4-yl)pyridin-2-yl)-3-hydroxy piperidin-4-yl)carbamate

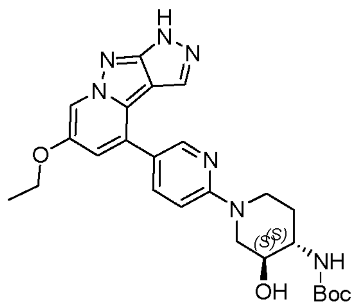

6-ethoxy-4-(6-fluoropyridin-3-yl)-1*H*-pyrazolo[3',4':3,4]pyrazolo[1,5-*a*]pyridine (50 mg, 0.17 mmol), tert-butyl ((3*S*,4*S*)-3-hydroxypiperidin-4-yl)carbamate (40 mg, 0.17 mmol), DIEA (65 mg, 0.5 mmol), and DMSO (3 mL) were added to a reaction flask. The mixture was reacted at 90 °C for 4 h. The reaction was quenched by adding water and stirring, extracted with dichloromethane, dried over anhydrous sodium sulfate, concentrated under reduced pressure, and separated by column chromatography to give a product (12 mg), m/z = 494 $[M+1]^+$, $^1$H NMR (400 MHz, DMSO-$d_6$) δ 12.64 (brs, 1H), 8.56 (s, 1H), 8.50 (s, 1H), 8.01 (d, *J* = 8.8Hz, 1H), 7.57 (s, 1H), 7.26 (s, 1H), 7.04 (d, *J* = 8.8Hz, 1H), 6.72 (brs, 1H), 4.98 (d, *J* = 4.8Hz, 1H), 4.43-4.46 (m, 1H), 4.20-4.27 (m, 1H), 4.15 (q, *J* = 6.8Hz, 2H), 3.35-3.41 (m, 2H), 2.96-3.02 (m, 1H), 2.75-2.80 (m, 1H), 1.86-1.88 (m, 1H), 1.35-1.42 (m, 13H).

**Example 34: Preparation of compound 89**

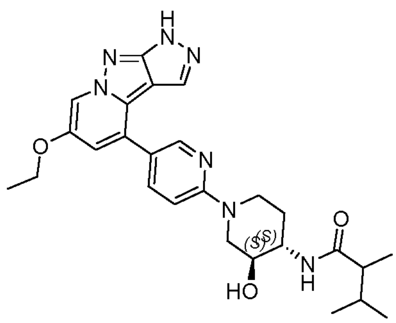

(3*S*,4*S*)-4-amino-1-(5-(6-ethoxy-1*H*-pyrazolo[3',4':3,4]pyrazolo[1,5-*a*]pyridin-4-yl)pyridin-2-yl)pip eridin-3-ol (256.5 mg, 0.51 mmol), 2,3-dimethylbutyric acid (60 mg, 0.51 mmol), DIEA (329 mg, 2.55 mmol), HATU (232 mg, 0.61 mmol), and DCM (10 mL) were added to a 50 mL reaction flask. The mixture was reacted at room temperature for 0.5 h. Water was added, and the resulting mixture was extracted with ethyl acetate, dried over anhydrous sodium sulfate, filtered, concentrated under reduced pressure, and separated by column chromatography to give a product (40 mg), m/z = 492 $[M+1]^+$, $^1$H NMR (400 MHz, DMSO-$d_6$): δ 12.64 (s, 1H), 8.50-8.60 (m, 2H), 8.05 (m, 1H), 7.65 (m, 1H), 7.60 (s, 1H), 7.10-7.15 (m, 1H), 4.30-4.50 (m, 1H), 4.10-4.30 (m, 3H), 3.70-3.80 (m, 1H), 3.50-3.60 (m, 2H), 3.06-3.39 (m, 1H), 2.75-2.95 (m, 1H), 1.88-1.96 (m, 2H), 1.66-1.70 (m, 1H), 1.33-1.41 (m, 4H), 0.83-0.98 (m, 9H).

**Example 35: Preparation of compound 91**

Step A: 2-chloro-*N*-((3*S*,4*S*)-(1-(5-(6-ethoxy-1*H*-pyrazolo[3',4':3,4]pyrazolo[1,5-*a*]pyridin-4-yl)pyrazin-2-yl )-3-hydroxypiperidin-4-yl)-5-fluorobenzamide

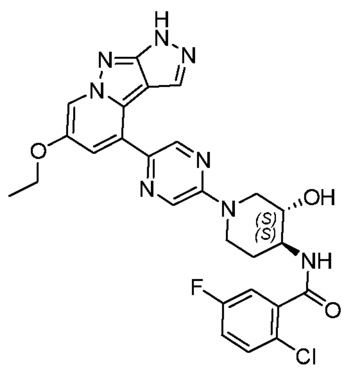

(3*S*,4*S*)-4-amino-1-(5-(6-ethoxy-1*H*-pyrazolo[3',4':3,4]pyrazolo[1,5-*a*]pyridin-4-yl)pyrazin-2-yl)-3-hydroxypiperidine hydrochloride (100 mg, 0.2 mmol), 2-chloro-5-fluorobenzoyl chloride (46 mg, 0.24 mmol), DIEA (129 mg, 1.0 mmol), and 2 mL of DMF were added to a reaction flask. The mixture was reacted at 25 °C for 1 h. After the reaction was completed, the reaction liquid was extracted with dichloromethane, dried over anhydrous sodium sulfate, concentrated under reduced pressure, and separated by column chromatography to give a product (8.5 mg), m/z = 551.1 [M+1]$^+$, $^1$H NMR (400 MHz, DMSO-$d_6$) δ 12.47 (brs, 1H), 8.91 (s, 1H), 8.72 (s, 1H), 8.55 (s, 1H), 8.47 (d, *J*=8.0Hz, 1H), 8.01 (s, 1H), 7.73 (s, 1H), 7.54 (dd, $J_1$=8.8Hz, $J_2$=4.0Hz, 1H), 7.40-7.43 (m, 1H), 7.31-7.36 (m, 1H), 5.25 (s, 1H), 4.42-4.46 (m, 1H), 4.28-4.32 (m, 1H), 4.17 (t, *J*=6.8Hz, 2H), 3.89-3.99 (m, 1H), 3.51-3.57 (m, 1H), 3.25-3.27 (m, 1H), 3.02-3.10 (m, 1H), 2.01-2.08 (m, 1H), 1.48-1.54 (m, 1H), 1.39 (t, *J*=6.8Hz, 3H).

**Example 36: Preparation of compound 101**

Step A: *tert*-butyl 4-(*N*-isopropylsulfamoyl)piperidine-1-carbonate

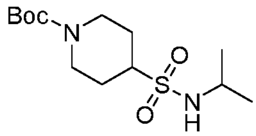

Isopropylamine (97 mg, 1.7 mmol) and dichloromethane (10 mL) were added to a reaction flask. The mixture was stirred, and triethylamine (300 mg, 3 mmol) was added. The mixture was cooled to 10 °C, and *tert*-butyl 4-(chlorosulfonyl)piperidine-1-carboxylate (426 mg, 1.5 mmol) was added. The mixture was reacted for 1 h. The reaction was quenched by adding water, extracted with dichloromethane, dried over anhydrous sodium sulfate, filtered, and concentrated under reduced pressure to give a product (400 mg), which was directly used in the next step, m/z = 307 [M+1]$^+$.

Step B: *N*-isopropylpiperidine-4-sulfonamide

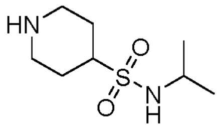

*Tert*-butyl 4-(*N*-isopropylsulfonyl)piperidine-1-carbonate (400 mg, 1.3 mmol) and a solution of HCl in 1,4-dioxane (4 M, 10 mL) were added to a reaction flask. The mixture was reacted at room temperature for 1 h. After the reaction was completed, the reaction liquid was concentrated under reduced pressure to give a product (340 mg), which was directly used in the next step, m/z = 207 [M 1]$^+$.

Step C: 1-(5-(6-ethoxy-1*H*-pyrazolo[3',4':3,4]pyrazolo[1,5-*a*]pyridin-4-yl)pyridin-2-yl)-*N*-isopropylpiperidi ne-4-sulfonamide

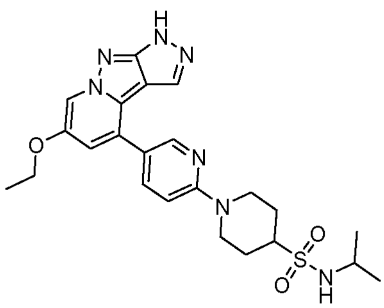

DMSO (2 mL) and DIEA (0.5 mL), *N*-isopropylpiperidine-4-sulfonamide (340 mg, 1.3 mmol), and 6-ethoxy-4-(6-fluoropyridin-3-yl)-1*H*-pyrazolo[3',4':3,4]pyrazolo[1,5-*a*]pyridine (100 mg, 0.3 mmol) were sequentially added to a reaction flask. The mixture was reacted at 100 °C for 48 h. The reaction was quenched by adding water and stirring. The reaction

liquid was extracted with dichloromethane, dried over anhydrous sodium sulfate, concentrated under reduced pressure, and separated by column chromatography to give a product (46 mg), m/z = 484 $[M+1]^+$. $^1H$-NMR (400 MHz, DMSO-$d_6$) δ 12.66 (s, 1H), 8.60 (d, *J* = 2.6 Hz, 1H), 8.53 (d, *J* = 2.1 Hz, 1H), 8.06 (dd, *J* = 8.9, 2.6 Hz, 1H), 7.58 (s, 1H), 7.28 (d, *J* = 2.0 Hz, 1H), 7.10 (dd, *J* = 19.8, 8.4 Hz, 2H), 4.56 (d, *J* = 13.3 Hz, 2H), 4.18 (q, *J* = 6.9 Hz, 2H), 3.46 (q, *J* = 6.7 Hz, 1H), 3.32 (s, 1H), 3.02 (t, *J* = 12.2 Hz, 2H), 2.06 (d, *J* = 11.3 Hz, 2H), 1.60 (qd, *J* = 12.5, 4.2 Hz, 2H), 1.40 (t, *J* = 6.9 Hz, 3H), 1.13 (d, *J* = 6.5 Hz, 6H).

**Example 37: Preparation of compound 102**

Step A: *tert*-butyl 4-hydroxy-4-(phenylsulfonamidomethylene)piperidine-1-carbonate

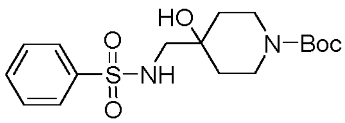

*Tert*-butyl 4-(amidomethylene)-4-hydroxypiperidine-1-carbonate (230 mg, 1 mmol), dichloromethane (10 mL), and triethylamine (200 mg, 2 mmol) were added to a reaction flask. The mixture was cooled to 10 °C, and benzoyl chloride (194 mg, 1.1 mmol) was added. The mixture was reacted for 1 h. The reaction was quenched by adding water and stirring. The reaction liquid was extracted with dichloromethane, dried over anhydrous sodium sulfate, concentrated under reduced pressure, and separated by column chromatography to give a product (300 mg), m/z = 371 $[M+1]^+$.

Step B: *N*-((4-hydroxypiperidin-4-yl)methylene)benzenesulfonamide

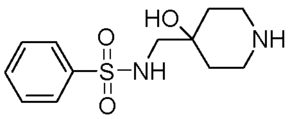

*Tert*-butyl 4-hydroxy-4-(phenylsulfonamidomethylene)piperidine-1-carbonate (300 mg, 0.8 mmol) and a solution of HCl in 1,4-dioxane (4 M, 10 mL) were sequentially added to a reaction flask. The mixture was reacted at room temperature for 1 h. After the reaction was completed, the reaction liquid was concentrated under reduced pressure to give a product (320 mg), which was directly used in the next step, m/z = 271 $[M+1]^+$.

Step C: *N*-((1-(5-(6-ethoxy-1*H*-pyrazolo[3',4':3,4]pyrazolo[1,5-*a*]pyridin-4-yl)pyridin-2-yl)-4-hydroxypiperi din-4-yl)methylene)benzenesulfonamide

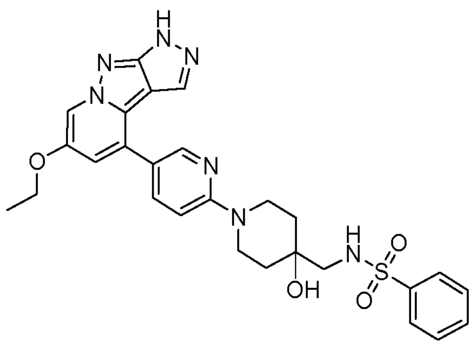

DMSO (2 mL) and DIEA (0.5 mL), *N*-((4-hydroxypiperidin-4-yl)methylene)benzenesulfonamide (300 mg, 0.8 mmol), and 6-ethoxy-4-(6-fluoropyridin-3-yl)-1*H*-pyrazolo[3',4':3,4]pyrazolo[1,5-*a*]pyridine (100 mg, 0.3 mmol) were sequentially added to a reaction flask. The mixture was reacted at 100 °C for 16 h. The reaction was quenched by adding

water and stirring. The reaction liquid was extracted with dichloromethane, dried over anhydrous sodium sulfate, concentrated under reduced pressure, and separated by column chromatography to give a product (93 mg), m/z = 548 $[M+1]^+$. $^1$H NMR (400 MHz, DMSO-$d_6$) δ 12.64 (s, 1H), 8.58 (d, *J* = 2.6 Hz, 1H), 8.51 (d, *J* = 2.1 Hz, 1H), 8.03 (dd, *J* = 8.9, 2.6 Hz, 1H), 7.86 - 7.78 (m, 2H), 7.69 - 7.53 (m, 5H), 7.26 (d, *J* = 2.1 Hz, 1H), 7.08 (d, *J* = 9.0 Hz, 1H), 4.60 (s, 1H), 4.23 - 4.07 (m, 4H), 3.35 - 3.24 (m, 2H), 2.72 (d, *J* = 6.7 Hz, 2H), 1.60 (td, *J* = 12.8, 12.1, 4.4 Hz, 2H), 1.48 (d, *J* = 13.2 Hz, 2H), 1.40 (t, *J* = 6.9 Hz, 3H).

**Example 38: Preparation of compound 103**

Step A: *tert*-butyl 4-(pyridin-2-yloxy)piperidine-1-carboxylate

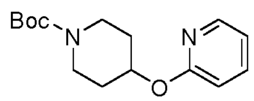

*Tert*-butyl 4-hydroxypiperidine-1-carboxylate (804 mg, 4 mmol) and tetrahydrofuran (10 mL) were added to a reaction flask. The mixture was stirred and cooled to 10 °C, and NaH (content: 60%, 192 mg, 4.8 mmol) was added. The mixture was reacted for 1 h, and 2-fluoropyridine (388 mg, 4 mmol) was added. The mixture was reacted for 24 h. The reaction was quenched by adding water and stirring. The reaction liquid was extracted with dichloromethane, dried over anhydrous sodium sulfate, concentrated under reduced pressure, and separated by column chromatography to give a product (950 mg), m/z = 279 $[M+1]^+$.

Step B: 2-(piperidin-4-yloxy)pyridine

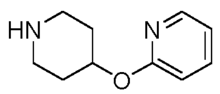

*Tert*-butyl 4-(pyridine-2-oxy)piperidine-1-carboxylate (950 mg, 3.4 mmol) and a solution of HCl in 1,4-dioxane (4 M, 10 mL) were sequentially added to a reaction flask. The mixture was reacted at room temperature for 1 h. After the reaction was completed, the reaction liquid was concentrated under reduced pressure to dryness to give a product (750 mg), which was directly used in the next step, m/z = 179 $[M+1]^+$.

Step C: 6-ethoxy-4-(6-(4-(pyridin-2-yloxy)piperidin-1-yl)pyridin-3-yl)-1*H*-pyrazolo[3',4':3,4]pyrazolo[1,5-*a*]pyridine

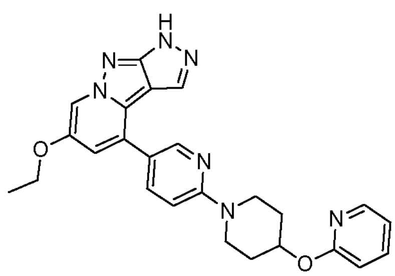

DMSO (2 mL) and DIEA (0.5 mL), *N*-((4-hydroxypiperidin-4-yl)methyl)benzenesulfonamide (150 mg, 0.7 mmol), and 6-ethoxy-4-(6-fluoropyridin-3-yl)-1*H*-pyrazolo[3',4':3,4]pyrazolo[1,5-*a*]pyridine (60 mg, 0.2 mmol) were sequentially added to a reaction flask. The mixture was reacted at 100 °C for 16 h. The reaction was quenched by adding water and stirring. The mixture was extracted with dichloromethane, dried over anhydrous sodium sulfate, concentrated under reduced pressure, and separated by column chromatography to give a product (22 mg), m/z = 456 $[M+1]^+$. $^1$H NMR (400 MHz, DMSO-$d_6$) δ 12.66 (s, 1H), 8.60 (d, *J* = 2.6 Hz, 1H), 8.53 (d, *J* = 2.1 Hz,1H), 8.19 (dd, *J* = 5.1, 2.0 Hz, 1H),

8.06 (dd, *J* = 8.9, 2.6 Hz, 1H), 7.72 (ddd, *J* = 8.8, 7.1, 2.1 Hz, 1H), 7.60 (s, 1H), 7.27 (d, *J* = 2.1 Hz, 1H), 7.13 (d, *J* = 8.9 Hz, 1H), 6.94-7.03 (m, 1H), 6.82 (d, *J* = 8.3 Hz, 1H), 5.31 (tt, *J* = 8.3, 3.9 Hz, 1H), 4.17 (dd, *J* = 13.4, 6.2 Hz, 4H), 3.45 (ddd, *J* = 13.1, 9.5, 3.2 Hz, 2H), 2.10 (dq, *J* = 8.1, 3.8 Hz, 2H), 1.71 (dtd, *J* = 12.7, 9.0, 3.7 Hz, 2H), 1.41 (t, *J* = 6.9 Hz, 3H).

**Example 39: Preparation of compound 104**

Step A: *tert*-butyl 4-((6-methylpyridazin-3-yl)oxy)piperidine-1-carbonate

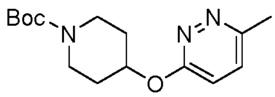

*Tert*-butyl 4-hydroxypiperidine-1-carboxylate (402 mg, 2 mmol) and tetrahydrofuran (10 mL) were added to a reaction flask. The mixture was stirred and cooled to 10 °C, and NaH (content: 60%, 96 mg, 2.4 mmol) was added. The mixture was reacted for 1 h, and 3-chloro-6-methylpyridazine (258 mg, 2 mmol) was added. The mixture was reacted for 24 h. Water was added, and the resulting mixture was extracted with ethyl acetate, dried over anhydrous sodium sulfate, filtered, concentrated under reduced pressure to dryness, and purified by silica gel column chromatography to give a product (250 mg), m/z = 294 $[M+1]^+$.

Step B: 3-methyl-6-(piperidin-4-yloxy)pyridazine

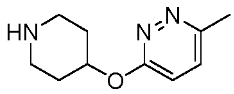

Tert-butyl 4-((6-methylpyridazin-3-yl)oxy)piperidine-1-carbonate (250 mg, 0.8 mmol) and a solution of HCl in 1,4-dioxane (4 M, 10 mL) were sequentially added to a reaction flask. The mixture was reacted at room temperature for 1 h. After the reaction was completed, the reaction liquid was concentrated under reduced pressure to give a product (210 mg), which was directly used in the next step, m/z = 194 $[M+1]^+$.

Step C: 6-ethoxy-4-(6-(4-((6-methylpyridazin-3-yl)oxy)piperidin-1-yl)pyridin-3-yl)-1*H*-pyrazolo[3',4':3,4]p yrazolo[1,5-*a*]pyridine

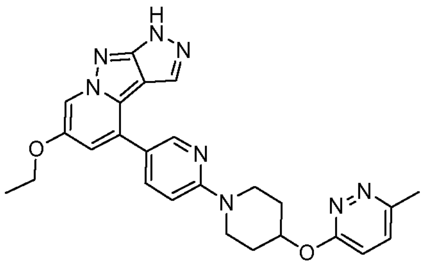

DMSO (2 mL) and DIEA (0.5 mL), 3-methyl-6-(pyridine-4-oxy)pyridazine (210 mg, 0.8 mmol), and 6-ethoxy-4-(6-fluoropyridin-3-yl)-1*H*-pyrazolo[3',4':3,4]pyrazolo[1,5-*a*]pyridine (80 mg, 0.2 mmol) were sequentially added to a reaction flask. The mixture was reacted at 100 °C for 16 h. The reaction was quenched by adding water and stirring. The reaction liquid was extracted with dichloromethane, dried over anhydrous sodium sulfate, concentrated under reduced pressure, and separated by column chromatography to give a product (27 mg), m/z = 471 $[M+1]^+$. $^1H$ NMR (400 MHz, DMSO-$d_6$) δ 12.65 (s, 1H), 8.60 (d, *J* = 2.5 Hz, 1H), 8.52 (d, *J* = 2.1 Hz, 1H), 8.06 (dd, *J* = 8.9, 2.6 Hz, 1H), 7.60 (s, 1H), 7.51 (d, *J* = 9.0 Hz, 1H), 7.27 (d, *J* = 2.1 Hz, 1H), 7.12 (dd, *J* = 9.0, 7.2 Hz, 2H), 5.46 (tt, *J* = 8.3, 3.8 Hz, 1H), 4.17 (p, *J* = 8.6, 7.7 Hz, 4H), 3.49 (ddd, *J* = 13.0, 9.2, 3.2 Hz, 2H), 2.53 (s, 3H), 2.21 - 2.11 (m, 2H), 1.76 (dtd, *J* = 12.7, 8.9, 3.8 Hz, 2H), 1.40 (t, *J* = 6.9 Hz, 3H).

**Example 40: Preparation of compound 107**

(3*S*,4*S*)-4-((2-chloro-6-fluorobenzyl)amino)-1-(5-(6-ethoxy-1*H*-pyrazolo[3',4':3,4]pyrazolo[1,5-*a*]p yridin-4-yl)pyridin-2-yl)piperidin-3-ol

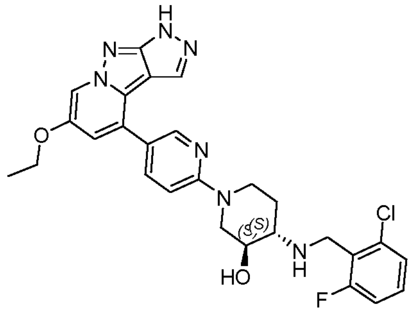

(3*S*,4*S*)-4-amino-1-(5-(6-ethoxy-1*H*-pyrazolo[3',4':3,4]pyrazolo[1,5-*a*]pyridin-4-yl)pyridin-2-yl)pip eridin-3-ol hydrochloride (100 mg, 0.2 mmol), 1-chloro-2-(chloromethyl)-3-fluorobenzene (50 mg, 0.2 mmol), $Cs_2CO_3$ (130 mg, 0.4 mmol), and DMF (3 mL) were added to a reaction flask. The mixture was reacted at 50 °C for 12 h. The reaction liquid was poured into 30 mL of water, and the resulting mixture was extracted with ethyl acetate. The organic phase was dried, concentrated by rotary evaporation to remove the solvent, and purified by column chromatography to give a product (4.6 mg), m/z = 535 $[M+1]^+$. $^1$H-NMR (400 MHz, DMSO-$d_6$) δ 12.65 (brs, 1H), 8.56 (s, 1H), 8.50 (s, 1H), 8.01 (d, *J* = 8.8 Hz, 1H), 7.58 (s, 1H), 7.31-7.42 (m, 2H), 7.22-7.30 (m, 2H), 7.05 (d, *J* = 8.8 Hz, 1H), 5.18 (d, *J* = 4.0 Hz, 1H), 4.42-4.48 (m, 1H), 4.29-4.35 (m, 1H), 4.14 (m, 2H), 3.81-3.98 (m, 2H), 3.20-3.28 (m, 2H), 2.88-2.98 (m, 1H), 2.66-2.74 (m, 1H), 2.41-2.47 (m, 2H), 2.10-2.20 (m, 1H), 1.37 (m, 3H).

**Example 41: Preparation of compound 118**

*N*-((3a*R*,6a*S*)-5-(5-(6-ethoxy-1*H*-pyrazolo[3',4':3,4]pyrazolo[1,5-*a*]pyridin-4-yl)pyridin-2-yl)hexahy dropyrrolo[3,4-*c*]pyrrol-2(1*H*)-yl)-2-chloro-6-fluorobenzamide

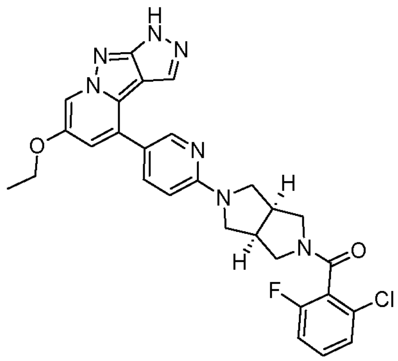

6-ethoxy-4-(6-((3a*R*,6a*S*)-hexahydropyrrolo[3,4-*c*]pyrrol-2(1*H*)-yl)pyridin-3-yl)-1*H*-pyrazolo[3',4': 3,4]pyrazolo[1,5-*a*]pyridine hydrochloride (200 mg, 0.4 mmol), 2-chloro-6-fluorobenzoyl chloride (77 mg, 0.4 mmol), DIEA (258 mg, 2 mmol), and DCM (10 mL)were added to a reaction flask. The mixture was reacted at 25 °C for 1 h. The reaction was quenched by adding water and stirring. The reaction liquid was extracted with dichloromethane, dried over anhydrous sodium sulfate, concentrated under reduced pressure, and separated by column chromatography to give a product (4.6 mg), m/z = 546 $[M+1]^+$. $^1$H-NMR (400 MHz, DMSO-$d_6$) δ 12.65 (brs, 1H), 8.55 (s, 1H), 8.51 (s, 1H), 8.01 (d, *J* = 8.8 Hz, 1H), 7.48-7.61 (m, 2H), 7.33-7.47 (m, 2H), 7.25 (s, 1H), 6.70-6.80 (m, 1H), 4.15 (q, *J* = 6.8 Hz, 2H), 3.70-3.86 (m, 3H), 3.46-3.53 (m, 4H), 3.06-3.24 (m, 3H), 1.38 (t, *J* = 6.8 Hz, 3H).

**Example 42: Preparation of compound 150**

Step A: ethyl 4-(2,5-difluorobenzamido)-1-(5-(6-ethoxy-1*H*-pyrazolo[3',4':3,4]pyrazolo[1,5-*a*]pyridin-4-yl)pyridi n-2-yl)piperidine-4-carboxylate

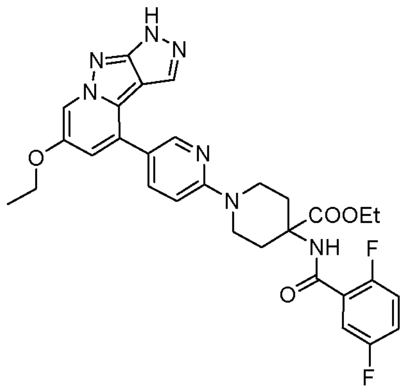

Ethyl

4-((*tert*-butylcarbonyl)amino)-1-(5-(6-ethoxy-1*H*-pyrazolo[3',4':3,4]pyrazolo[1,5-*a*]pyridin-4-yl)pyr idin-2-yl)piperidine-4-carboxylate hydrochloride (120 mg, 0.26 mmol), 2,5-difluorobenzoic acid (37.9 mg, 0.24 mmol), DIEA (100 mg, 0.78 mmol), HATU (108 mg, 0.28 mmol), and DMF (2 mL) were added to a reaction flask. The mixture was reacted at 25 °C for 12 h. The reaction was quenched by adding water and stirring. The reaction liquid was extracted with dichloromethane, dried over anhydrous sodium sulfate, concentrated under reduced pressure, and separated by column chromatography to give a product (78 mg), m/z = 590 $[M+1]^+$.

Step B: *N*-(1-(5-(6-ethoxy-1*H*-pyrazolo[3',4':3,4]pyrazolo[1,5-*a*]pyridin-4-yl)pyridin-2-yl)-4-(hydroxymeth yl)piperidin-4-yl)-2,5-difluorobenzamide

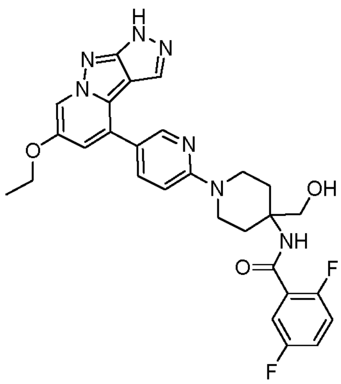

Ethyl

4-(2,5-difluorobenzamide)-1-(5-(6-ethoxy-1*H*-pyrazolo[3',4':3,4]pyrazolo[1,5-*a*]pyridin-4-yl)pyridi n-2-yl)piperidine-4-carboxylate (29.4 mg, 0.05 mmol) and 2 mL of THF were added to a reaction flask, and $LiAlH_4$ (1.9 mg, 0.05 mmol) was added at 0 °C. The mixture was stirred for 1 h. The reaction was quenched by adding water and stirring. The reaction liquid was extracted with dichloromethane, dried over anhydrous sodium sulfate, concentrated under reduced pressure, and separated by column chromatography to give a product (15 mg), m/z = 548 $[M+1]^+$, $^1H$ NMR (400 MHz, DMSO-$d_6$) $\delta$ 12.66 (s, 1H), 8.62 - 8.56 (m, 1H), 8.52 (d, *J* = 2.1 Hz, 1H), 8.08 - 7.99 (m, 2H), 7.60 (s, 1H), 7.47 - 7.29 (m, 3H), 7.27 (d, *J* = 2.1 Hz, 1H), 7.11 (d, *J* = 9.0 Hz, 1H), 4.88 (t, *J* = 5.9 Hz, 1H), 4.21 (m, 2H), 3.64 (d, *J* = 6.0 Hz,

2H), 3.33 (s, 2H), 3.23 (t, *J* = 12.4 Hz, 2H), 2.29 (d, *J* = 13.6 Hz, 2H), 1.64 (m, 2H), 1.40 (t, *J* = 6.9 Hz, 3H).

**Example 43: Preparation of compound 151**

Step A: *tert*-butyl 3-(5-(6-ethoxy-1*H*-pyrazolo[3',4':3,4]pyrazolo[1,5-*a*]pyridin-4-yl)pyridin-2-yl)-3,6-diazabicyclo[3. 1.1]heptane-6-carbonate

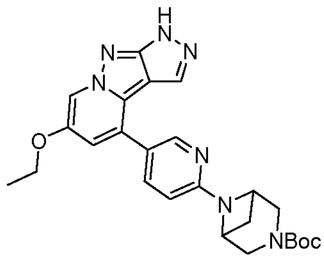

4-bromo-6-ethoxy-1*H*-pyrazolo[3',4':3,4]pyrazolo[1,5-*a*]pyridine (0.28 g, 1 mmol), 1,4-dioxane (2.8 mL), *tert*-butyl 6-(5-(4,4,5,5-tetramethyl-1,3,2-dioxaborolan-2-yl)pyridin-2-yl)-3,6-diazabicyclo[3.1.1]heptane-3-ca rbonate (0.52 g, 1.3 mmol), lithium carbonate (148 mg, 2 mmol), tetrakis(triphenylphosphine)palladium(0) (23 mg, 0.02 mmol), and water (1.4 mL) were added to a three-necked flask. The mixture was heated to 90 °C and reacted for 24 h. The reaction was quenched by adding water and stirring. The reaction liquid was extracted with dichloromethane, dried over anhydrous sodium sulfate, concentrated under reduced pressure, and separated by column chromatography to give a product (230 mg), m/z = 476.2 $[M+1]^+$.

Step B: 4-(6-(3,6-diazabicyclo[3.1.1]heptan-6-yl)pyridin-3-yl)-6-ethoxy-1*H*-pyrazolo[3',4':3,4]pyrazolo[1,5 -a]pyridine

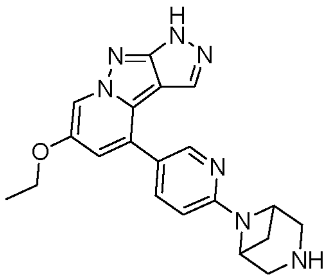

Tert-butyl

3-(5-(6-ethoxy-1*H*-pyrazolo[3',4':3,4]pyrazolo[1,5-*a*]pyridin-4-yl)pyridin-2-yl)-3,6-diazabicyclo[3. 1.1]heptane-6-carbonate (200 mg, 0.42 mmol), methanol (4 mL), and *m*-dimethoxy benzene (2.9 mg, 0.02 mmol) were sequentially added to a three-necked flask, and sulfuric acid (98%, 168 mg, 1.68 mmol) was added. The mixture was heated to 55 °C and reacted for 4 h. The reaction was quenched by adding water and stirring. The reaction liquid was extracted with dichloromethane, dried over anhydrous sodium sulfate, concentrated under reduced pressure, and separated by column chromatography to give a product (300 mg), m/z = 376.2 $[M+1]^+$.

Step C: 6-ethoxy-4-(6-(3-(((6-methoxypyridin-3-yl)methyl)-3,6-diazabicyclo[3.1.1]heptan-6-yl)pyridin-3-yl )-1*H*-pyrazolo[3',4':3,4]pyrazolo[1,5-*a*]pyridine

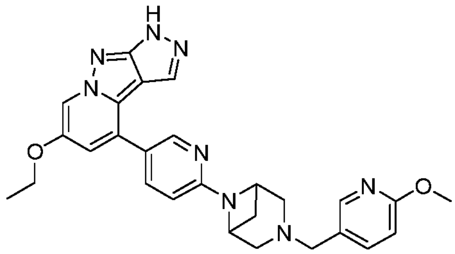

4-(6-(3,6-diazabicyclo[3.1.1]heptan-6-yl)pyridin-3-yl)-6-ethoxy-1*H*-pyrazolo[3',4':3,4]pyrazolo[1,5 -*a*]pyridine (180 mg, 0.27 mmol), n-propanol (3.6 mL), triethylamine (136.2 mg, 1.35 mmol), 6-methoxy-3-pyridinecarboxaldehyde (111 mg, 0.81 mmol), and Pic-$BH_3$ (86.7 mg, 0.81 mmol) were sequentially added to a three-necked flask. The mixture was stirred for 24 h with the temperature maintained at 50 °C. The reaction was quenched by adding water and stirring. The reaction liquid was extracted with dichloromethane, dried over anhydrous sodium sulfate, concentrated under reduced pressure, and separated by column chromatography to give a product (70 mg), m/z = 497 $[M+1]^+$, $^1H$ NMR (400MHz, DMSO-*d*6) δ 12.67 (s, 1H), 8.53-8.56 (m, 2H), 7.99-8.02 (m, 1H), 7.76-7.76 (m, 1H), 7.59 (s, 1H), 7.31-7.32 (m, 1H), 7.19-7.22 (m, 1H), 6.69-6.71 (m, 1H), 6.49-6.52 (m, 1H), 4.38-4.40 (m, 2H), 4.16-4.21 (m, 2H), 3.74 (s, 3H), 3.56 (s, 2H), 3.21-3.24 (m, 2H), 2.69-2.72 (m, 2H), 2.56-2.60 (m, 1H), 1.75-1.77 (d, 1H), 1.39-1.42 (m, 3H).

**Example 44: Preparation of compound 152**

Step A: *tert*-butyl (3a*R*,6a*S*)-5-(5-(6-ethoxy-1*H*-pyrazolo[3',4':3,4]pyrazolo[1,5-*a*]pyridin-4-yl)pyridin-2-yl)hexahydro pyrrolo[3,4-*c*]pyrrole-2(1*H*)-carboxylate

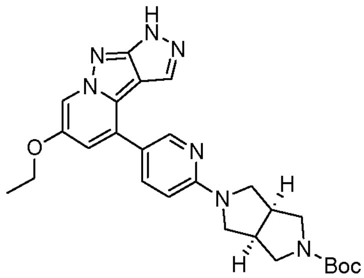

6-ethoxy-4-(6-fluoropyridin-3-yl)-1*H*-pyrazolo[3',4':3,4]pyrazolo[1,5-*a*]pyridine (700 mg, 2.4 mmol), *tert*-butyl (3a*R*,6a*S*)-hexahydropyrrolo[3,4-*c*]pyrrole-2(1*H*)-carboxylate (700 mg, 2.4 mmol), DIEA (1.5 g, 11.6 mmol), and DMSO (10 mL) were added to a reaction flask. The mixture was reacted at 90 °C for 12 h. The reaction was quenched by adding water and stirring. The reaction liquid was extracted with dichloromethane, dried over anhydrous sodium sulfate, concentrated under reduced pressure, and separated by column chromatography to give a product (1 g), m/z = 490 $[M+1]^+$.

Step B: 6-ethoxy-4-(6-((3a*R*,6a*S*)-hexahydropyrrolo[3,4-*c*]pyrrol-2(1*H*)-yl)pyridin-3-yl)-1*H*-pyrazolo[3',4': 3,4]pyrazolo[1,5-*a*]pyridine hydrochloride

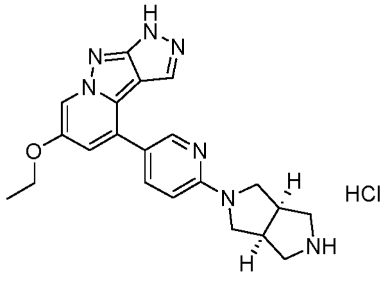

A solution of HCl in methanol (4 M, 20 mL) was added to *tert*-butyl (3a*R*,6a*S*)-5-(5-(6-ethoxy-1*H*-pyrazolo[3',4':3,4]pyrazolo[1,5-*a*]pyridin-4-yl) pyridin-2-yl)hexahydropyrrolo[3,4-*c*]pyrrole-2(1*H*)-carboxylate (1 g, 2.0 mmol). The mixture was reacted at 25 °C for 4 h. The reaction liquid was concentrated by rotary evaporation to remove the solvent to give a product (1.1 g), m/z = 390 [M+1]$^+$.

6-ethoxy-4-(6-((3a*R*,6a*S*)-5-((6-methoxypyridin-3-yl)methylene)-hexahydropyrrolo[3,4-*c*]pyrrol-2( 1*H*)-yl)pyridin-3-yl)-1*H*-pyrazolo[3',4':3,4]pyrazolo[1,5-*a*]pyridine

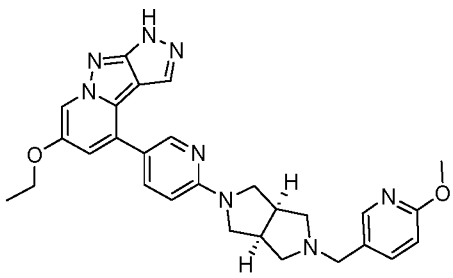

Step C:

6-ethoxy-4-(6-((3a*R*,6a*S*)-hexahydropyrrolo[3,4-*c*]pyrrol-2(1*H*)-yl)pyridin-3-yl)-1*H*-pyrazolo[3',4': 3,4]pyrazolo[1,5-*a*]pyridine hydrochloride (200 mg, 0.4 mmol), 6-methoxy-3-pyridinecarboxaldehyde (165 mg, 1.2 mmol), $Et_3N$ (202 mg, 2.0 mmol), Pic-$BH_3$ (128 mg, 1.2 mmol), and *n*-propanol (10 mL) were added to a reaction flask. The mixture was reacted at 40 °C for 48 h. The reaction was quenched by adding water and stirring. The reaction liquid was extracted with dichloromethane, dried over anhydrous sodium sulfate, concentrated under reduced pressure, and separated by column chromatography to give a product (67.2 mg), m/z = 511 [M+1]$^+$. $^1$H-NMR (400 MHz, DMSO-$d_6$) $\delta$ 12.64 (brs, 1H), 8.55 (s, 1H), 8.51 (s, 1H), 8.06 (s, 1H), 8.00 (d, *J* = 8.8 Hz, 1H), 7.58-7.64 (m, 2H), 7.23 (s, 1H), 6.72-6.78 (m, 2H), 4.15 (q, *J* = 6.8 Hz, 2H), 3.82 (s, 3H), 3.62-3.71 (m, 2H), 3.54 (s, 2H), 3.35-3.41 (m, 2H), 2.88-2.97 (m, 2H), 2.61-2.71 (m, 2H), 2.42-2.44 (m, 2H), 1.38 (t, *J* = 6.8 Hz, 3H).

**Example 45: Preparation of compound 155**

Step A: *tert*-butyl (1*R*,5*S*)-3-(5-(6-ethoxy-1*H*-pyrazolo[3',4':3,4]pyrazolo[1,5-*a*]pyridin-4-yl)pyridin-2-yl)-3,8-diazabi cyclo[3.2.1]octane-8-carbonate

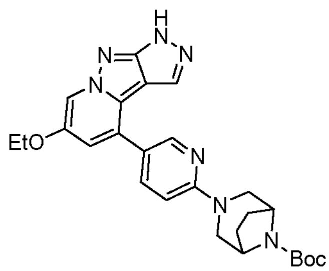

6-ethoxy-4-(6-fluoropyridin-3-yl)-1*H*-pyrazolo[3',4':3,4]pyrazolo[1,5-*a*]pyridine (292 mg, 1.0 mmol), *tert*-butyl 3,8-diazabicyclo[3.2.1]octane-8-carbonate (250 mg, 1.2 mmol), DIEA (506 mg, 3.9 mmol), and 3 mL of DMSO were added to a reaction flask. The mixture was reacted at 95 °C for 4 h. The reaction was quenched by adding water and stirring. The reaction liquid was extracted with dichloromethane, dried over anhydrous sodium sulfate, concentrated under reduced pressure, and separated by column chromatography to give a product (208 mg), m/z = 490 [M+1]$^+$.

Step B:

4-(6-((1*R*,5*S*)-3,8-diazabicyclo[3.2.1]octan-3-yl)pyridin-3-yl)-6-ethoxy-1*H*-pyrazolo[3',4':3,4]pyraz olo[1,5-

*a*]pyridine hydrochloride

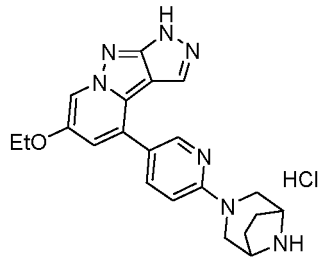

*Tert*-butyl

(1*R*,5*S*)-3-(5-(6-ethoxy-1*H*-pyrazolo[3',4':3,4]pyrazolo[1,5-*a*]pyridin-4-yl)pyridin-2-yl)-3,8-diazabi cyclo[3.2.1]octane-8-carbonate (200 mg, 0.4 mmol) and a 5 N methanolic hydrochloric acid solution (3 mL, 15 mmol) were added to a reaction flask. The mixture was reacted at 25 °C for 4 h. The reaction liquid was directly concentrated under reduced pressure to give a product (250 mg), m/z = 390 [M+1]$^+$.

Step C: (2-chloro-6-fluoro)((1*R*,5*S*)-3-(5-(6-ethoxy-1*H*-pyrazolo[3',4':3,4]pyrazolo[1,5-*a*]pyridin-4-yl)pyrid in-2-yl)-3,8-diazabicyclo[3.2.1]octan-8-yl)benzamide

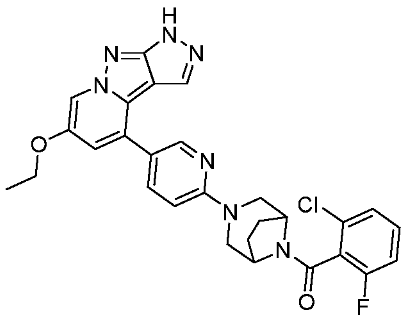

4-(6((1*R*,5*S*)-3,8-diazabicyclo[3.2.1]octan-3-yl)pyridin-3-yl)-6-ethoxy-1*H*-pyrazolo[3',4':3,4]pyrazolo[1,5-*a*]pyridine hydrochloride (120 mg, 0.24 mmol), 3-chloro-6-fluorobenzoyl chloride (10 mg, 0.26 mmol), DIEA (155 mg, 1.2 mmol), and 2 mL of DMF were added to a reaction flask. The mixture was reacted at 25 °C for 1 h. The reaction was quenched by adding water and stirring. The reaction liquid was extracted with dichloromethane, dried over anhydrous sodium sulfate, concentrated under reduced pressure, and separated by column chromatography to give a product (136 mg), m/z = 546 [M+1]$^+$. $^1$H NMR (400 MHz, $CD_3OD$-*$d_3$*) δ 8.58 (s, 1H), 8.31 (s, 1H), 8.01 (d, *J* = 8.8Hz, 1H), 7.65 (s, 1H), 7.49-7.55 (m, 1H), 7.39-7.42 (m, 1H), 7.24-7.30 (m, 2H), 7.00 (d, *J* = 8.8Hz, 1H), 5.03-5.04 (m, 1H), 4.31-4.35 (m, 1H), 4.13-4.20 (m, 3H), 3.92-3.94 (m, 1H), 3.18-3.25 (m, 1H), 3.04-3.07 (m, 1H), 1.92-2.13 (m, 4H), 1.45 (t, *J* = 6.8Hz, 3H).

**Example 46: Preparation of compound 156**

Step A:

6-ethoxy-4-(6-((1*R*,5*S*)-8-((6-methoxypyridin-3-yl)methyl)-3,8-diazabicyclo[3.2.1]octan-3-yl)pyridi n-3-yl)-1*H*-pyrazolo[3',4':3,4]pyrazolo[1,5-*a*]pyrazole

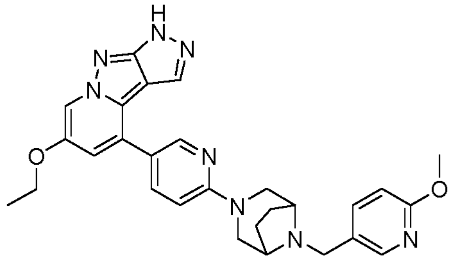

4-(6-((1*R*,5*S*)-3,8-diazabicyclo[3.2.1]octan-3-yl)pyridin-3-yl)-6-ethoxy-1*H*-pyrazolo[3',4':3,4]pyraz olo[1,5-*a*]pyridine hydrochloride (100 mg, 0.2 mmol), 6-methoxy-3-pyridinecarboxaldehyde (82 mg, 0.6 mmol), TEA (121 mg, 1.2 mmol), 2-picoline borane (64 mg, 0.6 mmol), and 3 mL of *n*-propanol were added to a reaction flask. The mixture was reacted at 45 °C for 12 h. The reaction was quenched by adding water and stirring. The reaction liquid was extracted with dichloromethane, dried over anhydrous sodium sulfate, concentrated under reduced pressure, and separated by column chromatography to give a product (37.6 mg), m/z = 511 $[M+1]^+$. $^1H$ NMR (400 MHz, $CD_3OD$-$d_3$) $\delta$ 8.53 (s, 1H), 8.27 (s, 1H), 8.13 (s, 1H), 7.95 (d, *J* = 8.8Hz, 1H), 7.79 (d, *J* = 8.4Hz, 1H), 7.66 (s, 1H), 7.19 (s, 1H), 6.89 (d, *J* = 8.8Hz, 1H), 6.80 (d, *J* = 8.4Hz, 1H), 4.11 (q, *J* = 6.8Hz, 2H), 3.93-3.97 (m, 2H), 3.91(s, 3H), 3.59 (s, 2H), 3.35-3.38 (m, 2H), 3.15-3.18 (m, 2H), 2.15-2.18 (m, 2H), 1.45 (t, *J* = 6.8Hz, 3H).

**Example 47: Preparation of compound 157**

Step A: *tert*-butyl (1*R*,5*S*)-8-(5-(6-ethoxy-1*H*-pyrazolo[3',4':3,4]pyrazolo[1,5-*a*]pyridin-4-yl)pyridin-2-yl)-3,8-diazabi cyclo[3.2.1]octane-3-carbonate

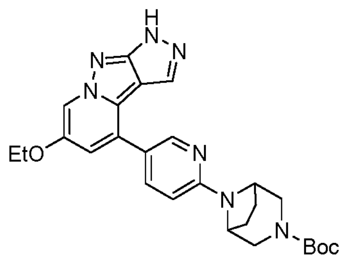

6-ethoxy-4-(6-fluoropyridin-3-yl)-1*H*-pyrazolo[3',4':3,4]pyrazolo[1,5-*a*]pyridine (292 mg, 1.0 mmol), *tert*-butyl 3,8-diazabicyclo[3.2.1]octane-3-carbonate (250 mg, 1.2 mmol), DIEA (506 mg, 3.9 mmol), and 2 mL of DMSO were added to a reaction flask. The mixture was reacted at 120 °C for 16 h. The reaction was quenched by adding water and stirring. The reaction liquid was extracted with dichloromethane, dried over anhydrous sodium sulfate, concentrated under reduced pressure, and separated by column chromatography to give a product (220 mg), m/z = 490 $[M+1]^+$.

Step B: 4-(6-((1*R*,5*S*)-3,8-diazabicyclo[3.2.1]octan-8-yl)pyridin-3-yl)-6-ethoxy-1*H*-pyrazolo[3',4':3,4]pyraz olo[1,5-*a*]pyridine hydrochloride

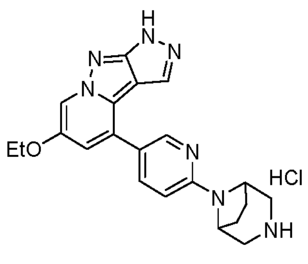

*Tert*-butyl

(1*R*,5*S*)-3-(5-(6-ethoxy-1*H*-pyrazolo[3',4':3,4]pyrazolo[1,5-*a*]pyridin-4-yl)pyridin-2-yl)-3,8-diazabi cyclo[3.2.1]octane-8-carbonate (200 mg, 0.4 mmol), 3 mL of methanol, and a 5 N methanolic hydrochloric acid solution (3 mL, 15 mmol) were added to a reaction flask. The mixture was reacted at 25 °C for 4 h. After the reaction was completed, the reaction liquid was concentrated under reduced pressure to give a product (280 mg), which was directly used in the next step, m/z = 390 $[M+1]^+$.

Step C: (2-chloro-6-fluoro)((1*R*,5*S*)-8-(5-(6-ethoxy-1*H*-pyrazolo[3',4':3,4]pyrazolo[1,5-*a*]pyridin-4-yl)pyrid in-2-yl)-3,8-diazabicyclo[3.2.1]octan-3-yl)benzamide

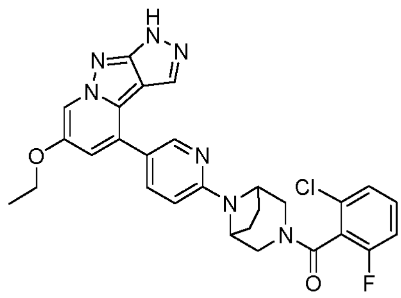

4-(6-((1*R*,5*S*)-3,8-diazabicyclo[3.2.1]octan-8-yl)pyridin-3-yl)-6-ethoxy-1*H*-pyrazolo[3',4':3,4]pyraz olo[1,5-*a*]pyridine hydrochloride (100 mg, 0.2 mmol), 3-chloro-6-fluorobenzoyl chloride (43 mg, 0.22 mmol), DIEA (155 mg, 1.2 mmol), and 2 mL of DMF were added to a reaction flask. The mixture was reacted at 25 °C for 1 h. The reaction was quenched by adding water and stirring. The reaction liquid was extracted with dichloromethane, dried over anhydrous sodium sulfate, concentrated under reduced pressure, and separated by column chromatography to give a product (23.2 mg), m/z = 546 $[M+1]^+$. $^1$H NMR (400 MHz, $CD_3OD$-$d_3$) δ 8.59 (s, 1H), 8.30 (s, 1H), 8.02 (d, *J* = 8.8Hz, 1H), 7.65 (s, 1H), 7.46-7.52 (m, 1H), 7.42-7.44 (m, 1H), 7.17-7.27 (m, 2H), 7.03 (d, *J* = 8.8Hz, 1H), 4.82-4.87 (m, 1H), 4.63-4.68 (m, 1H), 4.42 (t, *J* = 11.2Hz, 1H), 4.12 (q, *J* = 7.2Hz, 2H), 3.57 (t, *J* = 13.6Hz, 1H), 3.18-2.24 (m, 1H), 1.92-2.18 (m, 4H), 1.45 (t, *J* = 6.8Hz, 3H).

**Example 48: Preparation of compound 158**

Step A: 6-ethoxy-4-(6-((1*R*,5*S*)-3-((6-methoxypyridin-3-yl)methyl)-3,8-diazabicyclo[3.2.1]octan-3-yl)pyridi n-8-yl)-1*H*-pyrazolo[3',4':3,4]pyrazolo[1,5-*a*]pyrazole

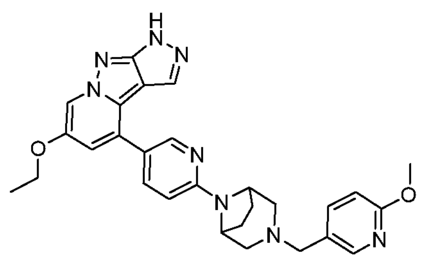

4-(6-((1*R*,5*S*)-3,8-diazabicyclo[3.2.1]octan-8-yl)pyridin-3-yl)-6-ethoxy-1*H*-pyrazolo[3',4':3,4]pyraz olo[1,5-*a*]pyridine hydrochloride (100 mg, 0.2 mmol), 6-methoxy-3-pyridinecarboxaldehyde (82 mg, 0.6 mmol), TEA (121 mg, 1.2 mmol), 2-picoline borane (64 mg, 0.6 mmol), and *n*-propanol (3 mL) were added to a reaction flask. The mixture was reacted at 45 °C for 12 h. The reaction was quenched by adding water and stirring. The mixture was extracted with dichloromethane, dried over anhydrous sodium sulfate, concentrated under reduced pressure, and separated by column chromatography to give a product (45.5 mg), m/z = 511 $[M+1]^+$. $^1$H NMR (400 MHz, $CD_3OD$-$d_3$) δ 8.55 (s, 1H), 8.32 (s, 1H), 8.00-8.10 (m, 2H), 7.68-7.72 (m, 2H), 7.25 (s, 1H), 6.95 (d, *J* = 9.2Hz, 1H), 6.78 (d, *J* = 8.8Hz, 1H), 4.63 (s, 2H),

4.17 (q, $J$ = 6.8Hz, 2H), 3.88(s, 3H), 3.42-3.45 (m, 2H), 2.68-2.72 (m, 2H), 2.42-2.48 (m, 2H), 2.02-2.11 (m, 2H), 1.95-2.00 (m, 2H), 1.46 (t, $J$ = 6.8Hz, 3H).

**Example 49: Preparation of compound 159**

Step A: *tert*-butyl 5-(5-(6-ethoxy-1*H*-pyrazolo[3',4':3,4]pyrazolo[1,5-*a*]pyridin-4-yl)pyridin-2-yl)-2,5-diazabicyclo[2. 2.2]octane-2-carboxylate

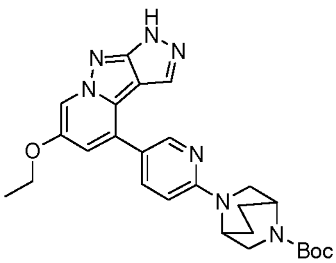

6-ethoxy-4-(6-fluoropyridin-3-yl)-1*H*-pyrazolo[3',4':3,4]pyrazolo[1,5-*a*]pyridine (318 mg, 1.1 mmol), *tert*-butyl 2,5-diazabicyclo[2.2.2]octane-2-carboxylate (250 mg, 1.2 mmol), DIEA (414 mg, 3.2 mmol), and DMSO (5 mL) were added to a reaction flask. The mixture was reacted at 90 °C for 12 h. The mixture was quenched by adding water and stirring. The reaction liquid was extracted with dichloromethane, dried over anhydrous sodium sulfate, concentrated under reduced pressure, and separated by column chromatography to give a product (500 mg), m/z = 490 $[M+1]^+$.

Step B: 4-(6-(2,5-diazabicyclo[2.2.2]octan-2-yl)pyridin-3-yl)-6-ethoxy-1*H*-pyrazolo[3',4':3,4]pyrazolo[1,5-*a*]pyridine hydrochloride

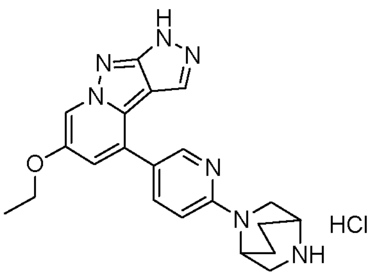

*Tert*-butyl

5-(5-(6-ethoxy-1*H*-pyrazolo[3',4':3,4]pyrazolo[1,5-*a*]pyridin-4-yl)pyridin-2-yl)-2,5-diazabicyclo[2. 2.2]octane-2-carboxylate (500 mg, 1.02 mmol) was added to 5 mL of MeOH/HCl (4 M). The mixture was reacted at 25 °C for 4 h. The reaction liquid was concentrated by rotary evaporation to remove the solvent to give a product (500 mg), which was directly used in the next step, m/z = 390 $[M+1]^+$.

Step C: (2-chloro-6-fluorophenyl)-5-(5-(6-ethoxy-1*H*-pyrazolo[3',4':3,4]pyrazolo[1,5-*a*]pyridin-4-yl)pyridin -2-yl)-2,5-diazabicyclo[2.2.2]octan-2-yl)methanone

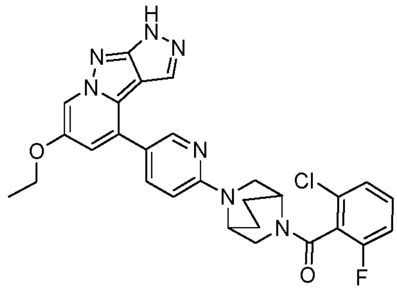

4-(6-(2,5-diazabicyclo[2.2.2]octan-2-yl)pyridin-3-yl)-6-ethoxy-1*H*-pyrazolo[3',4':3,4]pyrazolo[1,5-*a*]pyridine hydrochloride (500 mg, 1.18 mmol), 2-chloro-6-fluorobenzoyl chloride (250 mg, 1.29 mmol), $Et_3N$ (357.5 mg, 3.54 mmol), and DMF (5 mL) were added to a reaction flask. The mixture was reacted at 25 °C for 12 h. The reaction was quenched by adding water and stirring. The reaction liquid was extracted with dichloromethane, dried over anhydrous sodium sulfate, concentrated under reduced pressure, and separated by column chromatography to give a product (412 mg), m/z = 546 $[M+1]^+$. $^1H$ NMR (400 MHz, DMSO-$d_6$) δ 12.64 (s, 1H), 8.59 (d, *J* = 2.5 Hz, 1H), 8.52 (d, *J* = 1.9 Hz, 1H), 8.07 (dd, *J* = 8.9, 2.5 Hz, 1H), 7.63-7.58 (m, 1H), 7.58-7.51 (m, 1H), 7.51-7.46 (m, 1H), 7.45-7.36 (m, 1H), 7.25 (m, 1H), 6.81 (d, *J* = 8.9 Hz, 1H), 4.18 (m, 2H), 3.85-3.70 (m, 4H), 3.67-3.53 (m, 2H), 2.04-1.95 (m, 2H), 1.90 (d, *J* = 9.3 Hz, 2H), 1.40 (m, 3H).

**Example 50: Preparation of compound 160**

6-ethoxy-4-(6-(5-((6-methoxypyridin-3-yl)methyl)-2,5-diazabicyclo[2.2.2]octan-2-yl)pyridin-3-yl)-1*H*-pyrazolo[3',4':3,4]pyrazolo[1,5-*a*]pyridine

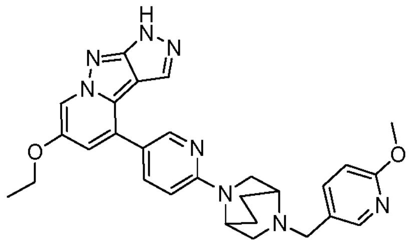

4-(6-(2,5-diazabicyclo[2.2.2]octan-2-yl)pyridin-3-yl)-6-ethoxy-1*H*-pyrazolo[3',4':3,4]pyrazolo[1,5-*a*]pyridine hydrochloride (500 mg, 1.18 mmol), 6-methoxy-3-pyridinecarboxaldehyde (176 mg, 1.29 mmol), $Et_3N$ (357.5 mg, 3.54 mmol), Pic-$BH_3$ (378.8 mg, 3.54 mmol), and *n*-propanol (5mL) were added to a reaction flask. The mixture was reacted at 25 °C for 12 h. The reaction was quenched by adding water and stirring, extracted with dichloromethane, dried over anhydrous sodium sulfate, concentrated under reduced pressure, and separated by column chromatography to give a product (351 mg), m/z = 511 $[M+1]^+$, $^1H$ NMR (400 MHz, DMSO-$d_6$) δ 12.64 (s, 1H), 8.54 (d, *J* = 2.5 Hz, 1H), 8.50 (d, *J* = 2.1 Hz, 1H), 8.11 (d, *J* = 2.3 Hz, 1H), 8.03 (m, 1H), 7.70 (m, 1H), 7.60 (s, 1H), 7.23 (d, *J* = 2.1 Hz, 1H), 6.79 (d, *J* = 8.4 Hz, 1H), 6.75 (d, *J* = 8.9 Hz, 1H), 4.17 (m, 2H), 3.84 (s, 3H), 3.71 (m, 2H), 3.41 (d, *J* = 10.9 Hz, 1H), 3.33 (d, *J* = 9.5 Hz, 3H), 2.99 (s, 1H), 2.94-2.80 (m, 2H), 2.08 (s, 1H), 1.85 (t, *J* = 8.0 Hz, 2H), 1.40 (t, *J* = 6.9 Hz, 3H).

**Example 51: Preparation of compound 161**

(2-chloro-6-fluorophenyl)(6-(5-(6-ethoxy-1*H*-pyrazolo[3',4':3,4]pyrazolo[1,5-*a*]pyridin-4-yl)pyridin -2-yl)-3,6-diazabicyclo[3.1.1]heptan-3-yl)methylene

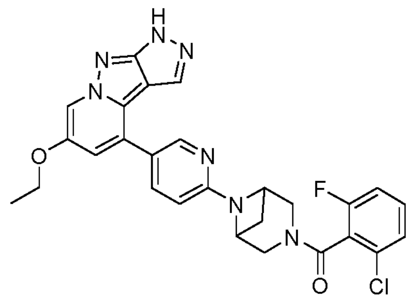

4-(6-(3,6-diazabicyclo[3.1.1]heptan-6-yl)pyridin-3-yl)-6-ethoxy-1*H*-pyrazolo[3',4':3,4]pyrazolo[1,5 -a]pyridine (120 mg, 0.18 mmol), DMF (2 mL), and triethylamine (90.5 mg, 0.9 mmol) were sequentially added to a reaction flask. The mixture was cooled to 5 °C, and 2-fluoro-5-chlorobenzoyl chloride (41.5 mg, 1.25 mmol) was added dropwise. The mixture was stirred at 5 °C for 2 h. The reaction liquid was quenched by adding water and stirring, extracted with dichloromethane, dried over anhydrous sodium sulfate, concentrated under reduced pressure, and separated by column chromatography to give a product (50 mg), m/z = 532 $[M+1]^+$, $^1H$ NMR (400MHz, DMSO-*d*6) δ 12.66 (s, 1H), 8.55-8.60 (m, 2H), 8.07 (s, 1H), 7.23-7.51 (m, 5H), 6.86-6.88 (m, 1H), 4.58 (s, 1H), 4.42 (s, 1H), 4.16-4.26 (m, 3H), 3.51-3.82 (m, 2H), 2.77-2.80 (m, 1H), 1.68-1.71 (d, 1H), 1.40-1.42 (m, 3H).

**Example 52: Preparation of compound 164**

Step A: *tert*-butyl 7-(5-(6-ethoxy-1*H*-pyrazolo[3',4':3,4]pyrazolo[1,5-*a*]pyridin-4-yl)pyridin-2-yl)-4,7-diazaspiro[2.5]o ctane-4-carboxylate

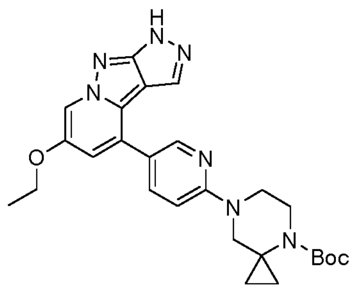

6-ethoxy-4-(6-fluoropyridin-3-yl)-1*H*-pyrazolo[3',4':3,4]pyrazolo[1,5-*a*]pyridine (700 mg, 2.4 mmol), *tert*-butyl 4,7-diazaspiro[2.5]octane-4-carboxylate (500 mg, 2.4 mmol), DIEA (1.5 g, 11.6 mmol), and DMSO (10 mL) were added to a reaction flask. The mixture was reacted at 90 °C for 12 h. The reaction was quenched by adding water and stirring. The reaction liquid was extracted with dichloromethane, dried over anhydrous sodium sulfate, concentrated under reduced pressure, and separated by column chromatography to give a product (670 mg), m/z = 491 $[M+1]^+$.

Step B: 4-(6-(4,7-diazaspiro[2.5]octan-7-yl)pyridin-3-yl)-6-ethoxy-1*H*-pyrazolo[3',4':3,4]pyrazolo[1,5-*a*]py ridine hydrochloride

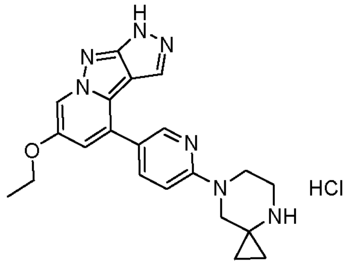

*Tert*-butyl

7-(5-(6-ethoxy-1*H*-pyrazolo[3',4':3,4]pyrazolo[1,5-*a*]pyridin-4-yl)pyridin-2-yl)-4,7-diazaspiro[2.5]o ctane-4-carboxylate (670 mg, 1.4 mmol) was added to a solution of HCl in methanol (4 M, 20 mL). The mixture was reacted at 25 °C for 4 h. The reaction liquid was concentrated by rotary evaporation to remove the solvent to give a product (680 mg), m/z = 391 $[M+1]^+$.

Step C: *N*-(7-(5-(6-ethoxy-1*H*-pyrazolo[3',4':3,4]pyrazolo[1,5-*a*]pyridin-4-yl)pyridin-2-yl)-4,7-diazaspiro[2. 5]octan-4-yl)-2-chloro-6-fluorobenzamide

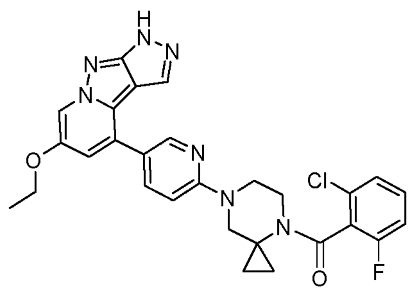

4-(6-(4,7-diazaspiro[2.5]octan-7-yl)pyridin-3-yl)-6-ethoxy-1*H*-pyrazolo[3',4':3,4]pyrazolo[1,5-*a*]py ridine hydrochloride (200 mg, 0.4 mmol), 2-chloro-6-fluorobenzoyl chloride (77 mg, 0.4 mmol), DIEA (258 mg, 2 mmol), and DCM (10 mL) were added to a reaction flask. The mixture was reacted at 25 °C for 1 h. The reaction liquid was quenched by adding water and stirring. The reaction liquid was extracted with dichloromethane, dried over anhydrous sodium sulfate, concentrated under reduced pressure, and separated by column chromatography to give a product (100 mg), m/z = 546 $[M+1]^+$, $^1$H-NMR (400 MHz, DMSO-$d_6$) δ 12.64 (brs, 1H), 8.58 (s, 1H), 8.52 (s, 1H), 8.06 (d, *J* = 8.8 Hz, 1H), 7.50-7.59 (m, 2H), 7.33-7.48 (m, 2H), 7.26 (s, 1H), 7.05 (t, *J* = 11.2 Hz, 1H), 4.05-4.28 (m, 3H), 3.48-3.88 (m, 4H), 3.38-3.46 (m, 1H),1.38 (t, *J* = 6.8 Hz, 3H), 1.02-1.28 (m, 2H), 0.76-0.82 (m, 1H), 0.68-0.75 (m, 1H).

**Example 53: Preparation of compound 165**

6-ethoxy-4-(6-(4-((6-methoxypyridin-3-yl)methylene)-4,7-diazaspiro[2.5]octan-7-yl)pyridin-3-yl)-1 *H*-pyrazolo[3',4':3,4]pyrazolo[1,5-*a]*pyridine

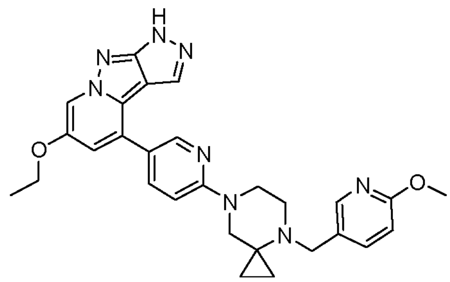

4-(6-(4,7-diazaspiro[2.5]octan-7-yl)pyridin-3-yl)-6-ethoxy-1*H*-pyrazolo[3',4':3,4]pyrazolo[1,5-*a*]py ridine hydrochloride (200 mg, 0.4 mmol), 6-methoxy-3-pyridinecarboxaldehyde (165 mg, 1.2 mmol), $Et_3N$ (202 mg, 2.0 mmol), Pic-$BH_3$ (128 mg, 1.2 mmol), and *n*-propanol (10 mL) were added to a reaction flask. The mixture was reacted at 40 °C for 48 h. The reaction was quenched by adding water and stirring. The reaction liquid was extracted with dichloromethane, dried over anhydrous sodium sulfate, concentrated under reduced pressure, and separated by column chromatography to give a product (30.8 mg), m/z = 511 $[M+1]^+$, $^1$H-NMR (400 MHz, DMSO-$d_6$) δ 12.64 (brs, 1H), 8.57 (s, 1H), 8.51 (s, 1H), 8.09 (s, 1H), 8.02 (d, *J* = 8.8 Hz, 1H), 7.62 (d, *J* = 8.4 Hz, 1H), 7.57 (s, 1H), 7.25 (s, 1H), 7.02 (d, *J* = 8.8 Hz, 1H), 6.78 (d, *J* = 8.4 Hz, 1H), 4.15 (q, *J* = 6.8 Hz, 2H), 3.86 (s, 2H), 3.84 (s, 3H), 3.62-68 (m, 2H), 3.56 (s, 2H), 2.78-2.86 (m, 2H),1.38 (t, *J* = 7.2 Hz, 3H), 0.60-0.71 (m, 4H).

**Example 54: Preparation of compound 169**

Step A: *tert*-butyl 7-(5-(6-ethoxy-1*H*-pyrazolo[3',4':3,4]pyrazolo[1,5-*a*]pyridin-4-yl)pyridin-2-yl)-3-oxa-7,9-diazabicy clo[3.3.1]nonane-9-carboxylate

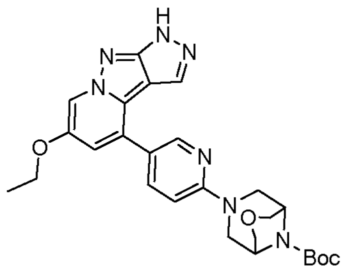

6-ethoxy-4-(6-fluoropyridin-3-yl)-1*H*-pyrazolo[3',4':3,4]pyrazolo[1,5-*a*]pyridine (120 mg, 0.4 mmol) *tert*-butyl 3-oxa-7,9-azabicyclo[3.3.1]nonane-9-carboxylate (100 mg, 0.44 mmol), DIEA (155 mg, 1.2 mmol), and DMSO (2 mL) were added to a reaction flask. The mixture was reacted at 90 °C for 16 h. The reaction was quenched by adding water and stirring. The reaction liquid was extracted with dichloromethane, dried over anhydrous sodium sulfate, concentrated under reduced pressure, and separated by column chromatography to give a product (150 mg), m/z = 506 $[M+1]^+$.

Step B: 7-(5-(6-ethoxy-1*H*-pyrazolo[3',4':3,4]pyrazolo[1,5-*a*]pyridin-4-yl)pyridin-2-yl)-3-oxa-7,9-diazabicy clo[3.3.1]nonane hydrochloride

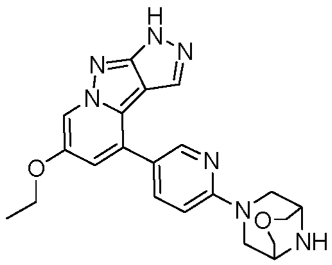

HCl

*Tert*-butyl

7-(5-(6-ethoxy-1*H*-pyrazolo[3',4':3,4]pyrazolo[1,5-*a*]pyridin-4-yl)pyridin-2-yl)-3-oxa-7,9-azabicycl o[3.3.1]nonane-9-carboxylate (150 mg, 0.3 mmol) was added to a solution of HCl in methanol (4 M, 5 mL). The mixture was reacted at 25 °C for 4 h. The reaction liquid was concentrated by rotary evaporation to remove the solvent to give a product (160 mg), m/z = 406 $[M+1]^+$.

Step C: *N*-(7-(5-(6-ethoxy-1*H*-pyrazolo[3',4':3,4]pyrazolo[1,5-*a*]pyridin-4-yl)pyridin-2-yl)-3-oxa-7,9-azabic yclo[3.3.1]nonan-9-yl)-2-chloro-6-fluorobenzamide

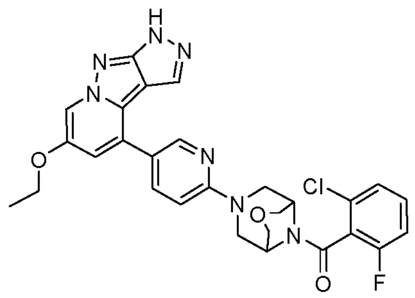

7-(5-(6-ethoxy-1*H*-pyrazolo[3',4':3,4]pyrazolo[1,5-*a*]pyridin-4-yl)pyridin-2-yl)-3-oxa-7,9-azabicycl o[3.3.1]nonane (60 mg; 0.1 mmol), 2-chloro-6-fluorobenzoyl chloride (23 mg, 0.1 mmol), $Et_3N$ (50 mg, 0.5 mmol), and DMF (2 mL) were added to a reaction flask. The mixture was reacted at 25 °C for 16 h. The reaction was quenched by adding water and stirring. The reaction liquid was extracted with dichloromethane, dried over anhydrous sodium sulfate, concentrated under reduced pressure, and separated by column chromatography to give a product (15 mg), m/z = 562 $[M+1]^+$, $^1H$-NMR (400 MHz, DMSO-$d_6$) $\delta$ 12.66 (s, 1H), 8.60 (d, *J* = 2.5 Hz, 1H), 8.53 (d, *J* = 2.1 Hz, 1H), 8.08 (dd, *J* = 8.9, 2.6 Hz, 1H), 7.60 (s, 1H), 7.53-7.64 (m, 1H), 7.50 (d, *J* = 8.3 Hz, 1H), 7.43 (t, *J* = 8.6 Hz, 1H), 7.27 (d, *J* = 2.0 Hz, 1H), 7.02 (d, *J* = 9.0 Hz, 1H), 4.75 (s, 1H), 4.69 (d, *J* = 11.5 Hz, 1H), 4.53 (t, *J* = 12.6 Hz, 1H), 4.18 (q, *J* = 7.0 Hz, 2H), 4.11 (d, *J* = 11.5 Hz, 1H), 3.94 (dd, *J* = 11.1, 5.5 Hz, 1H), 3.76 (s, 1H), 3.62 (d, *J* = 13.8 Hz, 1H), 3.32 (d, *J* = 11.8 Hz, 2H), 3.15 (d, *J* = 12.8 Hz, 1H), 1.40 (t, *J*= 6.9 Hz, 3H).

**Example 55: Preparation of compound 114**

Step A: 7-(5-(6-ethoxy-1*H*-pyrazolo[3',4':3,4]pyrazolo[1,5-*a*]pyridin-4-yl)pyridin-2-yl)-9-((6-methoxypyrid in-3-yl)methylene)-3-oxa-7,9-diazabicyclo[3.3.1]nonane

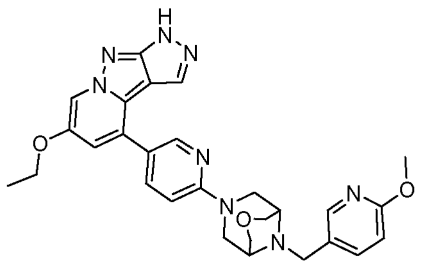

7-(5-(6-ethoxy-1*H*-pyrazolo[3',4':3,4]pyrazolo[1,5-*a*]pyridin-4-yl)pyridin-2-yl)-3-oxa-7,9-azabicycl o[3.3.1]nonane (100 mg, 0.2 mmol), 6-methoxy-3-pyridinecarboxaldehyde (83 mg, 0.6 mmol), Pic-$BH_3$ (64 mg, 0.6 mmol), $Et_3N$ (120 mg, 1.2 mmol), and *n*-propanol (5 mL) were added to a reaction flask. The mixture was reacted at 45 °C for 16 h. The reaction was quenched by adding water and stirring. The reaction liquid was extracted with dichloromethane, dried over anhydrous sodium sulfate, concentrated under reduced pressure, and separated by column chromatography to give a product (6.5 mg), m/z = 527 $[M+1]^+$, $^1H$ NMR (400 MHz, DMSO-$d_6$) $\delta$ 12.66 (s, 1H), 8.61 (d, *J* = 2.6 Hz, 1H), 8.52 (d, *J* = 2.0 Hz, 1H), 8.18 (d, *J* = 2.3 Hz, 1H), 8.06 (dd, *J* = 8.9, 2.6 Hz, 1H), 7.77 (dd, *J* = 8.5, 2.4 Hz, 1H), 7.60 (s, 1H), 7.26 (d, *J* = 2.1 Hz, 1H), 6.95 (d, *J* = 9.0 Hz, 1H), 6.83 (d, *J* = 8.5 Hz, 1H), 4.19 (q, *J* = 6.9 Hz, 2H), 4.05 (d, *J* = 13.1 Hz, 2H), 3.93 (s, 2H), 3.83 (d, *J* = 14.8 Hz, 7H), 3.47-3.57 (m, 2H), 2.83 (d, *J* = 3.3 Hz, 2H), 1.41 (t, *J* = 6.9 Hz, 3H).

**Example 56: Preparation of compound 149**

6-ethoxy-4-(6-(6-(4-(methylsulfonyl)benzylidene)-3,6-diazabicyclo[3.1.1]heptan-3-yl)-pyridin-3-yl )-1*H*-pyrazolo[3',4':3,4]pyrazolo[1,5-*a*]pyridine

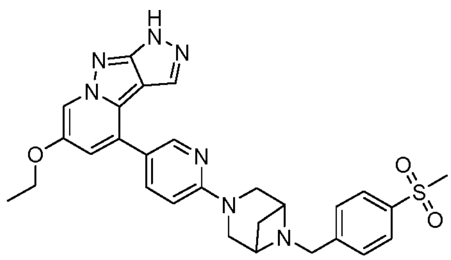

4-(6-(3,6-diazabicyclo[3.1.1]heptan-3-yl)-6-ethoxy-1*H*-pyrazolo[3',4':3,4]pyrazolo[1,5-*a*]pyridine (300 mg, 0.8 mmol), 6-methoxy-3-pyridinecarboxaldehyde (165 mg, 1.2 mmol), $Et_3N$ (202 mg, 2.0 mmol), Pic-$BH_3$ (128 mg, 1.2 mmol), and DMSO (10 mL) were added to a reaction flask. The mixture was reacted at 45 °C for 48 h. The reaction was quenched by adding water and stirring. The reaction liquid was extracted with dichloromethane, dried over anhydrous sodium sulfate, concentrated under reduced pressure, and separated by column chromatography to give a product (71.4 mg), m/z = 544 $[M+1]^+$, $^1$H-NMR (400 MHz, DMSO-$d_6$) δ 12.65 (brs, 1H), 8.65 (s, 1H), 8.52 (s, 1H), 8.11 (d, *J* = 8.8 Hz, 1H), 7.87 (d, *J* = 8.0 Hz, 1H), 7.58-7.70 (m, 3H), 7.27 (s, 1H), 6.91 (d, *J* = 8.8 Hz, 1H), 4.16-4.21 (m, 2H), 3.51-3.83 (m, 8H), 3.20 (s, 3H), 2.58-2.65 (m, 1H), 1.57-1.65 (m, 1H), 1.39-1.42 (m, 3H).

## Example 57: Preparation of compound 170

2-chloro-*N*-(((1*R*,5*S*,6*S*)-3-(5-(6-ethoxy-1*H*-pyrazolo[3',4':3,4]pyrazolo[1,5-*a*]pyridin-4-yl)pyridin-2-yl)-3-azabicyclo[3.1.0]hexan-6-yl)-6-fluorobenzamide

Step A: (1*R*,5*S*,6*S*)-3-(5-(6-ethoxy-1*H*-pyrazolo[3',4':3,4]pyrazolo[1,5-*a*]pyridin-4-yl)pyridin-2-yl)-3-azabic yclo[3.1.0]-6-hexylamine hydrochloride

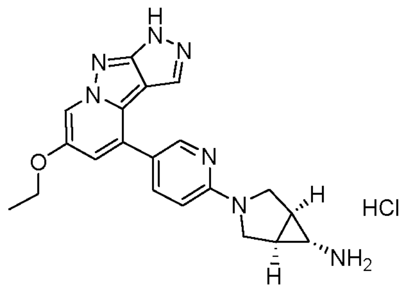

6-ethoxy-4-(6-fluoropyridin-3-yl)-1*H*-pyrazolo[3',4':3,4]pyrazolo[1,5-*a*]pyridine (311 mg, 1.05 mmol), DMSO (6 mL), DIEA (407 mg, 3.15 mmol), and ((1*R*,5*S*,6*S*)-3-azabicyclo[3.1.0]-6-hexylamine carbonate (250 mg, 1.26 mmol) were added to a reaction flask. The mixture was heated to 90 °C and reacted for 24 h. The reaction was stopped and quenched by adding water and stirring. The reaction liquid was extracted with dichloromethane, dried over anhydrous sodium sulfate, and concentrated under reduced pressure, and then methanol (2 mL) and 1,4-dioxane/HCl (4 M, 1 mL) were added. The mixture was stirred at room temperature for 1 h. The reaction liquid was directly concentrated to give a product (0.4 g), m/z = 376 $[M+1]^+$.

Step B: 2-chloro-*N*-((1*R*,5*S*,6*S*)-3-(5-(6-ethoxy-1*H*-pyrazolo[3',4':3,4]pyrazolo[1,5-*a*]pyridin-4-yl)pyridin-2 -yl)-3-azabicyclo[3.1.0]hexan-6-yl)-6-fluorobenzamide

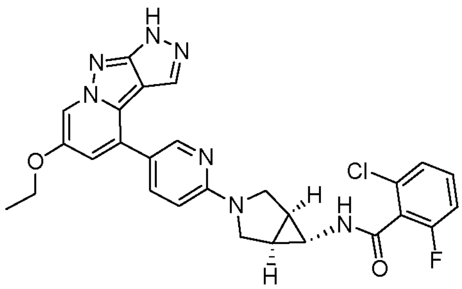

(1*R*,5*S*,6*S*)-3-(5-(6-ethoxy-1*H*-pyrazolo[3',4':3,4] pyrazolo[1,5-*a*]pyridin-4-yl)pyridin-2-yl)-3-azabicyclo[3.1.0]-6-hexylamine hydrochloride (200 mg, 0.41 mmol), 2 mL of DMF, and triethylamine (208 mg, 2.06 mmol) were added to a reaction flask. The mixture was cooled to 5 °C. 2-fluoro-5-chlorobenzoyl chloride was slowly added dropwise to the reaction liquid. After the dropwise addition, the mixture was stirred for 1 h. The reaction was quenched by adding water and stirring. The reaction liquid was extracted with dichloromethane, dried over anhydrous sodium sulfate, concentrated under reduced pressure, and separated by column chromatography to give a product (12 mg). M/z = 532 $[M+1]^+$, $^1H$ NMR (400MHz, DMSO-$d_6$) δ 12.64 (s, 1H), 8.94-8.95 (m, 1H), 8.46-8.56 (m, 2H), 8.01-8.04 (m, 1H), 7.52-7.58 (m, 1H), 7.47-7.51 (m, 1H), 7.38-7.40 (m, 1H), 7.29-7.34 (m, 1H), 7.23-7.24 (m, 1H), 6.73-6.75 (m, 1H), 4.15-4.20 (m, 2H), 3.86-3.96 (m, 2H), 3.55-3.58 (d, 2H), 2.66-2.67 (m, 1H), 1.95 (s, 1H), 1.37-1.41 (m, 3H).

**Example 58: Preparation of compound 171**

(1*R*,5*S*,6*S*)-3-(5-(6-ethoxy-1*H*-pyrazolo[3',4':3,4]pyrazolo[1,5-*a*]pyridin-4-yl)pyridin-2-yl)-*N*-((6-m ethoxypyridin-3-yl)methylene)-3-azabicyclo[3.1.0]6-hexylamine

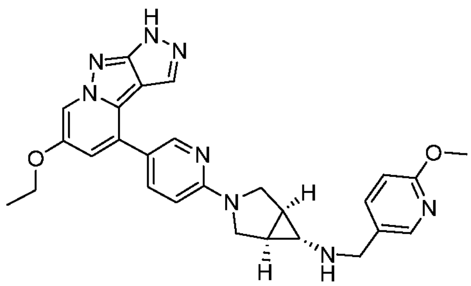

(1*R*,5*S*,6*S*)-3-(5-(6-ethoxy-1*H*-pyrazolo[3',4':3,4]pyrazolo[1,5-*a*]pyridin-4-yl)pyridin-2-yl)-3-azabic yclo[3.1.0]-6-hexylamine hydrochloride (200 mg, 0.41 mmol), DMSO (4 mL), triethylamine (208 mg, 2.06 mmol), and 6-methoxy-3-pyridinecarboxaldehyde (170 mg, 1.24 mmol) were added to a reaction flask, and 2-picoline borane (132.4 mg, 1.24 mmol) was added dropwise. The mixture was reacted at room temperature for 1 h. The reaction was quenched by adding water and stirring. The reaction liquid was extracted with dichloromethane, dried over anhydrous sodium sulfate, concentrated under reduced pressure, and separated by column chromatography to give a product (3 mg), m/z = 497 $[M+1]^+$, $^1H$ NMR (400MHz, DMSO-$d_6$) δ 12.63 (s, 1H), 8.50-8.56 (m, 2H), 7.98-8.08 (m, 2H), 7.57-7.65 (m, 2H), 7.20-7.23 (m, 1H), 6.81-6.87 (m, 1H), 6.61-6.64 (m, 1H), 4.14-4.19 (m, 2H), 3.85-3.90 (m, 5H), 3.60-3.63 (m, 5H), 1.52-1.55 (m, 2H), 1.39-1.46 (m, 3H), 1.20 (s, 1H).

**Example 59: Preparation of compound 172**

2-chloro-*N*-(((1*R*,5*S*,6*R*)-3-(5-(6-ethoxy-1*H*-pyrazolo[3',4':3,4]pyrazolo[1,5-*a*]pyridin-4-yl)pyridin-2-yl)-3-azabicyclo[3.1.0]hexyl-6-yl)-6-fluorobenzamide

Step A: (1*R*,5*S*,6*R*)-3-(5-(6-ethoxy-1*H*-pyrazolo[3',4':3,4]pyrazolo[1,5-*a*]pyridin-4-yl)pyridin-2-yl)-3-azabic yclo[3.1.0]-6-hexylamine hydrochloride

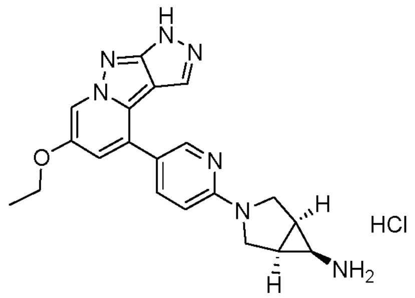

6-ethoxy-4-(6-fluoropyridin-3-yl)-1*H*-pyrazolo[3',4':3,4]pyrazolo[1,5-*a*]pyridine (311 mg, 1.05 mmol), DMSO (2 mL), DIEA (407 mg, 3.15 mmol), and ((1*R*,5*S*,6*R*)-3-azabicyclo[3.1.0]-6-hexylamine carbonate (250 mg, 1.26 mmol) were added to a reaction flask. The mixture was heated to 90 °C and reacted for 24 h. The reaction was quenched by adding water and stirring. The reaction liquid was extracted with dichloromethane, dried over anhydrous sodium sulfate, and concentrated under reduced pressure, and then methanol (2 mL) and 1,4-dioxane/HCl (4 M, 2 mL) were added to the residue. The mixture was stirred at room temperature for 2 h. The reaction liquid was concentrated under reduced pressure to give a product (0.4 g), which was directly used in the next step, m/z = 376 $[M+1]^+$.

Step B: 2-chloro-*N*-((1*R*,5*S*,6*R*)-3-(5-(6-ethoxy-1*H*-pyrazolo[3',4':3,4]pyrazolo[1,5-*a*]pyridin-4-yl)pyridin-2 -yl)-3-azabicyclo[3.1.0]hexan-6-yl)-6-fluorobenzamide

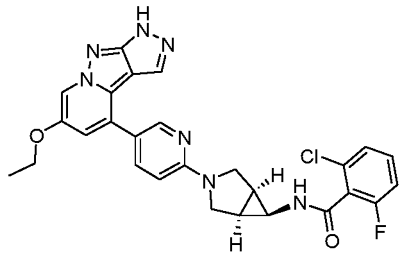

(1*R*,5*S*,6*R*)-3-(5-(6-ethoxy-1*H*-pyrazolo[3',4':3,4]pyrazolo[1,5-*a*]pyridin-4-yl)pyridin-2-yl)-3-azabic yclo[3.1.0]-6-hexylamine hydrochloride (200 mg, 0.41 mmol), 2 mL of DMF, and triethylamine (208 mg, 2.06 mmol) were added to a reaction flask. The mixture was cooled to 5 °C. 2-fluoro-5-chlorobenzoyl chloride was slowly added dropwise to the reaction liquid. After the dropwise addition, the mixture was stirred at room temperature for 1 h. The reaction was quenched by adding water and stirring. The reaction liquid was extracted with dichloromethane, dried over anhydrous sodium sulfate, concentrated under reduced pressure, and separated by column chromatography to give a product (65 mg), m/z = 532 $[M+1]^+$, $^1$H NMR (400MHz, DMSO-*d*6) δ 12.64 (s, 1H), 8.63-8.64 (m, 1H), 8.49-8.49 (m, 2H), 7.95-7.97 (m, 1H), 7.54 (s, 1H), 7.35-7.41 (m, 1H), 7.16-7.23 (m, 3H), 6.58-6.60 (m, 1H), 7.23-7.24 (m, 1H), 6.73-6.75 (m, 1H), 4.14-4.19 (m, 2H), 3.70 (s, 4H), 2.92-2.96 (m, 1H), 2.08-2.13 (m, 2H), 1.35-1.45 (m, 3H).

**Example 60: Preparation of compound 173**

(1*R*,5*S*,6*R*)-3-(5-(6-ethoxy-1*H*-pyrazolo[3',4':3,4]pyrazolo[1,5-*a*]pyridin-4-yl)pyridin-2-yl)-*N*-(((6-methoxypyridin-3-yl)methylene)-3-azabicyclo[3.1.0]-6-hexylamine

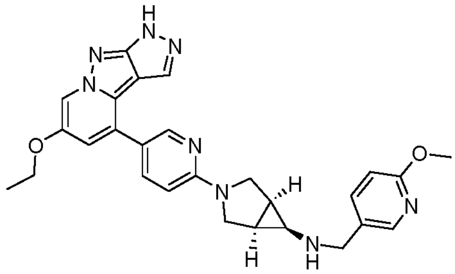

(1*R*,5*S*,6*R*)-3-(5-(6-ethoxy-1*H*-pyrazolo[3',4':3,4]pyrazolo[1,5-a]pyridin-4-yl)pyridin-2-yl)-3-azabic yclo[3.1.0]-6-hexylamine hydrochloride (200 mg, 0.41 mmol), n-propanol (4 mL), triethylamine (208 mg, 2.06 mmol), 6-methoxy-3-pyridinecarboxaldehyde (170 mg, 1.24 mmol), and 2-picoline borane (132.4 mg, 1.24 mol) were added to a reaction flask. The mixture was reacted at 40 °C for 20 h. The reaction was quenched by adding water and stirring with water, extracted with dichloromethane, dried over anhydrous sodium sulfate, concentrated under reduced pressure, and separated by column chromatography to give a product (4.8 mg), m/z = 497 $[M+1]^+$, $^1$H NMR (400MHz, DMSO-$d_6$) δ 12.63 (s, 1H), 8.48-8.53 (s, 2H), 7.97-8.01 (m, 2H), 7.51-7.58 (m, 2H), 7.21-7.21 (m, 1H), 6.55-6.63 (m, 2H), 4.14-4.20 (m, 2H), 3.75 (s, 3H), 3.65-3.72 (m, 4H), 3.33-3.35 (m, 3H), 2.22-2.25 (m, 1H), 1.83-1.84 (m, 2H), 1.38-1.41 (t, 3H).

**Example 61: Preparation of compound 174**

2-chloro-*N*-(((3a*R*,5*S*,6a*S*)-2-(5-(6-ethoxy-1*H*-pyrazolo[3',4':3,4]pyrazolo[1,5-*a*]pyridin-4-yl)pyridi n-2-yl)-octahydrocyclopenta[*c*]pyrrol-5-yl)-6-fluorobenzamide

Step A: 2-chloro-6-fluoro-*N*-(((3a*R*,5*S*,6a*S*)-octahydrocyclopenta[*c*]pyrrol-5-yl)benzamide hydrochloride

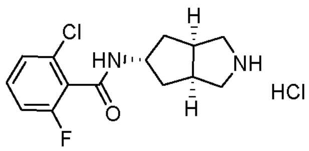

*Tert*-butyl (3a*R*,5*S*,6a*S*)-5-aminohexahydrocyclopenta[*c*]pyrrole-2(1*H*)-carbonate hydrochloride (120 mg, 0.53 mmol), dichloromethane (2 mL), triethylamine (107 mg, 1.06 mmol), and 6-fluoro-2-chlorobenzoyl chloride (113 mg, 0.58 mmol) were added to a reaction flask. The mixture was stirred at room temperature for 1 h. Water was poured into the reaction liquid, and the resulting mixture was extracted twice with DCM and concentrated until no fraction was produced. Methanol (1 mL) and 1,4-dioxane/HCl (4 M, 2 mL) were added to the residue, and the mixture was stirred at room temperature for 2 h. The reaction liquid was concentrated under reduced pressure to give a product (140 mg), which was directly used in the next step, m/z = 283 $[M+1]^+$.

Step B: 2-chloro-*N*-(((3a*R*,5*S*,6a*S*)-2-(5-(6-ethoxy-1*H*-pyrazolo[3',4':3,4]pyrazolo[1,5-*a*]pyridin-4-yl)pyridi n-2-yl)-octahydrocyclopenta[c]pyrrol-5-yl)-6-fluorobenzamide

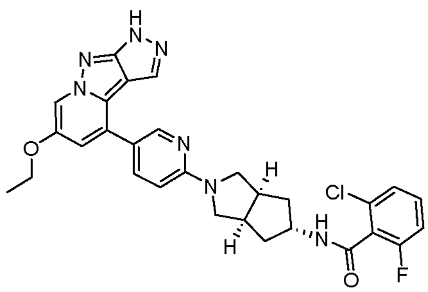

2-chloro-6-fluoro-*N*-(((3a*R*,5*S*,6a*S*)-octahydrocyclopenta[*c*]pyrrol-5-yl)benzamide hydrochloride (65 mg, 0.2 mmol), 2 mL of DMSO, DIEA (65.7 mg, 0.51 mmol), and 6-ethoxy-4-(6-fluoropyridin-3-yl)-1*H*-pyrazolo[3',4':3,4]pyrazolo[1,5-*a*]pyridine (65 mg, 0.2 mmol) were added to a reaction flask. The mixture was heated to 90 °C and reacted for 24 h. The reaction was quenched by adding water and stirring. The mixture was extracted with dichloromethane, dried over anhydrous sodium sulfate, concentrated under reduced pressure, and separated by column chromatography to give a product (30 mg), m/z = 560 $[M+1]^+$, $^1H$ NMR (400MHz, DMSO-*d*6) δ 12.64 (s, 1H), 8.80-8.82 (m, 1H), 8.50-8.55 (m, 2H), 7.80-8.02 (m, 1H), 7.57 (s, 1H), 7.43-7.48 (m, 1H), 7.35-7.37 (m, 1H), 7.26-7.31 (m, 1H), 7.21-7.22 (m, 1H), 6.72-6.74 (m, 1H), 4.29-4.35 (m, 1H), 4.14-4.19 (m, 2H), 3.59-3.63 (m, 2H), 3.49-3.52 (m, 2H), 2.78-2.80 (m, 2H), 2.32-2.38 (m, 2H), 1.37-1.50 (m, 5H).

**Example 62: Preparation of compound 176**

2-chloro-*N*-(((3a*R*,5*S*,6a*S*)-2-(5-(6-ethoxy-1*H*-pyrazolo[3',4':3,4]pyrazolo[1,5-*a*]pyridin-4-yl)pyridi n-2-yl)-octahydrocyclopenta[*c*]pyrrol-5-yl)-6-fluorobenzamide

Step A: 2-chloro-6-fluoro-*N*-((3a*R*,5*S*,6a*S*)-octahydrocyclopenta[*c*]pyrrol-5-yl)benzamide hydrochloride

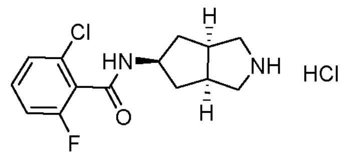

*Tert*-butyl (3a*R*,5*R*,6a*S*)-5-aminohexahydrocyclopenta[c]pyrrole-2(1*H*)-carbonate hydrochloride (120 mg, 0.53 mmol), dichloromethane (2 mL), triethylamine (107 mg, 1.06 mmol), and 6-fluoro-2-chlorobenzoyl chloride (113 mg, 0.58 mmol) were added to a reaction flask. The mixture was stirred at room temperature for 1 h. Water was poured into the reaction liquid, and the resulting mixture was extracted twice with DCM and concentrated until no fraction was produced. Methanol (1 mL) and 1,4-dioxane/HCl (4 M, 2 mL) were added to the residue, and the mixture was stirred at room temperature for 2 h. The reaction liquid was concentrated under reduced pressure to give a product (170 mg), which was directly used in the next step, m/z = 283 $[M+1]^+$.

Step B: 2-chloro-*N*-(((3a*R*,5*R*,6a*S*)-2-(5-(6-ethoxy-1*H*-pyrazolo[3',4':3,4]pyrazolo[1,5-*a*]pyridin-4-yl)pyridi n-2-yl)-octahydrocyclopenta[*c*]pyrrol-5-yl)-6-fluorobenzamide

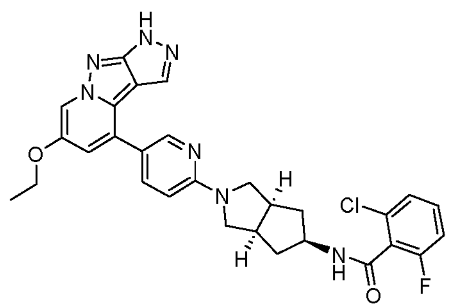

2-chloro-6-fluoro-*N*-(((3a*R*,5*R*,6a*S*)-octahydrocyclopenta[c]pyrrol-5-yl)benzamide hydrochloride (74 mg, 0.23 mmol), 2 mL of DMSO, DIEA (74.8 mg, 0.58 mmol), and 6-ethoxy-4-(6-fluoropyridin-3-yl)-1*H*-pyrazolo[3',4':3,4]pyrazolo[1,5-*a*]pyridine (57.4 mg, 0.19 mmol) were added to a reaction flask. The mixture was heated to 90 °C and reacted for 24 h. The reaction was quenched by adding water and stirring. The mixture was extracted with dichloromethane, dried over anhydrous sodium sulfate, concentrated under reduced pressure, and separated by column chromatography to give a product (17 mg), m/z = 560 $[M+1]^+$, $^1H$ NMR (400MHz, DMSO-*d*6) δ 12.64 (s, 1H), 8.78-8.80 (m, 1H), 8.50-8.59 (m, 2H), 8.00-8.03 (m, 1H), 7.60 (s, 1H), 7.45-7.50 (m, 1H), 7.37-7.38 (m, 1H), 7.28-7.32 (m, 1H), 7.22-7.23 (m, 1H), 6.72-6.75 (m, 1H), 4.37-4.45 (m, 1H), 4.14-4.25 (m, 2H), 3.63-3.67 (m, 2H), 3.40-3.43 (m, 2H), 2.91-2.95 (m, 2H), 1.86-2.01 (m, 4H), 1.38-1.41 (m, 3H).

**Example 63: Preparation of compound 180**

(2-chloro-6-fluorophenyl)((3a*R*,7a*S*)-2-(5-(6-ethoxy-1*H*-pyrazolo[3',4':3,4]pyrazolo[1,5-*a*]pyridin-4 -yl)pyridin-2-yl)octahydro-1*H*-4,7-epiminoisoindol-8-yl)methanone

Step A: (2-chloro-6-fluorophenyl)((3a*R*,7a*S*)-octahydro-1*H*-4,7-epiminoisoindol-8-yl)methanone hydrochloride

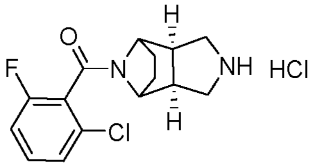

*Tert*-butyl (3a*R*,7a*S*)-octahydro-2*H*-4,7-epiminoisoindole-2-carbonate (100 mg, 0.35 mmol), dichloromethane (2 mL), triethylamine (70 mg, 0.69 mmol), and 6-fluoro-2-chlorobenzoyl chloride (80.3 mg, 0.42 mmol) were added to a reaction flask. The mixture was stirred at room temperature for 1 h. Water was poured into the reaction liquid, and the resulting mixture was extracted twice with DCM and concentrated until no fraction was produced. Methanol (1 mL) and 1,4-dioxane/HCl (4 M, 2 mL) were added, and the mixture was stirred at room temperature for 2 h. The reaction liquid was concentrated under reduced pressure to give a product (50 mg), which was directly used in the next step, m/z = 295 $[M+1]^+$.

Step B: (2-chloro-6-fluorophenyl)((3a*R*,7a*S*)-2-(5-(6-ethoxy-1*H*-pyrazolo[3',4':3,4]pyrazolo[1,5-*a*]pyridin-4 -yl)pyridin-2-yl)octahydro-1*H*-4,7-epiminoisoindol-8-yl)methanone

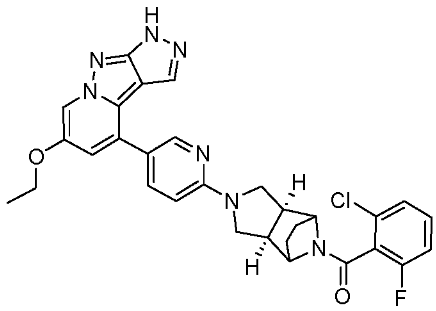

(2-chloro-6-fluorophenyl)((3a*R*,7a*S*)-octahydro-1*H*-4,7-epiminoisoindol-8-yl)methanone hydrochloride (50 mg, 0.15 mmol), 2 mL of DMSO, $K_2CO_3$ (69.5 mg, 0.5 mmol), and 6-ethoxy-4-(6-fluoropyridin-3-yl)-1*H*-pyrazolo[3',4':3,4]pyrazolo[1,5-*a*]pyridine (37.4 mg, 0.13 mmol) were added to a reaction flask. The mixture was heated to 90 °C and reacted for 24 h. The mixture was quenched by adding water. The reaction liquid was extracted with dichloromethane, dried over anhydrous sodium sulfate, concentrated under reduced pressure, and separated by column chromatography to give a product (5 mg), m/z = 572 $[M+1]^+$, $^1$H NMR (400 MHz, DMSO-d6) δ 12.64 (s, 1H), 8.51-8.57 (d, 2H), 8.04-8.07 (m, 1H), 7.37-7.58 (m, 5H), 7.25 (s, 1H), 6.83-6.85 (m, 1H), 4.73 (s, 1H), 4.15-4.20 (m, 2H), 3.85-3.88 (m, 1H), 3.75-3.78 (m, 2H), 3.03-3.18 (m, 5H), 3.90 (s, 1H), 1.59-1.62 (m, 4H), 1.34-1.41 (t, 3H), 2.78-2.80 (m, 2H), 2.32-2.38 (m, 2H), 1.37-1.50 (m, 5H).

**Example 64: Preparation of compound 128**

6-ethoxy-4-(6-(((3a*R*,7a*S*)-8-(((6-methoxypyridin-3-yl)methyl)octahydro-2*H*-4,7-epiaminoisoindol-2-yl)pyridin-3-yl)-1*H*-pyrazolo[3',4':3,4]pyrazolo[1,5-*a*]pyridine

Step A: (3a*R*,7a*S*)-8-(((6-methoxypyridin-3-yl)methyl)octahydro-1*H*-4,7-epiaminoisoindole hydrochloride

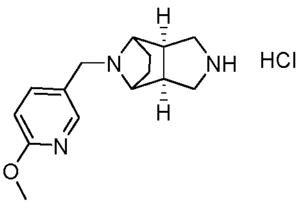

*Tert*-butyl (3aR,7aS)-octahydro-2*H*-4,7-epiaminoisoindole-2-carbonate (100 mg, 0.35 mmol), dichloromethane (2 mL), triethylamine (70 mg, 0.69 mmol), and 5-(chloromethyl)-2-methoxypyridine (80.3 mg, 0.42 mmol) were added to a reaction flask. The mixture was reacted at 50 °C for 16 h. Water was poured into the reaction liquid, and the resulting mixture was extracted twice with DCM and concentrated until no fraction was produced. Methanol (1 mL) and 1,4-dioxane/HCl (4 M, 2 mL) were added, and the mixture was stirred at room temperature for 2 h. The reaction liquid was concentrated under reduced pressure to give a product (30 mg), which was directly used in the next step, m/z = 260 $[M+1]^+$.

Step B: 6-ethoxy-4-(6-(((3a*R*,7a*S*)-8-(((6-methoxypyridin-3-yl)methyl)octahydro-2*H*-4,7-epiaminoisoindol-2-yl)pyridin-3-yl)-1*H*-pyrazolo[3',4':3,4]pyrazolo[1,5-*a*]pyridine

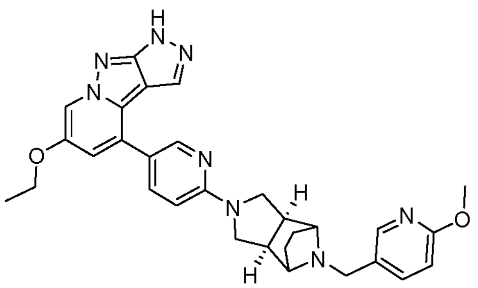

(3a*R*,7a*S*)-8-(((6-methoxypyridin-3-yl)methyl)octahydro-1*H*-4,7-epiaminoisoindole hydrochloride (30 mg, 0.10 mmol), 2 mL of DMSO, $K_2CO_3$ (69.5 mg, 0.5 mmol), and 6-ethoxy-4-(6-fluoropyridin-3-yl)-1*H*-pyrazolo[3',4':3,4]pyrazolo[1,5-*a*]pyridine (30 mg, 0.1 mmol) were added to a reaction flask. The mixture was heated to 90 °C and reacted for 24 h. The reaction was quenched by adding water. The reaction liquid was extracted with dichloromethane, dried over anhydrous sodium sulfate, concentrated under reduced pressure, and separated by column chromatography to give a product (4 mg), m/z = 537 $[M+1]^+$, $^1H$ NMR (400MHz, DMSO-*d*6) $\delta$ 12.65 (s, 1H), 8.51-8.58 (m, 2H), 8.04-8.14 (m, 2H), 7.76 (s, 1H), 7.59 (s, 1H), 7.26 (d, 1H), 6.83-6.85 (d, 2H), 4.15-4.20 (m, 2H), 3.80-3.85 (m, 5H), 3.09-3.18 (m, 2H), 2.89-2.93 (m, 2H), 1.40-1.46 (m, 4H), 1.21-1.29 (m, 5H), 1.18-1.98 (m, 1H), 1.09-1.10 (m, 1H).

**Example 65: Preparation of compound 183**

Step A: 3-phenyl 6-ethyl (1*R*,5*S*,6*R*)-3-azabicyclo[3.1.0]hexane-3,6-carboxylate

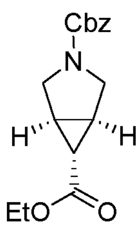

2,5-dihydro-1*H*-pyrrole-1-phenyl carbonate (5.0 g, 24.6 mmol), $Ph_2(OAc)_4$ (500 mg, 1.1 mmol), and 50 mL of DCE were added to a reaction flask. The mixture was heated to 80 °C under nitrogen atmosphere. 2-ethyl diazoacetate (14 g, 123 mmol) was dissolved in 50 mL of DCE, and the solution was added dropwise to the reaction liquid in 4 h. After the addition, the mixture was reacted at 80 °C for 16 h. The reaction was quenched by adding water and stirring.

The reaction liquid was extracted with dichloromethane, dried over anhydrous sodium sulfate, concentrated under reduced pressure, and separated by column chromatography to give a product (3.59 g), m/z = 290 $[M+1]^+$.

Step B: ethyl (1*R*,5*S*,6*r*)-3-azabicyclo[3.1.0]hexane-6-carbonate

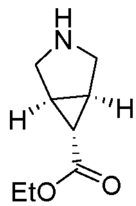

3-phenyl-6-ethyl (1*R*,5*S*,6*r*)-3-azabicyclo[3.1.0]hexane-3,6-carboxylate (3.5 g, 12 mmol), absolute methanol (30 mL), and palladium on carbon (600 mg) were added to a reaction flask. The mixture was purged with hydrogen twice and reacted at 25 °C for 18 h. After the reaction was completed, the reaction liquid was filtered through celite and concentrated under reduced pressure to give a crude product, ethyl (1*R*,5*S*,6*R*)-3-azabicyclo[3.1.0]hexane-6-carboxylate (2.0 g), which was directly used in the next step, m/z = 156 $[M+1]^+$.

Step C: ethyl (1*R*,5*S*,6*r*)-3-(5-bromopyridin-2-yl)-3-azabicyclo[3.1.0]hexane-6-carbonate

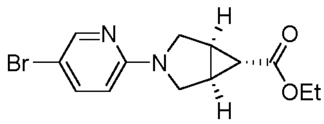

Ethyl 5-bromo-2-fluoropyridine (1.1 g, 6.4 mmol), (1*R*,5*S*,6*r*)-3-azabicyclo[3.1.0]hexane-6-carbonate (2.0 g), potassium carbonate (2.6 g, 19 mmol), and 10 mL of DMSO were added to a reaction flask. The mixture was reacted at 110 °C for 3 h. The reaction was quenched by adding water and stirring. The reaction liquid was extracted with dichloromethane, dried over anhydrous sodium sulfate, concentrated under reduced pressure, and separated by column chromatography to give a product (890 mg), m/z = 311 $[M+1]^+$.

Step D: ((1*R*,5*S*,6*r*)-3-(5-bromopyridin-2-yl)-3-azabicyclo[3.1.0]hexan-6-yl)methanol

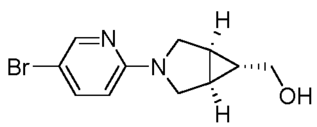

(1*R*,5*S*,6*r*)-3-(5-bromopyridin-2-yl)-3-azabicyclo[3.1.0]hexane-6-carbonate (750 mg, 2.4 mmol) and THF (20 mL) were added to a reaction flask, and $LiBH_4$ (209 mg, 9.6 mmol) was added under nitrogen atmosphere. The mixture was reacted at 50 °C for 5 h, and $LiBH_4$ (156 mg, 7.2 mmol) was added. The mixture was reacted at 50 °C for 16 h. The reaction was quenched by adding water and stirring. The reaction liquid was extracted with dichloromethane, dried over anhydrous sodium sulfate, concentrated under reduced pressure, and separated by column chromatography to give a product (340 mg), m/z = 269 $[M+1]^+$.

Step E: *tert*-butyl (((1*R*,5*S*,6*r*)-3-(5-bromopyridin-2-yl)-3-azabicyclo[3.1.0]hexan-6-yl)methyl)(*tert*-butoxycarbonyl)c arbamate

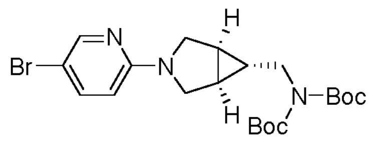

((1*R*,5*S*,6*r*)-3-(5-bromopyridin-2-yl)-3-azabicyclo[3.1.0]hexan-6-yl)methanol (340 mg, 1.2 mmol), $HN(Boc)_2$ (412 mg, 1.9 mmol), triphenylphosphine (660 mg, 2.5 mmol), and THF (15 mL) were added to a reaction flask. Diethyl azodicarboxylate (438 mg, 2.5 mmol) was added dropwise to the reaction liquid under reduced pressure. The mixture was reacted at 50 °C for 16 h. The reaction was quenched by adding water and stirring. The reaction liquid was extracted with dichloromethane, dried over anhydrous sodium sulfate, concentrated under reduced pressure, and separated by column chromatography to give a product (505 mg), m/z = 468 $[M+1]^+$.

Step F: *tert*-butyl (((1*R*,5*S*,6*R*)-3-(5-(6-ethoxy-1*H*-pyrazolo[3',4':3,4]pyrazolo[1,5-*a*]pyridin-4-yl)pyridin-2-yl)-3-azab icyclo[3.1.0]hexan-6-yl)methyl)(*tert*-butyloxycarbonyl)carbamate

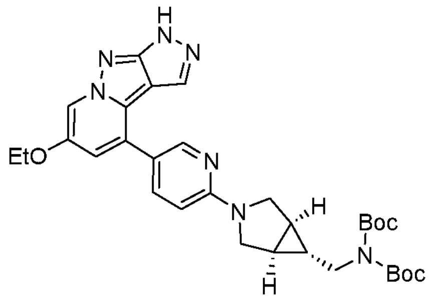

6-ethoxy-4-(4,4,5,5-tetramethyl-1,3,2-dioxaboran-2-yl)-1*H*-pyrazolo[3',4':3,4]pyrazolo[1,5-*a*]pyridi ne (360 mg, 1.1 mmol), (((1*R*,5*S*,6*s*)-3-(5-bromopyridin-2-yl)-3-azabicyclo[3.1.0]hexan-6-yl)methyl)(*tert*-butoxycarbonyl)c arbamate (500 mg, 1.1 mmol), cesium carbonate (1.0 g, 3.3 mmol), 1,4-dioxane (10 mL), and water (2 mL) were added to a reaction flask. The mixture was purged with nitrogen, and $Pd(dppf)Cl_2$ (73.1 mg, 0.1 mmol) was added. The mixture was reacted at 50 °C for 16 h. The reaction was quenched by adding water and stirring. The reaction liquid was extracted with dichloromethane, dried over anhydrous sodium sulfate, concentrated under reduced pressure, and separated by column chromatography to give a product (330 mg), m/z = 589 $[M+1]^+$.

Step G: ((1*R*,5*S*,6*s*)-3-(5-(6-ethoxy-1*H*-pyrazolo[3',4':3,4]pyrazolo[1,5-*a*]pyridin-4-yl)pyridin-2-yl)-3-azabi cyclo[3.1.0]hexan-6-yl)methanamine hydrochloride

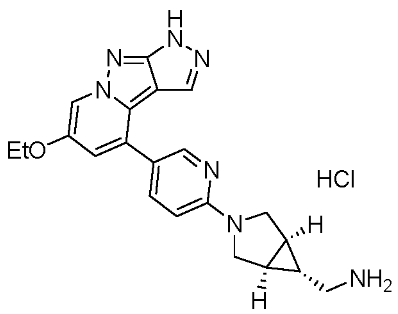

*Tert*-butyl

(((1*R*,5*S*,6*r*)-3-(5-(6-ethoxy-1*H*-pyrazolo[3',4':3,4]pyrazolo[1,5-*a*]pyridin-4-yl)pyridin-2-yl)-3-azabi cyclo[3.1.0]hexan-6-yl)methyl)(tert-butoxycarbonyl)carbamate (330 mg, 0.56 mmol), a 4 N hydrochloric acid-dioxane solution (5 mL, 20 mmol), and methanol (1 mL) were added to a reaction flask. The mixture was reacted at 25 °C for 16 h.

After the reaction was completed, the mixture was concentrated under reduced pressure to give a product (300 mg), which was directly used in the next step, m/z = 389 [M+1]+.

Step H: 2-chloro-*N*-(((1*R*,5*S*,6*s*)-3-(5-(6-ethoxy-1*H*-pyrazolo[3',4':3,4]pyrazolo[1,5-*a*]pyridin-4-yl)pyridin-2 -yl)-3-azabicyclo[3 .1.0]hexan-6-yl)methyl)-6-fluorobenzamide

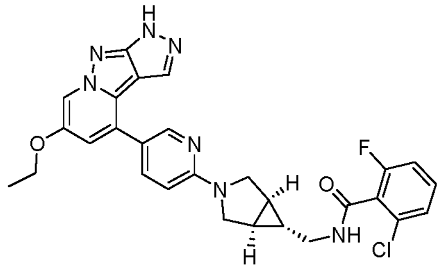

((1*R*,5*S*,6*s*)-3-(5-(6-ethoxy-1*H*-pyrazolo[3',4':3,4]pyrazolo[1,5-*a*]pyridin-4-yl)pyridin-2-yl)-3-azabi cyclo[3.1.0]hexan-6-yl)methanamine hydrochloride (120 mg, 0.3 mmol), 3-chloro-6-fluorobenzoyl chloride (59 mg, 0.3 mmol), DIEA (232 mg, 1.8 mmol), and 3 mL of DMF were added to a reaction flask. The mixture was reacted at 25 °C for 1 h. The reaction was quenched by adding water and stirring. The mixture was extracted with dichloromethane, dried over anhydrous sodium sulfate, concentrated under reduced pressure, and separated by column chromatography to give a product (11.5 mg), m/z = 546 [M+1]+, $^1$H NMR (400 MHz, DMSO-$d_6$) δ 12.63 (brs, 1H), 8.85-8.88 (m, 1H), 8.50-8.53 (m, 2H), 8.00 (d, *J*=8.8Hz, 1H), 7.44-7.59 (m, 2H), 7.29-7.42 (m, 2H), 7.21 (s, 1H), 6.68 (d, *J*=8.8Hz, 1H), 4.14 (q, *J*=6.8Hz, 1H), 3.74-3.76 (m, 2H), 3.47-3.50 (m, 2H), 3.25-3.28 (m, 2H), 1.75-1.79 (m, 2H), 1.39 (t, *J*=6.8Hz, 3H), 0.88-0.94 (m, 1H).

**Example 66: Preparation of compound 184**

Step A: *N*-(((1*R*,5*S*,6*s*)-3-(5-(6-ethoxy-1*H*-pyrazolo[3',4':3,4]pyrazolo[1,5-*a*]pyridin-4-yl)pyridin-2-yl)-3-az abicyclo[3.1.0]hexan-6-yl)methyl)-1-(6-methoxypyridin-3-yl)-N ((6-methoxypyridin-3-yl)methyl) methanamine

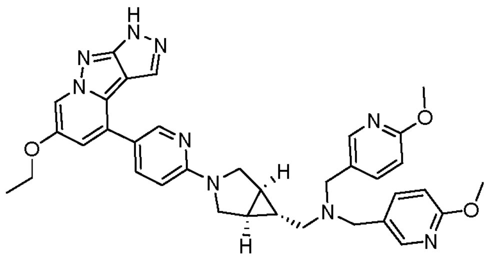

((1*R*,5*S*,6*s*)-3-(5-(6-ethoxy-1*H*-pyrazolo[3',4':3,4]pyrazolo[1,5-*a*]pyridin-4-yl)pyridin-2-yl)-3-azabi cyclo[3.1.0]hexan-6-yl)methanamine hydrochloride (170 mg, 0.44 mmol), 6-methoxy-3-pyridinecarboxaldehyde (178 mg, 1.3 mmol), TEA (212 mg, 2.1 mmol), 2-picoline borane (139 mg, 1.3 mmol), and *n*-propanol (10 mL) were added to a reaction flask. The mixture was reacted at 45 °C for 18 h. The reaction was quenched by adding water and stirring. The reaction liquid was extracted with dichloromethane, dried over anhydrous sodium sulfate, concentrated under reduced pressure, and separated by column chromatography to give a product (6.4 mg), m/z = 511 [M+1]+, $^1$H NMR (400 MHz, DMSO-$d_6$) δ 12.66 (brs, 1H), 8.50-8.55 (m, 2H), 8.10 (s, 1H), 8.00 (d, *J*=8.4Hz, 1H), 7.67-7.71 (m, 2H), 7.57 (s, 1H), 7.21 (s, 1H), 6.61 (d, *J*=8.8Hz, 1H), 4.14-4.18 (m, 2H), 3.74-3.76 (m, 2H), 3.52-3.61 (m, 3H), 3.37-3.42 (m, 2H), 2.32-2.45 (m, 2H), 1.41-1.48 (m, 2H), 1.39 (t, *J*=6.8Hz, 3H), 0.80-0.88 (m, 1H).

**Example 67: Preparation of compound 187**

Step A: *tert*-butyl 5-(5-(6-ethoxy-1*H*-pyrazolo[3',4':3,4]pyrazolo[1,5-*a*]pyridin-4-yl)pyridin-2-yl)-2,5-diazaspiro[3.4]o ctane-5-carboxylate

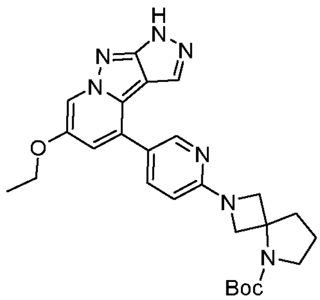

6-ethoxy-4-(6-fluoropyridin-3-yl)-1*H*-pyrazolo[3',4':3,4]pyrazolo[1,5-*a*]pyridine (318 mg, 1.1 mmol), *tert*-butyl 2,6-diazaspiro[3.4]octane-6-carboxylate (254 mg, 1.2 mmol), DIEA (414 mg, 3.2 mmol), and DMSO (5mL) were added to a reaction flask. The mixture was reacted at 90 °C for 12 h. The reaction was quenched by adding water and stirring. The reaction liquid was extracted with dichloromethane, dried over anhydrous sodium sulfate, concentrated under reduced pressure, and separated by column chromatography to give a product (511 mg), m/z = 490 $[M+1]^+$.

Step B: 4-(6-(2,5-diazaspiro[3.4]octan-2-yl)pyridin-3-yl)-6-methoxy-1*H*-pyrazolo[3',4':3,4]pyrazolo[1,5-*a*]p yridine

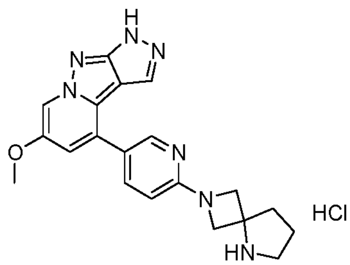

*Tert*-butyl

5-(5-(6-ethoxy-1*H*-pyrazolo[3',4':3,4]pyrazolo[1,5-*a*]pyridin-4-yl)pyridin-2-yl)-2,6-diazaspiro[3.4]o ctane-6-carboxylate (511 mg, 1.04 mmol) was added to 5 mL of MeOH/HCl (4 M). The mixture was reacted at 25 °C for 4 h. The reaction liquid was concentrated under reduced pressure to give a product (500 mg), which was directly used in the next step, m/z = 390 $[M+1]^+$.

Step C: (2-chloro-6-fluorophenyl)-5-(5-(6-ethoxy-1*H*-pyrazolo[3',4':3,4]pyrazolo[1,5-*a*]pyridin-4-yl)pyridin -2-yl)-2,5-diazaspiro[3.4]octan-5-yl)methanone

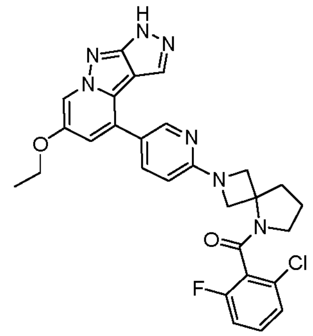

4-(6-(2,6-diazaspiro[3.4]octan-2-yl)pyridin-3-yl)-6-ethoxy-1*H*-pyrazolo[3',4':3,4]pyrazolo[1,5-*a*]py ridine hydrochloride (500 mg, 1.18 mmol), 2-chloro-6-fluorobenzoyl chloride (250 mg, 1.29 mmol), $Et_3N$ (358 mg, 3.54 mmol), and DMF (5 mL) were added to a reaction flask. The mixture was reacted at 25 °C for 12 h. The reaction was quenched by adding water, extracted with dichloromethane, dried over anhydrous sodium sulfate, concentrated under reduced pressure, and separated by column chromatography to give a product (398 mg), m/z = 546 $[M+1]^+$, $^1H$ NMR (400 MHz, DMSO-$d_6$): δ 12.65 (s, 1H), 8.57 (d, *J* = 2.4 Hz, 1H), 8.52 (d, *J* = 2.1 Hz, 1H), 8.06 (m,1H), 7.59 (s, 1H), 7.53 (m, 1H), 7.49-7.34 (m, 2H), 7.27 (d, *J* = 2.1 Hz, 1H), 6.69 (d, *J* = 8.6 Hz, 1H), 4.86 (t, *J* = 8.4 Hz, 2H), 4.18 (q, *J* = 6.9 Hz, 2H), 4.05 (dd, *J* = 9.6, 7.8 Hz, 2H), 3.23 (td, *J* = 6.6, 1.9 Hz, 2H), 2.42 (t, *J*= 6.8 Hz, 2H), 1.91-1.78 (m, 2H), 1.40 (t, *J*= 6.9 Hz, 3H).

**Example 68: Preparation of compound 188**

6-ethoxy-4-(6-(5-((6-methoxypyridin-3-yl)methyl)-2,5-diazaspiro[3.4]octan-2-yl)pyridin-3-yl)-1*H*-pyrazolo[3',4':3,4]pyrazolo[1,5-*a*]pyridine

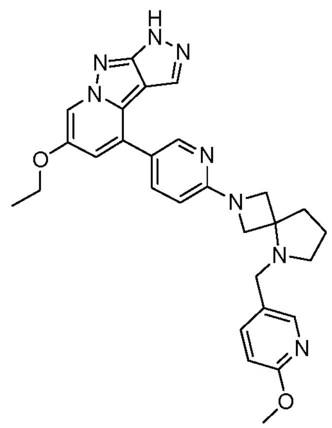

4-(6-(2,5-diazaspiro[3.4]octan-2-yl)pyridin-3-yl)-6-ethoxy-1*H*-pyrazolo[3',4':3,4]pyrazolo[1,5-*a*]py ridine hydrochloride (500 mg, 1.18 mmol), 6-methoxy-3-pyridinecarboxaldehyde (176 mg, 1.29 mmol), $Et_3N$ (357.5 mg, 3.54 mmol), Pic-$BH_3$ (378.8 mg, 3.54 mmol), and *n*-propanol (5 mL) were added to a reaction flask. The mixture was reacted at 25 °C for 12 h. The reaction was quenched by adding water and stirring. The reaction liquid was extracted with dichloromethane, dried over anhydrous sodium sulfate, concentrated under reduced pressure, and separated by column chromatography to give a product (346 mg), m/z= 511 $[M+1]^+$, $^1H$ NMR (400 MHz, DMSO-$d_6$) 12.65 (s, 1H), 8.54 (dd, *J* = 16.4, 2.2 Hz, 2H), 8.13-8.00 (m, 2H), 7.69 (dd, *J* = 8.5, 2.4 Hz, 1H), 7.59 (s, 1H), 7.24 (d, *J*= 2.1 Hz, 1H), 6.79 (d, *J*= 8.4 Hz, 1H), 6.66 (d, *J*= 8.6 Hz, 1H), 4.29 (d, *J*= 8.9 Hz, 2H), 4.18 (m, 2H), 3.95 (d, *J* = 8.9 Hz, 2H), 3.84 (s, 3H), 3.79 (s, 2H), 2.56 (t, *J* = 7.1 Hz, 2H), 2.14 (dd, *J* = 9.1, 6.4 Hz, 2H), 1.71 (t, *J* = 7.7 Hz, 2H), 1.40 (t, *J* = 6.9 Hz, 3H).

**Example 69: Preparation of compound 189**

Step A: tert-butyl 5-(5-(6-ethoxy-1*H*-pyrazolo[3',4':3,4]pyrazolo[1,5-*a*]pyridin-4-yl)pyridin-2-yl)-2,6-diazaspiro[3.4]o ctane-6-carboxylate

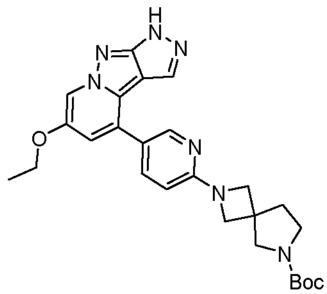

6-ethoxy-4-(6-fluoropyridin-3-yl)-1*H*-pyrazolo[3',4':3,4]pyrazolo[1,5-*a*]pyridine (318 mg, 1.1 mmol), tert-butyl 2,6-diazaspiro[3.4]octane-6-carboxylate (254.4 mg, 1.2 mmol), DIEA (414 mg, 3.2 mmol), and DMSO (5 mL) were added to a reaction flask. The mixture was reacted at 90 °C for 12 h. The reaction was quenched by adding water and stirring. The reaction liquid was extracted with dichloromethane, dried over anhydrous sodium sulfate, concentrated under reduced pressure, and separated by column chromatography to give a product (511 mg), m/z = 490 $[M+1]^+$.

Step B: 4-(6-(2,6-diazaspiro[3.4]octan-2-yl)pyridin-3-yl)-6-ethoxy-1*H*-pyrazolo[3',4':3,4]pyrazolo[1,5-*a*]py ridine hydrochloride

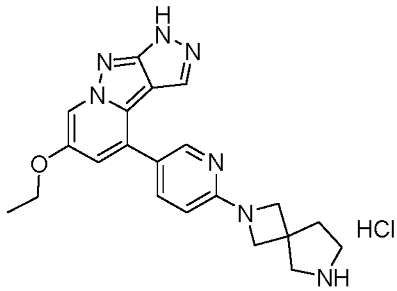

*Tert*-butyl

5-(5-(6-ethoxy-1*H*-pyrazolo[3',4':3,4]pyrazolo[1,5-*a*]pyridin-4-yl)pyridin-2-yl)-2,6-diazaspiro[3.4]o ctane-6-carboxylate (511 mg, 1.04 mmol) was added to 5 mL of MeOH/HCl (4 M). The mixture was reacted at 25 °C for 4 h. The reaction liquid was concentrated under reduced pressure to give a product (500 mg), which was directly used in the next step, m/z = 390 $[M+1]^+$.

Step C: (2-chloro-6-fluorophenyl)-5-(5-(6-ethoxy-1*H*-pyrazolo[3',4':3,4]pyrazolo[1,5-*a*]pyridin-4-yl)pyridin -2-yl)-2,6-diazaspiro[3.4]octan-6-yl) methanone

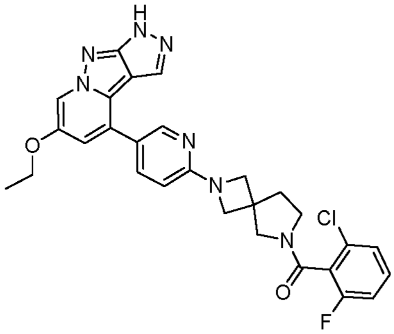

4-(6-(2,6-diazaspiro[3.4]octan-2-yl)pyridin-3-yl)-6-ethoxy-1*H*-pyrazolo[3',4':3,4]pyrazolo[1,5-*a*]py ridine hydrochloride (500 mg, 1.18 mmol), 2-chloro-6-fluorobenzoyl chloride (250 mg, 1.29 mmol), $Et_3N$ (357.5 mg, 3.54 mmol), and DMF (5 mL) were added to a reaction flask. The mixture was reacted at 25 °C for 12 h. The reaction was quenched by adding water and stirring. The reaction liquid was extracted with dichloromethane, dried over anhydrous sodium sulfate, concentrated under reduced pressure, and separated by column chromatography to give a product (398 mg), m/z = 546 $[M+1]^+$, $^1H$ NMR (400 MHz, DMSO-$d_6$) δ 12.64 (s, 1H), 8.55 (dd, *J* = 12.5, 2.5 Hz, 1H), 8.52 (m, 1H), 8.03 (m, 1H), 7.61-7.33 (m, 4H), 7.28-7.20 (m, 1H), 6.64 (dd, *J*= 21.1, 8.7 Hz, 1H), 4.18 (m, 2H), 4.13-3.91 (m, 4H), 3.89-3.73 (m, 1H), 3.71-3.54 (m, 1H), 3.43 (s, 1H), 3.25 (t, *J*= 6.9 Hz, 1H), 2.25 (m, 2H), 1.40 (m, 3H).

**Example 70: Preparation of compound 190**

6-ethoxy-4-(6-(6-((6-methoxypyridin-3-yl)methyl)-2,6-diazaspiro[3.4]octan-2-yl)pyridin-3-yl)-1*H*-pyrazolo[3',4':3,4]pyrazolo[1,5-*a*]pyridine

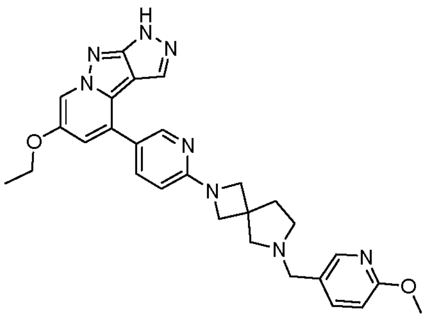

4-(6-(2,6-diazaspiro[3.4]octan-2-yl)pyridin-3-yl)-6-ethoxy-1*H*-pyrazolo[3',4':3,4]pyrazolo[1,5-*a*]py ridine hydrochloride (500 mg, 1.18 mmol), 6-methoxy-3-pyridinecarboxaldehyde (176 mg, 1.29 mmol), $Et_3N$ (357.5 mg, 3.54 mmol), Pic-$BH_3$ (378.8 mg, 3.54 mmol), and *n*-propanol (5 mL) were added to a reaction flask. The mixture was reacted at 25 °C for 12 h. The reaction was quenched by adding water and stirring. The reaction liquid was extracted with dichloromethane, dried over anhydrous sodium sulfate, concentrated under reduced pressure, and separated by column chromatography to give a product (346 mg), m/z = 511 $[M+1]^+$, $^1H$ NMR (400 MHz, DMSO-$d_6$) δ 12.65 (s, 1H), 8.52 (dd, *J* = 8.4, 2.3 Hz, 2H), 8.11 (s, 1H), 8.01 (dd, *J* = 8.7, 2.5 Hz, 1H), 7.69 (s, 1H), 7.56 (s, 1H), 7.22 (d, *J* = 2.1 Hz, 1H), 6.81 (d, *J* = 8.4 Hz, 1H), 6.59 (d, *J* = 8.6 Hz, 1H), 4.17 (m, 2H), 3.97 (t, *J* = 6.1 Hz, 4H), 3.84 (s, 3H), 3.06 (d, *J* = 7.1 Hz, 1H), 2.77 (s, 2H), 2.58 (s, 2H), 2.12 (s, 2H), 1.39 (t, *J* = 6.9 Hz, 3H), 1.19 (t, *J*= 7.3 Hz, 1H).

**Example 71: Preparation of compound 191**

Step A: *tert*-butyl 2-(5-(6-ethoxy-1*H*-pyrazolo[3',4':3,4]pyrazolo[1,5-*a*]pyridin-4-yl)pyridin-2-yl)-2,7-diazaspiro[3.5]n onane-7-carboxylate

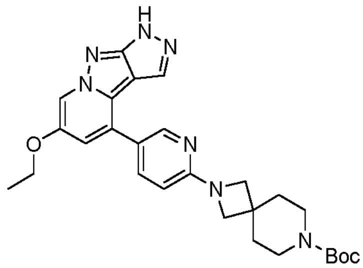

6-ethoxy-4-(6-fluoropyridin-3-yl)-1*H*-pyrazolo[3',4':3,4]pyrazolo[1,5-*a*]pyridine (250 mg, 0.8 mmol), *tert*-butyl 2,7-diazaspiro[3.5]nonane-7-carboxylate (230 mg, 0.9 mmol), DIEA (520 mg, 4 mmol), and DMSO (2 mL) were added to a reaction flask. The mixture was reacted at 90 °C for 16 h. The reaction was quenched by adding water and stirring. The reaction liquid was extracted with dichloromethane, dried over anhydrous sodium sulfate, concentrated under reduced pressure, and separated by column chromatography to give a product (390 mg), m/z = 504 $[M+1]^+$.

Step B: 4-(6-(2,7-diazaspiro[3.5]nonan-2-yl)pyridin-3-yl)-6-ethoxy-1*H*-pyrazolo[3',4':3,4]pyrazolo[1,5-*a*]py ridine hydrochloride

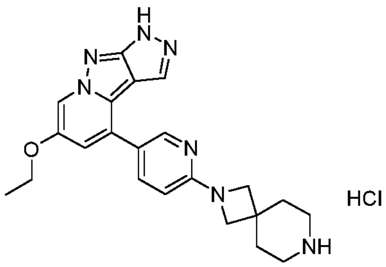

*Tert*-butyl

2-(5-(6-ethoxy-1*H*-pyrazolo[3',4':3,4]pyrazolo[1,5-*a*]pyridin-4-yl)pyridin-2-yl)-2,7-diazaspiro[3.5]n onane-7-carboxylate (390 mg, 0.7 mmol) was added to a solution of HCl in methanol (4 M, 5 ml). The mixture was reacted at 25 °C for 4 h. The reaction liquid was concentrated under reduced pressure to give a product (370 mg), which was directly used in the next step, m/z = 404 $[M+1]^+$.

Step C: *N*-(2-(5-(6-ethoxy-1*H*-pyrazolo[3',4':3,4]pyrazolo[1,5-*a*]pyridin-4-yl)pyridin-2-yl)-2,7-diazaspiro[3. 5]nonan-7-yl)-2-chloro-6-fluorobenzamide

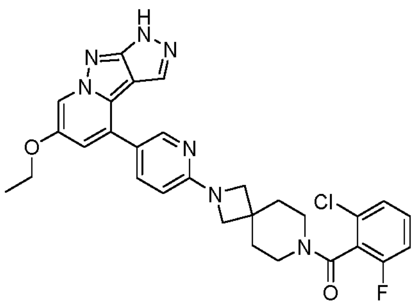

4-(6-(2,7-diazaspiro[3.5]nonan-2-yl)pyridin-3-yl)-6-ethoxy-1*H*-pyrazolo[3',4':3,4]pyrazolo[1,5-*a*]py ridine (100 mg, 0.2 mmol), 2-chloro-6-fluorobenzoyl chloride (46 mg, 0.2 mmol), $Et_3N$ (100 mg, 1.0 mmol), and DMF (2 mL) were added to a reaction flask. The mixture was reacted at 25 °C for 16 h. The reaction was quenched by adding water and stirring. The reaction liquid was extracted with dichloromethane, dried over anhydrous sodium sulfate, concentrated

under reduced pressure, and separated by column chromatography to give a product (47 mg), m/z = 560 [M+1]$^+$. $^1$H NMR (400 MHz, DMSO-$d_6$) δ 12.65 (s, 1H), 8.53 (dd, *J* = 10.4, 2.3 Hz, 2H), 8.03 (dd, *J* = 8.6, 2.5 Hz, 1H), 7.48-7.59 (m, 2H), 7.33-7.49 (m, 2H), 7.23 (d, *J*= 2.1 Hz, 1H), 6.60 (d, *J* = 8.7 Hz, 1H), 4.18 (q, *J* = 7.0 Hz, 2H), 3.80-3.91 (m, 4H), 3.73 (tq, *J* = 13.6, 7.4, 6.9 Hz, 2H), 3.15-3.26 (m, 2H), 1.71-1.91 (m, 4H), 1.40 (t, *J*= 6.9 Hz, 3H).

**Example 72: Preparation of compound 192**

6-ethoxy-4-(6-(7-((6-methoxypyridin-3-yl)methylene)-2,7-diazaspiro[3.5]nonan-2-yl)pyridin-3-yl)-1*H*-pyrazolo[3',4':3,4]pyrazolo[1,5-*a*]pyridine

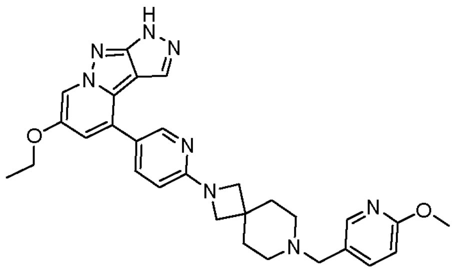

4-(6-(2,7-diazaspiro[3.5]nonan-2-yl)pyridin-3-yl)-6-ethoxy-1*H*-pyrazolo[3',4':3,4]pyrazolo[1,5-*a*]py ridine (100 mg, 0.2 mmol), 6-methoxynicotinaldehyde (83 mg, 0.6 mmol), Pic-$BH_3$ (64 mg, 0.6 mmol), $Et_3N$ (120 mg, 1.2 mmol), and n-propanol (5 mL) were added to a reaction flask. The mixture was reacted at 45 °C for 16 h. The reaction was quenched by adding water and stirring. The reaction liquid was extracted with dichloromethane, dried over anhydrous sodium sulfate, concentrated under reduced pressure, and separated by column chromatography to give a product (37 mg), m/z = 525 [M+1]$^+$, $^1$H NMR (400 MHz, DMSO-$d_6$) δ 12.65 (s, 1H), 8.52 (dd, *J* = 7.4, 2.3 Hz, 2H), 7.97-8.09 (m, 2H), 7.65 (dd, *J* = 8.4, 2.4 Hz, 1H), 7.56 (s, 1H), 7.23 (d, *J* = 2.1 Hz, 1H), 6.80 (d, *J* = 8.4 Hz, 1H), 6.59 (d, *J* = 8.7 Hz, 1H), 4.17 (q, *J* = 6.9 Hz, 2H), 3.85 (s, 3H), 3.76 (s, 4H), 3.42 (s, 2H), 2.35 (s, 4H), 1.78 (s, 3H), 1.78 (d, *J*= 11.2 Hz, 1H), 1.40 (t, *J*= 6.9 Hz, 3H).

**Example 73: Preparation of compound 193**

Step A: *tert*-butyl 2-(5-(6-ethoxy-1*H*-pyrazolo[3',4':3,4]pyrazolo[1,5-*a*]pyridin-4-yl)pyridin-2-yl)-2,6-diazaspiro[3.5]n onane-6-carboxylate

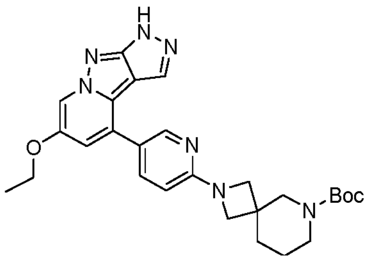

6-ethoxy-4-(6-fluoropyridin-3-yl)-1*H*-pyrazolo[3',4':3,4]pyrazolo[1,5-*a*]pyridine (250 mg, 0.8 mmol), tert-butyl 2,6-diazaspiro[3.5]nonane-6-carboxylate (230 mg, 0.9 mmol), DIEA (520 mg, 4 mmol), and DMSO (2 mL) were added to a reaction flask. The mixture was reacted at 90 °C for 16 h. The reaction was quenched by adding water and stirring. The reaction liquid was extracted with dichloromethane, dried over anhydrous sodium sulfate, concentrated under reduced pressure, and separated by column chromatography to give a product (370 mg), m/z = 504 [M+1]$^+$.

Step B: 4-(6-(2,6-diazaspiro[3.5]nonan-2-yl)pyridin-3-yl)-6-ethoxy-1*H*-pyrazolo[3',4':3,4]pyrazolo[1,5-*a*]py ridine hydrochloride

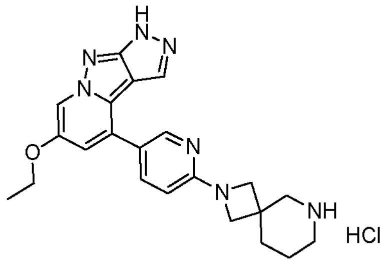

Tert-butyl

2-(5-(6-ethoxy-1*H*-pyrazolo[3',4':3,4]pyrazolo[1,5-*a*]pyridin-4-yl)pyridin-2-yl)-2,6-diazaspiro[3.5]n onane-6-carboxylate (370 mg, 0.7 mmol) was added to a solution of HCl in methanol (4 M, 5 mL). The mixture was reacted at 25 °C for 4 h. The reaction liquid was concentrated under reduced pressure to give a product (370 mg), which was directly used in the next step, m/z = 404 $[M+1]^+$.

Step C: N-(2-(5-(6-ethoxy-1*H*-pyrazolo[3',4':3,4]pyrazolo[1,5-*a*]pyridin-4-yl)pyridin-2-yl)-2,6-diazaspiro[3. 5]nonan-6-yl)-2-chloro-6-fluorobenzamide

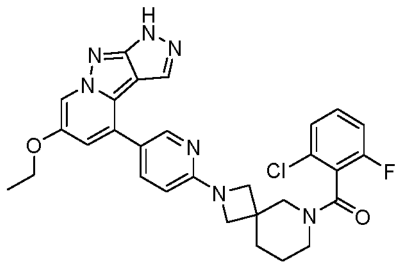

4-(6-(2,6-diazaspiro[3.5]nonan-2-yl)pyridin-3-yl)-6-ethoxy-1*H*-pyrazolo[3',4':3,4]pyrazolo[1,5-*a*]py ridine (100 mg, 0.2 mmol), 2-chloro-6-fluorobenzoyl chloride (46 mg, 0.2 mmol), $Et_3N$ (100 mg, 1.0 mmol), and DMF (2 mL) were added to a reaction flask. The mixture was reacted at 25 °C for 16 h. The reaction was quenched by adding water and stirring. The mixture was extracted with dichloromethane, dried over anhydrous sodium sulfate, concentrated under reduced pressure, and separated by column chromatography to give a product (36 mg), m/z = 560 $[M+1]^+$. $^1H$ NMR (400 MHz, DMSO-$d_6$) δ 12.65 (s, 1H), 8.59-8.48 (m, 2H), 8.03 (dd, *J* = 21.8, 8.6, 2.5 Hz, 1H), 7.41-7.62 (m, 3H), 7.34-7.43 (m, 1H), 7.23 (dd, *J* = 17.9, 2.1 Hz, 1H), 6.68 (d, *J* = 8.7 Hz, 1H), 4.12-4.23 (m, 2H), 4.08 (d, *J* = 12.8 Hz, 1H), 3.90 (dd, *J*= 11.8, 8.2 Hz, 1H), 3.79 (dq, *J* = 8.0, 4.1, 2.8 Hz, 3H), 3.70 (s, 1H), 3.44 (s, 1H), 3.13-3.22 (m, 1H), 1.84-1.98 (m, 2H), 1.59 (d, *J*= 28.4 Hz, 4H), 1.40 (td, *J* = 7.0, 3.5 Hz, 3H).

**Example 74: Preparation of compound 194**

6-ethoxy-4-(6-(6-((6-methoxypyridin-3-yl)methylene)-2,6-diazaspiro[3.5]nonan-2-yl)pyridin-3-yl)-1*H*-pyrazolo[3',4':3,4]pyrazolo[1,5-*a*]pyridine

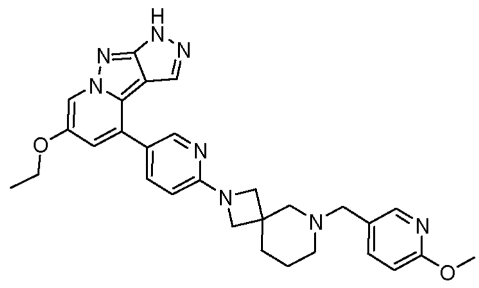

4-(6-(2,6-diazaspiro[3.5]nonan-2-yl)pyridin-3-yl)-6-ethoxy-1*H*-pyrazolo[3',4':3,4]pyrazolo[1,5-*a*]py ridine (100 mg, 0.2 mmol), 6-methoxy-3-pyridinecarboxaldehyde (83 mg, 0.6 mmol), Pic-$BH_3$ (64 mg, 0.6 mmol), $Et_3N$ (120 mg, 1.2 mmol), and n-propanol (5 mL) were added to a reaction flask. The mixture was reacted at 45 °C for 16 h. The reaction was quenched by adding water and stirring. The reaction liquid was extracted with dichloromethane, dried over anhydrous sodium sulfate, concentrated under reduced pressure, and separated by column chromatography to give a product (32 mg), m/z = 525 $[M+1]^+$, $^1H$ NMR (400 MHz, DMSO-$d_6$) δ 12.65 (s, 1H), 8.51 (t, *J* = 1.9 Hz, 2H), 8.08 (d, *J*= 2.3 Hz, 1H), 7.99 (dd, *J*= 8.7, 2.5 Hz, 1H), 7.67 (dd, *J*= 8.5, 2.4 Hz, 1H), 7.56 (s, 1H), 7.22 (d, *J* = 2.1 Hz, 1H), 6.81 (d, *J* = 8.4 Hz, 1H), 6.57 (d, *J* = 8.7 Hz, 1H), 4.17 (q, *J* = 6.9 Hz, 2H), 3.83 (s, 3H), 3.70 (s, 4H), 3.47 (s, 2H), 2.28-2.34 (m, 4H), 1.52-1.69 (m, 4H), 1.40 (t, *J*= 6.9 Hz, 3H).

**Example 75: Preparation of compound 195**

Step A: *tert*-butyl 2-(5-(6-ethoxy-1*H*-pyrazolo[3',4':3,4]pyrazolo[1,5-*a*]pyridin-4-yl)pyridin-2-yl)-2,8-diazaspiro[4.5]d ecane-8-carboxylate

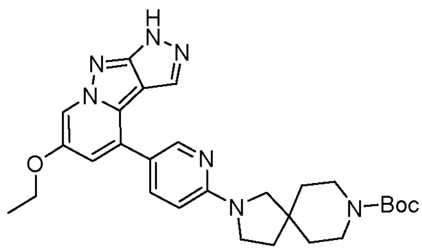

6-ethoxy-4-(6-fluoropyridin-3-yl)-1*H*-pyrazolo[3',4':3,4]pyrazolo[1,5-*a*]pyridine (250 mg, 0.8 mmol), *tert*-butyl 2,8-diazaspiro[4.5]decane-8-carboxylate (240 mg, 1.0 mmol), DIEA (520 mg, 4 mmol), and DMSO (2 mL) were added to a reaction flask. The mixture was reacted at 90 °C for 16 h. The reaction was quenched by adding water and stirring. The reaction liquid was extracted with dichloromethane, dried over anhydrous sodium sulfate, concentrated under reduced pressure, and separated by column chromatography to give a product (360 mg), m/z = 518 $[M+1]^+$.

Step B: 4-(6-(2,8-diazaspiro[4.5]decan-2-yl)pyridin-3-yl)-6-ethoxy-1*H*-pyrazolo[3',4':3,4]pyrazolo[1,5-*a*]py ridine

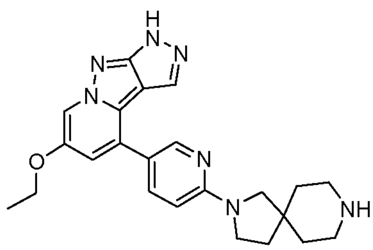

*Tert*-butyl

2-(5-(6-ethoxy-1*H*-pyrazolo[3',4':3,4]pyrazolo[1,5-*a*]pyridin-4-yl)pyridin-2-yl)-2,8-diazaspiro[4.5]d ecane-8-carboxylate (360 mg, 0.7 mmol) was added to a solution of HCl in methanol (4 M, 5 mL). The mixture was reacted at 25 °C for 4 h. The reaction was quenched by adding water and stirring. The reaction liquid was extracted with dichloromethane, dried over anhydrous sodium sulfate, concentrated under reduced pressure, and separated by column chromatography to give a product (370 mg), m/z = 418 $[M+1]^+$.

Step C: *N*-(2-(5-(6-ethoxy-1*H*-pyrazolo[3',4':3,4]pyrazolo[1,5-*a*]pyridin-4-yl)pyridin-2-yl)-2,8-diazaspiro[4. 5]decan-8-yl)2-chloro-6-fluorobenzamide

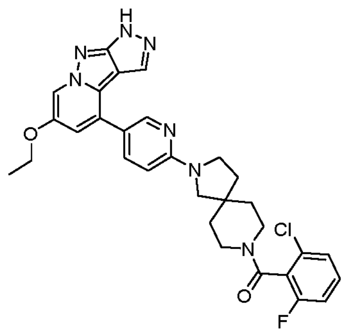

4-(6-(2,8-diazaspiro[4.5]decan-2-yl)pyridin-3-yl)-6-ethoxy-1*H*-pyrazolo[3',4':3,4]pyrazolo[1,5-*a*]py ridine (100 mg, 0.2 mmol), 2-chloro-6-fluorobenzoyl chloride (46 mg, 0.2 mmol), $Et_3N$ (100 mg, 1.0 mmol), and DMF (2 mL) were added to a reaction flask. The mixture was reacted at 25 °C for 16 h. The reaction was quenched by adding water and stirring. The reaction liquid was extracted with dichloromethane, dried over anhydrous sodium sulfate, concentrated under reduced pressure, and separated by column chromatography to give a product (55 mg), m/z = 574 $[M+1]^+$, $^1H$ NMR (400 MHz, DMSO-$d_6$) δ 12.64 (s, 1H), 8.53 (dd, *J* = 19.9, 2.3 Hz, 2H), 8.01 (dd, *J* = 8.8, 2.5 Hz, 1H), 7.57 (s, 1H), 7.52 (td, *J* = 8.2, 6.2 Hz, 1H), 7.45 (d, *J* = 8.0 Hz, 1H), 7.37 (t, *J* = 8.5 Hz, 1H), 7.22 (d, *J*= 2.1 Hz, 1H), 6.70 (d, *J*= 8.8 Hz, 1H), 4.18 (q, *J*= 7.0 Hz, 2H), 3.78-3.92 (m, 1H), 3.69 (dtd, *J* = 13.4, 8.7, 8.1, 3.8 Hz, 1H), 3.57 (q, *J* = 6.9 Hz, 2H), 3.40-3.55 (m, 2H), 3.27 (q, *J* = 4.9 Hz, 2H), 1.97 (dddd, *J* = 15.5, 12.3, 9.2, 5.0 Hz, 2H), 1.46-1.74 (m, 4H), 1.40 (t, *J* = 6.9 Hz, 3H).

**Example 76: Preparation of compound 196**

6-ethoxy-4-(6-(8-((6-methoxypyridin-3-yl)methylene)-2,8-diazaspiro[4.5]decan-2-yl)pyridin-3-yl)-1*H*-pyrazolo[3',4':3,4]pyrazolo[1,5-*a*]pyridine

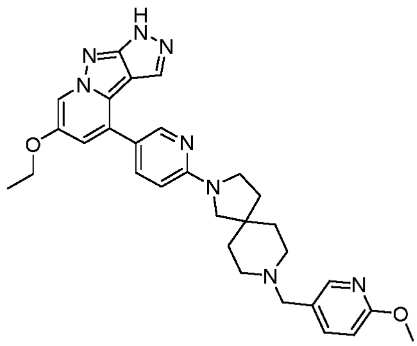

4-(6-(2,8-diazaspiro[4.5]decan-2-yl)pyridin-3-yl)-6-ethoxy-1*H*-pyrazolo[3',4':3,4]pyrazolo[1,5-*a*]py ridine (100 mg, 0.2 mmol), 6-methoxy-3-pyridinecarboxaldehyde (83 mg, 0.6 mmol), Pic-$BH_3$ (64 mg, 0.6 mmol), $Et_3N$ (120 mg, 1.2 mmol), and *n*-propanol (5 mL) were added to a reaction flask. The mixture was reacted at 45 °C for 16 h. The reaction was quenched by adding water and stirring. The reaction liquid was extracted with dichloromethane, dried over anhydrous sodium sulfate, concentrated under reduced pressure, and separated by column chromatography to give a product (15 mg), m/z = 539 $[M+1]^+$, $^1H$ NMR (400 MHz, DMSO-$d_6$) δ 12.65 (s, 1H), 8.52 (dd, *J*= 16.8, 2.3 Hz, 2H), 8.07 (s, 1H), 8.00 (dd, *J* = 8.8, 2.5 Hz, 1H), 7.65 (dd, *J* = 8.3, 2.4 Hz, 1H), 7.58 (s, 1H), 7.22 (d, *J* = 2.1 Hz, 1H), 6.80 (d, *J* = 8.4 Hz, 1H),

6.67 (d, *J* = 8.8 Hz, 1H), 4.17 (q, *J* = 6.9 Hz, 2H), 3.84 (s, 3H), 3.53 (t, *J* = 6.9 Hz, 2H), 3.45 (s, 3H), 3.33 (s, 2H), 3.27 (s, 1H), 2.51 (m, 2H), 2.21-2.42 (m, 3H), 1.87 (t, *J*= 7.0 Hz, 2H), 1.60 (s, 4H), 1.40 (t, *J*= 7.0 Hz, 3H).

**Example 77: Preparation of compound 197**

Step A: *tert*-butyl 2-(5-(6-ethoxy-1*H*-pyrazolo[3',4':3,4]pyrazolo[1,5-*a*]pyridin-4-yl)pyridin-2-yl)-2,7-diazaspiro[4.5]d ecane-7-carboxylate

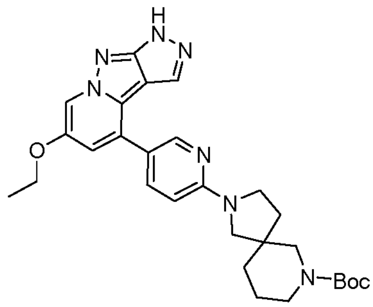

6-ethoxy-4-(6-fluoropyridin-3-yl)-1*H*-pyrazolo[3',4':3,4]pyrazolo[1,5-*a*]pyridine (310 mg, 1.04 mmol), *tert*-butyl 2,7-diazaspiro[4.5]octane-7-carboxylate (250 mg, 1.04 mmol), DIEA (430 mg, 3.12 mmol), and DMSO (5 mL) were added to a reaction flask. The mixture was reacted at 90 °C for 12 h. The reaction was quenched by adding water and stirring. The reaction liquid was extracted with dichloromethane, dried over anhydrous sodium sulfate, concentrated under reduced pressure, and separated by column chromatography to give a product (350 mg), m/z = 518 [M+1]$^+$.

Step B: 4-(6-(2,7-diazaspiro[4.5]decan-2-yl)pyridin-3-yl)-6-ethoxy-1*H*-pyrazolo[3',4':3,4]pyrazolo[1,5-*a*]py ridine hydrochloride

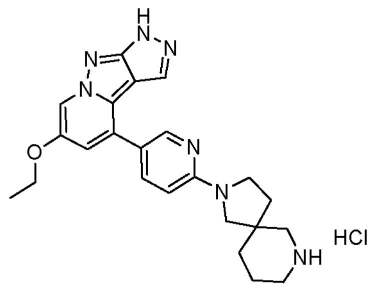

Tert-butyl

2-(5-(6-ethoxy-1*H*-pyrazolo[3',4':3,4]pyrazolo[1,5-*a*]pyridin-4-yl)pyridin-2-yl)-2,7-diazaspiro[4.5]o ctane-7-carboxylate (350 mg, 0.676 mmol) was added to a solution of HCl in methanol (4 M, 10 mL). The mixture was reacted at 25 °C for 4 h. The reaction liquid was concentrated under reduced pressure to give a product (320 mg), which was directly used in the next step, m/z = 418 [M+1]$^+$.

Step C: *N*(2-(5-(6-ethoxy-1*H*-pyrazolo[3',4':3,4]pyrazolo[1,5-a]pyridin-4-yl)pyridin-2-yl)-2,7-diazaspiro[4. 5]octan-7-yl)-2-chloro-6-fluorobenzamide

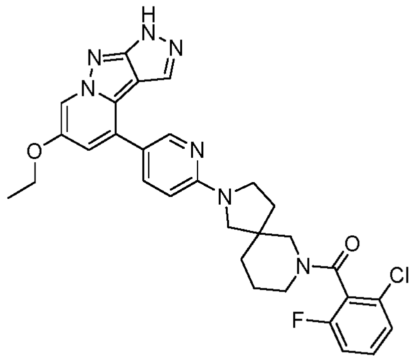

4-(6-(2,7-diazaspiro[4.5]octan-2-yl)pyridin-3-yl)-6-ethoxy-1*H*-pyrazolo[3',4':3,4]pyrazolo[1,5-*a*]py ridine hydrochloride (160 mg, 0.3 mmol), 2-chloro-6-fluorobenzoyl chloride (58 mg, 0.3 mmol), DIEA (194 mg, 1.5 mmol), and DCM (5 mL) were added to a reaction flask. The mixture was reacted at 25 °C for 1 h. The reaction liquid was quenched by adding water and stirring. The reaction liquid was extracted with dichloromethane, dried over anhydrous sodium sulfate, concentrated under reduced pressure, and separated by column chromatography to give a product (48.3 mg), m/z = 573[M+1]+, $^1$H-NMR (400MHz, DMSO-$d_6$) δ 12.63 (brs, 1H), 8.57 (s, 1H), 8.50 (s, 1H), 8.02 (d, *J* = 8.8 Hz, 1H), 7.32-7.59 (m, 4H), 7.23 (s, 1H), 6.68 (d, *J* = 8.8 Hz, 1H), 4.15-4.20 (m, 2H), 3.45-3.73 (m, 4H), 3.01-3.39 (m, 4H), 1.45-2.13 (m, 6H), 1.37-1.41 (m, 3H).

**Example 78: Preparation of compound 198**

6-ethoxy-4-(6-(7-((6-methoxypyridin-3-yl)methylene)-2,7-diazaspiro[4.5]decan-2-yl)pyridin-3-yl)-1*H*-pyrazolo[3',4':3,4]pyrazolo[1,5-*a*]pyridine

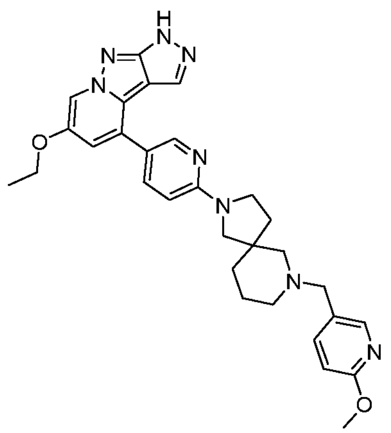

4-(6-(2,7-diazaspiro[4.5]octan-2-yl)pyridin-3-yl)-6-ethoxy-1*H*-pyrazolo[3',4':3,4]pyrazolo[1,5-*a*]py ridine hydrochloride (160 mg, 0.3 mmol), 6-methoxy-3-pyridinecarboxaldehyde (124 mg, 0.9 mmol), $Et_3N$ (152 mg, 1.5 mmol), and n-propanol (10 mL) were added to a reaction flask. The mixture was reacted at 45 °C for 48 h. The reaction was quenched by adding water and stirring. The reaction liquid was extracted with dichloromethane, dried over anhydrous sodium sulfate, concentrated under reduced pressure, and separated by column chromatography to give a product (16.0 mg), m/z = 539 [M+1]+, $^1$H-NMR (400 MHz, DMSO-$d_6$) δ 12.64 (s, 1H), 8.52 (dd, *J*= 17.8, 2.3 Hz, 2H), 7.95-8.07 (m, 2H), 7.62 (dd, *J* = 8.4, 2.4 Hz, 1H), 7.59 (s, 1H), 7.22 (d, *J* = 2.1 Hz, 1H), 6.68 (dd, *J* = 27.0, 8.6 Hz, 2H), 4.18 (q, *J* = 7.0 Hz, 2H), 3.79 (s, 3H), 3.55 (d, *J* = 10.4 Hz, 2H), 3.29-3.50 (m, 3H), 3.25 (d, *J* = 10.7 Hz, 1H), 2.46 (s, 1H), 2.29 (s, 2H), 2.15 (s, 1H), 1.97 (s, 1H), 1.78 (dt, *J*= 12.2, 8.0 Hz, 1H), 1.56 (d, *J*= 14.6 Hz, 3H), 1.40 (m, 4H).

**Example 79: Preparation of compound 199**

Step A: *tert*-butyl 2-(5-(6-ethoxy-1*H*-pyrazolo[3',4':3,4]pyrazolo[1,5-*a*]pyridin-4-yl)pyridin-2-yl)-2,6-diazaspiro[4.5]decane-6-carboxylate

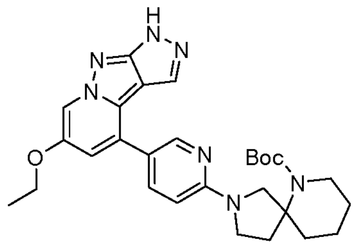

6-ethoxy-4-(6-fluoropyridin-3-yl)-1*H*-pyrazolo[3',4':3,4]pyrazolo[1,5-*a*]pyridine (125 mg, 0.4 mmol), *tert*-butyl 2,6-diazaspiro[4.5]octane-6-carboxylate (100 mg, 0.4 mmol), DIEA (1.5 g, 2.0 mmol), and DMSO (3 mL) were added to a reaction flask. The mixture was reacted at 90 °C for 12 h. The reaction was quenched by adding water and stirring. The reaction liquid was extracted with dichloromethane, dried over anhydrous sodium sulfate, concentrated under reduced pressure, and separated by column chromatography to give a product (200 mg), m/z = 518 $[M+1]^+$.

Step B: 4-(6-(2,6-diazaspiro[4.5]decan-2-yl)pyridin-3-yl)-6-ethoxy-1*H*-pyrazolo[3',4':3,4]pyrazolo[1,5-*a*]py ridine hydrochloride

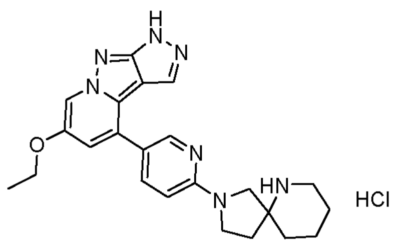

*Tert*-butyl

2-(5-(6-ethoxy-1*H*-pyrazolo[3',4':3,4]pyrazolo[1,5-*a*]pyridin-4-yl)pyridin-2-yl)-2,6-diazaspiro[4.5]o ctane-6-carboxylate (200 mg, 0.39 mmol) was added to a solution of HCl in 1,4-dioxane (4 M, 9 mL). The mixture was reacted at 25 °C for 4 h. The reaction liquid was concentrated under reduced pressure to give a product (170 mg), which was directly used in the next step, m/z = 418 $[M+1]^+$.

Step C: *N*-(2-(5-(6-ethoxy-1*H*-pyrazolo[3',4':3,4]pyrazolo[1,5-*a*]pyridin-4-yl)pyridin-2-yl)-2,6-diazaspiro[4. 5]octan-6-yl)-2-chloro-6-fluorobenzamide

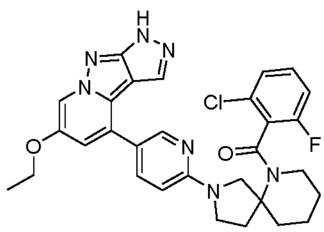

4-(6-(2,6-diazaspiro[4.5]octan-2-yl)pyridin-3-yl)-6-ethoxy-1*H*-pyrazolo[3',4':3,4]pyrazolo[1,5-*a*]py ridine hydrochloride (85 mg, 0.16 mmol), 2-chloro-6-fluorobenzoyl chloride (32 mg, 0.16 mmol), DIEA (125 mg, 1 mmol), and DCM (3 mL) were added to a reaction flask. The mixture was reacted at 25 °C for 1 h. The reaction was quenched by adding water and stirring. The reaction liquid was extracted with dichloromethane, dried over anhydrous sodium sulfate, concentrated under reduced pressure, and separated by column chromatography to give a product (60 mg), m/z = 574 $[M+1]^+$, $^1$H-NMR (400 MHz, DMSO-$d_6$) δ 12.64 (s, 1H), 8.58 (d, *J*= 2.5 Hz, 1H), 8.51 (d, *J*= 2.1 Hz, 1H), 8.05 (dt, *J* = 8.9, 1.7 Hz, 1H), 7.61 (s, 1H), 7.31-7.55 (m, 3H), 7.24 (d, *J*= 2.1 Hz, 1H), 6.75 (dd, *J* = 8.7, 4.7 Hz, 1H), 4.18 (q, *J* = 6.9 Hz, 2H), 4.07 (dd, *J* = 18.3, 7.7 Hz, 2H), 3.71 (s, 1H), 3.54 (s, 1H), 2.83 -2.70 (m, 1H), 2.51-2.65 (m, 1H), 1.46-1.93 (m, 8H), 1.41 (t, *J*= 6.9 Hz, 3H).

**Example 80: Preparation of compound 200**

6-ethoxy-4-(6-(6-((6-methoxypyridin-3-yl)methylene)-2,6-diazaspiro[4.5]decan-2-yl)pyridin-3-yl)-1*H*-pyrazolo[3',4':3,4]pyrazolo[1,5-*a*]pyridine

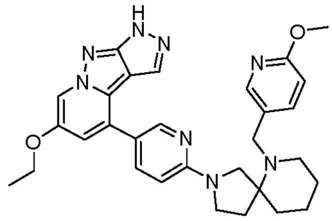

4-(6-(2,6-diazaspiro[4.5]octan-2-yl)pyridin-3-yl)-6-ethoxy-1*H*-pyrazolo[3',4':3,4]pyrazolo[1,5-*a*]py ridine hydrochloride (85 mg, 0.16 mmol), 6-methoxy-3-pyridinecarboxaldehyde (66 mg, 0.48 mmol), $Et_3N$ (81 mg, 0.8 mmol), Pic-$BH_3$ (52 mg, 0.48 mmol), and DMSO (3 mL) were added to a reaction flask. The mixture was reacted at 45 °C for 72 h. The reaction was quenched by adding water and stirring. The reaction liquid was extracted with dichloromethane, dried over anhydrous sodium sulfate, concentrated under reduced pressure, and separated by column chromatography to give a product (21.3 mg), m/z = 539 $[M+1]^+$, $^1$H-NMR (400 MHz, DMSO-$d_6$) δ 12.64 (s, 1H), 8.56 (d, *J*= 2.4 Hz, 1H), 8.51 (d, *J*= 2.1 Hz, 1H), 7.99-8.10 (m, 2H), 7.67 (dd, *J*= 8.5, 2.4 Hz, 1H), 7.58 (s, 1H), 7.23 (d, *J* = 2.1 Hz, 1H), 6.76 (dd, *J* = 12.8, 8.6 Hz, 2H), 4.18 (q, *J* = 6.9 Hz, 2H), 3.82 (s, 3H), 3.73 (m, 2H), 3.53 (dd, *J* = 14.8, 9.0 Hz, 4H), 3.31 (s, 2H), 2.44 (s, 2H), 1.70 (d, *J* = 5.9 Hz, 2H), 1.56-1.62 (m, 2H), 1.45 (d, *J*= 11.8 Hz, 2H), 1.40 (t, *J*= 6.9 Hz, 3H).

**Example 81: Preparation of compound 201**

Step A: *tert*-butyl 7-(5-(6-ethoxy-1*H*-pyrazolo[3',4':3,4]pyrazolo[1,5-*a*]pyridin-2-yl)pyridin-2-yl)-1,7-diazaspiro[3.5]n onane-1-carboxylate

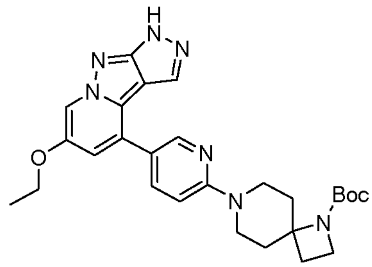

6-ethoxy-4-(6-fluoropyridin-3-yl)-1*H*-pyrazolo[3',4':3,4]pyrazolo[1,5-*a*]pyridine (300 mg, 1 mmol), tert-butyl 1,7-diazaspiro[3.5]nonane-1-carboxylate (226 mg, 1 mmol), DIEA (645 mg, 5 mmol), and DMSO (3 mL) were added to a reaction flask. The mixture was reacted at 90 °C for 12 h. The reaction was quenched by adding water and stirring. The reaction liquid was extracted with dichloromethane, dried over anhydrous sodium sulfate, concentrated under reduced pressure, and separated by column chromatography to give a product (497 mg), m/z = 504 $[M+1]^+$.

Step B: 4-(6-(1,7-diazaspiro[3.5]nonan-7-yl)pyridin-3-yl)-6-ethoxy-1*H*-pyrazolo[3',4':3,4]pyrazolo[1,5-*a*]py ridine hydrochloride

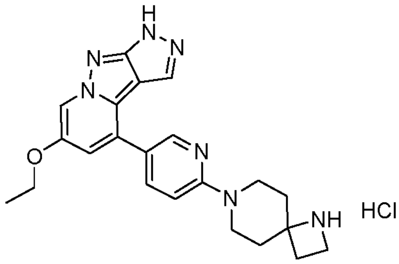

*Tert*-butyl

7-(5-(6-ethoxy-1*H*-pyrazolo[3',4':3,4]pyrazolo[1,5-*a*]pyridin-2-yl)pyridin-2-yl)-1,7-diazaspiro[3.5]n onane-1-carboxylate (497 mg, 1 mmol) was added to a solution of HCl in 1,4-dioxane (4 M, 10 mL). The mixture was reacted at 25 °C for 4 h. The reaction was quenched by adding water and stirring. The reaction liquid was extracted with dichloromethane, dried over anhydrous sodium sulfate, concentrated under reduced pressure, and separated by column chromatography to give a product (470 mg), m/z = 404 $[M+1]^+$.

Step C: *N*-(7-(5-(6-ethoxy-1*H*-pyrazolo[3',4':3,4]pyrazolo[1,5-*a*]pyridin-4-yl)pyridin-2-yl)-1,7-diazaspiro[3. 5]nonan-1-yl)-2-chloro-6-fluorobenzamide

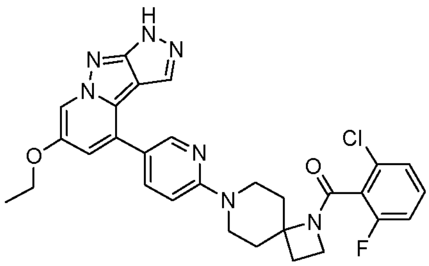

4-(6-(1,7-diazaspiro[3.5]nonan-7-yl)pyridin-3-yl)-6-ethoxy-1*H*-pyrazolo[3',4':3,4]pyrazolo[1,5-*a*]py ridine hydrochloride (50 mg, 0.1 mmol), 2-chloro-6-fluorobenzoyl chloride (19 mg, 0.1 mmol), DIEA (65 mg, 0.5 mmol), and DCM (3 mL) were added to a reaction flask. The mixture was reacted at 25 °C for 1 h. The reaction was quenched by adding water and stirring. The reaction liquid was extracted with dichloromethane, dried over anhydrous sodium sulfate, concentrated under reduced pressure, and separated by column chromatography to give a product (5.1 mg), m/z = 560 $[M+1]^+$, $^1H$ NMR (400 MHz, DMSO-$d_6$) δ 12.66 (s, 1H), 8.72 (t, *J* = 5.7 Hz, 1H), 8.60 (dd, *J* = 6.1, 2.6 Hz, 1H), 8.52 (t, *J* = 2.5 Hz, 1H), 8.11-8.02 (m, 1H), 7.60 (s, 1H), 7.57-7.41 (m, 1H), 7.46-7.24 (m, 2H), 7.02 (d, *J* = 9.0 Hz, 1H), 4.18 (q, *J* = 6.9 Hz, 2H), 4.03 (s, 2H), 3.83 (t, *J* = 5.6 Hz, 2H), 3.41 (q, *J*= 6.6 Hz, 2H), 3.00 (t, *J*= 12.6 Hz, 1H), 2.36-2.18 (m, 4H), 1.98 (d, *J*= 12.6 Hz, 1H), 1.40 (t, *J* = 7.0 Hz, 3H).

**Example 82: Preparation of compound 202**

Step A: *tert*-butyl 7-(5-(6-ethoxy-1*H*-pyrazolo[3',4':3,4]pyrazolo[1,5-*a*]pyridin-4-yl)pyridin-2-yl)-2,7-diazaspiro[3.5]n onane-2-carboxylate

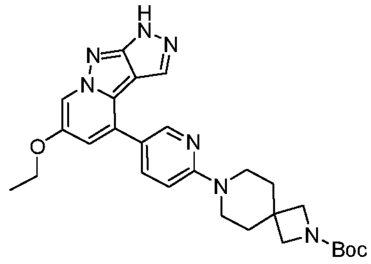

6-ethoxy-4-(6-fluoropyridin-3-yl)-1*H*-pyrazolo[3',4':3,4]pyrazolo[1,5-*a*]pyridine (600 mg, 2 mmol), *tert*-butyl 2,7-diazaspiro[3.5]nonane-2-carboxylate (100 mg, 2 mmol), DIEA (1.3 g, 10 mmol), and DMSO (10 mL) were added to a reaction flask. The mixture was reacted at 90 °C for 12 h. The reaction was quenched by adding water and stirring. The reaction liquid was extracted with dichloromethane, dried over anhydrous sodium sulfate, concentrated under reduced pressure, and separated by column chromatography to give a product (840 mg), m/z = 504 $[M+1]^+$.

Step B: 4-(6-(2,7-diazaspiro[3.5]nonan-7-yl)pyridin-3-yl)-6-ethoxy-1*H*-pyrazolo[3',4':3,4]pyrazolo[1,5-*a*]py ridine hydrochloride

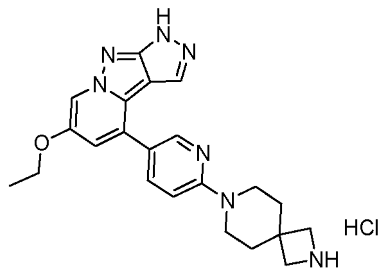

*Tert*-butyl

7-(5-(6-ethoxy-1*H*-pyrazolo[3',4':3,4]pyrazolo[1,5-*a*]pyridin-4-yl)pyridin-2-yl)-2,7-diazaspiro[3.5]n onane-2-carboxylate (840 mg, 1.67 mmol) was added to a solution of HCl in 1,4-dioxane (4 M, 20 mL). The mixture was reacted at 25 °C for 4 h. The reaction was quenched by adding water and stirring. The reaction liquid was extracted with dichloromethane, dried over anhydrous sodium sulfate, concentrated under reduced pressure, and separated by column chromatography to give a product (730 mg), m/z = 404 $[M+1]^+$.

Step C: *N*-(7-(5-(6-ethoxy-1*H*-pyrazolo[3',4':3,4]pyrazolo[1,5-*a*]pyridin-4-yl)pyridin-2-yl)-2,7-diazaspiro[3. 5]nonan-2-yl)-2-chloro-6-fluorobenzamide

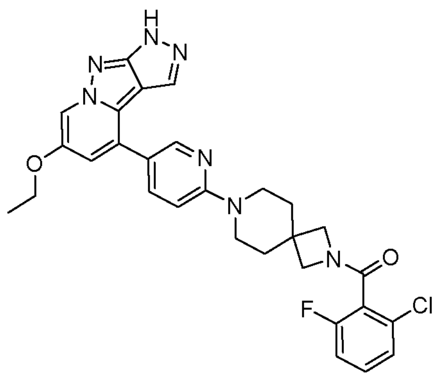

4-(6-(2,7-diazaspiro[3.5]nonan-7-yl)pyridin-3-yl)-6-ethoxy-1*H*-pyrazolo[3',4':3,4]pyrazolo[1,5-*a*]py ridine hydrochloride (50 mg, 0.1 mmol), 2-chloro-6-fluorobenzoyl chloride (19 mg, 0.1 mmol), DIEA (65 mg, 0.5 mmol), and DCM (3 mL) were added to a reaction flask. The mixture was reacted at 25 °C for 1 h. The reaction was quenched by adding water and stirring. The mixture was extracted with dichloromethane, dried over anhydrous sodium sulfate, concentrated under reduced pressure, and separated by column chromatography to give a product (19.2 mg), m/z = 560 $[M+1]^+$, $^1$H-NMR (400 MHz, DMSO-$d_6$) δ 12.64 (brs, 1H), 8.57 (s, 1H), 8.51 (s, 1H), 8.03 (d, *J* = 8.8 Hz, 1H), 7.57 (s, 1H), 7.51-7.55 (m, 1H), 7.44 (d, *J* = 8.0 Hz, 1H), 7.35-7.39 (m, 1H), 7.25 (s, 1H), 7.10 (d, *J* = 8.8 Hz, 1H), 4.14-4.20 (m, 2H), 3.89 (s, 2H), 3.50-3.71 (m, 6H), 1.70-1.89 (m, 4H), 1.38-1.43 (m, 3H).

**Example 83: Preparation of compound 203**

6-ethoxy-4-(6-(2-((6-methoxypyridin-3-yl)methylene)-2,7-diazaspiro[3.5]nonan-7-yl)pyridin-3-yl)-1*H*-pyrazolo[3',4':3,4]pyrazolo[1,5-*a*]pyridine

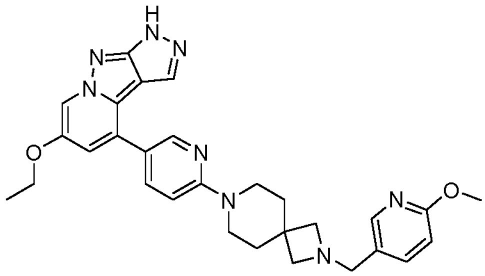

4-(6-(2,7-diazaspiro[3.5]nonan-7-yl)pyridin-3-yl)-6-ethoxy-1*H*-pyrazolo[3',4':3,4]pyrazolo[1,5-*a*]py ridine hydrochloride (100 mg, 0.19 mmol), 6-methoxy-3-pyridinecarboxaldehyde (80 mg, 0.58 mmol), $Et_3N$ (98 mg, 0.97 mmol), Pic-$BH_3$ (63 mg, 0.58 mmol), and DMSO (3 mL) were added to a reaction flask. The mixture was reacted at 45 °C for 12 h. The reaction was quenched by adding water and stirring. The reaction liquid was extracted with dichloromethane, dried over anhydrous sodium sulfate, concentrated under reduced pressure, and separated by column chromatography to give a product (6.1 mg), m/z = 525 $[M+1]^+$, $^1$H-NMR(400 MHz, DMSO-$d_6$) δ 12.64 (brs,1H), 8.56 (s, 1H), 8.50 (s, 1H), 8.06 (s, 1H), 8.02 (d, *J* = 8.8 Hz, 1H), 7.62 (d, *J* = 8.8 Hz, 1H), 7.56 (s, 1H), 7.24 (s, 1H), 7.06 (d, *J* = 8.0 Hz, 1H), 6.78 (d, *J* = 8.0 Hz, 1H), 4.14-4.19 (m,2H), 3.83 (s, 3H), 3.48-3.67 (m, 6H), 2.92-3.09 (m, 4H), 1.65-1.81 (m, 4H), 1.38-1.43 (m, 3H).

**Example 84: Preparation of compound 206**

Step A: *tert*-butyl 8-(5-(6-ethoxy-1*H*-pyrazolo[3',4':3,4]pyrazolo[1,5-*a*]pyridin-4-yl)pyridin-2-yl)-1,8-diazaspiro[4.5]d ecane-1-carboxylate

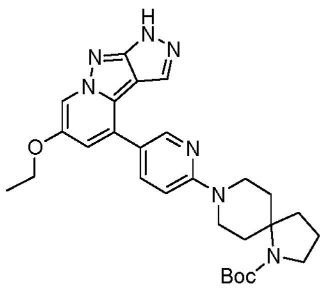

6-ethoxy-4-(6-fluoropyridin-3-yl)-1*H*-pyrazolo[3',4':3,4]pyrazolo[1,5-*a*]pyridine (150 mg, 0.5 mmol), tert-butyl 1,8-diazaspiro[4.5]decane-1-carboxylate (120 mg, 0.5 mmol), DIEA (323 mg, 2.5 mmol), and DMSO (3 mL) were added to a reaction flask. The mixture was reacted at 90 °C for 12 h. The reaction was quenched by adding water and stirring. The mixture was extracted with dichloromethane, dried over anhydrous sodium sulfate, concentrated under reduced pressure, and separated by column chromatography to give a product (181 mg), m/z = 518 $[M+1]^+$.

Step B: 4-(6-(1,8-diazaspiro[4.5]decan-8-yl)pyridin-3-yl)-6-ethoxy-1*H*-pyrazolo[3',4':3,4]pyrazolo[1,5-*a*]py ridine hydrochloride

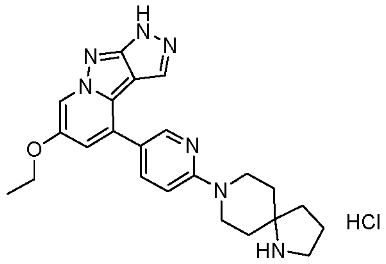

Tert-butyl

8-(5-(6-ethoxy-1*H*-pyrazolo[3',4':3,4]pyrazolo[1,5-*a*]pyridin-4-yl)pyridin-2-yl)-1,8-diazaspiro[4.5]d ecane-1-carboxylate (181 mg, 0.35 mmol) was added to a solution of HCl in 1,4-dioxane (4 M, 5 mL). The mixture was reacted at 25 °C for 4 h. The reaction liquid was concentrated under reduced pressure to give a product (150 mg), which was directly used in the next step, m/z = 418 $[M+1]^+$.

Step C: *N*-(8-(5-(6-ethoxy-1*H*-pyrazolo[3',4':3,4]pyrazolo[1,5-*a*]pyridin-4-yl)pyridin-2-yl)-1,8-diazaspiro[4. 5]decan-1-yl)2-chloro-6-fluorobenzamide

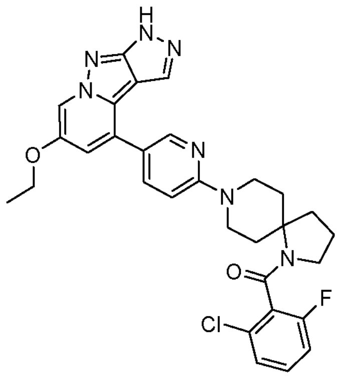

4-(6-(1,8-diazaspiro[4.5]decan-8-yl)pyridin-3-yl)-6-ethoxy-1*H*-pyrazolo[3',4':3,4]pyrazolo[1,5-*a*]py ridine hydrochloride, 2-chloro-6-fluorobenzoyl chloride (18 mg, 0.1 mmol), DIEA (65 mg, 0.5 mmol), and DCM (3 mL) were added to a reaction flask. The mixture was reacted at 25 °C for 1 h. The reaction was quenched by adding water and stirring. The reaction liquid was extracted with dichloromethane, dried over anhydrous sodium sulfate, concentrated under reduced pressure, and separated by column chromatography to give a product (2.6 mg), m/z = 574 $[M+1]^+$, $^1H$ NMR (400 MHz, DMSO-$d_6$) δ 12.65 (brs, 1H), 8.60 (s, 1H), 8.52 (s, 1H), 8.04 (d, *J* = 8.8 Hz, 1H), 7.60 (s, 1H), 7.55-7.63 (m, 2H), 7.27-7.38 (m, 2H), 7.12 (s, *J*= 11.2 Hz, 1H), 4.48-4.56 (m, 2H), 4.17 (q, *J* = 6.8 Hz, 2H), 3.16-3.24 (m, 2H), 2.89-3.08 (m, 4H), 2.12-2.20 (m, 2H), 1.80-1.87 (m, 2H), 1.52-1.58 (m, 2H), 1.38 (t, *J*= 6.8 Hz, 3H).

**Example 85: Preparation of compound 207**

6-ethoxy-4-(6-(1-((6-methoxypyridin-3-yl)methylene)-1,8-diazaspiro[4.5]octan-8-yl)pyridin-3-yl)-1 *H*-razolo[3',4':3,4]razolo[1,5-*a*]pyridine

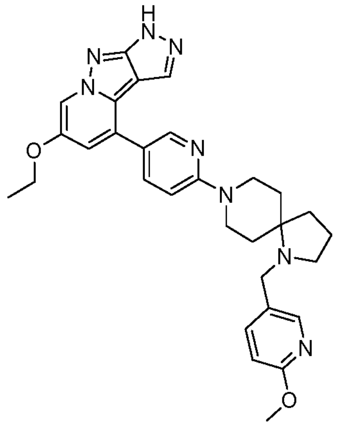

4-(6-(1,8-diazaspiro[4.5]decan-8-yl)pyridin-3-yl)-6-ethoxy-1*H*-pyrazolo[3',4':3,4]pyrazolo[1,5-*a*]py ridine hydrochloride (100 mg, 0.19 mmol), 6-methoxy-3-pyridinecarboxaldehyde (80 mg, 0.57 mmol), $Et_3N$ (98 mg, 0.8 mmol), Pic-$BH_3$ (63 mg, 0.57 mmol), and DMSO (3 mL) were added to a reaction flask. The mixture was reacted at 45 °C for 12 h. The reaction was quenched by adding water and stirring. The reaction liquid was extracted with dichloromethane, dried over anhydrous sodium sulfate, concentrated under reduced pressure, and separated by column chromatography to give a product (23.8 mg), m/z = 539 $[M+1]^+$, $^1$H-NMR (400 MHz, DMSO-$d_6$): δ 12.65 (brs, 1H), 8.58 (s, 1H), 8.51 (s, 1H), 7.99-8.05 (m, 2H), 7.54-7.63 (m, 2H), 7.25 (s, 1H), 7.09 (d, *J*= 8.8 Hz, 1H), 6.75 (d, *J* = 8.8 Hz, 1H), 4.46-4.55 (m, 2H), 4.15-4.20 (m, 2H), 3.81 (s, 3H), 3.50 (s, 2H), 2.93-3.00 (m, 2H), 2.55-3.59 (m, 2H), 1.85-1.92 (m, 2H), 1.65-1.84 (m, 4H), 1.40-1.51 (m, 2H), 1.39-1.41 (m, 3H).

**Example 86: Preparation of compound 208**

Step A: *tert*-butyl 8-(5-(6-ethoxy-1*H*-pyrazolo[3',4':3,4]pyrazolo[1,5-*a*]pyridin-4-yl)pyridin-2-yl)-2,8-diazaspiro[4.5]d ecane-2-carbonate

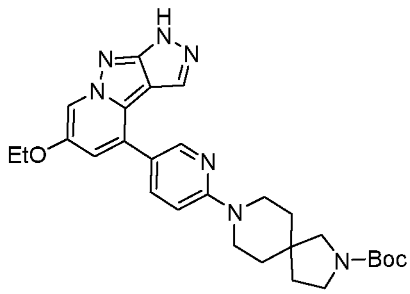

6-ethoxy-4-(6-fluoropyridin-3-yl)-1*H*-pyrazolo[3',4':3,4]pyrazolo[1,5-*a*]pyridine (297 mg, 1.0 mmol), *tert*-butyl 2,8-diazaspiro[4.5]decane-2-carboxylate (240 mg, 1.0 mmol), diisopropylethylamine (390 mg, 3.0 mmol), and DMSO (4 mL) were sequentially added to a reaction flask. The mixture was heated to 100 °C and reacted for 16 h. The reaction was quenched by adding water and stirring. The reaction liquid was extracted with dichloromethane, dried over anhydrous sodium sulfate, concentrated under reduced pressure, and separated by column chromatography to give a product (510 mg), m/z = 518 $[M+1]^+$.

Step B: 4-(6-(2,8-diazaspiro[4.5]decan-8-yl)pyridin-3-yl)-6-ethoxy-1*H*-pyrazolo[3',4':3,4]pyrazolo[1,5-*a*]py ridine hydrochloride

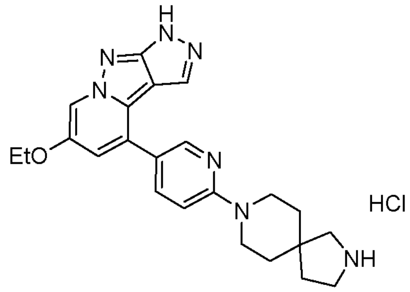

Methanol (5 mL), *tert*-butyl 9-(5-(6-ethoxy-1*H*-pyrazolo[3',4':3,4]pyrazolo[1,5-*a*]pyridin-4-yl)pyridin-2-yl)-3,9-diazaspiro[5.5]u ndecane-3-carbonate (510 mg, 1.0 mmol), and a 6 N solution of HCl in 1,4-dioxane (4.0 mL, 24.0 mmol) were sequentially added to a reaction flask. The mixture was reacted at room temperature for 30 min. The reaction liquid was directly concentrated under reduced pressure to give a product (320 mg), m/z = 418 [M+1]+.

Step C: *N*-(8-(5-(6-ethoxy-1*H*-pyrazolo[3',4':3,4]pyrazolo[1,5-*a*]pyridin-4-yl)pyridin-2-yl)-2,8-diazaspiro[4. 5]decan-2-yl)-2-chloro-6-fluorobenzamide

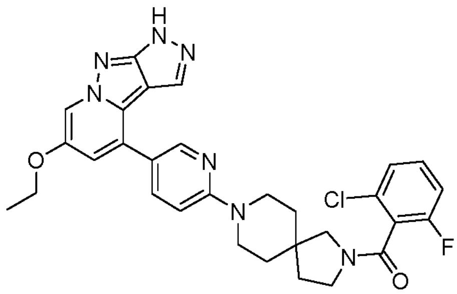

4-(6-(2,8-diazaspiro[4.5]decan-8-yl)pyridin-3-yl)-6-ethoxy-1*H*-pyrazolo[3',4':3,4]pyrazolo[1,5-*a*]py ridine (63 mg, 0.15 mmol), DMF (1 mL), and diisopropylethylamine (58 mg, 0.45 mmol) were sequentially added to a reaction flask. The mixture was cooled to 5 °C under an ice bath, and a solution of 2-chloro-6-fluorobenzoyl chloride (29 mg, 0.15 mmol) in DMF (0.2 mL) was added dropwise. The mixture was reacted at room temperature for 16 h. The reaction was quenched by adding water and stirring. The reaction liquid was extracted with dichloromethane, dried over anhydrous sodium sulfate, concentrated under reduced pressure, and separated by column chromatography to give a product (23 mg), m/z = 574 [M+1]+, $^1$H NMR (400 MHz, DMSO-$d_6$) δ 12.64 (brs, 1H), 8.55 (s, 1H), 8.50 (s, 1H), 8.00-8.04 (m, 1H), 7.35-7.56 (m, 4H), 7.23-7.26 (m, 1H), 7.04-7.08 (m, 1H), 4.16-4.18 (m, 2H), 3.52-3.79 (m, 6H), 3.20-3.26 (m, 1H), 3.06-3.10 (m, 1H), 1.50-1.71 (m, 4H), 1.38-1.43 (m, 3H).

**Example 87: Preparation of compound 209**

Step A: 6-ethoxy-4-(6-(2-((6-methoxypyridin-3-yl)methylene)-2,8-diazaspiro[4.5]decan-8-yl)pyridin-3-yl)-1*H*-pyrazolo[3',4':3,4]pyrazolo[1,5-*a*]pyridine

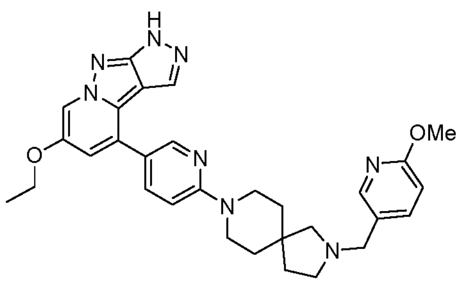

1,2-dichloroethane (2 mL), methanol (1 mL), 4-(6-(2,8-diazaspiro[4.5]decan-8-yl)pyridin-3-yl)-6-ethoxy-1*H*-pyrazolo[3',4':3,4]pyrazolo[1,5-*a*]py ridine (63 mg, 0.15 mmol), 6-methoxynicotinaldehyde (21 mg, 0.15 mmol), and sodium triacetoxyborohydride (96 mg, 0.45 mmol) were sequentially added to a reaction flask. The mixture was heated to 45 °C and reacted for 1 h. The reaction was quenched by adding water and stirring. The reaction liquid was extracted with dichloromethane, dried over anhydrous sodium sulfate, concentrated under reduced pressure, and separated by column chromatography to give a product (24 mg), m/z = 539 $[M+1]^+$, $^1$H-NMR (400 MHz, DMSO-$d_6$) $\delta$ 12.65 (brs, 1H), 8.56 (s, 1H), 8.51 (s, 1H), 8.02-8.15 (m, 1H), 8.02 (d, *J* = 8.8 Hz, 1H), 7.65-7.78 (m, 1H), 7.57 (s, 1H), 7.24 (s, 1H), 7.06 (d, *J* = 8.8 Hz, 1H), 6.76-6.89 (m, 1H), 4.14-4.20 (m, 2H), 3.84 (s, 3H), 3.50-3.75 (m, 6H), 3.30-3.50 (m, 3H), 1.91 (s, 3H),1.50-1.79 (m, 6H), 1.38-1.41 (m, 3H).

**Example 88: Preparation of compound 210**

Step A: *tert*-butyl 7-(5-(6-ethoxy-1*H*-pyrazolo[3',4':3,4]pyrazolo[1,5-*a*]pyridin-4-yl)pyridin-2-yl)-2,7-diazaspiro[4.5]d ecane-2-carbonate

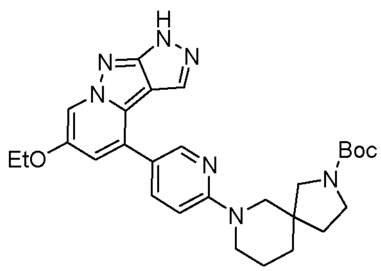

6-ethoxy-4-(6-fluoropyridin-3-yl)-1*H*-pyrazolo[3',4':3,4]pyrazolo[1,5-*a*]pyridine (150 mg, 0.5 mmol), *tert*-butyl 2,7-diazaspiro[4.5]decane-2-carboxylate hemioxalate (250 mg, 1.0 mmol), diisopropylethylamine (390 mg, 3.0 mmol), and DMSO (4 mL) were sequentially added to a reaction flask. The mixture was heated to 100 °C and reacted for 16 h. The reaction was quenched by adding water and stirring. The reaction liquid was extracted with dichloromethane, dried over anhydrous sodium sulfate, concentrated under reduced pressure, and separated by column chromatography to give a product (525 mg), m/z = 518 $[M+1]^+$.

Step B: 4-(6-(2,7-diazaspiro[4.5]decan-7-yl)pyridin-3-yl)-6-ethoxy-1*H*-pyrazolo[3',4':3,4]pyrazolo[1,5-*a*]py ridine

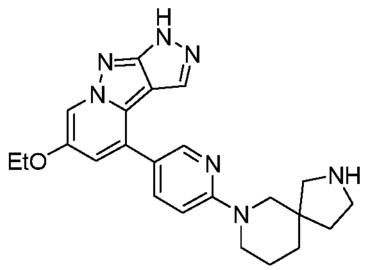

Methanol (5 mL), *tert*-butyl 7-(5-(6-ethoxy-1*H*-pyrazolo[3',4':3,4]pyrazolo[1,5-*a*]pyridin-4-yl)pyridin-2-yl)-2,7-diazaspiro[4.5]d ecane-2-carbonate (525 mg, 1.0 mmol) and a 6 N solution of HCl in 1,4-dioxane (4.0 mL, 24.0 mmol) were sequentially added to a reaction flask. The mixture was stirred at room temperature for 30 min. The reaction was quenched by adding water and stirring. The reaction liquid was adjusted to pH 10 with 1 N NaOH, extracted with dichloromethane, dried over anhydrous sodium sulfate, and concentrated to give a product (301 mg), which was directly used in the next step, m/z = 418 $[M+1]^+$.

Step C: *N*-(7-(5-(6-ethoxy-1*H*-pyrazolo[3',4':3,4]pyrazolo[1,5-*a*]pyridin-4-yl)pyridin-2-yl)-2,7-diazaspiro[4. 5]decan-2-yl)-2-chloro-6-fluorobenzamide

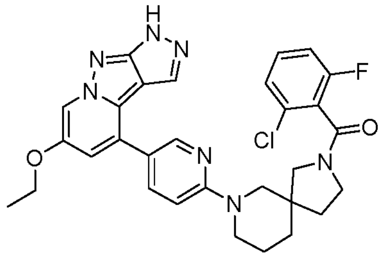

4-(6-(2,7-diazaspiro[4.5]decan-7-yl)pyridin-3-yl)-6-ethoxy-1*H*-pyrazolo[3',4':3,4]pyrazolo[1,5-*a*]py ridine (63 mg, 0.15 mmol), DMF (1 mL), and diisopropylethylamine (58 mg, 0.45 mmol) were sequentially added to a reaction flask. The mixture was cooled to 5 °C under an ice bath, and then a solution of 2-chloro-6-fluorobenzoyl chloride (29, 0.15 mmol) in DMF (0.2 mL) was added dropwise. The resulting mixture was stirred at room temperature for 16 h. The reaction was quenched by adding water and stirring. The reaction liquid was extracted with dichloromethane, dried over anhydrous sodium sulfate, concentrated under reduced pressure, and separated by column chromatography to give a product (23 mg), m/z = 574 $[M+1]^+$, $^1$H-NMR (400 MHz, DMSO-$d_6$) $\delta$ 12.64 (brs, 1H), 8.50-8.55 (m, 2H), 7.97-8.04 (m, 1H), 7.30-7.61 (m, 4H), 7.25 (s, 1H), 7.01-7.12 (m, 1H), 4.16-4.18 (m, 2H), 3.33-3.70 (m, 6H), 3.22-3.28 (m, 1H), 3.12-3.19 (m, 1H), 1.52-2.01 (m, 6H), 1.38-1.43 (m, 3H).

**Example 89: Preparation of compound 211**

Step A: 6-ethoxy-4-(6-(2-((6-methoxypyridin-3-yl)methylene)-2,8-diazaspiro[4.5]decan-8-yl)pyridin-3-yl)-1*H*-pyrazolo[3',4':3,4]pyrazolo[1,5-*a*]pyridine

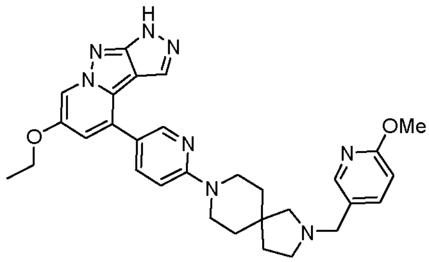

1,2-dichloroethane (2 mL), methanol (1 mL), 4-(6-(2,7-diazaspiro[4.5]decan-7-yl)pyridin-3-yl)-6-ethoxy-1*H*-pyrazolo[3',4':3,4]pyrazolo[1,5-*a*]py ridine (63 mg, 0.15 mmol), 6-methoxy-3-pyridinecarboxaldehyde (21 mg, 0.15 mmol), and acetic acid (2 drops) were sequentially added to a reaction flask. The mixture was heated to 45 °C and reacted for 1 h, and then sodium triacetoxyborohydride (96 mg, 0.45 mmol) was added. The mixture was successively stirred at 45 °C for 16 h. The reaction was quenched by adding water and stirring. The reaction liquid was extracted with dichloromethane, dried over anhydrous sodium sulfate, concentrated under reduced pressure, and separated by column chromatography to give a product (16 mg), m/z = 539 $[M+1]^+$, $^1$H-NMR (400 MHz, DMSO-$d_6$) $\delta$ 12.65 (brs, 1H), 8.53 (s, 1H), 8.51 (s, 1H), 8.02-8.15 (m, 1H), 7.98 (d, *J*= 8.8 Hz, 1H), 7.62-7.75 (m, 1H), 7.58 (s, 1H), 7.24 (s, 1H), 7.00 (d, *J*= 8.8 Hz, 1H), 6.69-6.79 (m, 1H), 4.15-4.20 (m, 2H), 3.76 (s, 3H), 3.34-3.75 (m, 8H), 3.16-3.18 (m, 1H), 1.91 (s, 1H), 1.49-1.68 (m, 6H), 1.38-1.42 (m, 3H).

**Example 90: Preparation of compound 212**

Step A: *tert*-butyl 7-(5-(6-ethoxy-1*H*-pyrazolo[3',4':3,4]pyrazolo[1,5-*a*]pyridin-4-yl)pyridin-2-yl)-1,7-diazaspiro[4.5]d ecane-1-carbonate

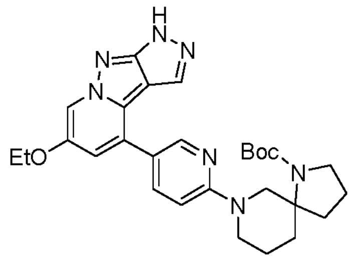

6-ethoxy-4-(6-fluoropyridin-3-yl)-1*H*-pyrazolo[3',4':3,4]pyrazolo[1,5-*a*]pyridine (297 mg, 1.0 mmol), *tert*-butyl 1,7-diazaspiro[4.5]decane-1-carboxylate (240 mg, 1.0 mmol), diisopropylethylamine (390 mg, 3.0 mmol), and DMSO (4 mL) were sequentially added to a reaction flask. The reaction liquid was heated to 100 °C and reacted for 16 h. The reaction was quenched by adding water and stirring. The reaction liquid was extracted with dichloromethane, dried over anhydrous sodium sulfate, concentrated under reduced pressure, and separated by column chromatography to give a product (500 mg), m/z = 518 $[M+1]^+$.

Step B: 4-(6-(1,7-diazaspiro[4.5]decan-7-yl)pyridin-3-yl)-6-ethoxy-1*H*-pyrazolo[3',4':3,4]pyrazolo[1,5-*a*]py ridine

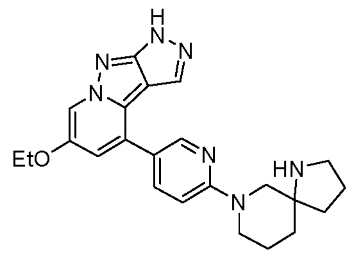

Methanol (5 mL), tert-butyl 7-(5-(6-ethoxy-1*H*-pyrazolo [3',4':3,4]pyrazolo[1,5-*a*]pyridin-4-yl)pyridin-2-yl)-1,7-diazaspiro[4.5]decane-1-carbonate (500 mg, 1.0 mmol) and a 6 N solution of HCl in 1,4-dioxane (4.0 mL, 24.0 mmol) were sequentially added to a reaction flask. The mixture was stirred at room temperature for 30 min. The reaction liquid was concentrated, and 10 mL of water was added for dilution. The dilution was adjusted to pH 10 with 1 N NaOH and extracted twice with dichloromethane (30 mL). The extract was washed with saturated brine (10 mL), dried over anhydrous sodium sulfate, and concentrated to give a product (310 mg), m/z = 418 $[M+1]^+$.

Step C: *N*-(7-(5-(6-ethoxy-1*H*-pyrazolo[3',4':3,4]pyrazolo[1,5-*a*]pyridin-4-yl)pyridin-2-yl)-1,7-diazaspiro[4. 5]decan-1-yl)-2-chloro-6-fluorobenzamide

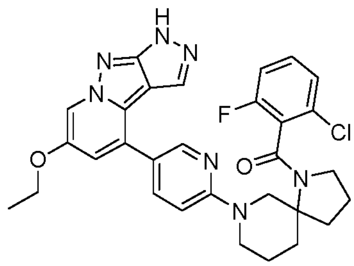

4-(6-(1,7-diazaspiro[4.5]decan-7-yl)pyridin-3-yl)-6-ethoxy-1*H*-pyrazolo[3',4':3,4]pyrazolo[1,5-*a*]py ridine (63 mg, 0.15 mmol), DMF (1 mL), and diisopropylethylamine (58 mg, 0.45 mmol) were sequentially added to a reaction flask. The mixture was cooled to 5 °C under an ice bath, and then a solution of 2-chloro-6-fluorobenzoyl chloride (29, 0.15 mmol) in DMF (0.2 mL) was added dropwise. The resulting mixture was stirred at room temperature for 16 h. The reaction was quenched by adding water and stirring. The reaction liquid was extracted with dichloromethane, dried over anhydrous sodium sulfate, concentrated under reduced pressure, and separated by column chromatography to give a product (27 mg), m/z = 574 $[M+1]^+$, $^1$H-NMR (400 MHz, DMSO-$d_6$) $\delta$ 12.64 (brs, 1H), 8.56 (s, 1H), 8.51 (s, 1H), 8.03 (d, *J* = 8.8Hz, 1H), 7.60 (s, 1H), 7.36-7.52 (m, 3H), 7.26 (s, 1H), 7.12-7.16 (m, 1H), 4.43-4.46 (m, 2H), 4.15-4.18 (m, 2H), 3.86-3.91 (m, 1H), 3.20-3.26 (m, 2H), 3.00-3.10 (m, 1H), 2.85-2.96 (m, 1H), 2.05-2.13 (m, 1H), 1.71-1.92 (m, 4H), 1.61-1.70 (m, 2H),1.38-1.42 (m, 3H).

**Example 91: Preparation of compound 213**

6-ethoxy-4-(6-(1-((6-methoxypyridin-3-yl)methylene)-1,7-diazaspiro[4.5]decan-7-yl)pyridin-3-yl)-1*H*-pyrazolo[3',4':3,4]pyrazolo[1,5-a]pyridine

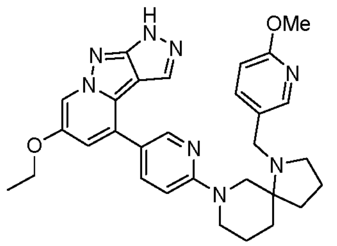

4-(6-(1,7-diazaspiro[4.5]decan-7-yl)pyridin-3-yl)-6-ethoxy-1*H*-pyrazolo[3',4':3,4]pyrazolo[1,5-*a*]py ridine (42 mg, 0.1 mmol), DMF (1 mL), potassium carbonate (40 mg, 0.3 mmol), and 5-(chloromethyl)-2-methoxypyridine (16 mg, 0.1 mmol) were sequentially added to a reaction flask. The mixture was heated to 60 °C and stirred for 25 h. The reaction was quenched by adding water and stirring. The reaction liquid was extracted with dichloromethane, dried over anhydrous sodium sulfate, concentrated under reduced pressure, and separated by column chromatography to give a product (4.1 mg), m/z = 539 $[M+1]^+$, $^1$H-NMR (400 MHz, DMSO-$d_6$) $\delta$ 12.65 (brs, 1H), 8.55 (s, 1H), 8.50 (s, 1H), 8.07 (s, 1H), 8.01 (d, *J*= 8.8 Hz, 1H), 7.66 (d, *J*= 8.8 Hz, 1H), 7.59 (s, 1H), 7.25 (s, 1H), 7.07 (d, *J* = 8.8 Hz, 1H), 6.78 (d, *J* = 8.8 Hz, 1H), 4.39-4.43 (m, 1H), 4.27 (d, *J* = 12.0 Hz, 1H), 4.15-4.20 (m, 2H), 3.84 (s, 3H), 3.67-3.79 (m, 2H), 2.93 (d, *J* = 12.0 Hz, 1H), 2.58-2.83 (m, 3H), 1.50-1.91 (m, 8H), 1.38-1.41 (m, 3H).

**Example 92: Preparation of compound 214**

Step A: tert-butyl 9-(5-(6-ethoxy-1*H*-pyrazolo[3',4':3,4]pyrazolo[1,5-*a*]pyridin-4-yl)pyridin-2-yl)-2,9-diazaspiro[5.5]u ndecane-2-carbonate

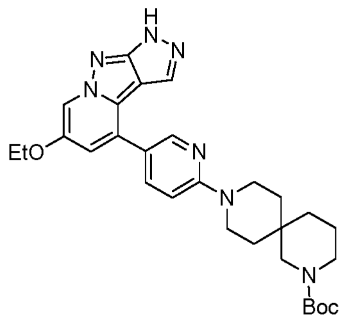

6-ethoxy-4-(6-fluoropyridin-3-yl)-1*H*-pyrazolo[3',4':3,4]pyrazolo[1,5-*a*]pyridine (297 mg, 1.0 mmol), *tert*-butyl 2,9-diazaspiro[5.5]undecane-2-carboxylate (254 mg, 1.0 mmol), diisopropylethylamine (390 mg, 3.0 mmol), and DMSO (4 mL) were sequentially added to a reaction flask. The reaction liquid was heated to 100 °C and reacted for 16 h. The reaction was quenched by adding water and stirring. The reaction liquid was extracted with dichloromethane, dried over anhydrous sodium sulfate, concentrated under reduced pressure, and separated by column chromatography to give a product (540 mg), m/z = 532 [M+1]$^+$.

Step B: 4-(6-(2,9-diazaspiro[5.5]undecan-9-yl)pyridin-3-yl)-6-ethoxy-1*H*-pyrazolo[3',4':3,4]pyrazolo[1,5-*a*] pyridine

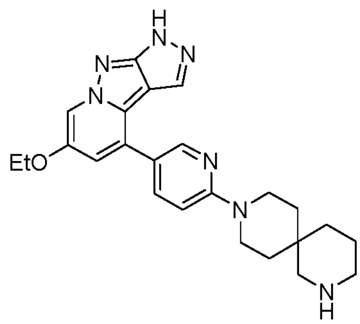

Methanol (5 mL), 9-(5-(6-ethoxy-1*H*-pyrazolo[3',4':3,4]pyrazolo[1,5-*a*]pyridin-4-yl)pyridin-2-yl)-2,9-diazaspiro[5.5]u ndecane-2-carbonate (540 mg, 1.0 mmol) and a 6 N solution of HCl in 1,4-dioxane (2.0 mL, 12.0 mmol) were sequentially added to a reaction flask. The mixture was stirred at room temperature for 30 min. The reaction liquid was concentrated, and water (3 mL) was added. The mixture was adjusted to pH 10 with 1 N NaOH and extracted twice with dichloromethane (30 mL × 2). The extract was washed with saturated brine (10 mL), dried over anhydrous sodium sulfate, and concentrated to give a product (345 mg), m/z = 432 [M+1]$^+$.

Step C: *N*-(9-(5-(6-ethoxy-1*H*-pyrazolo[3',4':3,4]pyrazolo[1,5-*a*]pyridin-4-yl)pyridin-2-yl)-2,9-diazaspiro[5. 5]undecan-2-yl)-2-chloro-6-fluorobenzamide

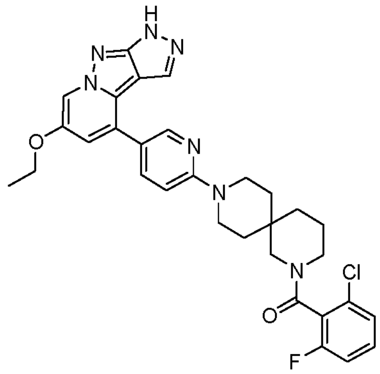

4-(6-(2,9-diazaspiro[5.5]undecan-9-yl)pyridin-3-yl)-6-ethoxy-1*H*-pyrazolo[3',4':3,4]pyrazolo[1,5-*a*] pyridine (130 mg, 0.3 mmol), DMF (2 mL), and diisopropylethylamine (117 mg, 0.9 mmol) were sequentially added to a reaction flask. The mixture was cooled to 5 °C under an ice bath, and then a solution of 2-chloro-6-fluorobenzoyl chloride (58 mg, 0.3 mmol) in DMF (0.2 mL) was added dropwise. The resulting mixture was stirred at room temperature for 16 h. The reaction was quenched by adding water and stirring. The reaction liquid was extracted with dichloromethane, dried over anhydrous sodium sulfate, concentrated under reduced pressure, and separated by column chromatography to give a product (45 mg), m/z = 588 $[M+1]^+$, $^1$H-NMR (400 MHz, DMSO-$d_6$) δ 12.65 (brs, 1H), 8.59 (s, 1H), 8.53 (s, 1H), 8.04 (d, *J* = 8.8 Hz, 1H), 7.60 (s, 1H), 7.36-7.56 (m, 3H), 7.27 (s, 1H), 7.10 (d, *J* = 8.8Hz, 1H), 4.14-4.20 (m, 2H), 3.80-3.95 (m, 2H), 3.48-3.72 (m, 4H), 3.15-3.28 (m, 2H), 1.47-1.77 (m, 8H), 1.38-1.42 (m, 3H).

**Example 93: Preparation of compound 215**

6-ethoxy-4-(6-(2-((6-methoxypyridin-3-yl)methylene)-2,9-diazaspiro[5.5]undecan-9-yl)pyridin-3-yl )-1*H*-razolo[3',4':3,4]razolo[1,5-*a*]pyridine

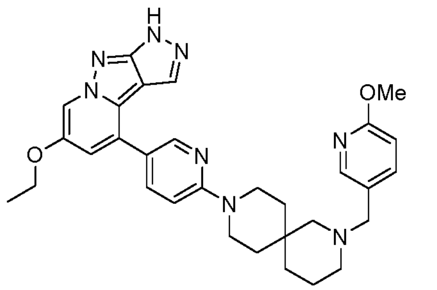

1,2-dichloroethane (2 mL), methanol (1 mL), 4-(6-(2,9-diazaspiro[5.5]undecan-9-yl)pyridin-3-yl)-6-ethoxy-1*H*-pyrazolo[3',4':3,4]pyrazolo[1,5-*a*] pyridine (65 mg, 0.15 mmol), 6-methoxy-3-pyridinecarboxaldehyde (62 mg, 0.45 mmol), and acetic acid (2 drops) were sequentially added to a reaction flask. The mixture was heated to 45 °C and reacted for 2 h, and then sodium triacetoxyborohydride (160 mg, 0.75 mmol) was added. The mixture was successively stirred at 45 °C for 16 h. The reaction was quenched by adding water and stirring. The reaction liquid was extracted with dichloromethane, dried over anhydrous sodium sulfate, concentrated under reduced pressure, and separated by column chromatography to give a product (17 mg), m/z = 553 $[M+1]^+$, $^1$H-NMR (400 MHz, DMSO-$d_6$) δ 12.65 (brs, 1H), 8.54 (s, 1H), 8.50 (s, 1H), 8.05 (s, 1H), 8.00 (d, *J*= 8.8 Hz, 1H), 7.65 (d, *J*= 8.8 Hz, 1H), 7.57 (s, 1H), 7.24 (s, 1H), 7.01 (d, *J*= 8.8 Hz, 1H), 6.79 (d, *J* = 8.8Hz, 1H), 4.14-4.20 (m, 2H), 3.82 (s, 3H), 3.55-3.67 (m, 2H), 3.42-3.50 (m, 2H), 3.40 (s, 1H), 3.16-3.18 (m, 1H), 2.10-2.42 (m, 4H),1.30-1.60 (m, 11H).

**Example 94: Preparation of compound 216**

Step A: *tert*-butyl 9-(5-(6-ethoxy-1*H*-pyrazolo[3',4':3,4]pyrazolo[1,5-*a*]pyridin-4-yl)pyridin-2-yl)-3,9-diazaspiro[5.5]u ndecane-3-carbonate

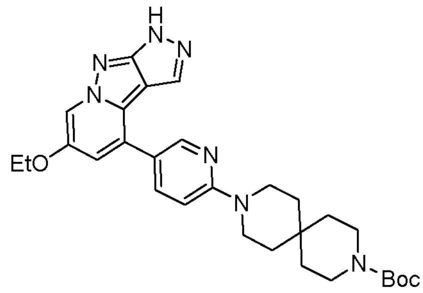

6-ethoxy-4-(6-fluoropyridin-3-yl)-1*H*-pyrazolo[3',4':3,4]pyrazolo[1,5-*a*]pyridine (297 mg, 1.0 mmol), *tert*-butyl 3,9-diazaspiro[5.5]undecane-3-carboxylate (254 mg, 1.0 mmol), diisopropylethylamine (390 mg, 3.0 mmol), and DMSO (3 mL) were sequentially added to a reaction flask. The reaction liquid was heated to 100 °C and reacted for 16 h. The reaction was quenched by adding water and stirring. The reaction liquid was extracted with dichloromethane, dried over anhydrous sodium sulfate, concentrated under reduced pressure, and separated by column chromatography to give a product (540 mg), m/z = 532 $[M+1]^+$.

Step B: 4-(6-(3,9-diazaspiro[5.5]undecan-3-yl)pyridin-3-yl)-6-ethoxy-1*H*-pyrazolo[3',4':3,4]pyrazolo[1,5-*a*] pyridine

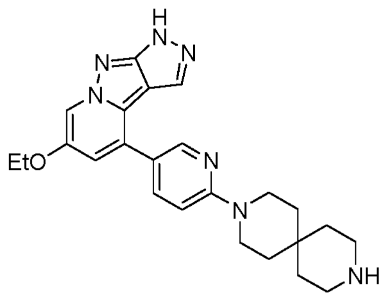

Methanol (5 mL), *tert*-butyl 9-(5-(6-ethoxy-1*H*-pyrazolo[3',4':3,4]pyrazolo[1,5-*a*]pyridin-4-yl)pyridin-2-yl)-3,9-diazaspiro[5.5]u ndecane-3-carbonate (540 mg, 1.0 mmol), and a 6N solution of HCl in 1,4-dioxane (4.0 mL, 24.0 mmol) were sequentially added to a reaction flask. The mixture was stirred at room temperature for 30 min. Water (3 mL) was added. The mixture was adjusted to pH 10 with 1 N NaOH and extracted twice with dichloromethane (15 mL). The extract was washed with saturated brine (10 mL), dried over anhydrous sodium sulfate, and concentrated to give a product (355 mg), m/z = 432 $[M+1]^+$.

Step C: *N*-(9-(5-(6-ethoxy-1*H*-pyrazolo[3',4':3,4]pyrazolo[1,5-*a*]pyridin-4-yl)pyridin-2-yl)-3,9-diazaspiro[5. 5]undecan-3-yl)-2-chloro-6-fluorobenzamide

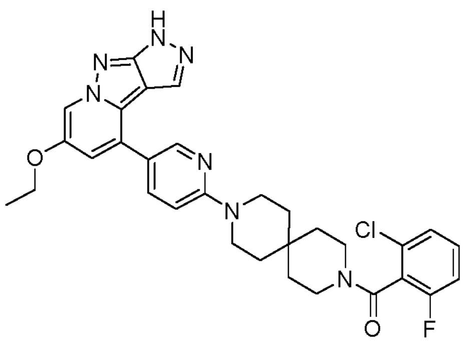

4-(6-(3,9-diazaspiro[5.5]undecan-3-yl)pyridin-3-yl)-6-ethoxy-1*H*-pyrazolo[3',4':3,4]pyrazolo[1,5-*a*] pyridine

(130 mg, 0.3 mmol), DMF (2 mL), and diisopropylethylamine (117 mg, 0.9 mmol) were sequentially added to a reaction flask. The mixture was cooled to 5 °C under an ice bath, and then a solution of 2-chloro-6-fluorobenzoyl chloride (58 mg, 0.3 mmol) in DMF (0.2 mL) was added dropwise. The resulting mixture was stirred at room temperature for 16 h. The reaction was quenched by adding water and stirring, extracted with dichloromethane, dried over anhydrous sodium sulfate, concentrated under reduced pressure, and separated by column chromatography to give a product (62.9 mg), m/z = 588 $[M+1]^+$, $^1$H-NMR (400 MHz, DMSO-$d_6$) δ 12.65 (brs, 1H), 8.54 (s, 1H), 8.50 (s, 1H), 7.98 (d, *J* = 8.8 Hz, 1H), 7.57 (s, 1H), 7.48 (d, *J* = 8.8 Hz, 1H), 7.48 (d, *J* = 8.8 Hz, 1H), 7.33-7.36 (m, 1H), 7.25 (s, 1H), 7.13 (d, *J* = 8.8Hz, 1H), 4.14-4.20 (m, 2H), 3.59-3.76 (m, 6H), 3.15-3.29 (m, 2H), 1.46-1.64 (m, 8H), 1.37-1.41 (m, 3H).

**Example 95: Preparation of compound 217**

6-ethoxy-4-(6-(9-((6-methoxypyridin-3-yl)methylene)-3,9-diazaspiro[5.5]undecan-3-yl)pyridin-3-yl )-1*H*-pyrazolo[3',4':3,4]pyrazolo[1,5-*a*]pyridine

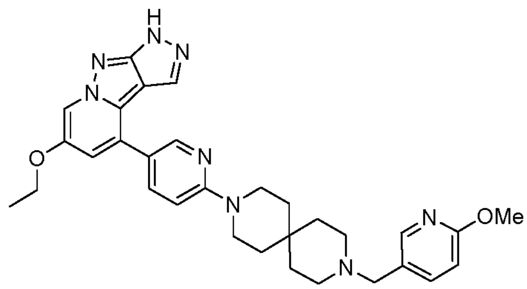

4-(6-(3,9-diazaspiro[5.5]undecan-3-yl)pyridin-3-yl)-6-ethoxy-1*H*-pyrazolo[3',4':3,4]pyrazolo[1,5-*a*] pyridine (43 mg, 0.1 mmol), DMF (1 mL), potassium carbonate (35 mg, 0.3 mmol), and 5-(chloromethyl)-2-methoxypyridine (16 mg, 0.1 mmol) were sequentially added to a reaction flask.

The mixture was stirred at room temperature for 16 h. The reaction was quenched by adding water and stirring. The reaction liquid was extracted with dichloromethane, dried over anhydrous sodium sulfate, concentrated under reduced pressure, and separated by column chromatography to give a product (12.0 mg), m/z = 553 $[M+1]^+$, $^1$H-NMR (400 MHz, DMSO-$d_6$) δ 12.64 (brs, 1H), 8.56 (s, 1H), 8.50 (s, 1H), 8.04 (s, 1H), 8.01 (d, *J* = 8.8 Hz, 1H), 7.63 (d, *J* = 8.8 Hz, 1H), 7.57 (s, 1H), 7.24 (s, 1H), 7.02 (d, *J* = 8.8 Hz, 1H), 6.79 (d, *J* = 8.8 Hz, 1H), 4.14-4.19 (m, 2H), 3.82 (s, 3H), 3.58-3.65 (m, 4H), 3.42 (s, 2H), 3.28-3.41 (m, 4H), 1.41-1.59 (m, 8H), 1.37-1.41 (m, 3H).

**Example 96: Preparation of compound 218**

Step A: *tert*-butyl 2-(5-(6-ethoxy-1*H*-pyrazolo[3',4':3,4]pyrazolo[1,5-*a*]pyridin-4-yl)pyridin-2-yl)-2,9-diazaspiro[5.5]u ndecane-9-carbonate

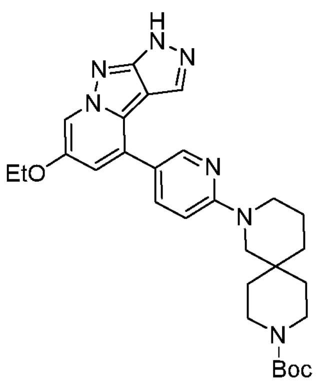

6-ethoxy-4-(6-fluoropyridin-3-yl)-1*H*-pyrazolo[3',4':3,4]pyrazolo[1,5-*a*]pyridine (297 mg, 1.0 mmol), *tert*-butyl

2,9-diazaspiro[5.5]undecane-9-carboxylate (254 mg, 1.0 mmol), diisopropylethylamine (390 mg, 3.0 mmol), and DMSO (3 mL) were sequentially added to a reaction flask. The reaction liquid was heated to 100 °C and reacted for 16 h. The reaction liquid was cooled, and then water (20 mL) was added. The mixture was stirred for 30 min. The reaction was quenched by adding water and stirring. The reaction liquid was extracted with dichloromethane, dried over anhydrous sodium sulfate, concentrated under reduced pressure, and separated by column chromatography to give a product (550 mg), m/z = 532 $[M+1]^+$.

Step B: 4-(6-(2,9-diazaspiro[5.5]undecan-2-yl)pyridin-3-yl)-6-ethoxy-1*H*-pyrazolo[3',4':3,4]pyrazolo[1,5-*a*] pyridine

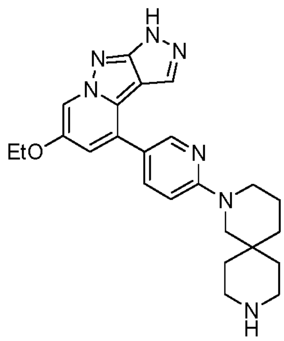

Methanol (5 mL), *tert*-butyl 2-(5-(6-ethoxy-1*H*-pyrazolo[3',4':3,4]pyrazolo[1,5-*a*]pyridin-4-yl)pyridin-2-yl)-2,9-diazaspiro[5.5]u ndecane-9-carbonate (550 mg, 1.0 mmol) and a 6 N solution of HCl in 1,4-dioxane (4.0 mL, 24.0 mmol) were sequentially added to a reaction flask. The mixture was stirred at room temperature for 30 min. Water (3 mL) was added. The mixture was adjusted to pH 10 with 1 N NaOH and extracted twice with dichloromethane (30 mL). The extract was washed with saturated brine (10 mL), dried over anhydrous sodium sulfate, and concentrated to give a product (355 mg), m/z = 432 $[M+1]^+$.

Step C: *N*-(2-(5-(6-ethoxy-1*H*-pyrazolo[3',4':3,4]pyrazolo[1,5-*a*]pyridin-4-yl)pyridin-2-yl)-2,9-diazaspiro[5. 5]undecan-9-yl)-2-chloro-6-fluorobenzamide

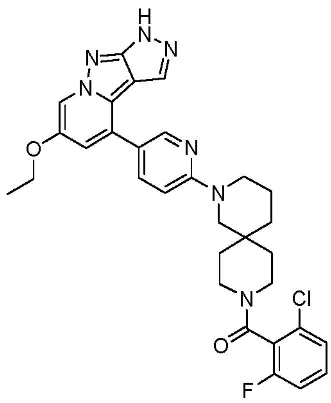

4-(6-(2,9-diazaspiro[5.5]undecan-2-yl)pyridin-3-yl)-6-ethoxy-1*H*-pyrazolo[3',4':3,4]pyrazolo[1,5-*a*] pyridine (130 mg, 0.3 mmol), DMF (2 mL), and diisopropylethylamine (119 mg, 0.9 mmol) were sequentially added to a reaction flask. The mixture was cooled to 5 °C under an ice bath, and then a solution of 2-chloro 6-fluorobenzoyl chloride (58 mg, 0.3 mmol) in DMF (0.2 mL) was added dropwise. The resulting mixture was reacted at room temperature for 2 h. The reaction was quenched by adding water and stirring. The reaction liquid was extracted with dichloromethane, dried over anhydrous sodium sulfate, concentrated under reduced pressure, and separated by column chromatography to give a product (62.9 mg), m/z = 588 $[M+1]^+$, $^1$H-NMR (400 MHz, DMSO-$d_6$) $\delta$ 12.65 (brs, 1H), 8.54 (s, 1H), 8.50 (s, 1H), 7.98-8.01 (m, 1H), 7.57 (s, 1H), 7.33-7.50 (m, 3H), 7.25 (s, 1H), 7.11-7.15 (m, 3H), 4.14-4.20 (m, 2H), 3.75-3.90 (m, 1H), 3.59-3.73 (m, 5H), 3.25-3.32 (m, 1H), 3.10-3.20 (m, 1H), 1.30-1.68 (m, 11H).

**Example 97: Preparation of compound 219**

6-ethoxy-4-(6-(9-((6-methoxypyridin-3-yl)methylene)-2,9-diazaspiro[5.5]undecan-2-yl)pyridin-3-yl )-1*H*-pyrazolo[3',4':3,4]pyrazolo[1,5-*a*]pyridine

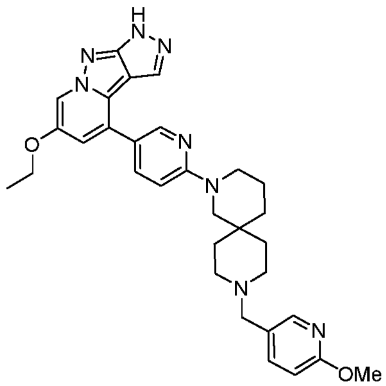

1,2-dichloroethane (2 mL), methanol (1 mL), 4-(6-(2,9-diazaspiro[5.5]undecan-2-yl)pyridin-3-yl)-6-ethoxy-1*H*-pyrazolo[3',4':3,4]pyrazolo[1,5-*a*] pyridine (65 mg, 0.15 mmol), 6-methoxy-3-pyridinecarboxaldehyde (24 mg, 0.17 mmol), and acetic acid (2 drops) were sequentially added to a reaction flask. The mixture was heated to 45 °C and reacted for 2 h, and then sodium triacetoxyborohydride (96 mg, 0.45 mmol) was added. The mixture was successively stirred at 45 °C for 16 h. The reaction was quenched by adding water and stirring. The reaction liquid was extracted with dichloromethane, dried over anhydrous sodium sulfate, concentrated under reduced pressure, and separated by column chromatography to give a product (15.8 mg), m/z = 553 $[M+1]^+$, $^1$H-NMR (400 MHz, DMSO-$d_6$) δ 12.64 (brs, 1H), 8.55 (s, 1H), 8.50 (s, 1H), 8.04-8.18 (m, 1H), 8.00 (d, *J*= 8.8 Hz, 1H), 7.65-7.73 (m, 1H), 7.58 (s, 1H), 7.25 (s, 1H), 7.03-7.15 (m, 1H), 6.75-6.90 (m, 1H), 4.14-4.20 (m, 2H), 3.83 (s, 3H), 3.30-3.65 (m, 8H), 3.17 (s, 1H), 1.91 (s, 1H), 1.38-1.68 (m, 11H).

**Example 98: Preparation of compound 220**

Step A: *tert*-butyl 6-(5-(6-ethoxy-1*H*-pyrazolo[3',4':3,4]pyrazolo[1,5-*a*]pyridin-4-yl)pyridin-2-yl)-2,6-diazaspiro[3.4]o ctane-2-carbonate

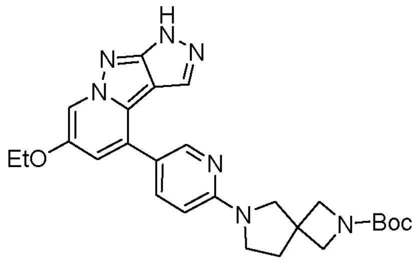

6-ethoxy-4-(6-fluoropyridin-3-yl)-1*H*-pyrazolo[3',4':3,4]pyrazolo[1,5-*a*]pyridine (297 mg, 1.0 mmol), *tert*-butyl 2,6-diazaspiro[3.4]octane-2-carboxylate (212 mg, 1.0 mmol), diisopropylethylamine (390 mg, 3.0 mmol), and DMSO (4 mL) were sequentially added to a reaction flask. The reaction liquid was heated to 100 °C and reacted for 16 h. The reaction was quenched by adding water and stirring. The reaction liquid was extracted with dichloromethane, dried over anhydrous sodium sulfate, concentrated under reduced pressure, and separated by column chromatography to give a product (520 mg), m/z = 490 $[M+1]^+$.

Step B: 4-(6-(2,6-diazaspiro[3.4]octan-6-yl)pyridin-3-yl)-6-ethoxy-1*H*-pyrazolo[3',4':3,4]pyrazolo[1,5-*a*]py ridine

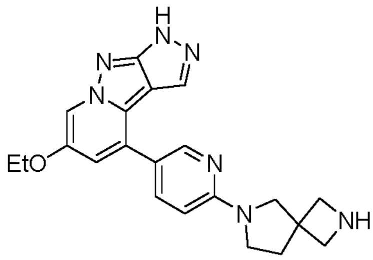

Methanol (4 mL), *tert*-butyl 6-(5-(6-ethoxy-1*H*-pyrazolo[3',4':3,4]pyrazolo[1,5-*a*]pyridin-4-yl)pyridin-2-yl)-2,6-diazaspiro[3.4]o ctane-2-carbonate (520 mg, 1.0 mmol) and a 6 N solution of HCl in 1,4-dioxane (2.0 mL, 12.0 mmol) were sequentially added to a reaction flask. The mixture was stirred at room temperature for 30 min. Water (10 mL) was added. The mixture was adjusted to pH 10 with 1 N NaOH and extracted twice with dichloromethane (40 mL). The extract was dried over anhydrous sodium sulfate and concentrated to give a product (310 mg), m/z = 390 $[M+1]^+$.

Step C: *N*-(6-(5-(6-ethoxy-1*H*-pyrazolo[3',4':3,4]pyrazolo[1,5-*a*]pyridin-4-yl)pyridin-2-yl)-2,6-diazaspiro[3. 4]octan-2-yl)-2-chloro-6-fluorobenzamide

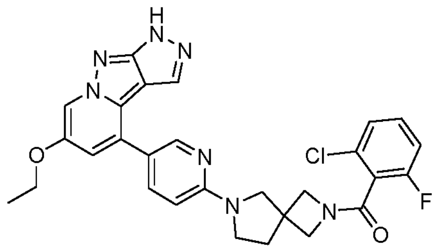

4-(6-(2,6-diazaspiro[3.4]octan-6-yl)pyridin-3-yl)-6-ethoxy-1*H*-pyrazolo[3',4':3,4]pyrazolo[1,5-*a*]py ridine (78 mg, 0.2 mmol), DMF (2 mL), and diisopropylethylamine (78 mg, 0.6 mmol) were sequentially added to a reaction flask. The mixture was cooled to 5 °C under an ice bath, and then a solution of 2-chloro-6-fluorobenzoyl chloride (40 mg, 0.2 mmol) in DMF (0.2 mL) was added dropwise. The resulting mixture was stirred at room temperature for 16 h. The reaction was quenched by adding water and stirring. The reaction liquid was extracted with dichloromethane, dried over anhydrous sodium sulfate, concentrated under reduced pressure, and separated by column chromatography to give a product (29 mg), m/z = 546 $[M+1]^+$, $^1$H-NMR (400 MHz, DMSO-$d_6$) $\delta$ 12.64 (brs, 1H), 8.55 (s, 1H), 8.50 (s, 1H), 8.01 (d, *J*= 8.8 Hz, 1H), 7.50-7.56 (m, 2H), 7.44 (m, 1H), 7.35-7.39 (m, 1H), 7.22 (s, 1H), 6.68 (d, *J* = 8.8Hz, 1H), 4.11-4.19 (m, 4H), 3.85-3.94 (m, 2H), 3.65-3.78 (m, 2H), 3.45-3.60 (m, 2H), 2.16-2.33 (m, 2H), 1.33-1.45 (m, 3H).

**Example 99: Preparation of compound 221**

6-ethoxy-4-(6-(2-((6-methoxypyridin-3-yl)methylene)-2,6-diazaspiro[3.4]nonan-6-yl)pyridin-3-yl)-1*H*-pyrazolo[3',4':3,4]pyrazolo[1,5-*a*]pyridine

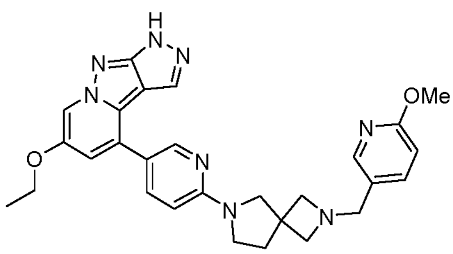

1,2-dichloroethane (2 mL), methanol (1 mL), 4-(6-(2,6-diazaspiro[3.4]octan-6-yl)pyridin-3-yl)-6-ethoxy-1*H*-pyrazolo[3',4':3,4]pyrazolo[1,5-a]py ridine (60 mg, 0.15 mmol), 6-methoxy-3-pyridinecarboxaldehyde (62 mg, 0.45 mmol),

and acetic acid (2 drops) were sequentially added to a reaction flask. The mixture was reacted at 45 °C for 2 h, and then sodium triacetoxyborohydride (160 mg, 0.75 mmol) was added. The mixture was successively stirred at 45 °C for 16 h. The reaction was quenched by adding water and stirring. The reaction liquid was extracted with dichloromethane, dried over anhydrous sodium sulfate, concentrated under reduced pressure, and separated by column chromatography to give a product (29 mg), m/z = 511 $[M+1]^+$, $^1$H-NMR (400 MHz, DMSO-$d_6$) δ 12.65 (brs, 1H), 8.54 (s, 1H), 8.50 (s, 1H), 8.08 (s, 1H), 8.00 (d, *J* = 8.8 Hz, 1H), 7.64 (d, *J* = 8.8 Hz, 1H), 7.57 (s, 1H), 7.22 (s, 1H), 6.78 (d, *J* = 8.8Hz, 1H), 6.66 (d, *J* = 8.8Hz, 1H), 4.14-4.19 (m, 2H), 3.83 (s, 3H), 3.40-3.68 (m, 6H), 3.15-3.30 (m, 3H), 2.15-2.19 (m, 2H), 1.91 (s, 1H), 1.37-1.44 (m, 3H).

**Example 100: Preparation of compound 227**

4-(6-(6-((6-methoxypyridin-3-yl)methylene)-3,6-diazabicyclo[3.1.1]heptan-3-yl)pyridin-3-yl)-6-(pr op-2-yn-1-yloxy)-1*H*-pyrazolo[3',4':3,4]pyrazolo[1,5-*a*]pyridine

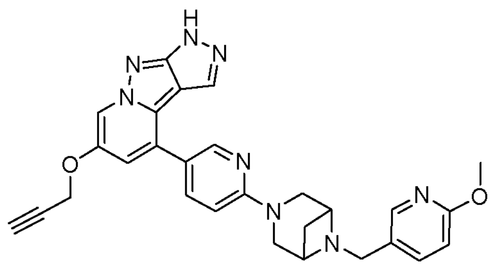

4-(6-(6-((6-methoxypyridin-3-yl)methylene)-3,6-diazabicyclo[3.1.1]heptan-3-yl)pyridin-3-yl)-1*H*-p yrazolo[3',4':3,4]pyrazolo[1,5-*a*]pyridin-6-ol (46.8 mg, 0.1 mmol), propargyl chloride (8.2 mg, 0.11 mmol), $Cs_2CO_3$ (48 mg, 0.15 mmol), and DMF (2 mL) were added to a 10 mL reaction flask. The mixture was heated to 50 °C for 5 h. The reaction was quenched by adding water. The reaction liquid was extracted with dichloromethane, dried over anhydrous sodium sulfate, concentrated under reduced pressure, and separated by column chromatography to give a product (9.4 mg), m/z = 507 $[M+1]^+$, $^1$H NMR (400 MHz, DMSO-$d_6$): δ 12.69 (s, 1H), 8.60-8.66 (m, 2H), 8.1-8.13 (m, 2H), 7.60-7.78 (m, 2H), 7.34 (s, 1H), 6.77-6.94 (m, 2H), 5.00 (s, 2H), 3.70-3.86 (m, 7H), 3.30-3.32 (m, 7H).

**Example 101: Preparation of compound 228**

Methyl

2-((4-(6-(6-((6-methoxypyridin-3-yl)methylene)-3,6-diazabicyclo[3.1.1]heptan-3-yl)pyridin-3-yl)-1 *H*-pyrazolo[3',4':3,4]pyrazolo[1,5-*a*]pyridin-6-yl)oxy)acetate

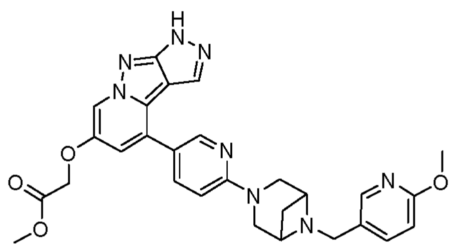

4-(6-(6-((6-methoxypyridin-3-yl)methylene)-3,6-diazabicyclo[3.1.1]heptan-3-yl)pyridin-3-yl)-1*H*-p yrazolo[3',4':3,4]pyrazolo[1,5-*a*]pyridin-6-ol (70 mg, 0.15 mmol), methyl chloroacetate (19 mg, 0.18 mmol), $Cs_2CO_3$ (73 mg, 0.225 mmol), and DMF (5 mL) were added to a 10 mL reaction flask. The mixture was heated to 50 °C for 3 h. The reaction was quenched by adding water. The reaction liquid was extracted with dichloromethane, dried over anhydrous sodium sulfate, concentrated under reduced pressure, and separated by column chromatography to give a product (29.6 mg), m/z = 541 $[M+1]^+$, $^1$H NMR (400 MHz, DMSO-$d_6$): δ 12.68 (s, 1H), 8.60-8.66 (m, 2H), 8.10-8.16 (m, 2H), 7.64-7.72

(m, 2H), 7.35 (s, 1H), 6.91-6.93 (m, 1H), 6.76-6.79 (m, 1H), 5.0 (s, 2H), 3.80-3.97 (m, 4H), 3.62-3.80 (m, 6H), 3.42-3.62 (m, 4H), 3.31-3.33 (m, 1H), 2.51-2.67 (m, 1H).

**Example 102: Preparation of compound 229**

Step A: ethyl 1-(5-(6-ethoxy-1*H*-pyrazolo[3',4':3,4]pyrazolo[1,5-*a*]pyridin-4-yl)pyridin-2-yl)-4-(((6-methoxypyri din-3-yl)methyl)amino)piperidine-4-carboxylate

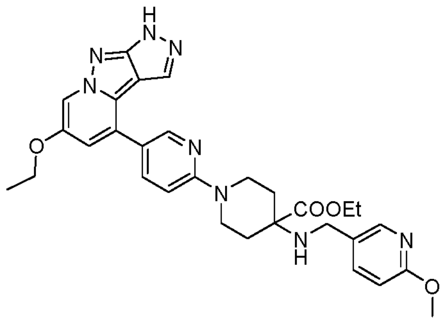

Ethyl

4-amino-1-(5-(6-ethoxy-1*H*-pyrazolo[3',4':3,4]pyrazolo[1,5-*a*]pyridin-4-yl)pyridin-2-yl)piperidine-4-carboxylate (449 mg, 1.0 mmol), 6-methoxynicotinaldehyde (164 mg, 1.2 mmol), $Et_3N$ (303 mg, 3.0 mmol), and *n*-propanol (5 mL) were added to a reaction flask. The mixture was reacted at 25 °C for 12 h. The reaction was quenched by adding water and stirring. The reaction liquid was extracted with dichloromethane, dried over anhydrous sodium sulfate, concentrated under reduced pressure, and separated by column chromatography to give a product (268 mg), m/z=571 $[M+1]^+$.

Step B: (1-(5-(6-ethoxy-1*H*-pyrazolo[3',4':3,4]pyrazolo[1,5-*a*]pyridin-4-yl)pyridin-2-yl)-4-(((6-methoxypyri din-3-yl)methyl)amino)piperidin-4-yl)methanol

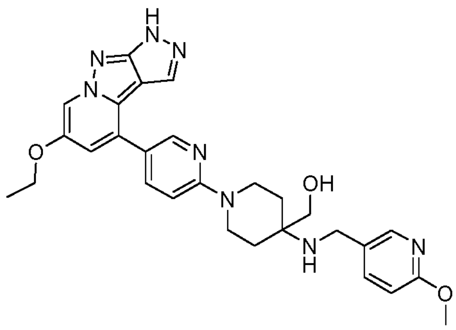

Ethyl

1-(5-(6-ethoxy-1*H*-pyrazolo[3',4':3,4]pyrazolo[1,5-*a*]pyridin-4-yl)pyridin-2-yl)-4-(((6-methoxypyri din-3-yl)methyl)amino)piperidine-4-carboxylate (268 mg, 0.47 mmol) and 5 mL of THF were added to a reaction flask, and $LiAlH_4$ (19.6 mg, 0.51 mmol) was added at 0 °C. The mixture was reacted for 1 h. The reaction was quenched by adding water and stirring. The reaction liquid was extracted with dichloromethane, dried over anhydrous sodium sulfate, concentrated under reduced pressure, and separated by column chromatography to give a product (101 mg), m/z= 529 $[M+1]^+$, $^1H$ NMR (400 MHz, DMSO-$d_6$) $\delta$ 12.66 (s, 1H), 8.58 (s, 1H), 8.52 (d, *J*= 2.0 Hz, 1H), 8.15 (s, 1H), 8.03 (d, *J* = 9.1 Hz, 1H), 7.76 (d, *J* = 8.4 Hz, 1H), 7.60 (m, 2H), 7.26 (d, *J* = 2.1 Hz, 1H), 7.08 (s, 1H), 6.81 (s, 1H), 4.66 (s, 1H), 4.18 (m, 2H), 3.99 (s, 2H), 3.83 (s, 3H), 3.64 (s, 2H), 2.89 (m, 3H), 1.65 (s, 2H), 1.53 (s, 2H), 1.40 (t, *J*=6.9 Hz, 3H), 0.90 (d, *J*=6.7 Hz, 1H).

**Example 103: Preparation of compound 232**

Step A: 4-(6-(6-((6-methoxypyridin-3-yl)methylene)-3,6-diazabicyclo[3.1.1]heptan-3-yl)pyridin-3-yl)-1*H*-p yrazolo[3',4':3,4]pyrazolo[1,5-*a*]pyridin-6-yl trifluoromethanesulfonate

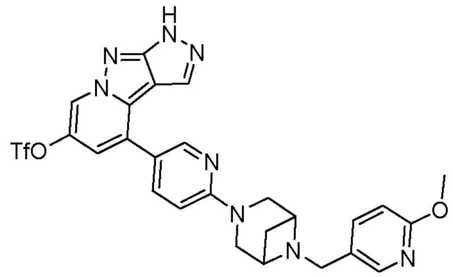

4-(6-(6-((6-methoxypyridin-3-yl)methyl)-3,6-diazabicyclo[3.1.1]heptan-3-yl)pyridin-3-yl)-1*H*-pyra zolo[3',4':3,4]pyrazolo[1,5-*a*]pyridin-6-ol (0.936 g, 2 mmol), DIEA (0.516 g, 4 mmol), and 10 mL of DMSO were added to a reaction flask. 1,1,1-trifluoro-N-phenyl-N-((trifluoromethyl)sulfonyl) methanesulfonamide (0.714 g, 2 mmol) was added under an ice bath. The mixture was reacted at room temperature for 2 h. The reaction was quenched by adding water and stirring. The reaction liquid was extracted with dichloromethane, dried over anhydrous sodium sulfate, concentrated under reduced pressure, and separated by column chromatography to give a product (1.0 g), m/z = 601 $[M+1]^+$.

Step B: 4-(6-(6-((6-methoxypyridin-3-yl)methyl)-3,6-diazabicyclo[3.1.1]heptan-3-yl)pyridin-3-yl)-6-(4,4,5, 5-tetramethyl-1,3,2-dioxaborolan-2-yl)-1*H*-pyrazolo[3',4':3,4]pyrazolo[1,5-*a*]pyridine

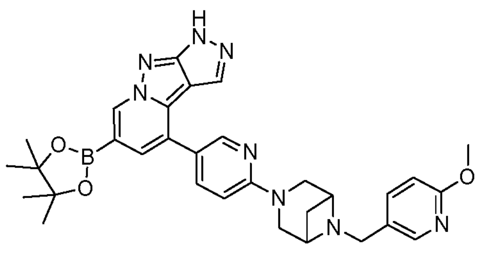

4-(6-(6-((6-methoxypyridin-3-yl)methyl)-3,6-diazabicyclo[3.1.1]heptan-3-yl)pyridin-3-yl)-1*H*-pyra zolo[3',4':3,4]pyrazolo[1,5-*a*]pyridine-6-trifluoromethylsulfonate (300 mg, 0.5 mmol), bis(pinacolato)diboron (381 mg, 1.5 mmol), $Pd(dppf)Cl_2$ (73 mg, 0.1 mmol), potassium acetate (245 mg, 2.5 mmol), and 10 mL of 1,4-dioxane were added to a reaction flask. The mixture was reacted at 90 °C for 4 h. The reaction was quenched by adding water and stirring. The reaction liquid was extracted with dichloromethane, dried over anhydrous sodium sulfate, concentrated under reduced pressure, and separated by column chromatography to give a product (178 mg), m/z = 579 $[M+H]^+$.

Step C: 4-(6-(6-((6-methoxypyridin-3-yl)methyl)-3,6-diazabicyclo[3.1.1]heptan-3-yl)pyridin-3-yl)-1*H*-pyra zolo[3',4':3,4]pyrazolo[1,5-*a*]pyridin-6-yl)boronic acid

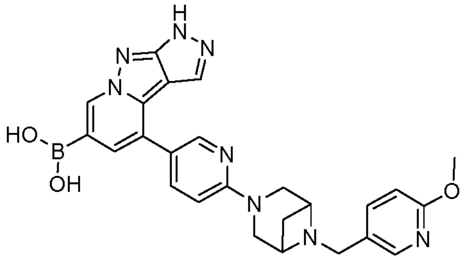

(4-(6-(6-((6-methoxypyridin-3-yl)methyl)-3,6-diazabicyclo[3.1.1]heptan-3-yl)pyridin-3-yl)-6-(4,4,5 ,5-tetramethyl-1,3,2-dioxaborolan-2-yl)-1*H*-pyrazolo[3',4':3,4]pyrazolo[1,5-*a*]pyridine (180 mg, 0.31 mmol), $NaIO_4$ (200 mg, 0.93 mmol), and THF (8 mL)/water (2 mL) were added to a reaction flask. The mixture was reacted at 25 °C for 0.5 h. 1 N HCl (3 mL) was added to the reaction liquid described above, which was then stirred overnight. The reaction liquid was poured into 100 mL of water, and the resulting mixture was extracted with ethyl acetate, dried over anhydrous sodium sulfate, and concentrated under reduced pressure to give a product (55 mg), m/z = 496 $[M+1]^+$.

Step D: 6-(azetidin-1-yl)-4-(6-(6-((6-methoxypyridin-3-yl)methyl)-3,6-diazabicyclo[3.1.1]heptan-3-yl)pyrid in-3-yl)-1*H*-pyrazolo[3',4':3,4]pyrazolo[1,5-*a*]pyridine

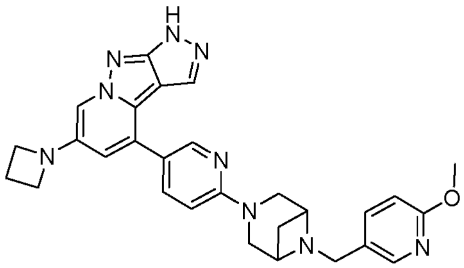

4-(6-(6-((6-methoxypyridin-3-yl)methyl)-3,6-diazabicyclo[3.1.1]heptan-3-yl)pyridin-3-yl)-1*H*-pyrazolo[3',4':3,4]pyrazolo[1,5-*a*]pyridin-6-yl)boronic acid (50 mg, 0.1 mmol), azetidine (114 mg, 2 mmol), copper(II) acetate (37 mg, 0.2 mmol), pyridine (45 mg, 0.5 mmol), 4A molecular sieves (2 g), and 5 mL of DMF were added to a reaction flask. The mixture was reacted at 50 °C for 12 h. The reaction liquid was filtered, and the filtrate was poured into 20 mL of water, extracted with ethyl acetate, dried over anhydrous sodium sulfate, and concentrated under reduced pressure to give a product (20 mg), m/z = 508 $[M+1]^+$.

**Example 104: Preparation of compound 233**

Synthesis of *N*-((3*R*,4*S*)-1-(5-(6-ethoxy-1*H*-pyrazolo[3',4':3,4]pyrazolo[1,5-*a*]pyridin-4-yl)pyridin-2-yl)-piperidi ne-3-ol

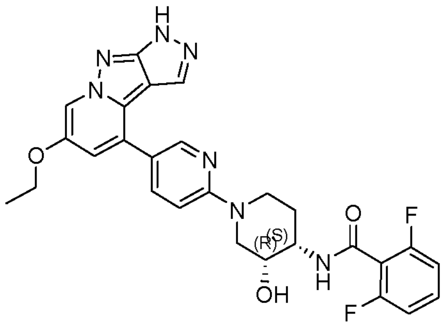

(3*S*,4,*S*)-4-amino-1-(5-(6-ethoxy-1*H*-pyrazolo[3',4':3,4]pyrazolo[1,5-*a*]pyridin-4-yl)pyridin-2-yl)pip eridin-3-ol

hydrochloride (100 mg, 0.2 mmol), 2,6-difluorobenzoic acid (32 mg, 0.2 mmol), DIEA (129 mg, 1.0 mmol), HATU (76 mg, 0.2 mmol), and DMF (3 mL) were added to a reaction flask. The mixture was reacted at 25 °C for 1 h. The reaction liquid was poured into 30 mL of water, and the resulting mixture was extracted with EA. The organic phase was dried, concentrated by rotary evaporation to remove the solvent, and purified by column chromatography (DCM:MeOH = 10:1) to give a product (5.0 mg), m/z = 534 $[M+1]^+$. $^1$H NMR (400 MHz, DMSO-$d_6$): δ 12.65 (s, 1H), 8.61 (d, *J* = 8.0 Hz, 1H), 8.59 (s, 1H), 8.57 (s, 1H), 8.02-8.05 (m, 1H), 7.58 (s, 1H), 7.07-7.49 (m, 5H), 5.07 (br, 1H), 4.40-4.44 (m, 1H), 4.14-4.22 (m, 3H), 3.92-3.95 (m, 1H), 3.50-3.51 (m, 1H), 3.16-3.22 (m, 1H), 2.96-3.01 (m, 1H), 2.01-2.11 (m, 1H), 1.37-1.48 (m, 4H).

**Example 105: Preparation of compound 234**

Step A: *tert*-butyl (1-(5-bromopyridin-2-yl)-4-((dimethylamino)methyl)piperidin-4-yl)carbamate

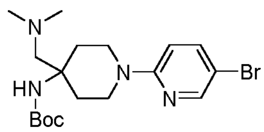

*Tert*-butyl (1-(5-bromopyridin-2-yl)-4-formylpiperidin-4-yl)carbamate (200 mg, 0.52 mmol), 1,2-dichloromethane (2 mL), dimethylamine (0.78 mL, 1.56 mmol), and sodium borohydride acetate (220 mg, 1.04 mmol) were added to a 50 mL reaction flask. The mixture was stirred at room temperature for 16 h. The reaction was quenched by adding water. The reaction liquid was concentrated and separated by column chromatography to give a product (177 mg), m/z = 413/415 $[M+1]^+$.

Step B: 1-(5-bromopyridin-2-yl)-4-((dimethylamino)methyl)piperidin-4-amine hydrochloride

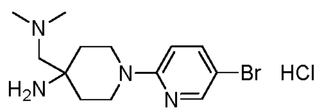

*Tert*-butyl (1-(5-bromopyridin-2-yl)-4-((dimethylamino)methyl)piperidin-4-yl)carbamate (177 mg, 0.43 mmol), methanolic hydrochloric acid (4 M, 2 mL) was added to a reaction flask. The mixture was reacted at 25 °C for 5 h. The reaction liquid was directly concentrated by rotary evaporation to give a product (250 mg), m/z = 313/315$[M+1]^+$.

Step C: *N*-(1-(1-bromopyridin-2-yl)-4-((dimethylamino)methyl)piperidin-4-yl)-2,6-difluorobenzamide

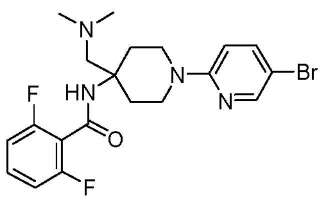

1-(5-bromopyridin-2-yl)-4-((dimethylamino)methyl)piperidin-4-amine hydrochloride (250 mg, 0.8 mmol), 2,6-difluorobenzoic acid (152 mg, 0.96 mmol), HATU (334 mg, 0.88 mmol), DIEA (309mg, 2.4 mmol), and DMF (2 mL) were added to a reaction flask. The mixture was reacted at 25 °C for 15 h. The reaction was quenched by adding water and stirring. The reaction liquid was extracted with dichloromethane. The organic phase was dried over anhydrous sodium sulfate, concentrated under reduced pressure, and separated by column chromatography to give a product (117 mg), m/z = 453/455 $[M+1]^+$.

Step D: *N*-(4-((dimethylamino)methyl)-1-(5-(6-ethoxy-1*H*-pyrazolo[3',4':3,4]pyrazolo[1,5-*a*]pyridin-4-yl)py ridin-2-yl)piperidin-4-yl)-2,6-difluorobenzamide

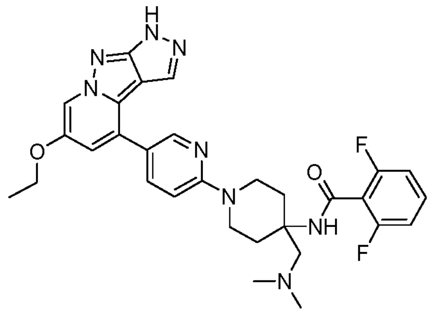

N-(1-(1-bromopyridin-2-yl)-4-((dimethylamino)methyl)piperidin-4-yl)-2,6-difluorobenzamide (117 mg, 0.35 mmol), 6-ethoxy-4-(4,4,5,5-tetramethyl-1,3,2-dioxaboran-2-yl)-1*H*-pyrazolo[3',4':3,4]pyrazolo[1,5-*a*]pyridi ne (161 mg, 0.35 mmol), cesium carbonate (326 mg, 1.0 mmol), water (1 mL), 1,4-dioxane (5 mL), and Pd(dppf)$Cl_2$ (25 mg, 0.035 mmol) were sequentially added to a reaction flask. The mixture was heated to 90 °C for 16 h. The reaction liquid was poured into water, and the resulting mixture was extracted with dichloromethane and separated by column chromatography to give a product (50 mg), m/z=575 $[M+1]^+$, $^1H$ NMR (400 MHz, DMSO-$d_6$): δ 12.64 (s, 1H), 8.61-8.56 (m, 1H), 8.51 (m, 1H), 8.44 (s, 1H), 8.04 (m, 1H), 7.59 (s, 1H), 7.49 (m, 1H), 7.26 (m, 1H), 7.21-7.06 (m, 3H), 4.22 (m, 2H), 4.18 (m, 2H), 3.17 (m, 2H), 2.67 (s, 2H), 2.34 (m, 2H), 2.29 (s, 6H), 1.57 (m, 2H), 1.40 (m, 3H).

**Example 106: Preparation of compound 237**

(4-(6-(6-((6-methoxypyridin-3-yl)methyl)-3,6-diazabicyclo[3.1.1]heptan-3-yl)pyridin-3-yl)-1*H*-pyr azolo[3',4':3,4]pyrazolo[1,5-*a*]pyridin-6-yl)dimethylphosphine oxide

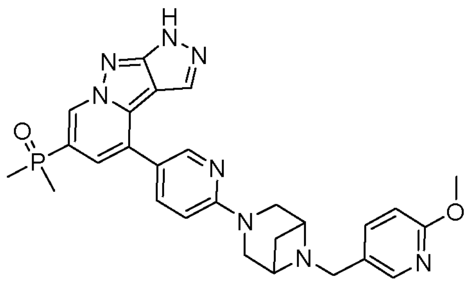

4-(6-(6-(((6-methoxypyridin-3-yl)methyl)-3,6-diazabicyclo[3.1.1]heptan-3-yl)pyridin-3-yl)-1*H*-pyr azolo[3',4':3,4]pyrazolo[1,5-*a*]pyridin-6-yl trifluoromethanesulfonate (30 mg, 0.05 mmol), phosphorus dioxide (9.7 mg, 0.125 mmol), palladium(II) acetate (0.56 mg, 0.0025 mmol), potassium phosphate (11 mg, 0.055 mmol), and DMF (1 mL), XantPhos (29 mg, 0.05 mmol) were sequentially added to a reaction flask. The mixture was heated to 150 °C and reacted for 2 h under nitrogen atmosphere. The reaction liquid was poured into water, and the resulting mixture was extracted with dichloromethane and separated by column chromatography to give a product (19 mg), m/z=529 $[M+1]^+$. $^1$HNMR (400MHz, DMSO-$d_6$): 12.90 (s, 1H), 9.02 (m, 1H), 8.70 (m, 1H), 8.20-8.08 (m, 2H), 7.83-7.65 (m, 3H), 6.95 (m, 1H), 6.79 (m, 1H), 3.83 (m, 5H), 3.70 (m, 2H), 3.60 (s, 2H), 3.54 (s, 2H), 3.34 (s, 1H), 1.85 (s, 3H), 1.82 (s, 3H), 1.60 (m, 1H).

**Example 107: Preparation of compound 238**

Step A: 4-(6-(6((6-methoxypyridin-3-yl)methylene)-3,6-diazacyclo[3.1.1]heptan-3-yl)-6-hydroxy-1*H*-pyraz olo[3',4':3,4]pyrazolo[1,5-*a*]pyridin-4-yl)pyridine

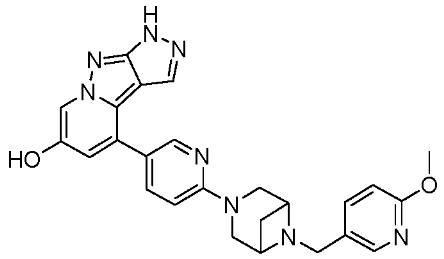

6-((4-methoxy)benzyl)-4-(6-(6((6-methoxypyridin-3-yl)methylene)-3,6-diazacyclo[3.1.1]heptan-3-yl)-6-hydroxy-1*H*-pyrazolo[3',4':3,4]pyrazolo[1,5-*a*]pyridin-4-yl)pyridine (1 g, 1.7 mmol) and DCM (5 mL) were added to a reaction flask, and TFA (5 mL) was added dropwise at 0 °C. The mixture was reacted at room temperature for 4 h. The reaction liquid was directly concentrated by rotary evaporation to remove the solvent to give a product (1.55 g), m/z = 469 $[M+1]^+$.

Step B: 4-(6-(6((6-methoxypyridin-3-yl)methylene)-3,6-diazacyclo[3.1.1]heptan-3-yl)-6-trifluoromethanesu lfonyl-1*H*-pyrazolo[3',4':3,4]pyrazolo[1,5-*a*]pyridin-4-yl)pyridine

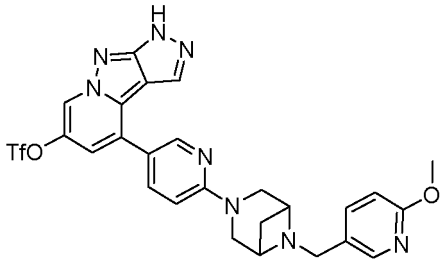

4-(6-(6((6-methoxypyridin-3-yl)methylene)-3,6-diazacyclo[3.1.1]heptan-3-yl)-6-hydroxy-1*H*-pyraz olo[3',4':3,4]pyrazolo[1,5-*a*]pyridin-4-yl)pyridine (1.5 g, 3.2 mmol), *N*-phenyl-bis(trifluoromethanesulfonimide) (1.26 g, 3.5 mmol), DIEA (2 g, 16 mmol), and DMF (10 mL) were sequentially added to a reaction flask. The mixture was reacted at 25 °C for 4 h. After the reaction was completed as monitored by LCMS, the reaction liquid was poured into 30 mL of water, and the resulting mixture was extracted with EA. The organic solvent was dried, concentrated, and purified by silica gel column chromatography (DCM:MeOH = 10:1) to give a product (810 mg), m/z = 601 $[M+1]^+$.

Step C: 4-(6-(6-((6-methoxypyridin-3-yl)methylene)-3,6-diazabicyclo[3.1.1]heptan-3-yl)-6-(4,4,5,5-tetrame thyl-1,3,2-dioxaborolan-2-yl)-1*H*-pyrazolo[3',4':3,4]pyrazolo[1,5-*a*]pyridin-4-yl)pyridine

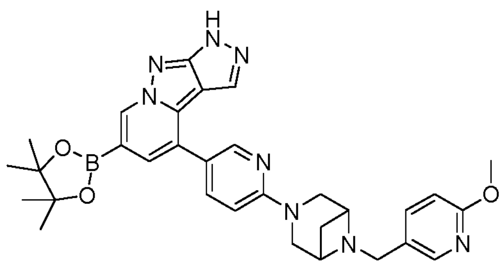

4-(6-(6((6-methoxypyridin-3-yl)methylene)-3,6-diazacyclo[3.1.1]heptan-3-yl)-6-trifluoromethanesu lfonyl-1*H*-pyrazolo[3',4':3,4]pyrazolo[1,5-*a*]pyridin-4-yl)pyridine (810 mg, 0.19 mmol), bis(pinacolato)diboron (377 mg, 1.49 mmol), KOAc (400 mg, 4.05 mmol), $Pd(dppf)Cl_2$ (100 mg, 0.14 mmol), and 1,4-dioxane (10 mL) were sequentially added to a reaction flask. The mixture was purged with $N_2$ 3 times and reacted at 95 °C for 12 h. After the reaction was completed as monitored by LCMS, the reaction liquid was directly purified by silica gel column chromatography (DCM:MeOH = 10:1) to give a product (510 mg), m/z=579$[M+1]^+$.

Step D: (4-(6-(6-((6-methoxypyridin-3-yl)methylene)-3,6-diazabicyclo[3.1.1]heptan-3-yl)-1*H*-pyrazolo[3',4' :3,4]pyrazolo[1,5-*a*]pyridin-4-yl)pyridin-6-yl)boronic acid

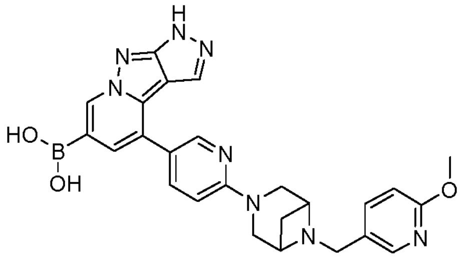

4-(6-(6((6-methoxypyridin-3-yl)methylene)-3,6-diazacyclo[3.1.1]heptan-3-yl)-6-(4,4,5,5-tetramethy 1-1,3,2-dioxaborolan-2-yl)-1*H*-pyrazolo[3',4':3,4]pyrazolo[1,5-*a*]pyridin-4-yl)pyridine (110 mg, 0.19 mmol) and THF (5 mL) were added to a reaction flask and stirred, and sodium periodate (41 mg, 0.19 mmol) was added to the reaction flask. The mixture was stirred for 0.5 h, and an aqueous HCl solution (1 N, 3 mL) was added. The resulting mixture was reacted at 25 °C for 4 h. The reaction was quenched by adding an aqueous sodium thiosulfate solution. The reaction liquid was extracted with dichloromethane, dried over anhydrous sodium sulfate, concentrated, and purified and separated by silica gel column to give a product (38.7 mg), m/z = 497 $[M+1]^+$. $^1$H-NMR (400 MHz, DMSO-$d_6$): δ 12.69 (s, 1H), 9.00-9.20 (m, 1H), 8.63-8.69 (m, 1H), 8.46-8.50 (m, 1H), 8.05-8.11 (m, 2H), 7.59 (s, 1H), 7.28 (m, 1H), 7.13 (m, 1H), 6.53-6.90 (m, 1H), 3.82-3.88 (m, 5H), 3.66-3.72 (m, 4H), 3.50-3.58 (m, 3H), 1.60 (m, 1H).

**Example 108: Preparation of compound 239**

4-(6-(6-((6-methoxypyridin-3-yl)methyl)-3,6-diazabicyclo[3.1.1]heptan-3-yl)pyridin-3-yl)-1*H*-pyra zolo[3',4':3,4]pyrazolo[1,5-*a*]pyridine-6-carboxylic acid

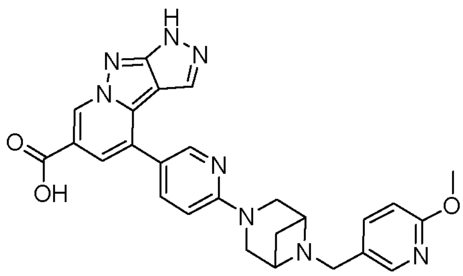

MeOH (3 mL) and an aqueous NaOH solution (5 M, 3 mL), and 4-(6-(6-(((6-methoxypyridin-3-yl)methyl)-3,6-diazabicyclo[3.1.1]heptan-3-yl)pyridin-3-yl)-1*H*-pyr azolo[3',4':3,4]pyrazolo[1,5-*a*]pyridine-6-carbonitrile (15 mg, 0.03 mmol) were sequentially added to a reaction flask. The mixture was reacted at 50 °C for 16 h. After the reaction was completed, an HCl aqueous solution (1 M) was added to adjust the pH to 7. The reaction liquid was concentrated under reduced pressure and separated by column chromatography to give a product (8 mg), m/z=497$[M+1]^+$. $^1$H NMR (400 MHz, DMSO-$d_6$): δ 12.79 (brs, 1H), 9.11 (s, 1H), 8.64 (s, 1H), 8.11 (s, 1H), 8.09 (s, 1H), 7.84 (s, 1H), 7.73-7.69 (m, 2H), 6.92 (d, *J* = 8.4Hz, 1H), 6.77 (d, *J* = 8.4Hz, 1H), 3.86-3.75 (m, 5H), 3.72-3.68 (m, 2H), 3.66-3.51 (m, 4H), 1.60 (d, *J*= 8.4Hz, 1H).

**Example 109: Preparation of compound 240**

Step A: ethyl 2-((4-(6-(6-((6-methoxypyridin-3-yl)methyl)-3,6-diazabicyclo[3.1.1]heptan-3-yl)pyridin-3-yl)-1*H*-p yrazolo[3',4':3,4]pyrazolo[1,5-*a*]pyridin-6-yl)oxy)acetate

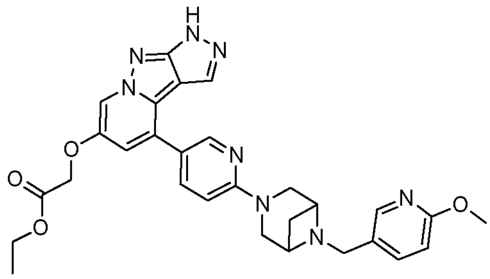

4-(6-(6-((6-methoxypyridin-3-yl)methyl)-3,6-diazabicyclo[3.1.1]heptan-3-yl)pyridin-3-yl)-1*H*-pyra zolo[3',4':3,4]pyrazolo[1,5-*a*]pyridine-6-hydroxy (100 mg, 0.21 mmol), cesium carbonate (138.8 mg, 0.42 mmol), and *N*,*N*-dimethylformamide (3 mL) were added to a reaction flask. The mixture was stirred homogeneously, and then ethyl chloroacetate (39.1 mg, 0.32 mmol) was added. The resulting mixture was reacted at room temperature for 10 h. The reaction was quenched by adding water. The reaction liquid was extracted with ethyl acetate and separated by silica gel column chromatography to give a product (60 mg), m/z = 555.2 $[M+1]^+$.

Step B: 2-((4-(6-(6-((6-methoxypyridin-3-yl)methyl)-3,6-diazabicyclo[3.1.1]heptan-3-yl)pyridin-3-yl)-1*H*-p yrazolo[3',4':3,4]pyrazolo[1,5-*a*]pyridin-6-yl)oxy)acetic acid

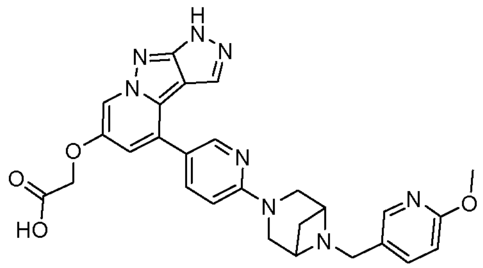

Ethyl

2-((4-(6-(6-((6-methoxypyridin-3-yl)methyl)-3,6-diazabicyclo[3.1.1]heptan-3-yl)pyridin-3-yl)-1*H*-p yrazolo[3',4':3,4]pyrazolo[1,5-*a*]pyridin-6-yl)oxy)acetate (60 mg, 0.11 mmol), methanol (1 mL), tetrahydrofuran (1 mL), water (1 mL), and lithium hydroxide (9 mg, 0.22 mmol) were sequentially added to a reaction flask. The mixture was stirred and reacted at 60 °C for 3 h. After the reaction was completed, the reaction liquid was concentrated. 2 mL of water was added, and hydrochloric acid was added to adjust the pH to 6 to precipitate a product, which was filtered and dried to give a product (20 mg), m/z = 527.1 $[M+1]^+$. $^1$H-NMR (400 MHz, DMSO-$d_6$): δ 12.66 (s, 1H), 8.67 (d, *J*= 2.4 Hz, 1H), 8.56 (d, *J*= 2.4 Hz, 1H), 8.27 (s, 1H), 8.15 (m, 1H), 7.80 (s, 1H), 7.62 (s, 1H), 7.34 (s, 1H), 6.95 (d, *J* = 8.8 Hz, 1H), 6.85 (s, 1H), 4.89 (s, 2H), 3.85 (m, 7H), 3.56 (s, 2H), 3.50 (s, 2H), 2.51 (s, 1H), 1.79 (s, 1H).

**Example 110: Preparation of compound 242**

4-(6-(6-((6-methoxypyridin-3-yl)methyl)-3,6-diazabicyclo[3.1.1]heptan-3-yl)pyridin-3-yl)-1*H*-pyra zolo[3',4':3,4]pyrazolo[1,5-*a*]pyridin-6-yl acrylate

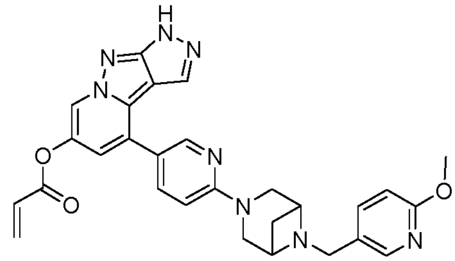

4-(6-(6-((6-methoxypyridin-3-yl)methyl)-3,6-diazabicyclo[3.1.1]heptan-3-yl)pyridin-3-yl)-1*H*-pyra zolo[3',4':3,4]pyrazolo[1,5-*a*]pyridin-6-ol (156 mg, 0.33 mmol) and 3 mL of NMP were added to a reaction flask. NaH (60%, 20 mg, 0.49 mmol) was added under an ice-bath, and the mixture was stirred for 30 min. Then, acryloyl chloride (45 mg, 0.49 mmol) was slowly added under an ice bath, and the mixture was reacted for 0.5 h. After the reaction was completed, the reaction liquid was poured into 30 mL of ice water, and the resulting mixture was extracted with dichloromethane, dried over sodium sulfate, concentrated under reduced pressure, and purified by silica gel column chromatography to give a product (10 mg), m/z = 523 $[M+1]^+$, $^1$H NMR (400MHz, DMSO-$d_6$): δ 12.80 (s, 1H), 8.99 (s, 1H), 8.66 (s, 1H), 8.10 - 8.13 (m, 2H), 7.68 - 7.72 (m, 2H), 7.52 (s, 1H), 6.92-6.94 (m, 1H), 6.76-6.78 (m, 1H), 6.60-6.65 (m, 1H), 6.47-6.53 (m, 1H), 6.22-6.25 (m, 1H), 3.76-3.82 (m, 6H), 3.53-3.69 (m, 7H).

**Example 111: Preparation of compound 244**

(4-(5-(6-ethoxy-1-methyl-1*H*-pyrazolo[3',4':3,4]pyrazolo[1,5-*a*]pyridin-4-yl)pyridin-2-yl)-1-((6-met hoxypyridin-3-yl)methylene)piperazin-2-yl)methanol

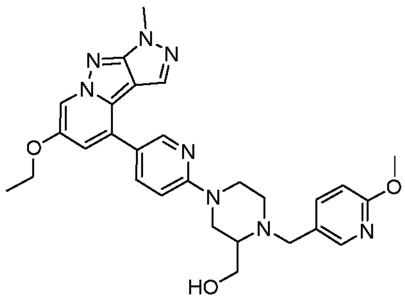

4-(5-(6-ethoxy-1*H*-pyrazolo[3',4':3,4]pyrazolo[1,5-*a*]pyridin-4-yl)pyridin-2-yl)1-((6-methoxypyridi n-3-yl)methylene)piperazin-2-yl)methanol (50 mg, 0.1 mmol), $K_2CO_3$ (41 mg, 0.3 mmol), $CH_3I$ (16 mg, 0.1 mmol), and DMF (3 mL) were added to a reaction flask. The mixture was reacted at 25 °C for 1 h. The reaction liquid was poured into 30 mL of water and the resulting mixture was extracted with EA. The organic phase was dried, concentrated by rotary evaporation to remove the solvent, and purified by column chromatography to give a product (9.8 mg), m/z = 529 $[M+1]^+$, $^1$H-NMR (400 MHz, DMSO-$d_6$): δ 8.57 (s, 1H), 8.51 (s, 1H), 8.09 (s, 1H), 8.02 (d, *J*= 8.8 Hz, 1H), 7.62 (d, *J* = 8.4 Hz, 1H), 7.57 (s, 1H), 7.25 (s, 1H), 7.02 (d, *J* = 8.8 Hz, 1H), 6.78 (d, *J* = 8.4 Hz, 1H), 4.74-4.77 (m, 1H), 4.10-4.20 (m, 3H), 3.99-4.00 (m, 1H), 3.84-3.90 (m, 3H), 3.79-3.84 (m, 6H), 3.53-3.54 (m, 1H), 3.16-3.38 (m, 2H), 2.67-2.68 (m, 1H), 2.26-2.33 (m, 1H), 1.40 (t, *J* = 7.2 Hz, 3H).

**Example 112: Preparation of compound 245**

Step A: 4-(6-(3-(chloromethyl)-4-((6-methoxypyridin-3-yl)methylene)piperazin-1-yl)pyridin-3-yl)-6-ethoxy -1*H*-pyrazolo[3',4':3,4]pyrazolo[1,5-*a*]pyridin-4-yl)pyridine

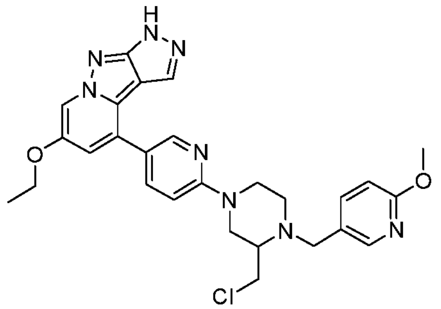

4-(5-(6-ethoxy-1*H*-pyrazolo[3',4':3,4]pyrazolo[1,5-*a*]pyridin-4-yl)pyridin-2-yl)1-((6-methoxypyridi n-3-yl)methylene)piperazin-2-yl)methanol (100 mg, 0.2 mmol), DMF (2 mg, 0.02 mmol), and $SOCl_2$ (1 mL) were added to a reaction flask. The mixture was reacted at 80 °C for 4 h. After the reaction was completed as monitored by LCMS, the reaction liquid was poured into 20 mL of ice water, and a 4 M aqueous NaOH solution was added to adjust the pH to 7. The resulting mixture was extracted with dichloromethane, dried over anhydrous sodium sulfate, concentrated under reduced pressure, and separated by column chromatography to give a product (50 mg), m/z = 533 $[M+1]^+$.

Step B: 1-(4-(5-(6-ethoxy-1*H*-pyrazolo[3',4':3,4]pyrazolo[1,5-*a*]pyridin-4-yl)pyridin-2-yl)-1-((6-methoxypy ridin-3-yl)methylene)piperazin-2-yl)-*N*,*N*-dimethylmethanamine

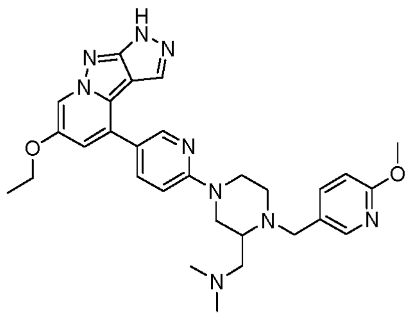

4-(6-(3-(chloromethyl)-4-((6-methoxypyridin-3-yl)methylene)piperazin-1-yl)pyridin-3-yl)-6-ethoxy -1*H*-pyrazolo[3',4':3,4]pyrazolo[1,5-a]pyridin-4-yl)pyridine (50 mg, 0.1 mmol), a solution of dimethylamine in tetrahydrofuran (1 M, 0.1 mL, 0.1 mmol), and DMF (2 mL) were added to a reaction flask. The mixture was reacted at 80 °C for 1 h. The reaction liquid was poured into 20 mL of water, and the resulting mixture was extracted with dichloromethane, dried over anhydrous sodium sulfate, concentrated under reduced pressure, and separated by column chromatography to give a product (22.8 mg), m/z=542 $[M+1]^+$, $^1$H-NMR (400 MHz, DMSO-$d_6$): δ 12.64 (brs, 1H), 8.57 (s, 1H), 8.51 (s, 1H), 8.09 (s, 1H), 8.02 (d, *J* = 8.8 Hz, 1H), 7.62 (d, *J* = 8.4 Hz, 1H), 7.57 (s, 1H), 7.25 (s, 1H), 7.02 (d, *J* = 8.8 Hz, 1H), 6.78 (d, *J* = 8.4 Hz, 1H), 4.74-4.77 (m, 1H), 4.10-4.20 (m, 3H), 3.99-4.00 (m, 1H), 3.79-3.84 (m, 6H), 3.53-3.54 (m, 1H), 3.16-3.38 (m, 2H), 2.67-2.68 (m, 1H), 2.49-2.51 (m, 6H), 2.26-2.33 (m, 1H),1.36 (t, *J* = 7.2 Hz, 3H).

**Example 113: Preparation of compound 246**

Step A: 4-(6-(6-((6-methoxypyridin-3-yl)methyl)-3,6-diazabicyclo[3.1.1]heptan-3-yl)pyridin-3-yl)-1*H*-pyra zolo[3',4':3,4]pyrazolo[1,5-*a*]pyridin-6-yl trifluoromethanesulfonate

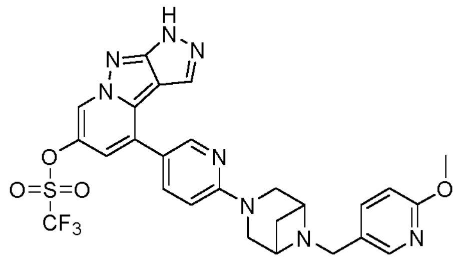

DMSO (5 mL), DIEA (1 mL), and 4-(6-(6-(((6-methoxypyridin-3-yl)methyl)-3,6-diazabicyclo[3.1.1]heptan-3-yl)pyridin-3-yl)-1*H*-pyr azolo[3',4':3,4]pyrazolo[1,5-*a*]pyridin-6-ol (510 mg, 1.09 mmol) were sequentially added to a reaction flask, and then 1,1,1-trifluoro-*N*-phenyl-*N*-((trifluoromethyl)sulfonyl)methanesulfonamide (389 mg, 1.09 mmol) was added in portions. The mixture was reacted at room temperature for 2 h. The reaction was quenched by adding water and stirring. The reaction liquid was extracted with dichloromethane. The organic phase was dried over anhydrous sodium sulfate, concentrated under reduced pressure, and separated by column chromatography to give a product (500 mg), m/z = 601 $[M+1]^+$. $^1$HNMR (400 MHz, DMSO-$d_6$): δ 13.00 (brs, 1H), 9.51 (d, *J* = 2.0Hz, 1H), 8.68 (s, 1H), 8.14 (d, *J*= 8.0Hz, 1H), 8.11 (s, 1H), 7.79-7.70 (m, 3H), 6.95 (d, *J* = 8.8Hz, 1H), 6.77 (d, *J*= 8.4Hz, 1H), 3.76-3.91 (m, 5H), 3.47-3.75 (m, 6H), 3.31 (s, 2H), 1.61-1.59 (m, 1H).

**Example 114: Preparation of compound 247**

Step A: 7-bromo-6-ethoxy-4-(6-(6-((6-methoxypyridin-3-yl)methyl)-3,6-diazabicyclo[3.1.1]heptan-3-yl)pyr idin-3-yl)-1*H*-pyrazolo[3',4':3,4]pyrazolo[1,5-*a*]pyridine

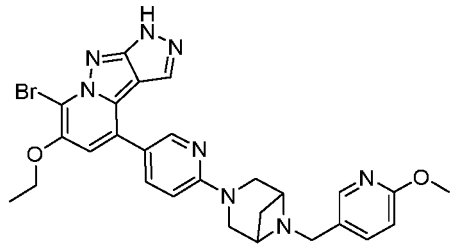

6-ethoxy-4-(6-(6-((6-methoxypyridin-3-yl)methyl)-3,6-diazabicyclo[3.1.1]heptan-3-yl)pyridin-3-yl) -1*H*-pyrazolo[3',4':3,4]pyrazolo[1,5-*a*]pyridine (5 g, 10.06 mmol) and *N*,*N*-dimethylformamide (50 mL) were added to a reaction flask. The mixture was dissolved under stirring, and then NBS (2.15 g, 12.08 mmol) was added. The resulting mixture was reacted at room temperature for 1 h. The reaction was quenched by adding water. The reaction liquid was extracted with ethyl acetate and separated by silica gel column chromatography to give a product (1.5 g), m/z = 575/577 $[M+1]^+$.

Step B: 7-amino-6-ethoxy-4-(6-(6-((6-methoxypyridin-3-yl)methyl)-3,6-diazabicyclo[3.1.1]heptan-3-yl)pyr idin-3-yl)-1*H*-pyrazolo[3',4':3,4]pyrazolo[1,5-*a*]pyridine

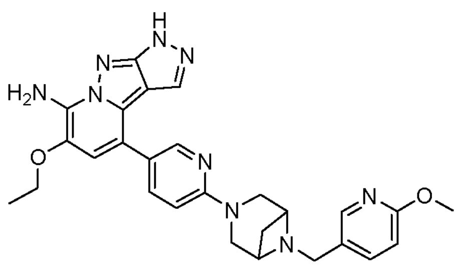

7-bromo-6-ethoxy-4-(6-(6-((6-methoxypyridin-3-yl)methyl)-3,6-diazabicyclo[3.1.1]heptan-3-yl)pyr idin-3-yl)-1*H*-pyrazolo[3',4':3,4]pyrazolo[1,5-*a*]pyridine (200 mg, 0.35 mmol), dimethylsulfoxide (3 mL), copper(I) iodide (200 mg), and aqueous ammonia (1 mL) were sequentially added to a reaction flask. The mixture was purged with nitrogen and reacted at 90 °C for 13 h. The reaction was quenched by adding water and stirring. The reaction liquid was extracted with dichloromethane, dried over anhydrous sodium sulfate, concentrated under reduced pressure, and separated by column chromatography to give a product (5.2 mg), m/z = 512.1 $[M+1]^+$, $^1$H-NMR (400 MHz, DMSO-$d_6$): δ 12.66 (s, 1H), 8.57 (s, 1H), 8.10 (s, 1H), 8.01 (m, 1H), 7.70 (m, 1H), 7.62 (s, 1H), 7.44 (s, 1H), 6.87 (m, 1H), 6.78 (m, 1H), 6.51 (s, 2H), 4.22-4.16 (q, *J* = 8 Hz, 2H), 3.83 (s, 3H), 3.78-3.70 (m, 5H), 3.54 (m, 4H), 1.70 (m, 1H), 1.41-1.38 (m, 3H).

**Example 115: Preparation of compound 248**

Step A: 3-bromo-6-ethoxy-4-(6-(6-((6-methoxypyridin-3-yl)methyl)-3,6-diazabicyclo[3.1.1]heptan-3-yl)pyr idin-3-yl)-1*H*-pyrazolo[3',4':3,4]pyrazolo[1,5-*a*]pyridine

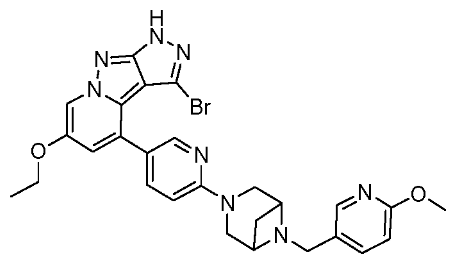

6-ethoxy-4-(6-(6-((6-methoxypyridin-3-yl)methyl)-3,6-diazabicyclo[3.1.1]heptan-3-yl)pyridin-3-yl) -1*H*-pyrazolo[3',4':3,4]pyrazolo[1,5-*a*]pyridine (5 g, 10.06 mmol), and *N*,*N*-dimethylformamide (50 mL) were added to a reaction flask. The mixture was dissolved under stirring, and then NBS (2.15 g, 12.08 mmol) was slowly added at room temperature. The resulting mixture was reacted for 1 h. The reaction was quenched by adding water. The reaction liquid was extracted with ethyl acetate, and separated by silica gel column chromatography to give a product (1 g), m/z = 575/577$[M+1]^+$.

Step B: 3-cyano-6-ethoxy-4-(6-(6-((6-methoxypyridin-3-yl)methyl)-3,6-diazabicyclo[3.1.1]heptan-3-yl)pyri din-3-yl)-1*H*-pyrazolo[3',4':3,4]pyrazolo[1,5-*a*]pyridine

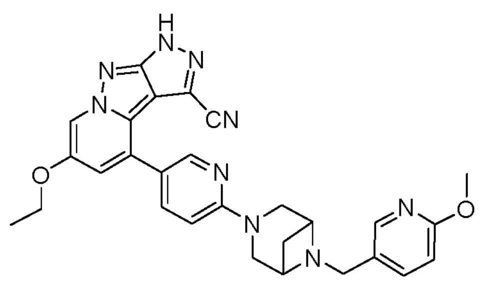

3-bromo-6-ethoxy-4-(6-(6-((6-methoxypyridin-3-yl)methyl)-3,6-diazabicyclo[3.1.1]heptan-3-yl)pyr idin-3-yl)-1*H*-pyrazolo[3',4':3,4]pyrazolo[1,5-*a*]pyridine (100 mg, 0.17 mmol), *N*,*N*-dimethylacetamide (1 mL), water (0.5 mL), potassium ferrocyanide (32 mg, 0.09 mmol), XantPhos (20 mg, 0.03 mmol), and allylpalladium(II) chloride dimer (6.3 mg, 0.017 mmol) were sequentially added to a reaction flask. The mixture was purged with nitrogen and reacted at 90 °C for 13 h. The reaction liquid was extracted with water and ethyl acetate, and separated by silica gel column chromatography to give a product (2 mg), m/z = 522 $[M+1]^+$. $^1$H-NMR (400 MHz, DMSO-$d_6$): δ 12.85 (s, 1H), 8.69 (s, 1H), 8.15-8.10 (m, 2H), 7.71-7.57 (m, 3H), 6.98-6.92 (m, 1H), 6.79-6.77 (d, *J*= 8.4 Hz, 1H), 4.38-4.33 (q, *J*= 6.8 Hz, 2H), 3.83-3.77 (m, 5H), 3.70-3.53 (m, 7H), 1.61-1.59 (d, *J*= 8.4 Hz, 1H), 1.41-1.40 (t, *J*= 3.2 Hz, 3H).

**Example 116: Preparation of compound 249**

Step A: *tert*-butyl 4-(5-(6-ethoxy-1*H*-pyrazolo[3',4':3,4]pyrazolo[1,5-*a*]pyridin-4-yl)pyridin-2-yl)-2-(hydroxymethyl)p iperazine-1-carboxylate

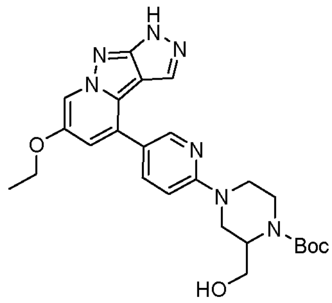

6-ethoxy-4-(6-fluoropyridin-3-yl)-1*H*-pyrazolo[3',4':3,4]pyrazolo[1,5-*a*]pyridine (1.4 g, 4.7 mmol), *tert*-butyl 2-(hydroxymethyl)piperazine-1-carboxylate (1 g, 4.7 mmol), DIEA (1.8 g, 14.1 mmol), and DMSO (15 mL) were added to a reaction flask. The mixture was reacted at 90 °C for 12 h. The reaction was quenched by adding water and stirring. The reaction liquid was extracted with dichloromethane, dried over anhydrous sodium sulfate, concentrated under reduced pressure, and separated by column chromatography to give a product (1.6 g), m/z = 494 $[M+1]^+$.

Step B: 4-(5-(6-ethoxy-1*H*-pyrazolo[3',4':3,4]pyrazolo[1,5-*a*]pyridin-4-yl)pyridin-2-yl)-2-(hydroxymethyl)p iperazine hydrochloride

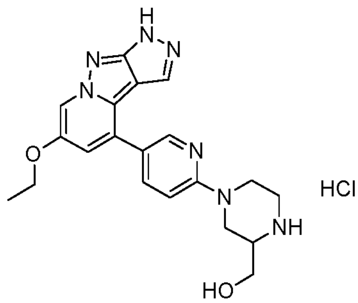

*Tert*-butyl

4-(5-(6-ethoxy-1*H*-pyrazolo[3',4':3,4]pyrazolo[1,5-*a*]pyridin-4-yl)pyridin-2-yl)-2-(hydroxymethyl)p iperazine-1-carboxylate (1.6 g, 3.2 mmol) was added to a solution of HCl in 1,4-dioxane (4 M, 20 mL). The mixture was reacted at 25 °C for 4 h. The reaction liquid was directly concentrated by rotary evaporation to remove the solvent to give a product (1.7 g), m/z = 394 $[M+1]^+$.

Step C: (4-(5-(6-ethoxy-1*H*-pyrazolo[3',4':3,4]pyrazolo[1,5-*a*]pyridin-4-yl)pyridin-2-yl)-1-((6-methoxypyri din-3-yl)methylene)piperazin-2-yl)methanol

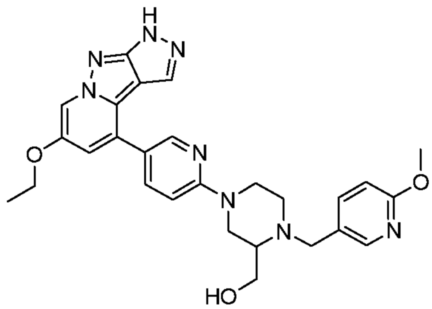

4-(5-(6-ethoxy-1*H*-pyrazolo[3',4':3,4]pyrazolo[1,5-*a*]pyridin-4-yl)pyridin-2-yl)-2-(hydroxymethyl)p iperazine hydrochloride (100 mg, 0.19 mmol), 6-methoxy-3-pyridinecarboxaldehyde (80 mg, 0.57 mmol), $Et_3N$ (98 mg, 0.8 mmol), Pic-$BH_3$ (63 mg, 0.57 mmol), and DMSO (3 mL) were added to a reaction flask. The mixture was reacted at 45 °C for 12 h. The reaction was quenched by adding water and stirring. The reaction liquid was extracted with dichloromethane, dried over anhydrous sodium sulfate, concentrated under reduced pressure, and separated by column chromatography to give a product (10.1 mg), m/z = 515 $[M+1]^+$. $^1$H-NMR (400 MHz, DMSO-$d_6$): δ 12.64 (brs, 1H), 8.57 (s, 1H), 8.51 (s, 1H), 8.09 (s, 1H), 8.02 (d, *J* = 8.8 Hz, 1H), 7.62 (d, *J* = 8.4 Hz, 1H), 7.57 (s, 1H), 7.25 (s, 1H), 7.02 (d, *J* = 8.8 Hz, 1H), 6.78 (d, *J* = 8.4 Hz, 1H), 4.74-4.77 (m, 1H), 4.10-4.20 (m, 3H), 3.99-4.00 (m, 1H), 3.79-3.84 (m, 6H), 3.53-3.54 (m, 1H), 3.16-3.38 (m, 2H),2.67-2.68 (m, 1H), 2.26-2.33 (m, 1H),1.39 (t, *J* = 7.2 Hz, 3H).

**Example 117: Preparation of compound 250**

1-(3-(5-(6-ethoxy-1*H*-pyrazolo[3',4':3,4]pyrazolo[1,5-*a*]pyridin-4-yl)pyridin-2-yl)-3,6-diazabicyclo[ 3.1.1]heptan-6-yl)prop-2-en-1-one

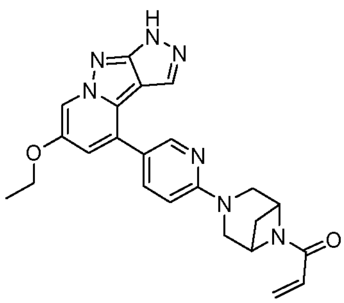

4-(6-(3,6-diazabicyclo[3.1.1]heptan-3-yl)pyridin-3-yl)-6-ethoxy-1*H*-pyrazolo[3',4':3,4]pyrazolo[1,5 -*a*]pyridine sulfate (150 mg, 0.2 mmol), DIEA (193 mg, 1.5 mmol), and DMF (2 mL) were added to a reaction flask, and acryloyl chloride (22 mg, 0.25 mmol) was added under an ice bath. The mixture was reacted at 25 °C for 2 h. The reaction was quenched by adding water and stirring. The reaction liquid was extracted with dichloromethane, dried over anhydrous sodium sulfate, concentrated under reduced pressure, and separated by column chromatography to give a product (15.2 mg), m/z = 430 $[M+1]^+$. $^1$HNMR (400 MHz, DMSO-$d_6$) δ 12.64 (brs, 1H), 8.60 (d, *J*= 2.4Hz, 1H), 8.51 (s, 1H), 8.05 (dd, $J_1$ = 2.4Hz, $J_2$ = 8.8Hz, 1H), 7.60 (s, 1H), 7.24 (s, 1H), 6.83 (d, *J* = 8.8Hz, 1H), 6.44-6.51 (m, 1H), 6.10 (d, *J* = 16.8Hz, 1H), 5.67 (d, *J* = 10.4Hz, 1H), 4.87-4.88 (m, 1H), 4.53-4.54 (m, 1H), 4.15 (q, *J* = 6.8Hz, 2H), 3.98-4.04 (m, 1H), 3.75-3.78 (m, 2H), 3.59-3.68 (m, 1H), 2.71-2.77 (m, 1H), 1.65-1.68 (m, 1H), 1.39 (t, *J* = 6.8Hz, 3H).

**Example 118: Preparation of compound 251**

6-ethoxy-4-(6-(3-(methoxymethyl)-4-((6-methoxypyridin-3-yl)methylene)piperazin-1-yl)pyridin-3-yl)-1*H*-pyrazolo[3',4':3,4]pyrazolo[1,5-*a*]pyridin-4-yl)pyridine

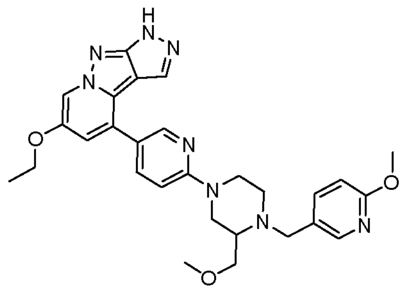

4-(6-(3-(chloromethyl)-4-((6-methoxypyridin-3-yl)methylene)piperazin-1-yl)pyridin-3-yl)-6-ethoxy -1*H*-pyrazolo[3',4':3,4]pyrazolo[1,5-*a*]pyridin-4-yl)pyridine (150 mg, 0.28 mmol), sodium methoxide (17 mg, 0.31 mmol), and MeOH (2 mL) were added to a reaction flask. The mixture was reacted at 25 °C for 1 h. The reaction was quenched by adding water and stirring. The reaction liquid was extracted with dichloromethane, dried over anhydrous sodium sulfate, concentrated under reduced pressure, and separated by column chromatography to give a product (6.5 mg), m/z = 529 $[M+1]^+$. $^1$H-NMR (400 MHz, DMSO-$d_6$) δ 12.64 (brs, 1H), 8.57 (s, 1H), 8.51 (s, 1H), 7.99-8.10 (m, 2H), 7.57-7.71 (m, 2H), 7.26 (s, 1H), 7.04 (d, *J* = 8.8 Hz, 1H), 6.77 (d, *J* = 8.4 Hz, 1H), 4.20-4.24 (m, 2H), 4.02-4.18 (m, 2H), 3.91-3.98 (m, 4H), 3.82-3.84 (m, 2H), 3.30-3.33 (m, 6H), 3.21-3.22 (m, 1H), 2.49-2.51 (m, 2H), 1.39 (t, *J*= 7.2 Hz, 3H).

**Example 119: Preparation of compound 258 and compound 259**

Compound 258: (1*S*,4*R*)-4-(6-ethoxy-1*H*-pyrazolo[3',4':3,4]pyrazolo[1,5-*a*]pyridin-4-yl)-*N*-((*S*)-1-(6-(4-fluoro-1*H*-p yrazol-1-yl)pyridin-3-yl)ethyl)-1-methoxycyclohexane-1-carboxamide

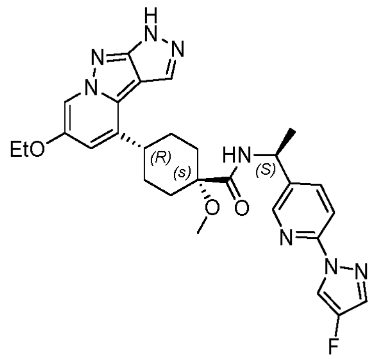

Compound 259: (1*R*,4*S*)-4-(6-ethoxy-1*H*-pyrazolo[3',4':3,4]pyrazolo[1,5-*a*]pyridin-4-yl)-*N*-((*S*)-1-(6-(4-fluoro-1*H*-p yrazol-1-yl)pyridin-3-yl)ethyl)-1-methoxycyclohexane-1-carboxamide

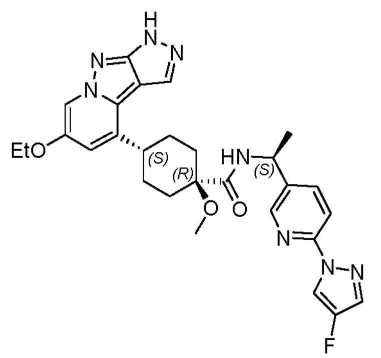

Step A: *tert*-butyl (*S*)-(1-(6-bromopyridin-3-yl)ethyl)carbamate

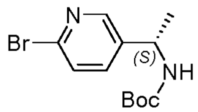

(*S*)-1-(6-bromopyridin-3-yl)ethan-1-amine (210 mg, 1.05 mmol), Boc anhydride (273 mg, 1.25 mmol), TEA (318 mg, 3.15 mmol), and acetonitrile (10 mL) were added to a reaction flask. The mixture was reacted at room temperature for 4 h. The reaction was quenched by adding water and stirring. The reaction liquid was extracted with dichloromethane, dried over anhydrous sodium sulfate, concentrated under reduced pressure, and separated by column chromatography to give a product (300 mg), m/z = 301 $[M+1]^+$.

Step B: *tert*-butyl (*S*)-(1-(6-(4-(4-fluoro-1*H*-pyrazol-1-yl)pyridin-3-yl)ethyl)carbamate

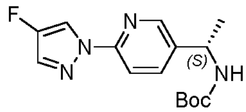

*Tert*-butyl-(*S*)-(1-(6-bromopyridin-3-yl)ethyl)carbamate (290 mg, 0.96 mmol), 4-fluoro-1*H*-pyrazole (249 mg, 29.3 mmol), $N_1$,$N_2$-dimethylcyclohexane-1,2-diamine (341 mg, 2.4 mmol), copper(I) iodide (183 mg, 0.96 mmol), and acetonitrile (5 mL) were added to a reaction flask. The mixture was reacted at 108 °C for 4 h, and then cooled to room temperature. 50 mL of water and 50 mL of ethyl acetate were added, and the resulting mixture was extracted, followed by liquid separation. The organic phase was washed with 50 mL of saturated brine, dried over anhydrous sodium sulfate, filtered, concentrated, and separated by column chromatography (EA/PE = 20%) to give a product (245 mg), m/z = $307[M+1]^+$.

Step C: (*S*)-1-(6-(4-(4-fluoro-1*H*-pyrazol-1-yl)pyridin-3-yl)ethan-1-amine hydrochloride

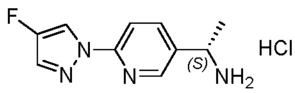

*Tert*-butyl (*S*)-(1-(6-(4-(4-fluoro-1*H*-pyrazol-1-yl)pyridin-3-yl)ethyl)carbamate (240 mg, 0.78 mmol) and a 4 M hydrochloric acid/dioxane solution (10 mL) were added to a reaction flask. The mixture was reacted at room temperature for 2 h. After the reaction was completed, the reaction liquid was concentrated under reduced pressure to give a product (180 mg), m/z = 207 $[M+1]^+$.

Step D: methyl 1-methoxy-4-(((trifluoromethyl)sulfonyl)oxy)cyclohex-3-ene-1-carboxylate

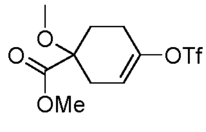

Methyl 1-methoxy-4-oxocyclohexane-1-carboxylate (680 mg, 3.6 mmol) and THF (15 mL) were added to a reaction flask. Then, LDA (2 N, 2.7 mL) was added dropwise to the reaction liquid at -70 °C to -80 °C, and the mixture was reacted for 1 h with the temperature maintained at this range. 1,1,1-trifluoro-N phenyl-N ((trifluoromethyl)sulfonyl)methanesulfonamide (1.5 g, 4.3 mmol) was dissolved in 10 mL of tetrahydrofuran, and then the reaction liquid

described above was added dropwise. After the dropwise addition, the reaction liquid was naturally warmed to room temperature and reacted for 2 h. The reaction was quenched by adding water and stirring. The reaction liquid was extracted with dichloromethane, dried over anhydrous sodium sulfate, concentrated under reduced pressure, and separated by column chromatography to give a product (820 mg).

Step E: methyl 4-(6-ethoxy-1*H*-pyrazolo[3',4':3,4]pyrazolo[1,5-*a*]pyridin-4-yl)-1-methoxycyclohex-3-ene-1-carbox ylate

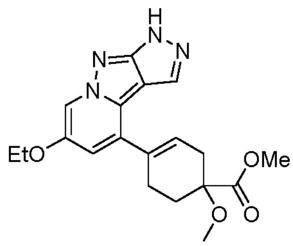

6-ethoxy-4-(4,4,5,5-tetramethyl-1,3,2-dioxaborolan-2-yl)-1*H*-pyrazolo[3',4':3,4]pyrazolo[1,5-*a*]pyri dine (938 mg, 2.9 mmol), methyl 1-methoxy-4-(((trifluoromethyl)sulfonyl)oxy)cyclohex-3-ene-1-carboxylate (1.0 g, 3.1 mmol), cesium carbonate (1.8 g, 5.7 mmol), Pd(dppf)$Cl_2$ (21 mg, 0.28 mmol), 1,4-dioxane (25 mL), and water (5 mL) were added to a reaction flask. The mixture was purged with nitrogen for 2 min, heated to 100 °C, and reacted for 6 h. The reaction was quenched by adding water and stirring. The reaction liquid was extracted with dichloromethane, dried over anhydrous sodium sulfate, concentrated under reduced pressure, and separated by column chromatography to give a product (700 mg), m/z = 371 $[M+1]^+$.

Step F: methyl 4-(6-ethoxy-1*H*-pyrazolo[3',4':3,4]pyrazolo[1,5-*a*]pyridin-4-yl)-1-methoxycyclohexane-1-carboxyla te

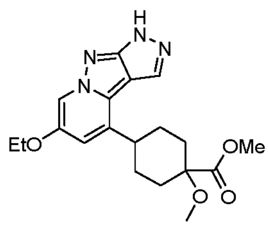

Methyl

4-(6-ethoxy-1*H*-pyrazolo[3',4':3,4]pyrazolo[1,5-*a*]pyridin-4-yl)-1-methoxycyclohex-3-ene-1-carbox ylate (420 mg, 1.1 mmol) and absolute ethanol (30 mL) were added to a reaction flask. The mixture was purged with nitrogen, and then $Pd(OH)_2$ (100 mg) was added. The resulting mixture was purged with hydrogen and reacted at 80 °C for 48 h under hydrogen atmosphere. After the reaction was completed, the reaction liquid was cooled, filtered, rinsed with 20 mL of ethanol, concentrated, and purified by column chromatography to give a product (330 mg), m/z = 373 $[M+1]^+$.

Step G: 4-(6-ethoxy-1*H*-pyrazolo[3',4':3,4]pyrazolo[1,5-*a*]pyridin-4-yl)-1-methoxycyclohexane-1-carboxyli c acid

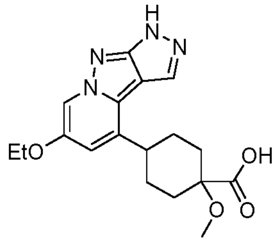

Methyl

4-(6-ethoxy-1*H*-pyrazolo[3',4':3,4]pyrazolo[1,5-*a*]pyridin-4-yl)-1-methoxycyclohexane-1-carboxyla te (320 mg, 0.8 mmol), THF (10 mL), methanol (2 mL), and lithium hydroxide (81 mg, 3.4 mmol) were added to a reaction flask. Lithium hydroxide was dissolved in 1 mL of water, and the solution was added to the reaction system described above. The mixture was reacted at 60 °C for 18 h. After the reaction was completed, the reaction liquid was concentrated under reduced pressure to give a product (280 mg), m/z = 359 $[M+1]^+$.

Step H: (*S*)-4-(6-ethoxy-1*H*-pyrazolo[3',4':3,4]pyrazolo[1,5-*a*]pyridin-4-yl)-*N*-(1-(6-(4-fluoro-1*H*-pyrazol-1-yl)pyridin-3-yl)ethyl)-1-methoxycyclohexane-1-carboxamide

4-(6-ethoxy-1*H*-pyrazolo[3',4':3,4]pyrazolo[1,5-*a*]pyridin-4-yl)-1-methoxycyclohexane-1-carboxyli c acid (250 mg, 0.7 mmol), (*S*)-(1-(6-(4-(4-fluoro-1*H*-pyrazol-1-yl)pyridin-3-yl)ethan-1-amine hydrochloride (180 mg, 0.7 mmol), Py-Bop (400 mg, 0.77 mmol), DIEA (271 mg, 2.1 mmol), and DMF (10 mL) were added to a reaction flask. The mixture was reacted at room temperature for 12 h. After the reaction was completed, the reaction was quenched by adding 10 mL of saturated sodium bicarbonate. The reaction liquid was added with 50 mL of water for dilution and extracted with ethyl acetate (3 × 30 mL). The organic phases were mixed, washed with saturated brine, dried over anhydrous sodium sulfate, concentrated, and separated by column chromatography to give a product (340 mg), which was then subjected to SFC chiral resolution to give compound 258 (104.2 mg), m/z = 547 $[M+1]^+$; $^1$H NMR (400 MHz, DMSO-$d_6$): δ 12.55 (brs, 1H), 8.68 (d, *J* = 4.4Hz, 1H), 8.55 (d, *J*= 8.4Hz, 1H), 8.45 (s, 1H), 8.37 (s, 1H), 8.00 (d, *J*= 8.4Hz, 1H), 7.94-7.88 (m, 3H), 6.99 (s, 1H), 5.06 (t, *J* = 7.2Hz, 1H), 4.08 (q, *J* = 7.2Hz, 2H), 3.21 (s, 3H), 3.07-2.96 (m, 1H), 2.09-1.86 (m, 5H), 1.84-1.72 (m, 3H), 1.48 (d, *J* = 7.2Hz, 3H), 1.34 (t, *J* = 7.2Hz, 3H); and compound 259 (75.8 mg), m/z = 547 $[M+1]^+$. $^1$HNMR (400 MHz, DMSO-$d_6$): δ 12.50 (brs, 1H), 8.65 (d, *J* = 4.4Hz, 1H), 8.59 (d, *J* = 8.4Hz, 1H), 8.45 (s, 1H), 8.35 (s, 1H), 8.00 (d, *J* = 8.4Hz, 1H), 7.92-7.86 (m, 3H), 6.83 (s, 1H), 5.15 (t, *J* = 7.6Hz, 1H), 4.04 (q, *J* = 7.2Hz, 2H), 3.11 (s, 3H), 3.05-2.93 (m, 1H), 2.37-2.32 (m, 1H), 1.94-1.72 (m, 5H), 1.70-1.62 (m, 2H), 1.48 (d, *J* = 7.2Hz, 3H), 1.32 (t, *J* = 7.2Hz, 3H).

**Example 120: Preparation of compound 267**

*N*-((3*R*,4*S*)-1-(5-(6-(2-fluoroethoxy)-1*H*-pyrazolo[3',4':3,4]pyrazolo[1,5-*a*]pyridin-4-yl)pyridin-2-yl )-3-hydroxypiperidin-4-yl)-2,6-difluorobenzamide

Step A: 4-bromo-6-(2-fluoroethoxy)-2-fluoropyrazolo[1,5-*a*]pyridine-3-carbaldehyde

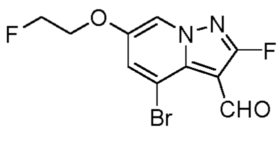

4-bromo-6-hydroxy-2-fluoropyridine[1,5-*a*]pyridine-3-carbaldehyde (5 g, 19.3 mmol), $K_2CO_3$ (5.3 g, 38.6 mmol), 1-fluoro-2-bromoethane (2.7 g, 21.2 mmol), and DMF (50 mL) were added to a reaction flask. The mixture was reacted at 65 °C for 2 h. After the reaction was completed as monitored by LCMS, the reaction liquid was poured into ice water, and the resulting mixture was extracted with dichloromethane and purified by column chromatography to give a product (2.65 g), m/z = 305, 307 $[M+1]^+$.

Step B: 4-bromo-6-(2-fluoroethoxy)-1*H*-pyrazolo[3',4':3,4]pyrazolo[1,5-*a*]pyridine

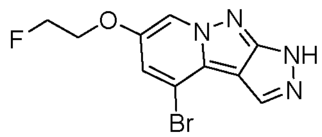

4-bromo-6-(2-fluoroethoxy)-2-fluoropyrazolo[1,5-*a*]pyridine-3-carbaldehyde (2.55 g, 13.6 mmol), hydrazine hydrate (85%, 4.0 g, 68.1 mmol), and DMSO (50 mL) were added to a reaction flask. The mixture was reacted at 130 °C for 12 h. After the reaction was completed as monitored by LCMS, the reaction liquid was poured into water, and the resulting mixture was extracted with EA. The organic phase was dried, filtered, concentrated by rotary evaporation under reduced pressure to remove the solvent, and purified by column chromatography to give a product (1.6 g), m/z = 299, 301 $[M+1]^+$.

Step C: 6-(2-fluoroethoxy)-4-(6-fluoropyridin-3-yl)-1*H*-pyrazolo[3',4':3,4]pyrazolo[1,5-*a*]pyridine

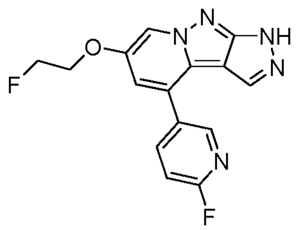

4-bromo-6-(2-fluoroethoxy)-1*H*-pyrazolo[3',4':3,4]pyrazolo[1,5-*a*]pyridine (1.6 g, 5.3 mmol), 6-fluoropyridine-3-boronic acid (0.75 g, 5.9 mmol), $Cs_2CO_3$ (3.49 g, 10.7 mmol), Pd(dppf)$Cl_2$ (390 mg, 0.5 mmol), dioxane (20 mL), and water (5 mL) were added to a reaction flask. The mixture was purged with nitrogen 3 times and reacted at 90 °C for 16 h. After the reaction was completed as monitored by LCMS, the reaction liquid was poured into water, and the resulting mixture was extracted with EA. The organic phase was dried, concentrated under reduced pressure, and purified by column chromatography to give a product (837 mg), m/z = 316 $[M+1]^+$.

Step D: *tert*-butyl ((3*R*,4*S*)-1-(5-(6-(2-fluoroethoxy)-1*H*-pyrazolo[3',4':3,4]pyrazolo[1,5-*a*]pyridin-4-yl)pyridin-2-yl)-3 -hydroxypiperidin-4-yl)aminocarbonate

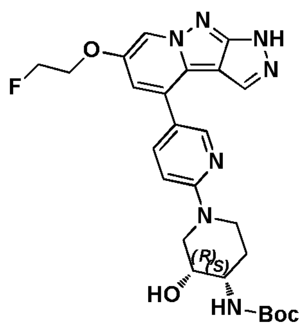

6-(2-fluoroethoxy)-4-(6-fluoropyridin-3-yl)-1*H*-pyrazolo[3',4':3,4]pyrazolo[1,5-*a*]pyridine (410 mg, 1.3 mmol), tert-butyl ((3*R*,4*S*)-3-hydroxypiperidin-4-yl)carbonate (309 mg, 1.4 mmol), DIEA (840 mg, 6.5 mmol), and DMSO (5 mL) were added to a reaction flask. The mixture was reacted at 90 °C for 40 h. The reaction liquid was cooled to room temperature and poured into water, and the resulting mixture was extracted with dichloromethane. The organic phase

was dried, concentrated under reduced pressure, and purified by column chromatography to give a product (420 mg), m/z = 512 $[M+1]^+$.

Step E: (3*R*,4*S*)-4-amino-1-(5-(6-(2-fluoroethoxy)-1*H*-pyrazolo[3',4':3,4]pyrazolo[1,5-*a*]pyridin-4-yl)pyridi n-2-yl)-3-hydroxypiperidine hydrochloride

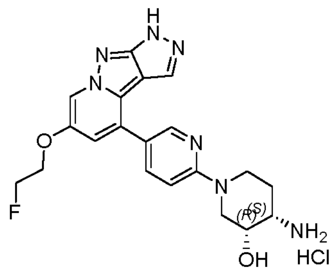

Tert-butyl

((3*R*,4*S*)-1-(5-(6-(2-fluoroethoxy)-1*H*-pyrazolo[3',4':3,4]pyrazolo[1,5-*a*]pyridin-4-yl)pyridin-2-yl)-3 -hydroxypiperidin-4-yl)aminocarbonate (420 mg, 0.8 mmol) and a 4 N hydrochloric acid/dioxane solution (8 mL) were added to a reaction flask. The mixture was reacted at 25 °C for 1 h. After the reaction was completed, the reaction liquid was concentrated under reduced pressure to dryness to give a product (400 mg), m/z = 412 $[M+1]^+$.

Step F: *N*-((3*R*,4*S*)-1-(5-(6-(2-fluoroethoxy)-1*H*-pyrazolo[3',4':3,4]pyrazolo[1,5-*a*]pyridin-4-yl)pyridin-2-yl )-3-hydroxypiperidin-4-yl)-2,6-difluorobenzamide

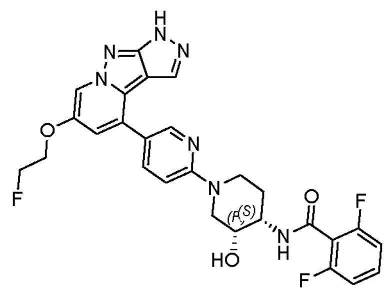

(3*R*,4*S*)-4-amino-1-(5-(6-(2-fluoroethoxy)-1*H*-pyrazolo[3',4':3,4]pyrazolo[1,5-*a*]pyridin-4-yl)pyridi n-2-yl)-3-hydroxypiperidine hydrochloride (400 mg, 0.9 mmol), 2,6-difluorobenzoic acid (151 mg, 1.0 mmol), DIEA (512 mg, 4.3 mmol), HATU (363 mg, 1.0 mmol), and DMF (5 mL) were reacted at 25 °C for 2 h. The reaction liquid was poured into water, and the resulting mixture was extracted with dichloromethane. The organic phase was dried, concentrated under reduced pressure, and purified by column chromatography to give a product (161.5 mg), m/z = 552 $[M+1]^+$. $^1H$ NMR (400 MHz, DMSO-$d_6$) $\delta$ 12.68 (s, 1H), 8.57-8.63 (m, 3H), 8.02-8.05 (m, 1H), 7.70 (m, 1H), 7.48-7.61 (m, 1H), 7.32-7.33 (m, 1H), 7.11-7.15 (m, 3H), 5.01-5.01 (m, 1H), 4.87-4.89 (m, 1H), 4.75-4.77 (m, 1H), 4.38-4.46 (m, 2H), 4.17-4.21 (m, 3H), 3.94-3.94 (m, 1H), 3.36-3.41 (m, 1H), 3.11-3.14 (m, 1H), 1.93-1.96 (m, 1H), 1.69-1.72 (m, 1H).

**Example 121: Preparation of compound 268**

*N*-((3*R*,4*S*)-1-(5-(6-(2,2-difluoroethoxy)-1*H*-pyrazolo[3',4':3,4]pyrazolo[1,5-*a*]pyridin-4-yl)pyridin-2-yl)-3-hydroxypiperidin-4-yl)-2,6-difluorobenzamide

Step A: 4-bromo-6-(2,2-difluoroethoxy)-2-fluoropyrazolo[1,5-*a*]pyridine-3-carbaldehyde

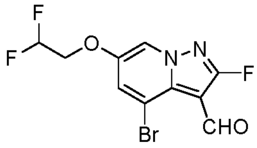

4-bromo-6-hydroxy-2-fluoropyridine[1,5-*a*]pyridine-3-carbaldehyde (15 g, 54 mmol), $K_2CO_3$ (14.9 g, 108 mmol), 1,1-difluoro-2-iodoethane (11.4 g, 60 mmol), and DMF (140 mL) were added to a reaction flask. The mixture was reacted at 65 °C for 24 h. After the reaction was completed as monitored by LCMS, the reaction liquid was poured into water, and the resulting mixture was extracted with EA. The organic phase was dried, concentrated under reduced pressure, and purified by column chromatography to give a product (4.4 g), m/z = 323, 325 $[M+1]^+$.

Step B: 4-bromo-6-(2,2-difluoroethoxy)-1*H*-pyrazolo[3',4':3,4]pyrazolo[1,5-*a*]pyridine

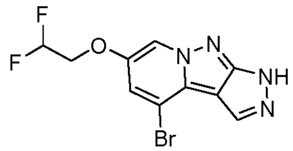

4-bromo-6-(2,2-difluoroethoxy)-2-fluoropyrazolo[1,5-*a*]pyridine-3-carbaldehyde (4.4 g, 13.6 mmol), hydrazine hydrate (85%, 4.0 g, 68.1 mmol), and DMSO (50 mL) were added to a reaction flask. The mixture was reacted at 130 °C for 12 h. After the reaction was completed as monitored by LCMS, the reaction liquid was poured into water, and the resulting mixture was extracted with EA. The organic phase was dried, concentrated under reduced pressure, and purified by column chromatography to give a product (3.4 g), m/z = 317, 319 $[M+1]^+$.

Step C: 6-(2,2-difluoroethoxy)-4-(6-fluoropyridin-3-yl)-1*H*-pyrazolo[3',4':3,4]pyrazolo[1,5-*a*]pyridine

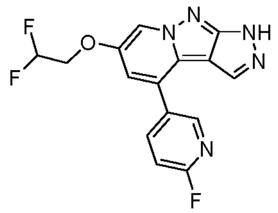

4-bromo-6-(2,2-difluoroethoxy)-1*H*-pyrazolo[3',4':3,4]pyrazolo[1,5-*a*]pyridine (2.9 g, 9.1 mmol), 6-fluoropyridine-3-boronic acid (1.4 g, 10.0 mmol), $Cs_2CO_3$ (6.0 g, 18.3 mmol), Pd(dppf)$Cl_2$ (670 mg, 0.9 mmol), dioxane (34 mL), and water (7 mL) were added to a reaction flask. The mixture was purged with nitrogen 3 times and reacted at 90 °C for 2 h. After the reaction was completed as monitored by LCMS, the reaction liquid was poured into water, and the resulting mixture was extracted with EA. The organic phase was dried, concentrated under reduced pressure, and purified by column chromatography to give a product (1.6 g), m/z = 334 $[M+1]^+$.

Step D: *tert*-butyl ((3*R*,4*S*)-1-(5-(6-(2,2-difluoroethoxy)-1*H*-pyrazolo[3',4':3,4]pyrazolo[1,5-*a*]pyridin-4-yl)pyridin-2-yl)-3-hydroxypiperidin-4-yl)aminocarbonate

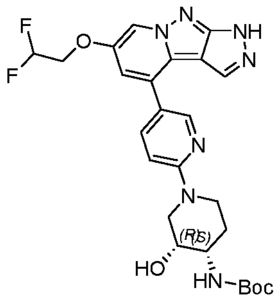

6-(2,2-difluoroetoxy)-4-(6-fluoropyridin-3-yl)-1*H*-pyrazolo[3',4':3,4]pyrazolo[1,5-*a*]pyridine(1 g, 3.0 mmol), *tert*-butyl ((3*R*,4*S*)-3-hydroxypiperidin-4-yl)carbonate (700 mg, 3.3 mmol), DIEA (1900 mg, 15.0 mmol), and DMSO (10 mL) were added to a reaction flask. The mixture was reacted at 90 °C for 40 h. The reaction liquid was cooled to room temperature and poured into water, and the resulting mixture was extracted with dichloromethane. The organic phase was dried, concentrated under reduced pressure, and purified by column chromatography to give a product (1 g), m/z = 530 $[M+1]^+$.

Step E: (3*R*,4*S*)-4-amino-1-(5-(6-(2,2-difluoroethoxy)-1*H*-pyrazolo[3',4':3,4]pyrazolo[1,5-*a*]pyridin-4-yl)py ridin-2-yl)-3 -hydroxypiperidine hydrochloride

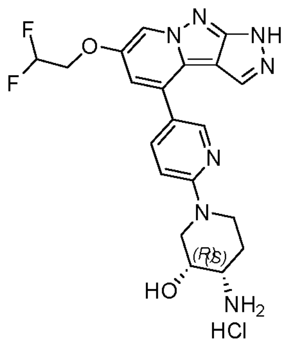

*Tert*-butyl

((3*R*,4*S*)-1-(5-(6-(2,2-difluoroethoxy)-1*H*-pyrazolo[3',4':3,4]pyrazolo[1,5-*a*]pyridin-4-yl)pyridin-2-yl)-3-hydroxypiperidin-4-yl)aminocarbonate (1 g, 1.9 mmol) and a 4 N hydrochloric acid/dioxane solution (20 mL) were added to a reaction flask. The mixture was reacted at 25 °C for 1 h. After the reaction was completed, the reaction liquid was concentrated under reduced pressure to dryness to give a product (1.1 g), m/z = 430 [M+1]+.

Step F: *N*-((3*R*,4*S*)-1-(5-(6-(2,2-difluoroethoxy)-1*H*-pyrazolo[3',4':3,4]pyrazolo[1,5-*a*]pyridin-4-yl)pyridin-2-yl)-3-hydroxypiperidin-4-yl)-2,6-difluorobenzamide

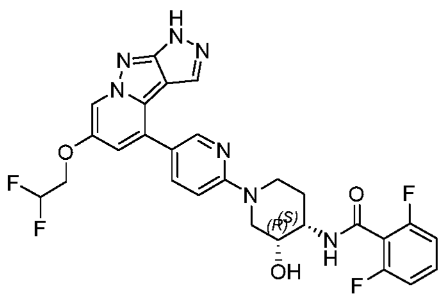

(3*R*,4*S*)-4-amino-1-(5-(6-(2,2-difluoroethoxy)-1*H*-pyrazolo[3',4':3,4]pyrazolo[1,5-*a*]pyridin-4-yl)py ridin-2-yl)-3-hydroxypiperidine hydrochloride (400 mg, 0.9 mmol), 2,6-difluorobenzoic acid (163 mg, 1.0 mmol), DIEA (553 mg, 4.3 mmol), HATU (392 mg, 1.0 mmol), and DMF (5 mL) were reacted at 25 °C for 2 h. The reaction liquid was poured into water, and the resulting mixture was extracted with dichloromethane. The organic phase was dried, concentrated under reduced pressure, and purified by column chromatography to give a product (115.4 mg), m/z = 570 $[M+1]^+$. $^1$H NMR (400 MHz, DMSO-$d_6$) δ 12.71 (s, 1H), 8.57-8.69 (m, 3H), 8.01-8.04 (m, 1H), 7.50-7.62 (m, 1H), 7.34-7.35 (m, 1H), 7.12-7.17 (m, 1H), 7.06-7.08 (m, 3H), 6.35-6.62 (m, 1H), 5.00-5.00 (m, 1H), 4.47-4.56 (m, 2H), 4.20-4.20 (m, 3H), 3.94-3.94 (m, 1H), 3.36-3.44 (m, 1H), 3.24-3.29 (m, 1H), 1.93-1.96 (m, 1H), 1.71-1.72 (m, 1H).

**Example 122: Preparation of compound 269**

*N*-((3*R*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-2,6-difluorobenzenesulfonamide

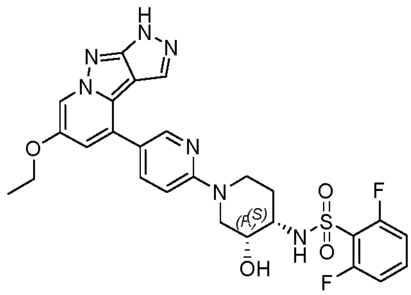

(3*R*,4*S*)-4-amino-1-(5-(6-ethoxy-1*H*-pyrazolo[3',4':3,4]pyrazolo[1,5-*a*]pyridin-4-yl)pyridin-2-yl)-3-hydroxypiperidine hydrochloride (100 mg, 0.2 mmol), 2,6-difluorobenzoyl chloride (43 mg, 0.2 mmol), DIEA (129 mg, 1.0 mmol), and DCM (10 mL) were added to a reaction flask. The mixture was reacted at 25 °C for 1 h. After the reaction was completed, the reaction liquid was poured into water, and the resulting mixture was extracted with dichloromethane. The organic phase was dried, concentrated under reduced pressure, and purified by column chromatography to give a product (15.5 mg), m/z = 570.2 $[M+1]^+$. $^1$HNMR (400 MHz, DMSO-$d_6$): δ 12.64 (brs, 1H), 8.54-8.50 (m, 2H), 8.02-7.96 (m, 2H), 7.70 (t, *J*=6.4Hz, 1H), 7.56 (s, 1H), 7.36-7.22 (m, 3H), 7.00 (d, *J*=9.2Hz, 1H), 4.92 (s, 1H), 4.28-4.08 (m, 4H), 3.72-3.70 (m, 1H), 3.68-3.59 (m, 1H), 3.45-3.41 (m, 1H), 3.20-3.14 (m, 1H), 1.90-1.85 (m, 1H), 1.52-1.48 (m, 1H), 1.39 (t, *J*=6.8Hz, 3H).

**Example 123: Preparation of compound 273**

*N*-((3*R*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-2,5-difluorobenzamide

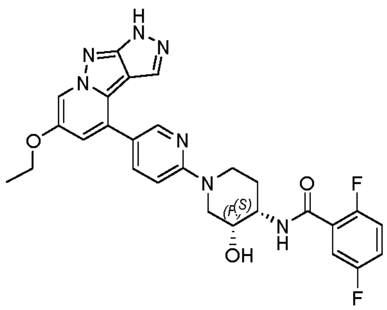

2,5-difluorobenzoic acid (158 mg, 1.0 mmol) and dichloromethane (3 mL) were sequentially added to a reaction flask. The mixture was cooled to 5 °C under an ice bath, and then oxalyl chloride (139 mg, 1.1 mmol) and a catalytic amount of DMF (4 mg, 0.05 mmol) were sequentially added dropwise. After the addition, the reaction liquid was stirred at room temperature for 2 h. The reaction liquid was concentrated and added with DMF (5 mL) for dilution. 1 mL of the

solution was added dropwise to a solution of (3*R*,4*S*)-4-amino-1-(5-(6-ethoxy-1*H*-pyrazolo[3',4':3,4]pyrazolo[1,5-*a*]pyridin-4-yl)pyridin-2-yl)-3-hydroxypiperidine hydrochloride (100 mg, 0.2 mmol) and diisopropylethylamine (129 mg, 1.0 mmol) in DMF (4 mL) under an ice bath. The reaction liquid was stirred at room temperature for 2 h. After the reaction was completed as monitored by LCMS, the reaction liquid was poured into water, and the resulting mixture was extracted with dichloromethane. The organic phase was dried, concentrated under reduced pressure, and purified by column chromatography to give a product (45 mg), m/z = 534 $[M+1]^+$. $^1$H-NMR (400 MHz, DMSO-$d_6$) δ 12.65 (brs, 1H), 8.56 (s, 1H), 8.55 (s, 1H), 7.99-8.07 (m, 2H), 7.59 (s, 1H), 7.49-7.53 (m, 1H), 7.36-7.42 (m, 2H), 7.25 (s, 1H), 7.07 (d, *J* = 9.2 Hz, 1H), 5.11 (d, *J* = 4.4 Hz, 1H), 4.15-4.38 (m, 5H), 3.90-3.99 (m, 1H), 3.28-3.35 (m, 1H), 3.11-3.21 (m, 1H), 1.89-2.01 (m, 1H), 1.67-1.79 (m, 1H), 1.36-1.48 (m, 3H).

**Example 124: Preparation of compound 274**

*N*-((3*R*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-2,3,6-trifluorobenzamide

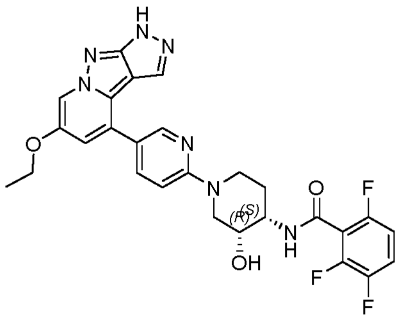

2,3,6-trifluorobenzoic acid (176 mg, 1.0 mmol) and dichloromethane (3 mL) were sequentially added to a reaction flask. The mixture was cooled to 5 °C under an ice bath, and then the oxalyl chloride (139 mg, 1.1 mmol) and a catalytic amount of DMF (4 mg, 0.05 mmol) were sequentially added dropwise. After the addition, the reaction liquid was stirred at room temperature for 2 h. The reaction liquid was concentrated and added with DMF (5 mL) for dilution. 1 mL of the solution was added dropwise to a solution of (3*R*,4*S*)-4-amino-1-(5-(6-ethoxy-1*H*-pyrazolo[3',4':3,4]pyrazolo[1,5-*a*]pyridin-4-yl)pyridin-2-yl)-3-hydroxypiperidine hydrochloride (100 mg, 0.2 mmol) and diisopropylethylamine (129 mg, 1.0 mmol) in DMF (4 mL) under an ice bath. The reaction liquid was stirred at room temperature for 2 h. After the reaction was completed as monitored by LCMS, the reaction liquid was poured into water, and the resulting mixture was extracted with dichloromethane. The organic phase was dried, concentrated under reduced pressure, and purified by column chromatography to give a product (35 mg), m/z = 552 $[M+1]^+$. $^1$H-NMR (400 MHz, DMSO-$d_6$) δ 12.65 (brs, 1H), 8.75 (d, *J* = 8.0 Hz, 1H), 8.56 (s, 1H), 8.51 (s, 1H), 8.02 (d, *J* = 8.0 Hz, 1H), 7.50-7.61 (m, 2H), 7.15-7.26 (m, 2H), 7.07 (d, *J* = 8.0 Hz, 1H), 5.11 (d, *J* = 4.4 Hz, 1H), 4.15-4.38 (m, 5H), 3.90-3.99 (m, 1H), 3.21-3.41(m, 2H), 1.89-2.01 (m, 1H), 1.67-1.79 (m, 1H), 1.38-1.42 (m, 3H).

**Example 125: Preparation of compound 275**

*N*-((3*R*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-2-chloro-6-fluorobenzamide

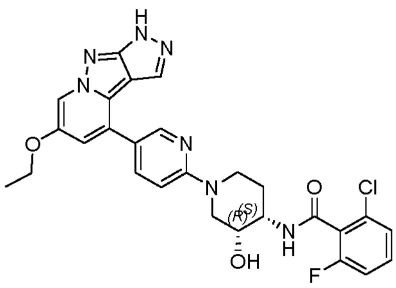

(3*R*,4*S*)-4-amino-1-(5-(6-ethoxy-1*H*-pyrazolo[3',4':3,4]pyrazolo[1,5-*a*]pyridin-4-yl)pyridin-2-yl)-3-hydroxypiperidine hydrochloride (100 mg, 0.2 mmol), DMF (2 mL), and diisopropylethylamine (129 mg, 1.0 mmol) were sequentially added to a reaction flask. The mixture was cooled to 5 °C under an ice bath, and then a solution of 2-chloro-6-fluorobenzoyl chloride (39 mg, 0.2 mmol) in DMF (0.2 mL) was added dropwise. The resulting mixture was stirred at room temperature for 2 h. After the reaction was completed as monitored by LCMS, the reaction liquid was poured into water, and the resulting mixture was extracted with dichloromethane. The organic phase was dried, concentrated under reduced pressure, and purified by column chromatography to give a product (47 mg), m/z = 550 $[M+1]^+$. $^1$H-NMR (400 MHz, DMSO-$d_6$) δ 12.65 (brs, 1H), 8.60 (d, *J* = 8.0 Hz, 1H), 8.56 (s, 1H), 8.51 (s, 1H), 8.60 (d, *J* = 8.0 Hz, 1H), 7.58 (s, 1H), 7.45-7.48 (m, 1H), 7.36 (d, *J* = 8.0 Hz, 1H), 7.21-7.28 (m, 2H), 7.07 (d, *J* = 9.2 Hz, 1H), 5.11 (d, *J* = 4.4 Hz, 1H), 4.15-4.38 (m, 5H), 3.90-3.99 (m, 1H), 3.21-3.35(m, 1H), 3.31-3.47 (m, 1H), 1.89-2.01 (m, 1H), 1.67-1.79 (m, 1H), 1.36-1.48 (m, 3H).

**Example 126: Preparation of compound 276**

2-chloro-*N*-((3*R*,4*S*)-1-(5-(6-ethoxy-1*H*-pyrazolo[3',4':3,4]pyrazolo[1,5-*a*]pyridin-4-yl)pyridin-2-yl) -3-hydroxypiperidin-4-yl)-5-fluorobenzamide

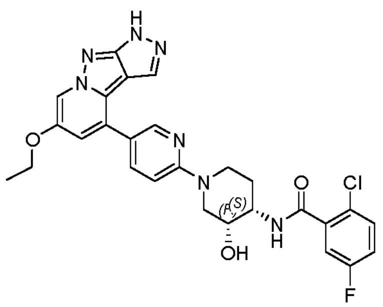

(3*R*,4*S*)-4-amino-1-(5-(6-ethoxy-1*H*-pyrazolo[3',4':3,4]pyrazolo[1,5-*a*]pyridin-4-yl)pyridin-2-yl)-3-hydroxypiperidine hydrochloride (100 mg, 0.25 mmol), 5-chloro-2-fluorobenzoyl chloride (52 mg, 0.25 mmol), DIEA (194 mg, 1.80 mmol), and DMF (3 mL) were added to a reaction flask. The mixture was reacted at 25 °C for 1 h. The reaction was quenched by adding water and stirring. The reaction liquid was extracted with ethyl acetate. The organic phase was dried over anhydrous sodium sulfate, concentrated under reduced pressure, and separated by column chromatography to give a product (18 mg), m/z = 550 $[M+1]^+$. $^1$HNMR (400 MHz, DMSO-$d_6$) δ 12.65 (brs, 1H), 8.55 (s, 1H), 8.51 (s, 1H), 8.33 (d, *J*=7.6Hz, 1H), 8.00 (d, J=8.8Hz, 1H), 7.58-7.53 (m, 2H), 7.42-7.30 (m, 2H), 7.25 (s, 1H), 7.06 (d, *J*=8.0Hz, 1H), 4.96 (s, 1H), 4.33-4.13 (m, 5H), 3.97-3.93 (m, 1H), 3.42-3.40 (m, 1H), 3.24-3.18 (m, 1H), 1.99-1.89 (m, 1H), 1.75-1.66 (m, 1H), 1.39 (t, *J*=6.8Hz, 3H).

**Example 127: Preparation of compound 277**

*N*-((3*R*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-5-fluoro-2-methylbenzamide

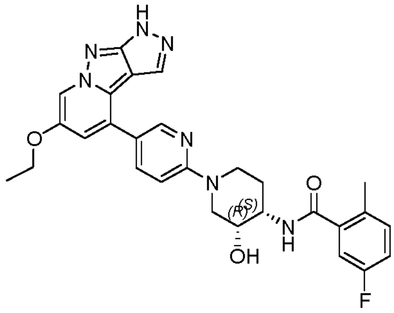

(3*R*,4*S*)-4-amino-1-(5-(6-ethoxy-1*H*-pyrazolo[3',4':3,4]pyrazolo[1,5-*a*]pyridin-4-yl)pyridin-2-yl)-3-hydroxypiperidine hydrochloride (100 mg, 0.25 mmol), 5-fluoro-2-methylbenzoyl chloride (44 mg, 0.25 mmol), DIEA (194 mg, 1.80 mmol), and DMF (3 mL) were added to a reaction flask. The mixture was reacted at 25 °C for 1 h. The reaction was quenched by adding water and stirring. The reaction liquid was extracted with ethyl acetate. The organic phase was dried over anhydrous sodium sulfate, concentrated under reduced pressure, and separated by column chromatography to give a product (30 mg), m/z = 530 $[M+1]^+$. $^1$HNMR (400 MHz, DMSO-$d_6$) δ 12.65 (brs, 1H), 8.55 (s, 1H), 8.51 (s, 1H), 8.09 (d, *J*=7.6Hz, 1H), 7.99 (d, *J*=8.8Hz, 1H), 7.58 (s, 1H), 7.30-7.15 (m, 4H), 7.06 (d, *J*=9.2Hz, 1H), 4.95 (s, 1H), 4.34-4.14 (m, 5H), 3.97-3.93 (m, 1H), 3.42-3.40 (m, 1H), 3.24-3.18 (m, 1H), 2.33 (s, 3H), 1.99-1.90 (m, 1H), 1.75-1.66 (m, 1H), 1.39 (t, *J*=6.8Hz, 3H).

**Example 128: Preparation of compound 278**

2-chloro-*N*-((3*R*,4*S*)-1-(5-(6-ethoxy-1*H*-pyrazolo[3',4':3,4]pyrazolo[1,5-*a*]pyridin-4-yl)pyridin-2-yl) -3-hydroxypiperidin-4-yl)-6-methylbenzamide

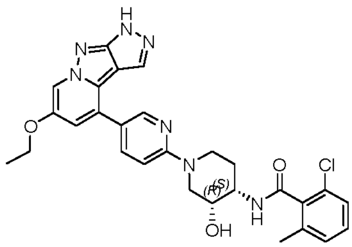

(3*R*,4*S*)-4-amino-1-(5-(6-ethoxy-1*H*-pyrazolo[3',4':3,4]pyrazolo[1,5-*a*]pyridin-4-yl)pyridin-2-yl)-3-hydroxypiperidine hydrochloride (100 mg, 0.25 mmol), 2-chloro-6-methylbenzoic acid (42 mg, 0.25 mmol), DIEA (194 mg, 1.80 mmol), HATU (95 mg, 0.25 mmol), and DMF (3 mL) were added to a reaction flask. The mixture was reacted at 25 °C for 1 h. The reaction was quenched by adding water and stirring. The reaction liquid was extracted with ethyl acetate. The organic phase was dried over anhydrous sodium sulfate, concentrated under reduced pressure, and separated by column chromatography to give a product (28 mg), m/z = 546 $[M+1]^+$. $^1$HNMR (400 MHz, DMSO-$d_6$) δ 12.65 (brs, 1H), 8.55 (s, 1H), 8.51 (s, 1H), 8.32 (d, *J*=8.0Hz, 1H), 7.99 (d, *J*=8.8Hz, 1H), 7.58 (s, 1H), 7.30-7.20 (m, 4H), 7.05 (d, *J*=9.2Hz, 1H), 4.89 (s, 1H), 4.24-4.13 (m, 5H), 3.97-3.93 (m, 1H), 3.42-3.40 (m, 1H), 3.24-3.18 (m, 1H), 2.33 (s, 3H), 1.99-1.90 (m, 1H), 1.75-1.68 (m, 1H), 1.39 (t, *J*=6.8Hz, 3H).

**Example 129: Preparation of compound 279**

*N*-((3*R*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-2-(trifluoromethyl)benzamide

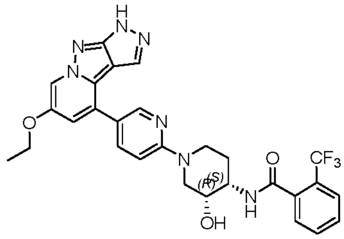

(3*R*,4*S*)-4-amino-1-(5-(6-ethoxy-1*H*-pyrazolo[3',4':3,4]pyrazolo[1,5-*a*]pyridin-4-yl)pyridin-2-yl)-3-hydroxypiperidine hydrochloride (100 mg, 0.2 mmol), 2-trifluoromethylbenzoyl chloride (41 mg, 0.2 mmol), DIEA (129 mg, 1.0 mmol), and DCM (10 mL) were added to a reaction flask. The mixture was reacted at 25 °C for 1 h. After the reaction was completed, the reaction liquid was poured into water, and the resulting mixture was extracted with dichloromethane. The organic phase was dried, concentrated under reduced pressure, and purified by column chromatography to give a product (28.2 mg) as a light yellow solid, m/z = 566.2 $[M+1]^+$. $^1$HNMR (400 MHz, DMSO-$d_6$) $\delta$ 12.65 (brs, 1H), 8.56 (s, 1H), 8.51 (s, 1H), 8.27 (d, *J*=8.0Hz, 1H), 8.01 (d, *J*=8.8Hz, 1H), 7.79-7.41 (m, 2H), 7.66-7.56 (m, 3H), 7.21 (s, 1H), 7.06 (d, *J*=8.0Hz, 1H), 4.93 (s, 1H), 4.42-4.12 (m, 5H), 4.97-4.93 (m, 1H), 3.42-3.40 (m, 1H), 3.28-3.19 (m, 1H), 1.98-1.89 (m, 1H), 1.75-1.68 (m, 1H), 1.39 (t, *J*=6.8Hz, 3H).

**Example 130: Preparation of compound 280**

2-cyano-*N*-((3*R*,4*S*)-1-(5-(6-ethoxy-1*H*-pyrazolo[3',4':3,4]pyrazolo[1,5-*a*]pyridin-4-yl)pyridin-2-yl) -3-hydroxypiperidin-4-yl)benzamide

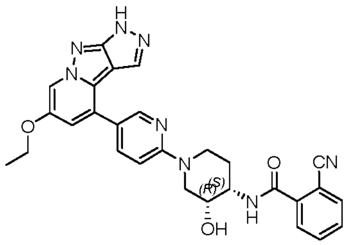

(3*R*,4*S*)-4-amino-1-(5-(6-ethoxy-1*H*-pyrazolo[3',4':3,4]pyrazolo[1,5-*a*]pyridin-4-yl)pyridin-2-yl)-3-hydroxypiperidine hydrochloride (100 mg, 0.2 mmol), 2-cyanobenzoyl chloride (33 mg, 0.2 mmol), DIEA (129 mg, 1.0 mmol), and DCM (10 mL) were added to a reaction flask. The mixture was reacted at 25 °C for 1 h. After the reaction was completed, the reaction liquid was poured into water, and the resulting mixture was extracted with dichloromethane. The organic phase was dried, concentrated under reduced pressure, and purified by column chromatography to give a product (12.4 mg) as a light yellow solid, m/z = 523.2 $[M+1]^+$. $^1$HNMR (400 MHz, DMSO-$d_6$) $\delta$ 12.65 (brs, 1H), 10.27 (s, 1H), 8.58-8.51 (m, 2H), 8.20 (d, *J*=6.8Hz, 1H), 8.01 (d, *J*=8.8Hz, 1H), 7.85-7.72 (m, 3H), 7.59 (s, 1H), 7.26 (s, 1H), 7.09 (d, *J*=8.4Hz, 1H), 6.05 (s, 1H), 4.85-4.51 (m, 2H), 4.27-4.15 (m, 4H), 3.22-3.20 (m, 1H), 3.08-2.98 (m, 2H), 1.75-1.68 (m, 1H), 1.39 (t, *J*=6.8Hz, 3H).

**Example 131: Preparation of compound 281**

*N*-((3*R*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-1-(trifluoromethyl)cyclobutane-1-carboxamide

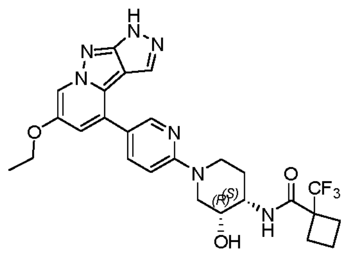

(3*R*,4*S*)-4-amino-1-5-(6-ethoxy-1*H*-pyrazolo[3',4':3,4]pyrazolo[1,5-*a*]pyridin-4-yl)pyridin-2-yl)-3-h ydroxypiperidine hydrochloride (120.6 mg, 0.24 mmol), DMF (2 mL), DIEA (154.8 mg, 1.2 mmol), and 1-trifluorocyclobutane-1-carboxylic acid (33.6 mg, 0.2 mmol) were sequentially added to a reaction flask. HATU (114 mg, 0.3 mmol) was added at room temperature, and the mixture was reacted at room temperature for 1 h. After the reaction was completed as monitored by LCMS, the reaction liquid was poured into water, and the resulting mixture was extracted with dichloromethane. The organic phase was dried, concentrated under reduced pressure, and purified by column chromatography to give a product (55 mg), m/z = 544.1 $[M+1]^+$. $^1H$ NMR (400 MHz, DMSO-$d_6$) δ 12.65 (s, 1H), 8.55-8.50 (m, 2H), 8.01-7.98 (m, 1H), 7.58 (s, 1H),7.50-7.48 (d, 1H), 7.24-7.23 (m, 1H), 7.07-7.05 (m,1H), 5.01 (s, 1H), 4.42-4.32 (m, 2H), 4.20-4.15 (m, 3H), 4.01-3.98 (m, 1H), 3.88 (s, 1H), 3.35-3.05 (m, 2H), 2.37-2.34 (m, 2H), 1.96-1.83 (m, 4H), 1.61-1.58 (m, 1H), 1.43-1.38 (t, 3H).

**Example 132: Preparation of compound 282**

*N*-((3*R*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-1-fluorocyclopropane-1-carboxamide

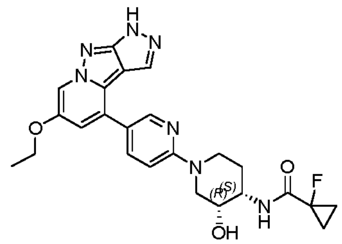

(3*R*,4*S*)-4-amino-1-5-(6-ethoxy-1*H*-pyrazolo[3',4':3,4]pyrazolo[1,5-*a*]pyridin-4-yl)pyridin-2-yl)-3-h ydroxypiperidine hydrochloride (120.6 mg, 0.24 mmol), DMF (2 mL), DIEA (154.8 mg, 1.2 mmol), and 1-fluorocyclopropane-1-carboxylic acid (20.8 mg, 0.2 mmol) were sequentially added to a reaction flask. HATU (114 mg, 0.3 mmol) was added at room temperature, and the mixture was reacted at room temperature for 1 h. After the reaction was completed as monitored by LCMS, the reaction liquid was poured into water, and the resulting mixture was extracted with ethyl acetate. The organic phase was dried, concentrated under reduced pressure, and purified by column chromatography to give a product (6 mg), m/z = 480.1 $[M+1]^+$. $^1H$ NMR (400 MHz, DMSO-$d_6$) δ 12.65 (s, 1H), 8.55-8.50 (m, 2H), 8.01-7.98 (m, 1H), 7.58 (s, 1H), 7.50-7.48 (d, 1H), 7.24-7.23 (m, 1H), 7.07-7.05 (m,1H), 5.01 (s, 1H), 4.20-4.15 (m, 3H), 3.60-3.58 (m, 1H), 3.37-3.04 (m, 4H), 1.70-1.85 (m, 2H), 1.43-1.38 (t, 3H), 1.00-1.25(m, 4H).

**Example 133: Preparation of compound 283**

*N*-((3*R*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-2,3-dimethylbutanamide

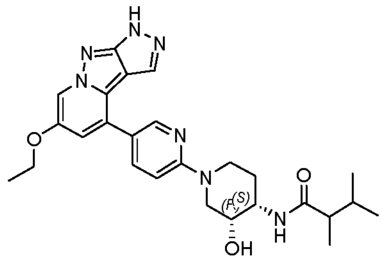

(3*R*,4*S*)-4-amino-1-5-(6-ethoxy-1*H*-pyrazolo[3',4':3,4]pyrazolo[1,5-*a*]pyridin-4-yl)pyridin-2-yl)-3-h ydroxypiperidine hydrochloride (238 mg, 0.51 mmol), 2,3-dimethylbutanoic acid (60 mg, 0.51 mmol), DIEA (329 mg, 2.55 mmol), HATU (232 mg, 0.61 mmol), and DMF (5 mL) were added to a reaction flask. The mixture was reacted at 25 °C for 1 h. After the reaction was completed as monitored by LCMS, the reaction liquid was poured into water, and the resulting mixture was extracted with dichloromethane. The organic phase was dried, concentrated under reduced pressure, and purified by column chromatography to give a product (25 mg), m/z = 492.3 $[M+1]^+$. $^1$HNMR (400 MHz, DMSO-$d_6$) δ 12.64 (brs, 1H), 8.50-8.54 (m, 2H), 7.98-8.01 (m, 1H), 7.58 (s, 1H), 7.48-7.49 (m, 1H), 7.24 (m, 1H), 7.00-7.02 (m, 1H), 4.87-4.93 (m, 1H), 4.16-4.18 (m, 4H), 3.80-3.94 (m, 2H), 3.10-3.20 (m, 1H), 2.00-2.10 (m, 1H), 1.50-1.90 (m, 3H), 1.40 (t, *J*=6.8Hz, 3H), 0.84-0.98 (m, 10H).

**Example 134: Preparation of compound 284**

*N*-((3*R*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-3-(trifluoromethyl)picolinamide

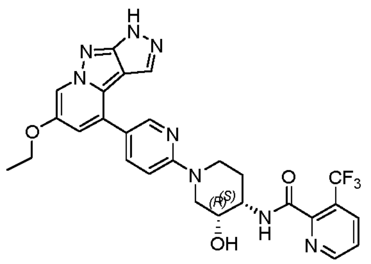

(3*R*,4*S*)-4-amino-1-5-(6-ethoxy-1*H*-pyrazolo[3',4':3,4]pyrazolo[1,5-*a*]pyridin-4-yl)pyridin-2-yl)-3-h ydroxypiperidine hydrochloride (150 mg, 0.30 mmol), DMF (2 mL), DIEA (154.8 mg, 1.2 mmol), and 3-trifluoromethyl-2-carboxylic acid pyridine (38 mg, 0.2 mmol) were sequentially added to a reaction flask. HATU (114 mg, 0.3 mmol) was added at room temperature, and the mixture was reacted at room temperature for 1 h. After the reaction was completed as monitored by LCMS, the reaction liquid was poured into water, and the resulting mixture was extracted with ethyl acetate. The organic phase was dried, concentrated under reduced pressure, and purified by column chromatography to give a product (6 mg), m/z = 567 $[M+1]^+$. $^1$H NMR (400 MHz, DMSO-$d_6$) δ 12.65 (s, 1H), 8.55-8.50 (m, 3H), 8.01-7.98 (m, 2H), 7.58 (s, 1H),7.50-7.48 (d, 2H), 7.24-7.23 (m, 1H), 7.07-7.05 (m,1H), 5.01 (s, 1H), 4.20-4.15 (m, 3H), 4.01-3.98 (m, 1H), 3.88 (s, 1H), 3.35-3.05 (m, 2H), 2.37-2.34 (m, 2H), 1.61-1.58 (m, 1H), 1.43-1.38 (t, 3H).

**Example 135: Preparation of compound 285**

*N*-((3*R*,4*S*)-1-(5-(6-ethoxy-1*H*-pyrazolo[3',4':3,4]pyrazolo[1,5-*a*]pyridin-4-yl)pyridin-2-yl)piperidin -3-hydroxypiperidin-4-yl)-3-chloro-5-fluoropicolinamide

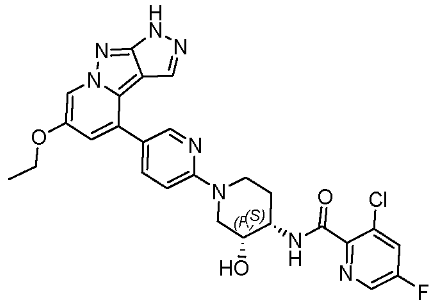

(3*R*,4*S*)-4-amino-1-5-(6-ethoxy-1*H*-pyrazolo[3',4':3,4]pyrazolo[1,5-*a*]pyridin-4-yl)pyridin-2-yl)-3-h ydroxypiperidine hydrochloride (100 mg, 0.2 mmol), 3-chloro-5-fluoropicolinic acid (32 mg, 0.2 mmol), DIEA (129 mg, 1.0 mmol), HATU (76 mg, 0.2 mmol), and DMF (3 mL) were added to a reaction flask. The mixture was reacted at 25 °C for 1 h. After the reaction was completed as monitored by LCMS, the reaction liquid was poured into water, and the resulting mixture was extracted with dichloromethane. The organic phase was dried, concentrated under reduced pressure, and purified by column chromatography to give a product (41.6 mg), m/z = 551 $[M+1]^+$. $^1$HNMR (400 MHz, DMSO-$d_6$) δ 12.65 (brs, 1H), 8.63 (s, 1H), 8.55 (s, 1H), 8.51-8.49 (m, 2H), 8.32 (d, *J*=8.0 Hz, 1H), 8.00 (d, *J*=8.8 Hz, 1H), 7.92-7.87 (m, 1H), 7.73-7.69 (m, 1H), 7.60 (s, 1H), 7.25 (s, 1H), 7.07 (d, *J*=9.2 Hz, 1H), 5.25 (s, 1H), 4.45-4.36 (m, 2H), 4.20-4.14 (m, 3H), 3.95-3.93 (m, 1H), 3.33-3.30 (m, 1H), 3.15-3.10 (m, 1H), 1.95-1.92 (m, 1H), 1.78-1.74 (m, 1H), 1.38 (t, *J*=6.8 Hz, 3H).

**Example 136: Preparation of compound 286**

*N*-((3*R*,4*S*)-1-(5-(6-ethoxy-1*H*-pyrazolo[3',4':3,4]pyrazolo[1,5-*a*]pyridin-4-yl)pyridin-2-yl)piperidin -3-hydroxypiperidin-4-yl)-3-fluoropicolinamide

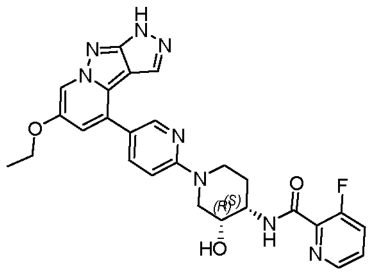

(3*R*,4*S*)-4-amino-1-5-(6-ethoxy-1*H*-pyrazolo[3',4':3,4]pyrazolo[1,5-*a*]pyridin-4-yl)pyridin-2-yl)-3-h ydroxypiperidine hydrochloride (100 mg, 0.2 mmol), 3-fluoropicolinic acid (32 mg, 0.2 mmol), DIEA (129 mg, 1.0 mmol), HATU (76 mg, 0.2 mmol), and DMF (3 mL) were added to a reaction flask. The mixture was reacted at 25 °C for 1 h. After the reaction was completed as monitored by LCMS, the reaction liquid was poured into water, and the resulting mixture was extracted with dichloromethane. The organic phase was dried, concentrated under reduced pressure, and purified by column chromatography to give a product (39.9 mg), m/z = 517 $[M+1]^+$. $^1$HNMR (400 MHz, DMSO-$d_6$) δ 12.65 (brs, 1H), 8.63 (s, 1H), 8.55 (s, 1H), 8.51 (s, 1H), 8.35 (d, *J*=8.0 Hz, 1H), 8.20 (d, *J*=8.8 Hz, 1H), 8.00 (d, *J*=8.8 Hz, 1H), 7.60 (s, 1H), 7.24 (s, 1H), 7.07 (d, *J*=9.2 Hz, 1H), 5.14 (s, 1H), 4.34-4.27 (m, 2H), 4.20-4.15 (m, 2H), 3.95-3.93 (m, 1H), 3.42-3.40 (m, 1H), 3.20-3.15 (m, 1H), 1.99-1.90 (m, 1H), 1.78-1.70 (m, 1H), 1.39 (t, *J*=6.8 Hz, 3H).

**Example 137: Preparation of compound 291**

*N*-(1-(5-(6-ethoxy-1*H*-pyrazolo[3',4':3,4]pyrazolo[1,5-*a*]pyridin-4-yl)pyridin-2-yl)-4-(hydroxymeth yl)piperidin-4-yl)-2,5-difluorobenzamide

Step A: ethyl 4-(2,5-difluorobenzamido)-1-(5-(6-ethoxy-1*H*-pyrazolo[3',4':3,4]pyrazolo[1,5-*a*]pyridin-4-yl)pyridi n-2-yl)piperidine-4-carboxylate

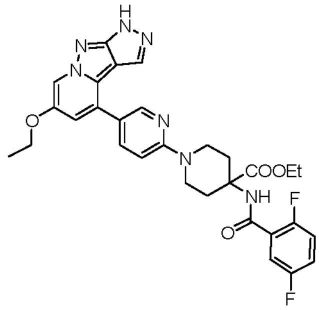

Ethyl

4-amino-1-(5-(6-ethoxy-1*H*-pyrazolo[3',4':3,4]pyrazolo[1,5-*a*]pyridin-4-yl)pyridin-2-yl)piperidine-4-carboxylate (120 mg, 0.26 mmol), 2,5-difluorobenzoic acid (38 mg, 0.24 mmol), DIEA (100 mg, 0.78 mmol), HATU (108 mg, 0.28 mmol), and DMF (2 mL) were added to a reaction flask. The mixture was reacted at 25 °C for 12 h. After the reaction was completed as monitored by LCMS, the reaction was quenched by adding water and stirring. The reaction liquid was extracted with ethyl acetate. The organic phase was dried over anhydrous sodium sulfate, concentrated under reduced pressure, and separated by column chromatography to give a product (100 mg), m/z = 590 $[M+1]^+$.

Step B: *N*-(1-(5-(6-ethoxy-1*H*-pyrazolo[3',4':3,4]pyrazolo[1,5-*a*]pyridin-4-yl)pyridin-2-yl)-4-(hydroxymeth yl)piperidin-4-yl)-2,5-difluorobenzamide

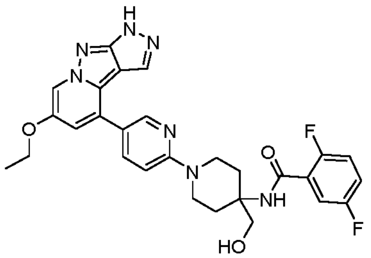

Ethyl

4-(2,5-difluorobenzamide)-1-(5-(6-ethoxy-1*H*-pyrazolo[3',4':3,4]pyrazolo[1,5-*a*]pyridin-4-yl)pyridi n-2-yl)piperidine-4-carboxylate (30 mg, 0.05 mmol) and 2 mL of THF were added to a reaction flask, and $LiAlH_4$ (1.9 mg, 0.05 mmol) was added at 0 °C. The mixture was reacted for 1 h. After the reaction was completed as monitored by LCMS, the reaction was quenched by adding water and stirring. The reaction liquid was extracted with ethyl acetate. The organic phase was dried over anhydrous sodium sulfate, concentrated under reduced pressure, and separated by column chromatography to give a product (20 mg), m/z = 548 $[M+1]^+$. $^1H$ NMR (400 MHz, DMSO-$d_6$) $\delta$ 12.61 (s, 1H), 8.55 (d, *J* = 2.4 Hz, 1H), 8.48 (d, *J* = 2.4 Hz, 1H), 8.32 (s, 1H), 8.04 (dd, *J* = 8.8, 2.6 Hz, 1H), 7.60 (s, 1H), 7.50 (s, 1H), 7.27 (d, *J* = 2.4 Hz, 1H), 7.25-7.18 (m, 3H), 4.85 (t, *J* = 5.6 Hz, 1H), 4.20 (d, *J* = 13.4 Hz, 2H), 4.15 (q, *J* = 7.6 Hz, 2H), 3.58 (d, *J* = 5.6 Hz, 2H), 3.15 (t, *J* = 12.4 Hz, 2H), 2.20 (d, *J* = 13.4 Hz, 2H), 1.68 (td, *J* = 13.4, 4.4 Hz, 2H), 1.38 (t, *J* = 6.8 Hz, 3H).

**Example 138: Preparation of compound 292**

*N*-((3*R*,4*S*)-1-(5-(6-(2,2-difluoroethoxy)-1*H*-pyrazolo[3',4':3,4]pyrazolo[1,5-*a*]pyridin-4-yl)pyridin-2-yl)piperidin-3-hydroxypiperidin-4-yl)-2-chloro-6-fluorobenzamide

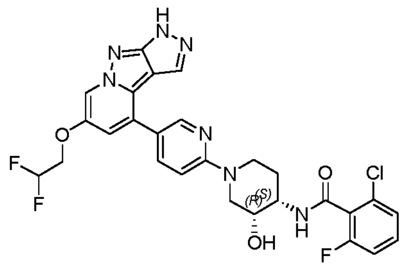

(3*R*,4*S*)-4-amino-1-(5-(6-(2,2-difluoroethoxy)-1*H*-pyrazolo[3',4':3,4]pyrazolo[1,5-*a*]pyridin-4-yl)py ridin-2-yl)piperidin-3-ol hydrochloride (100 mg, 0.2 mmol), DCM (3 mL), DIEA (138 mg, 1.0 mmol), and 2-chloro-6-fluorobenzoyl chloride (41 mg, 0.2 mmol) were added to a reaction flask. The mixture was reacted at 0 °C for 15 min. After the reaction was completed as monitored by LCMS, the reaction liquid was poured into water, and the resulting mixture was extracted with dichloromethane. The organic phase was dried, concentrated under reduced pressure, and purified by column chromatography to give a product (43.5 mg), m/z = 586 $[M+1]^+$. $^1H$ NMR (400 MHz, DMSO-$d_6$) δ 12.70 (s, 1H), 8.57-8.69 (m, 3H), 8.01-8.02 (m, 1H), 7.61-7.61 (m, 1H), 7.45-7.47 (m, 1H), 7.28-7.37 (m, 3H), 7.06-7.06 (m, 1H), 6.34-6.62 (m, 1H), 4.94-4.95 (m, 1H), 4.48-4.52 (m, 2H), 4.19-4.20 (m, 3H), 3.93-3.93 (m, 1H), 3.42-3.45 (m, 1H), 3.27-3.34 (m, 1H), 1.94-1.97 (m, 1H), 1.70-7.73 (m, 1H).

**Example 139: Preparation of compound 293**

*N*-((3*R*,4*S*)-1-(5-(6-(2,2-difluoroethoxy)-1*H*-pyrazolo[3',4':3,4]pyrazolo[1,5-*a*]pyridin-4-yl)pyridin-2-yl)piperidin-3-hydroxypiperidin-4-yl)-2-chloro-5-fluorobenzamide

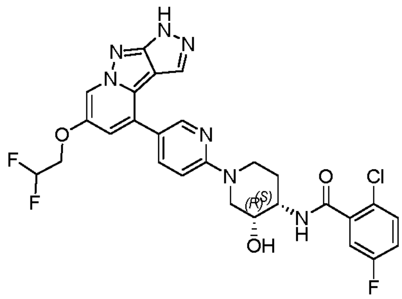

(3*R*,4*S*)-4-amino-1-(5-(6-(2,2-difluoroethoxy)-1*H*-pyrazolo[3',4':3,4]pyrazolo[1,5-*a*]pyridin-4-yl)py ridin-2-yl)piperidin-3-ol hydrochloride (100 mg, 0.2 mmol), DCM (3 mL), DIEA (138 mg, 1.0 mmol), and 2-chloro-5-fluorobenzoyl chloride (41 mg, 0.2 mmol) were added to a reaction flask. The mixture was reacted at 0 °C for 15 min. After the reaction was completed as monitored by LCMS, the reaction liquid was poured into water, and the resulting mixture was extracted with dichloromethane. The organic phase was dried, concentrated under reduced pressure, and purified by column chromatography to give a product (11.0 mg), m/z = 586 $[M+1]^+$. $^1H$ NMR (400 MHz, DMSO-$d_6$) δ 12.70 (s, 1H), 8.68-8.69 (m, 1H), 8.57-8.58 (m, 1H), 8.34-8.36 (m, 1H), 8.01-8.04 (m, 1H), 7.53-7.61 (m, 1H), 7.39-7.41 (m, 1H), 7.33-7.35 (m, 3H), 7.07-7.09 (m, 1H), 6.35-6.62 (m, 1H), 4.97-4.98 (m, 1H), 4.47-4.56 (m, 2H), 4.14-4.25 (m, 3H), 3.96-3.96 (m, 1H), 3.40-3.40 (m, 1H), 3.22-3.37 (m, 1H), 1.94-1.94 (m, 1H), 1.67-1.71 (m, 1H).

**Example 140: Preparation of compound 294**

*N*-((3*S*,4*S*)-1-(5-(6-ethoxy-1*H*-pyrazolo[3',4':3,4]pyrazolo[1,5-*a*]pyridin-4-yl)pyridin-2-yl)-3-hydro xypiperidin-4-yl)-2,6-difluorobenzamide

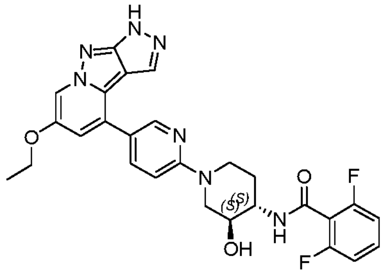

(3*S*,4*S*)-4-amino-1-5-(6-ethoxy-1*H*-pyrazolo[3',4':3,4]pyrazolo[1,5-*a*]pyridin-4-yl)pyridin-2-yl)-3-h ydroxypiperidine hydrochloride (200 mg, 0.51 mmol), DMF (2 mL), DIEA (197 mg, 1.5 mmol), and 2,6-difluorobenzoic acid (88.4 mg, 0.56 mmol) were sequentially added to a reaction flask. HATU (290 mg, 0.76 mmol) was added at room temperature, and the mixture was reacted at room temperature for 1 h. After the reaction was completed as monitored by LCMS, the reaction liquid was poured into water, and the resulting mixture was extracted with ethyl acetate. The organic phase was dried, concentrated under reduced pressure, and purified by column chromatography to give a product (7 mg), m/z = 534 $[M+1]^+$. $^1H$ NMR (400 MHz, DMSO-$d_6$) δ 12.64 (s, 1H), 8.61-8.50 (m, 3H), 8.02-7.99 (m, 1H), 7.60 (s, 1H), 7.52-7.47 (m, 1H), 7.24 (s, 1H), 7.17-7.12 (m, 2H), 7.07-7.05 (d, 1H), 4.97 (s, 1H), 4.24-4.15 (m, 5H), 3.93 (s, 1H), 3.44-3.43 (d, *J* = 1.6 Hz, 1H), 3.28-3.26 (d, 1H), 1.95-1.93 (m, 1H), 1.71-1.68 (m, 1H), 1.43 (t, 3H).

**Example 141: Preparation of compound 295**

*N*-((3*S*,4*S*)-1-(5-(6-(2-fluoroethoxy)-1*H*-pyrazolo[3',4':3,4]pyrazolo[1,5-*a*]pyridin-4-yl)pyridin-2-yl )-3-hydroxypiperidin-4-yl)-2,6-difluorobenzamide

Step A: tert-butyl ((3*S*,4*S*)-1-(5-(6-(2-fluoroethoxy)-1*H*-pyrazolo[3',4':3,4]pyrazolo[1,5-*a*]pyridin-4-yl)pyridin-2-yl)-3 -hydroxypiperidin-4-yl)aminocarbonate

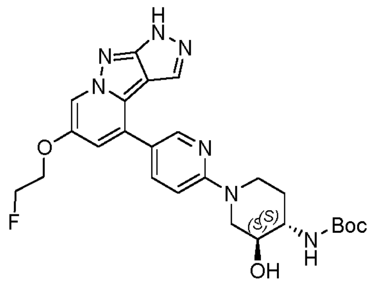

6-(2-fluoroethoxy)-4-(6-fluoropyridin-3-yl)-1*H*-pyrazolo[3',4':3,4]pyrazolo[1,5-*a*]pyridine (410 mg, 1.3 mmol), *tert*-butyl ((3*S*,4*S*)-3-hydroxypiperidin-4-yl)carbonate (309 mg, 1.4 mmol), DIEA (840 mg, 6.5 mmol), and DMSO (5 mL) were added to a reaction flask. The mixture was reacted at 90 °C for 40 h. The reaction liquid was cooled to room temperature and poured into water, and the resulting mixture was extracted with dichloromethane. The organic phase was dried, concentrated under reduced pressure, and purified by column chromatography to give a product (430 mg), m/z = 512 $[M+1]^+$.

Step B: ((3*S*,4*S*)-1-(5-(6-(2-fluoroethoxy)-1*H*-pyrazolo[3',4':3,4]pyrazolo[1,5-*a*]pyridin-4-yl)pyridin-2-yl)-3 -hydroxypiperidin-4-yl)amine hydrochloride

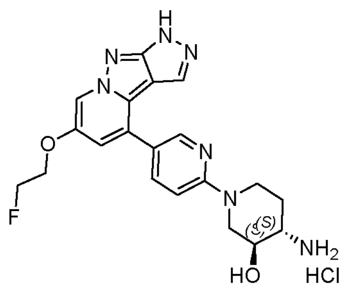

*Tert*-butyl

((3*S*,4*S*)-1-(5-(6-(2-fluoroethoxy)-1*H*-pyrazolo[3',4':3,4]pyrazolo[1,5-*a*]pyridin-4-yl)pyridin-2-yl)-3 -hydroxypiperidin-4-yl)aminocarbonate (430 mg, 0.8 mmol) and a 4 N hydrochloric acid/dioxane solution (8 mL) were added to a reaction flask. The mixture was reacted at 25 °C for 0.5 h. After the reaction was completed, the reaction liquid was concentrated under reduced pressure to dryness to give a product (320 mg), m/z = 412 $[M+1]^+$.

Step C: *N*-((3*S*,4*S*)-1-(5-(6-(2-fluoroethoxy)-1*H*-pyrazolo[3',4':3,4]pyrazolo[1,5-*a*]pyridin-4-yl)pyridin-2-yl )-3-hydroxypiperidin-4-yl)-2,6-difluorobenzamide

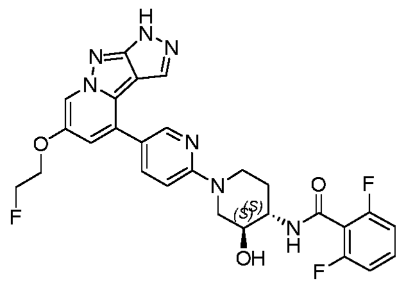

((3*S*,4*S*)-1-(5-(6-(2-fluoroethoxy)-1*H*-pyrazolo[3',4':3,4]pyrazolo[1,5-*a*]pyridin-4-yl)pyridin-2-yl)-3 -hydroxypiperidin-4-yl)amine hydrochloride (320 mg, 0.8 mmol), 2,6-difluorobenzoic acid (110 mg, 0.8 mmol), DIEA (502 mg, 4.0 mmol), HATU (325 mg, 0.9 mmol) and DMF (5 mL) were reacted at 25 °C for 2 h. The reaction liquid was poured into water, and the resulting mixture was extracted with dichloromethane. The organic phase was dried, concentrated under reduced pressure, and purified by column chromatography to give a product (144.2 mg), m/z = 552 $[M+1]^+$. $^1H$ NMR (400 MHz, DMSO-$d_6$) δ 12.68 (s, 1H), 8.60-9.00 (m, 3H), 8.05-8.07 (m, 1H), 7.61 (m, 1H), 7.50-7.50 (m, 1H), 7.32-7.33 (m, 1H), 7.09-7.16 (m, 3H), 5.11-5.11 (m, 1H), 4.76-4.88 (m, 1H), 4.76-4.46 (m, 1H), 4.38-4.39 (m, 3H), 4.24-4.27 (m, 1H), 3.94-3.95 (m, 1H), 3.49-3.50 (m, 1H), 3.16-3.36 (m, 1H), 2.91-2.97 (m, 1H), 2.04-2.07 (m, 1H), 1.45-1.48 (m, 1H).

**Example 142: Preparation of compound 296**

*N*-((3*S*,4*S*)-1-(5-(6-(2,2-difluoroethoxy)-1*H*-pyrazolo[3',4':3,4]pyrazolo[1,5-*a*]pyridin-4-yl)pyridin-2-yl)-3-hydroxypiperidin-4-yl)-2,6-difluorobenzamide

Step A: *tert*-butyl ((3*S*,4*S*)-1-(5-(6-(2,2-difluoroethoxy)-1*H*-pyrazolo[3',4':3,4]pyrazolo[1,5-*a*]pyridin-4-yl)pyridin-2-y l)-3-hydroxypiperidin-4-yl)aminocarbonate

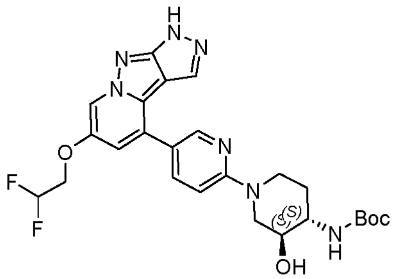

6-(2,2-difluoroethoxy)-4-(6-fluoropyridin-3-yl)-1*H*-pyrazolo[3',4':3,4]pyrazolo[1,5-*a*]pyridine (600 mg, 1.8 mmol), *tert*-butyl ((3*S*,4*S*)-3-hydroxypiperidin-4-yl)carbonate (430 mg, 2.0 mmol), DIEA (1160 mg, 9.0 mmol), and DMSO (6 mL) were added to a reaction flask. The mixture was reacted at 90 °C for 40 h. The reaction liquid was cooled to room temperature and poured into water, and the resulting mixture was extracted with dichloromethane. The organic phase was dried, concentrated under reduced pressure, and purified by column chromatography to give a product (600 mg), m/z = 530 $[M+1]^+$.

Step B: ((3*S*,4*S*)-1-(5-(6-(2,2-difluoroethoxy)-1*H*-pyrazolo[3',4':3,4]pyrazolo[1,5-*a*]pyridin-4-yl)pyridin-2-y l)-3-hydroxypiperidin-4-yl)amine hydrochloride

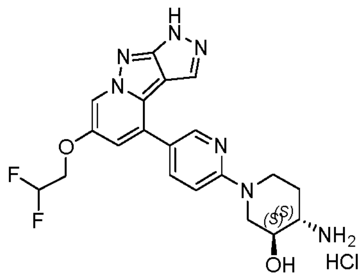

Tert-butyl

((3*S*,4*S*)-1-(5-(6-(2,2-difluoroethoxy)-1*H*-pyrazolo[3',4':3,4]pyrazolo[1,5-*a*]pyridin-4-yl)pyridin-2-y l)-3-hydroxypiperidin-4-yl)aminocarbonate (600 mg, 1.1 mmol) and a 4 N hydrochloric acid/dioxane solution (10 mL) were added to a reaction flask. The mixture was reacted at 25 °C for 0.5 h. After the reaction was completed, the reaction liquid was concentrated under reduced pressure to dryness to give a product (600 mg), m/z = 430 $[M+1]^+$.

Step C: *N*-((3*S*,4*S*)-1-(5-(6-(2,2-difluoroethoxy)-1*H*-pyrazolo[3',4':3,4]pyrazolo[1,5-*a*]pyridin-4-yl)pyridin-2-yl)-3-hydroxypiperidin-4-yl)-2,6-difluorobenzamide

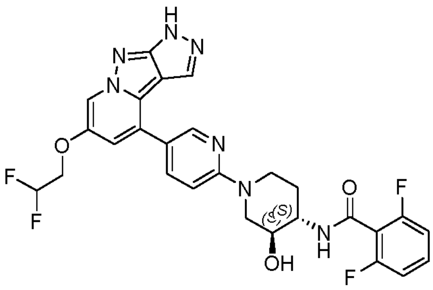

((3*S*,4*S*)-1-(5-(6-(2,2-difluoroethoxy)-1*H*-pyrazolo[3',4':3,4]pyrazolo[1,5-*a*]pyridin-4-yl)pyridin-2-y l)-3-hydroxypiperidin-4-yl)amine hydrochloride (400 mg, 0.9 mmol), 2,6-difluorobenzoic acid (163 mg, 1.0 mmol), DIEA (553 mg, 4.3 mmol), HATU (392 mg, 1.0 mmol) and DMF (5 mL) were reacted at 25 °C for 2 h. The reaction liquid was poured into

water, and the resulting mixture was extracted with dichloromethane. The organic phase was dried, concentrated under reduced pressure, and purified by column chromatography to give a product (111.2 mg), m/z = 570 $[M+1]^+$. $^1H$ NMR (400 MHz, DMSO-$d_6$) δ 12.71 (s, 1H), 8.60-8.70 (m, 3H), 8.05-8.08 (m, 1H), 7.61-7.61 (m, 1H), 7.50-7.50 (m, 1H), 7.36-7.37 (m, 1H), 7.11-7.18 (m, 3H), 6.34-6.62 (m, 1H), 5.11-5.12 (m, 1H), 4.48-4.52 (m, 3H), 4.24-4.27 (m, 1H), 3.94-3.95 (m, 1H), 3.48-3.51 (m, 1H), 3.13-3.18 (m, 1H), 2.94-2.97 (m, 1H), 2.04-2.08 (m, 1H), 1.45-1.47 (m, 1H).

**Example 143: Preparation of compound 297**

6-ethoxy-4-(6-(6-((6-methoxypyridin-3-yl)methyl)-3,6-diazabicyclo[3.1.1]heptan-3-yl)pyridin-3-yl) -1*H*-pyrrolo[2',3':3,4]pyrazolo[1,5-*a*]pyridine

Step A: ethyl 2-(5-bromo-3-chloropyridin-2-yl)-2-cyanoacetate

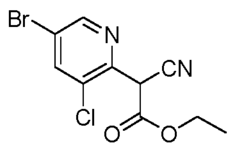

5-bromo-2,3-dichloropyridine (45.4 g, 0.2 mol), ethyl cyanoacetate (45.2 g, 0.4 mol), cesium carbonate (195 g, 0.6 mol), and DMSO (500 mL) were added to a reaction flask. The mixture was reacted at 90 °C for 15 h. After the reaction was completed as monitored by LCMS, the reaction liquid was poured into water, and the resulting mixture was extracted with dichloromethane. The organic phase was dried and concentrated under reduced pressure to give a product (50 g), m/z = 303 $[M+1]^+$.

Step B: 2-(5-bromo-3-chloropyridin-2-yl)acetonitrile

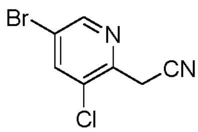

Ethyl 2-(5-bromo-3-chloropyridin-2-yl)-2-cyanoacetate (60 g, 0.2 mol), sodium chloride (3.5 g, 0.06 mol), and DMSO (500 mL) were added to a reaction flask. The mixture was reacted at 160 °C for 2 h. After the reaction was completed as monitored by LCMS, the reaction liquid was poured into water, and the resulting mixture was extracted with dichloromethane. The organic phase was dried and concentrated under reduced pressure to give a product (40 g), m/z = 231 $[M+1]^+$.

Step C: 2,4,6-trimethylbenzenesulfonic acid 1-amino-5-bromo-3-chloro-2-(cyanomethyl)pyridine-1-onium salt

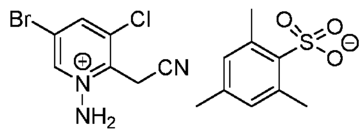

2-(5-bromo-3-chloropyridin-2-yl)acetonitrile (7.4 g, 32.0 mmol) was added to a solution of 2-[(aminooxy)sulfonyl]-1,3,5-trimethylbenzene (6.8 g, 31.7 mmol) in dichloromethane (50 mL) at 0 °C. The mixture was stirred at 0 °C for 3 h to precipitate a large quantity of white solids. After the reaction was completed, diethyl ether (50 mL) was added to the reaction system at 0 °C, and the resulting mixture was stirred for 10 min. The reaction liquid was filtered under reduced pressure, rinsed with diethyl ether, and dried in vacuum to give a product (15 g), which was directly used in the

next step without further purification. M/z = 246 [M+1]+.

Step D: 6-bromo-4-chloropyrazolo[1,5-*a*]pyridin-2-amine

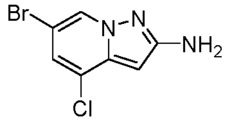

Triethylamine (1.1 g, 11.0 mmol) was added to a solution of 2,4,6-trimethylbenzenesulfonic acid 1-amino-5-bromo-3-chloro-2-(cyanomethyl)pyridin-1-ium salt (1.0 g, 2.2 mmol) in DMF (30 mL) at 0 °C. The mixture was warmed to room temperature and stirred for 12 h. After the reaction was completed, the reaction was quenched by adding brine. The reaction liquid was extracted with ethyl acetate. The organic phases were combined, washed with brine, concentrated under reduced pressure, and separated by column chromatography to give a product (350 mg), m/z = 246 [M+1]+.

Step E: 1-(2-amino-6-bromo-4-chloropyrazolo[1,5-*a*]pyridin-3-yl)-2-chloroethan-1-one

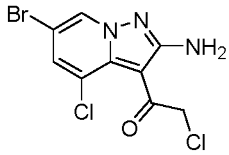

6-bromo-4-chloropyrazolo[1,5-a]pyridin-2-amine (2.5 g, 10.0 mmol), chloroacetonitrile (1.5 g, 20.0 mmol), boron trichloride (12 mL, 12.0 mmol), aluminum trichloride (2.7 g, 20.0 mmol), and chlorobenzene (20 mL) were added to a reaction flask. The mixture was reacted at 120 °C for 12 h. Diluted hydrochloric acid (1 N, 30 mL) was added, and the mixture was reacted at 100 °C for 1 h. After the reaction was completed, the reaction liquid was extracted with ethyl acetate. The organic phases were combined, washed with water, concentrated under reduced pressure, and separated by column chromatography to give a product (1.5 g), m/z = 322 [M+1]+.

Step F: 1-(2-amino-6-bromo-4-chloropyrazolo[1,5-*a*]pyridin-3-yl)-2-chloroethan-1-ol

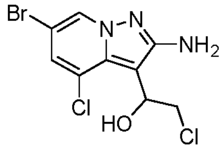

1-(2-amino-6-bromo-4-chloropyrazolo[1,5-*a*]pyridin-3-yl)-2-chloroethyl-1-one (2.0 g, 6.2 mmol), dioxane (20 mL), and water (2 mL) were added to a reaction flask. The reaction system was cooled to 0 °C. Sodium borohydride (0.3 g, 7.9 mmol) was added in portions, and the mixture was warmed to room temperature and reacted for 5 h. After the reaction was completed, the reaction was quenched by adding water, and the reaction liquid was extracted with ethyl acetate. The organic phases were combined, washed with water, concentrated under reduced pressure, and separated by column chromatography to give a product (1.0 g), m/z = 324 [M+1]+.

G: 6-bromo-4-chloro-1*H*-pyrrolo[2',3':3,4]pyrazolo[1,5-*a*]pyridine

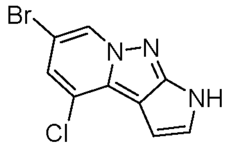

1-(2-amino-6-bromo-4-chloropyrazolo[1,5-a]pyridin-3-yl)-2-chloroethyl-1-ol (323 mg, 1.0 mmol) and tetrahydrofuran (10 mL) were added to a reaction flask. The reaction system was cooled to 0 °C. Isopropyl magnesium chloride (1.5 mL, 3.0 mmol) was slowly added dropwise, and the mixture was reacted at 80 °C for 1 h. After the reaction was completed, the reaction was quenched by adding a saturated aqueous ammonium chloride solution, and the resulting mixture was extracted with ethyl acetate. The organic phases were combined, washed with water, concentrated under reduced pressure, and separated by column chromatography to give a product (178 mg), m/z = 270[M+1]$^+$.

Step H: 4-chloro-6-(4,4,5,5-tetramethyl-1,3,2-dioxaborolan-2-yl)-1*H*-pyrrolo[2',3':3,4]pyrazolo[1,5-*a*] pyridine

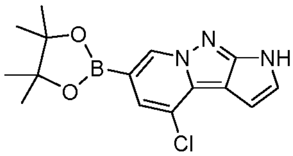

Bis(pinacolato)diboron (736 mg, 2.9 mmol), potassium acetate (705 mg, 7.2 mmol), and 1,4-dioxane (10 mL), Pd(dppf)$Cl_2$ (175 mg, 0.24 mmol) were added to 6-bromo-4-chloro-1*H*-pyrrolo[2',3':3,4]pyrazolo[1,5-*a*]pyridine (650 mg, 2.4 mmol). The mixture was purged with nitrogen and reacted at 80 °C for 12 hours. The reaction liquid was filtered, concentrated, and separated by column chromatography to give a product (523 mg), m/z = 318 [M+1]$^+$.

Step I: 4-chloro-1*H*-pyrazolo[2',3':3,4]pyrazolo[1,5-*a*]pyridine-6-hydroxy

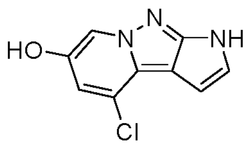

4-chloro-6-(4,4,5,5-tetramethyl-1,3,2-dioxaborolan-2-yl)-1*H*-pyrrolo[2',3':3,4]pyrazolo[1,5-*a*]pyridi ne (634 mg, 2.0 mmol) and tetrahydrofuran (10 mL) were added to a reaction flask. The reaction system was cooled to 0 °C. Hydrogen peroxide (340 mg, 10.0 mmol) was slowly added dropwise, and the mixture was reacted at 0 °C for 1 h. After the reaction was completed, the reaction was quenched by adding a saturated aqueous sodium metabisulfite solution, and the reaction liquid was extracted with ethyl acetate. The organic phases were combined, washed with water, concentrated under reduced pressure, and separated by column chromatography to give a product (500 mg), m/z = 208[M+1]$^+$.

Step J: 4-chloro-6-ethoxy-1*H*-pyrrolo[2',3':3,4]pyrazolo[1,5-*a*]pyridine

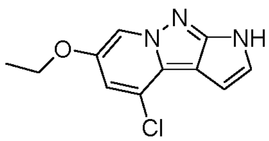

4-chloro-1*H*-pyrazolo[2',3':3,4]pyrazolo[1,5-*a*]pyridine-6-hydroxy (416 mg, 2.0 mmol), iodoethane (374 mg, 2.4 mmol), potassium carbonate (552 mg, 4.0 mmol), and DMF (5 mL) were added to a reaction flask. The reaction system was heated to 50 °C and reacted for 5 h. After the reaction was completed, the reaction was quenched by adding water,

and the reaction liquid was extracted with ethyl acetate. The organic phases were combined, washed with water, concentrated under reduced pressure, and separated by column chromatography to give a product (400 mg), m/z = 236 $[M+1]^+$.

Step J: 6-ethoxy-4-(6-(6-((6-methoxypyridin-3-yl)methyl)-3,6-diazabicyclo[3.1.1]heptan-3-yl)pyridin-3-yl) -1*H*-pyrrolo[2',3':3,4]pyrazolo[1,5-*a*]pyridine

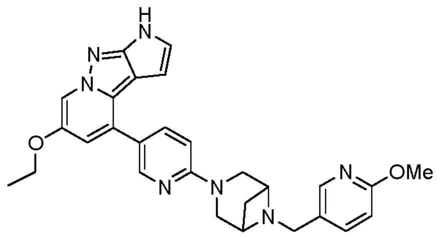

4-chloro-6-ethoxy-1*H*-pyrrolo[2',3':3,4]pyrazolo[1,5-*a*]pyridine (235 mg, 1.0 mmol), 6-((6-methoxypyridin-3-yl)methyl)-3-(5-(4,4,5,5-tetramethyl-1,3,2-dioxolan-2-yl)pyridin-2-yl)-3,6-diazabicyclo[3.1.1]heptane (422 mg, 1.0 mmol), Xphos (95.2 mg, 0.2 mmol), palladium(II) acetate (22.4 mg, 0.1 mmol), potassium phosphate (424 mg, 2.0 mmol), 1,4-dioxane (10 mL), and water (2 mL) were added to a reaction flask. The mixture was purged with nitrogen and reacted at 100 °C for 12 h. The reaction liquid was filtered, and the filtrate was concentrated and separated by column chromatography to give a product (250 mg), m/z = 496 $[M+1]^+$. $^1H$ NMR (400 MHz, DMSO-$d_6$) δ 10.81 (s, 1H), 8.61 (d, J = 2.4 Hz, 1H), 8.33 (d, J = 2.1 Hz, 1H), 8.11-7.99 (m, 2H), 7.69 (d, J = 8.7 Hz, 1H), 7.01-6.92 (m, 2H), 6.86 (d, J = 8.9 Hz, 1H), 6.76 (d, J = 8.5 Hz, 1H), 6.04 (d, J = 3.2 Hz, 1H), 4.10 (q, J = 6.9 Hz, 2H), 3.81 (m, 5H), 3.68 (s, 2H), 3.52 (s, 2H), 3.31 (s, 2H), 1.58 (s, 1H), 1.37 (t, J = 7.0 Hz, 3H).

Compounds 1-265 were prepared by the methods described above or by referring to the methods described above. Their chemical structural formulas and mass spectrometry data are summarized in Table 1.

Table 1: chemical structures and mass spectrometry data of example compound 1-example compound 265

| No. | Chemical structure | m/z [M+1] |
|---|---|---|
| 1 | 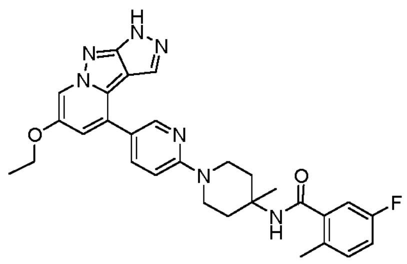 | 528 |
| 2 | 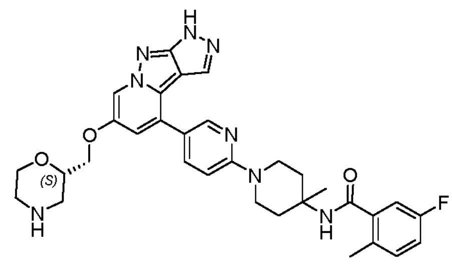 | 599 |

(continued)

| No. | Chemical structure | m/z [M+1] |
|---|---|---|
| 3 | 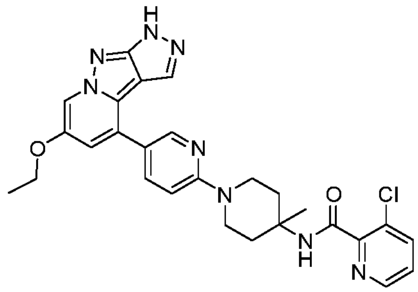 | 531 |
| 4 | 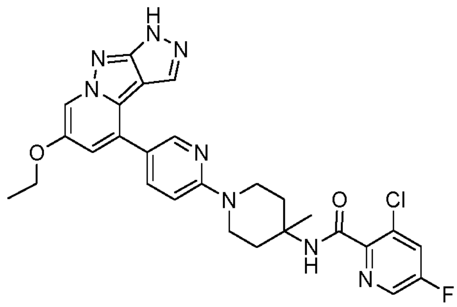 | 549 |
| 5 | 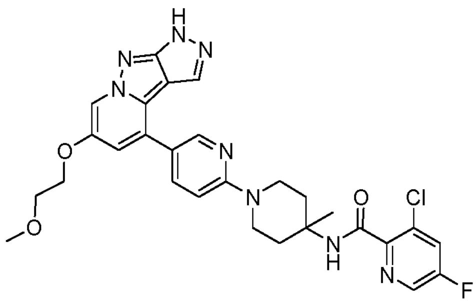 | 579 |
| 6 | 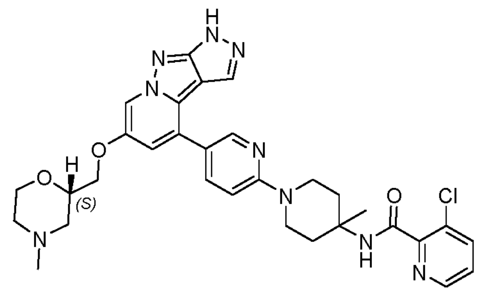 | 616 |
| 7 | 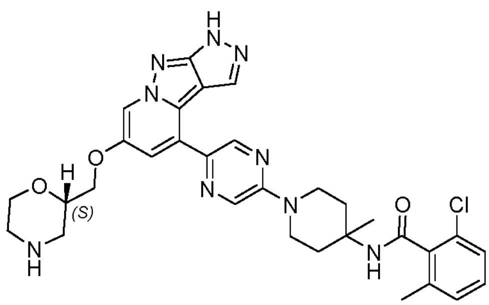 | 616 |

(continued)

| No. | Chemical structure | m/z [M+1] |
|---|---|---|
| 8 | 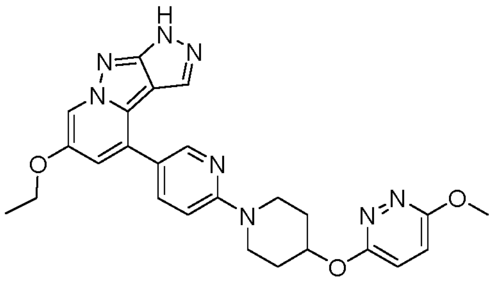 | 487 |
| 9 | 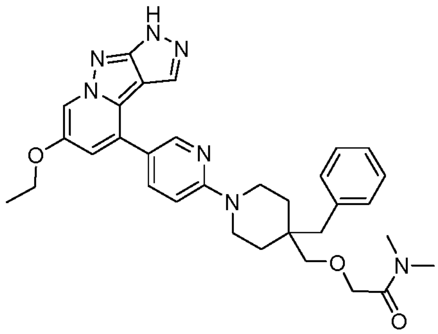 | 568 |
| 10 | 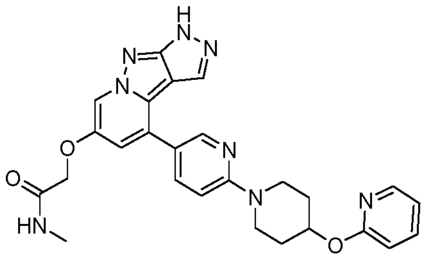 | 499 |
| 11 | 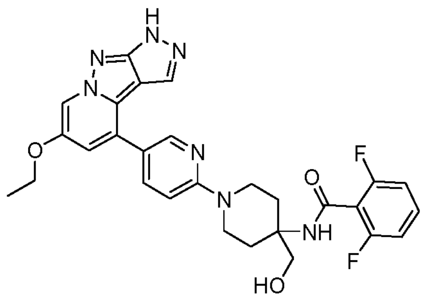 | 548 |
| 12 | 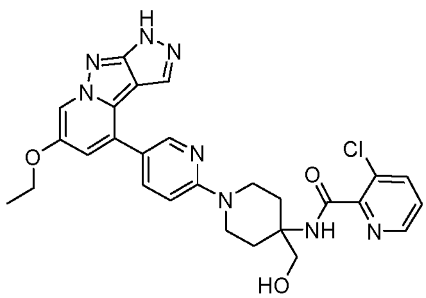 | 547 |

(continued)

| No. | Chemical structure | m/z [M+1] |
|---|---|---|
| 13 | 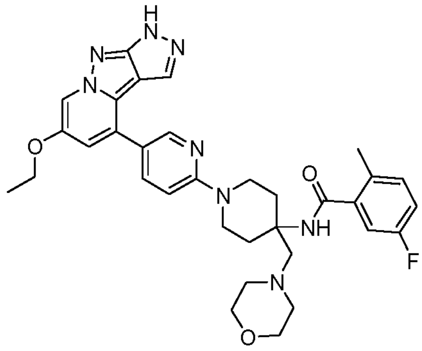 | 613 |
| 14 | 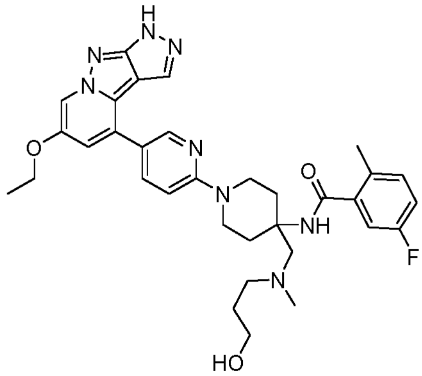 | 615 |
| 15 | 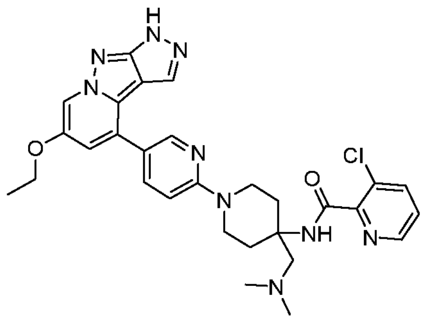 | 574 |
| 16 | 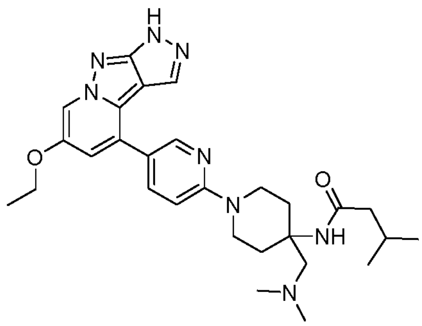 | 519 |

(continued)

| No. | Chemical structure | m/z [M+1] |
|---|---|---|
| 17 | 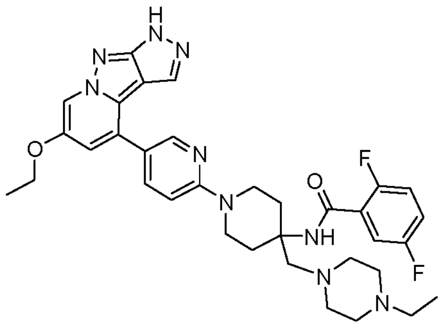 | 644 |
| 18 | 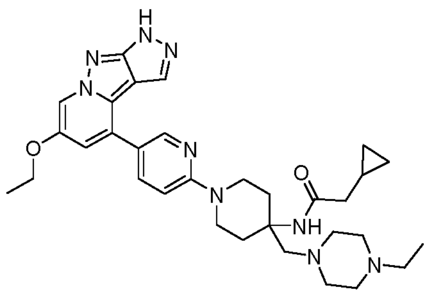 | 586 |
| 19 | 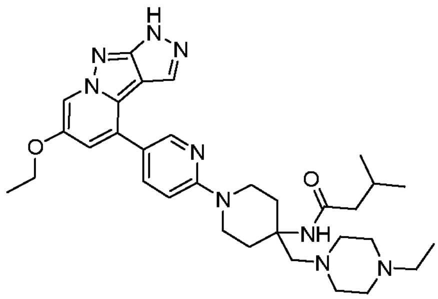 | 588 |
| 20 | 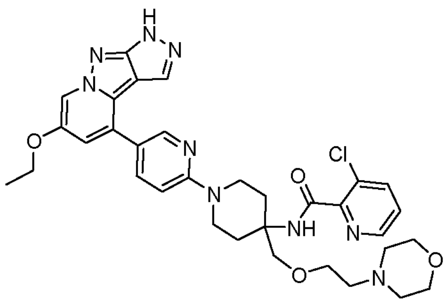 | 660 |
| 21 | 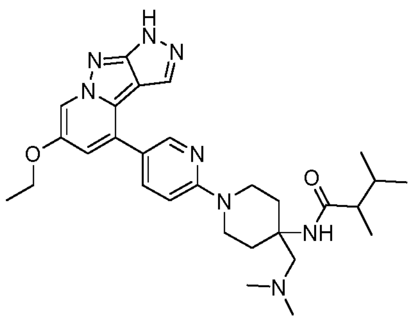 | 533 |

(continued)

| No. | Chemical structure | m/z [M+1] |
|---|---|---|
| 22 | 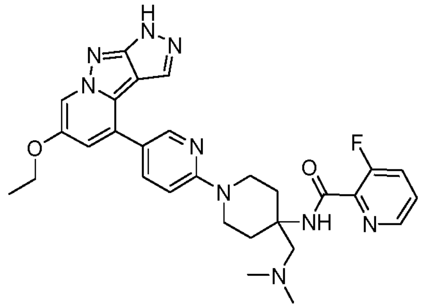 | 558 |
| 23 | 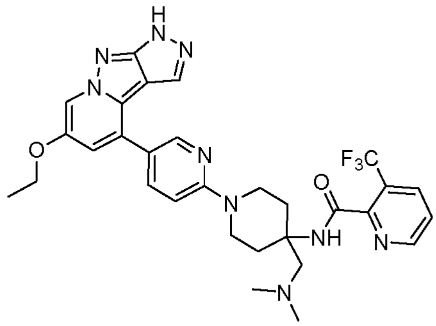 | 608 |
| 24 | 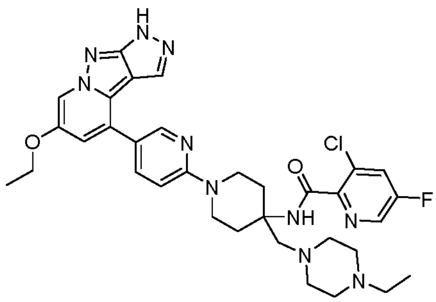 | 661 |
| 25 | 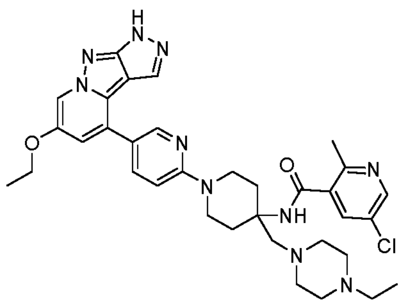 | 657 |
| 26 | 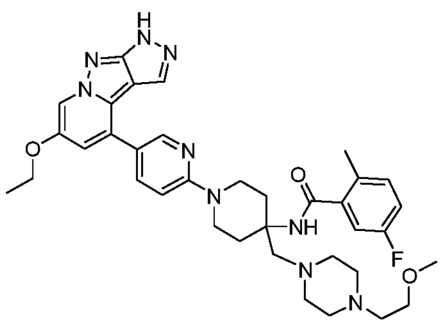 | 670 |

(continued)

| No. | Chemical structure | m/z [M+1] |
|---|---|---|
| 27 | 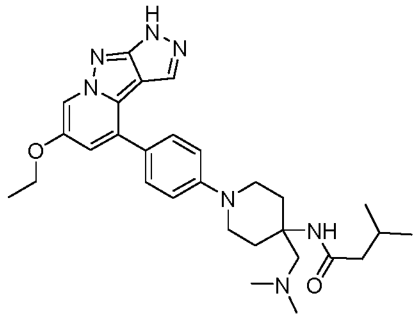 | 518 |
| 28 | 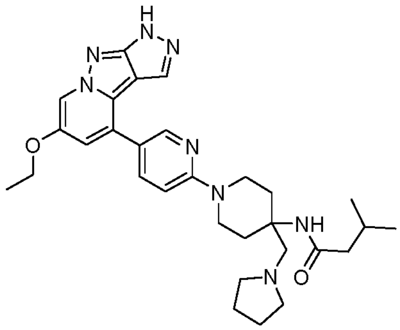 | 545 |
| 29 | 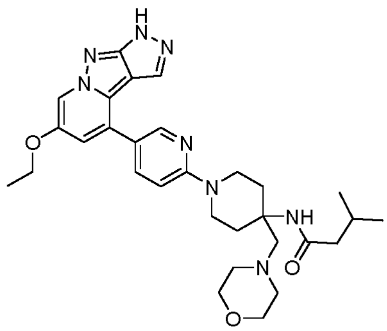 | 561 |
| 30 | 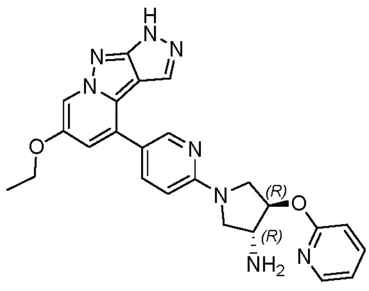 | 457 |
| 31 | 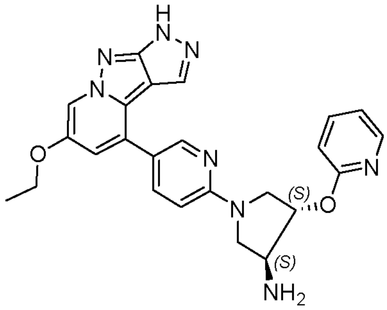 | 457 |

(continued)

| No. | Chemical structure | m/z [M+1] |
|---|---|---|
| 32 | 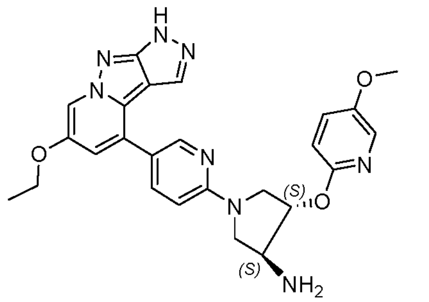 | 487 |
| 33 | 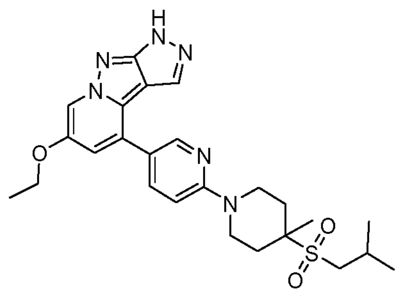 | 497 |
| 34 | 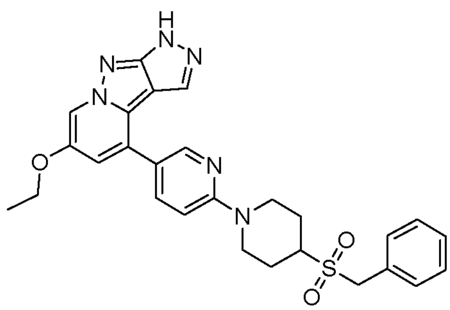 | 517 |
| 35 | 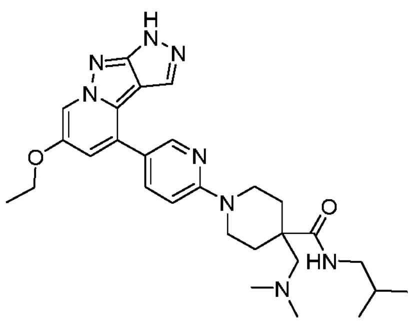 | 519 |
| 36 | 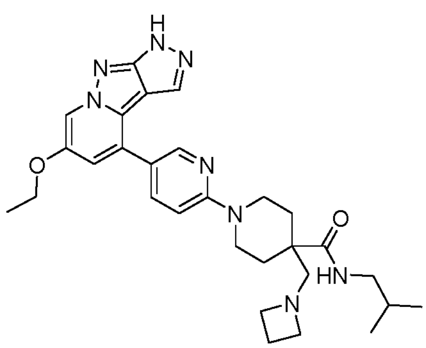 | 531 |

(continued)

| No. | Chemical structure | m/z [M+1] |
|---|---|---|
| 37 | 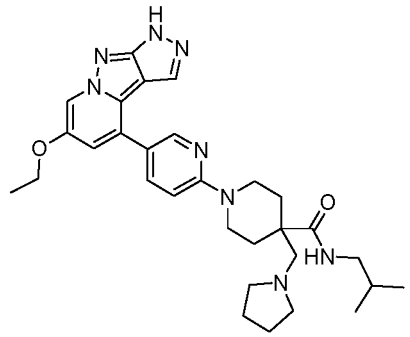 | 545 |
| 38 | 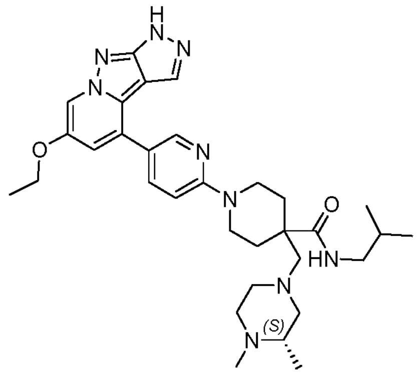 | 588 |
| 39 | 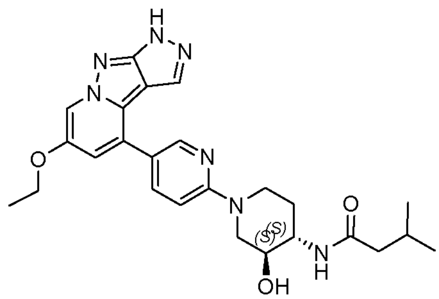 | 478 |
| 40 | 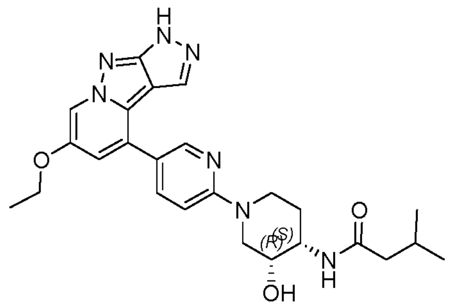 | 448 |

(continued)

| No. | Chemical structure | m/z [M+1] |
|---|---|---|
| 41 | 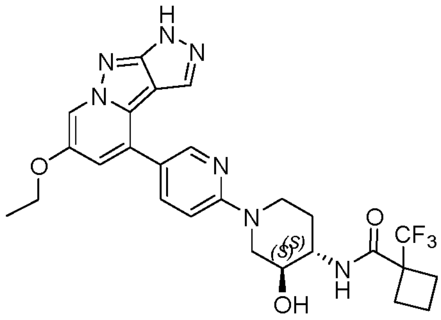 | 544 |
| 42 | 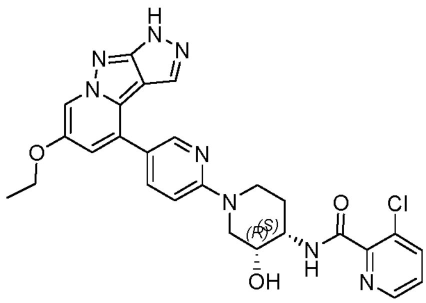 | 533 |
| 43 | 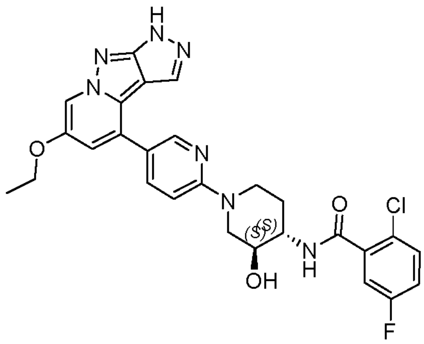 | 550 |
| 44 | 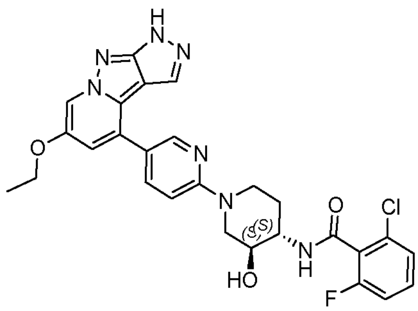 | 550 |
| 45 | 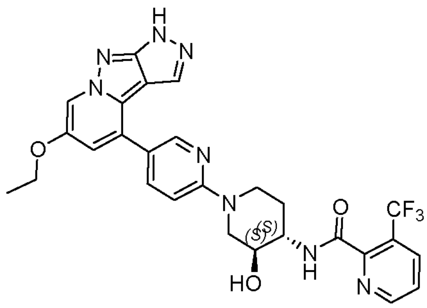 | 567 |

(continued)

| No. | Chemical structure | m/z [M+1] |
|---|---|---|
| 46 | 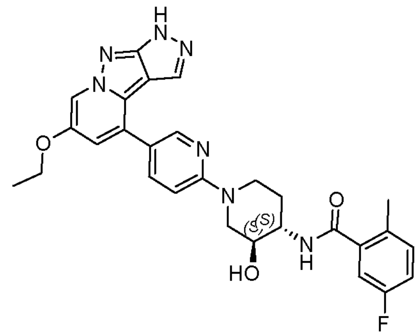 | 530 |
| 47 | 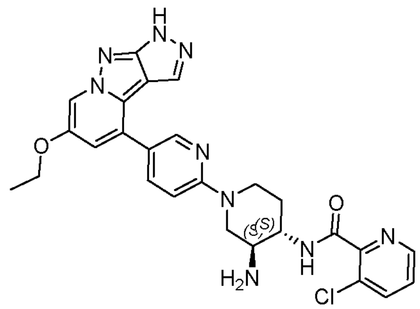 | 532 |
| 48 | 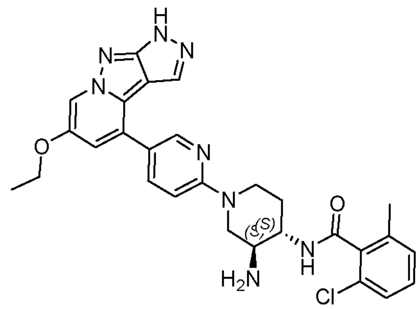 | 545 |
| 49 | 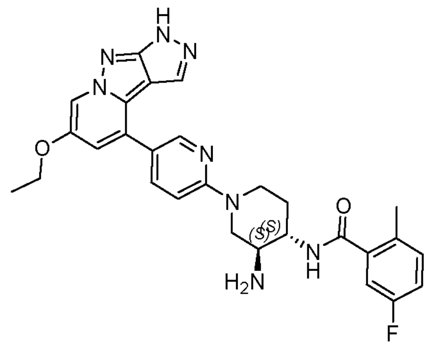 | 529 |

(continued)

| No. | Chemical structure | m/z [M+1] |
|---|---|---|
| 50 | 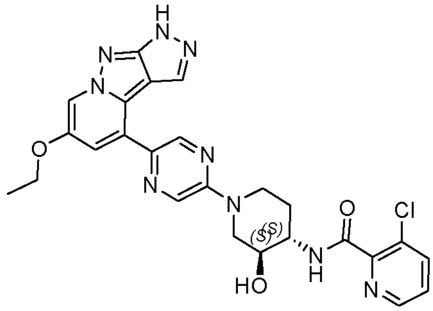 | 534 |
| 51 | 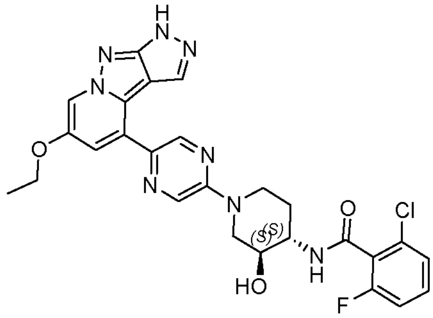 | 551 |
| 52 | 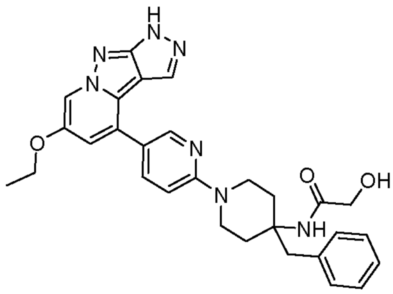 | 526 |
| 53 | 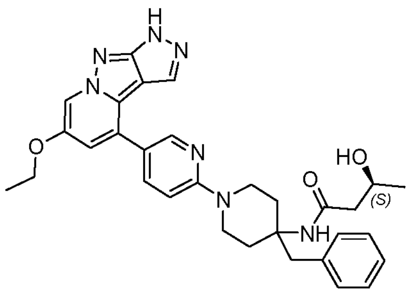 | 554 |
| 54 | 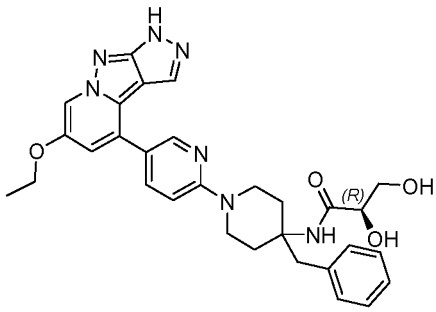 | 556 |

(continued)

| No. | Chemical structure | m/z [M+1] |
|---|---|---|
| 55 | 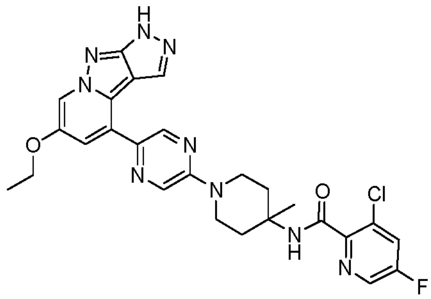 | 550 |
| 56 | 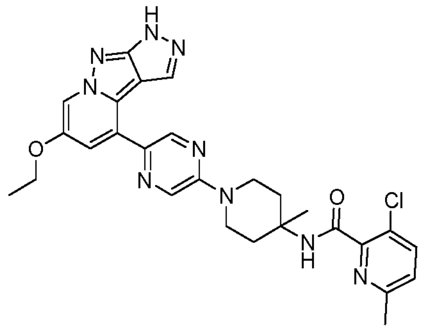 | 546 |
| 57 | 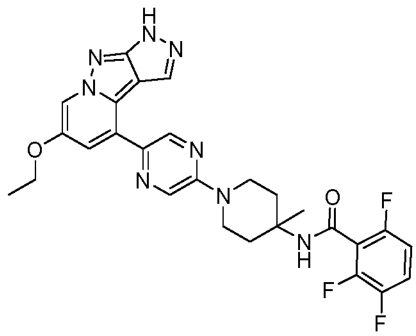 | 551 |
| 58 | 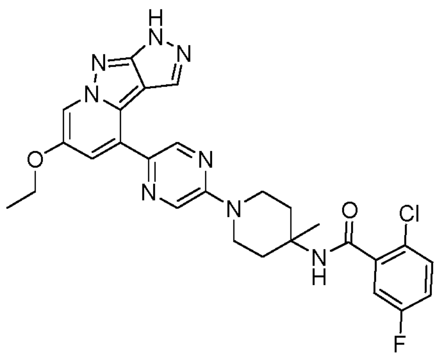 | 549 |

(continued)

| No. | Chemical structure | m/z [M+1] |
|---|---|---|
| 59 | 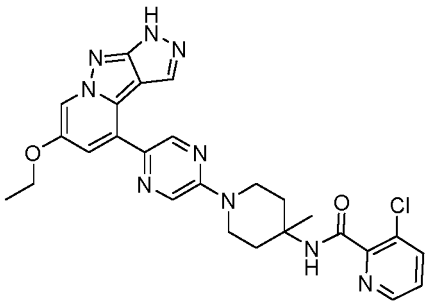 | 532 |
| 60 | 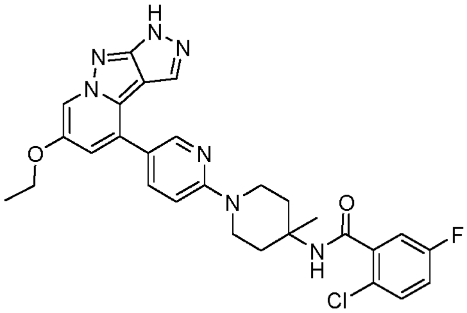 | 548 |
| 61 | 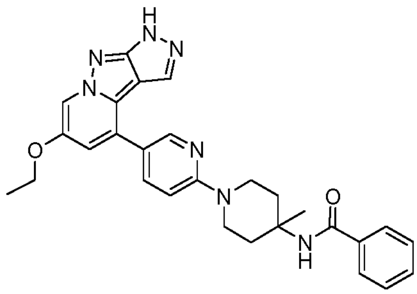 | 496 |
| 62 | 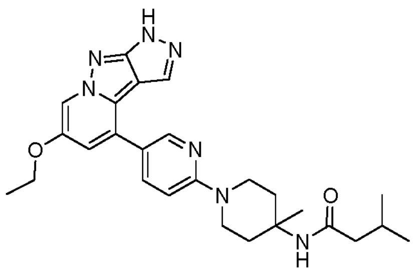 | 476 |
| 63 | 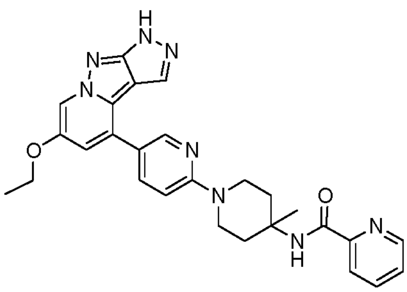 | 497 |

(continued)

| No. | Chemical structure | m/z [M+1] |
|---|---|---|
| 64 | 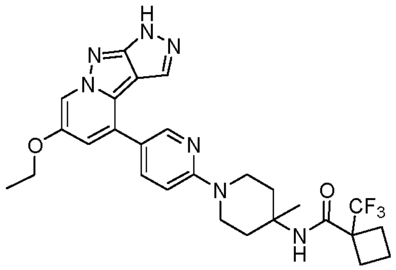 | 542 |
| 65 | 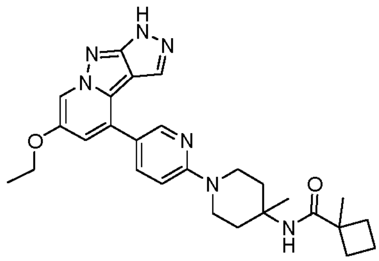 | 488 |
| 66 | 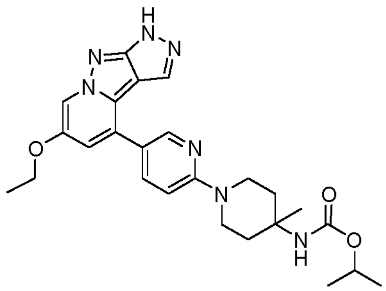 | 478 |
| 67 | 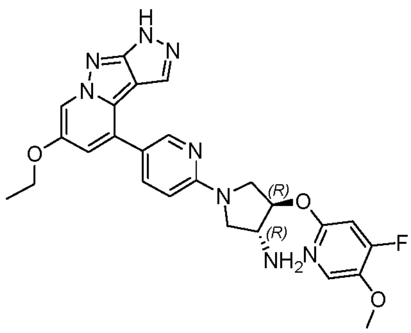 | 505 |
| 68 | 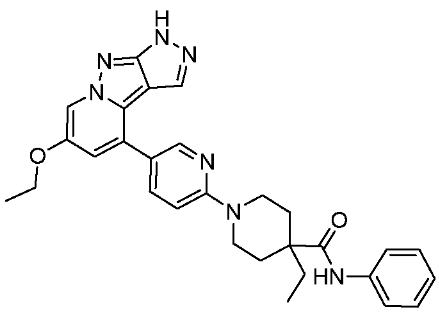 | 510 |

(continued)

| No. | Chemical structure | m/z [M+1] |
|---|---|---|
| 69 | 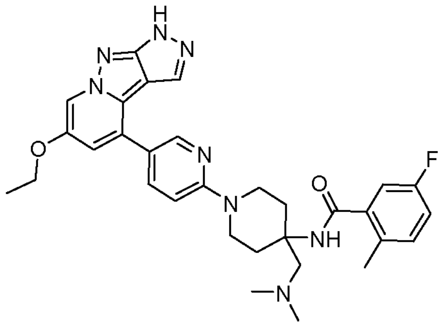 | 571 |
| 70 | 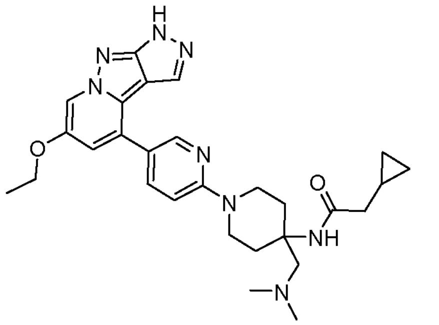 | 517 |
| 71 | 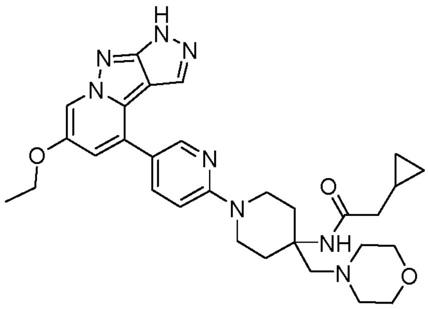 | 559 |
| 72 | 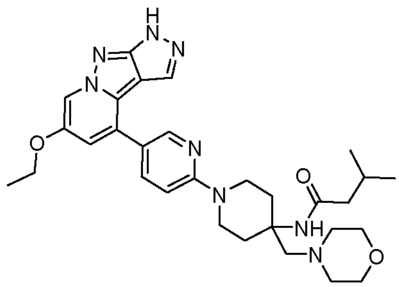 | 561 |
| 73 | 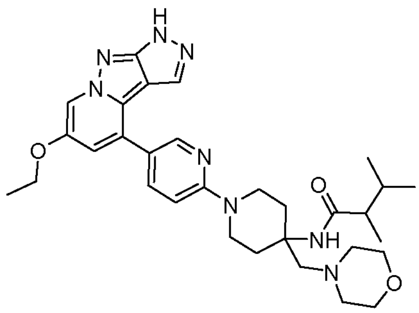 | 575 |

(continued)

| No. | Chemical structure | m/z [M+1] |
|---|---|---|
| 74 | 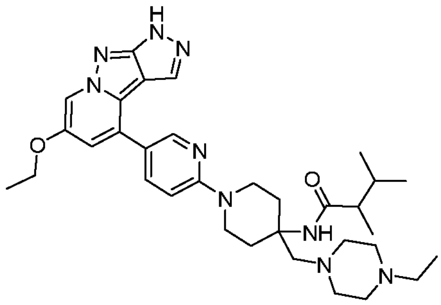 | 602 |
| 75 | 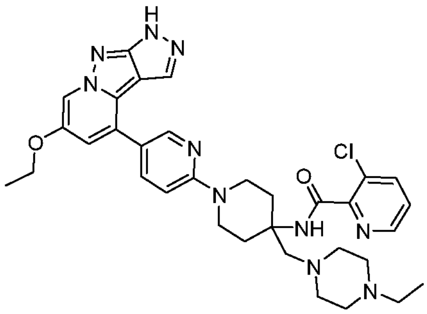 | 643 |
| 76 | 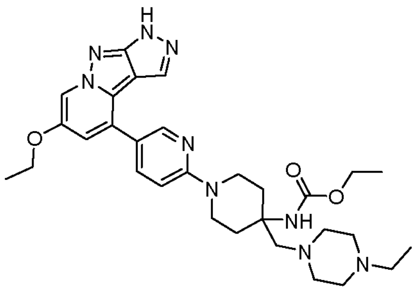 | 576 |
| 77 | 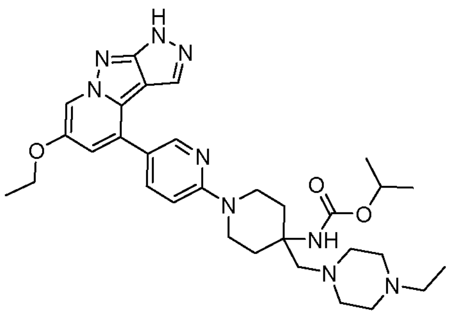 | 590 |
| 78 | 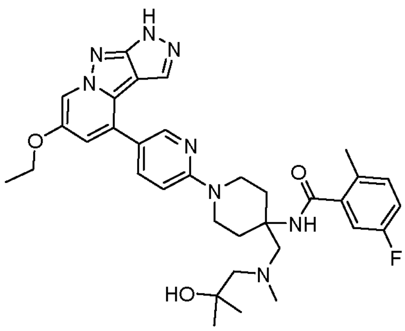 | 629 |

(continued)

| No. | Chemical structure | m/z [M+1] |
|---|---|---|
| 79 | 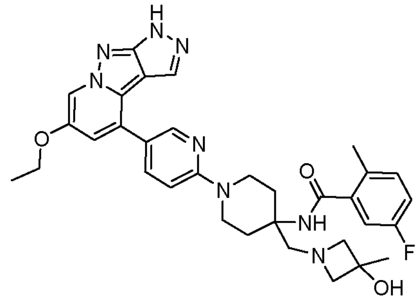 | 613 |
| 80 | 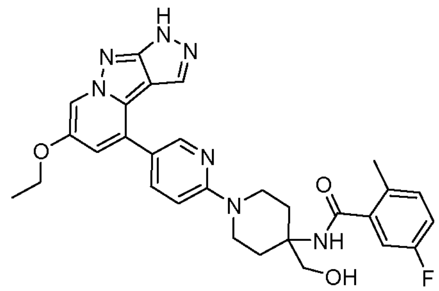 | 544 |
| 81 | 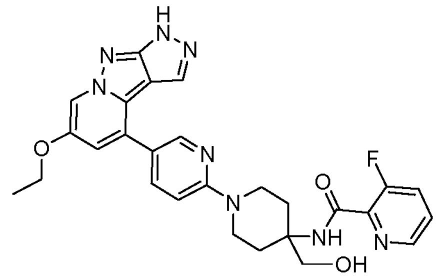 | 531 |
| 82 | 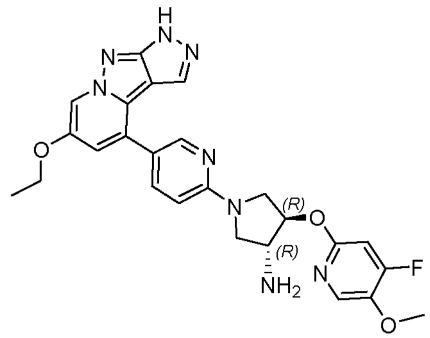 | 505 |
| 83 | 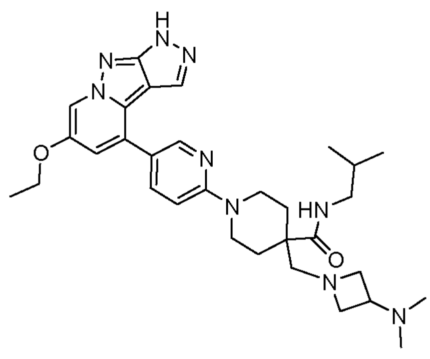 | 574 |

(continued)

| No. | Chemical structure | m/z [M+1] |
|---|---|---|
| 84 | 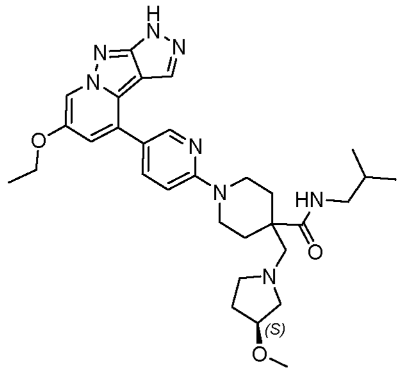 | 575 |
| 85 | 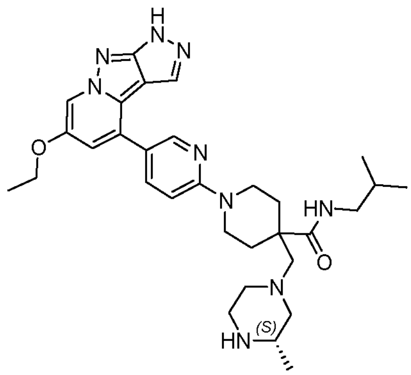 | 574 |
| 86 | 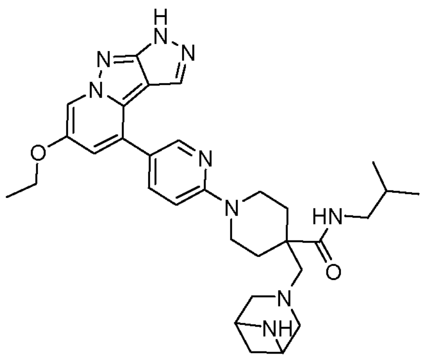 | 572 |
| 87 | 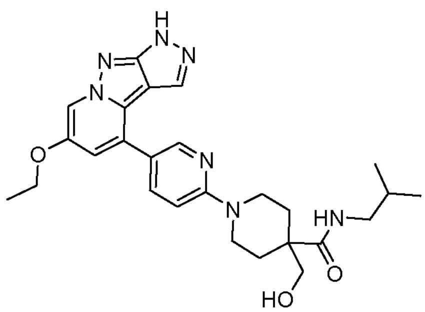 | 492 |

(continued)

| No. | Chemical structure | m/z [M+1] |
|---|---|---|
| 88 | 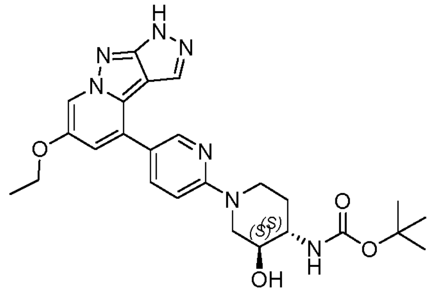 | 494 |
| 89 | 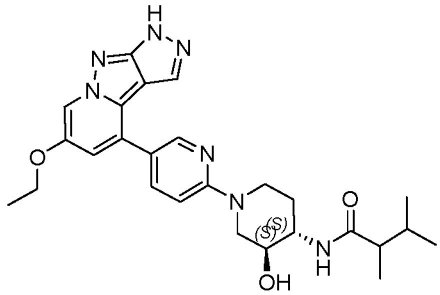 | 492 |
| 90 | 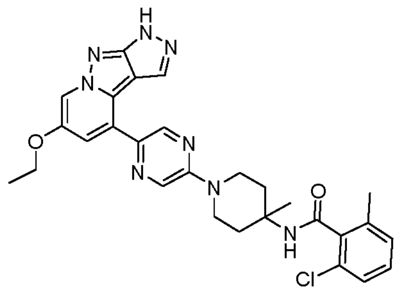 | 545 |
| 91 | 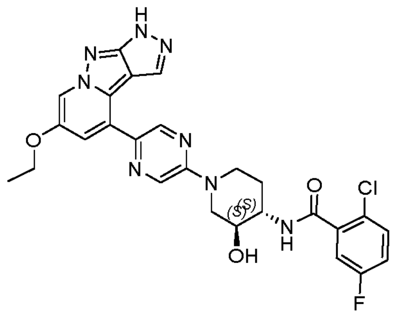 | 551 |
| 92 | 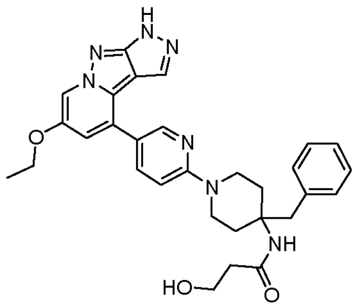 | 540 |

(continued)

| No. | Chemical structure | m/z [M+1] |
|---|---|---|
| 93 | 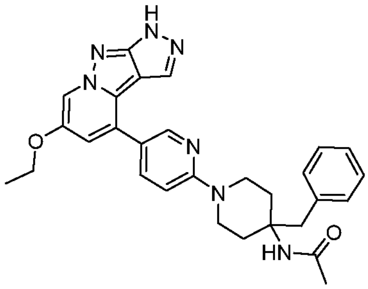 | 510 |
| 94 | 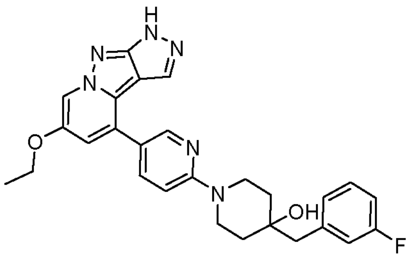 | 487 |
| 95 | 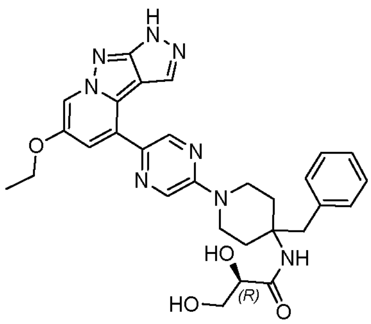 | 557 |
| 96 | 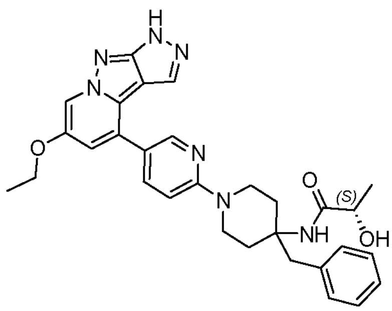 | 540 |
| 97 | 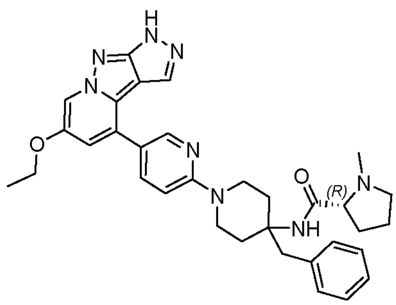 | 579 |

(continued)

| No. | Chemical structure | m/z [M+1] |
|---|---|---|
| 98 | 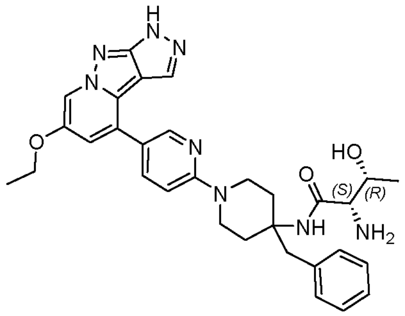 | 569 |
| 99 | 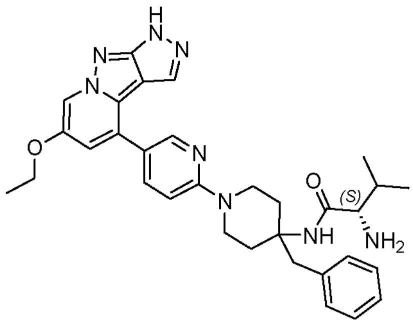 | 567 |
| 100 | 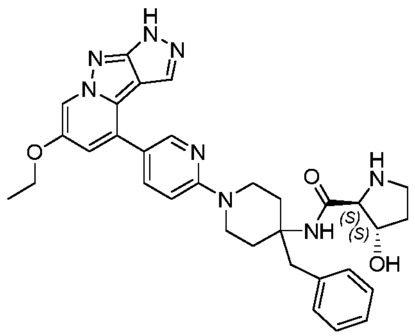 | 581 |
| 101 | 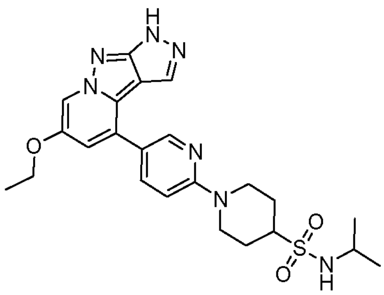 | 484 |
| 102 | 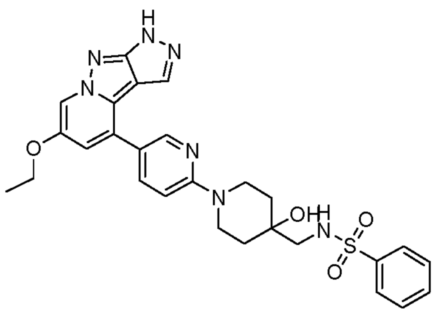 | 548 |

(continued)

| No. | Chemical structure | m/z [M+1] |
|---|---|---|
| 103 | 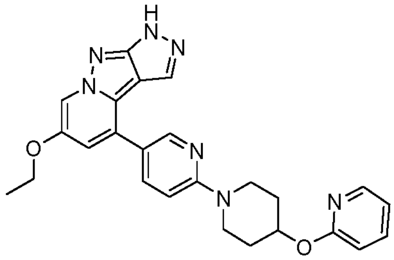 | 456 |
| 104 | 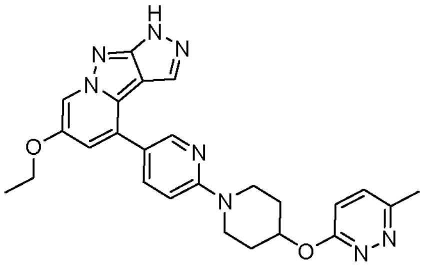 | 471 |
| 105 | 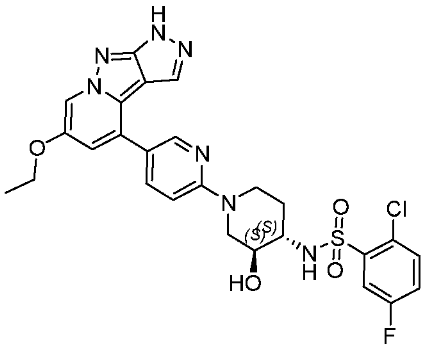 | 586 |
| 106 | 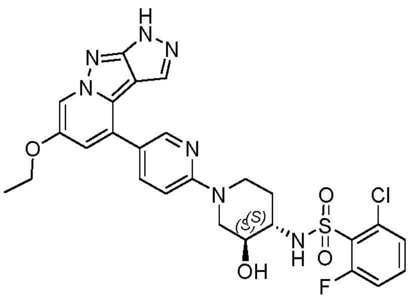 | 586 |
| 107 | 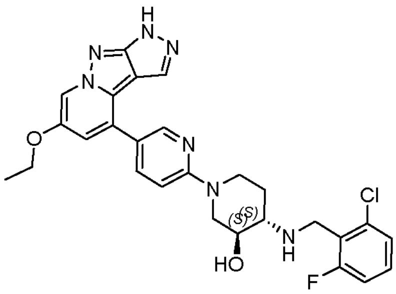 | 536 |

(continued)

| No. | Chemical structure | m/z [M+1] |
|---|---|---|
| 108 | 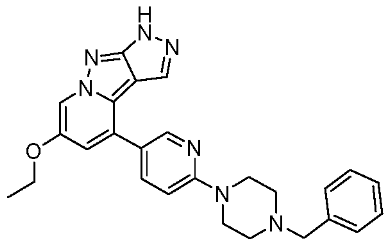 | 454 |
| 109 | 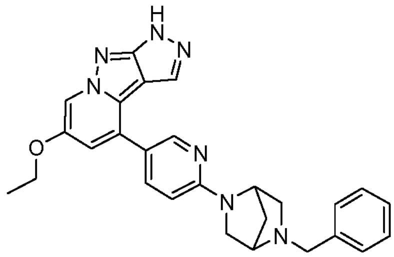 | 466 |
| 110 | 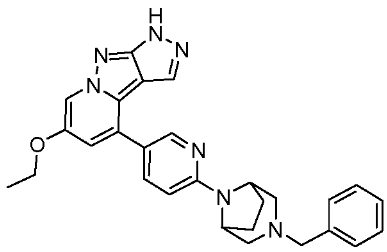 | 480 |
| 111 | 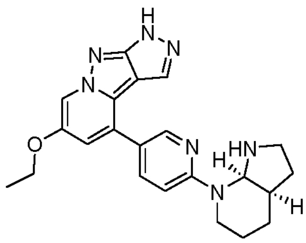 | 404 |
| 112 | 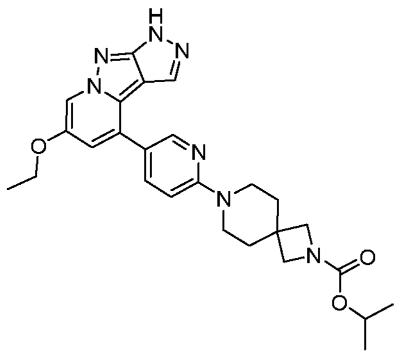 | 490 |

(continued)

| No. | Chemical structure | m/z [M+1] |
|---|---|---|
| 113 | 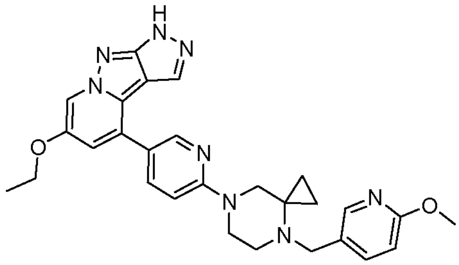 | 511 |
| 114 | 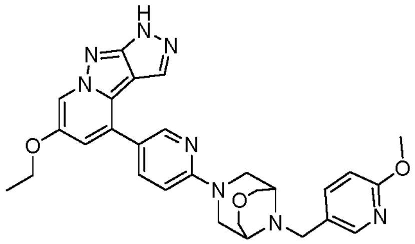 | 527 |
| 115 | 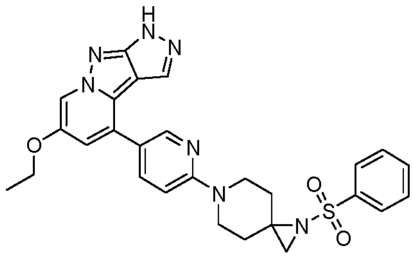 | 530 |
| 116 | 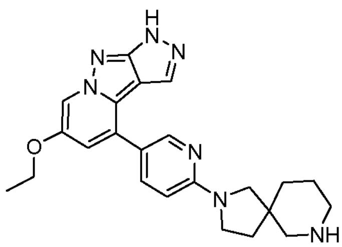 | 418 |
| 117 | 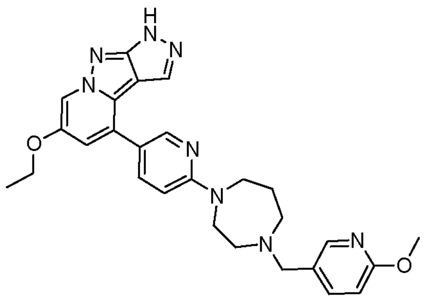 | 499 |

(continued)

| No. | Chemical structure | m/z [M+1] |
|---|---|---|
| 118 | 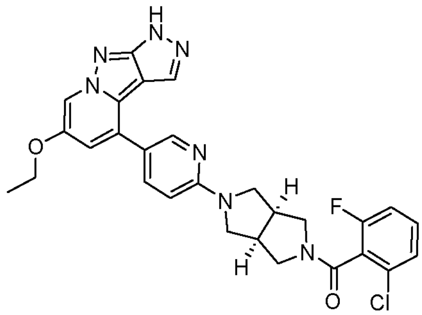 | 546 |
| 119 | 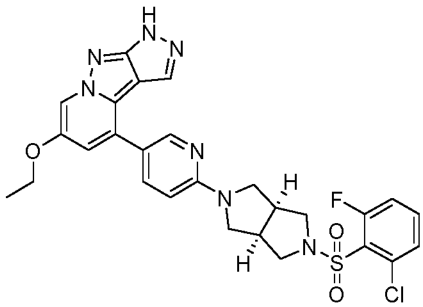 | 582 |
| 120 | 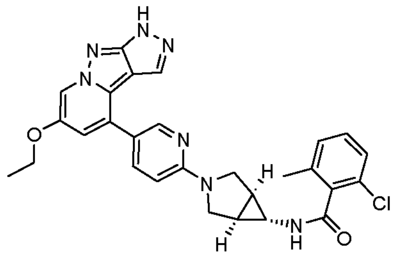 | 528 |
| 121 | 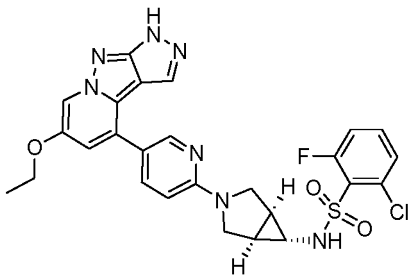 | 568 |
| 122 | 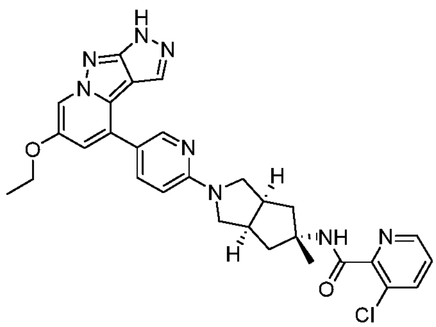 | 557 |

(continued)

| No. | Chemical structure | m/z [M+1] |
|---|---|---|
| 123 | 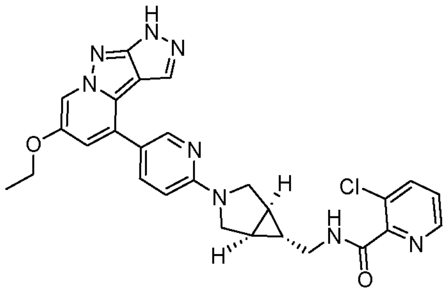 | 529 |
| 124 | 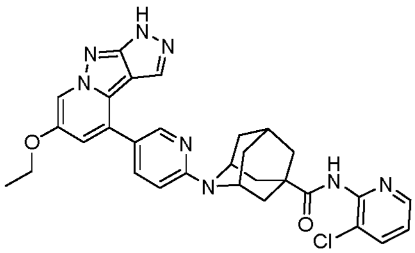 | 569 |
| 125 | 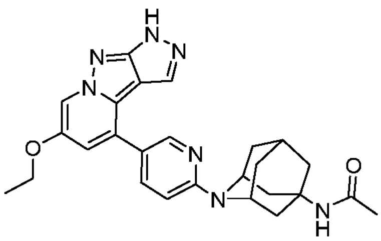 | 472 |
| 126 | 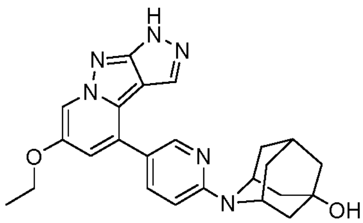 | 431 |
| 127 | 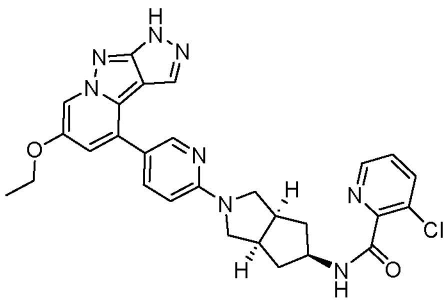 | 543 |

(continued)

| No. | Chemical structure | m/z [M+1] |
|---|---|---|
| 128 | 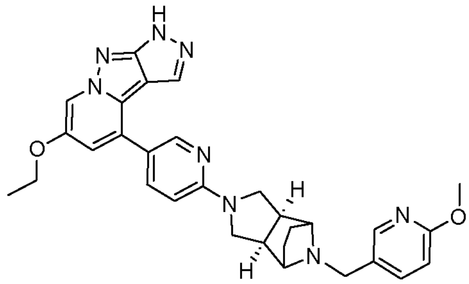 | 537 |
| 129 | 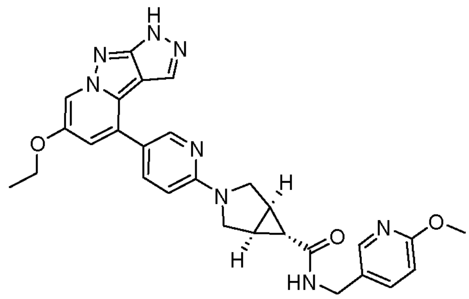 | 525 |
| 130 | 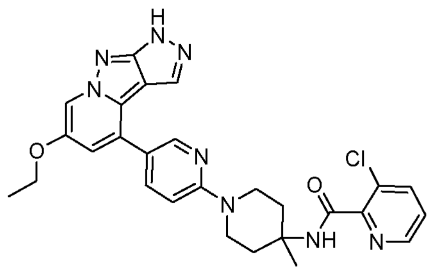 | 531 |
| 131 | 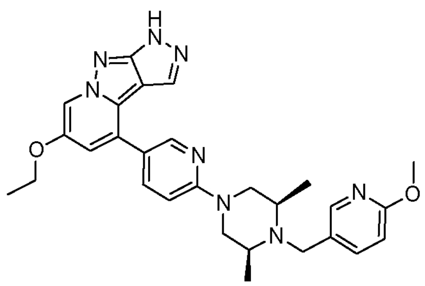 | 513 |
| 132 | 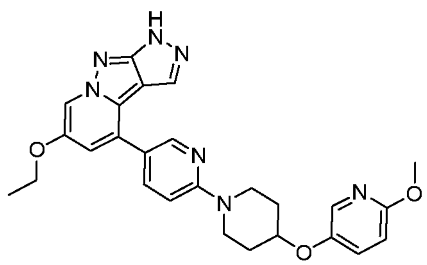 | 486 |

(continued)

| No. | Chemical structure | m/z [M+1] |
|---|---|---|
| 133 | 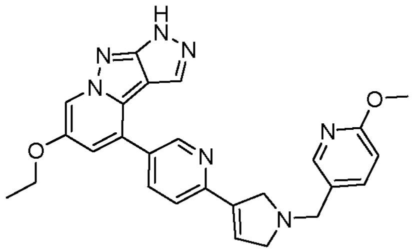 | 468 |
| 134 | 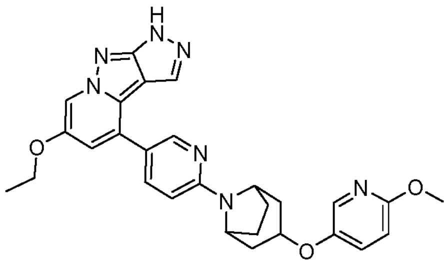 | 512 |
| 135 | 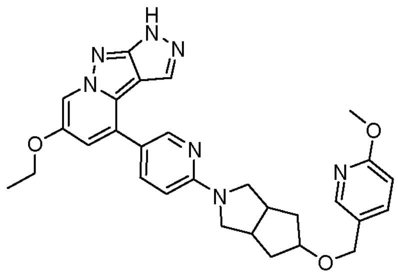 | 526 |
| 136 | 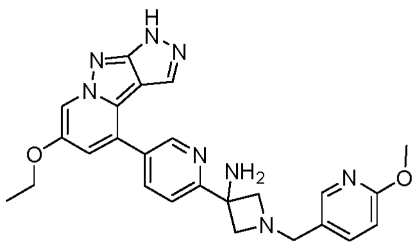 | 471 |
| 137 | 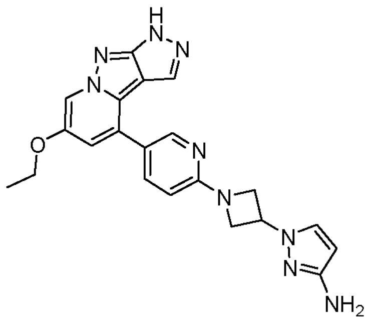 | 416 |

(continued)

| No. | Chemical structure | m/z [M+1] |
|---|---|---|
| 138 | 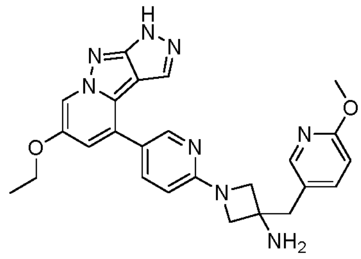 | 471 |
| 139 | 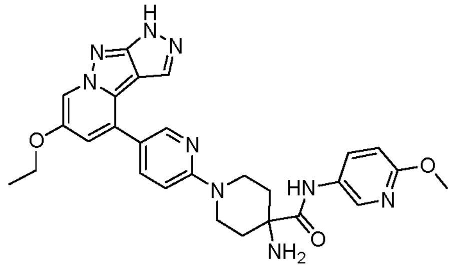 | 528 |
| 140 | 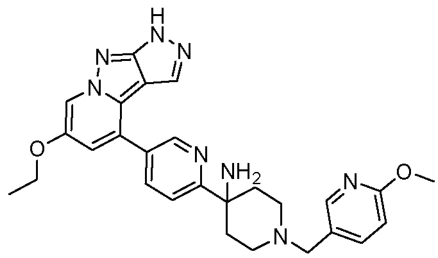 | 499 |
| 141 | 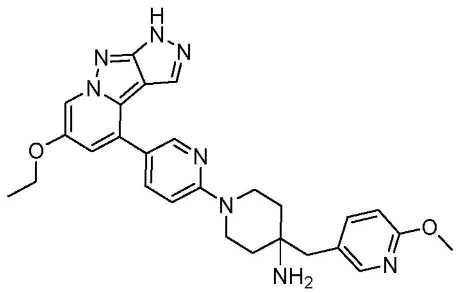 | 499 |
| 142 | 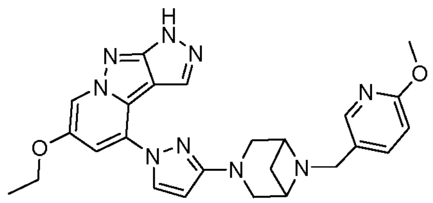 | 486 |
| 143 | 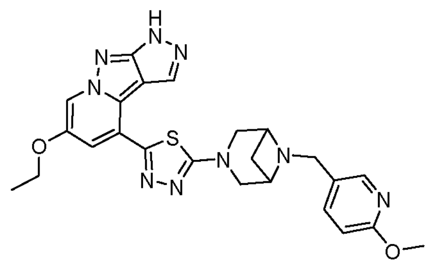 | 504 |

(continued)

| No. | Chemical structure | m/z [M+1] |
|---|---|---|
| 144 | 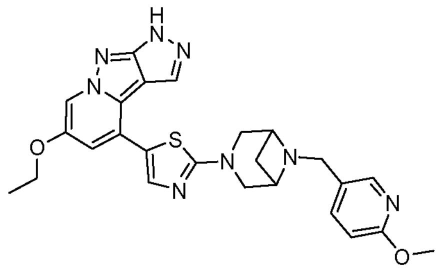 | 503 |
| 145 | 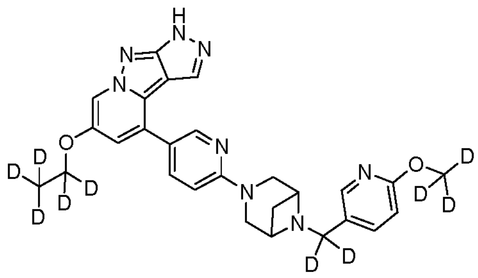 | 507 |
| 146 | 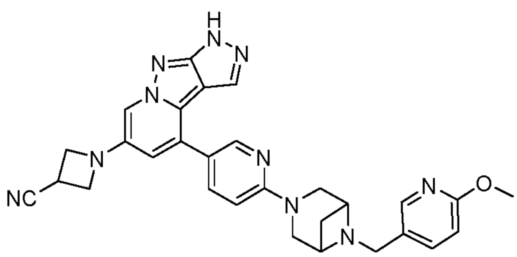 | 533 |
| 147 | 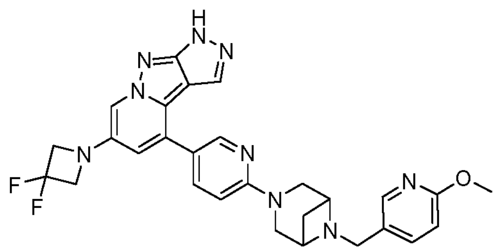 | 544 |
| 148 | 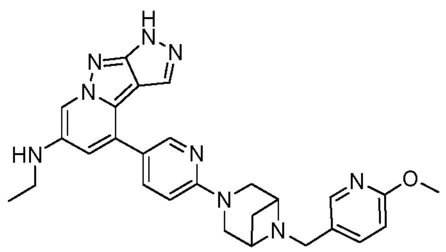 | 496 |
| 149 | 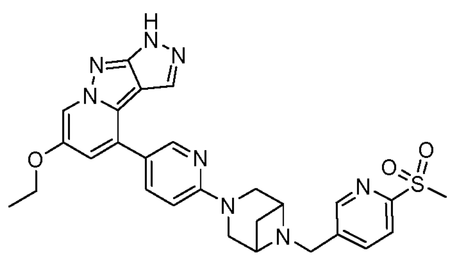 | 544 |

(continued)

| No. | Chemical structure | m/z [M+1] |
|---|---|---|
| 150 | 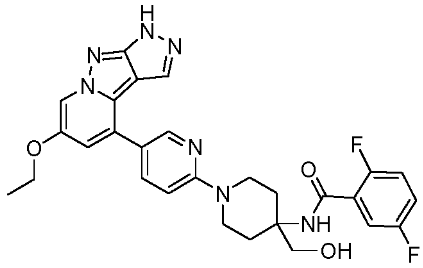 | 548 |
| 151 | 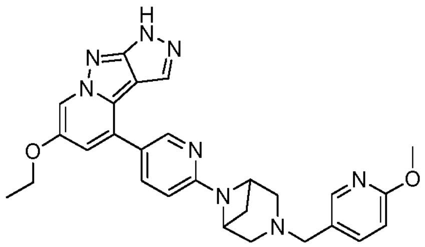 | 497 |
| 152 | 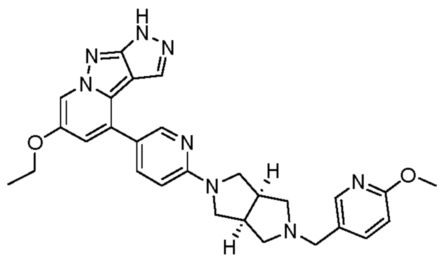 | 511 |
| 153 | 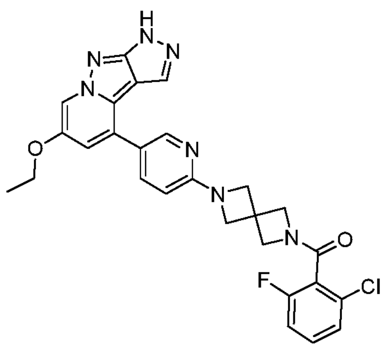 | 532 |
| 154 | 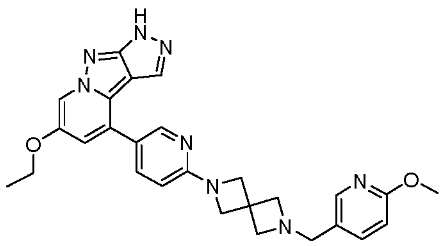 | 497 |

(continued)

| No. | Chemical structure | m/z [M+1] |
|---|---|---|
| 155 | 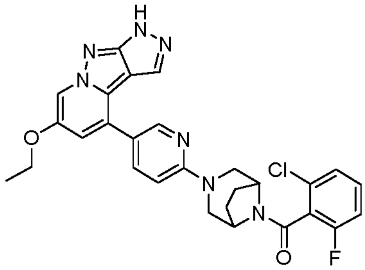 | 546 |
| 156 | 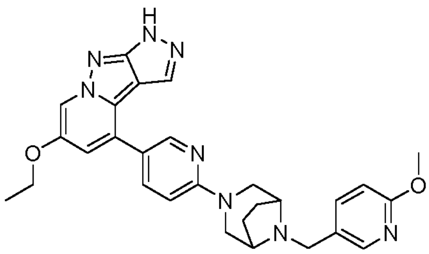 | 511 |
| 157 | 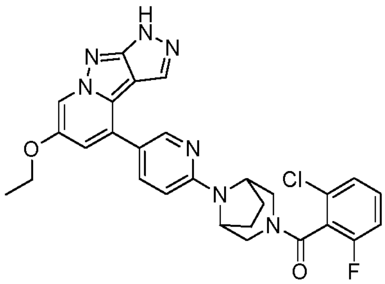 | 546 |
| 158 | 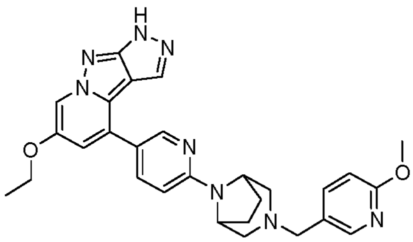 | 511 |
| 159 | 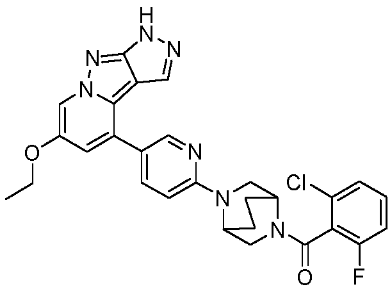 | 546 |

(continued)

| No. | Chemical structure | m/z [M+1] |
|---|---|---|
| 160 | 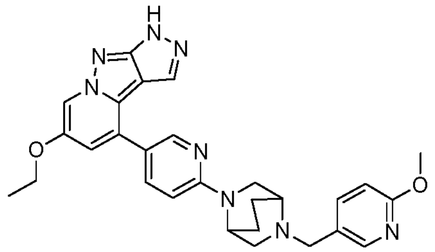 | 511 |
| 161 | 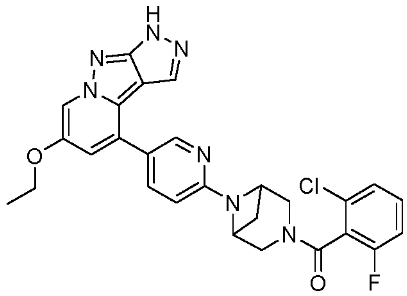 | 532 |
| 162 | 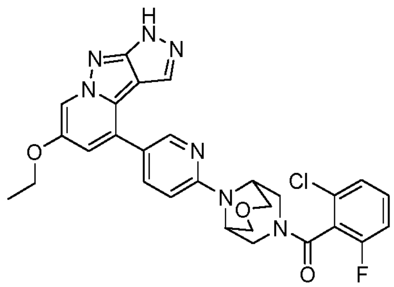 | 562 |
| 163 | 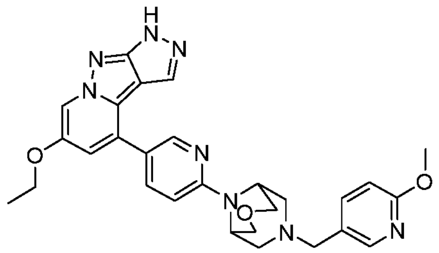 | 527 |
| 164 | 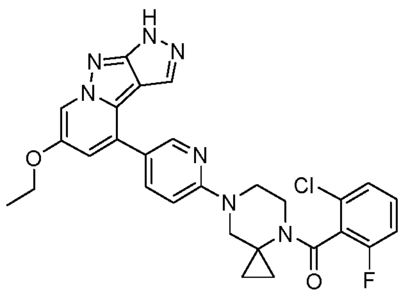 | 546 |

(continued)

| No. | Chemical structure | m/z [M+1] |
|---|---|---|
| 165 | 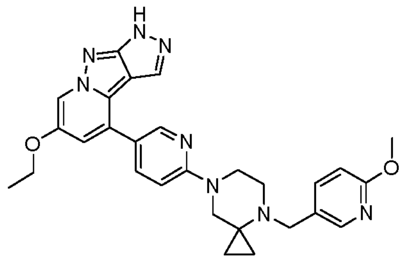 | 511 |
| 166 | 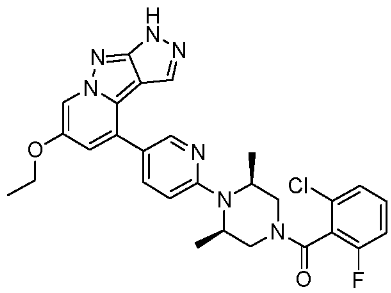 | 548 |
| 167 | 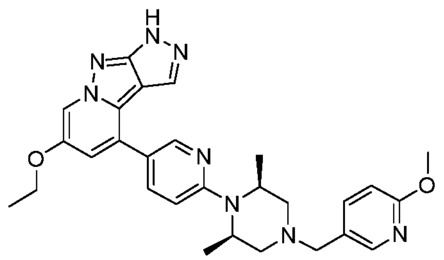 | 513 |
| 168 | 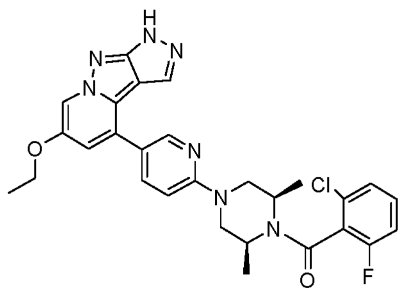 | 548 |
| 169 | 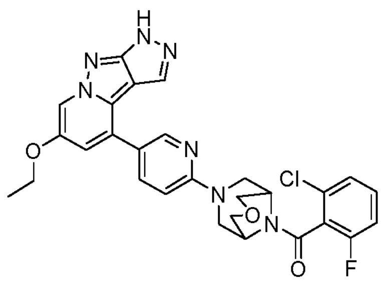 | 562 |

(continued)

| No. | Chemical structure | m/z [M+1] |
|---|---|---|
| 170 | 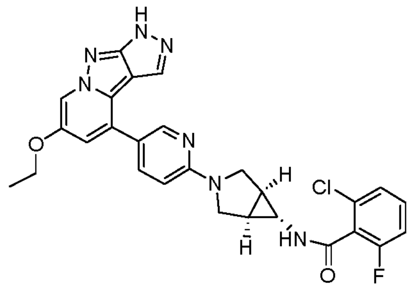 | 532 |
| 171 | 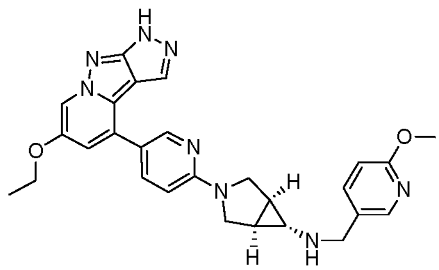 | 497 |
| 172 | 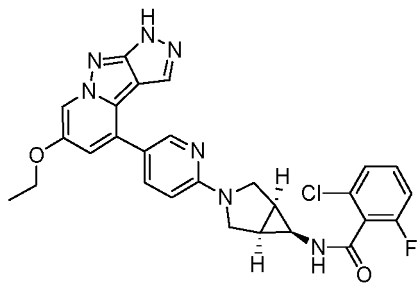 | 532 |
| 173 | 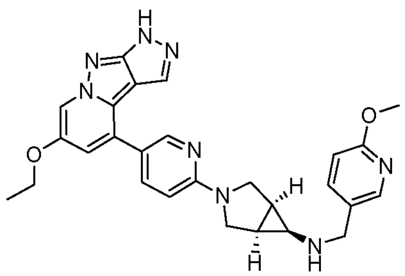 | 497 |
| 174 | 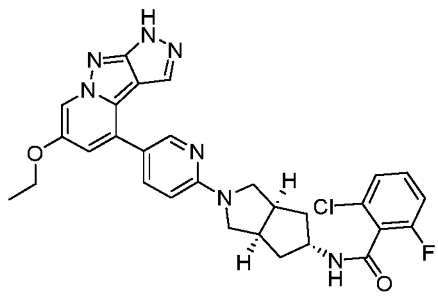 | 560 |

(continued)

| No. | Chemical structure | m/z [M+1] |
|---|---|---|
| 175 | 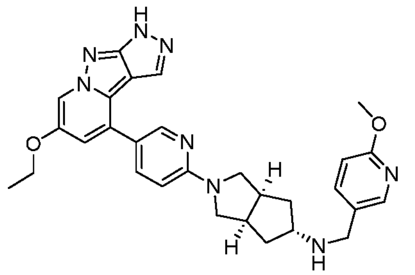 | 525 |
| 176 | 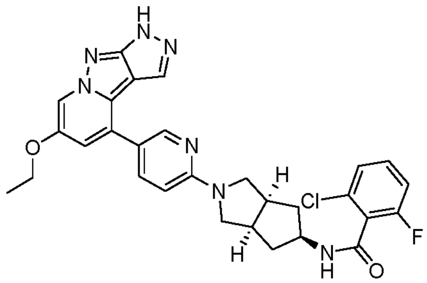 | 560 |
| 177 | 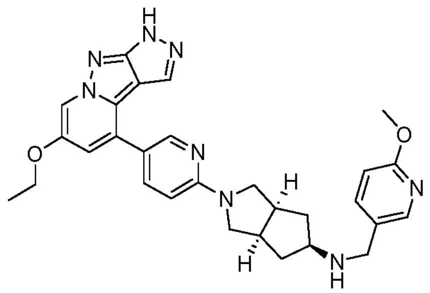 | 525 |
| 178 | 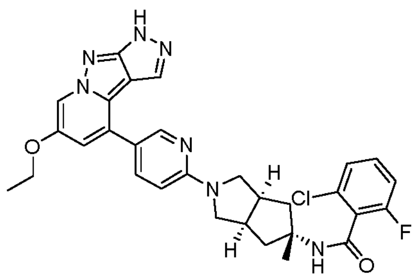 | 574 |
| 179 | 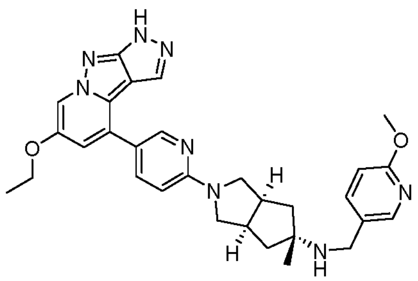 | 539 |

(continued)

| No. | Chemical structure | m/z [M+1] |
|---|---|---|
| 180 | 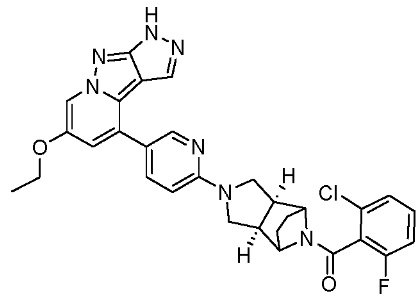 | 572 |
| 181 | 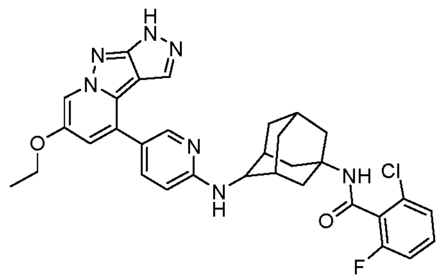 | 600 |
| 182 | 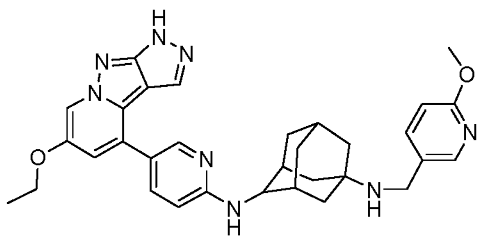 | 565 |
| 183 | 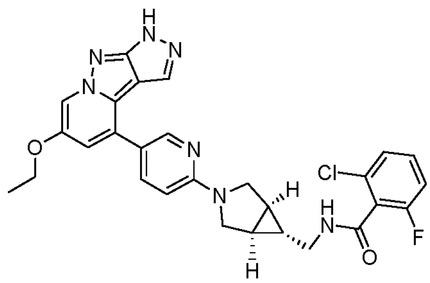 | 546 |
| 184 | 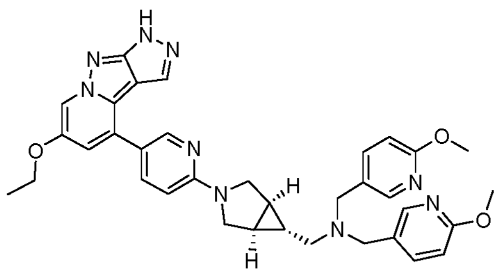 | 632 |

(continued)

| No. | Chemical structure | m/z [M+1] |
|---|---|---|
| 185 | 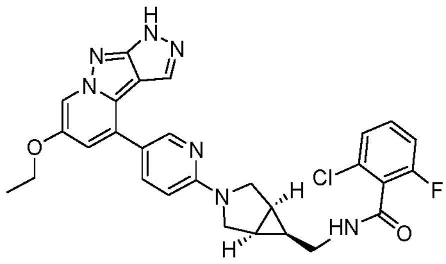 | 546 |
| 186 | 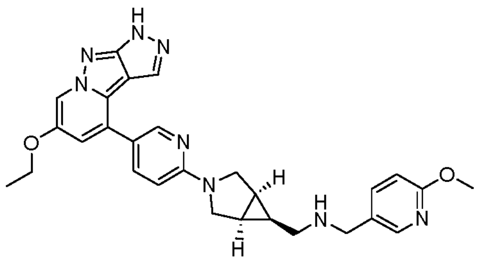 | 511 |
| 187 | 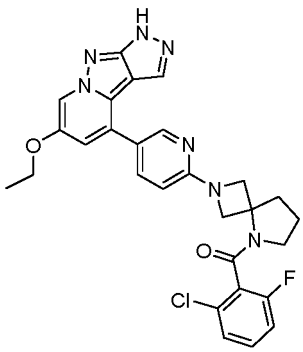 | 546 |
| 188 | 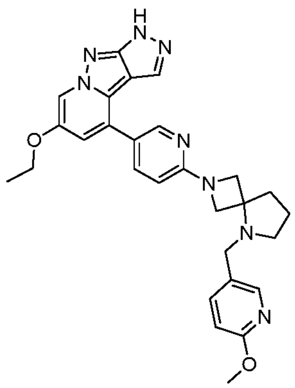 | 511 |
| 189 | 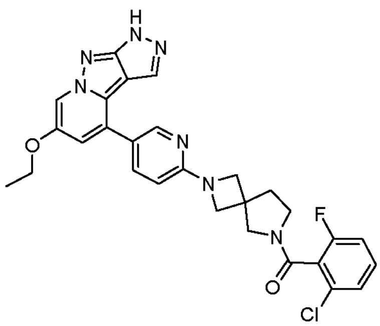 | 546 |

(continued)

| No. | Chemical structure | m/z [M+1] |
|---|---|---|
| 190 | 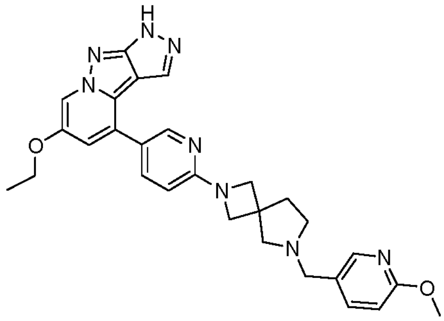 | 511 |
| 191 | 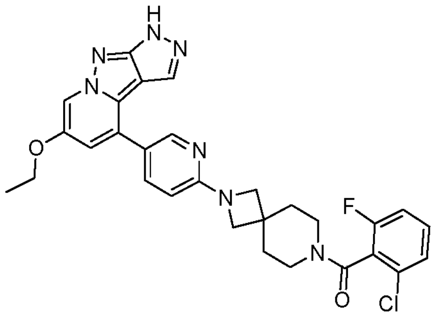 | 560 |
| 192 | 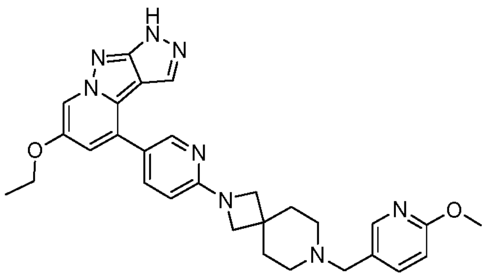 | 525 |
| 193 | 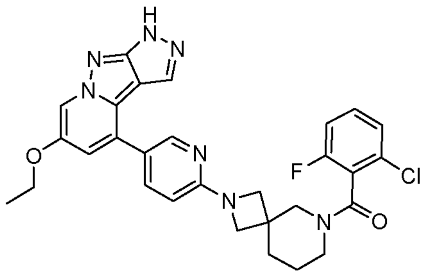 | 560 |
| 194 | 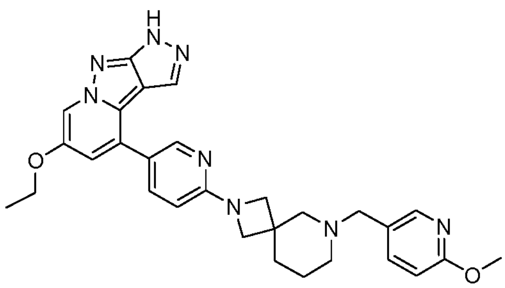 | 525 |

(continued)

| No. | Chemical structure | m/z [M+1] |
|---|---|---|
| 195 | 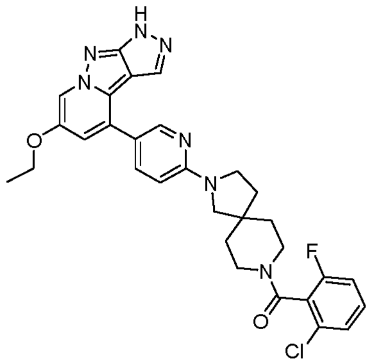 | 574 |
| 196 | 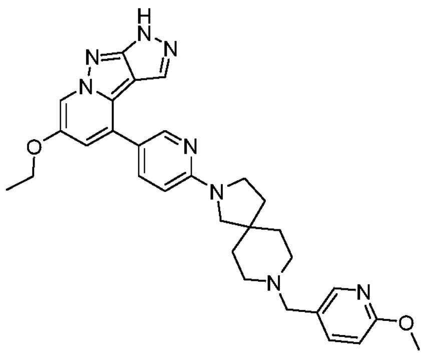 | 539 |
| 197 | 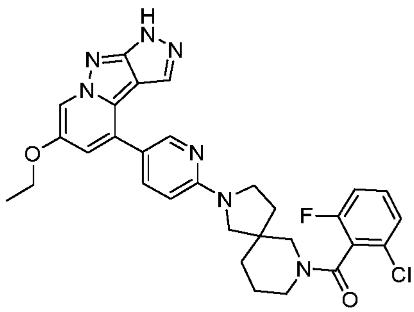 | 574 |
| 198 | 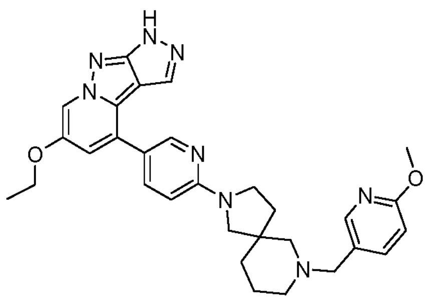 | 539 |
| 199 | 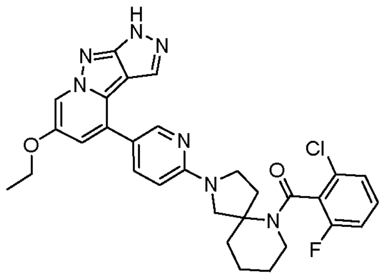 | 574 |

(continued)

| No. | Chemical structure | m/z [M+1] |
| --- | --- | --- |
| 200 | 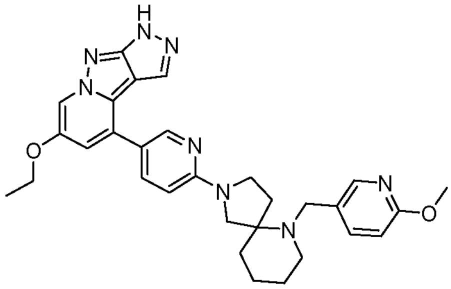 | 539 |
| 201 | 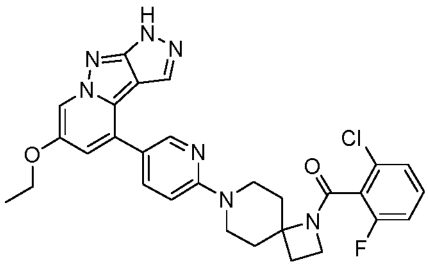 | 560 |
| 202 | 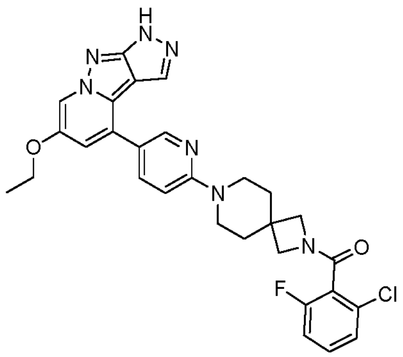 | 560 |
| 203 | 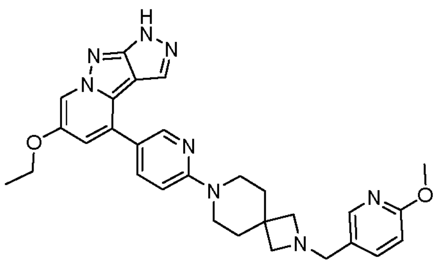 | 525 |
| 204 | 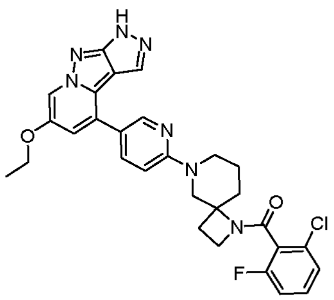 | 560 |

(continued)

| No. | Chemical structure | m/z [M+1] |
|---|---|---|
| 205 | 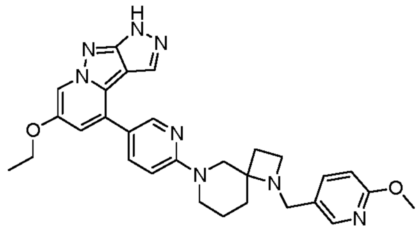 | 525 |
| 206 | 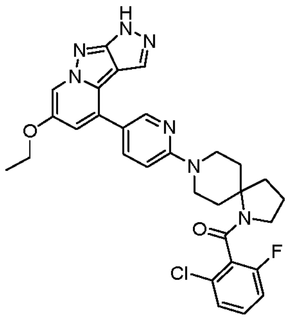 | 574 |
| 207 | 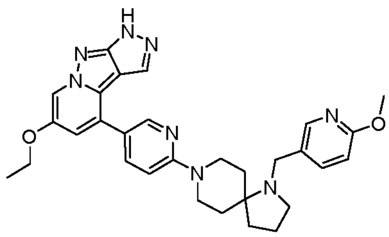 | 539 |
| 208 | 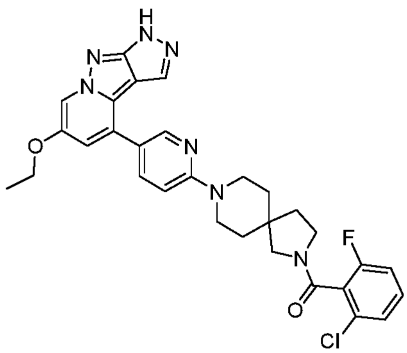 | 574 |
| 209 | 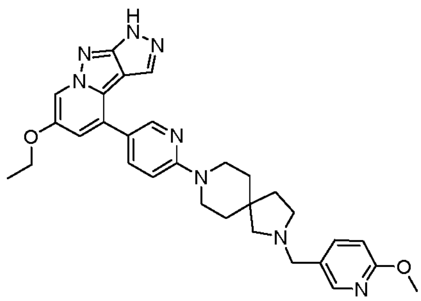 | 539 |

(continued)

| No. | Chemical structure | m/z [M+1] |
| --- | --- | --- |
| 210 | 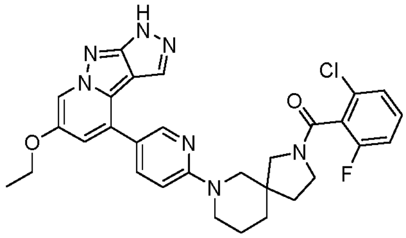 | 574 |
| 211 | 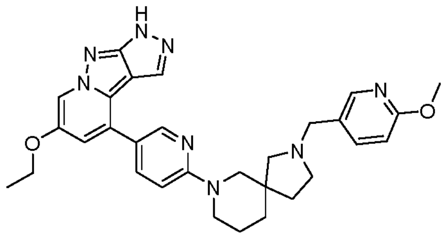 | 539 |
| 212 | 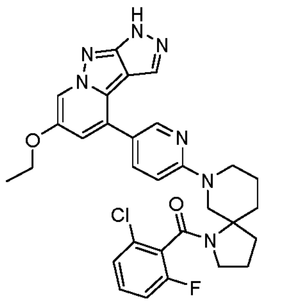 | 574 |
| 213 | 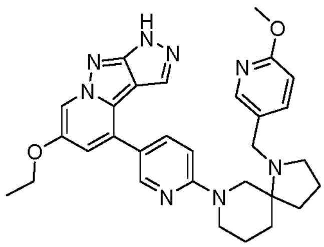 | 539 |
| 214 | 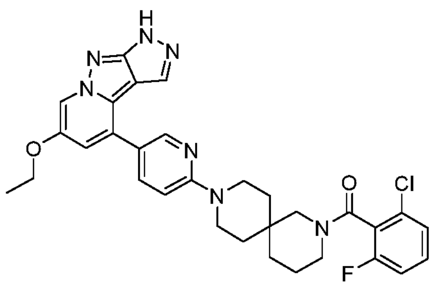 | 588 |

(continued)

| No. | Chemical structure | m/z [M+1] |
|---|---|---|
| 215 | 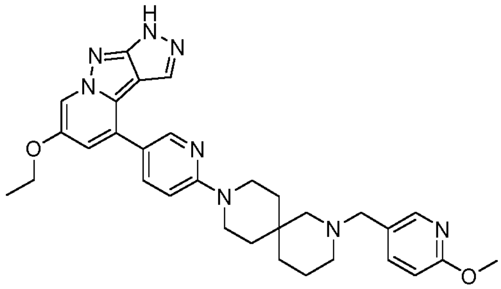 | 553 |
| 216 | 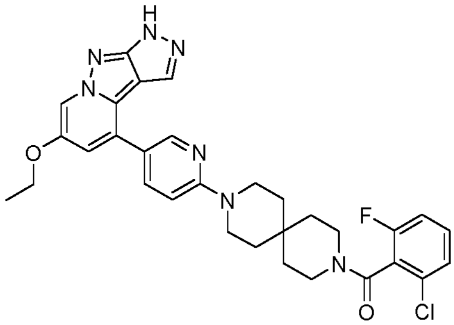 | 588 |
| 217 | 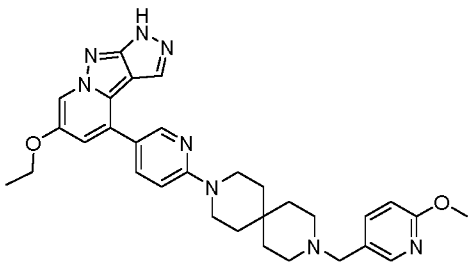 | 553 |
| 218 | 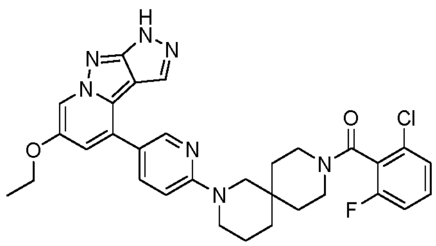 | 588 |
| 219 | 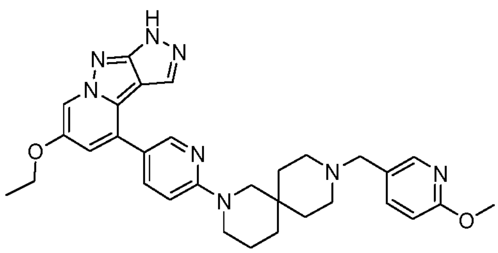 | 553 |

(continued)

| No. | Chemical structure | m/z [M+1] |
|---|---|---|
| 220 | 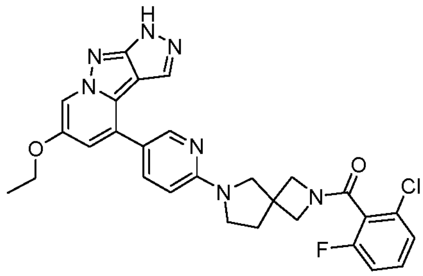 | 546 |
| 221 | 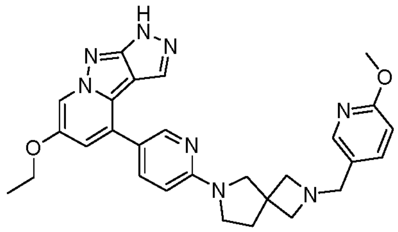 | 511 |
| 222 | 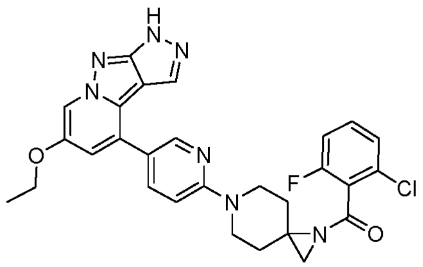 | 546 |
| 223 | 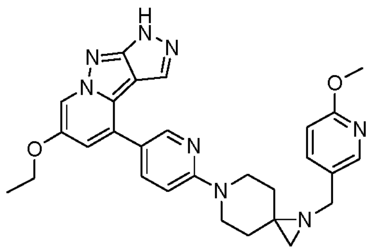 | 511 |
| 224 | 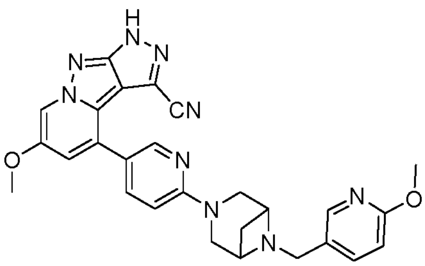 | 508 |
| 225 | 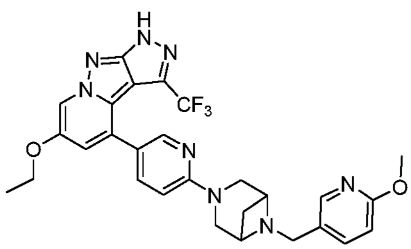 | 565 |

(continued)

| No. | Chemical structure | m/z [M+1] |
|---|---|---|
| 226 | 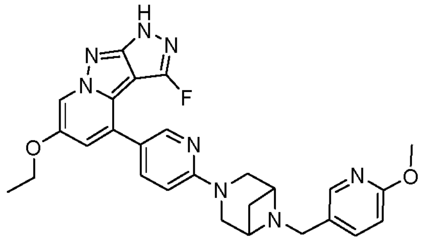 | 515 |
| 227 | 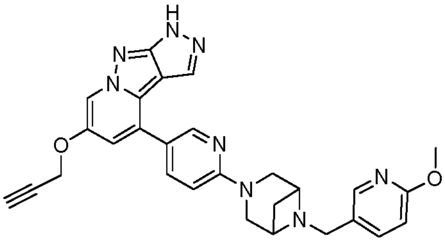 | 507 |
| 228 | 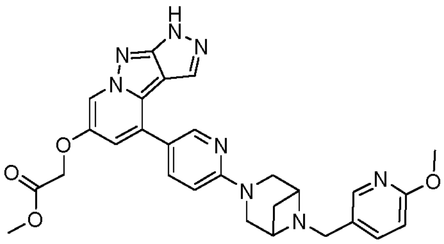 | 541 |
| 229 | 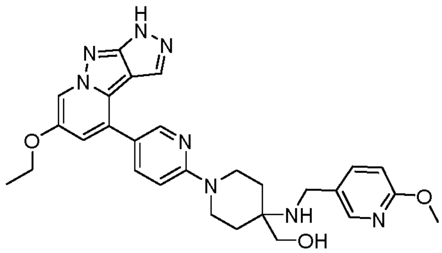 | 529 |
| 230 | 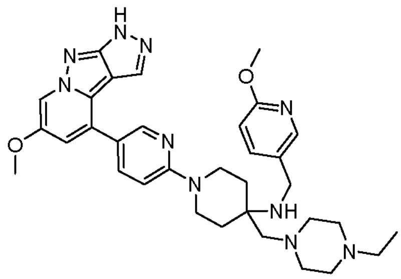 | 611 |
| 231 | 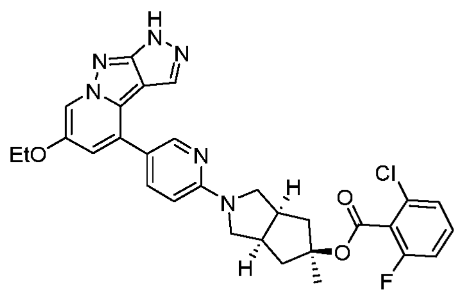 | 575 |

(continued)

| No. | Chemical structure | m/z [M+1] |
|---|---|---|
| 232 | 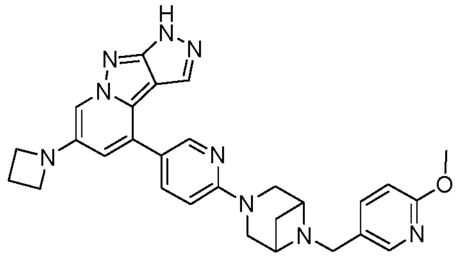 | 508 |
| 233 | 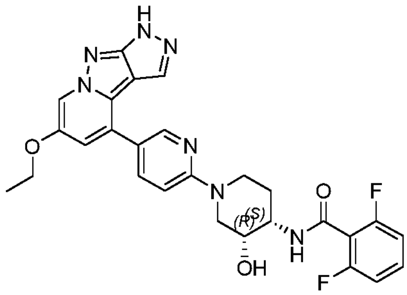 | 534 |
| 234 | 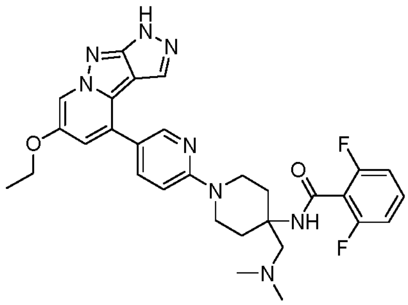 | 575 |
| 235 | 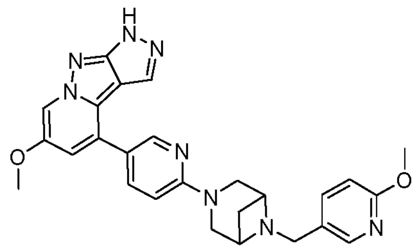 | 483 |
| 236 | 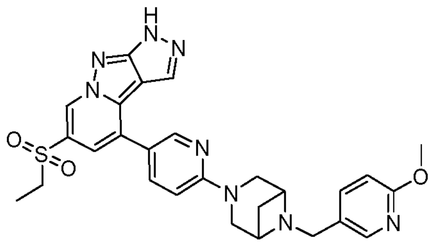 | 545 |

(continued)

| No. | Chemical structure | m/z [M+1] |
|---|---|---|
| 237 | 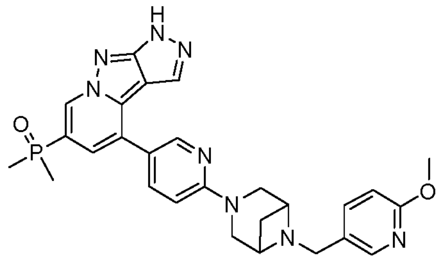 | 529 |
| 238 | 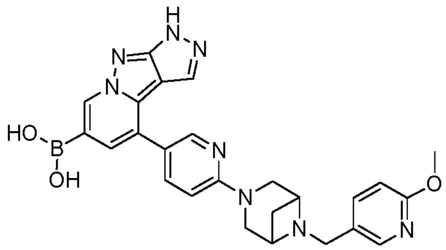 | 497 |
| 239 | 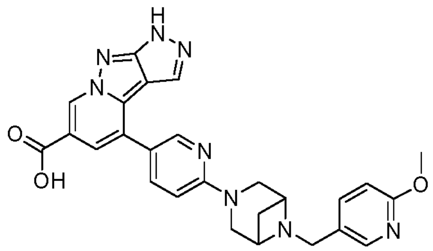 | 497 |
| 240 | 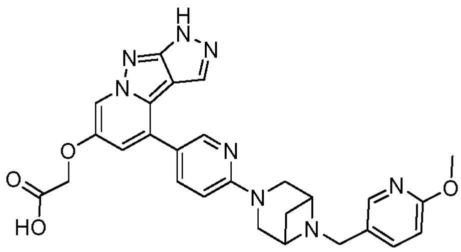 | 527 |
| 241 | 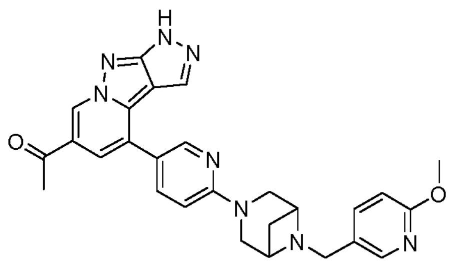 | 495 |
| 242 | 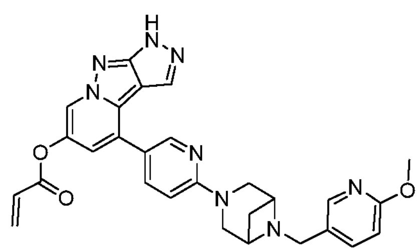 | 523 |

(continued)

| No. | Chemical structure | m/z [M+1] |
|---|---|---|
| 243 | 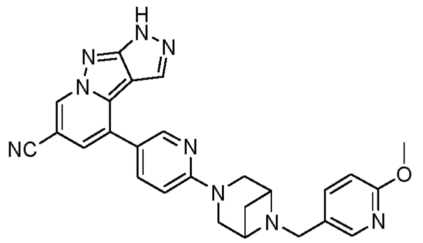 | 478 |
| 244 | 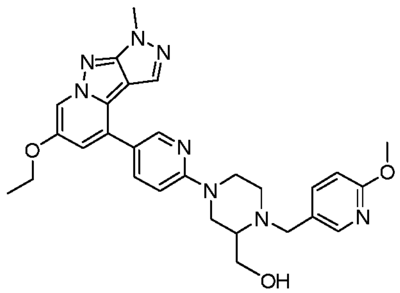 | 529 |
| 245 | 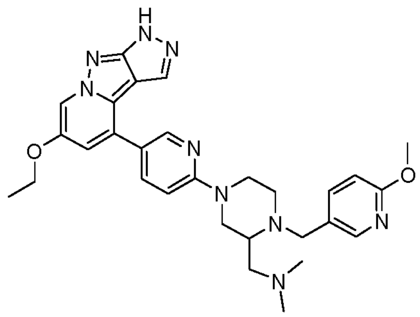 | 542 |
| 246 | 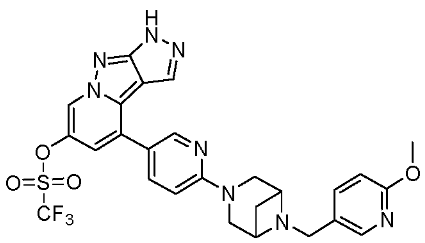 | 601 |
| 247 | 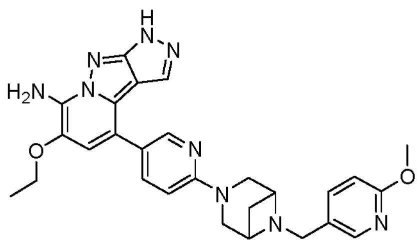 | 512 |
| 248 | 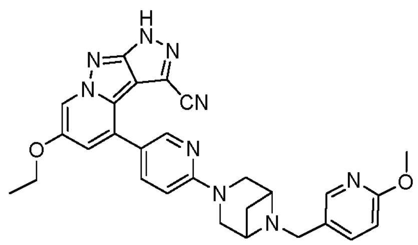 | 522 |

(continued)

| No. | Chemical structure | m/z [M+1] |
|---|---|---|
| 249 | 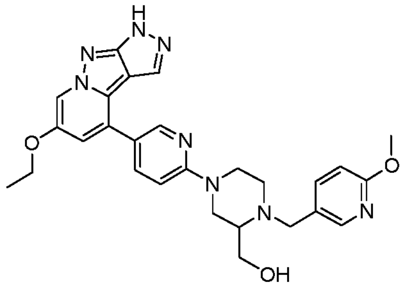 | 515 |
| 250 | 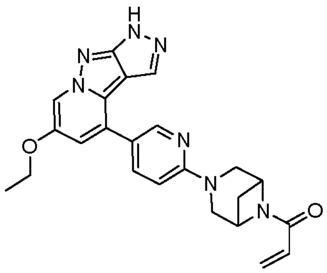 | 430 |
| 251 | 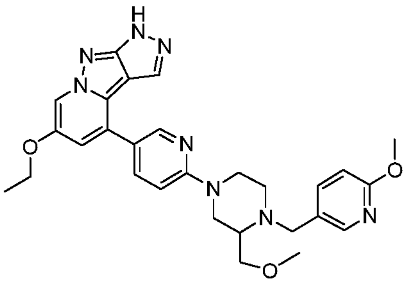 | 529 |
| 252 | 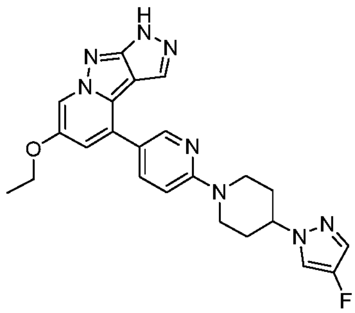 | 447 |
| 253 | 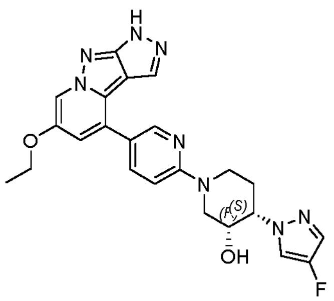 | 463 |

(continued)

| No. | Chemical structure | m/z [M+1] |
|---|---|---|
| 254 | 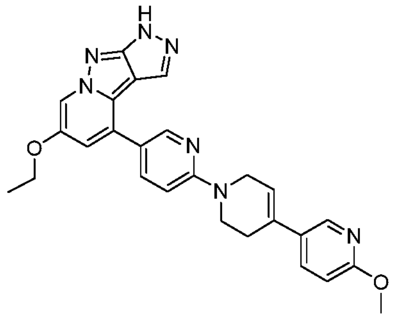 | 468 |
| 255 | 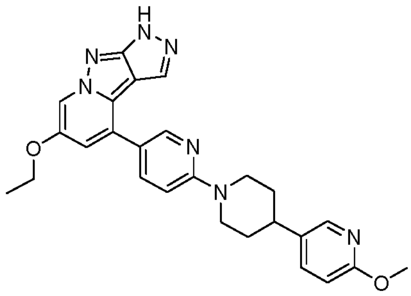 | 470 |
| 256 | 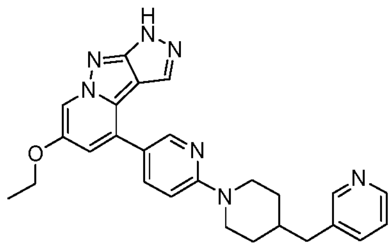 | 454 |
| 257 | 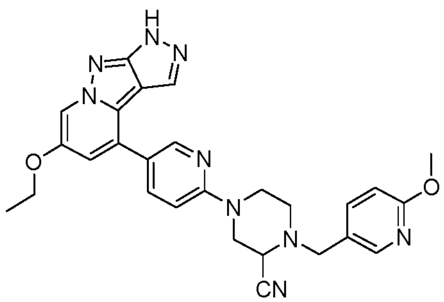 | 510 |
| 258 | 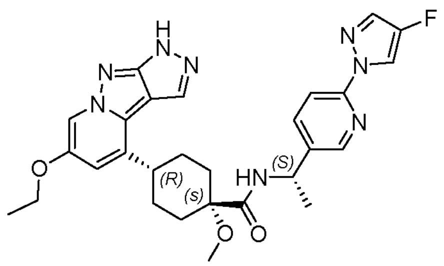 | 547 |

(continued)

| No. | Chemical structure | m/z [M+1] |
|---|---|---|
| 259 | 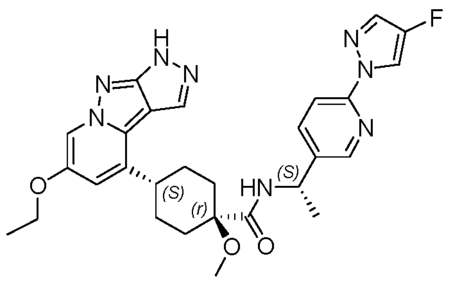 | 547 |
| 260 | 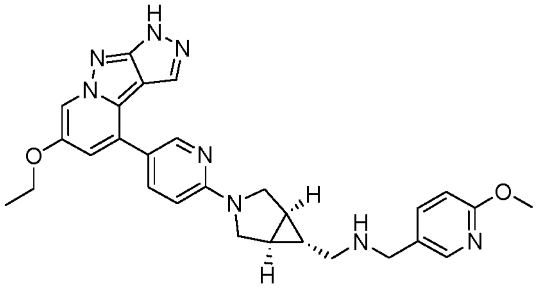 | 511 |
| 261 | 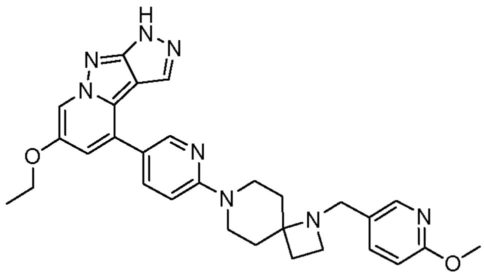 | 525 |
| 262 | 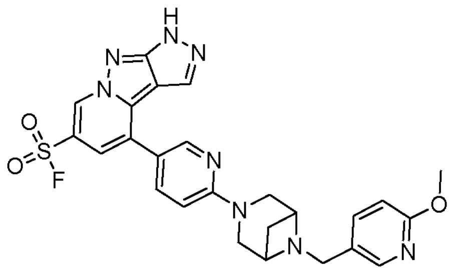 | 535 |
| 263 | 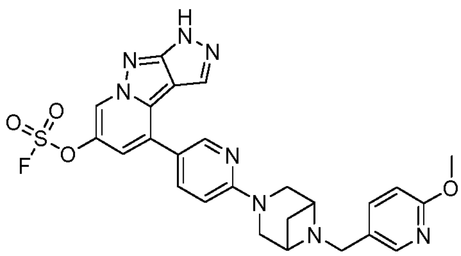 | 551 |

(continued)

| No. | Chemical structure | m/z [M+1] |
|---|---|---|
| 264 | 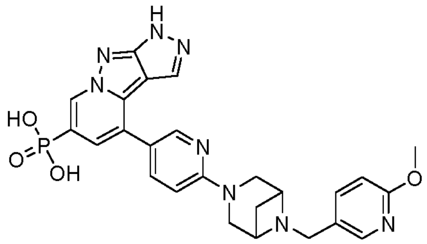 | 533 |
| 265 | 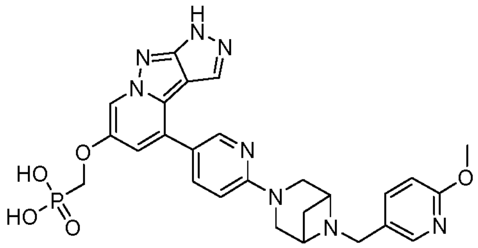 | 563 |

| No. | Chemical structure | m/z [M+1] |
|---|---|---|
| 266 | 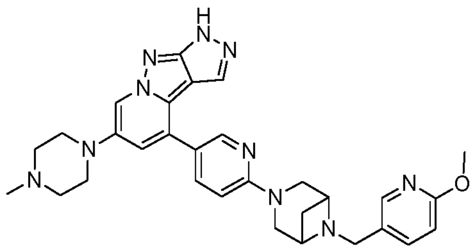 | 551 |
| 267 | 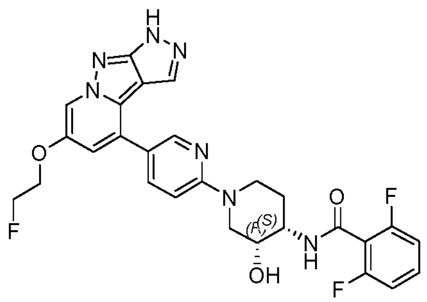 | 552 |
| 268 | 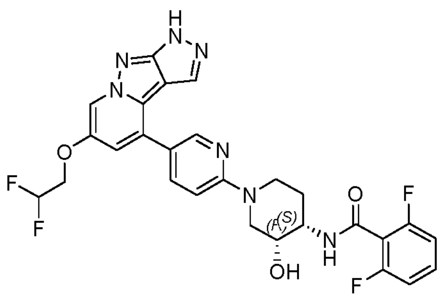 | 570 |

(continued)

| No. | Chemical structure | m/z [M+1] |
|---|---|---|
| 269 | 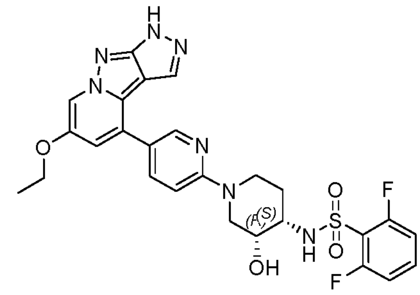 | 570 |
| 270 | 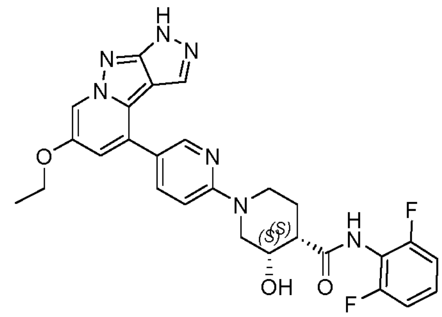 | 584 |
| 271 | 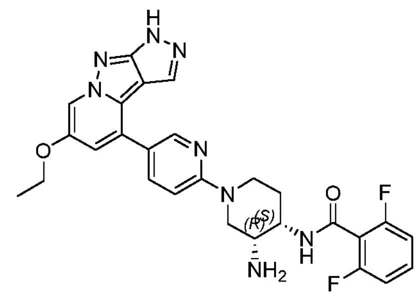 | 533 |
| 272 | 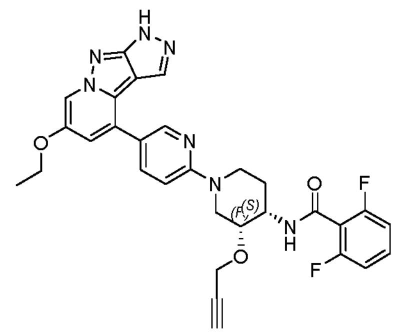 | 572 |
| 273 | 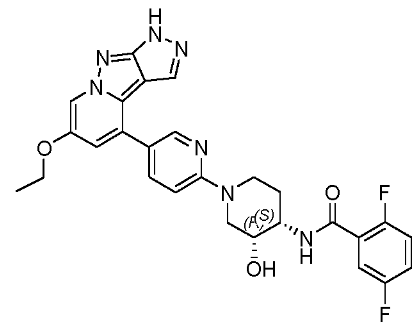 | 534 |

(continued)

| No. | Chemical structure | m/z [M+1] |
|---|---|---|
| 274 | 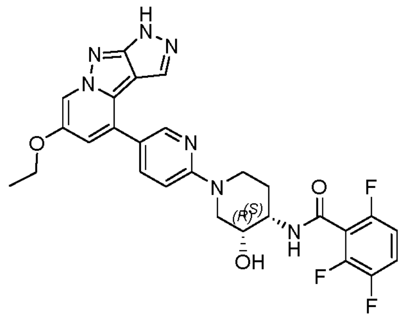 | 552 |
| 275 | 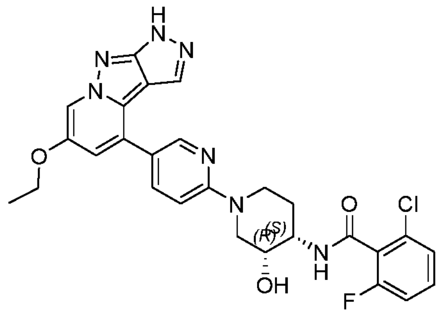 | 550 |
| 276 | 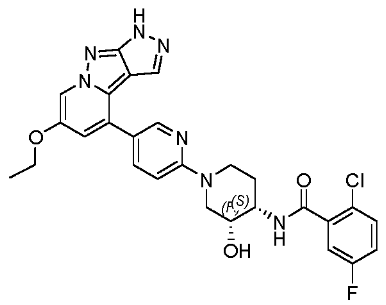 | 550 |
| 277 | 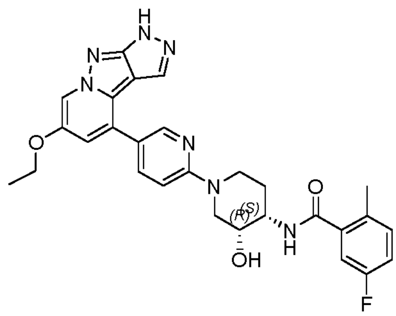 | 530 |
| 278 | 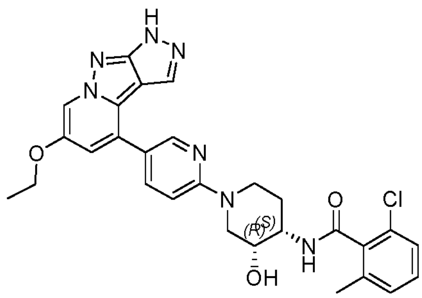 | 546 |

(continued)

| No. | Chemical structure | m/z [M+1] |
|---|---|---|
| 279 | 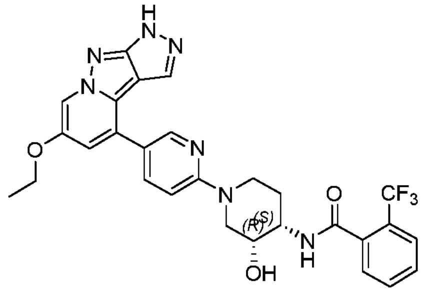 | 566 |
| 280 | 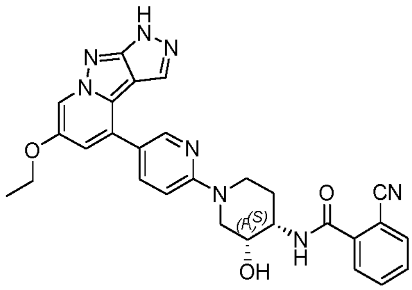 | 523 |
| 281 | 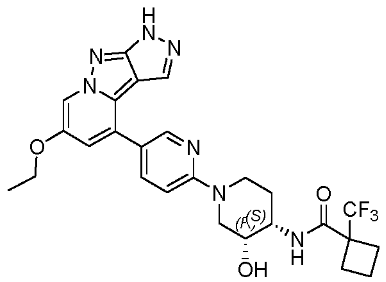 | 544 |
| 282 | 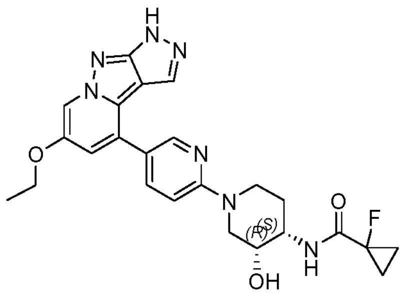 | 480 |
| 283 | 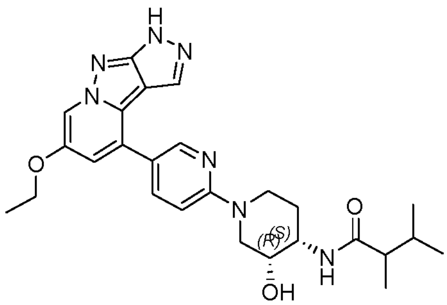 | 492 |

(continued)

| No. | Chemical structure | m/z [M+1] |
|---|---|---|
| 284 | 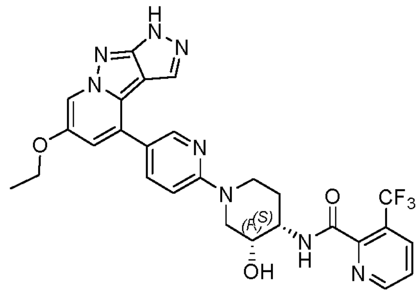 | 567 |
| 285 | 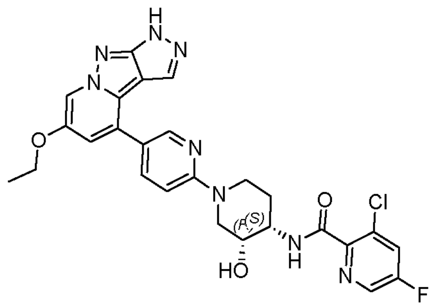 | 551 |
| 286 | 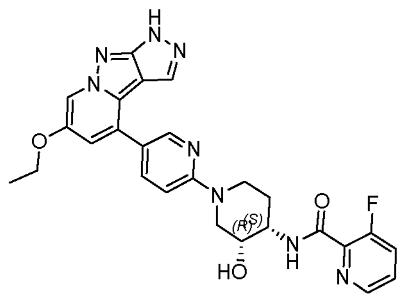 | 517 |
| 287 | 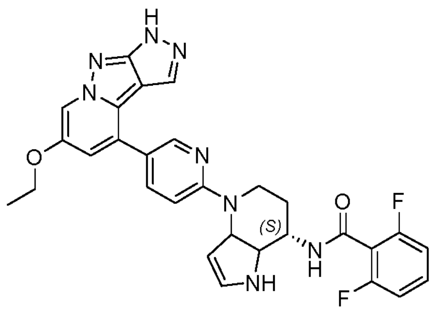 | 557 |
| 288 | 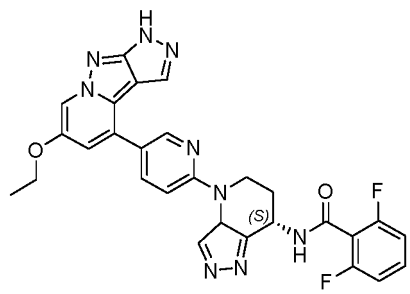 | 557 |

(continued)

| No. | Chemical structure | m/z [M+1] |
|---|---|---|
| 289 | 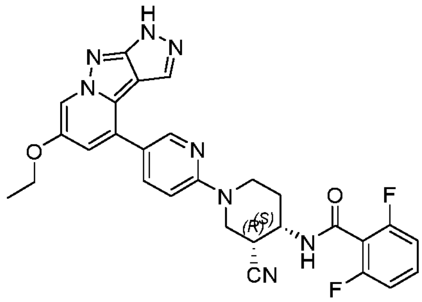 | 543 |
| 290 | 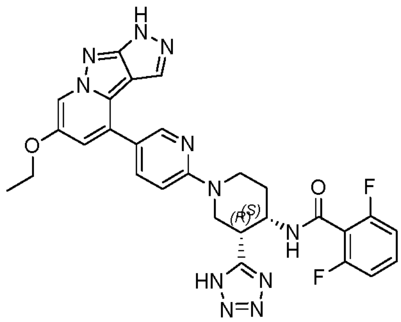 | 586 |
| 291 | 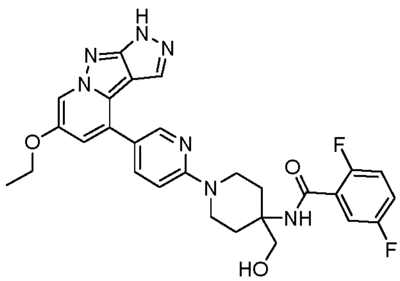 | 548 |
| 292 | 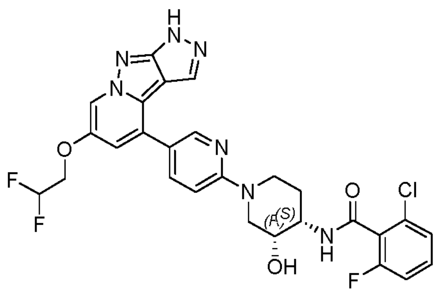 | 586 |
| 293 | 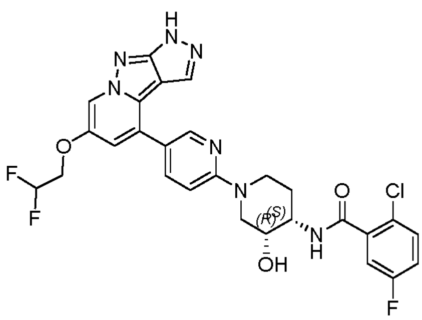 | 586 |

(continued)

| No. | Chemical structure | m/z [M+1] |
| --- | --- | --- |
| 294 | 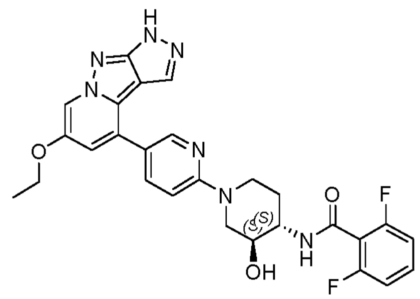 | 534 |
| 295 | 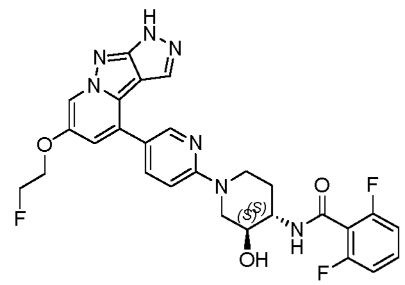 | 552 |
| 296 | 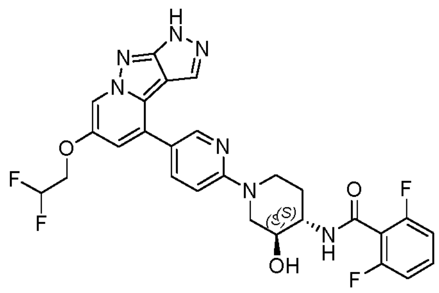 | 570 |
| 297 | 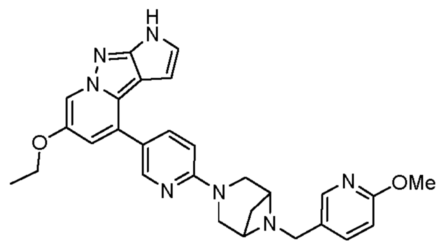 | 496 |

**Bioactivity Test Example**

**Test Example 1**

Rearranged during transfection (RET) is an identified proto-oncogene. It encodes a single transmembrane receptor tyrosine kinase that is necessary for the development, maturation and maintenance of many tissues and cell types. Under normal conditions, the binding of the ligand of glial cell line-derived neurotrophic factor (GDNF) family to RET on the cell surface leads to the dimerization and autophosphorylation of tyrosine residues in the cell. This in turn leads to the activation of downstream RAS-MAPK, PI3K-AKT, and phospholipase Cγ (PLCγ) pathways, and increases the survival and proliferation of cells. Examples of mutations that activate RET include C634W, M918T, and the gatekeeper mutations V804L, V804M, and G810R.

In this test, peptide substrates and a single proprietary monoclonal antibody are combined with HTRF technology, which is a highly sensitive and stable technology for detecting molecular interactions of proteins. The enzyme phosphorylates the substrates, and then the Eu-labeled antibody binds to the phosphorylated substrates, and streptavidin-XI,665 binds to all of the substrates. The TR-FRET signal is generated according to the HTRF principle. Once the inhibitor

(test compound) is added, a relatively weak TR-FRET signal is obtained. Based on this, the inhibitory effect is evaluated.

Reagents and consumables for kinase activity test

| Materials and reagents | Supplier | Model |
|---|---|---|
| HTRF KinEASE-TK kit | Cisbio | 62TK0PEC |
| Ret-wt | Carna | 08-159 |
| RET (V804M), activation | Signalchem | R02-12GG |
| RET G810R | Proqinase | 1724-0000-1 |
| DMSO | Sigma | D8418-1L |
| ATP | Promega | A769 |
| DTT | Sigma | D0632 |
| $MgCl_2$ | Sigma | M1028 |
| Cabozantinib | MCE | HY-13016 |
| Staurosporine | MCE | HY-15141 |
| Plate oscillator | Thermo | 4625-1CECN/THZ Q |
| Centrifuge | Eppendorf | 5810R |
| Envision 2104 multilable microplate reader | PerkinElmer | 2104-0010 |
| Echo | Labcyte | 550 |
| 384 polystyrene Shallow flat white | Greiner | 784075 |
| Microplate low speed centrifuge | Xiangzhi | TD5B |
| Biotek microplate reader | Biotek | Synergy 4 |
| 1.2 Preparation of solution | | |

All the compounds were dissolved in DMSO to prepare 10 mM stock solutions.
Cabozantinib was subjected to 3-fold serial dilution with DMSO from 10 mM and 1 mM, each for a total of 10 concentrations.
Other compounds were subjected to 3-fold serial dilution with DMSO from 10 mM (stock solution) for a total of 10 concentrations.
1000× positive control (1 mM cabozantinib and 0.2 mM staurosporine) and 1000× negative control

(100% DMSO) were prepared,
and shaken on a plate oscillator for 5 min.
1.3 Preparation of 1 × kinase buffer
4 volumes of distilled water were added to 1 volume of enzyme buffer 5×; 5 mM $MgCl_2$; 1 mM DTT.
1.4 Screening method

a) 10 nL of compound dilution was transferred to each well of the test plate;
b) the compound plate was centrifuged at 1000 g for 1 min;
c) the test plate was sealed;
d) 5× Ret-wt (0.2 ng/μL), 5× Ret V804M (0.5 ng/μL), and 2.5× RET G810R (2.5 ng/μL) in 1× kinase buffer were prepared;
e) 2 μL of 5× Ret-wt, 2 μL of Ret V804L, or 2 μL of RET G810R was added to a 384-well test plate;
f) 4 μL of 1× kinase buffer was added to each well of the test plate, and the sample plate was centrifuged at 1000g for 30 s and left to stand at room temperature for 10 min;
g) a solution of 5× TK-substrate-biotin (5 μM) in kinase buffer and 5× ATP (50 μM) in kinase buffer were prepared;
h) the reaction was initiated by adding 2 μL of STK-substrate-biotin and 2 μL of ATP (prepared in step g);

i) the sample plate was centrifuged at 1000 g for 30 s, and the test plate was sealed and left to stand at room temperature for 30 min;

j) 4× Sa-XI, 665 (250 nM) in HTRF assay buffer was prepared;

k) 5 μL of Sa-XI, 665 and 5 μL of TK-antibody-Cryptate (prepared in step i) were added to each well of the test plate;

l) the plate was centrifuged at 1000 g for 30 s, and left to stand at room temperature for 1 h; and m) fluorescence signal values of 615 nm/620 nm (G810R) (Cryptate) and 665 nm (XI,665) were read on an Envision 2104 microplate reader or BioTek microplate reader.

1.5 Data analysis

The ratio (665 nm/615 nm) in each well was calculated.
The inhibition rate % was calculated as follows:

$$\text{Inhibition rate \%} = [1 - (\text{test compound fluorescence signal value} — \text{positive control fluorescence signal value})/(\text{average negative control ratio} — \text{average positive control ratio})]*100\%$$

Ratio: generated from the measured fluorescence signal value
The average positive control ratio is the average ratio of positive control (20 μM cabozantinib) in the sample plate.
The average negative control ratio is the average ratio of negative control (0.1% DMSO) in the sample plate.
Some nonlinear fitting formulas were used to obtain the $IC_{50}$ values (half maximal inhibitory concentration) of the compounds: GraphPad 6.0 software was used for data analysis.

$$Y = Bottom + (Top\text{-}Bottom)/(1 + 10\text{^}((LogIC50\text{-}X)*Hill\ Slope))$$

X: Log value of compound concentration Y: Inhibition rate (% inhibition) Z' factor equation:

$$Z' = 1 - 3(SDmin+SDmax)/(AVEmax\text{-}AVEmin)$$

Min represents the positive control 20 μM Cabozantinib Ratio(665/620 nM* 10000), and Max represents the negative control DMSO Ration(665/620 nM* 10000).
SD represents the standard error, and AVE represents the average value of Ration(665/620 nM* 10000).
The kinase results for the example compounds are shown in Tables 2, 3, and 4:

Table 2

| Compound No. | RET-wt $IC_{50}$ (nM) |
|---|---|
| Compound 3 | 0.1 |
| Compound 4 | 0.1 |
| Compound 11 | 0.1 |
| Compound 39 | 0.1 |
| Compound 40 | 0.1 |
| Compound 41 | 0.1 |
| Compound 42 | 0.1 |
| Compound 44 | 0.1 |
| Compound 46 | 0.1 |
| Compound 88 | 0.1 |
| Cabozantinib | 20.2 |

Table 3

| Compound No. | RET V804M $IC_{50}$ (nM) |
|---|---|
| Compound 3 | 0.1 |
| Compound 4 | 0.03 |
| Compound 11 | 0.1 |
| Compound 39 | 0.1 |
| Compound 40 | 0.1 |
| Compound 41 | 0.1 |
| Compound 42 | 0.05 |
| Compound 44 | 0.1 |
| Compound 46 | 0.06 |
| Compound 88 | 0.2 |
| Cabozantinib | 89.3 |

Table 4

| Compound No. | RET G810R $IC_{50}$ (nM) |
|---|---|
| Compound 1 | 6.5 |
| Compound 3 | 2.9 |
| Compound 4 | 2.3 |
| Compound 8 | 8.7 |
| Compound 11 | 0.36 |
| Compound 12 | 2.1 |
| Compound 15 | 1.5 |
| Compound 17 | 2.1 |
| Compound 29 | 8.8 |
| Compound 31 | 2.6 |
| Compound 33 | 2.5 |
| Compound 34 | 4.9 |
| Compound 35 | 5.1 |
| Compound 36 | 5.7 |
| Compound 37 | 6.3 |
| Compound 39 | 4.2 |
| Compound 40 | 2.2 |
| Compound 41 | 2.8 |
| Compound 42 | 1.4 |
| Compound 43 | 1.9 |
| Compound 44 | 0.8 |
| Compound 45 | 5.2 |
| Compound 46 | 1.6 |

(continued)

| Compound No. | RET G810R $IC_{50}$ (nM) |
|---|---|
| Compound 48 | 2.9 |
| Compound 50 | 17.9 |
| Compound 51 | 4.3 |
| Compound 55 | 4.5 |
| Compound 56 | 23.6 |
| Compound 57 | 6.9 |
| Compound 58 | 5.6 |
| Compound 59 | 4.6 |
| Compound 87 | 9.4 |
| Compound 88 | 7.8 |
| Compound 89 | 2.0 |
| Compound 91 | 2.2 |
| Compound 101 | 6.6 |
| Compound 102 | 3.6 |
| Compound 103 | 11.1 |
| Compound 104 | 6.4 |
| Compound 107 | 14.5 |
| Compound 114 | 3.6 |
| Compound 118 | 7.7 |
| Compound 149 | 4.7 |
| Compound 150 | 4.4 |
| Compound 152 | 4.1 |
| Compound 155 | 14.2 |
| Compound 156 | 11.4 |
| Compound 157 | 8.6 |
| Compound 158 | 18.7 |
| Compound 159 | 16.1 |
| Compound 160 | 8.2 |
| Compound 165 | 38.3 |
| Compound 169 | 4.8 |
| Compound 170 | 27.3 |
| Compound 172 | 8.2 |
| Compound 173 | 12.5 |
| Compound 174 | 3.5 |
| Compound 176 | 12.1 |
| Compound 180 | 9.7 |
| Compound 183 | 8.0 |
| Compound 188 | 16.9 |

(continued)

| Compound No. | RET G810R $IC_{50}$ (nM) |
|---|---|
| Compound 189 | 15.3 |
| Compound 193 | 33.7 |
| Compound 201 | 4.1 |
| Compound 207 | 49.0 |
| Compound 208 | 24.1 |
| Compound 214 | 47.3 |
| Compound 219 | 66.4 |
| Compound 220 | 26.2 |
| Compound 227 | 4.01 |
| Compound 228 | 5.7 |
| Compound 229 | 38.1 |
| Compound 233 | 0.96 |
| Compound 234 | 1.6 |
| Compound 235 | 2.2 |
| Compound 237 | 112.1 |
| Compound 239 | 33.7 |
| Compound 240 | 4.0 |
| Compound 242 | 4.7 |
| Compound 243 | 89.0 |
| Compound 245 | 20.8 |
| Compound 248 | 15.5 |
| Compound 249 | 13.5 |
| Compound 250 | 40.8 |
| Compound 258 | 9.9 |
| Compound 267 | 3.0 |
| Compound 268 | 2.8 |
| Compound 269 | 6.2 |
| Compound 273 | 1.8 |
| Compound 274 | 1.3 |
| Compound 275 | 0.8 |
| Compound 276 | 0.7 |
| Compound 277 | 1.2 |
| Compound 278 | 0.9 |
| Compound 279 | 2.2 |
| Compound 280 | 2.1 |
| Compound 281 | 1.5 |
| Compound 282 | 3.5 |
| Compound 283 | 9.9 |

(continued)

| Compound No. | RET G810R $IC_{50}$ (nM) |
|---|---|
| Compound 284 | 4.2 |
| Compound 285 | 5.0 |
| Compound 286 | 10.3 |
| Compound 291 | 1.6 |
| Compound 292 | 1.8 |
| Compound 293 | 1.6 |
| Compound 294 | 4.1 |
| Compound 295 | 2.1 |
| Compound 296 | 2.1 |
| Compound 297 | 9.6 |
| Selpercatinib(LOXO-292) | 43.2 |
| Cabozantinib | 325.5 |

The results of the kinase and cell tests described above showed that the compounds of the present disclosure, in particular, the compounds of the examples described above, had excellent inhibitory activity against RET-wt, RET V804M, and RET G810R. Among them, compound 3, compound 4, compound 11, compound 39, compound 40, compound 41, compound 42, compound 44, compound 46, and compound 88 all had significantly superior inhibitory activity against RET-wt and RET V804M to that of cabozantinib. Compound 3, compound 4, compound 11, compound 12, compound 15, compound 17, compound 31, compound 33, compound 34, compound 39, compound 40, compound 41, compound 42, compound 43, compound 44, compound 46, compound 48, compound 51, compound 55, compound 59, compound 88, compound 89, compound 91, compound 101, compound 102, compound 103, compound 104, compound 107, compound 114, compound 118, compound 149, compound 150, compound 152, compound 155, compound 156, compound 157, compound 158, compound 159, compound 160, compound 165, compound 169, compound 170, compound 172, compound 173, compound 174, compound 176, compound 180, compound 183, compound 188, compound 189, compound 193, compound 201, compound 208, compound 220, compound 227, compound 228, compound 229, compound 233, compound 234, compound 235, compound 239, compound 240, compound 242, compound 245, compound 248, compound 249, compound 258, compound 267, compound 268, compound 269, compound 273, compound 274, compound 275, compound 276, compound 277, compound 278, compound 279, compound 280, compound 281, compound 282, compound 283, compound 284, compound 285, compound 286, compound 291, compound 292, compound 293, compound 294, compound 295, compound 296, and compound 297 also had significantly superior inhibitory activity against RET G810R to that of cabozantinib and selpercatinib (LOXO-292).

In particular, compound 3, compound 4, compound 11, compound 12, compound 15, compound 17, compound 31, compound 33, compound 40, compound 41, compound 42, compound 43, compound 44, compound 46, compound 48, compound 89, compound 91, compound 102, compound 114, compound 174, compound 233, compound 234, compound 235, compound 267, compound 268, compound 269, compound 273, compound 274, compound 275, compound 276, compound 277, compound 278, compound 279, compound 280, compound 281, compound 282, compound 291, compound 292, compound 293, compound 295, compound 296, and compound 297 had inhibitory activity against RET G810R which is 10 times or higher than that of selpercatinib (LOXO-292). Therefore, the compounds of the present disclosure have a significant effect on the solvent front G810 drug-resistant mutation generated after treatment with selective RET inhibitors such as selpercatinib (LOXO-292), and are expected to be used for treating patients with resistance to the selective RET inhibitors.

The exemplary embodiments of the present disclosure have been described above. However, it should be understood that the protection scope of the present disclosure is not limited to the exemplary embodiments described above. Any modifications, equivalents, improvements and the like made without departing from the spirit and principle of the present disclosure shall fall within the protection scope of the present disclosure.

## Claims

1. A compound represented by formula I, or a stereoisomer, a racemate, a tautomer, an isotopically labeled compound, a nitrogen oxide or a pharmaceutically acceptable salt thereof:

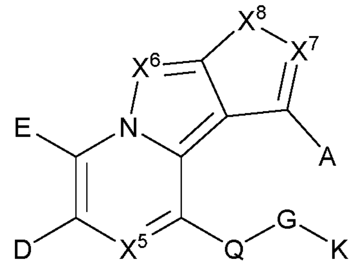

I

wherein Q is selected from

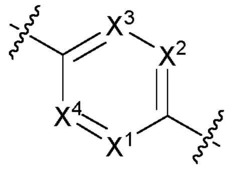

and

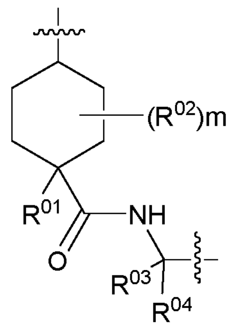

;

$X^1$, $X^2$, $X^3$, $X^4$, $X^5$, $X^6$, and $X^7$ are identical or different, and are independently selected from $CR^1$ and N;
$X^8$ is selected from $CR^1R^{1'}$ and $NR^1$;
wherein each $R^1$ and $R^{1'}$ is identical or different, and is independently selected from H, halogen, CN, OH, and the following groups unsubstituted or optionally substituted with one, two, or more substituents independently selected from $R^a$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $NR^2R^3$, $-C(O)R^4$, $-OCR^5$, $-S(O)_2R^6$, and $OS(O)_2R^7$;
A is selected from H, halogen, CN, OH, $NH_2$, and the following groups unsubstituted or optionally substituted with one, two, or more substituents independently selected from $R^b$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $NR^2R^3$, $-C(O)R^4$, $-OCR^5$, $-S(O)_2R^6$, and $OS(O)_2R^7$;
D and E are identical or different, and are independently selected from H, halogen, CN, OH, $B(OH)_2$, $-C(O)R^4$, $-OCR^5$, $-S(O)_2R^6$, $OS(O)_2R^7$, $-O-R^{21}$, $C(O)OR^{22}$, $-P(O)R^{23}R^{24}$, and the following groups unsubstituted or optionally substituted with one, two, or more substituents independently selected from $R^c$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, and $NH_2$, provided that at least one of D and E is not H, e.g., at least one of D and E is selected from $-O-R^{21}$, or at least one of D and E is selected from the following groups unsubstituted or optionally substituted with one, two, or more substituents independently selected from $R^c$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, and $NH_2$;
$R^{21}$, $R^{22}$, $R^{23}$, and $R^{24}$ are identical or different, and are independently selected from H and the following groups unsubstituted or optionally substituted with one, two, or more substituents independently selected from $R^b$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, and 3- to 20-membered heterocyclyl;
G is selected from halogen and the following groups unsubstituted or optionally substituted with one, two, or more substituents independently selected from $R^e$: 5- to 20-membered heteroaryl having at least one heteroatom selected from N, 3- to 20-membered heterocyclyl having at least one heteroatom selected from N, and $C_{3-40}$ cycloalkyl-NH-;

K is absent or selected from H, halogen, CN, OH, and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$, $-OCR^5$, $-S(O)_2R^6$, and $OS(O)_2R^7$;

each $R^2$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $-C(O)R^4$, and $-S(O)_2R^6$;

each $R^3$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $-C(O)R^4$, and $-S(O)_2R^6$;

or, $R^2$ and $R^3$, together with the N atom attached thereto, form the following groups unsubstituted or optionally substituted with one, two, or more substituents independently selected from $R^f$: 5- to 20-membered heteroaryl and 3- to 20-membered heterocyclyl;

each $R^4$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, and $-NR^2R^3$;

each $R^5$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkylcarbonyl, $C_{2-40}$ alkenylcarbonyl, $C_{2-40}$ alkynylcarbonyl, $C_{3-40}$ cycloalkylcarbonyl, $C_{3-40}$ cycloalkenylcarbonyl, $C_{3-40}$ cycloalkynylcarbonyl, $C_{6-20}$ arylcarbonyl, 5- to 20-membered heteroarylcarbonyl, and 3- to 20-membered heterocyclylcarbonyl;

each $R^6$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, and $-NR^2R^3$;

each $R^7$ is identical or different, and is independently selected from H and the following groups unsubstituted or optionally substituted with one, two, or more substituents independently selected from $R^f$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, and 3- to 20-membered heterocyclyl;

m is 0, 1, 2, 3, 4, 5, 6, 7, or 8;

each $R^{01}$, $R^{02}$, $R^{03}$, and $R^{04}$ is identical or different, and is independently selected from H, halogen, CN, OH, SH, oxo (=O), $NO_2$, and the following groups unsubstituted or optionally substituted with one, two, or more substituents independently selected from $R^g$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$, $-OCR^5$, $-S(O)_2R^6$, and $OS(O)_2R^7$;

each $R^a$, $R^b$, $R^c$, $R^d$, $R^e$, and $R^f$ is identical or different, and is independently selected from halogen, CN, OH, SH, oxo (=O), $NO_2$, and the following groups unsubstituted or optionally substituted with one, two, or more substituents independently selected from $R^g$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$,

$-OCR^5$, $-S(O)_2R^6$, and $OS(O)_2R^7$;

each $R^g$ is identical or different, and is independently selected from halogen, CN, OH, SH, oxo (=O), $NO_2$, and the following groups unsubstituted or optionally substituted with one, two, or more substituents independently selected from $R^h$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$, $-OCR^5$, $-S(O)_2R^6$, and $OS(O)_2R^7$; or, when a cyclic group (including but not limited to, $C_{3-40}$ cycloalkyl, $C_{3-40}$ cycloalkenyl, $C_{3-40}$ cycloalkynyl, 3- to 20-membered heterocyclyl, and the like) is substituted with two or more substituents at different positions, two of the substituents can also form a bridged ring with the cyclic group, wherein the bridge atoms other than the bridgehead atoms in the bridged ring can comprise 1, 2, 3, 4, or 5 divalent groups selected from $CH_2$, O, and NH;

each $R^h$ is identical or different, and is independently selected from halogen, CN, OH, SH, oxo (=O), $NO_2$, and the following groups unsubstituted or optionally substituted with one, two, or more substituents independently selected from $R^i$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$, $-OCR^5$, $-S(O)_2R^6$, and $OS(O)_2R^7$;

each $R^i$ is identical or different, and is independently selected from halogen, CN, OH, SH, oxo (=O), $NO_2$, and the following groups unsubstituted or optionally substituted with one, two, or more substituents $R^j$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5- to 20-membered heteroaryl, 3- to 20-membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5- to 20-membered heteroaryloxy, 3- to 20-membered heterocyclyloxy, $C_{6-20}$ aryl $C_{1-40}$ alkyl, 5- to 20-membered heteroaryl $C_{1-40}$ alkyl, 3- to 20-membered heterocyclyl $C_{1-40}$ alkyl, $-NR^2R^3$, $-C(O)R^4$, $-OCR^5$, $-S(O)_2R^6$, and $OS(O)_2R^7$;

or, when a cyclic group (including but not limited to, $C_{3-40}$ cycloalkyl, $C_{3-40}$ cycloalkenyl, $C_{3-40}$ cycloalkynyl, 3- to 20-membered heterocyclyl, and the like) is substituted with two or more substituents at different positions, two of the substituents can also form a bridged ring with the cyclic group, wherein the bridge atoms other than the bridgehead atoms in the bridged ring can comprise 1, 2, 3, 4, or 5 divalent groups selected from $CH_2$, O, and NH;

or, when one atom (e.g., carbon atom or nitrogen atom) is substituted with two or more substituents, two of the substituents can also, together with the shared atom connected thereto, form the following groups unsubstituted or optionally substituted with one, two, or more substituents independently selected from $R^f$: cyclic groups unsubstituted or optionally substituted with one, two, or more substituents independently selected from $R^f$ (including but not limited to, the following groups unsubstituted or optionally substituted with one, two, or more substituents independently selected from $R^f$: $C_{3-40}$ cycloalkyl, $C_{3-40}$ cycloalkenyl, $C_{3-40}$ cycloalkynyl, 5- to 20-membered heteroaryl, 3- to 20-membered heterocyclyl, and the like).

**2.** The compound, or the stereoisomer, the racemate, the tautomer, the isotopically labeled compound, the nitrogen oxide or the pharmaceutically acceptable salt thereof as claimed in claim 1, wherein $X^1$, $X^2$, $X^3$, $X^4$, $X^5$, $X^6$, and $X^7$ are identical or different, and are independently selected from $CR^1$ and N; e.g., at least one of $X^1$, $X^2$, $X^3$, $X^4$, $X^5$, $X^6$, and $X^7$ is N;

$X^8$ is selected from $CR^1R^{1'}$ and $NR^1$;

each $R^1$ and $R^{1'}$ is identical or different, and is independently selected from H, halogen, CN, OH, $C_{1-6}$ alkyl, $C_{3-10}$ cycloalkyl, and $C_{1-6}$ alkoxy;

A is selected from H, halogen, CN, OH, $C_{1-6}$ alkyl, and $C_{1-6}$ alkyloxy;

D and E are identical or different, and are independently selected from H, halogen, CN, $NH_2$ unsubstituted or optionally substituted with one, two, or more substituents independently selected from $R^c$, and $-O-R^{21}$, provided that at least one of D and E is not H, e.g., at least one of D and E is selected from $-O-R^{21}$ and $NH_2$ unsubstituted or optionally substituted with one, two, or more substituents independently selected from $R^c$;

$R^{21}$ is selected from $C_{1-6}$ alkyl unsubstituted or optionally substituted with one, two, or more substituents $R^d$;

$R^2$ and $R^3$, together with the N atom attached thereto, form the following groups unsubstituted or optionally substituted with one, two, or more substituents independently selected from $R^f$: 5- to 20-membered heteroaryl and 3- to 20-membered heterocyclyl, e.g., 5-, 6-, or 7-membered heteroaryl or 3-, 4-, 5-, 6-, or 7-membered

heterocyclyl;

each $R^{01}$, $R^{02}$, $R^{03}$, and $R^{04}$ is identical or different, and is independently selected from H, $C_{1-6}$ alkyl, and $C_{1-6}$ alkoxy;

each $R^a$, $R^b$, $R^c$, $R^d$, $R^e$, and $R^f$ is identical or different, and is independently selected from halogen, $NH_2$, CN, OH, and the following groups unsubstituted or optionally substituted with one, two, or more substituents $R^g$: $C_{1-6}$ alkyl, $C_{1-6}$ alkyloxy, $C_{3-10}$ cycloalkyl, $C_{3-10}$ cycloalkyloxy, $C_{2-6}$ alkynyloxy, 3- to 8-membered heterocyclyl, and 3- to 8-membered heteroaryl;

each $R^g$ is identical or different, and is independently selected from OH, halogen, and $C_{3-10}$ cycloalkyl;

G is selected from halogen, $C_{3-10}$ cycloalkyl, $C_{3-10}$ cycloalkyl-NH-, $C_{6-14}$ aryl, 5- to 14-membered heteroaryl, 3- to 12-membered heterocyclyl, e.g., 6- to 12-membered heterocyclyl having a monocyclic, bicyclic, tricyclic, or bridged ring structure comprising 1, 2, 3, or 4 heteroatoms independently selected from N, O, and S, provided that at least one of the heteroatoms is selected from N, e.g., 1, 2, or 3 heteroatoms are selected from N;

K is selected from -$C_{1-6}$ alkyl-$C_{3-10}$ cycloalkyl, -$C_{1-6}$ alkyl-$C_{6-14}$ aryl, -$C_{1-6}$ alkyl-5- to 14-membered heteroaryl, -$C_{1-6}$ alkyl-3- to 10-membered heterocyclyl, -C(O)$NH_2$, -C(O)-$C_{3-10}$ cycloalkyl, -C(O)-$C_{6-14}$ aryl, -C(O)-5- to 14-membered heteroaryl, -C(O)-3- to 10-membered heterocyclyl, -C(O)-$C_{1-6}$ alkyl-$C_{3-10}$ cycloalkyl, -C(O)-$C_{1-6}$ alkyl-$C_{6-14}$ aryl, -C(O)-$C_{1-6}$ alkyl-5- to 14-membered heteroaryl, and -C(O)-$C_{1-6}$ alkyl-3- to 10-membered heterocyclyl, wherein a group on a ring or acyclic group of the $C_{3-10}$ cycloalkyl, $C_{6-14}$ aryl, 5- to 14-membered heteroaryl, 3- to 10-membered heterocyclyl, -C(O)-$C_{3-10}$ cycloalkyl, -C(O)-$C_{6-14}$ aryl, -C(O)-5- to 14-membered heteroaryl, -C(O)-3- to 10-membered heterocyclyl, -C(O)-$C_{1-6}$ alkyl-$C_{3-10}$ cycloalkyl, -C(O)-$C_{1-6}$ alkyl-$C_{6-14}$ aryl, -C(O)-$C_{1-6}$ alkyl-5- to 14-membered heteroaryl, or -C(O)-$C_{1-6}$ alkyl-3- to 10-membered heterocyclyl, or -C(O)NHz is further optionally substituted with one, two, or more groups selected from OH, halogen, CN, $C_{1-6}$ alkyl, and $C_{1-6}$ alkyloxy; wherein the heterocyclyl can be pyridinyl (e.g., pyridin-2-yl, pyridin-3-yl, pyridin-4-yl, pyridin-5-yl, or pyridin-6-yl), and the aryl can be phenyl;

or, K is absent or selected from H, OH, and the following groups unsubstituted or optionally substituted with one, two, or more substituents independently selected from $R^f$: phenyl-C(O)-, phenyl-C(O)-NH-, phenyl-C(O)-NH-$C_{1-6}$ alkyl-, phenyloxy-C(O)-NH-, phenylalkyl-NH-C(O)-, phenylalkyl-C(O)-NH-, pyridinyl-C(O)-, pyridinyl-C(O)-NH-, pyridinyl-C(O)-NH-$C_{1-6}$ alkyl-, pyridinyloxy-C(O)-NH-, pyridinylalkyl-NH-C(O)-, pyridinylalkyl-C(O)-NH-, pyrrolidinyl-C(O)-NH-, pyrrolidinyloxy-C(O)-NH-, $C_{1-6}$ alkyl-C(O)-, $C_{1-6}$ alkyl-C(O)-NH-, $C_{1-6}$ alkoxy-C(O)-NH-, $C_{3-8}$ cycloalkyl-C(O)-NH-, $C_{3-8}$ cycloalkoxy-C(O)-NH-, pyridinyl-NH-C(O)-, $C_{1-6}$ alkyl-NH-C(O)-, $C_{3-8}$ cycloalkyl-NH-C(O)-, pyridinyloxy-, pyridinylalkoxy-, pyridinyloxyalkyl-, phenyloxy-, phenylalkoxy-, phenyloxyalkyl-, $C_{1-6}$ alkyl-S(O)z-, $C_{1-6}$ alkyl-S(O)z-NH-, $C_{1-6}$ alkyl-NH-S(O)z-, pyridinyl $C_{1-6}$ alkyl-, pyridinyl-S(O)z-, pyridinyl-$C_{1-6}$ alkyl-S(O)z-, pyridinyl-S(O)z-NH-, pyridinyl-NH-S(O)z-, pyridinyl-$C_{1-6}$ alkyl-NH-, phenyl $C_{1-6}$ alkyl-, phenyl-S(O)z-, phenyl-$C_{1-6}$ alkyl-S(O)z-, phenyl-S(O)z-NH-, phenyl-NH-S(O)z-, phenyl-$C_{1-6}$ alkyl-NH-, $C_{1-6}$ alkoxy-C(O)-,

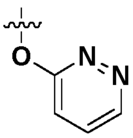
, and
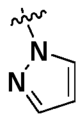
.

3. The compound, or the stereoisomer, the racemate, the tautomer, the isotopically labeled compound, the nitrogen oxide or the pharmaceutically acceptable salt thereof as claimed in claim 1 or 2, wherein $X^1$, $X^2$, $X^3$, $X^4$, $X^5$, $X^6$, and $X^7$ are identical or different, and are independently selected from CH and N; e.g., at least one of $X^1$, $X^2$, $X^3$, $X^4$, $X^5$, $X^6$, and $X^7$ is N;

$X^8$ is selected from $NR^1$;
$R^{01}$ is methoxy;
$R^{02}$ is H;
$R^{03}$ is methyl;
$R^{04}$ is H;
$R^1$ is H;
A is selected from H, $NH_2$, methyl, ethyl, propyl, and isopropyl;
E is selected from H and $NH_2$;
D is selected from the following groups: halogen, BnO-, H, CN, $NH_2$, $OCH_3$, COOH, $B(OH)_2$,

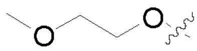 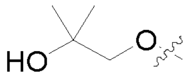 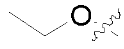

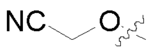 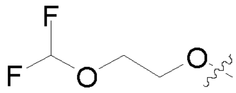 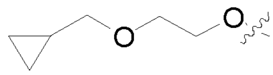

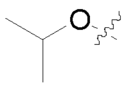 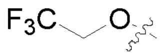 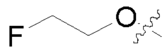

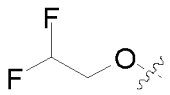 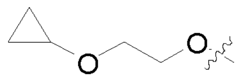 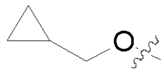

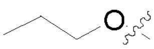 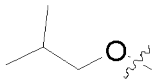 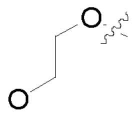

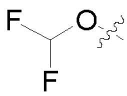 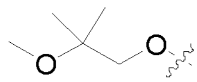

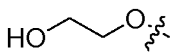 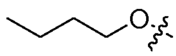 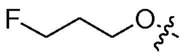 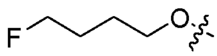,

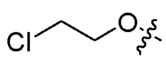 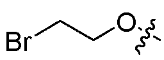 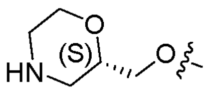, 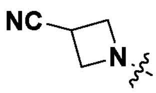, 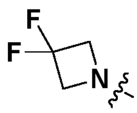, 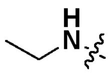, 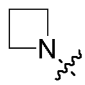,

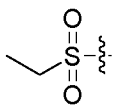, 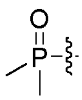, 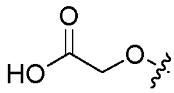, 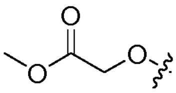, 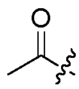, 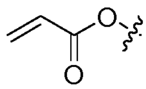, 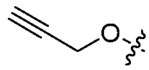,

and

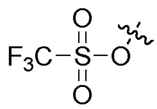;

G is selected from F and the following groups unsubstituted or optionally substituted with one, two, or more substituents independently selected from $R^e$:

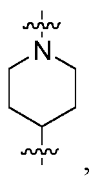, 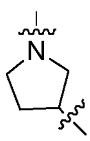, 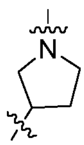, 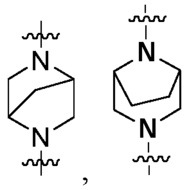,

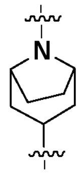, 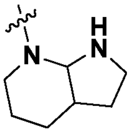, 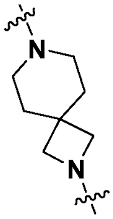, 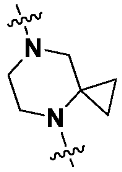, 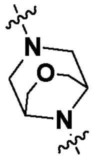, 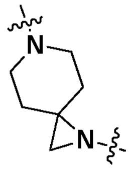, 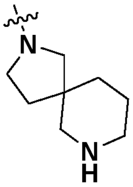, 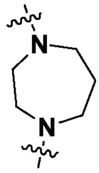, 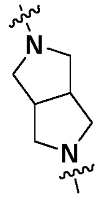,

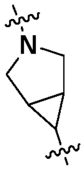, 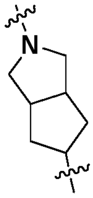, 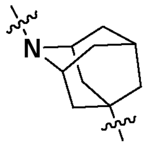, 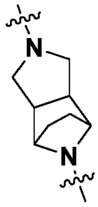, 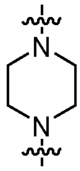, 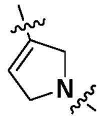, 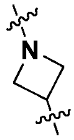, 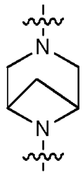, 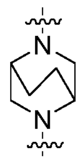, 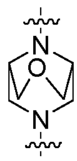,

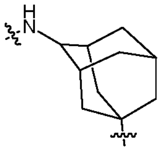, 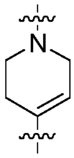, 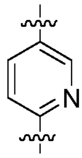, 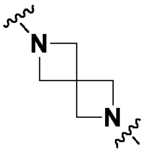, 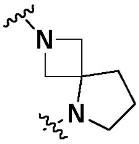, 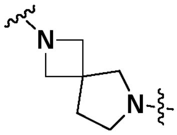, 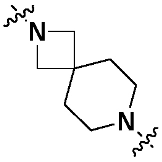,

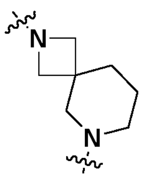, 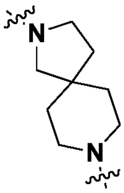, 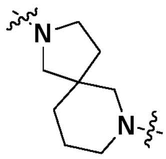, 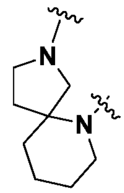, 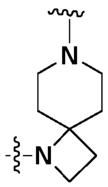, 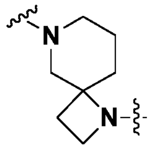, 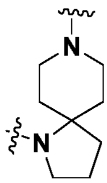, 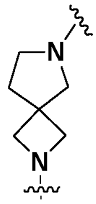,

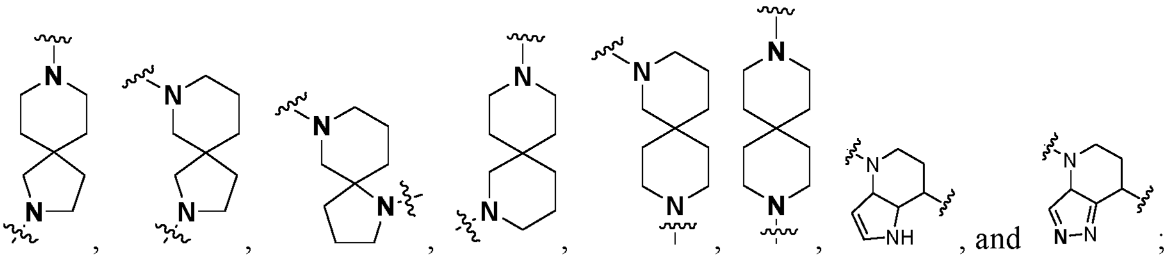
, , , , , , , and ;

preferably, when group G is substituted with $R^e$, $R^e$ can substitute H on $-CH_2-$ or $-CH=$ constituting group G, forming, e.g., a group selected from:

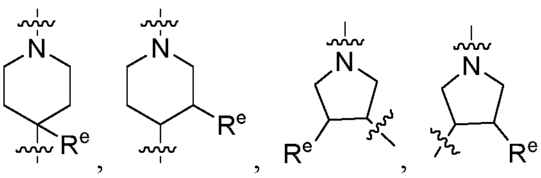
, , , ,

and

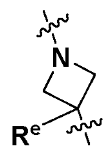
;

K is absent or selected from H, OH, and the following groups unsubstituted or optionally substituted with one, two, or more substituents independently selected from $R^f$: phenyl-C(O)-, phenyl-C(O)-NH-, phenyl-C(O)-NH-$C_{1-6}$ alkyl-, phenyloxy-C(O)-NH-, phenylalkyl-NH-C(O)-, phenylalkyl-C(O)-NH-, pyridinyl-C(O)-, pyridinyl-C(O)-NH-, pyridinyl-C(O)-NH-$C_{1-6}$ alkyl-, pyridinyloxy-C(O)-NH-, pyridinylalkyl-NH-C(O)-, pyridinylalkyl-C(O)-NH-, pyrrolidinyl-C(O)-NH-, pyrrolidinyloxy-C(O)-NH-, $C_{1-6}$ alkyl-C(O)-, $C_{2-6}$ alkenyl-C(O)-, C, $C_{1-6}$ alkyl-C(O)-NH-, $C_{1-6}$ alkoxy-C(O)-NH-, $C_{3-8}$ cycloalkyl-C(O)-NH-, $C_{3-8}$ cycloalkoxy-C(O)-NH-, pyridinyl-NH-C(O)-, $C_{1-6}$ alkyl-NH-C(O)-, $C_{3-8}$ cycloalkyl-NH-C(O)-, pyridinyloxy-, pyridinylalkoxy-, pyridinyloxyalkyl-, phenyloxy-, phenylalkoxy-, phenyloxyalkyl-, $C_{1-6}$ alkyl-S(O)z-, $C_{1-6}$ alkyl-S(O)z-NH-, $C_{1-6}$ alkyl-NH-S(O)z-, pyridinyl $C_{1-6}$ alkyl-, pyridinyl-S(O)z-, pyridinyl-$C_{1-6}$ alkyl-S(O)z-, pyridinyl-S(O)z-NH-, pyridinyl-NH-S(O)z-, pyridinyl-$C_{1-6}$ alkyl-NH-, phenyl $C_{1-6}$ alkyl-, phenyl-S(O)z-, phenyl-$C_{1-6}$ alkyl-S(O)z-, phenyl-S(O)z-NH-, phenyl-NH-S(O)z-, phenyl-$C_{1-6}$ alkyl-NH-, $C_{1-6}$ alkoxy-C(O)-,

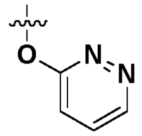
,
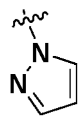
,
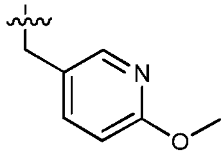
,
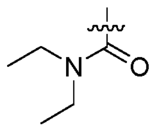
,

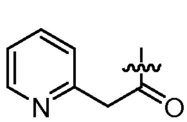
,
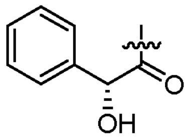
,
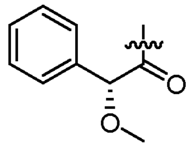
, and
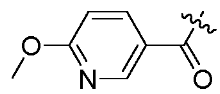
;

preferably, the compound represented by formula I has a structure represented by the following formula II, III, IV, or V:

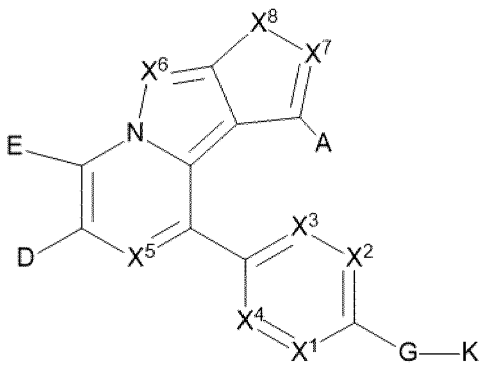
II

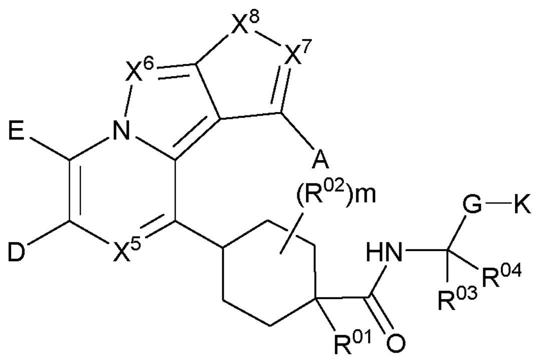
III
;

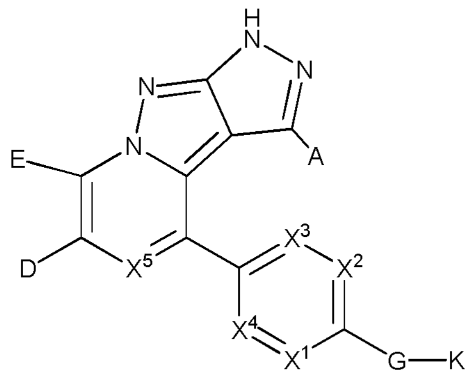

IV

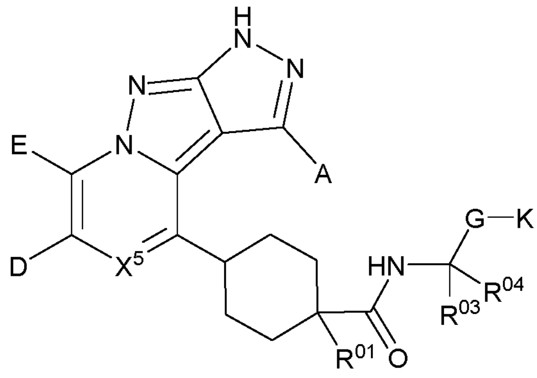

V

;

wherein $X^1$, $X^2$, $X^3$, $X^4$, $X^5$, $X^6$, $X^7$, $X^8$, $R^{01}$, $R^{02}$, $R^{03}$, $R^{04}$, A, D, E, G, K, and m are as defined in any one of claims 1-3.

**4.** The compound, or the stereoisomer, the racemate, the tautomer, the isotopically labeled compound, the nitrogen oxide, or the pharmaceutically acceptable salt thereof as claimed in any one of claims 1-3, wherein the compound represented by formula I is selected from the following compounds:

| No. | Chemical structure | No. | Chemical structure |
|---|---|---|---|
| 1 | 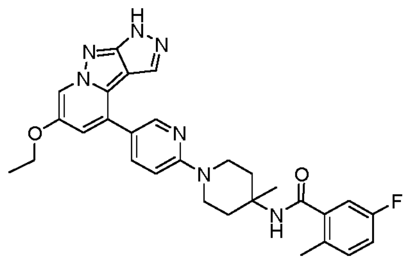 | 134 | 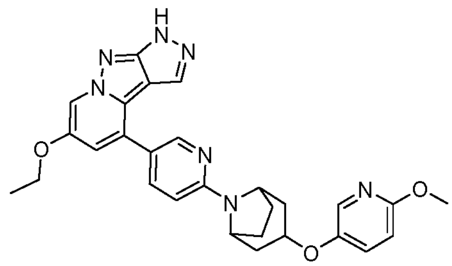 |
| 2 | 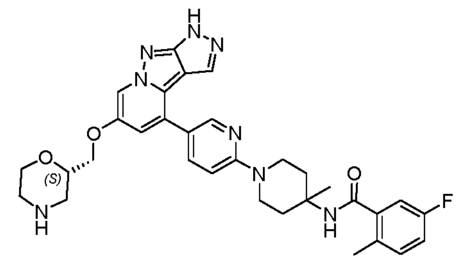 | 135 | 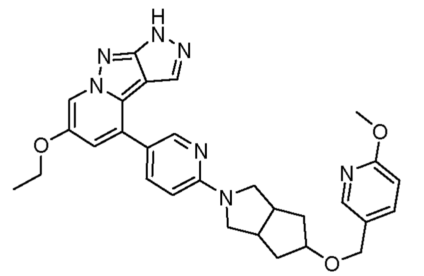 |
| 3 | 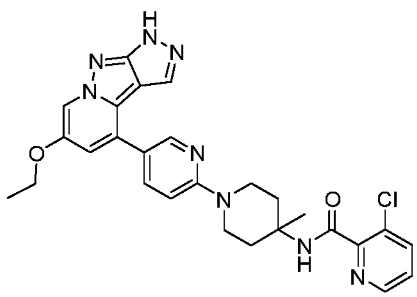 | 136 | 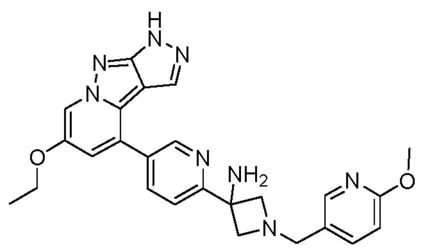 |

(continued)

| No. | Chemical structure | No. | Chemical structure |
|---|---|---|---|
| 4 | 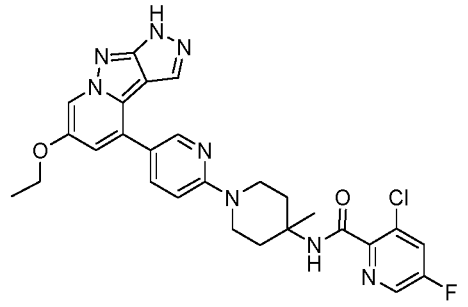 | 137 | 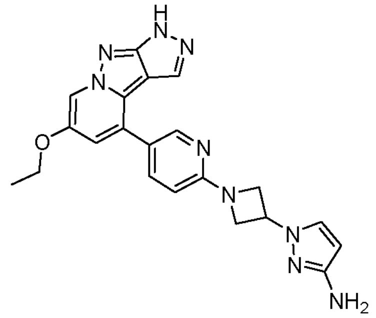 |
| 5 | 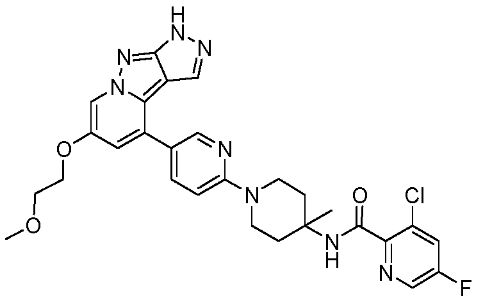 | 138 | 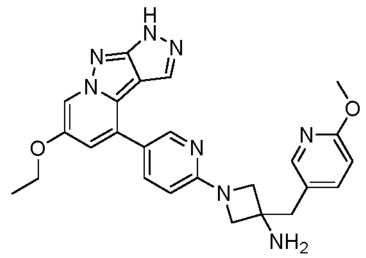 |
| 6 | 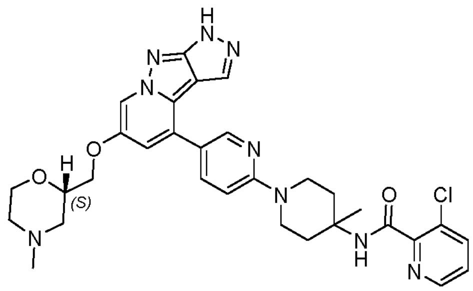 | 139 | 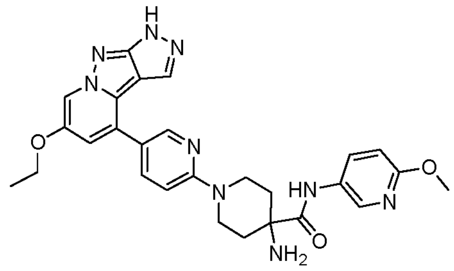 |
| 7 | 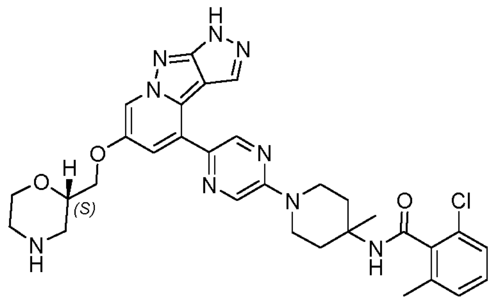 | 140 | 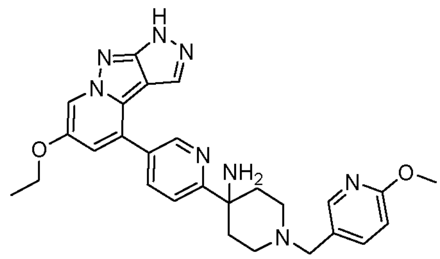 |
| 8 | 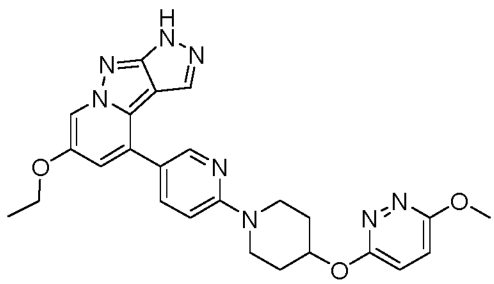 | 141 | 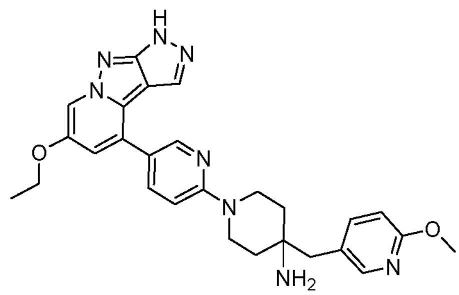 |

(continued)

| No. | Chemical structure | No. | Chemical structure |
|---|---|---|---|
| 9 | 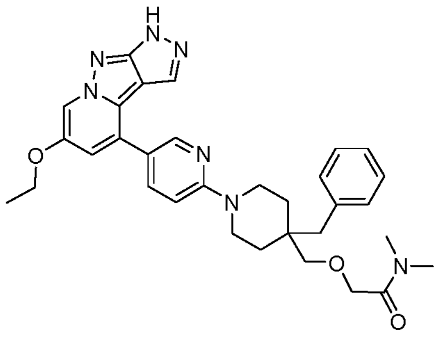 | 142 | 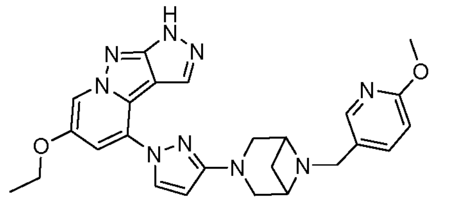 |
| 10 | 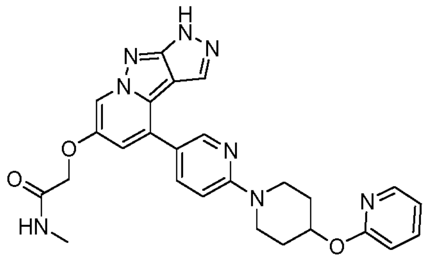 | 143 | 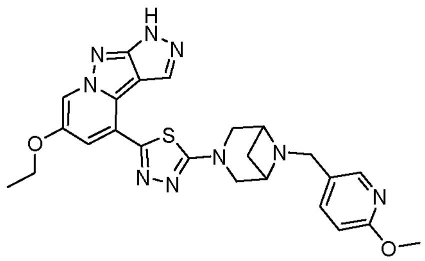 |
| 11 | 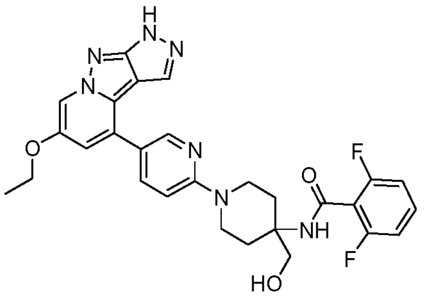 | 144 | 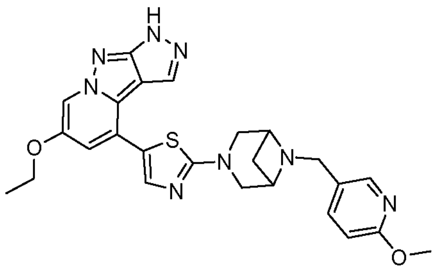 |
| 12 | 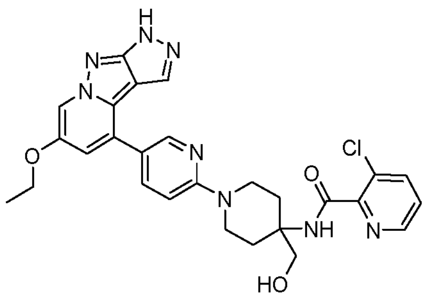 | 145 | 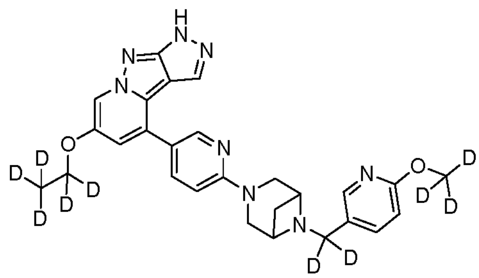 |
| 13 | 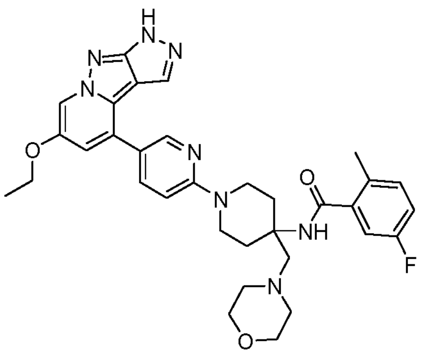 | 146 | 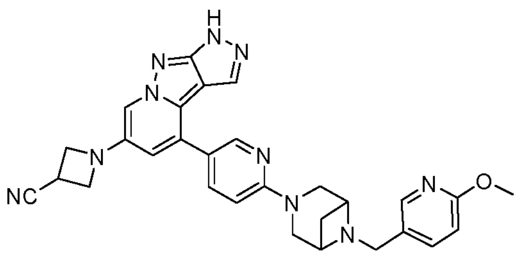 |

(continued)

| No. | Chemical structure | No. | Chemical structure |
|---|---|---|---|
| 14 | 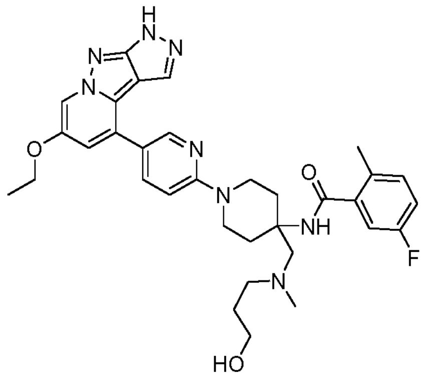 | 147 | 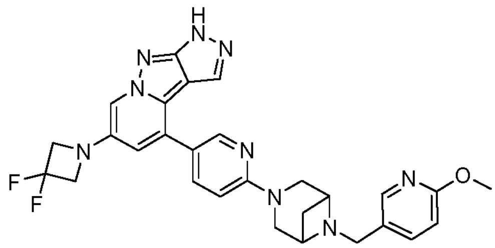 |
| 15 | 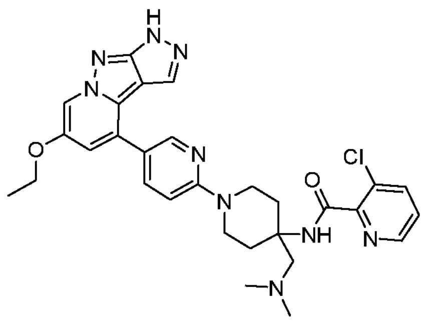 | 148 | 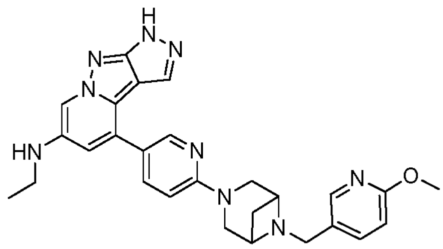 |
| 16 | 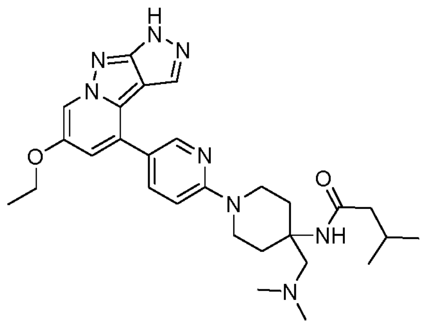 | 149 | 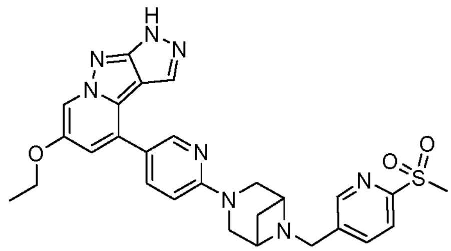 |
| 17 | 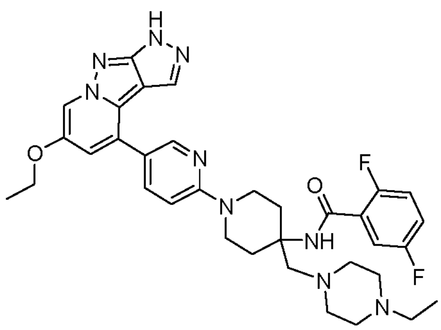 | 150 | 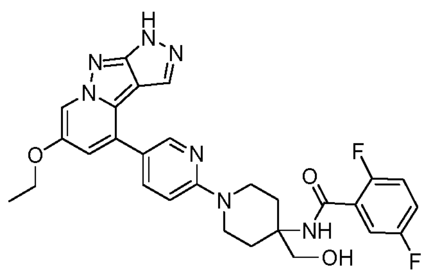 |

(continued)

| No. | Chemical structure | No. | Chemical structure |
|---|---|---|---|
| 18 | 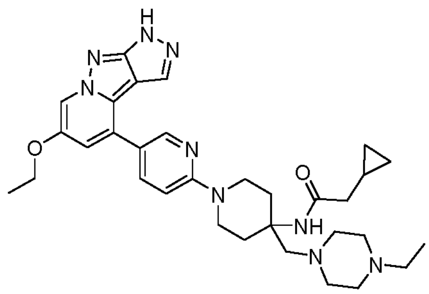 | 151 | 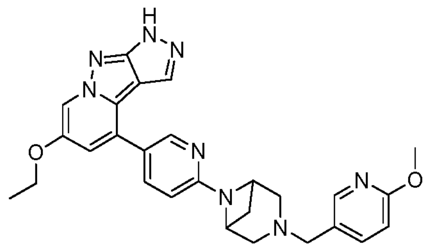 |
| 19 | 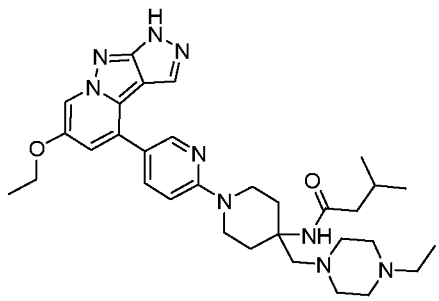 | 152 | 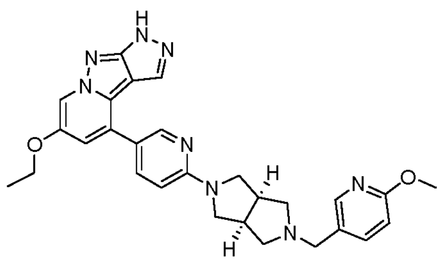 |
| 20 | 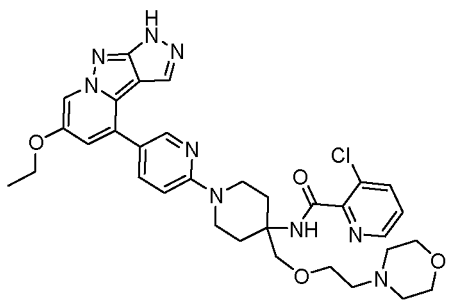 | 153 | 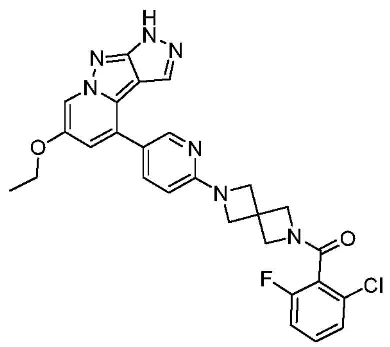 |
| 21 | 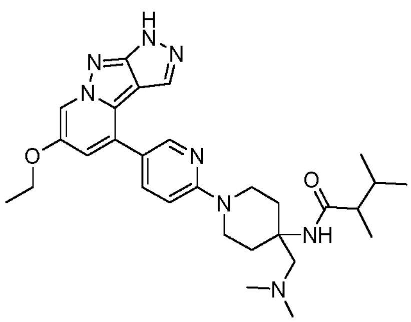 | 154 | 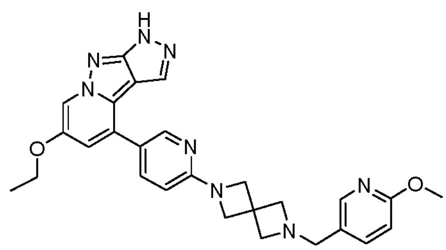 |
| 22 | 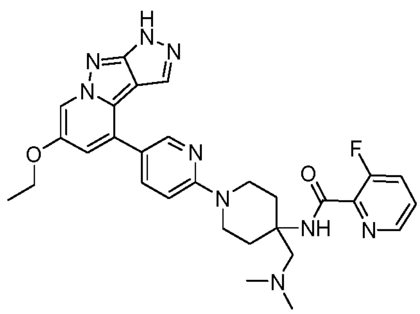 | 155 | 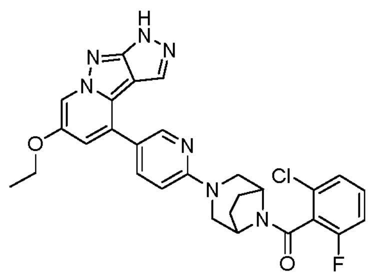 |

(continued)

| No. | Chemical structure | No. | Chemical structure |
|---|---|---|---|
| 23 | 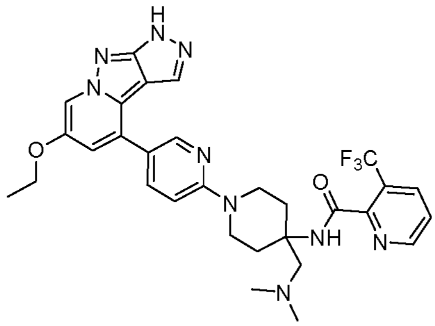 | 156 | 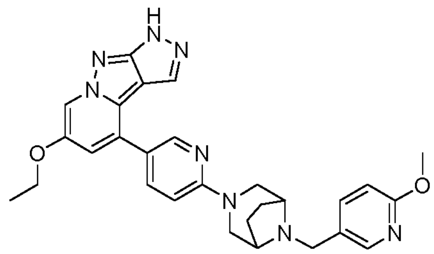 |
| 24 | 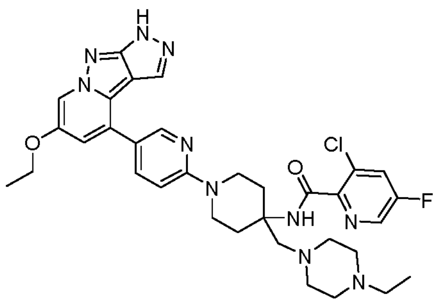 | 157 | 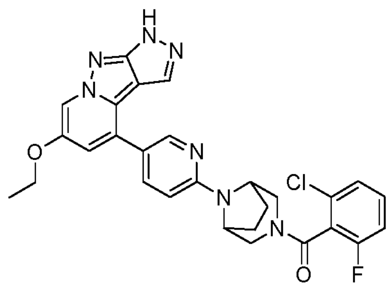 |
| 25 | 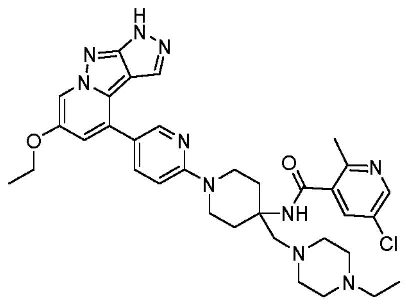 | 158 | 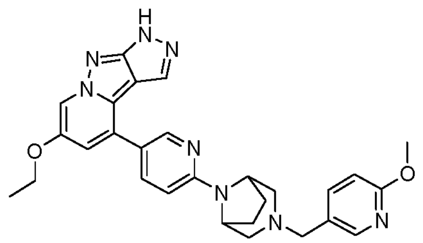 |
| 26 | 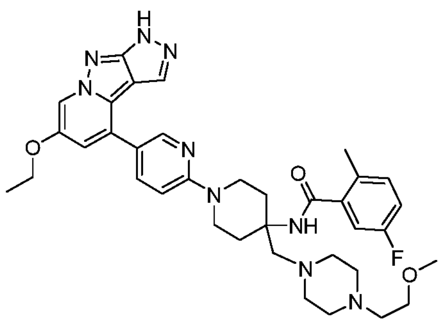 | 159 | 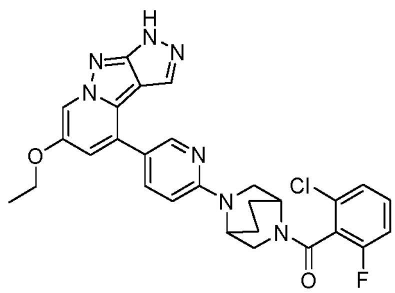 |
| 27 | 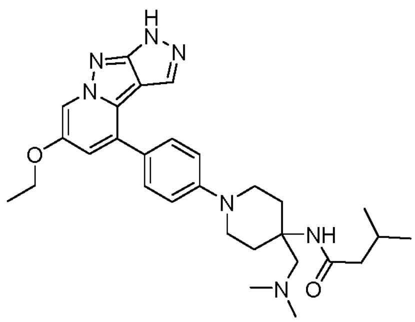 | 160 | 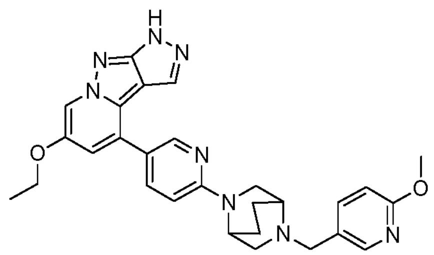 |

(continued)

| No. | Chemical structure | No. | Chemical structure |
|---|---|---|---|
| 28 | 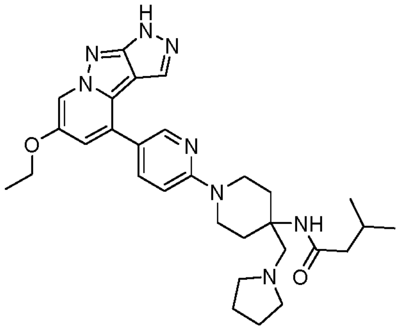 | 161 | 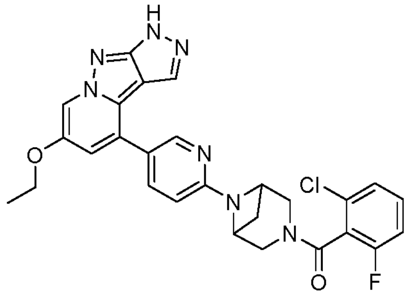 |
| 29 | 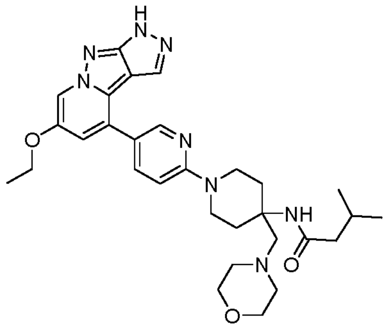 | 162 | 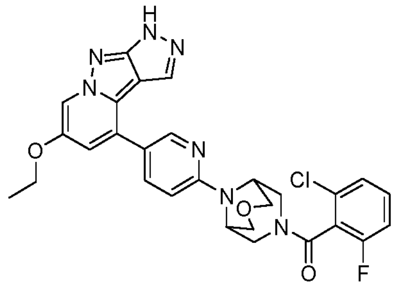 |
| 30 | 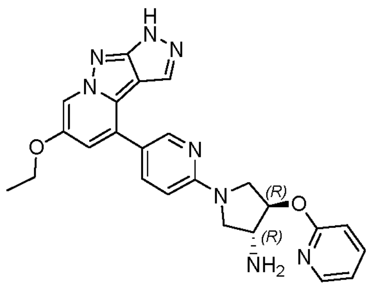 | 163 | 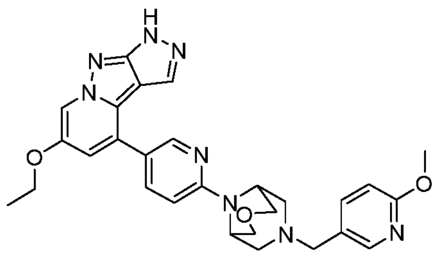 |
| 31 | 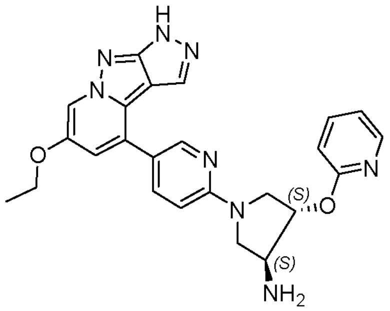 | 164 | 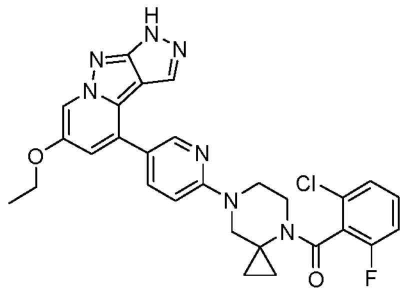 |
| 32 | 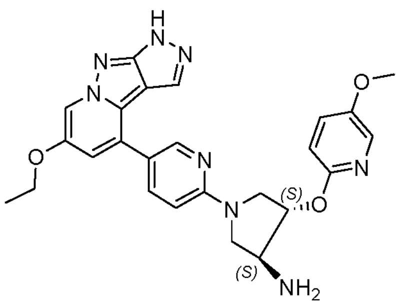 | 165 | 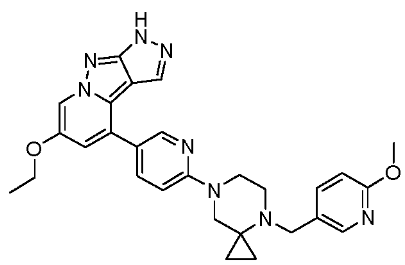 |

(continued)

| No. | Chemical structure | No. | Chemical structure |
|---|---|---|---|
| 33 | 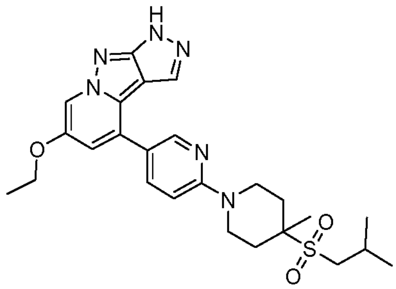 | 166 | 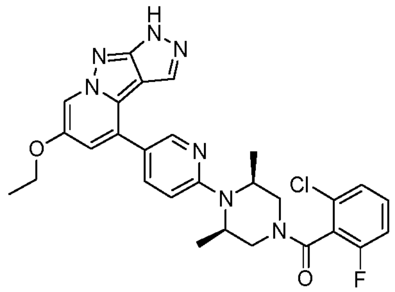 |
| 34 | 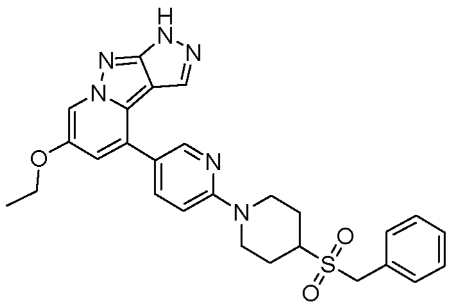 | 167 | 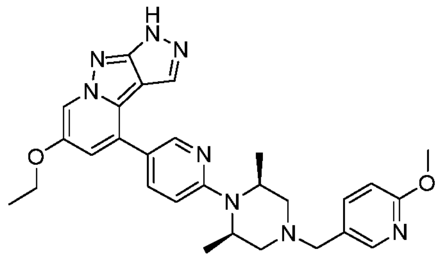 |
| 35 | 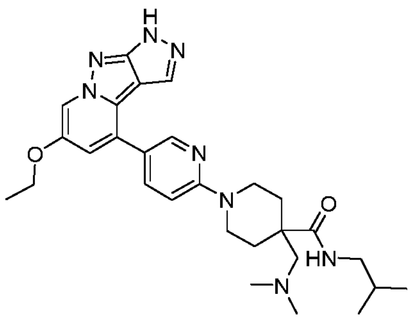 | 168 | 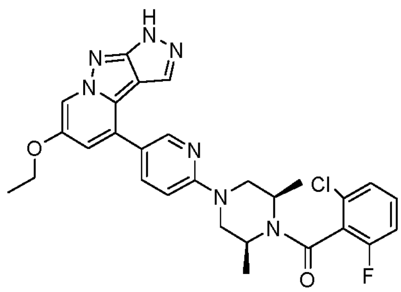 |
| 36 | 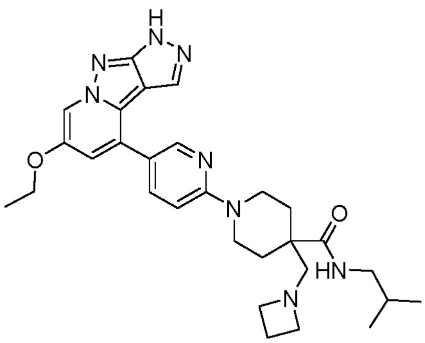 | 169 | 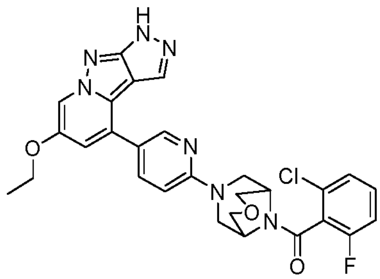 |
| 37 | 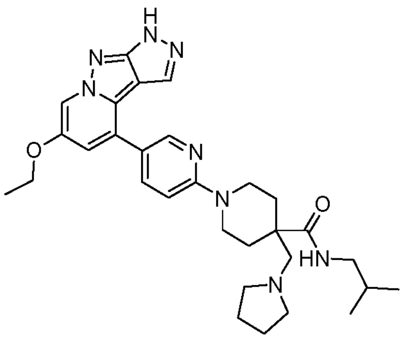 | 170 | 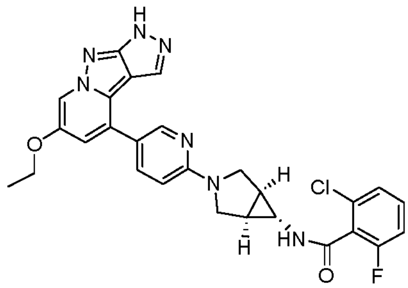 |

(continued)

| No. | Chemical structure | No. | Chemical structure |
|---|---|---|---|
| 38 | 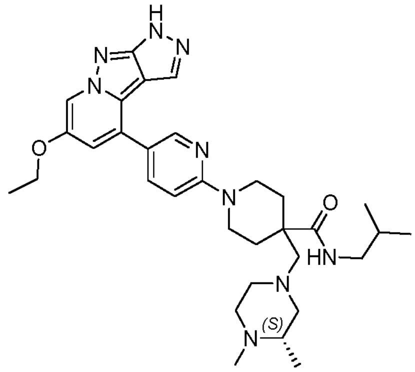 | 171 | 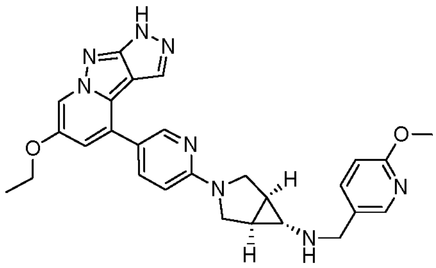 |
| 39 | 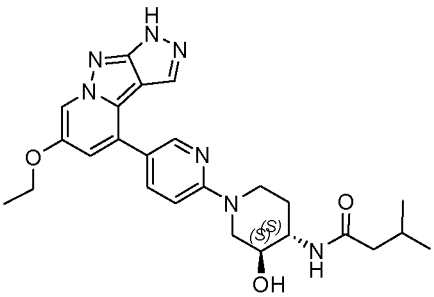 | 172 | 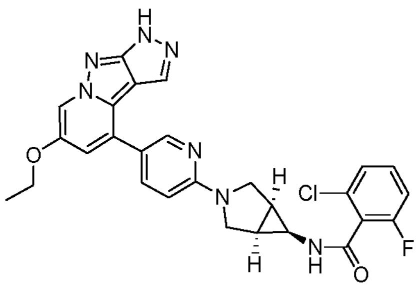 |
| 40 | 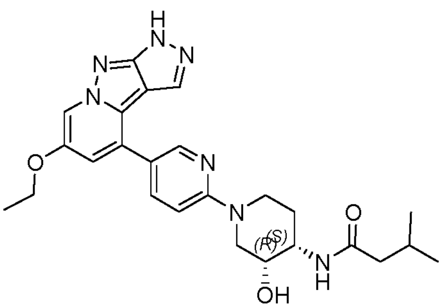 | 173 | 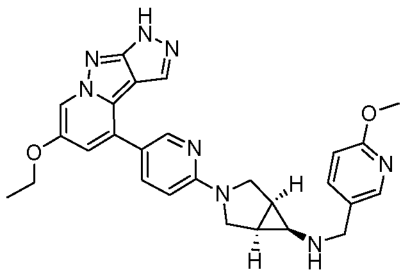 |
| 41 | 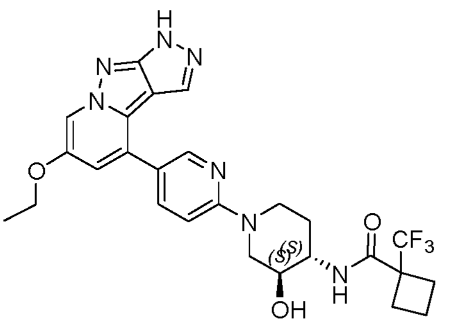 | 174 | 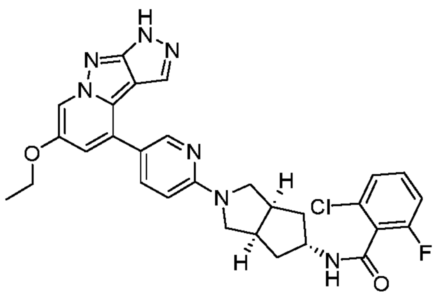 |
| 42 | 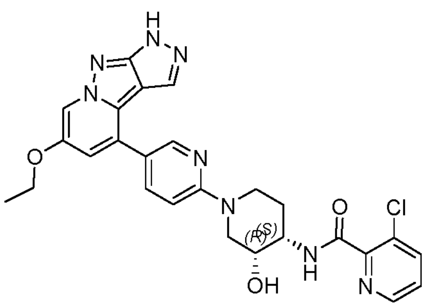 | 175 | 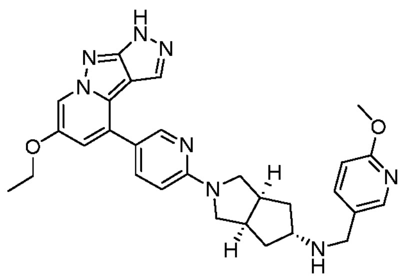 |

(continued)

| No. | Chemical structure | No. | Chemical structure |
|---|---|---|---|
| 43 | 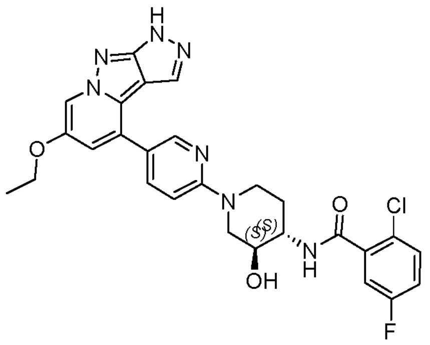 | 176 | 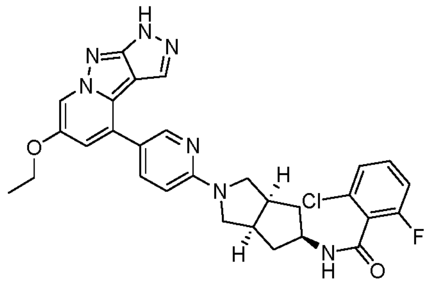 |
| 44 | 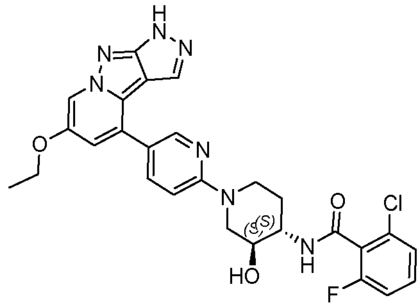 | 177 | 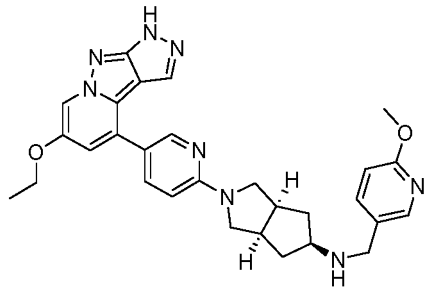 |
| 45 | 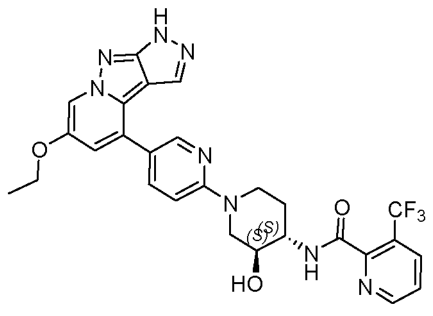 | 178 | 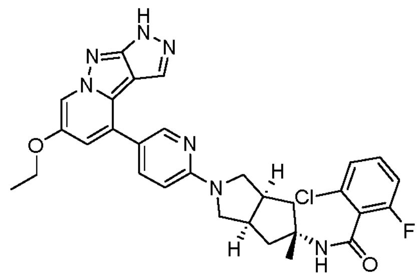 |
| 46 | 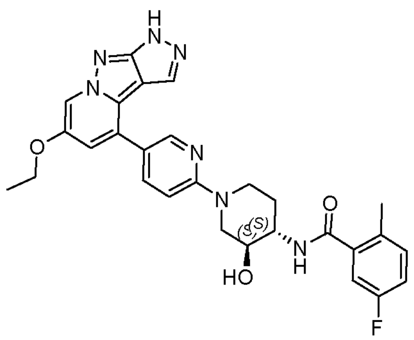 | 179 | 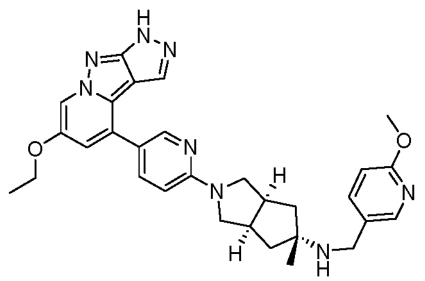 |
| 47 | 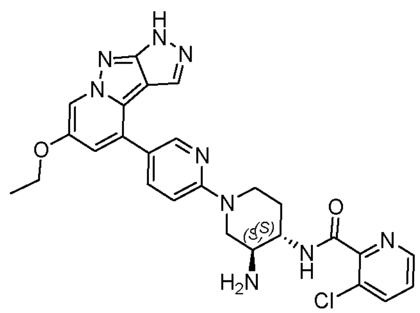 | 180 | 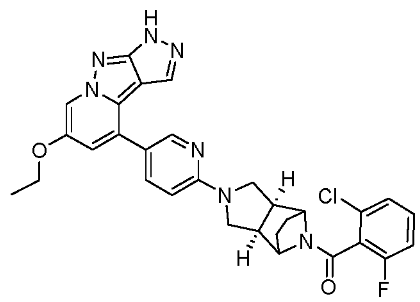 |

(continued)

| No. | Chemical structure | No. | Chemical structure |
|---|---|---|---|
| 48 | 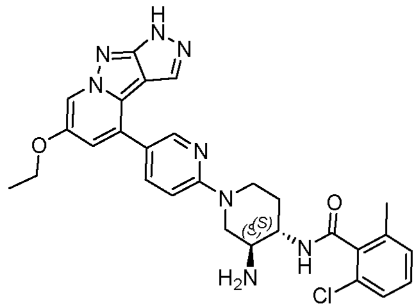 | 181 | 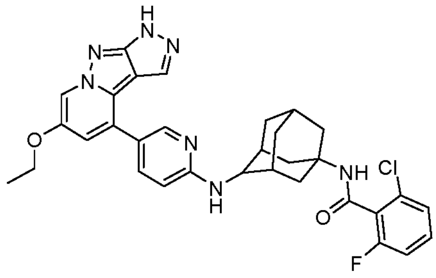 |
| 49 | 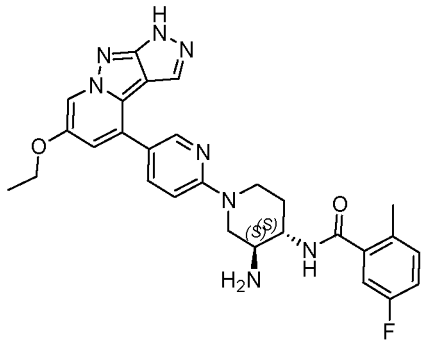 | 182 | 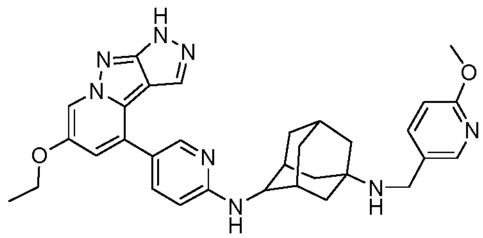 |
| 50 | 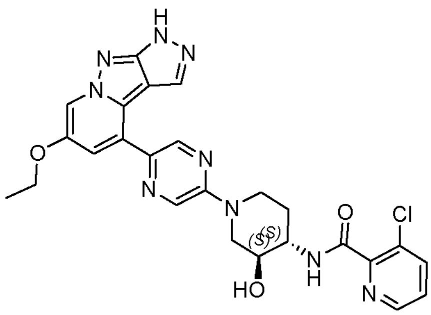 | 183 | 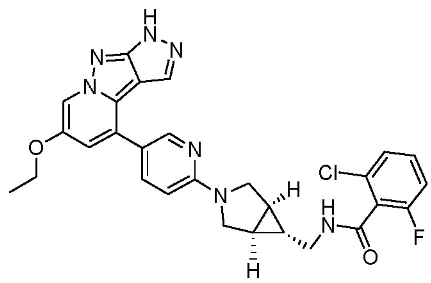 |
| 51 | 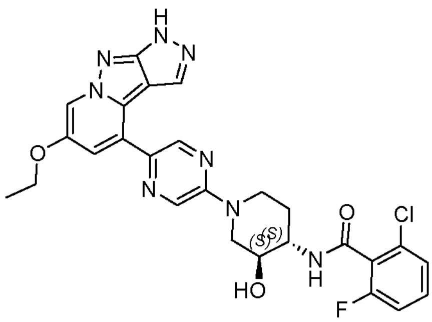 | 184 | 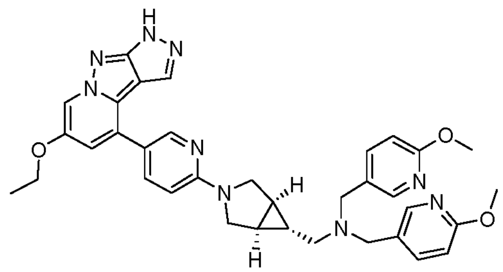 |
| 52 | 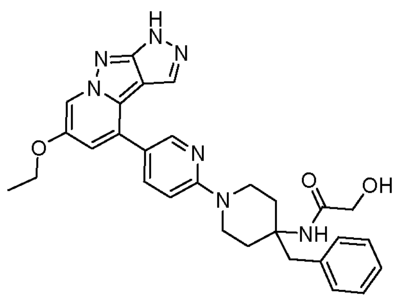 | 185 | 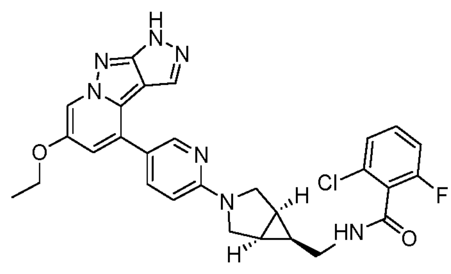 |

(continued)

| No. | Chemical structure | No. | Chemical structure |
|---|---|---|---|
| 53 | 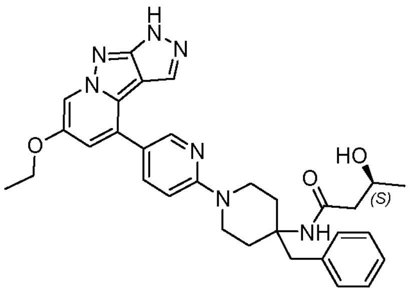 | 186 | 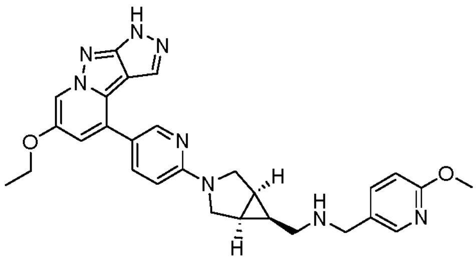 |
| 54 | 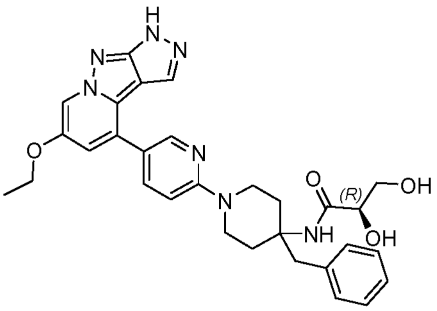 | 187 | 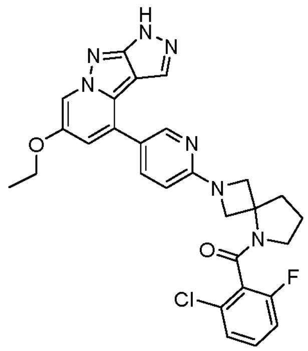 |
| 55 | 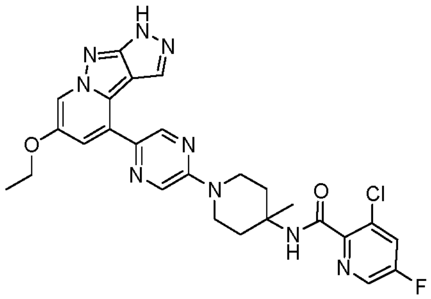 | 188 | 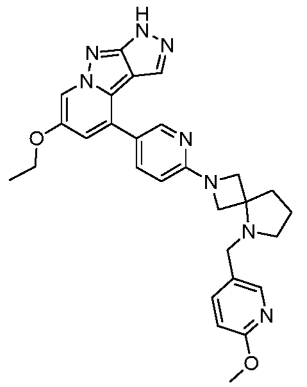 |
| 56 | 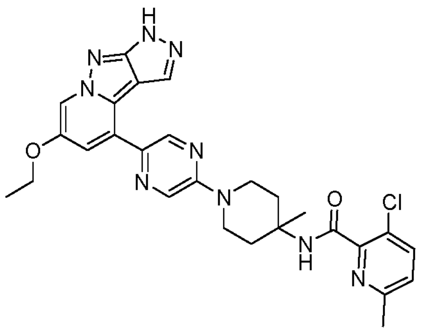 | 189 | 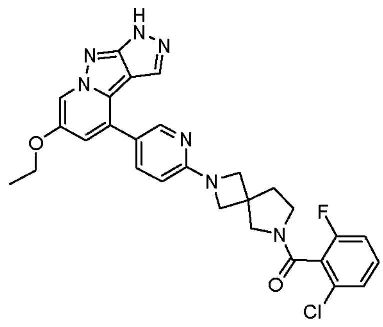 |

(continued)

| No. | Chemical structure | No. | Chemical structure |
|---|---|---|---|
| 57 | 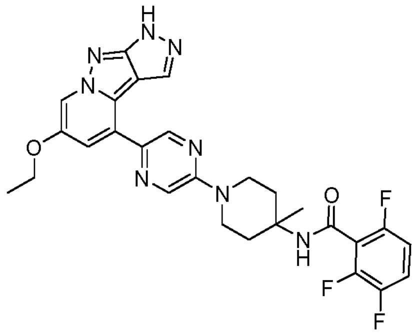 | 190 | 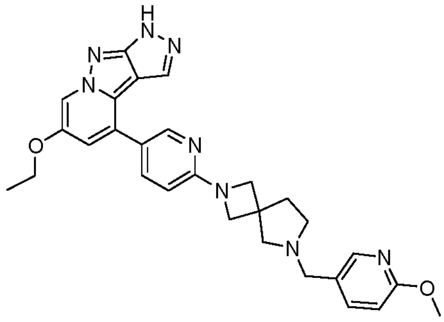 |
| 58 | 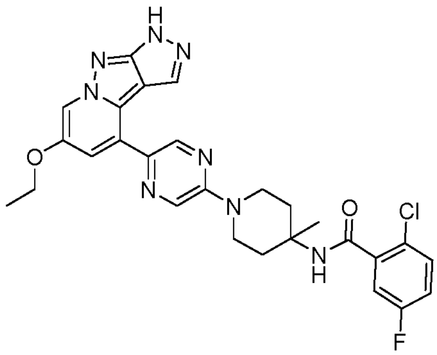 | 191 | 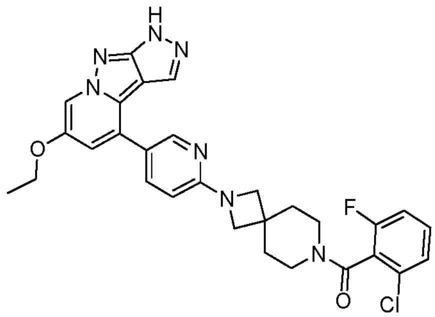 |
| 59 | 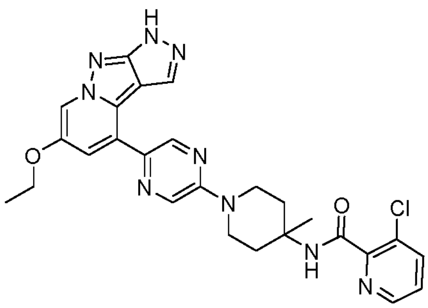 | 192 | 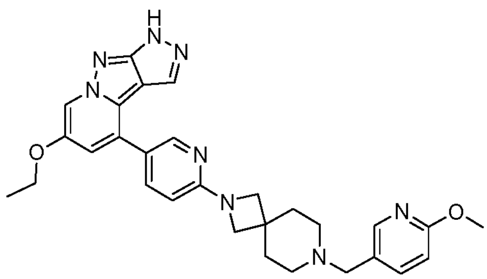 |
| 60 | 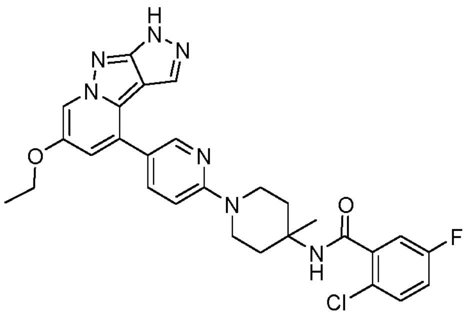 | 193 | 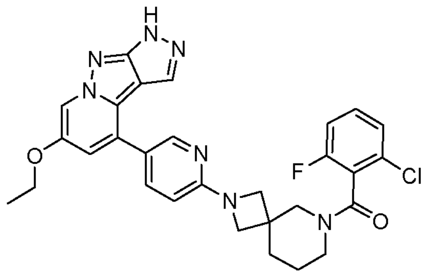 |
| 61 | 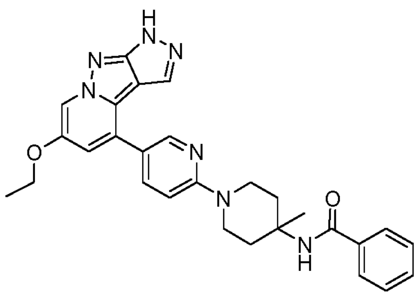 | 194 | 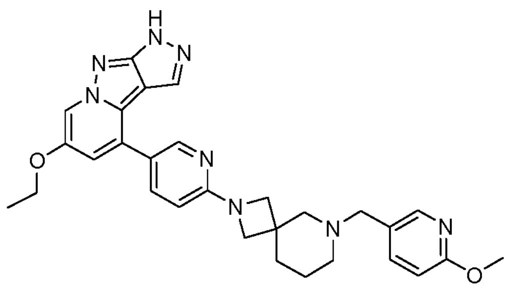 |

(continued)

| No. | Chemical structure | No. | Chemical structure |
|---|---|---|---|
| 62 | 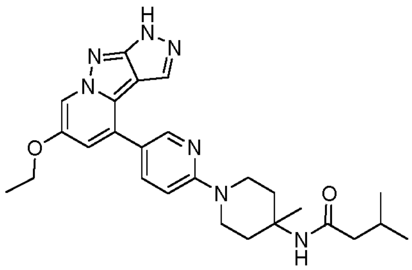 | 195 | 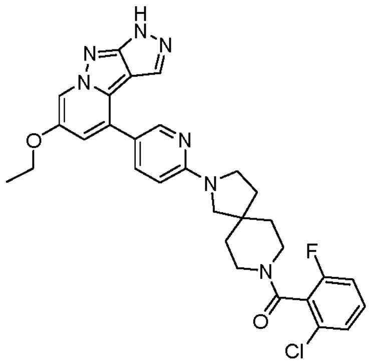 |
| 63 | 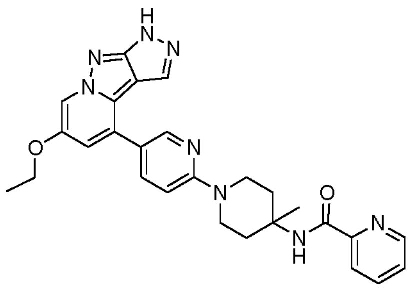 | 196 | 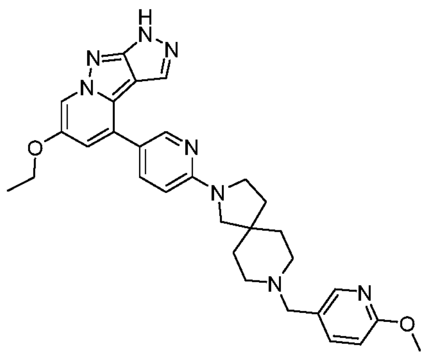 |
| 64 | 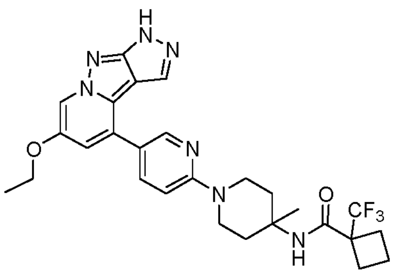 | 197 | 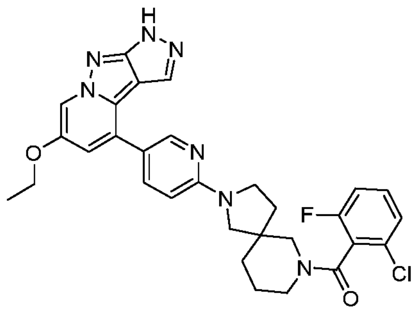 |
| 65 | 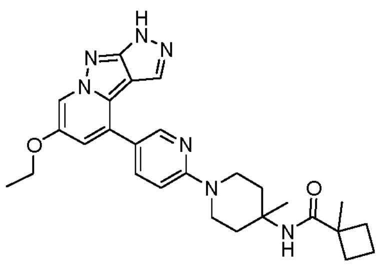 | 198 | 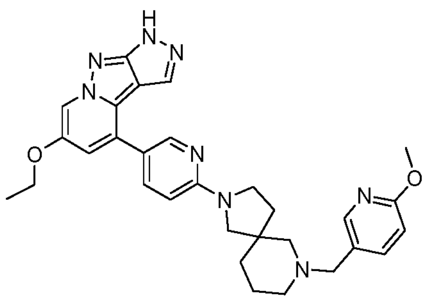 |

(continued)

| No. | Chemical structure | No. | Chemical structure |
|---|---|---|---|
| 66 | 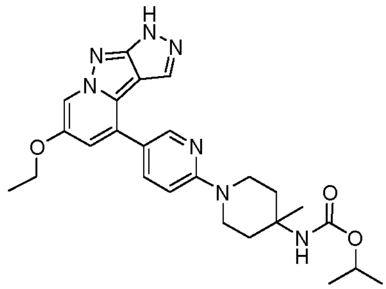 | 199 | 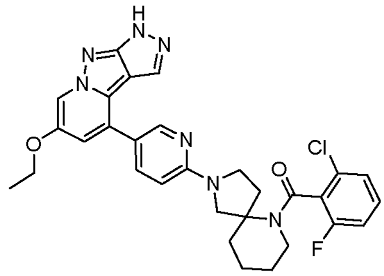 |
| 67 | 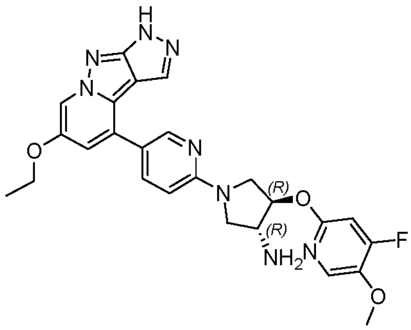 | 200 | 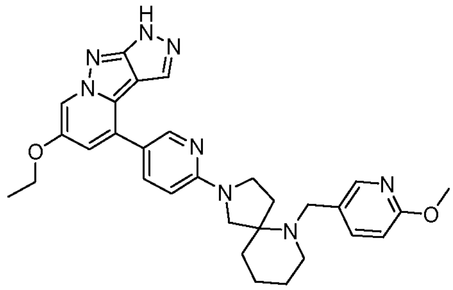 |
| 68 | 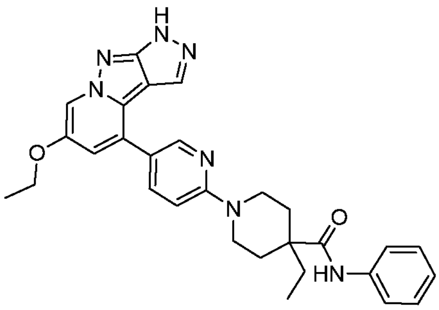 | 201 | 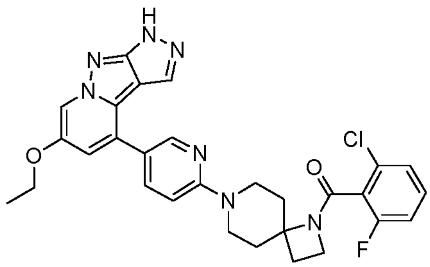 |
| 69 | 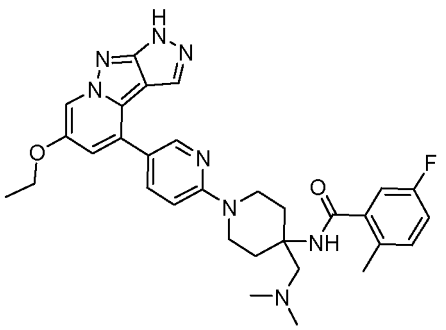 | 202 | 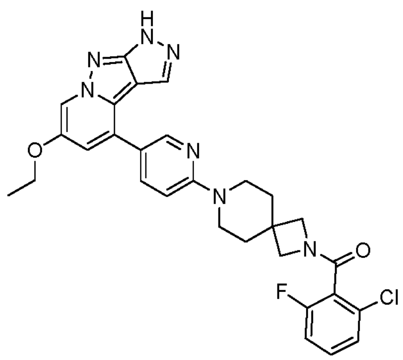 |

(continued)

| No. | Chemical structure | No. | Chemical structure |
|---|---|---|---|
| 70 | 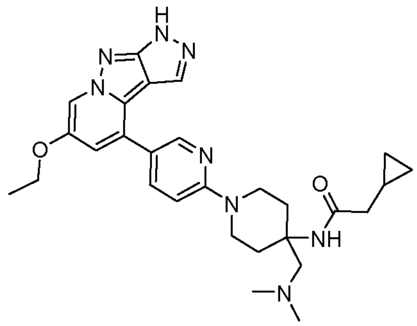 | 203 | 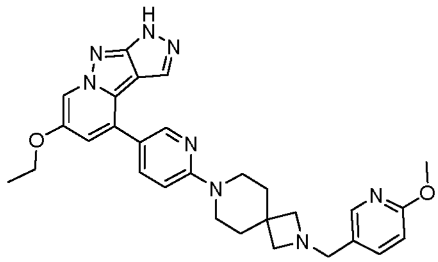 |
| 71 | 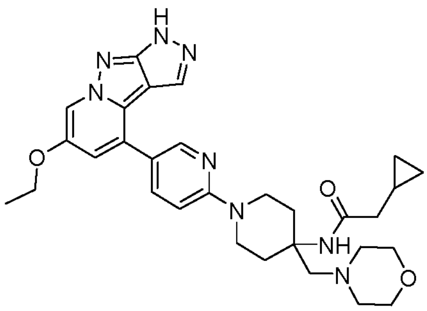 | 204 | 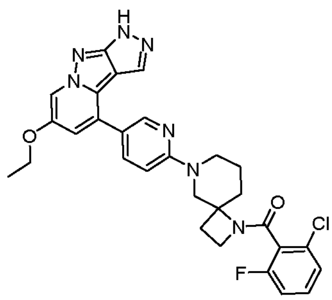 |
| 72 | 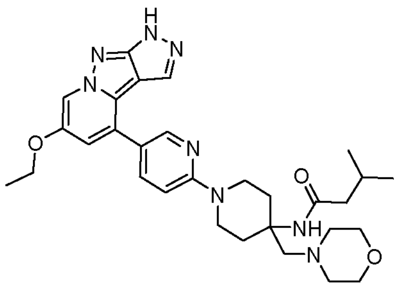 | 205 | 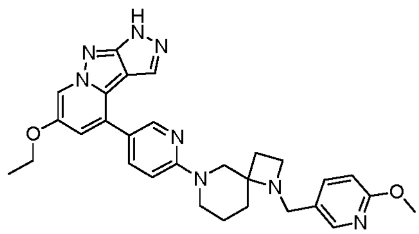 |
| 73 | 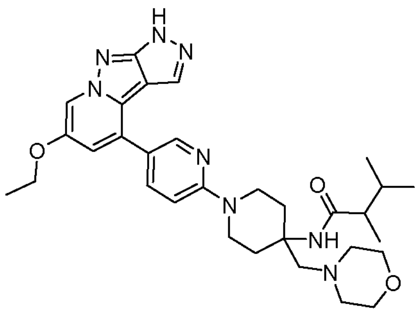 | 206 | 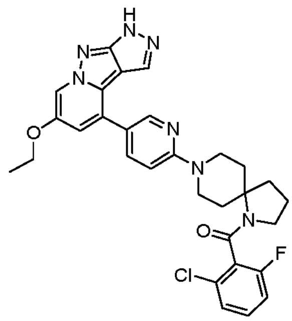 |
| 74 | 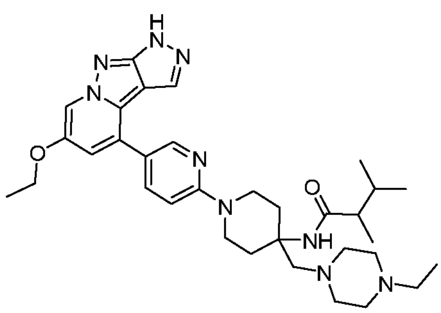 | 207 | 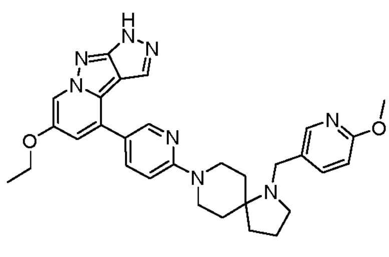 |

(continued)

| No. | Chemical structure | No. | Chemical structure |
|---|---|---|---|
| 75 | 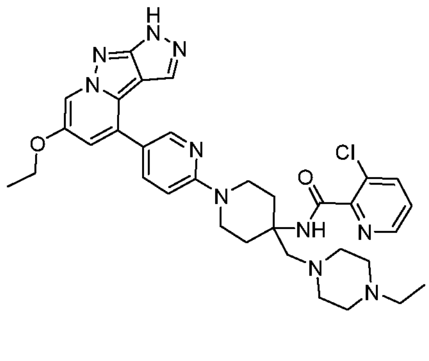 | 208 | 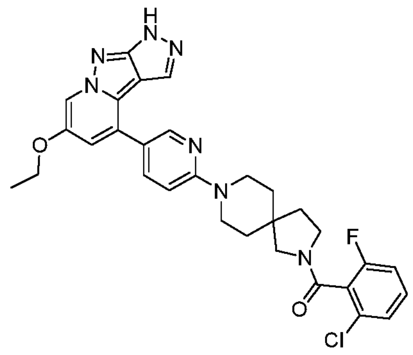 |
| 76 | 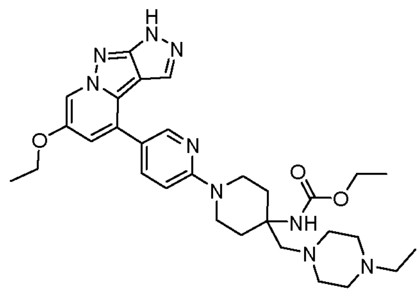 | 209 | 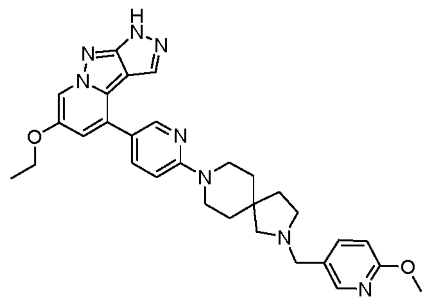 |
| 77 | 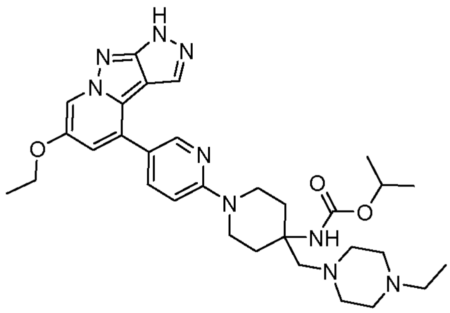 | 210 | 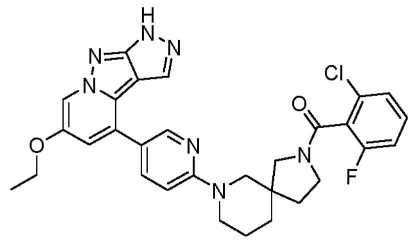 |
| 78 | 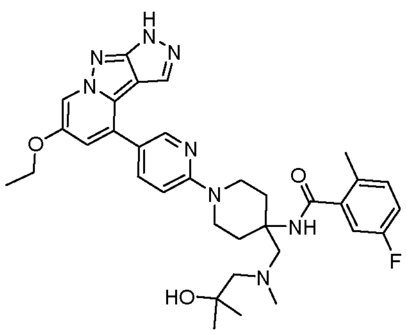 | 211 | 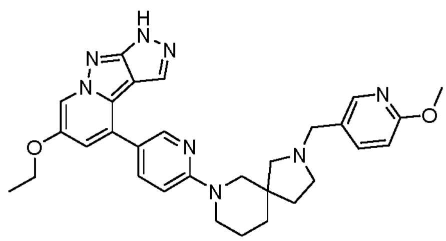 |
| 79 | 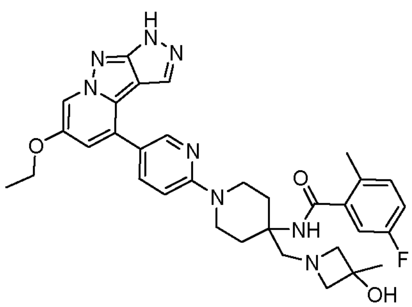 | 212 | 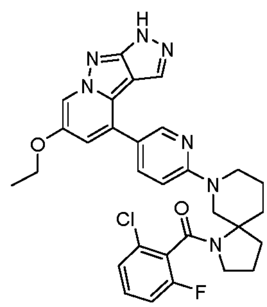 |

(continued)

| No. | Chemical structure | No. | Chemical structure |
|---|---|---|---|
| 80 | 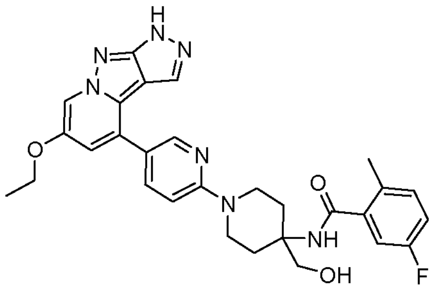 | 213 | 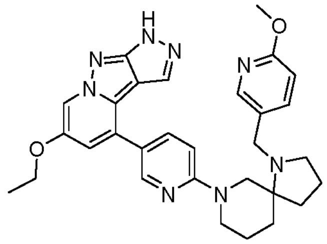 |
| 81 | 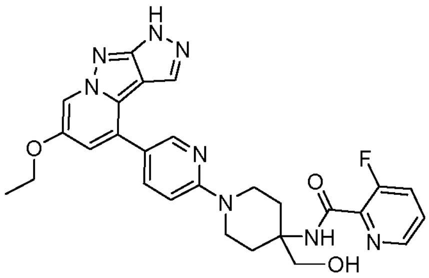 | 214 | 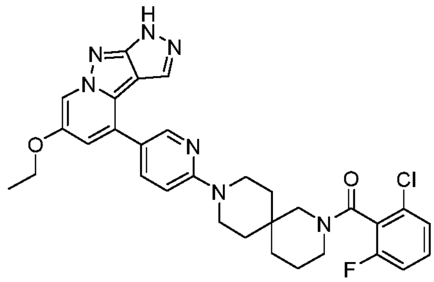 |
| 82 | 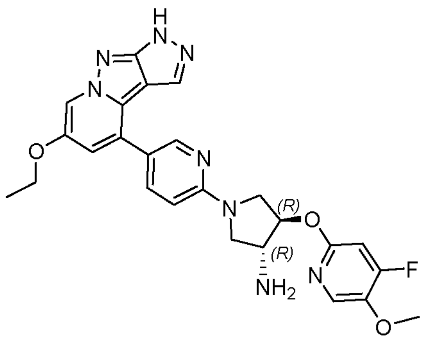 | 215 | 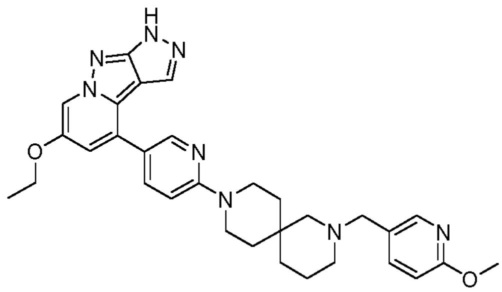 |
| 83 | 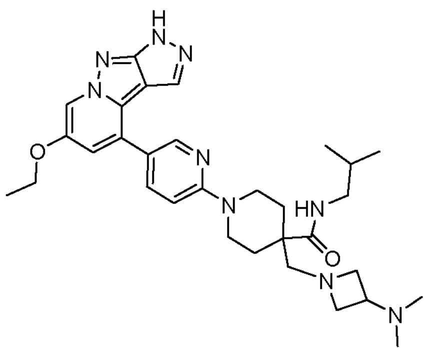 | 216 | 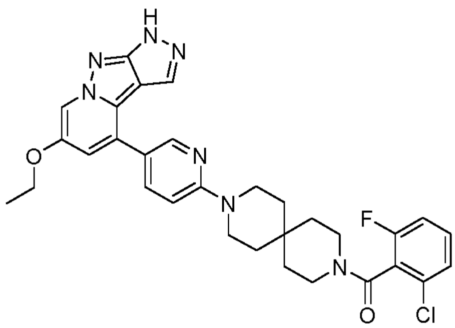 |

(continued)

| No. | Chemical structure | No. | Chemical structure |
|---|---|---|---|
| 84 | 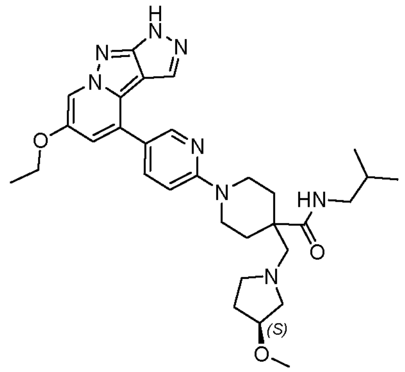 | 217 | 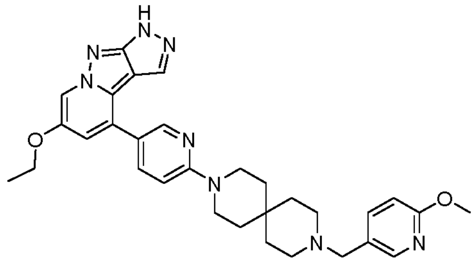 |
| 85 | 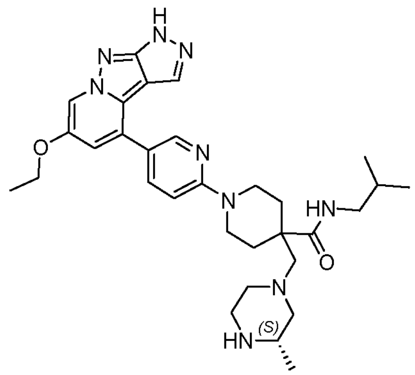 | 218 | 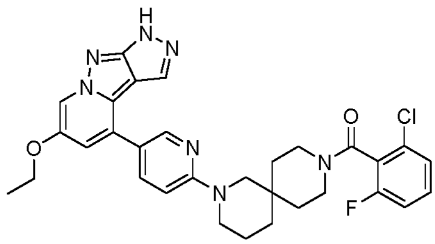 |
| 86 | 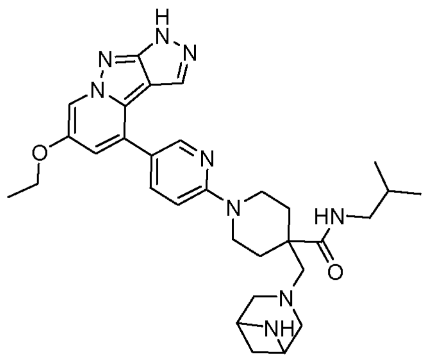 | 219 | 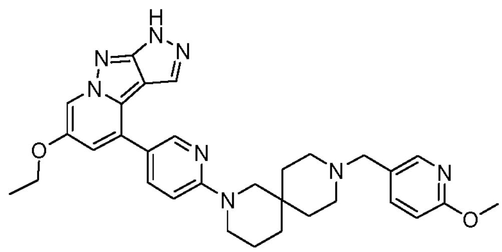 |
| 87 | 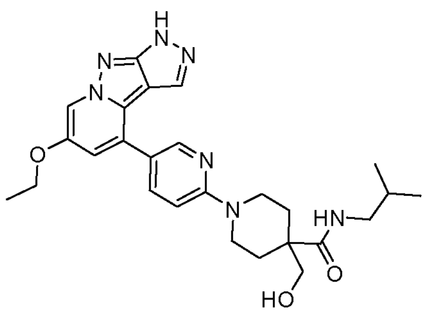 | 220 | 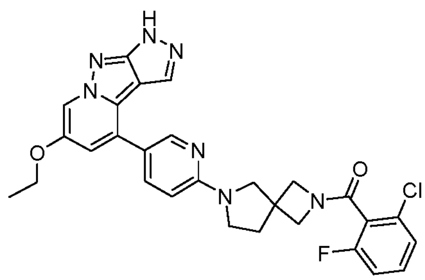 |

(continued)

| No. | Chemical structure | No. | Chemical structure |
|---|---|---|---|
| 88 | 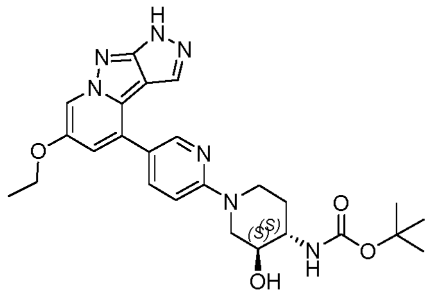 | 221 | 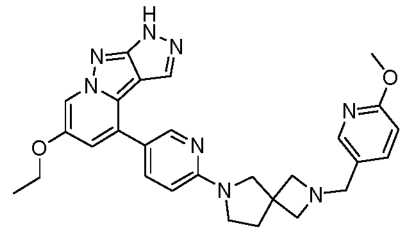 |
| 89 | 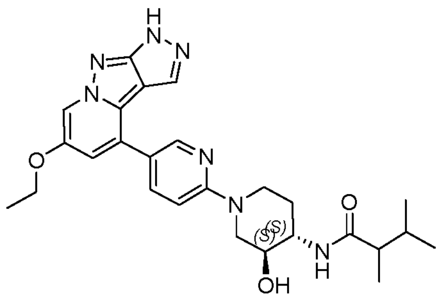 | 222 | 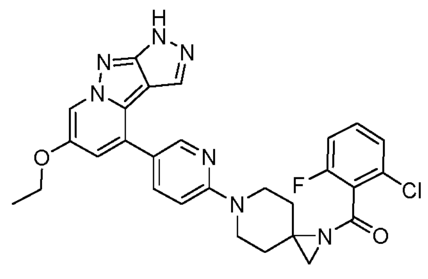 |
| 90 | 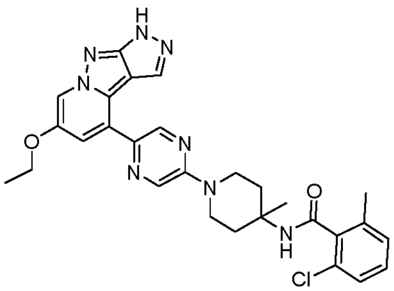 | 223 | 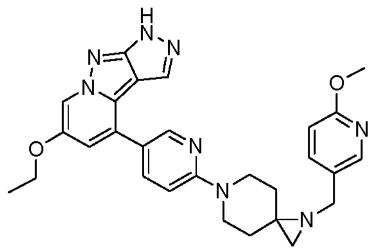 |
| 91 | 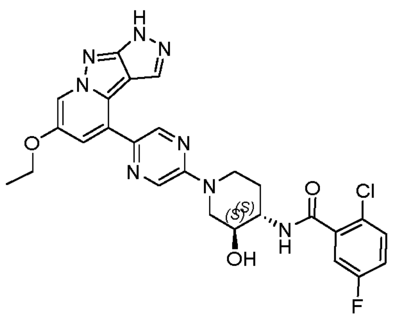 | 224 | 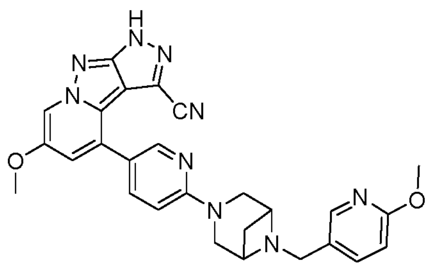 |
| 92 | 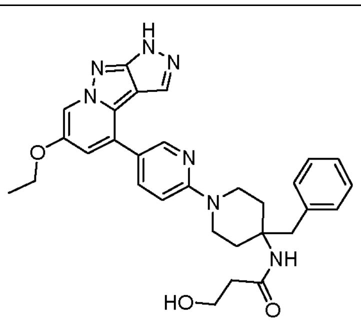 | 225 | 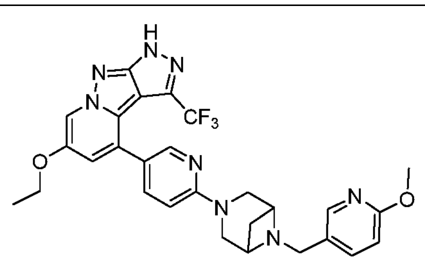 |

(continued)

| No. | Chemical structure | No. | Chemical structure |
|---|---|---|---|
| 93 | 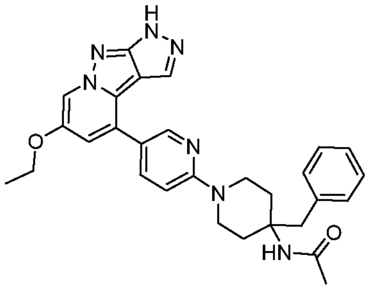 | 226 | 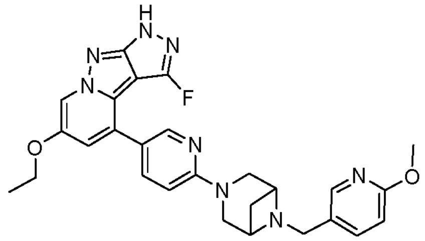 |
| 94 | 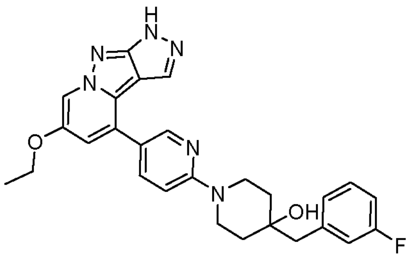 | 227 | 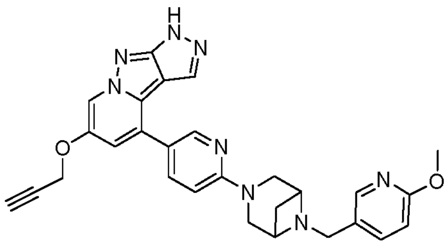 |
| 95 | 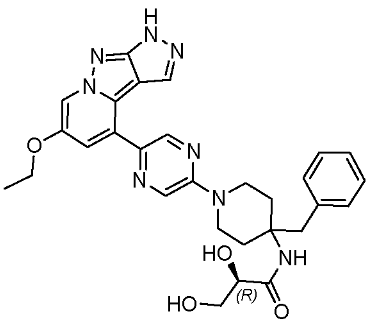 | 228 | 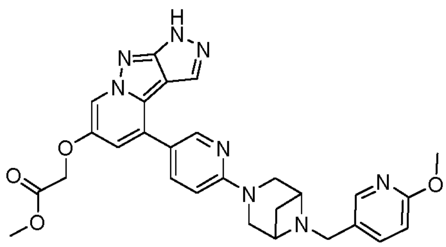 |
| 96 | 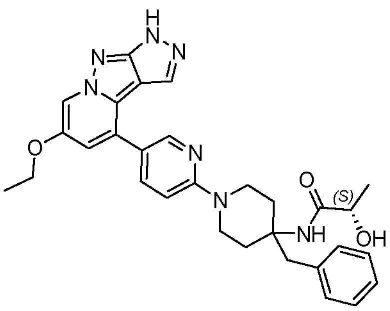 | 229 | 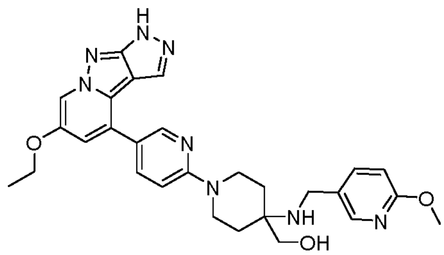 |
| 97 | 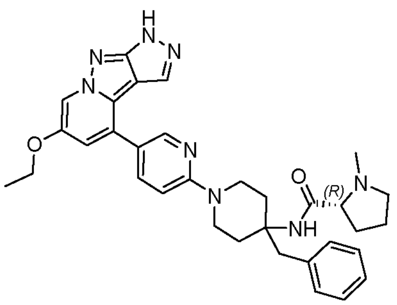 | 230 | 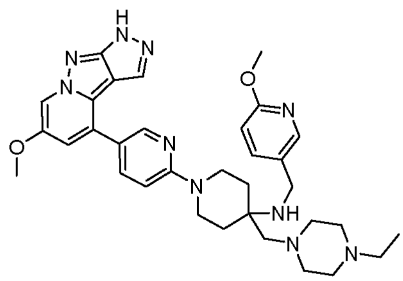 |

(continued)

| No. | Chemical structure | No. | Chemical structure |
|---|---|---|---|
| 98 | 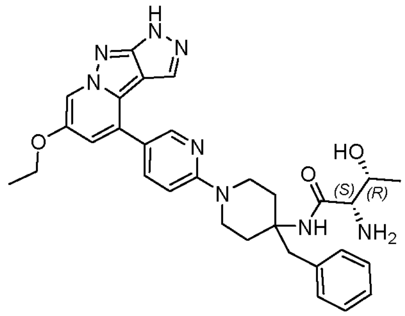 | 231 | 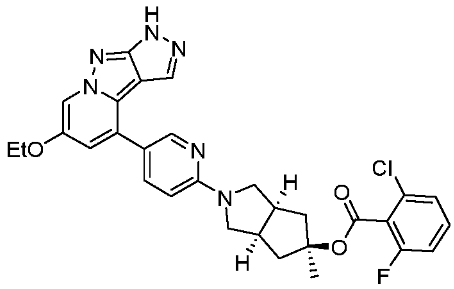 |
| 99 | 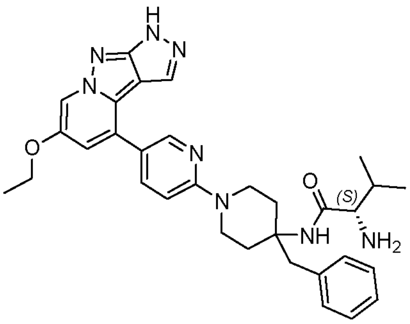 | 232 | 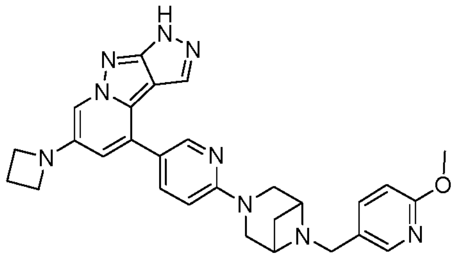 |
| 100 | 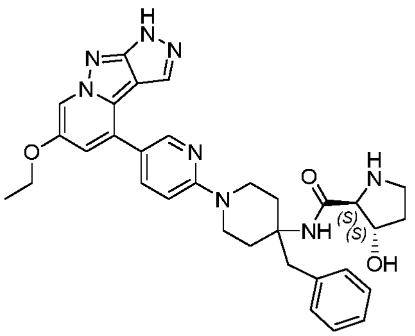 | 233 | 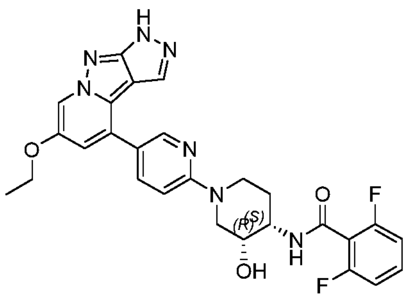 |
| 101 | 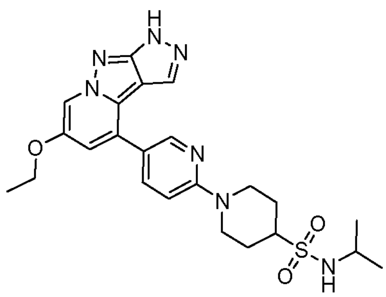 | 234 | 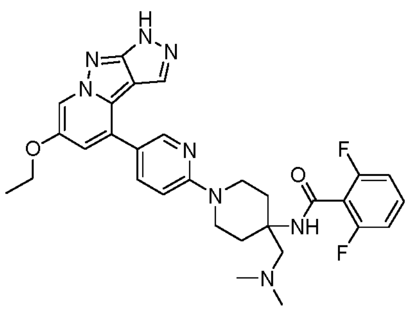 |
| 102 | 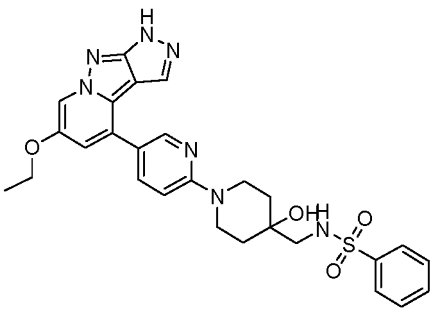 | 235 | 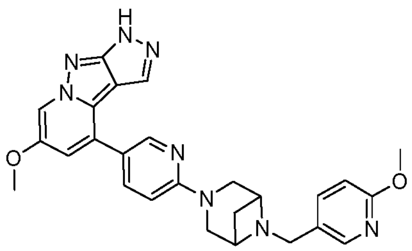 |

(continued)

| No. | Chemical structure | No. | Chemical structure |
|---|---|---|---|
| 103 | 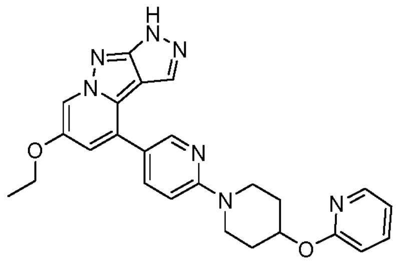 | 236 | 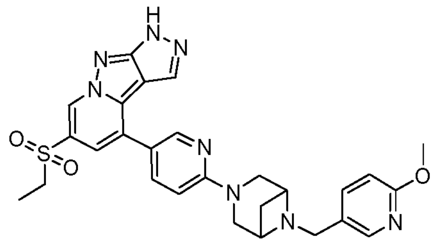 |
| 104 | 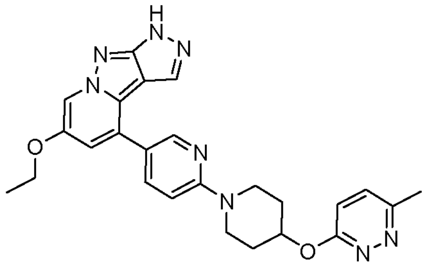 | 237 | 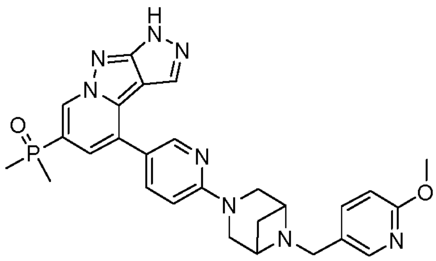 |
| 105 | 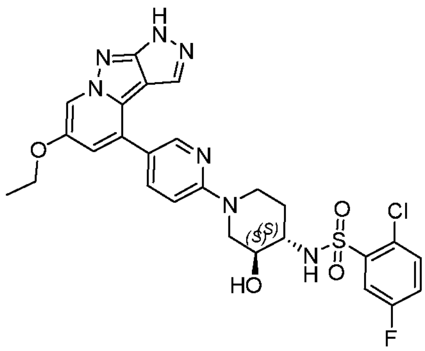 | 238 | 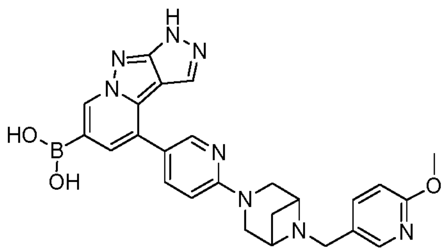 |
| 106 | 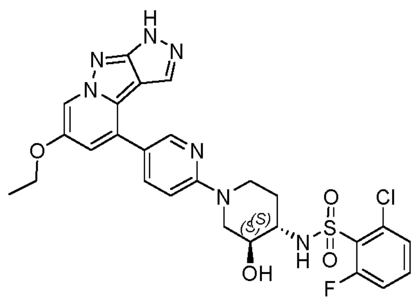 | 239 | 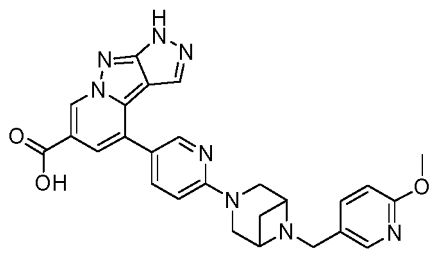 |
| 107 | 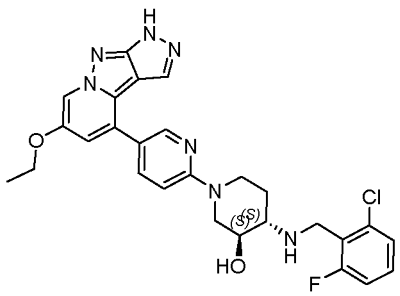 | 240 | 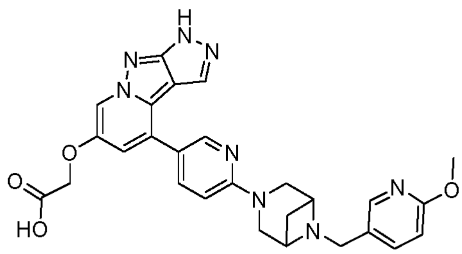 |

(continued)

| No. | Chemical structure | No. | Chemical structure |
|---|---|---|---|
| 108 | 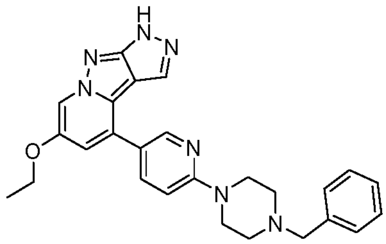 | 241 | 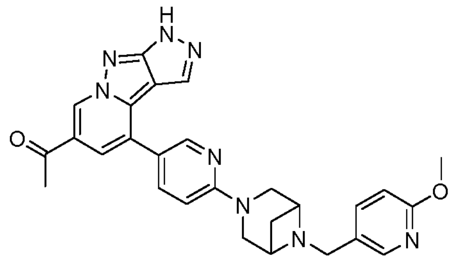 |
| 109 | 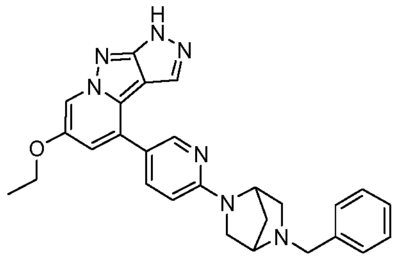 | 242 | 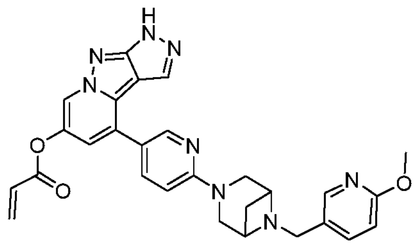 |
| 110 | 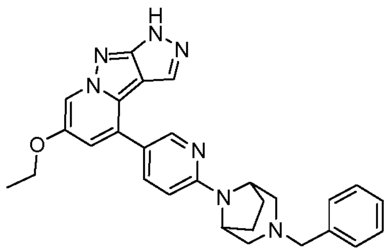 | 243 | 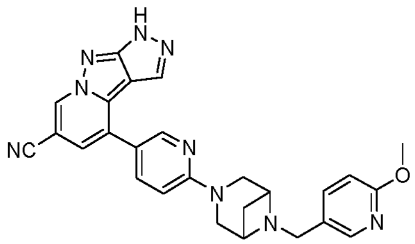 |
| 111 | 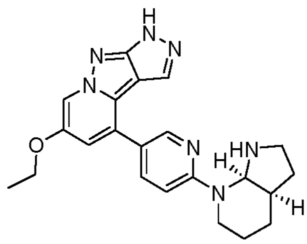 | 244 | 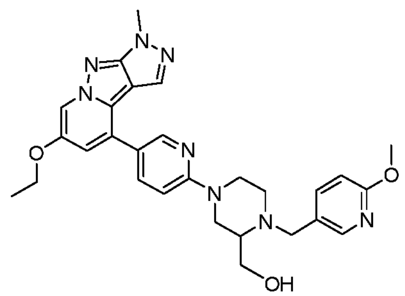 |
| 112 | 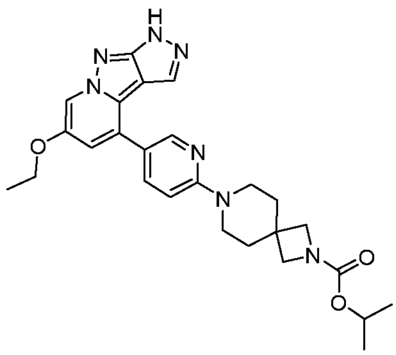 | 245 | 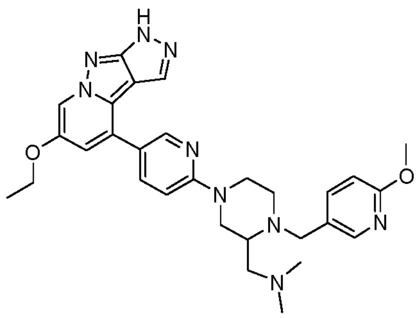 |

(continued)

| No. | Chemical structure | No. | Chemical structure |
|---|---|---|---|
| 113 | 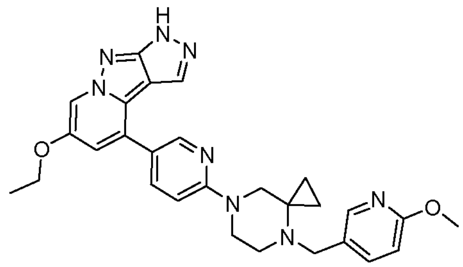 | 246 | 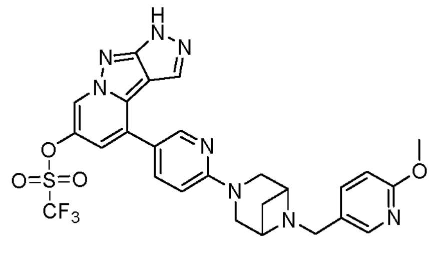 |
| 114 | 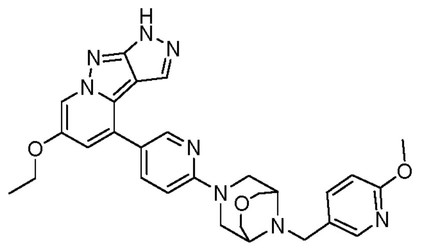 | 247 | 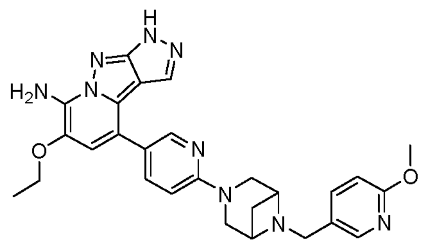 |
| 115 | 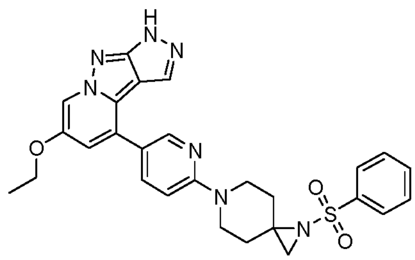 | 248 | 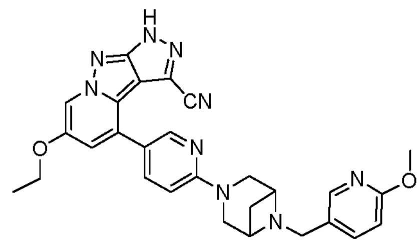 |
| 116 | 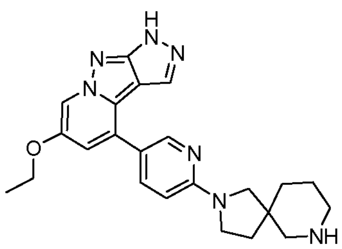 | 249 | 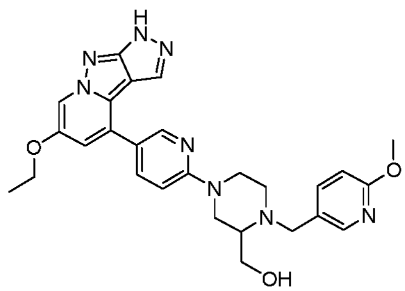 |
| 117 | 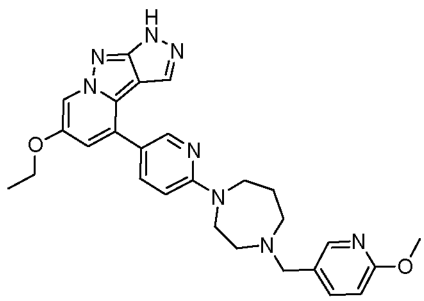 | 250 | 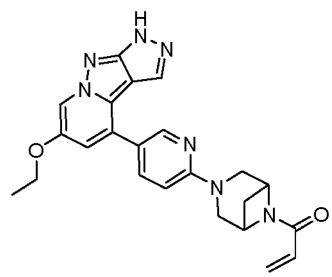 |

(continued)

| No. | Chemical structure | No. | Chemical structure |
|---|---|---|---|
| 118 | 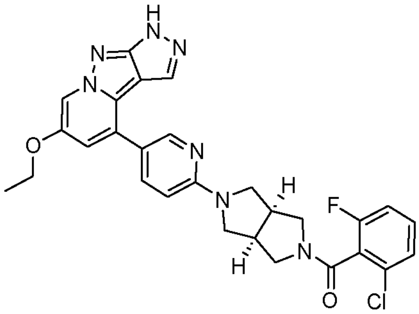 | 251 | 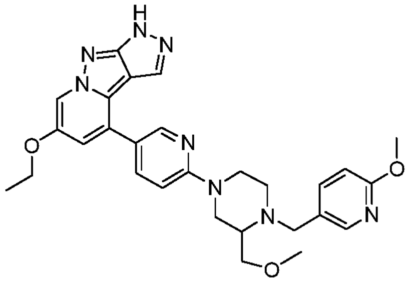 |
| 119 | 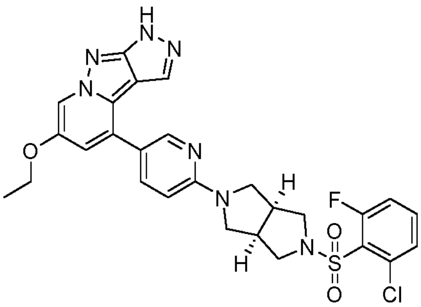 | 252 | 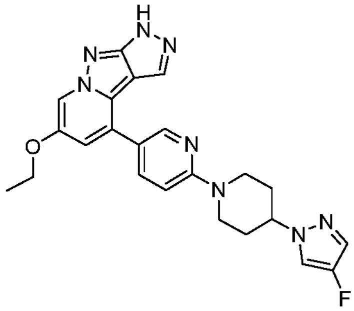 |
| 120 | 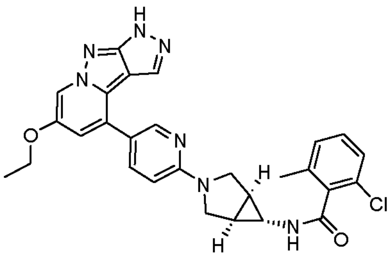 | 253 | 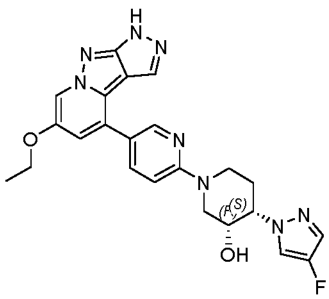 |
| 121 | 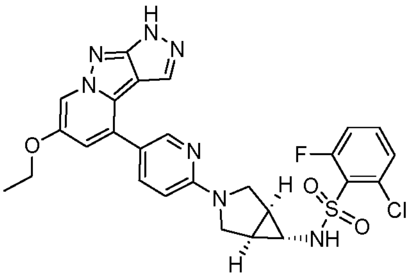 | 254 | 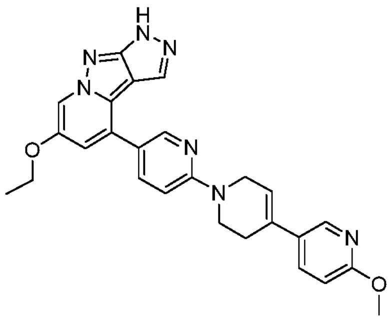 |
| 122 | 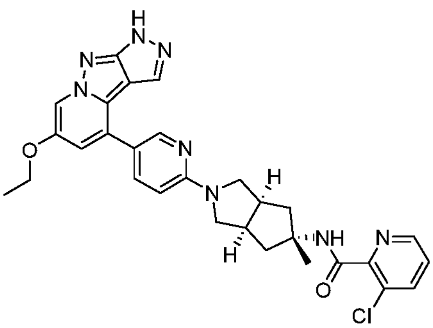 | 255 | 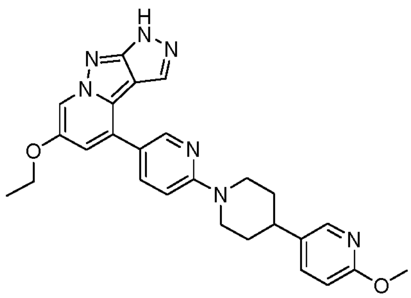 |

(continued)

| No. | Chemical structure | No. | Chemical structure |
|---|---|---|---|
| 123 | 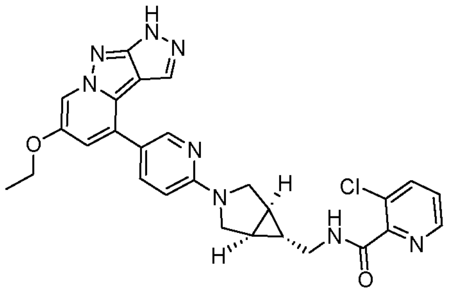 | 256 | 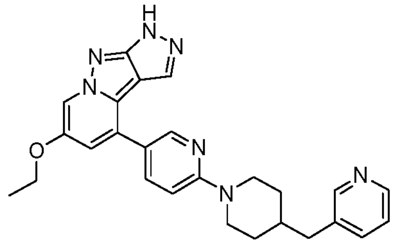 |
| 124 | 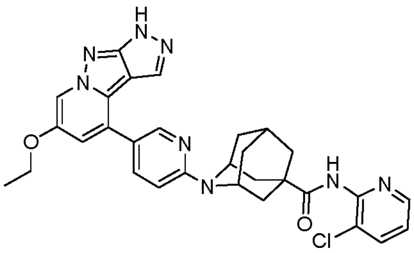 | 257 | 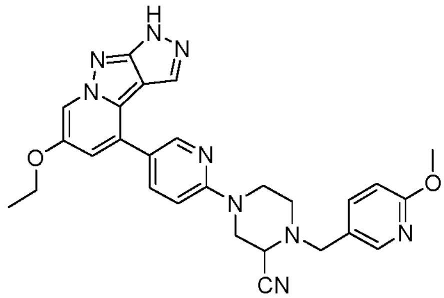 |
| 125 | 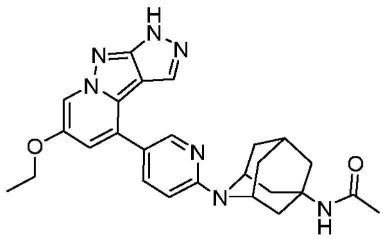 | 258 | 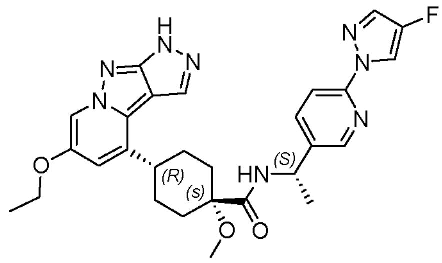 |
| 126 | 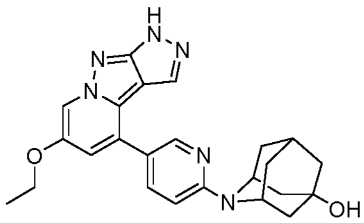 | 259 | 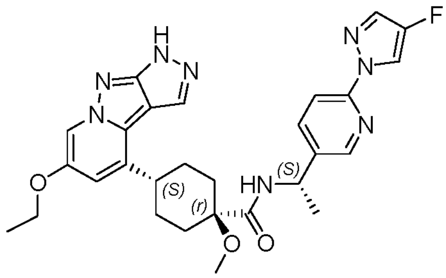 |
| 127 | 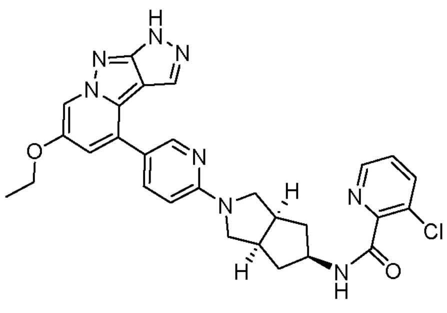 | 260 | 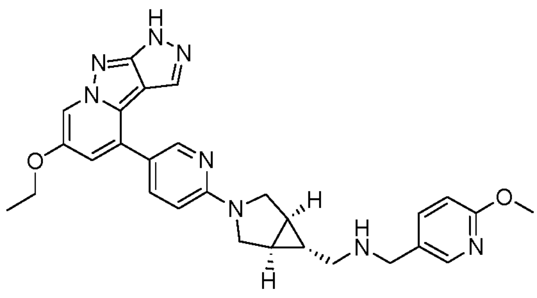 |

(continued)

| No. | Chemical structure | No. | Chemical structure |
|---|---|---|---|
| 128 | 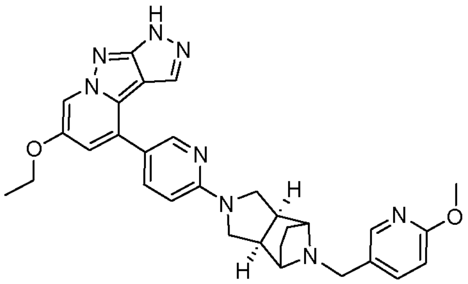 | 261 | 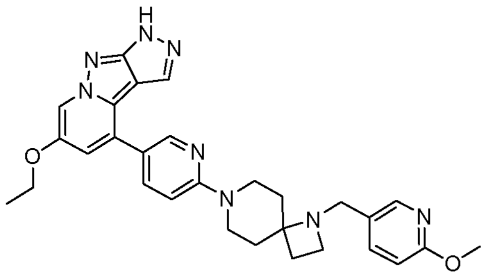 |
| 129 | 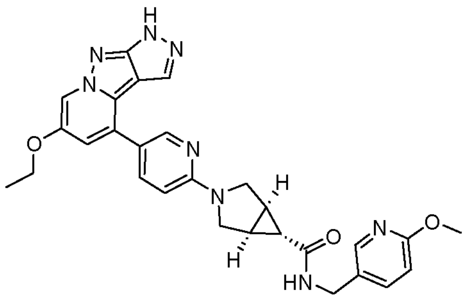 | 262 | 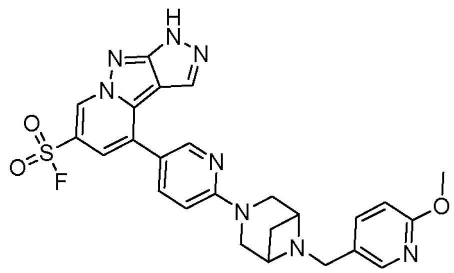 |
| 130 | 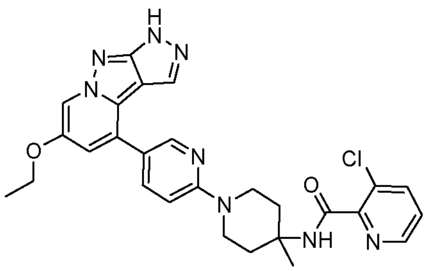 | 263 | 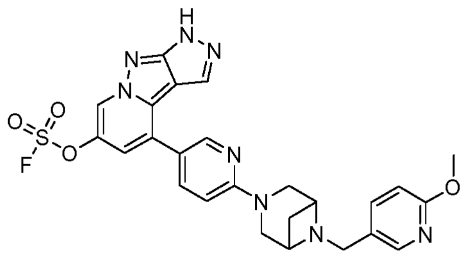 |
| 131 | 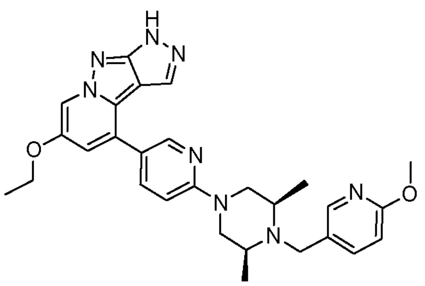 | 264 | 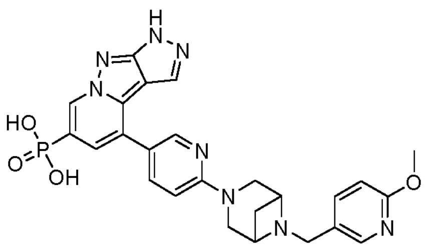 |
| 132 | 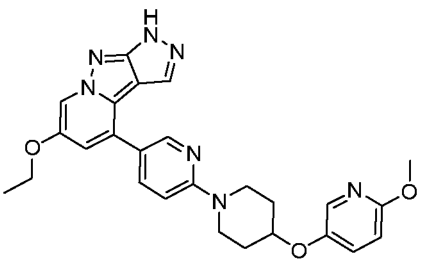 | 265 | 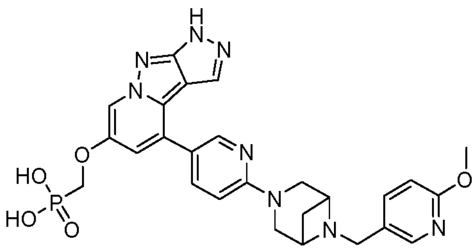 |

(continued)

| No. | Chemical structure | No. | Chemical structure |
|---|---|---|---|
| 133 | 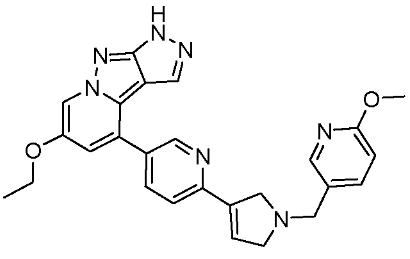 | 266 | 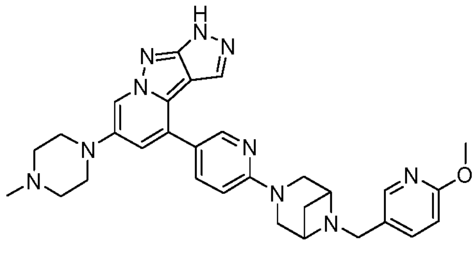 |
| 267 | 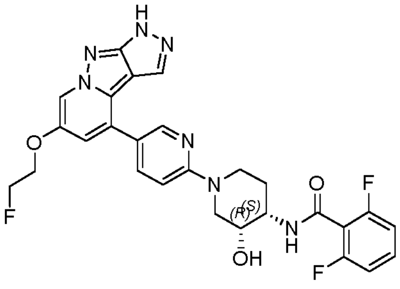 | 283 | 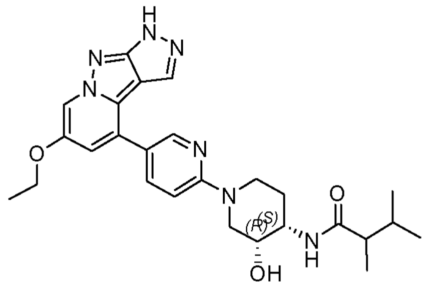 |
| 268 | 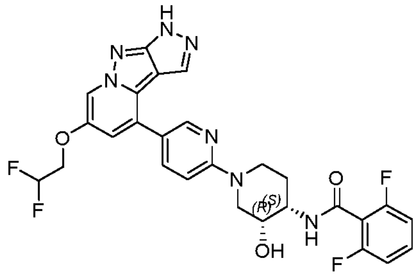 | 284 | 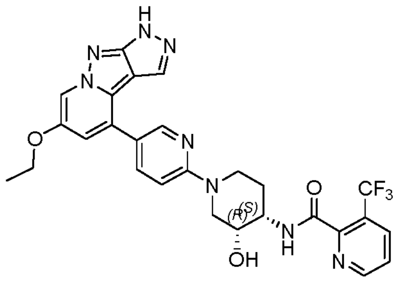 |
| 269 | 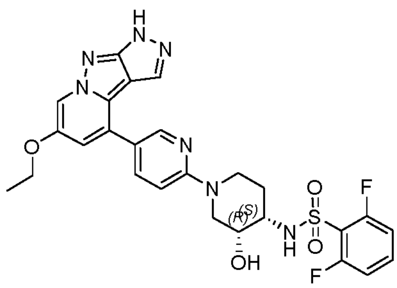 | 285 | 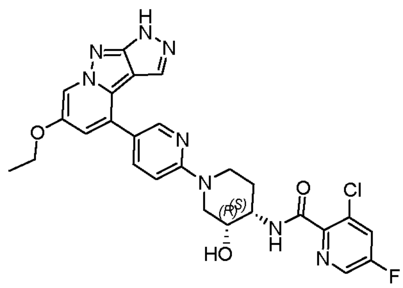 |
| 270 | 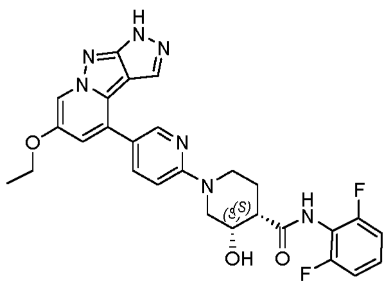 | 286 | 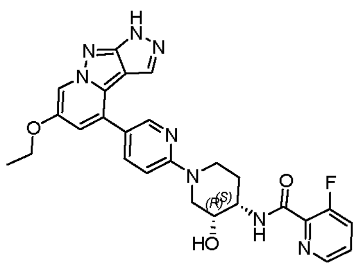 |

(continued)

| No. | Chemical structure | No. | Chemical structure |
|---|---|---|---|
| 271 | 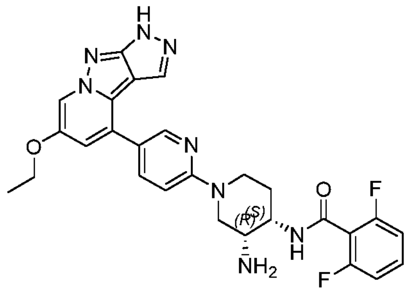 | 287 | 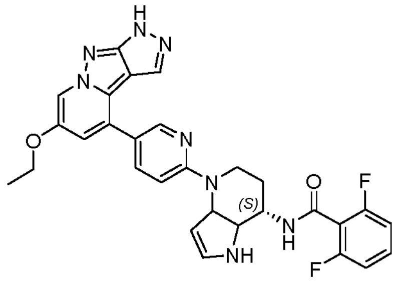 |
| 272 | 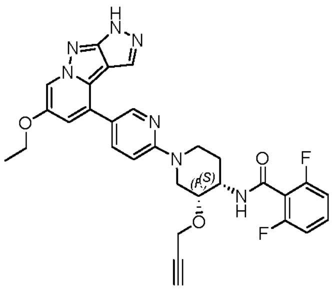 | 288 | 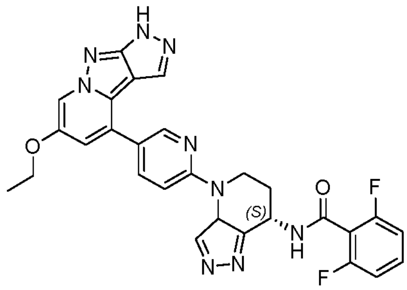 |
| 273 | 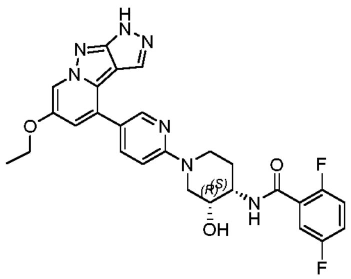 | 289 | 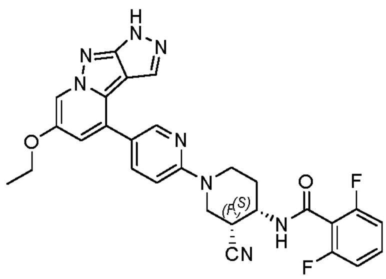 |
| 274 | 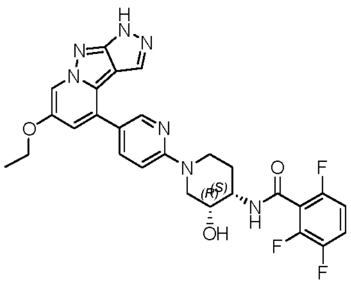 | 290 | 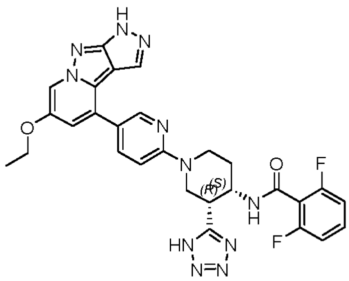 |
| 275 | 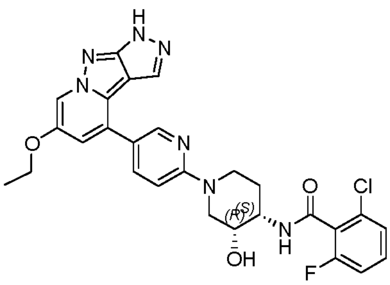 | 291 | 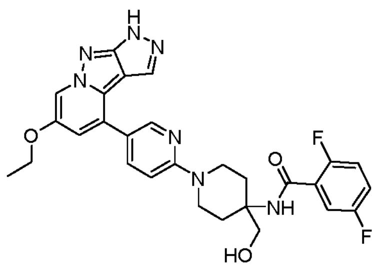 |

(continued)

| No. | Chemical structure | No. | Chemical structure |
|---|---|---|---|
| 276 | 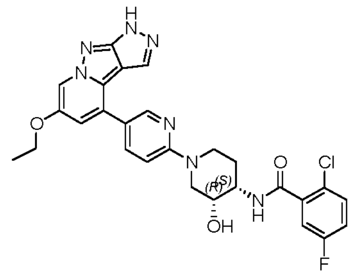 | 292 | 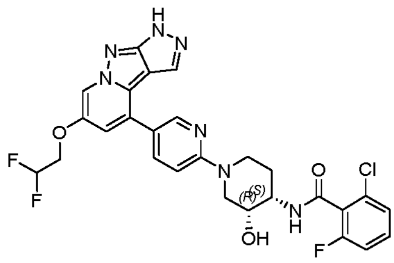 |
| 277 | 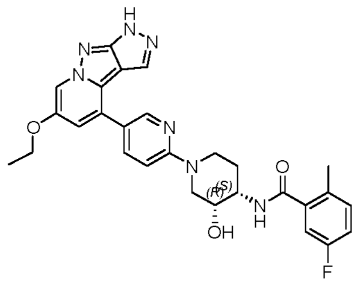 | 293 | 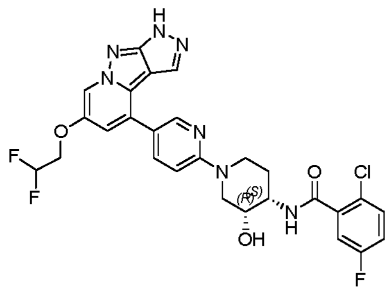 |
| 278 | 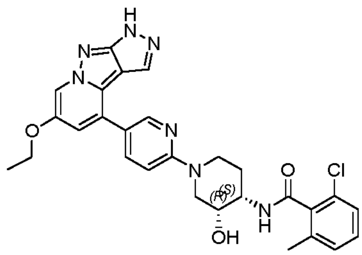 | 294 | 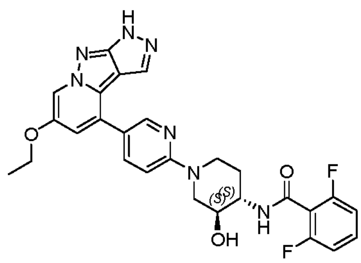 |
| 279 | 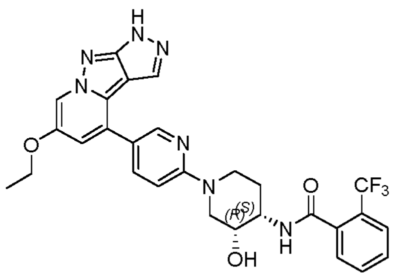 | 295 | 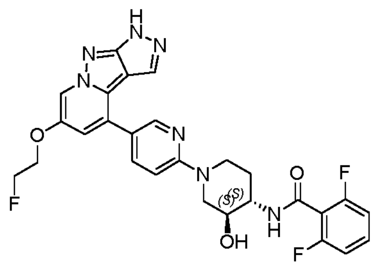 |
| 280 | 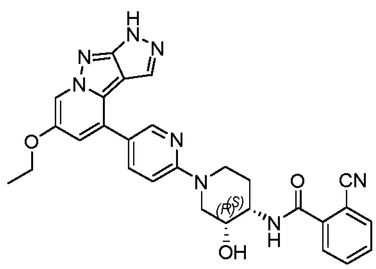 | 296 | 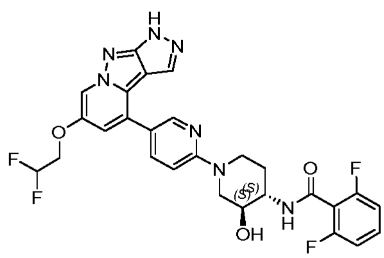 |

(continued)

| No. | Chemical structure | No. | Chemical structure |
|---|---|---|---|
| 281 | 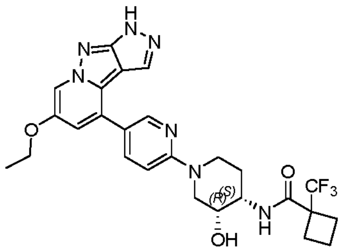 | 297 | 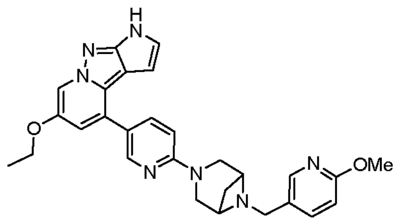 |
| 282 | 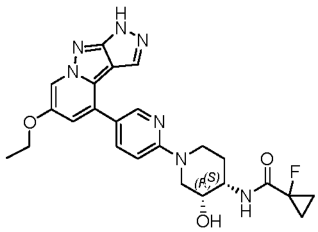 | 298 | 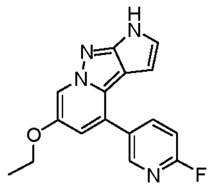 |

**5.** A preparation method for the compound as claimed in any one of claims 1-4, comprising the following steps:

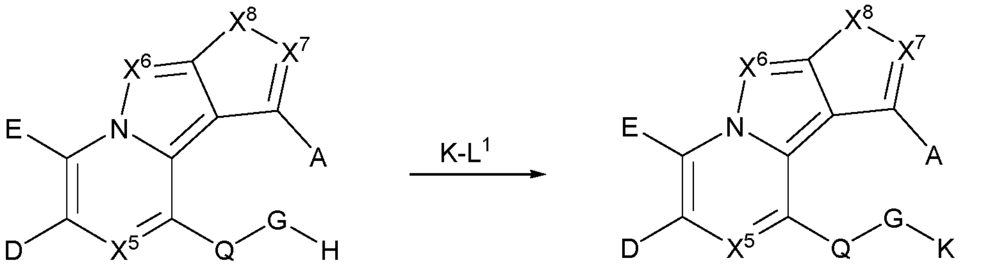


I-1 I

wherein A, D, E, Q, G, K, $X^5$, $X^6$, $X^7$, and $X^8$ are as defined in any one of claims 1-4; $L^1$ is selected from a leaving group;
or, the preparation method comprises the following steps:

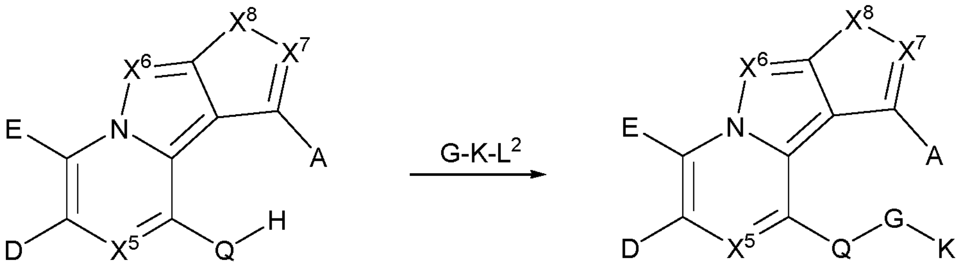


I-2 I

wherein A, D, E, Q, G, K, $X^5$, $X^6$, $X^7$, and $X^8$ are as defined in any one of claims 1-4; $L^2$ is selected from a leaving group;
or, the preparation method comprises the following steps:

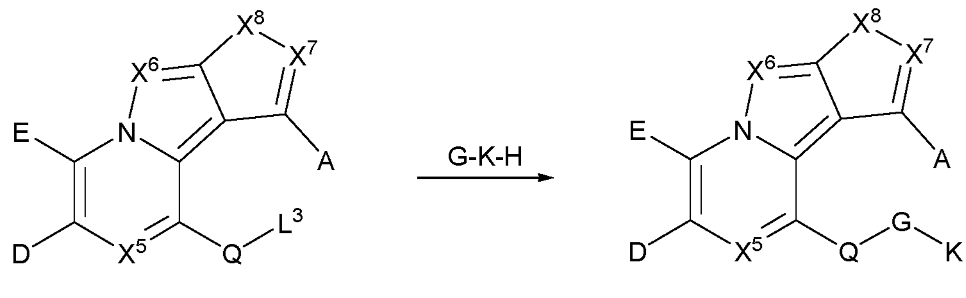


I-3 I

wherein A, D, E, Q, G, K, $X^5$, $X^6$, $X^7$, and $X^8$ are as defined in any one of claims 1-4; $L^3$ is selected from a leaving group;

or, the preparation method comprises reacting a compound represented by formula I substituted with a protecting group under a condition where the protecting group is removed to give the compound represented by formula I.

**6.** A preparation method for a compound represented by formula II, wherein the preparation method comprises the following steps: reacting a compound represented by formula II-1 with a compound $R^{21}$-L to give the compound represented by formula II:

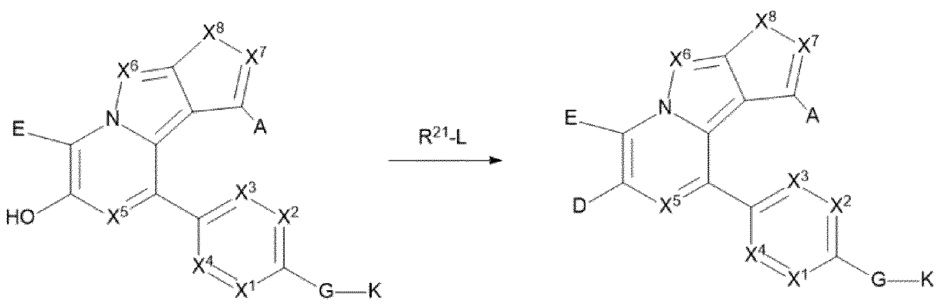


II-1 II

wherein A, E, G, K, $X^1$, $X^2$, $X^3$, $X^4$, $X^5$, $X^6$, $X^7$, $X^8$, and $R^{21}$ are as defined in any one of claims 1-4; D is selected from -O-$R^{21}$; L is selected from a leaving group; L is selected from a leaving group;

preferably, a preparation method for the compound represented by formula II-1 comprises reacting a compound represented by formula II-2 to give the compound represented by formula II-1:

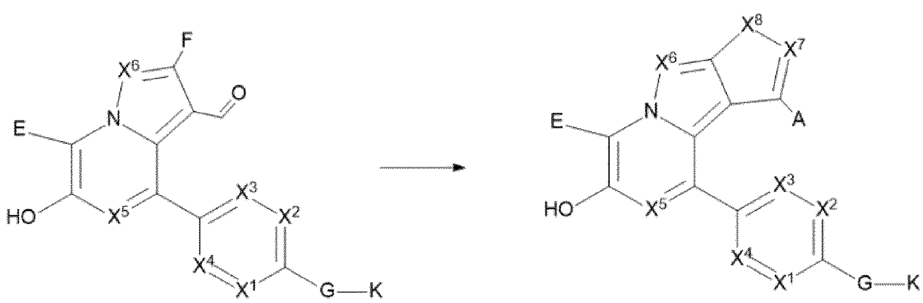

II-2 II-1

wherein A, D, E, G, K, $X^1$, $X^2$, $X^3$, $X^4$, $X^5$, $X^6$, $X^7$, and $X^8$ are as defined in any one of claims 1-4.

**7.** A compound selected from the compound represented by formula I-1, formula I-2, or formula I-3 as claimed in claim 5 and the compound represented by formula II-1 or formula II-2 as claimed in claim 6.

**8.** Use of the compound as claimed in claim 7 for manufacturing the compound represented by formula I as claimed in any one of claims of 1-4.

**9.** A pharmaceutical composition, comprising at least one selected from a compound represented by formula I, and a stereoisomer, a racemate, a tautomer, an isotopically labeled compound, a nitrogen oxide and a pharmaceutically acceptable salt thereof, e.g., a therapeutically effective amount of at least one selected from the compound represented by formula I, and the stereoisomer, the racemate, the tautomer, the isotopically labeled compound, the nitrogen oxide, and the pharmaceutically acceptable salt as claimed in any one of claims 1-4;
wherein preferably, the pharmaceutical composition further comprises one, two, or more pharmaceutically acceptable auxiliary materials, such as carriers and/or excipients.

**10.** Use of at least one of the compound represented by formula I, or the stereoisomer, the racemate, the tautomer, the isotopically labeled compound, the nitrogen oxide or the pharmaceutically acceptable salt thereof as claimed in any one of claims 1-4 for manufacturing a medicament for treating a RET kinase-mediated disease, inhibiting RET kinase activity, treating a cancer and/or inhibiting a metastasis associated with the cancer, treating irritable bowel syndrome (IBS) or a pain associated with IBS, providing supportive care for a cancer patient, treating a disease or condition associated with RET, reversing or preventing acquired resistance to an anti-cancer drug, or delaying and/or preventing the development of resistance to an anti-cancer drug in an individual or an increased possibility of developing resistance to an anti-cancer drug;

wherein preferably, the support care comprises preventing or minimizing a gastrointestinal condition associated with a treatment (including a chemotherapy treatment), e.g., diarrhea; preferably, the disease or condition associated with RET is selected from a disease mediated by a RET gene and/or a RET kinase, wherein the RET, the RET gene, or the RET kinase is selected from RET genes and RET kinases including but not limited to, RET-wt, V804M, V804L, V804E, G810R, G810S, G810C, G810V, and S904F;
preferably, the cancer is selected from a hematological cancer and a solid tumor.

## INTERNATIONAL SEARCH REPORT

International application No.
**PCT/CN2022/073467**

**A. CLASSIFICATION OF SUBJECT MATTER**

A61K 31/444(2006.01)i; C07D 519/00(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; C07D; A61P

Documentation searched other than minimum documentation to the extent that such documents are included in the fields searched

Electronic data base consulted during the international search (name of data base and, where practicable, search terms used)

CNTXT; CNABS; DWPI; SIPOABS; CNKI; Web of Science; STNext: RET, 肿瘤, 癌, 志健金瑞, TYROSINE KINASE RECEPTOR RET, cancer, tumor, structure search based on general formula compounds

**C. DOCUMENTS CONSIDERED TO BE RELEVANT**

| Category* | Citation of document, with indication, where appropriate, of the relevant passages | Relevant to claim No. |
|---|---|---|
| PX | WO 2021023209 A1 (APPLIED PHARMACEUTICAL SCIENCE, INC.) 11 February 2021 (2021-02-11) claims 1-107 | 1-10 |
| A | CN 110382494 A (ARRAY BIOPHARMA, INC.) 25 October 2019 (2019-10-25) claims 1-146, description pages 236-440 embodiment compounds 1-561 | 1-10 |
| X | CN 110382494 A (ARRAY BIOPHARMA, INC.) 25 October 2019 (2019-10-25) claims 1-146, description pages 236-440 embodiment compounds 1-561 | 7 |
| A | WO 2020064009 A1 (APPLIED PHARMACEUTICAL SCIENCE, INC.) 02 April 2020 (2020-04-02) claims 1-10, description pages 32-67 embodiment compounds 1-72 | 1-10 |
| X | WO 2020064009 A1 (APPLIED PHARMACEUTICAL SCIENCE, INC.) 02 April 2020 (2020-04-02) claims 1-10, description pages 32-67 embodiment compounds 1-72 | 7 |
| A | WO 2020233641 A1 (ZHEJIANG TONGYUANKANG MEDICINE CO., LTD.) 26 November 2020 (2020-11-26) claims 1-14 | 1-10 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 April 2022** | **22 April 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/073467**

**C. DOCUMENTS CONSIDERED TO BE RELEVANT**

| Category* | Citation of document, with indication, where appropriate, of the relevant passages | Relevant to claim No. |
|---|---|---|
| X | WO 2020233641 A1 (ZHEJIANG TONGYUANKANG MEDICINE CO., LTD.) 26 November 2020 (2020-11-26)<br>claims 1-14 | 7 |
| A | US 2009042876 A1 (PIERRE FABRE MEDICAMENT) 12 February 2009 (2009-02-12)<br>abstract, claims 1-22, description pages 121-122 table VII compounds 265, 268, page 124 table VIII compound 277 | 1-10 |
| A | CN 108349969 A (ARRAY BIOPHARMA, INC.) 31 July 2018 (2018-07-31)<br>claims 1-160 | 1-10 |
| A | WO 2020228756 A1 (Shanghai Hansoh BioMedical Co., Ltd. et al.) 19 November 2020 (2020-11-19)<br>claims 1-35 | 1-10 |
| A | CN 111285874 A (GUANGDONG HEC PHARMACEUTICAL) 16 June 2020 (2020-06-16)<br>claims 1-29 | 1-10 |
| A | CN 110267960 A (ARRAY BIOPHARMA, INC.) 20 September 2019 (2019-09-20)<br>claims 1-136 | 1-10 |

Form PCT/ISA/210 (second sheet) (January 2015)

**INTERNATIONAL SEARCH REPORT**
**Information on patent family members**

International application No.
**PCT/CN2022/073467**

| Patent document cited in search report | Publication date (day/month/year) | Patent family member(s) | Publication date (day/month/year) |
|---|---|---|---|
| WO 2021023209 A1 | 11 February 2021 | None | |
| CN 110382494 A | 25 October 2019 | IL 277576 D0 | 30 November 2020 |
| | | DK 3523301 T3 | 03 August 2020 |
| | | JP 2021035944 A | 04 March 2021 |
| | | RS 60536 B1 | 31 August 2020 |
| | | CL 2019000941 A1 | 13 December 2019 |
| | | MD 3523301 T2 | 30 November 2020 |
| | | KR 20190076976 A | 02 July 2019 |
| | | EP 3753939 A1 | 23 December 2020 |
| | | PL 3523301 T3 | 28 December 2020 |
| | | UA 125030 C2 | 29 December 2021 |
| | | JO P20190076 A1 | 16 June 2017 |
| | | EC SP19032676 A | 31 July 2019 |
| | | US 2018134702 A1 | 17 May 2018 |
| | | AU 2017342022 A1 | 23 May 2019 |
| | | MX 2019004205 A | 21 August 2019 |
| | | US 2018133213 A1 | 17 May 2018 |
| | | PE 20190918 A1 | 26 June 2019 |
| | | LT 3523301 T | 27 July 2020 |
| | | US 2018133200 A1 | 17 May 2018 |
| | | DO P2019000090 A | 30 June 2019 |
| | | US 2021186959 A1 | 24 June 2021 |
| | | PT 3523301 T | 26 August 2020 |
| | | HU E051424 T2 | 01 March 2021 |
| | | TW 201825488 A | 16 July 2018 |
| | | BR 112019007144 A2 | 19 April 2018 |
| | | PH 12019500775 A1 | 01 July 2019 |
| | | CO 2019004650 A2 | 21 May 2019 |
| | | ES 2805087 T3 | 10 February 2021 |
| | | JP 2020503247 A | 30 January 2020 |
| | | IL 265916 D0 | 30 June 2019 |
| | | EP 3523301 A1 | 14 August 2019 |
| | | US 10953005 B1 | 23 March 2021 |
| | | SI 3523301 T1 | 31 August 2020 |
| | | HR P20201008 T1 | 16 October 2020 |
| | | WO 2018071447 A1 | 19 April 2018 |
| | | CA 3039760 A1 | 19 April 2018 |
| | | TN 2019000110 A1 | 05 October 2020 |
| | | CR 20190218 A | 14 October 2019 |
| | | US 2019183886 A1 | 20 June 2019 |
| | | SG 11201903144 P A | 30 May 2019 |
| WO 2020064009 A1 | 02 April 2020 | KR 20210070286 A | 14 June 2021 |
| | | JP 2022508533 A | 19 January 2022 |
| | | CN 112771043 A | 07 May 2021 |
| | | EP 3845531 A1 | 07 July 2021 |
| | | US 2021379056 A1 | 09 December 2021 |
| | | CN 110964008 A | 07 April 2020 |
| WO 2020233641 A1 | 26 November 2020 | CN 113784963 A | 10 December 2021 |
| | | AU 2020280699 A1 | 27 January 2022 |
| US 2009042876 A1 | 12 February 2009 | DE 602006014405 D1 | 01 July 2010 |

Form PCT/ISA/210 (patent family annex) (January 2015)

**INTERNATIONAL SEARCH REPORT**
**Information on patent family members**

International application No.
**PCT/CN2022/073467**

| Patent document cited in search report | | | Publication date (day/month/year) | Patent family member(s) | | | Publication date (day/month/year) |
|---|---|---|---|---|---|---|---|
| | | | | JP | 2008545001 | A | 11 December 2008 |
| | | | | TW | 200740808 | A | 01 November 2007 |
| | | | | ES | 2346564 | T3 | 18 October 2010 |
| | | | | EP | 1899335 | A1 | 19 March 2008 |
| | | | | WO | 2007003611 | A1 | 11 January 2007 |
| | | | | AR | 054813 | A1 | 18 July 2007 |
| | | | | CA | 2613211 | A1 | 11 January 2007 |
| | | | | AT | 468338 | T | 15 June 2010 |
| | | | | FR | 2887881 | A1 | 05 January 2007 |
| CN | 108349969 | A | 31 July 2018 | US | 2019127373 | A1 | 02 May 2019 |
| | | | | LT | 3322706 | T | 10 March 2021 |
| | | | | HR | P20210177 | T1 | 19 March 2021 |
| | | | | US | 2019127374 | A1 | 02 May 2019 |
| | | | | JP | 2018532690 | A | 08 November 2018 |
| | | | | PT | 3322706 | T | 08 March 2021 |
| | | | | AU | 2016291676 | A1 | 08 February 2018 |
| | | | | IL | 256898 | D0 | 29 March 2018 |
| | | | | EA | 201890318 | A1 | 31 August 2018 |
| | | | | CA | 2992586 | A1 | 19 January 2017 |
| | | | | AU | 2001291676 | A1 | 30 May 2002 |
| | | | | US | 2018186791 | A1 | 05 July 2018 |
| | | | | US | 2018186790 | A1 | 05 July 2018 |
| | | | | HK | 1258905 | A1 | 22 November 2019 |
| | | | | MX | 2018000577 | A | 05 September 2018 |
| | | | | US | 2017096425 | A1 | 06 April 2017 |
| | | | | RS | 61485 | B1 | 31 March 2021 |
| | | | | ZA | 201800771 | B | 26 May 2021 |
| | | | | KR | 20180041135 | A | 23 April 2018 |
| | | | | PL | 3322706 | T4 | 19 July 2021 |
| | | | | DK | 3322706 | T3 | 01 February 2021 |
| | | | | CL | 2018000119 | A1 | 29 June 2018 |
| | | | | EP | 3322706 | A1 | 23 May 2018 |
| | | | | SI | 3322706 | T1 | 30 April 2021 |
| | | | | PH | 12018500121 | A1 | 23 July 2018 |
| | | | | ES | 2857081 | T3 | 28 September 2021 |
| | | | | BR | 112018000808 | A2 | 04 September 2018 |
| | | | | WO | 2017011776 | A1 | 19 January 2017 |
| | | | | US | 2019127375 | A1 | 02 May 2019 |
| | | | | HU | E053067 | T2 | 28 June 2021 |
| | | | | TN | 2018000027 | A1 | 08 July 2019 |
| | | | | US | 2018179203 | A1 | 28 June 2018 |
| WO | 2020228756 | A1 | 19 November 2020 | BR | 112021022897 | A2 | 25 January 2022 |
| | | | | TW | 202108582 | A | 01 March 2021 |
| | | | | KR | 20220008322 | A | 20 January 2022 |
| | | | | CA | 3137887 | A1 | 19 November 2020 |
| | | | | AU | 2020276802 | A1 | 06 January 2022 |
| | | | | EP | 3971187 | A1 | 23 March 2022 |
| | | | | CN | 112368283 | A | 12 February 2021 |
| CN | 111285874 | A | 16 June 2020 | JP | 2022510689 | A | 27 January 2022 |
| | | | | WO | 2020114494 | A1 | 11 June 2020 |

Form PCT/ISA/210 (patent family annex) (January 2015)

**INTERNATIONAL SEARCH REPORT**
**Information on patent family members**

International application No.
**PCT/CN2022/073467**

| Patent document cited in search report | Publication date (day/month/year) | Patent family member(s) | Publication date (day/month/year) |
|---|---|---|---|
| | | CA 3117850 A1 | 11 June 2020 |
| | | EP 3891148 A1 | 13 October 2021 |
| | | KR 20210100155 A | 13 August 2021 |
| | | SG 11202104686 Q A | 29 June 2021 |
| | | AU 2019394519 A1 | 20 May 2021 |
| CN 110267960 A | 20 September 2019 | EP 3571203 A1 | 27 November 2019 |
| | | CA 3049136 A1 | 26 July 2018 |
| | | US 2020055860 A1 | 20 February 2020 |
| | | JP 2020506902 A | 05 March 2020 |
| | | WO 2018136661 A1 | 26 July 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202110172167

### Non-patent literature cited in the description


- *Cell,* 1990, vol. 60 (4), 557-563
- *hyroid,* 2009, vol. 19 (6), 565-612
- *Endocr Rev,* 2006, vol. 27 (5), 535-560
- *Proc Natl Acad Sci USA,* 2000, vol. 97 (1), 268-273
- *Nat Med,* 2012, vol. 18 (3), 375-377
- *Cancer,* 2013, vol. 119 (8), 1486-1494
- *Genome Res,* 2012, vol. 22 (3), 436-445
- *Cancer,* 2011, vol. 117 (12), 2709-2718
- **QIAN et al.** *Mol Cancer,* 2014, vol. 13, 176
- RET Solvent Front Mutations Mediate Acquired Resistance to Selective RET Inhibition in RET-Driven Malignancies. *Journal of Thoracic Oncology,* 2020, vol. 15 (4), 541-549
- *Nature Reviews Cancer,* 2014, vol. 14, 173-186